(19) Europäisches Patentamt
European Patent Office
Office européen des brevets

(11) EP 1 319 657 A1

(12) **EUROPEAN PATENT APPLICATION**
published in accordance with Art. 158(3) EPC

(43) Date of publication:
**18.06.2003 Bulletin 2003/25**

(21) Application number: **01970187.9**

(22) Date of filing: **21.09.2001**

(51) Int Cl.[7]: **C07D 231/40**, C07D 401/12, C07D 403/12, C07D 405/12, C07D 409/12, C07D 413/12, C07D 417/12, A01N 43/50

(86) International application number:
**PCT/JP01/08242**

(87) International publication number:
**WO 02/024656 (28.03.2002 Gazette 2002/12)**

(84) Designated Contracting States:
**AT BE CH CY DE DK ES FI FR GB GR IE IT LI LU MC NL PT SE TR**
Designated Extension States:
**AL LT LV MK RO SI**

(30) Priority: **22.09.2000 JP 2000289484**
**25.04.2001 JP 2001128225**

(71) Applicant: **Nihon Nohyaku Co., Ltd.**
**Tokyo 103-8236 (JP)**

(72) Inventors:
- **YAMAGUCHI, Hiroshi**
**Osaka-shi, Osaka 543-0052 (JP)**
- **ENDOH, Kazuyoshi**
**Sapporo-shi, Hokkaido 001-0045 (JP)**
- **MACHIYA, Kouzou**
**Sennan-gun, Osaka 590-0452 (JP)**
- **TAKEMOTO, Tsuyosi**
**Kawachinagano-shi, Osaka 586-0024 (JP)**
- **BABA, Koji**
**Kawachinagano-shi, Osaka 586-0044 (JP)**
- **MORIMOTO, Masayuki**
**Kawachinagano-shi, Osaka 586-0024 (JP)**

(74) Representative: **Grünecker, Kinkeldey, Stockmair & Schwanhäusser Anwaltssozietät**
**Maximilianstrasse 58**
**80538 München (DE)**

(54) **N-(4-PYRAZOLYL)AMIDE DERIVATIVES, CHEMICALS FOR AGRICULTURAL AND HORTICULTURAL USE, AND USAGE OF THE SAME**

(57) N-(4-Pyrazolyl)amide derivatives of the general formula (I),

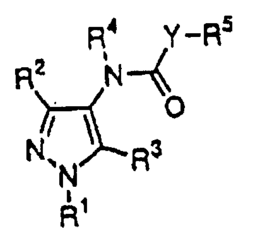

(I)

exhibiting excellent control activities as bacteriocides, fungicides, insecticides, or nematicides for agricultural and horticultural use; chemicals for agricultural and horticultural use; and usage of the same, wherein $R^1$ is H, optionally halogenated ($C_1$-$C_6$)alkyl, optionally substituted phenylsulfonyl, optionally substituted phenyl, or the like; $R^2$ and $R^3$ are each H, halogeno, CN, $NO_2$, OH, SH, $NH_2$, optionally halogenated ($C_1$-$C_6$)alkyl, optionally substituted phenyl, or the like; $R^4$ is H, optionally halogenated ($C_1$-$C_6$)alkyl, cyano($C_1$-$C_6$)alkyl, optionally substituted phenyl($C_1$-$C_6$)alkyl, or the like; $R^5$ is a group of formula (a) or (b), optionally substituted naphthyl, or the like; and Y is ($C_1$-$C_6$)alkylene, optionally halogenated ($C_1$-$C_6$)alkyl($C_1$-$C_6$)alkyl($C_1$-$C_6$)alkylene, or the like.

EP 1 319 657 A1

## Description

TECHNICAL FIELD

The present invention relates to an N-(4-pyrazolyl)amide derivative represented by the general formula (I), an agricultural and horticultural chemical, especially a germicide, a fungicide, an insecticide, or a nematocide, for an agricultural or horticultural use, and a usage thereof.

BACKGROUND ART

A 5-isothiazoyl amide derivative, a 4-pyridylamide derivative, and a 5-pyrazolylamide derivative are known as a controlling agent for a harmful organism, and are described, for instance, in European Patent Publication Unexamined No. 0623282, WO 95/31444, DE-A-19736545, 19727162, 19630814, 19628569, 19601139 and 19542327, WO 2000/11951 and 2000/06266, DE-A-19750401, 19750402 and 19750403, JP-A-5-221990, J. Agric. Food. Chem., 1977, 45, 1920 and 1999, 47, 3381, WO 2000/20415, etc. However, an N-(4-pyrazolyl) amide derivative according to the present invention has not been described.

DISCLOSURE OF THE INVENTION

The present inventors have intensively studied to develop a novel agricultural chemical, and as a result, they have found that an N-(4-pyrazolyl)amide derivative according to the present invention is a novel compound which has not yet been described in any literature, and is useful as an agricultural and horticultural chemical, especially a germicide, a fungicide, an insecticide, or a nematocide. Thus, the present invention has been accomplished.

Namely, the present invention relates to an N-(4-pyrazolyl)amide derivative represented by the general formula (I) as shown below, an agricultural and horticultural chemical, especially a germicide, a fungicide, an insecticide, or a nematocide ,containing said compound as an active ingredient, and a usage thereof:

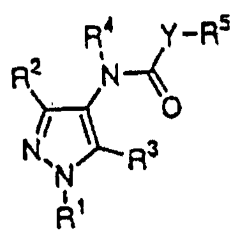

(I)

wherein $R^1$ represents a hydrogen atom; a $(C_1-C_6)$alkyl group; a halo$(C_1-C_6)$alkyl group; a hydroxy$(C_1-C_6)$alkyl group; a cyano$(C_1-C_6)$alkyl group; a formyl$(C_1-C_6)$alkyl group; a $(C_2-C_6)$alkenyl group; a halo$(C_2-C_6)$alkenyl group; a $(C_2-C_6)$ alkynyl group; a halo$(C_2-C_6)$alkynyl group; a $(C_1-C_6)$alkoxy$(C_1-C_6)$alkyl group; a halo$(C_1-C_6)$alkoxy$(C_1-C_6)$alkyl group; a $(C_1-C_6)$alkylthio$(C_1-C_6)$-alkyl group; a halo$(C_1-C_6)$alkylthio$(C_1-C_6)$alkyl group; a $(C_1-C_6)$alkylsulfinyl$(C_1-C_6)$alkyl group; a halo$(C_1-C_6)$-alkylsulfinyl$(C_1-C_6)$alkyl group; a $(C_1-C_6)$alkylsulfonyl $(C_1-C_6)$alkyl group; a halo$(C_1-C_6)$alkylsulfonyl-$(C_1-C_6)$alkyl group; a mono $(C_1-C_6)$alkylamino$(C_1-C_6)$alkyl group; a di$(C_1-C_6)$alkylamino$(C_1-C_6)$alkyl group whose $(C_1-C_6)$alkyl groups may be the same or different; a $(C_1-C_6)$alkylsulfonyl group; a halo$(C_1-C_6)$alkylsulfonyl group; a mono$(C_1-C_6)$alkylaminosulfonyl group; a di$(C_1-C_6)$alkyl- aminosulfonyl group whose $(C_1-C_6)$alkyl groups may be the same or different; a $(C_1-C_6)$alkyloxycarbonyl$(C_1-C_6)$alkyl group; a mono $(C_1-C_6)$alkylaminocarbonyl$(C_1-C_6)$alkyl group; a di$(C_1-C_6)$alkylaminocarbonyl$(C_1-C_6)$alkyl group whose $(C_1-C_6)$alkyl groups may be the same or different; a $(C_1-C_6)$ alkyloxyimino-$(C_1-C_6)$alkyl group; a $(C_1-C_6)$alkylsulfonyloxy$(C_1-C_6)$alkyl group; a halo$(C_1-C_6)$alkylsulfonyloxy$(C_1-C_6)$ alkyl group; a $(C_1-C_6)$alkylcarbonyloxy$(C_1-C_6)$alkyl group; a phenylsulfonyl group; a substituted phenylsulfonyl group having at least one substituent which may be the same or different and is selected from the group consisting of a halogen atom, a cyano group, a nitro group, a hydroxyl group, an amino group, a mercapto group, a $(C_1-C_6)$alkyl group, a halo$(C_1-C_6)$alkyl group, a $(C_2-C_6)$alkenyl group, a halo$(C_2-C_6)$alkenyl group, a $(C_2-C_6)$alkynyl group, a halo$(C_2-C_6)$ alkynyl group, a $(C_1-C_6)$alkoxyl group, a halo$(C_1-C_6)$alkoxyl group, a $(C_1-C_6)$alkylthio group, a halo$(C_1-C_6)$alkylthio group, a $(C_1-C_6)$alkylsulfinyl group, a halo$(C_1-C_6)$alkylsulfinyl group, a $(C_1-C_6)$ alkylsulfonyl group, a halo$(C_1-C_6)$alkylsulfonyl group, a mono$(C_1-C_6)$alkylamino group, a di$(C_1-C_6)$alkylamino group whose $(C_1-C_6)$alkyl groups may be the same or different, a $(C_1-C_6)$alkylsulfonylamino group, and a halo$(C_1-C_6)$alkylsulfonylamino group; a $(C_3-C_6)$cycloalkyl group; a halo$(C_3-C_6)$cycloalkyl group; a $(C_3-C_6)$cycloalkenyl group; a halo$(C_3-C_6)$cycloalkenyl group; a phenyl group;

a substituted phenyl group having at least one substituent which may be the same or different and is selected from the group consisting of a halogen atom, a cyano group, a nitro group, a hydroxyl group, an amino group, a mercapto group, a $(C_1-C_6)$alkyl group, a halo$(C_1-C_6)$alkyl group, a $(C_2-C_6)$alkenyl group, a halo$(C_2-C_6)$alkenyl group, a $(C_2-C_6)$alkynyl group, a halo$(C_2-C_6)$alkynyl group, a $(C_1-C_6)$alkoxyl group, a halo$(C_1-C_6)$alkoxyl group, a $(C_1-C_6)$alkylthio group, a halo$(C_1-C_6)$alkylthio group, a $(C_1-C_6)$alkylsulfinyl group, a halo$(C_1-C_6)$alkylsulfinyl group, a $(C_1-C_6)$alkylsulfonyl group, a halo$(C_1-C_6)$alkylsulfonyl group, a mono$(C_1-C_6)$alkylamino group, a di$(C_1-C_6)$alkylamino group whose $(C_1-C_6)$alkyl groups may be the same or different, a $(C_1-C_6)$alkylsulfonyl amino group, and a halo$(C_1-C_6)$alkylsulfonylamino group; or a $-COZ_m(R^6)$ group {wherein, $R^6$ represents a hydrogen atom; a $(C_1-C_6)$alkyl group; a halo$(C_1-C_6)$alkyl group; a $(C_2-C_6)$alkenyl group; a halo$(C_2-C_6)$alkenyl group; a $(C_2-C_6)$alkynyl group; a halo$(C_2-C_6)$alkynyl group; a $(C_1-C_6)$alkoxy$(C_1-C_6)$alkyl group; a halo$(C_1-C_6)$alkoxy-$(C_1-C_6)$alkyl group; a $(C_1-C_6)$alkylthio $(C_1-C_6)$alkyl group; a halo$(C_1-C_6)$alkylthio $(C_1-C_6)$alkyl group; a $(C_1-C_6)$-alkylsulfinyl $(C_1-C_6)$alkyl group; a halo$(C_1-C_6)$alkylsulfinyl $(C_1-C_6)$alkyl group; a $(C_1-C_6)$alkylsulfonyl-$(C_1-C_6)$alkyl group; a halo$(C_1-C_6)$alkylsulfonyl-$(C_1-C_6)$alkyl group; a mono$(C_1-C_6)$alkylamino $(C_1-C_6)$alkyl group; a di$(C_1-C_6)$alkylamino $(C_1-C_6)$alkyl group whose $(C_1-C_6)$alkyl groups may be the same or different; a $(C_3-C_6)$cycloalkyl group; a halo$(C_3-C_6)$cycloalkyl group; a $(C_3-C_6)$cycloalkenyl group; a halo$(C_3-C_6)$cycloalkenyl group; a phenyl group; or a substituted phenyl group having at least one substituent which may be the same or different and is selected from the group consisting of a halogen atom, a cyano group, a nitro group, a hydroxyl group, an amino group, a mercapto group, a $(C_1-C_6)$alkyl group, a halo$(C_1-C_6)$alkyl group, a $(C_2-C_6)$alkenyl group, a halo $(C_2-C_6)$alkenyl group, a $(C_2-C_6)$alkynyl group, a halo$(C_2-C_6)$alkynyl group, a $(C_1-C_6)$alkoxyl group, a halo$(C_1-C_6)$alkoxyl group, a $(C_1-C_6)$alkylthio group, a halo$(C_1-C_6)$alkylthio group, a $(C_1-C_6)$alkylsulfinyl group, a halo$(C_1-C_6)$alkylsulfinyl group, a $(C_1-C_6)$alkylsulfonyl group, a halo$(C_1-C_6)$alkylsulfonyl group, a mono$(C_1-C_6)$alkylamino group, a di$(C_1-C_6)$alkylamino group whose $(C_1-C_6)$alkyl groups may be the same or different, a $(C_1-C_6)$alkylsulfonylamino group, and a halo $(C_1-C_6)$alkylsulfonylamino group; Z represents an oxygen atom; a sulfur atom; or $N(R^7)$ [wherein, $R^7$ represents a hydrogen atom; a $(C_1-C_6)$alkyl group; a halo$(C_1-C_6)$alkyl group; a $(C_2-C_6)$alkenyl group; a halo$(C_2-C_6)$alkenyl group; a $(C_2-C_6)$alkynyl group; a halo$(C_2-C_6)$alkynyl group; a $(C_1-C_6)$alkoxy $(C_1-C_6)$alkyl group; a halo$(C_1-C_6)$alkoxy$(C_1-C_6)$alkyl group; a $(C_1-C_6)$alkylthio$(C_1-C_6)$alkyl group; a halo$(C_1-C_6)$-alkylthio$(C_1-C_6)$alkyl group; a $(C_1-C_6)$alkylsulfinyl-$(C_1-C_6)$alkyl group; a halo$(C_1-C_6)$alkylsulfinyl$(C_1-C_6)$alkyl group; a $(C_1-C_6)$alkylsulfonyl$(C_1-C_6)$alkyl group; a halo $(C_1-C_6)$alkylsulfonyl$(C_1-C_6)$alkyl group; a mono-$(C_1-C_6)$alkylamino$(C_1-C_6)$alkyl group; a di $(C_1-C_6)$alkylamino$(C_1-C_6)$alkyl group whose $(C_1-C_6)$alkyl groups may be the same or different; a $(C_3-C_6)$cycloalkyl group; a halo$(C_3-C_6)$cycloalkyl group; a $(C_3-C_6)$cycloalkenyl group; or a halo$(C_3-C_6)$cycloalkenyl group]; and m represents an integer of 0 to 1};

$R^2$ and $R^3$ may be same or different, and represent a hydrogen atom, a halogen atom; a cyano group; a nitro group; a hydroxyl group; a mercapto group; an amino group; a $(C_1-C_6)$alkyl group; a halo-$(C_1-C_6)$alkyl group; a $(C_2-C_6)$alkenyl group; a halo$(C_2-C_6)$-alkenyl group; a $(C_2-C_6)$alkynyl group; a halo$(C_2-C_6)$-alkynyl group; a $(C_1-C_6)$alkoxyl group; a halo$(C_1-C_6)$-alkoxyl group; a $(C_1-C_6)$alkylthio group; a halo$(C_1-C_6)$-alkylthio group; a $(C_1-C_6)$alkylsulfinyl group; a halo$(C_1-C_6)$alkylsulfinyl group; a $(C_1-C_6)$alkylsulfonyl group; a halo$(C_1-C_6)$alkylsulfonyl group; a mono $(C_1-C_6)$alkylamino group; a di$(C_1-C_6)$alkylamino group whose $(C_1-C_6)$alkyl groups may be the same or different; a $(C_1-C_6)$alkoxy$(C_1-C_6)$alkyl group; a halo$(C_1-C_6)$alkoxy-$(C_1-C_6)$alkyl group; a $(C_1-C_6)$alkylthio$(C_1-C_6)$alkyl group; a halo$(C_1-C_6)$alkylthio$(C_1-C_6)$alkyl group; a $(C_1-C_6)$alkylsulfinyl $(C_1-C_6)$alkyl group; a halo$(C_1-C_6)$alkylsulfinyl-$(C_1-C_6)$alkyl group; a $(C_1-C_6)$alkylsulfonyl$(C_1-C_6)$alkyl group; a halo$(C_1-C_6)$alkylsulfonyl $(C_1-C_6)$alkyl group; a mono $(C_1-C_6)$alkylamino $(C_1-C_6)$alkyl group; a di $(C_1-C_6)$alkylamino$(C_1-C_6)$alkyl group whose $(C_1-C_6)$alkyl groups may be the same or different; a $(C_3-C_6)$cycloalkyl group; a halo$(C_3-C_6)$cycloalkyl group; a $(C_3-C_6)$cycloalkenyl group; a halo$(C_3-C_6)$cycloalkenyl group; a phenyl group; a substituted phenyl group having at least one substituent which may be the same or different and is selected from the group consisting of a halogen atom, a cyano group, a nitro group, a hydroxyl group, an amino group, a mercapto group, a $(C_1-C_6)$alkyl group, a halo-$(C_1-C_6)$alkyl group, a $(C_2-C_6)$alkenyl group, a halo$(C_2-C_6)$-alkenyl group, a $(C_2-C_6)$alkynyl group, a halo$(C_2-C_6)$-alkynyl group, a $(C_1-C_6)$alkoxyl group, a halo$(C_1-C_6)$-alkoxyl group, a $(C_1-C_6)$alkylthio group, a halo$(C_1-C_6)$-alkylthio group, a $(C_1-C_6)$alkylsulfinyl group, a halo$(C_1-C_6)$alkylsulfinyl group, a $(C_1-C_6)$alkylsulfonyl group, a halo$(C_1-C_6)$alkylsulfonyl group, a mono$(C_1-C_6)$-alkylamino group, a di$(C_1-C_6)$alkylamino group whose $(C_1-C_6)$alkyl groups may be the same or different, a $(C_1-C_6)$alkylsulfonylamino group, and a halo$(C_1-C_6)$alkylsulfonylamino group; a phenoxy group; a substituted phenoxy group having at least one substituent which may be the same or different and is selected from the group consisting of a halogen atom, a cyano group, a nitro group, a hydroxyl group, an amino group, a mercapto group, a $(C_1-C_6)$alkyl group, a halo$(C_1-C_6)$alkyl group, a $(C_2-C_6)$alkenyl group, a halo$(C_2-C_6)$alkenyl group, a $(C_2-C_6)$alkynyl group, a halo$(C_2-C_6)$alkynyl group, a $(C_1-C_6)$alkoxyl group, a halo $(C_1-C_6)$alkoxyl group, a $(C_1-C_6)$alkylthio group, a halo$(C_1-C_6)$alkylthio group, a $(C_1-C_6)$alkylsulfinyl group, a halo$(C_1-C_6)$alkylsulfinyl group, a $(C_1-C_6)$alkylsulfonyl group, a halo$(C_1-C_6)$alkylsulfonyl group, a mono$(C_1-C_6)$alkylamino group, a di$(C_1-C_6)$alkylamino group whose $(C_1-C_6)$alkyl groups may be the same or different, a $(C_1-C_6)$alkylsulfonylamino group, and a halo$(C_1-C_6)$alkylsulfonylamino group; a phenylthio group; a substituted phenylthio group having at least one substituent which may be the same or different and is selected from the group consisting of a halogen atom, a cyano group, a nitro group, a hydroxyl group, an amino group, a mercapto group, a $(C_1-C_6)$alkyl group, a halo$(C_1-C_6)$alkyl group, a $(C_2-C_6)$alkenyl group, a halo$(C_2-C_6)$alkenyl group, a $(C_2-C_6)$alkynyl group, a halo$(C_2-C_6)$alkynyl group, a

$(C_1-C_6)$alkoxyl group, a halo$(C_1-C_6)$alkoxyl group, a $(C_1-C_6)$alkylthio group, a halo$(C_1-C_6)$alkylthio group, a $(C_1-C_6)$alkylsulfinyl group, a halo$(C_1-C_6)$alkylsulfinyl group, a $(C_1-C_6)$alkylsulfonyl group, a halo$(C_1-C_6)$alkylsulfonyl group, a mono$(C_1-C_6)$alkylamino group, a di$(C_1-C_6)$alkylamino group whose $(C_1-C_6)$alkyl groups may be the same or different, a $(C_1-C_6)$alkylsulfonyl amino group, and a halo$(C_1-C_6)$alkylsulfonyl amino group; a -$ZCOZ_m(R^6)$ group (wherein, $R^6$, Z, and m are the same as described above); or a -$COZ_m(R^6)$ group (wherein, $R^6$, Z, and m are the same as described above);

$R^4$ represents a hydrogen atom; a $(C_1-C_6)$alkyl group; a halo$(C_1-C_6)$alkyl group; a cyano$(C_1-C_6)$alkyl group; a $(C_2-C_6)$alkenyl group; a halo$(C_2-C_6)$alkenyl group; a $(C_2-C_6)$alkynyl group; a halo$(C_2-C_6)$alkynyl group; a $(C_1-C_6)$alkoxy$(C_1-C_6)$alkyl group; a halo$(C_1-C_6)$-alkoxy$(C_1-C_6)$alkyl group; a $(C_1-C_6)$alkoxy$(C_1-C_6)$-alkoxy$(C_1-C_6)$alkyl group; a $(C_1-C_6)$alkylthio$(C_1-C_6)$alkyl group; a halo$(C_1-C_6)$alkylthio$(C_1-C_6)$alkyl group; a $(C_1-C_6)$alkylsulfinyl$(C_1-C_6)$alkyl group; a halo$(C_1-C_6)$-alkylsulfinyl$(C_1-C_6)$alkyl group; a $(C_1-C_6)$alkylsulfonyl-$(C_1-C_6)$alkyl group; a halo$(C_1-C_6)$alkylsulfonyl$(C_1-C_6)$-alkyl group; a mono$(C_1-C_6)$alkylamino$(C_1-C_6)$alkyl group; a di $(C_1-C_6)$alkylamino$(C_1-C_6)$alkyl group whose $(C_1-C_6)$alkyl groups may be the same or different; a $(C_3-C_6)$cycloalkyl group; a halo$(C_3-C_6)$cycloalkyl group; a $(C_3-C_6)$cycloalkenyl group; a halo$(C_3-C_6)$cycloalkenyl group; a -$COZ_m(R^6)$ group (wherein, $R^6$, Z, and m are the same as described above), a -$CSNH(R^6)$ group (wherein, $R^6$, is the same as described above), a $(C_1-C_6)$alkylcarbonyl $(C_1-C_6)$ alkyl group; a $(C_1-C_6)$alkoxycarbonyl$(C_1-C_6)$alkyl group; a phenyl$(C_1-C_6)$alkyl group; a substituted phenyl$(C_1-C_6)$alkyl group having on the ring at least one substituent which may be the same or different and is selected from the group consisting of a halogen atom, a cyano group, a nitro group, a hydroxyl group, an amino group, a mercapto group, a $(C_1-C_6)$alkyl group, a halo-$(C_1-C_6)$alkyl group, a $(C_2-C_6)$alkenyl group, a halo(C2-C6)-alkenyl group, a $(C_2-C_6)$alkynyl group, a halo(C2-C6)-alkynyl group, a $(C_1-C_6)$alkoxyl group, a halo$(C_1-C_6)$-alkoxyl group, a $(C_1-C_6)$alkylthio group, a halo$(C_1-C_6)$-alkylthio group, a $(C_1-C_6)$alkylsulfinyl group, a halo$(C_1-C_6)$alkylsulfinyl group, a $(C_1-C_6)$alkylsulfonyl group, a halo $(C_1-C_6)$alkylsulfonyl group, a mono$(C_1-C_6)$-alkylamino group, a di$(C_1-C_6)$alkylamino group whose $(C_1-C_6)$alkyl groups may be the same or different, a $(C_1-C_6)$alkylsulfonylamino group, and a halo$(C_1-C_6)$-alkylsulfonylamino group; a heterocyclic$(C_1-C_6)$alkyl group (a heterocyclic ring represents a pyridyl group; a pyridine-N-oxide group; a pyridazinyl group; a pyrimidinyl group; a pyrazyl group; a piperidyl group; a piperazino group; a morpholinyl group; a morpholino group; a furyl group; a tetrahydrofuryl group; a thienyl group; a tetrahydrothienyl group; a pyrrolyl group; a pyrrolidyl group; an imidazolidinyl group; an oxazolyl group; an isoxazolyl group; an oxadiazolyl group; a thiazolyl group; an isothiazolyl group; a thiadiazolyl group; an imidazolyl group; a triazolyl group; a pyrazolyl group; an indolyl group; a benzofuryl group; a benzothienyl group; an indazolyl group; a quinolyl group; an isoquinolyl group; or a quinazolyl group); or a substituted heterocyclic-$(C_1-C_6)$alkyl group (the heterocyclic group is the same as the above-described) having on the ring at least one substituent which may be the same or different and is selected from the group consisting of a halogen atom, a cyano group, a nitro group, a hydroxyl group, an amino group, a mercapto group, a $(C_1-C_6)$alkyl group, a halo-$(C_1-C_6)$alkyl group, a $(C_2-C_6)$alkenyl group, a halo-$(C_2-C_6)$alkenyl group, a $(C_2-C_6)$alkynyl group, a halo-$(C_2-C_6)$alkynyl group, a $(C_1-C_6)$alkoxyl group, a halo-$(C_1-C_6)$alkoxyl group, a $(C_1-C_6)$alkylthio group, a halo$(C_1-C_6)$alkylthio group, a $(C_1-C_6)$alkylsulfinyl group, a halo$(C_1-C_6)$alkylsulfinyl group, a $(C_1-C_6)$alkylsulfonyl group, a halo $(C_1-C_6)$alkylsulfonyl group, a mono$(C_1-C_6)$-alkylamino group, a di$(C_1-C_6)$alkylamino group whose $(C_1-C_6)$alkyl groups may be the same or different, a $(C_1-C_6)$alkylsulfonylamino group, and a halo$(C_1-C_6)$-alkylsulfonylamino group;

$R^5$ represents at least one group selected from the group consisting of (i), (ii), (iii), (iv), (v), and (vi) as described below;

(i) a group represented by the formula (a):

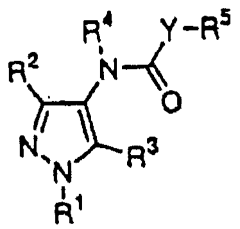

(I)

wherein X may be the same or different, and represents a halogen atom; a cyano group; a nitro group; a hydroxyl group; a mercapto group; an amino group; a $(C_1-C_6)$alkyl group; a halo$(C_1-C_6)$alkyl group; a cyano-$(C_1-C_6)$alkyl group; a $(C_2-C_6)$alkenyl group; a halo-$(C_2-C_6)$alkenyl group; a $(C_2-C_6)$alkynyl group; a halo-$(C_2-C_6)$alkynyl group; a $(C_1-C_6)$alkoxyl group; a halo-$(C_1-C_6)$alkoxyl group; a $(C_1-C_6)$alkylthio group; a halo$(C_1-C_6)$alkylthio group; a $(C_1-C_6)$alkylsulfinyl group; a halo$(C_1-C_6)$alkylsulfinyl group; a $(C_1-C_6)$alkylsulfonyl group; a halo$(C_1-C_6)$alkylsulfonyl group; a mono$(C_1-C_6)$alkylamino group; a di$(C_1-C_6)$alkylamino group whose $(C_1-C_6)$alkyl groups may be the same or different; a $(C_1-C_6)$alkoxy$(C_1-C_6)$alkyl group; a halo$(C_1-C_6)$-alkoxy$(C_1-C_6)$alkyl group; a $(C_1-C_6)$alkylthio$(C_1-C_6)$alkyl group; a halo$(C_1-C_6)$

alkylthio($C_1$-$C_6$)alkyl group; a ($C_1$-$C_6$)alkylsulfinyl ($C_1$-$C_6$)alkyl group; a halo($C_1$-$C_6$)-alkylsulfinyl($C_1$-$C_6$)alkyl group; a ($C_1$-$C_6$)alkylsulfonyl-($C_1$-$C_6$)alkyl group; a halo($C_1$-$C_6$)alkylsulfonyl($C_1$-$C_6$)-alkyl group; a mono($C_1$-$C_6$)alkylamino ($C_1$-$C_6$)alkyl group; a di ($C_1$-$C_6$)alkylamino ($C_1$-$C_6$)alkyl group whose ($C_1$-$C_6$)alkyl groups may be the same or different; a ($C_3$-$C_6$)cycloalkyl group; a halo($C_3$-$C_6$)cycloalkyl group; a ($C_1$-$C_6$)alkylenedioxy group; a ($C_1$-$C_6$)alkyl($C_1$-$C_6$)alkylenedioxy group; a phenyl($C_1$-$C_6$)alkylenedioxy group; a substituted phenyl($C_1$-$C_6$)alkylenedioxy group having on the ring at least one substituent which may be the same or different and is selected from the group consisting of a halogen atom, a cyano group, a nitro group, a hydroxyl group, an amino group, a mercapto group, a ($C_1$-$C_6$)alkyl group, a halo ($C_1$-$C_6$)alkyl group, a ($C_2$-$C_6$)alkenyl group, a halo($C_2$-$C_6$)alkenyl group, a ($C_2$-$C_6$)alkynyl group, a halo($C_2$-$C_6$)alkynyl group, a ($C_1$-$C_6$)alkoxyl group, a halo($C_1$-$C_6$)alkoxyl group, a ($C_1$-$C_6$)alkylthio group, a halo($C_1$-$C_6$)alkylthio group, a ($C_1$-$C_6$)alkylsulfinyl group, a halo($C_1$-$C_6$)alkylsulfinyl group, a ($C_1$-$C_6$)alkylsulfonyl group, a halo($C_1$-$C_6$)alkylsulfonyl group, a mono($C_1$-$C_6$)alkylamino group, a di($C_1$-$C_6$)alkylamino group whose ($C_1$-$C_6$)alkyl groups may be the same or different, a ($C_1$-$C_6$)alkylsulfonyl amino group, and a halo($C_1$-$C_6$)alkylsulfonyl amino group; a phenyl($C_1$-$C_6$)alkyl($C_1$-$C_6$) alkylenedioxy group; a substituted phenyl($C_1$-$C_6$)alkyl($C_1$-$C_6$)alkylenedioxy group having on the ring at least one substituent which may be the same or different and is selected from the group consisting of a halogen atom, a cyano group, a nitro group, a hydroxyl group, an amino group, a mercapto group, a ($C_1$-$C_6$)alkyl group, a halo($C_1$-$C_6$)alkyl group, a ($C_2$-$C_6$)alkenyl group, a halo($C_2$-$C_6$)alkenyl group, a ($C_2$-$C_6$)alkynyl group, a halo($C_2$-$C_6$)alkynyl group, a ($C_1$-$C_6$)alkoxyl group, a halo($C_1$-$C_6$)alkoxyl group, a ($C_1$-$C_6$)alkylthio group, a halo($C_1$-$C_6$)alkylthio group, a ($C_1$-$C_6$) alkylsulfinyl group, a halo($C_1$-$C_6$)alkylsulfinyl group, a ($C_1$-$C_6$)alkylsulfonyl group, a halo($C_1$-$C_6$)alkylsulfonyl group, a mono($C_1$-$C_6$)alkylamino group, a di($C_1$-$C_6$)alkylamino group whose ($C_1$-$C_6$)alkyl groups may be the same or different, a ($C_1$-$C_6$)alkylsulfonyl amino group, and a halo($C_1$-$C_6$)alkylsulfonyl amino group; a hydroxyimino($C_1$-$C_6$)alkyl group; a ($C_1$-$C_6$)alkoxyimino-($C_1$-$C_6$)alkyl group; a ($C_2$-$C_6$)alkenyloxyimino ($C_1$-$C_6$)alkyl group; a phenyl($C_1$-$C_6$)alkoxyimino ($C_1$-$C_6$)alkyl group; a substituted phenyl($C_1$-$C_6$)alkoxyimino($C_1$-$C_6$)alkyl group having on the ring at least one substituent which may be the same or different and is selected from the group consisting of a halogen atom, a cyano group, a nitro group, a hydroxyl group, an amino group, a mercapto group, a ($C_1$-$C_6$)alkyl group, a halo($C_1$-$C_6$)alkyl group, a ($C_2$-$C_6$)alkenyl group, a halo($C_2$-$C_6$)alkenyl group, a ($C_2$-$C_6$)alkynyl group, a halo ($C_2$-$C_6$)alkynyl group, a ($C_1$-$C_6$) alkoxyl group, a halo($C_1$-$C_6$)alkoxyl group, a ($C_1$-$C_6$)alkylthio group, a halo($C_1$-$C_6$)alkylthio group, a ($C_1$-$C_6$)alkylsulfinyl group, a halo($C_1$-$C_6$)alkylsulfinyl group, a ($C_1$-$C_6$)alkylsulfonyl group, a halo($C_1$-$C_6$)alkylsulfonyl group, a mono($C_1$-$C_6$) alkylamino group, a di($C_1$-$C_6$)alkylamino group whose ($C_1$-$C_6$)alkyl groups may be the same or different, a ($C_1$-$C_6$) alkylsulfonylamino group, and a halo($C_1$-$C_6$)alkylsulfonylamino group; a heterocyclic ($C_1$-$C_6$)alkoxyimino($C_1$-$C_6$)alkyl group (the heterocyclic ring is the same as described above); a substituted heterocyclic ($C_1$-$C_6$)alkoxyimino($C_1$-$C_6$) alkyl group (the heterocyclic group is the same as described above) having on the ring at least one substituent which may be the same or different and is selected from the group consisting of a halogen atom, a cyano group, a nitro group, a hydroxyl group, an amino group, a mercapto group, a ($C_1$-$C_6$)alkyl group, a halo($C_1$-$C_6$)alkyl group, a ($C_2$-$C_6$)alkenyl group, a halo($C_2$-$C_6$)alkenyl group, a ($C_2$-$C_6$)alkynyl group, a halo($C_2$-$C_6$)alkynyl group, a ($C_1$-$C_6$)alkoxyl group, a halo ($C_1$-$C_6$)alkoxyl group, a ($C_1$-$C_6$)alkylthio group, a halo($C_1$-$C_6$)alkylthio group, a ($C_1$-$C_6$)alkylsulfinyl group, a halo($C_1$-$C_6$) alkylsulfinyl group, a ($C_1$-$C_6$)alkylsulfonyl group, a halo-($C_1$-$C_6$)alkylsulfonyl group, a mono($C_1$-$C_6$)alkylamino group, a same or different di($C_1$-$C_6$)alkylamino group, a ($C_1$-$C_6$) alkylsulfonylamino group, and a halo($C_1$-$C_6$)alkylsulfonylamino group; a ($C_1$-$C_6$)alkylcarbonyl($C_1$-$C_6$)alkyl group; a ($C_1$-$C_6$)alkoxycarbonyl ($C_1$-$C_6$)alkyl group; the following group:

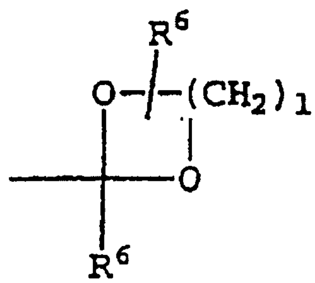

(wherein, $R^6$ is same or different, and the same as described above, and 1 is an integer of 2 to 4); a -$COZ_m(R^6)$ group (wherein, $R^6$, Z, and m are the same as described above); a -$ZCOZ_m(R^6)$ group (wherein, $R^6$, Z, and m are the same as described above); a ($C_1$-$C_6$)-alkylsulfonylamino group; a halo($C_1$-$C_6$)alkylsulfonylamino group; a phenyl group; a substituted phenyl group having at least one substituent which may be the same or different and is selected from the group consisting of a halogen atom, a cyano group, a nitro group, a hydroxyl group, an amino group, a mercapto group, a ($C_1$-$C_6$)alkyl group, a halo($C_1$-$C_6$)alkyl group, a ($C_2$-$C_6$)alkenyl group, a halo($C_2$-$C_6$)alkenyl group, a ($C_2$-$C_6$)alkynyl group, a halo($C_2$-$C_6$)alkynyl group, a ($C_1$-$C_6$)alkoxyl group, a halo($C_1$-$C_6$)alkoxyl group, a ($C_1$-$C_6$)alkylthio group, a

halo($C_1$-$C_6$)alkylthio group, a ($C_1$-$C_6$)alkylsulfinyl group, a halo($C_1$-$C_6$)alkylsulfinyl group, a ($C_1$-$C_6$)alkylsulfonyl group, a halo($C_1$-$C_6$)alkylsulfonyl group, a mono($C_1$-$C_6$)alkylamino group, a di($C_1$-$C_6$)alkylamino group whose ($C_1$-$C_6$)alkyl groups may be the same or different, a -$COZ_m(R^6)$ group (wherein, $R^6$, Z, and m are the same as described above), a -$ZCOZ_m(R^6)$ group (wherein, $R^6$, Z, and m are the same as described above), a ($C_1$-$C_6$)alkylsulfonylamino group, a halo($C_1$-$C_6$)-alkylsulfonylamino group, a hydroxyimino($C_1$-$C_6$)alkyl group, a ($C_1$-$C_6$)alkoxyimino($C_1$-$C_6$)alkyl group, a heterocyclic group (a heterocyclic ring is the same as described above), and a substituted heterocyclic group (a heterocyclic ring is the same as described above) having at least one substituent which may be the same or different and is selected from the group consisting of a halogen atom, a cyano group, a nitro group, a hydroxyl group, an amino group, a mercapto group, a ($C_1$-$C_6$)alkyl group, a halo($C_1$-$C_6$)alkyl group, a ($C_2$-$C_6$)alkenyl group, a halo($C_2$-$C_6$)alkenyl group, a ($C_2$-$C_6$)alkynyl group, a halo($C_2$-$C_6$)alkynyl group, a ($C_1$-$C_6$)alkoxyl group, a halo($C_1$-$C_6$)alkoxyl group, a ($C_1$-$C_6$)alkylthio group, a halo($C_1$-$C_6$)alkylthio group, a ($C_1$-$C_6$)alkylsulfinyl group, a halo($C_1$-$C_6$)alkylsulfinyl group, a ($C_1$-$C_6$)alkylsulfonyl group, a halo($C_1$-$C_6$)alkylsulfonyl group, a mono($C_1$-$C_6$)alkylamino group, a di($C_1$-$C_6$)alkylamino group whose ($C_1$-$C_6$)alkyl groups may be the same or different, a ($C_1$-$C_6$)alkylsulfonylamino group, and a halo($C_1$-$C_6$) alkylsulfonylamino group; a naphthyl group; a substituted naphthyl group having at least one substituent which may be the same or different and is selected from the group consisting of a halogen atom, a cyano group, a nitro group, a hydroxyl group, an amino group, a mercapto group, a ($C_1$-$C_6$)alkyl group, a halo($C_1$-$C_6$)alkyl group, a ($C_2$-$C_6$)alkenyl group, a halo($C_2$-$C_6$)alkenyl group, a ($C_2$-$C_6$)alkynyl group, a halo ($C_2$-$C_6$)alkynyl group, a ($C_1$-$C_6$)alkoxyl group, a halo ($C_1$-$C_6$)alkoxyl group, a ($C_1$-$C_6$)alkylthio group, a halo($C_1$-$C_6$)alkylthio group, a ($C_1$-$C_6$)alkylsulfinyl group, a halo($C_1$-$C_6$) alkylsulfinyl group, a ($C_1$-$C_6$)alkylsulfonyl group, a halo($C_1$-$C_6$)alkylsulfonyl group, a mono($C_1$-$C_6$)alkylamino group, a di($C_1$-$C_6$)alkylamino group whose ($C_1$-$C_6$)alkyl groups may be the same or different, a -$COZ_m(R^6)$ group (wherein, $R^6$, Z, and m are the same as described above), a -$ZCOZ_m(R^6)$ group (wherein, $R^6$, Z, and m are the same as described above), a ($C_1$-$C_6$)alkylsulfonylamino group, and a halo-($C_1$-$C_6$)alkylsulfonylamino group; a heterocyclic group (a heterocyclic ring is the same as described above); a substituted heterocyclic group (a heterocyclic ring is the same as described above) having at least one substituent which may be the same or different and is selected from the group consisting of a halogen atom, a cyano group, a nitro group, a hydroxyl group, an amino group, a mercapto group, a ($C_1$-$C_6$)alkyl group, a halo($C_1$-$C_6$)alkyl group, a ($C_2$-$C_6$)alkenyl group, a halo($C_2$-$C_6$)alkenyl group, a ($C_2$-$C_6$)alkynyl group, a halo($C_2$-$C_6$)alkynyl group, a ($C_1$-$C_6$)alkoxyl group, a halo($C_1$-$C_6$)alkoxyl group, a ($C_1$-$C_6$)alkylthio group, a halo($C_1$-$C_6$)alkylthio group, a ($C_1$-$C_6$)alkylsulfinyl group, a halo($C_1$-$C_6$)alkylsulfinyl group, a ($C_1$-$C_6$)alkylsulfonyl group, a halo($C_1$-$C_6$)alkylsulfonyl group, a mono($C_1$-$C_6$)alkylamino group, a di($C_1$-$C_6$)alkylamino group whose ($C_1$-$C_6$)alkyl groups may be the same or different, a -$COZ_m(R^6)$ group (wherein, $R^6$, Z, and m are the same as described above), a -$ZCOZ_m(R^6)$ group (wherein, $R^6$, Z, and m are the same as described above), a ($C_1$-$C_6$)alkylsulfonylamino group, a halo($C_1$-$C_6$)alkylsulfonylamino group, a hydroxyimino($C_1$-$C_6$)alkyl group, and a ($C_1$-$C_6$)-alkoxyimino($C_1$-$C_6$)alkyl group; or -Q-Ar [wherein Q represents an oxygen atom; a sulfur atom; $N(R^7)$ (wherein, $R^7$ is same as described above); a ($C_1$-$C_6$)-alkylene group; a ($C_1$-$C_6$)alkyl($C_1$-$C_6$)alkylene group; a halo($C_1$-$C_6$)alkyl($C_1$-$C_6$)alkylene group; a hydroxy ($C_1$-$C_6$)-alkyl ($C_1$-$C_6$)alkylene group; a ($C_1$-$C_6$)alkoxy($C_1$-$C_6$)-alkyl($C_1$-$C_6$)alkylene group; a ($C_2$-$C_6$)alkenyl($C_1$-$C_6$)-alkylene group; a halo($C_2$-$C_6$)alkenyl($C_1$-$C_6$)alkylene group; a ($C_2$-$C_6$)alkynyl($C_1$-$C_6$)alkylene group; a halo-($C_2$-$C_6$) alkynyl($C_1$-$C_6$)alkylene group; a hydroxy ($C_1$-$C_6$)-alkylene group; a ($C_1$-$C_6$)alkoxy($C_1$-$C_6$)alkylene group; a halo($C_1$-$C_6$) alkoxy($C_1$-$C_6$)alkylene group; a ($C_1$-$C_6$)alkylcarbonyloxy($C_1$-$C_6$)alkylene group; a cyano($C_1$-$C_6$)alkylene group; a halo ($C_1$-$C_6$)alkylene group; a ($C_3$-$C_6$)cycloalkyl($C_1$-$C_6$)alkylene group; a ($C_3$-$C_6$)cycloalkenyl($C_1$-$C_6$)-alkylene group; a ($C_1$-$C_6$)alkylcarbonyl($C_1$-$C_6$)alkylene group; a ($C_1$-$C_6$)alkoxycarbonyl($C_1$-$C_6$)alkylene group; an amino($C_1$-$C_6$)alkylene group; a mono($C_1$-$C_6$)alkylamino-($C_1$-$C_6$)alkylene group; a di($C_1$-$C_6$)alkylamino($C_1$-$C_6$)-alkylene group whose ($C_1$-$C_6$) alkyl groups may be the same or different; a ($C_1$-$C_6$)alkylcarbonylamino($C_1$-$C_6$)-alkylene group; a ($C_1$-$C_6$)alkoxycarbonylamino($C_1$-$C_6$)-alkylene group; a mercapto($C_1$-$C_6$)alkylene group; a ($C_1$-$C_6$)alkylthio ($C_1$-$C_6$)alkylene group; an alkylsulfinyl ($C_1$-$C_6$)alkylene group; a ($C_1$-$C_6$)alkylsulfonyl ($C_1$-$C_6$)alkylene group; a ($C_3$-$C_6$)cycloalkylene group; a ($C_2$-$C_6$)alkenylene group; a ($C_1$-$C_6$)alkyl($C_2$-$C_6$)alkenylene group; a halo($C_1$-$C_6$)alkyl($C_2$-$C_6$)alkenylene group; a hydroxy($C_1$-$C_6$)alkyl($C_2$-$C_6$)alkenylene group; a ($C_1$-$C_6$)-alkoxy($C_1$-$C_6$)alkyl($C_2$-$C_6$)alkenylene group; a ($C_2$-$C_6$)-alkenyl ($C_2$-$C_6$)alkenylene group; a halo($C_2$-$C_6$)alkenyl-($C_2$-$C_6$)alkenylene group; a ($C_2$-$C_6$)alkynyl($C_2$-$C_6$)-alkenylene group; a halo($C_2$-$C_6$)alkynyl($C_2$-$C_6$)alkenylene group; a hydroxy($C_2$-$C_6$)alkenylene group; a ($C_1$-$C_6$)alkoxy ($C_2$-$C_6$)alkenylene group; a halo ($C_1$-$C_6$)alkoxy ($C_2$-$C_6$)-alkenylene group; a ($C_1$-$C_6$)alkylcarbonyloxy($C_2$-$C_6$)-alkenylene group; a cyano ($C_2$-$C_6$)alkenylene group; a halo($C_2$-$C_6$)alkenylene group; a ($C_3$-$C_6$)cycloalkyl($C_2$-$C_6$)-alkenylene group; a ($C_3$-$C_6$)cycloalkenyl($C_2$-$C_6$)alkenylene group; a ($C_1$-$C_6$)alkylcarbonyl($C_2$-$C_6$)alkenylene group; a ($C_1$-$C_6$)alkoxylcarbonyl($C_2$-$C_6$) alkenylene group; an amino($C_2$-$C_6$)alkenylene group; a mono($C_1$-$C_6$)alkylamino-($C_2$-$C_6$)alkenylene group; a di($C_1$-$C_6$) alkylamino($C_2$-$C_6$)-alkenylene group whose ($C_1$-$C_6$)alkyl groups may be the same or different; a ($C_1$-$C_6$)alkylcarbonylamino($C_2$-$C_6$)-alkenylene group; a ($C_1$-$C_6$)alkoxycarbonylamino($C_2$-$C_6$)-alkenylene group; a mercapto($C_2$-$C_6$)alkenylene group; a ($C_1$-$C_6$)alkylthio($C_2$-$C_6$)alkenylene group; a ($C_1$-$C_6$)alkylsulfinyl($C_2$-$C_6$)alkenylene group; a ($C_1$-$C_6$) alkylsulfonyl($C_2$-$C_6$)alkenylene group; a ($C_2$-$C_6$)alkynylene group; a ($C_1$-$C_6$)alkylenoxy group; a ($C_1$-$C_6$)alkyl($C_1$-$C_6$)-alkylenoxy group; a ($C_1$-$C_6$)alkylenethio group; a ($C_1$-$C_6$)-alkyl($C_1$-$C_6$)alkylenethio group; a ($C_1$-$C_6$)alkyleneamino group; a ($C_1$-$C_6$)alkyl($C_1$-$C_6$)alkylene amino group; a carbonyl group; a hydroxyimino group; a ($C_1$-$C_6$)alkoxyimino

group; a $(C_1-C_6)$alkylenedioxy$(C_1-C_6)$alkylene group; or a $Z(R^6)_mCO$ group (wherein, $R^6$, Z, and m are the same as described above) ;

Ar represents a phenyl group; a substituted phenyl group having at least one substituent which may be the same or different and is selected from the group consisting of a halogen atom, a cyano group, a nitro group, a hydroxyl group, an amino group, a mercapto group, a $(C_1-C_6)$alkyl group, a halo$(C_1-C_6)$alkyl group, a $(C_2-C_6)$alkenyl group, a halo$(C_2-C_6)$alkenyl group, a $(C_2-C_6)$alkynyl group, a halo$(C_2-C_6)$alkynyl group, a $(C_1-C_6)$alkoxyl group, a halo$(C_1-C_6)$alkoxyl group, a $(C_1-C_6)$alkylthio group, a halo$(C_1-C_6)$alkylthio group, a $(C_1-C_6)$alkylsulfinyl group, a halo$(C_1-C_6)$alkylsulfinyl group, a $(C_1-C_6)$alkylsulfonyl group, a halo$(C_1-C_6)$alkylsulfonyl group, a mono$(C_1-C_6)$alkylamino group, a di $(C_1-C_6)$alkylamino group whose $(C_1-C_6)$alkyl groups may be the same or different, a hydroxyimino$(C_1-C_6)$alkyl group, a $(C_1-C_6)$alkoxyimino$(C_1-C_6)$ alkyl group, a $(C_2-C_6)$alkenyloxyimino $(C_1-C_6)$alkyl group, a $-COZ_m(R^6)$ group (wherein, $R^6$, Z, and m are the same as described above), a $-ZCOZ_m(R^6)$ group (wherein, $R^6$, Z, and m are the same as described above), a $(C_1-C_6)$alkylsulfonylamino group, and a halo$(C_1-C_6)$alkylsulfonylamino group; a naphthyl group; a substituted naphthyl group having at least one substituent which may be the same or different and is selected from the group consisting of a halogen atom, a cyano group, a nitro group, a hydroxyl group, an amino group, a mercapto group, a $(C_1-C_6)$alkyl group, a halo$(C_1-C_6)$alkyl group, a $(C_2-C_6)$alkenyl group, a halo$(C_2-C_6)$alkenyl group, a $(C_2-C_6)$alkynyl group, a halo$(C_2-C_6)$alkynyl group, a $(C_1-C_6)$alkoxyl group, a halo$(C_1-C_6)$alkoxyl group, a $(C_1-C_6)$alkylthio group, a halo$(C_1-C_6)$alkylthio group, a $(C_1-C_6)$alkylsulfinyl group, a halo$(C_1-C_6)$alkylsulfinyl group, a $(C_1-C_6)$alkylsulfonyl group, a halo$(C_1-C_6)$alkylsulfonyl group, a mono$(C_1-C_6)$alkylamino group, a di$(C_1-C_6)$alkylamino group whose $(C_1-C_6)$alkyl groups may be the same or different, a $-COZ_m(R^6)$ group (wherein, $R^6$, Z, and m are the same as described above), a $-ZCOZ_m(R^6)$ group (wherein, $R^6$, Z, and m are the same as described above), a $(C_1-C_6)$alkylsulfonylamino group, and a halo-$(C_1-C_6)$alkylsulfonylamino group; a heterocyclic group (a heterocyclic ring is the same as described above); or a substituted heterocyclic group (a heterocyclic ring is the same as described above) having at least one substituent which may be the same or different and is selected from the group consisting of a halogen atom, a cyano group, a nitro group, a hydroxyl group, an amino group, a mercapto group, a $(C_1-C_6)$alkyl group, a halo$(C_1-C_6)$alkyl group, a $(C_2-C_6)$alkenyl group, a halo $(C_2-C_6)$alkenyl group, a $(C_2-C_6)$alkynyl group, a halo-$(C_2-C_6)$alkynyl group, a $(C_1-C_6)$ alkoxyl group, a halo-$(C_1-C_6)$alkoxyl group, a $(C_1-C_6)$alkylthio group, a halo$(C_1-C_6)$alkylthio group, a $(C_1-C_6)$alkylsulfinyl group, a halo$(C_1-C_6)$alkylsulfinyl group, a $(C_1-C_6)$alkylsulfonyl group, a halo$(C_1-C_6)$alkylsulfonyl group, a mono$(C_1-C_6)$-alkylamino group, a di$(C_1-C_6)$alkylamino group whose $(C_1-C_6)$alkyl groups may be the same or different, a $-COZ_m(R^6)$ group (wherein, $R^6$, Z, and m are the same as described above), a $-ZCOZ_m(R^6)$ group (wherein, $R^6$, Z, and m are the same as described above), a $(C_1-C_6)$alkylsulfonylamino group, a halo$(C_1-C_6)$alkylsulfonylamino group, a hydroxyimino $(C_1-C_6)$alkyl group, a $(C_1-C_6)$-alkoxyimino$(C_1-C_6)$alkyl group, and a $(C_2-C_6)$alkenyloxyimino$(C_1-C_6)$alkyl group]; and

n represents an integer of 1 to 5;

(ii) a group represented by the formula (b):

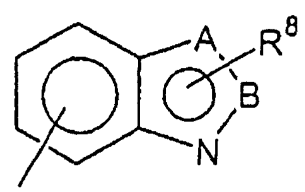

(b)

wherein $R^8$ represents a hydrogen atom; a halogen atom; a cyano group; a nitro group; a hydroxyl group; an amino group; a mercapto group; a $(C_1-C_6)$alkyl group; a halo$(C_1-C_6)$alkyl group; a cyano$(C_1-C_6)$alkyl group; a $(C_3-C_6)$cycloalkyl group; a halo$(C_3-C_6)$cycloalkyl group; a cyano$(C_3-C_6)$cycloalkyl group; a $(C_1-C_6)$alkyl$(C_3-C_6)$cycloalkyl group; a $(C_1-C_6)$alkyl halo$(C_3-C_6)$cycloalkyl group; a $(C_3-C_6)$cycloalkyl$(C_1-C_6)$alkyl group; a $(C_2-C_6)$alkenyl group; a $(C_3-C_6)$ cycloalkenyl group; a halo$(C_2-C_6)$alkenyl group; a $(C_3-C_6)$cycloalkenyl$(C_1-C_6)$alkyl group; a $(C_2-C_6)$alkynyl group; a halo$(C_2-C_6)$alkynyl group; a $(C_1-C_6)$alkoxyl group; a halo$(C_1-C_6)$alkoxyl group; a $(C_1-C_6)$alkylthio group; a halo$(C_1-C_6)$ alkylthio group; a $(C_1-C_6)$alkylsulfinyl group; a halo$(C_1-C_6)$alkylsulfinyl group; a $(C_1-C_6)$alkylsulfonyl group; a halo $(C_1-C_6)$alkylsulfonyl group; a mono$(C_1-C_6)$alkylamino group; a di$(C_1$-C6)alkylamino group whose $(C_1-C_6)$alkyl groups may be the same or different; a $(C_1-C_6)$alkoxy$(C_1-C_6)$alkyl group; a halo$(C_1-C_6)$alkoxy$(C_1-C_6)$alkyl group; a $(C_1-C_6)$ alkylthio-$(C_1-C_6)$alkyl group; a halo$(C_1-C_6)$alkylthio $(C_1-C_6)$alkyl group; a $(C_1-C_6)$alkylsulfinyl$(C_1-C_6)$alkyl group; a halo $(C_1-C_6)$alkylsulfinyl $(C_1-C_6)$alkyl group; a $(C_1-C_6)$-alkylsulfonyl$(C_1-C_6)$alkyl group; a halo$(C_1-C_6)$alkylsulfonyl$(C_1-C_6)$ alkyl group; a mono$(C_1-C_6)$alkylamino$(C_1-C_6)$alkyl group; a di$(C_1-C_6)$alkylamino $(C_1-C_6)$alkyl group whose $(C_1-C_6)$alkyl groups may be the same or different; a carboxy$(C_1-C_6)$alkyl group; a $(C_1-C_6)$alkylcarbonyl$(C_1-C_6)$alkyl group; a $(C_1-C_6)$ alkoxycarbonyl $(C_1-C_6)$alkyl group; an aminocarbonyl$(C_1-C_6)$alkyl group; a mono$(C_1-C_6)$alkylaminocarbonyl$(C_1-C_6)$ alkyl group; a di$(C_1-C_6)$-alkylaminocarbonyl$(C_1-C_6)$alkyl group whose $(C_1-C_6)$alkyl groups may be the same or different; a $-COZ_m(R^6)$ group (in the formula, $R^6$, Z, and m are the same as described above); a $-ZCOZ_m(R^6)$ group (wherein,

$R^6$, Z, and m are the same as described above); a ($C_1$-$C_6$)alkylsulfonyl amino group; a halo($C_1$-$C_6$)alkylsulfonylamino group; a hydroxyimino($C_1$-$C_6$)alkyl group; a ($C_1$-$C_6$)alkoxyimino($C_1$-$C_6$)alkyl group; a ($C_2$-$C_6$)alkenyloxyimino($C_1$-$C_6$) alkyl group; a phenyl group; a substituted phenyl group having at least one substituent which may be the same or different and is selected from the group consisting of a halogen atom, a cyano group, a nitro group, a hydroxyl group, an amino group, a mercapto group, a ($C_1$-$C_6$)alkyl group, a halo ($C_1$-$C_6$)alkyl group, a ($C_2$-$C_6$)alkenyl group, a halo ($C_2$-$C_6$)alkenyl group, a ($C_2$-$C_6$)alkynyl group, a halo($C_2$-$C_6$)alkynyl group, a ($C_1$-$C_6$)alkoxyl group, a halo($C_1$-$C_6$)alkoxyl group, a ($C_1$-$C_6$)alkylthio group, a halo($C_1$-$C_6$)alkylthio group, a ($C_1$-$C_6$)alkylsulfinyl group, a halo($C_1$-$C_6$)alkylsulfinyl group, a ($C_1$-$C_6$)alkylsulfonyl group, a halo($C_1$-$C_6$)alkylsulfonyl group, a mono($C_1$-$C_6$)alkylamino group, a di ($C_1$-$C_6$) alkylamino group whose ($C_1$-$C_6$)alkyl groups may be the same or different, a methylenedioxy group, a -$COZ_m$($R^6$) group (wherein, $R^6$, Z, and m are the same as described above), a -$ZCOZ_m$ ($R^6$) group (wherein, $R^6$, Z, and m are the same as described above), a ($C_1$-$C_6$)alkylsulfonylamino group, and a halo($C_1$-$C_6$)alkylsulfonylamino group; a phenyl ($C_1$-$C_6$)alkyl group; a substituted phenyl($C_1$-$C_6$)alkyl group having on the ring at least one substituent which may be the same or different and is selected from the group consisting of a halogen atom, a cyano group, a nitro group, a hydroxyl group, an amino group, a mercapto group, a ($C_1$-$C_6$)alkyl group, a halo($C_1$-$C_6$)alkyl group, a ($C_2$-$C_6$)alkenyl group, a halo($C_2$-$C_6$)alkenyl group, a ($C_2$-$C_6$)alkynyl group, a halo($C_2$-$C_6$)alkynyl group, a ($C_1$-$C_6$)alkoxyl group, a halo ($C_1$-$C_6$)alkoxyl group, a ($C_1$-$C_6$)alkylthio group, a halo($C_1$-$C_6$)alkylthio group, a ($C_1$-$C_6$)alkylsulfinyl group, a halo($C_1$-$C_6$) alkylsulfinyl group, a ($C_1$-$C_6$)alkylsulfonyl group, a halo($C_1$-$C_6$)alkylsulfonyl group, a mono($C_1$-$C_6$)-alkylamino group, a same or different di($C_1$-$C_6$)alkylamino group, a -$COZ_m$($R^6$) group (wherein, $R^6$, Z, and m are the same as described above), a -$ZCOZ_m$($R^6$) group (wherein, $R^6$, Z, and m are the same as described above), a ($C_1$-$C_6$)alkylsulfonyl amino group, and a halo($C_1$-$C_6$)-alkylsulfonylamino group; a naphthyl group; a substituted naphthyl group having at least one substituent which may be the same or different and is selected from the group consisting of a halogen atom, a cyano group, a nitro group, a hydroxyl group, an amino group, a mercapto group, a ($C_1$-$C_6$)alkyl group, a halo($C_1$-$C_6$)alkyl group, a ($C_2$-$C_6$)alkenyl group, a halo($C_2$-$C_6$)alkenyl group, a ($C_2$-$C_6$)alkynyl group, a halo($C_2$-$C_6$)alkynyl group, a ($C_1$-$C_6$)alkoxyl group, a halo($C_1$-$C_6$)alkoxyl group, a ($C_1$-$C_6$)alkylthio group, a halo($C_1$-$C_6$)alkylthio group, a ($C_1$-$C_6$) alkylsulfinyl group, a halo($C_1$-$C_6$)alkylsulfinyl group, a ($C_1$-$C_6$)alkylsulfonyl group, a halo($C_1$-$C_6$)alkylsulfonyl group, a mono($C_1$-$C_6$)-alkylamino group, a di($C_1$-$C_6$)alkylamino group whose ($C_1$-$C_6$)alkyl groups may be the same or different, a -$COZ_m$($R^6$) group (wherein, $R^6$, Z, and m are the same as described above), a -$ZCOZ_m$($R^6$) group (wherein, $R^6$, Z, and m are the same as described above), a ($C_1$-$C_6$)alkylsulfonylamino group, and a halo($C_1$-$C_6$)alkylsulfonylamino group; a heterocyclic group (a heterocyclic ring is the same as described above); a substituted heterocyclic group (a heterocyclic ring is the same as described above) having at least one substituent which may be the same or different and is selected from the group consisting of a halogen atom, a cyano group, a nitro group, a hydroxyl group, an amino group, a mercapto group, a ($C_1$-$C_6$)alkyl group, a halo($C_1$-$C_6$)alkyl group, a ($C_2$-$C_6$)alkenyl group, a halo($C_2$-$C_6$)alkenyl group, a ($C_2$-$C_6$)alkynyl group, a halo($C_2$-$C_6$)alkynyl group, a ($C_1$-$C_6$)alkoxyl group, a halo($C_1$-$C_6$)alkoxyl group, a ($C_1$-$C_6$)alkylthio group, a halo($C_1$-$C_6$)alkylthio group, a ($C_1$-$C_6$)alkylsulfinyl group, a halo($C_1$-$C_6$)alkylsulfinyl group, a ($C_1$-$C_6$)alkylsulfonyl group, a halo-($C_1$-$C_6$)alkylsulfonyl group, a mono($C_1$-$C_6$)alkylamino group, a di($C_1$-$C_6$)alkylamino group whose ($C_1$-$C_6$)alkyl groups may be the same or different, a -$COZ_m$($R^6$) group (wherein, $R^6$, Z, and m are the same as described above), a -$ZCOZ_m$($R^6$) group (wherein, $R^6$, Z, and m are the same as described above), a ($C_1$-$C_6$) alkylsulfonylamino group, and a halo($C_1$-$C_6$)alkylsulfonylamino group; a heterocyclic($C_1$-$C_6$)alkyl group; a substituted heterocyclic($C_1$-$C_6$)alkyl group (a heterocyclic ring is the same as described above) having at least one substituent which may be the same or different and is selected from the group consisting of same or different a halogen atom, a cyano group, a nitro group, a hydroxyl group, an amino group, a mercapto group, a ($C_1$-$C_6$)alkyl group, a halo($C_1$-$C_6$) alkyl group, a ($C_2$-$C_6$)alkenyl group, a halo($C_2$-$C_6$)alkenyl group, a ($C_2$-$C_6$)alkynyl group, a halo($C_2$-$C_6$)alkynyl group, a ($C_1$-$C_6$)alkoxyl group, a halo($C_1$-$C_6$)alkoxyl group, a ($C_1$-$C_6$)alkylthio group, a halo($C_1$-$C_6$)alkylthio group, a ($C_1$-$C_6$) alkylsulfinyl group, a halo($C_1$-$C_6$)alkylsulfinyl group, a ($C_1$-$C_6$)alkylsulfonyl group, a halo($C_1$-$C_6$)alkylsulfonyl group, a mono($C_1$-$C_6$)alkylamino group, a di($C_1$-$C_6$)alkylamino group whose ($C_1$-$C_6$)alkyl groups may be the same or different, a ($C_1$-$C_6$)alkylsulfonylamino group, and a halo($C_1$-$C_6$)alkylsulfonylamino group; or -Q-Ar (wherein, Q and Ar are the same as described above);

A represents an oxygen atom; a sulfur atom; N=; N($R^7$) (wherein, $R^7$ is the same as described above); or C ($R^9$) [wherein, $R^9$ represents a hydrogen atom; a halogen atom; a ($C_1$-$C_6$)alkyl group; a halo($C_1$-$C_6$)alkyl group; a ($C_2$-$C_6$)alkenyl group; a halo($C_2$-$C_6$)alkenyl group; a ($C_2$-$C_6$)alkynyl group; a halo($C_2$-$C_6$)alkynyl group; a ($C_1$-$C_6$)alkoxy ($C_1$-$C_6$)alkyl group; a halo($C_1$-$C_6$)-alkoxy($C_1$-$C_6$)alkyl group; a ($C_1$-$C_6$)alkylthio ($C_1$-$C_6$)alkyl group; a halo($C_1$-$C_6$) alkylthio($C_1$-$C_6$)alkyl group; a ($C_1$-$C_6$)alkylsulfinyl($C_1$-$C_6$)alkyl group; a halo($C_1$-$C_6$)-alkylsulfinyl($C_1$-$C_6$)alkyl group; a ($C_1$-$C_6$)alkylsulfonyl($C_1$-$C_6$)alkyl group; a halo($C_1$-$C_6$)alkylsulfonyl ($C_1$-$C_6$)alkyl group; a mono ($C_1$-$C_6$)alkylamino ($C_1$-$C_6$)alkyl group; a di($C_1$-$C_6$)alkylamino ($C_1$-$C_6$)alkyl group whose ($C_1$-$C_6$)alkyl groups may be the same or different; a ($C_3$-$C_6$)cycloalkyl group; a halo($C_3$-$C_6$)cycloalkyl group; a ($C_3$-$C_6$)cycloalkenyl group; or a halo($C_3$-$C_6$)cycloalkenyl group]; and

B represents N=; N($R^8$) (wherein, $R^8$ is the same as described above); or C($R^8$) (wherein, $R^8$ is the same as described above);

(iii) a naphthyl group;

(iv) a substituted naphthyl group having at least one substituent which may be the same or different and is selected from the group consisting of a halogen atom, a cyano group, a nitro group, a hydroxyl group, an amino group, a mercapto group, a $(C_1-C_6)$alkyl group, a halo$(C_1-C_6)$alkyl group, a $(C_2-C_6)$alkenyl group, a halo$(C_2-C_6)$alkenyl group, a $(C_2-C_6)$ alkynyl group, a halo$(C_2-C_6)$alkynyl group, a $(C_1-C_6)$alkoxyl group, a halo$(C_1-C_6)$alkoxyl group, a $(C_1-C_6)$alkylthio group, a halo$(C_1-C_6)$alkylthio group, a $(C_1-C_6)$alkylsulfinyl group, a halo$(C_1-C_6)$alkylsulfinyl group, a $(C_1-C_6)$alkylsulfonyl group, a halo$(C_1-C_6)$alkylsulfonyl group, a mono$(C_1-C_6)$-alkylamino group, a di$(C_1-C_6)$alkylamino group whose $(C_1-C_6)$alkyl groups may be the same or different, a -$COZ_m(R^6)$ group (wherein, $R^6$, Z, and m are the same as described above), a -$ZCOZ_m(R^6)$ group (wherein, $R^6$, Z, and m are the same as described above), a $(C_1-C_6)$alkylsulfonylamino group, and a halo$(C_1-C_6)$alkylsulfonylamino group;

(v) a heterocyclic group (a heterocyclic ring is the same as described above); or

(vi) a substituted heterocyclic group (a heterocyclic ring is the same as described above) having at least one substituent which may be the same or different and is selected from the group consisting of a halogen atom; a cyano group; a nitro group; a hydroxyl group; a mercapto group; an amino group; a halo$(C_1-C_6)$alkyl group (with the proviso of excluding a trifluoromethyl group); a $(C_2-C_6)$alkenyl group; a halo$(C_2-C_6)$alkenyl group; a $(C_2-C_6)$alkynyl group; a halo$(C_2-C_6)$ alkynyl group; a halo$(C_1-C_6)$alkoxyl group; a halo$(C_1-C_6)$alkylthio group; a $(C_1-C_6)$alkylsulfinyl group; a halo$(C_1-C_6)$ alkylsulfinyl group; a $(C_1-C_6)$alkylsulfonyl group; a halo$(C_1-C_6)$alkylsulfonyl group; a mono$(C_1-C_6)$alkylamino group; a di$(C_1-C_6)$alkylamino group whose $(C_1-C_6)$alkyl groups may be the same or different; a $(C_1-C_6)$alkoxy-$(C_1-C_6)$alkyl group; a halo$(C_1-C_6)$alkoxy$(C_1-C_6)$alkyl group; a $(C_1-C_6)$alkylthio$(C_1-C_6)$alkyl group; a halo-$(C_1-C_6)$alkylthio$(C_1-C_6)$ alkyl group; a $(C_1-C_6)$alkylsulfinyl $(C_1-C_6)$alkyl group; a halo$(C_1-C_6)$alkylsulfinyl $(C_1-C_6)$alkyl group; a $(C_1-C_6)$alkylsulfonyl$(C_1-C_6)$alkyl group; a halo$(C_1-C_6)$alkylsulfonyl$(C_1-C_6)$alkyl group; a mono-$(C_1-C_6)$alkylamino $(C_1-C_6)$alkyl group; a di $(C_1-C_6)$alkylamino$(C_1-C_6)$alkyl group whose $(C_1-C_6)$alkyl groups may be the same or different; a $(C_3-C_6)$cycloalkyl group, a halo$(C_3-C_6)$cycloalkyl group; a hydroxyimino$(C_1-C_6)$alkyl group, a $(C_1-C_6)$alkoxyimino $(C_1-C_6)$alkyl group, a $(C_2-C_6)$-alkenyloxyimino$(C_1-C_6)$alkyl group; a -$COZ_m(R^6)$ group (wherein, $R^6$, Z, and m are the same as described above); a -$ZCOZ_m(R^6)$ group (wherein, $R^6$, Z, and m are the same as described above); a $(C_1-C_6)$alkylsulfonyl amino group; a halo$(C_1-C_6)$alkylsulfonylamino group; a phenyl group; a substituted phenyl group having at least one substituent which may be the same or different and is selected from the group consisting of an amino group, a mercapto group, a $(C_1-C_6)$alkyl group, a halo$(C_1-C_6)$alkyl group (with the proviso of excluding a trifluoromethyl group), a $(C_2-C_6)$ alkenyl group, a halo$(C_2-C_6)$alkenyl group, a $(C_2-C_6)$alkynyl group, a halo$(C_2-C_6)$alkynyl group, a $(C_1-C_6)$alkoxyl group, a halo$(C_1-C_6)$alkoxyl group, a $(C_1-C_6)$alkylthio group, a halo$(C_1-C_6)$alkylthio group, a $(C_1-C_6)$alkylsulfinyl group, a halo $(C_1-C_6)$alkylsulfinyl group, a $(C_1-C_6)$alkylsulfonyl group, a halo-$(C_1-C_6)$alkylsulfonyl group, a mono$(C_1-C_6)$alkylamino group, a di$(C_1-C_6)$alkylamino group whose $(C_1-C_6)$alkyl groups may be the same or different, a -$COZ_m(R^6)$ group (wherein, $R^6$, Z, and m are the same as described above), a -$ZCOZ_m(R^6)$ group (wherein, $R^6$, Z, and m are the same as described above), a $(C_1-C_6)$alkylsulfonyl amino group, and a halo$(C_1-C_6)$alkylsulfonylamino group; a naphthyl group; a substituted naphthyl group having at least one substituent which may be the same or different and is selected from the group consisting of a halogen atom, a cyano group, a nitro group, a hydroxyl group, an amino group, a mercapto group, a $(C_1-C_6)$alkyl group, a halo-$(C_1-C_6)$alkyl group, a $(C_2-C_6)$alkenyl group, a halo-$(C_2-C_6)$alkenyl group, a $(C_2-C_6)$ alkynyl group, a halo-$(C_2-C_6)$alkynyl group, a $(C_1-C_6)$alkoxyl group, a halo-$(C_1-C_6)$alkoxyl group, a $(C_1-C_6)$alkylthio group, a halo$(C_1-C_6)$alkylthio group, a $(C_1-C_6)$alkylsulfinyl group, a halo$(C_1-C_6)$alkylsulfinyl group, a $(C_1-C_6)$alkylsulfonyl group, a halo$(C_1-C_6)$alkylsulfonyl group, a mono$(C_1-C_6)$alkylamino group, a di$(C_1-C_6)$alkylamino group whose $(C_1-C_6)$alkyl groups may be the same or different, a -$COZ_m(R^6)$ group (wherein, $R^6$, Z, and m are the same as described above), a -$ZCOZ_m(R^6)$ group (wherein, $R^6$, Z, and m are the same as described above), a $(C_1-C_6)$alkylsulfonylamino group, and a halo$(C_1-C_6)$alkylsulfonylamino group; a heterocyclic group (a heterocyclic ring is the same as described above); a substituted heterocyclic group (a heterocyclic ring is the same as described above) having at least one substituent which may be the same or different and is selected from the group consisting of a halogen atom, a cyano group, a nitro group, a hydroxyl group, an amino group, a mercapto group, a $(C_1-C_6)$alkyl group, a halo $(C_1-C_6)$alkyl group, a $(C_2-C_6)$alkenyl group, a halo$(C_2-C_6)$alkenyl group, a $(C_2-C_6)$alkynyl group, a halo$(C_2-C_6)$alkynyl group, a $(C_1-C_6)$alkoxyl group, a halo$(C_1-C_6)$alkoxyl group, a $(C_1-C_6)$alkylthio group, a halo$(C_1-C_6)$alkylthio group, a $(C_1-C_6)$ alkylsulfinyl group, a halo$(C_1-C_6)$alkylsulfinyl group, a $(C_1-C_6)$alkylsulfonyl group, a halo$(C_1-C_6)$alkylsulfonyl group, a mono$(C_1-C_6)$alkylamino group, a di$(C_1-C_6)$alkylamino group whose $(C_1-C_6)$alkyl groups may be the same or different, a -$COZ_m(R^6)$ group (wherein, $R^6$, Z, and m are the same as described above), a -$ZCOZ_m(R^6)$ group (wherein, $R^6$, Z, and m are the same as described above), a $(C_1-C_6)$alkylsulfonylamino group, and a halo$(C_1-C_6)$alkylsulfonylamino group; and -Q-Ar (wherein, Q and Ar are the same as described above);

Y represents a $(C_1-C_6)$alkylene group; a $(C_1-C_6)$alkyl$(C_1-C_6)$alkylene group; a halo$(C_1-C_6)$alkyl-$(C_1-C_6)$ alkylene group; a hydroxy$(C_1-C_6)$alkyl$(C_1-C_6)$-alkylene group; a $(C_1-C_6)$alkoxy$(C_1-C_6)$alkyl $(C_1-C_6)$-alkylene group; a $(C_2-C_6)$alkenyl$(C_1-C_6)$alkylene group; a halo$(C_2-C_6)$alkenyl$(C_1-C_6)$alkylene group; a $(C_2-C_6)$alkynyl $(C_1-C_6)$alkylene group; a halo $(C_2-C_6)$alkynyl $(C_1-C_6)$alkylene group; a hydroxy$(C_1-C_6)$alkylene group; a $(C_1-C_6)$alkoxy-$(C_1-C_6)$alkylene group; a halo$(C_1-C_6)$alkoxy $(C_1-C_6)$alkylene group; a $(C_1-C_6)$alkylcarbonyloxy$(C_1-C_6)$alkylene group; a cyano $(C_1-C_6)$

alkylene group; a halo($C_1$-$C_6$)alkylene group; a ($C_3$-$C_6$)cycloalkyl($C_1$-$C_6$)alkylene group; a ($C_3$-$C_6$)cycloalkenyl($C_1$-$C_6$) alkylene group; a ($C_1$-$C_6$)alkylcarbonyl-($C_1$-$C_6$)alkylene group; a ($C_1$-$C_6$)alkoxycarbonyl($C_1$-$C_6$)-alkylene group; an amino($C_1$-$C_6$)alkylene group; a mono($C_1$-$C_6$)alkyl amino($C_1$-$C_6$)alkylene group; a di($C_1$-$C_6$)alkylamino($C_1$-$C_6$)alkylene group whose ($C_1$-$C_6$)alkyl groups may be the same or different; a ($C_1$-$C_6$)alkylcarbonylamino($C_1$-$C_6$)alkylene group; a ($C_1$-$C_6$)alkoxycarbonylamino($C_1$-$C_6$)alkylene group; a mercapto($C_1$-$C_6$)alkylene group; a ($C_1$-$C_6$)alkylthio-($C_1$-$C_6$) alkylene group; an ($C_1$-$C_6$)alkylsulfinyl($C_1$-$C_6$)-alkylene group; a ($C_1$-$C_6$)alkylsulfonyl($C_1$-$C_6$)alkylene group; a ($C_3$-$C_6$) cycloalkylene group; a ($C_2$-$C_6$)alkenylene group; a ($C_1$-$C_6$)alkyl($C_2$-$C_6$)alkenylene group; a halo($C_1$-$C_6$)alkyl($C_2$-$C_6$) alkenylene group; a hydroxy-($C_1$-$C_6$)alkyl($C_2$-$C_6$)alkenylene group; a ($C_1$-$C_6$)alkoxy-($C_1$-$C_6$)alkyl($C_2$-$C_6$)alkenylene group; a ($C_2$-$C_6$)alkenyl-($C_2$-$C_6$)alkenylene group; a halo($C_2$-$C_6$)alkenyl($C_2$-$C_6$)-alkenylene group; a ($C_2$-$C_6$)alkynyl ($C_2$-$C_6$)alkenylene group; a halo($C_2$-$C_6$)alkynyl($C_2$-$C_6$)alkenylene group; a hydroxy($C_2$-$C_6$)alkenylene group; a ($C_1$-$C_6$) alkoxy($C_2$-$C_6$)-alkenylene group; a halo($C_1$-$C_6$)alkoxy($C_2$-$C_6$)alkenylene group; a ($C_1$-$C_6$)alkylcarbonyloxy($C_2$-$C_6$)alkenylene group; a cyano($C_2$-$C_6$)alkenylene group; a halo($C_2$-$C_6$)alkenylene group; a ($C_3$-$C_6$)cycloalkyl($C_2$-$C_6$)alkenylene group; a ($C_3$-$C_6$)cycloalkenyl ($C_2$-$C_6$)alkenylene group; a ($C_1$-$C_6$)alkylcarbonyl ($C_2$-$C_6$)alkenylene group; a ($C_1$-$C_6$) alkoxylcarbonyl ($C_2$-$C_6$)alkenylene group; an amino($C_2$-$C_6$)alkenylene group; a mono($C_1$-$C_6$)alkylamino-($C_2$-$C_6$)alkenylene group; a di($C_1$-$C_6$)alkylamino($C_2$-$C_6$)-alkenylene group whose ($C_1$-$C_6$)alkyl groups may be the same or different; a ($C_1$-$C_6$)alkylcarbonylamino($C_2$-$C_6$)-alkenylene group; a ($C_1$-$C_6$)alkoxycarbonylamino($C_2$-$C_6$)-alkenylene group; a mercapto ($C_2$-$C_6$)alkenylene group; a ($C_1$-$C_6$)alkylthio($C_2$-$C_6$)alkenylene group; a ($C_1$-$C_6$)alkylsulfinyl($C_2$-$C_6$)alkenylene group; a ($C_1$-$C_6$)alkylsulfonyl-(C2-C6)alkenylene group; a ($C_2$-$C_6$)alkynylene group; a ($C_1$-$C_6$)alkyleneoxy group; a ($C_1$-$C_6$)alkyl($C_1$-$C_6$)alkyleneoxy group; a ($C_1$-$C_6$)alkylenethio group; a ($C_1$-$C_6$)alkyl-($C_1$-$C_6$)alkylenethio group; a ($C_1$-$C_6$)alkyleneamino group; a ($C_1$-$C_6$)alkyl($C_1$-$C_6$)alkyleneamino group; a carbonyl group; a carbonyl($C_1$-$C_6$) alkylene group; a hydroxyimino group; a hydroxyimino($C_1$-$C_6$)alkylene group; a ($C_1$-$C_6$)-alkoxyimino group; a ($C_1$-$C_6$) alkoxyimino($C_1$-$C_6$)alkylene group; or a ($C_1$-$C_6$)alkylenedioxy($C_1$-$C_6$)alkylene group; with the proviso that when $R^5$ represents the formula (b), then Y excludes a ($C_1$-$C_6$)alkylenoxy group; and when $R^5$ represents the formula (a), $R^1$ represents a hydrogen atom or a ($C_1$-$C_6$)alkyl group, and $R^3$ represents a carbamoyl group and a nitrile group, then X represents -Q-Ar [wherein, Q and Ar are the same as described above, but Ar excludes a heterocyclic group (a heterocyclic group is the same as described above), or a substituted heterocyclic group (a heterocyclic group is the same as described above)].

BEST MODE FOR CARRYING OUT THE INVENTION

In the definitions of the general formulae of (I), (I-1), (I-2), and (I-3) of an N-(4-pyrazolyl)amide derivative according to the present invention, "a halogen atom" represents a chlorine atom, a bromine atom, an iodine atom, or a fluorine atom; "a ($C_1$-$C_6$)alkyl" represents a linear or branched alkyl having carbon atoms of 1 to 6 such as methyl, ethyl, n-propyl, i-propyl, n-butyl, i-butyl, s-butyl, t-butyl, n-pentyl, n-hexyl; "n", "i", "s" and "t" represent normal, iso, secondary and tertiary, respectively; "a halo($C_1$-$C_6$)alkyl" represents a linear or branched alkyl group having carbon atoms of 1 to 6 which is substituted with at least one same or different halogen atom which may be the same or different; "a ($C_2$-$C_6$)alkenyl" represents a linear or branched alkenyl group having carbon atoms of 2 to 6 such as allyl, 2-butenyl, 2-methyl-2-propenyl, 1-methyl-2-propenyl, 1-methyl-2-butenyl, 2-methyl-2-butenyl, 2-pentenyl and 2-hexenyl; "a ($C_2$-$C_6$)alkynyl" represents a linear or branched alkynyl group having carbon atoms of 2 to 6 such as propargyl, 2-butynyl and 1-methyl-2-propynyl; "a ($C_3$-$C_6$)cycloalkyl" represents a linear or branched cycloalkyl group having carbon atoms of 3 to 6 such as cyclopropyl, cyclobutyl, cyclohexyl and 2-methylcyclopentyl; and "a ($C_1$-$C_6$)alkylene" represents a linear or branched alkylene group having carbon atoms of 1 to 6 such as methylene, ethylene, propylene, trimethylene, dimethylmethylene, tetramethylene, isobutylene and dimethylethylene.

"a heterocyclic group" represents, for example, a pyridyl group, a pyridine-N-oxide group, a pyridazinyl group, a pyrimidinyl group, a pyrazyl group, a piperidyl group, a piperazino group, a morpholinyl group, a morpholino group, a furyl group, a tetrahydrofuryl group, a thienyl group, a tetrahydrothienyl group, a pyrrolyl group, a pyrrolidyl group, an imidazolidinyl group, an oxazolyl group, an isoxazolyl group, an oxadiazolyl group, a thiazolyl group, an isothiazolyl group, a thiadiazolyl group, an imidazolyl group, a triazolyl group, a pyrazolyl group, an indolyl group, a benzofuryl group, a benzothienyl group, an indazolyl group, a quinolyl group, an isoquinolyl group or a quinazolyl group.

Further, in the definition of Q, "a ($C_3$-$C_6$)cycloalkylene group" represents, for example, an alkylene group substituted with a cycloalkyl group, "a ($C_3$-$C_6$)cycloalkyl($C_2$-$C_6$)alkylene group" represents, for example, an alkylene group substituted with a cycloalkyl group, "a hydroxyimino group" represents a group shown below,

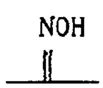

and "a ($C_1$-$C_6$)alkylenedioxy($C_1$-$C_6$)alkylene group" represents, for example, a group such as a group shown below.

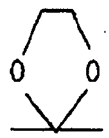

A preferable compound of an N-(4-pyrazolyl)amide derivative represented by the general formula (I) according to the present invention is, for example, an N-(4-pyrazolyl)amide derivative represented by the general formula as shown below.

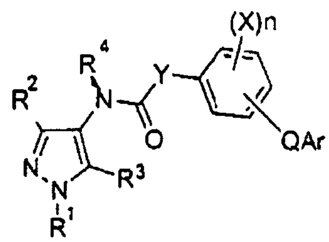

Preferably, $R^1$ is a ($C_1$-$C_6$)alkyl group, a halo($C_1$-$C_6$)alkyl group, a cyano($C_1$-$C_6$)alkyl group or the like; $R^2$ is a hydrogen atom, a halogen atom, a ($C_1$-$C_6$)alkyl group, a halo($C_1$-$C_6$)alkyl group or the like; $R^3$ is a halogen atom, a ($C_1$-$C_6$)alkoxyl group, a ($C_1$-$C_6$)alkoxycarbonyl group or the like; $R^4$ is a hydrogen atom, a ($C_1$-$C_6$)alkyl group, a ($C_1$-$C_6$) alkoxy($C_1$-$C_6$)alkyl group, a ($C_1$-$C_6$)alkylcarbonyl group, a ($C_1$-$C_6$)alkoxycarbonyl group or the like; $(X)_n$ is a hydrogen atom, a halogen atom, a hydroxyl group, a ($C_1$-$C_6$)alkoxyl group, a ($C_1$-$C_6$)alkylamino group or the like; and a substituting position of $(X)_n$ is a 3-position. Q is preferably an oxygen atom or a ($C_1$-$C_6$)alkylenoxy group, and Ar is a phenyl group substituted with a halogen atom, a phenyl group substituted with a cyano group, or a pyridyl group substituted with a halo($C_1$-$C_6$)alkyl group.

More preferably, $R^1$ is a methyl group, an ethyl group, a fluoroethyl group, a cyanomethyl group or the like; $R^2$ is a hydrogen atom, a chlorine atom, a methyl group, an ethyl group, a trifluoromethyl group or the like; $R^3$ is a chlorine atom, a bromine atom, a methoxy group, an ethoxycarbonyl group or the like; $R^4$ is a hydrogen atom, an ethyl group, an ethoxymethyl group, an acetyl group, an ethoxycarbonyl group or the like, $(X)_n$ is a hydrogen atom, a chlorine atom, a hydroxyl group, a methoxy group, a methylamino group or the like, and a substituting position of $(X)_n$ is a 3-position. Q is an oxygen atom or a methylene-oxy group, and Ar is a 4-fluorophenyl group, a 4-cyanophenyl group, a 5-trifluoromethylpyridine-2-yl group, or a 2-trifluoromethylpyridine-5-yl group.

An N-(4-pyrazolyl) amide derivative represented by the general formula (I) according to the present invention can be produced, for instance, by the production processes as shown below.

Production process 1

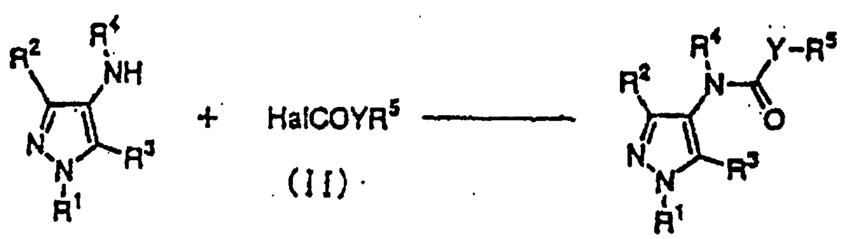

(III) (II) (I)

wherein $R^1$, $R^2$, $R^3$, $R^4$, $R^5$ and Y are the same as described above, and Hal represents a halogen atom.

An N-(4-pyrazolyl) amide derivative represented by the general formula (I) can be produced by reacting a compound represented by the general formula (III) with a compound represented by the general formula (II) in the presences of an inert solvent and a base.

The inert solvent which can be used in the present reaction, may be any solvent so long as it is not markedly detrimental to proceeding of the present reaction, and is exemplified by inert solvents including aromatic hydrocarbons such as benzene, toluene and xylene; halogenated hydrocarbons such as methylene chloride, chloroform and carbon tetrachloride; halogenated aromatic hydrocarbons such as chlorobenzene and dichlorobenzene; linear or cyclic ethers such as diethyl ether, dioxane and tetrahydrofuran; and esters such as ethyl acetate. These inert solvents can be used singly or in combination of two or more kinds thereof.

As the base, an inorganic base or an organic base can be used. An inorganic base includes a hydroxide of an alkali metal atom such as sodium hydroxide and potassium hydroxide, and an organic base includes an organic base such as triethylamine and pyridine. An amount to be used thereof can be appropriately selected in the range of 0.5 to 3 equivalents based on that of the compound represented by the general formula (II).

As the present reaction is an equimolar reaction, each of reactants may be used in an equimolar amount, and an amount of the compound represented by the general formula (III) can be used by appropriately selecting in the range of 0.5 to 2 equivalents based on that of the compound represented by the general formula (II).

With regard to a reaction temperature, the reaction can be conducted at a temperature from 0°C to the reflux temperature of an inert solvent used. A reaction time can be appropriately selected in the range from several minutes to 48 hours, though it is varied depending on a reaction scale, a reaction temperature and the like.

After finishing the reaction, the desired compound may be isolated from the reaction system containing it by a conventional method, and if necessary, purified by recrystallization, column chromatography or the like to obtain the desired compound.

Production process 2

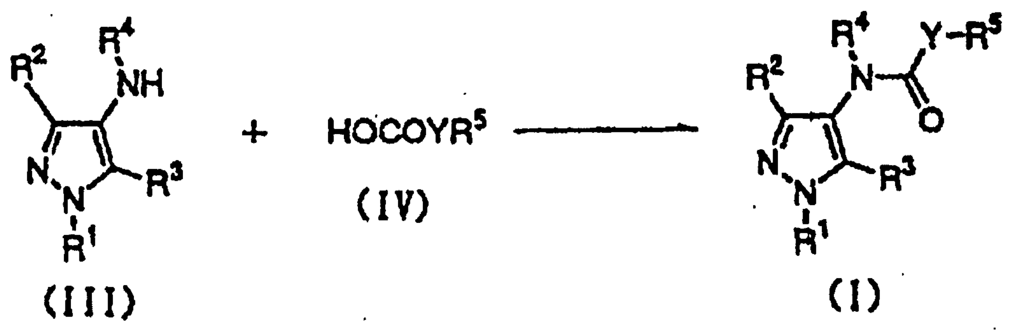

wherein $R^1$, $R^2$, $R^3$, $R^4$, $R^5$ and Y are the same as described above.

An N-(4-pyrazolyl) amide derivative represented by the general formula (I) can be produced by reacting a compound represented by the general formula (III) with a compound represented by the general formula (IV) in the presences of an inert solvent and a base.

The inert solvent which can be used in the present reaction includes tetrahydrofuran, diethyl ether, dioxane, chloroform, methylene chloride and the like. A condensation agent which can be used in the present reaction may be any condensation agent as long as it is used in a conventional amide synthesis, and includes, for example, Mukaiyama reagent (2-chloro-N-methylpyridinium iodide), DCC (1,3-dicyclohexylcarbodiimide), CDI (carbonyl diimidazole) and DEPC (cyanodiethyl phosphate). An amount to be used thereof can be appropriately selected in the range from an equimolar amount to an excessive molar amount based on that of the compound represented by the general formula (IV).

The base which can be used in the present reaction includes, for example, an organic base such as triethylamine and pyridine, and an inorganic base such as potassium carbonate. An amount to be used thereof can be appropriately selected in the range from an equimolar amount to an excessive molar amount based on that of the compound represented by the general formula (IV).

With regard to a reaction temperature, the reaction can be conducted at a temperature in the range from 0°C to a boiling point of the inert solvent used. A reaction time can be appropriately selected in the range from several minutes to 48 hours, though it is varied depending on a reaction scale, a reaction temperature and the like.

After finishing the reaction, the desired compound may be isolated from the reaction system containing it by

a conventional method, and if necessary, purified by recrystallization, column chromatography, or the like to obtain the desired compound.

Production process 3.

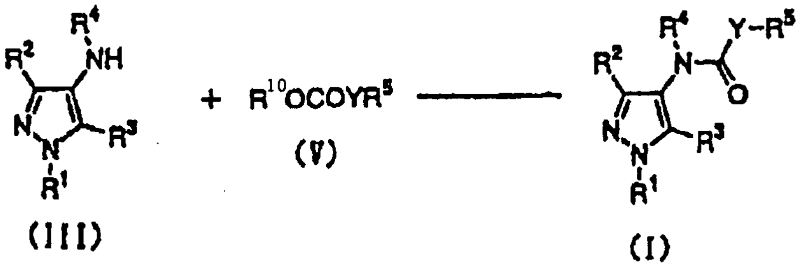

wherein $R^{10}$ represents a $(C_1-C_6)$alkyl group, a phenyl group, or a substituted phenyl group having at least one substituent selected from the group consisting of same or different, a halogen atom, a cyano group, a nitro group, a halo $(C_1-C_6)$alkyl group, a $(C_1-C_6)$alkylsulfinyl group, and a $(C_1-C_6)$alkylsulfonyl group, and $R^1$, $R^2$, $R^3$, $R^4$, $R^5$ and Y are the same as described above.

An N-(4-pyrazolyl)amide derivative represented by the general formula (I) can be produced by reacting a compound represented by the general formula (III) with a compound represented by the general formula (V) in the presences of an inert solvent and a base.

The inert solvent which can be used in the present reaction, may be any solvent so long as it is not markedly detrimental to proceeding of the present reaction, and is exemplified by inert solvents including aromatic hydrocarbons such as benzene, toluene and xylene; halogenated hydrocarbons such as methylene chloride, chloroform and carbon tetrachloride; chlorinated aromatic hydrocarbons such as chlorobenzene and dichlorobenzene; linear or cyclic ethers such as diethyl ether, dioxane and tetrahydrofuran; esters such as ethyl acetate. These inert solvents can be used singly or in combination of two or more kinds thereof.

As the base, an inorganic base and an organic base can be used. An inorganic base includes a hydroxide of an alkali metal atom such as sodium hydroxide and potassium hydroxide, and an organic base includes an organic base such as triethylamine and pyridine. The amount to be used thereof can be appropriately selected in the range of 0.5 to 3 equivalent based on that of the compound represented by the general formula (III).

As the present reaction is an equimolar reaction, each of reactants may be used in an equimolar amount, and the amount of the compound represented by the general formula (V) can be used by appropriately selecting in the range of 0.5 to 2 equivalents based on that of the compound represented by the general formula (III).

With regard to a reaction temperature, the reaction can be conducted at a temperature from 0°C to the reflux temperature of the inert solvent used. A reaction time can be appropriately selected in the range from several minutes to 48 hours, though it is varied depending on a reaction scale, a reaction temperature and the like.

After finishing the reaction, the desired compound may be isolated from the reaction system containing it by a conventional method, and if necessary, purified by recrystallization, column chromatography, or the like to obtain the desired compound can be produced.

Production process 4

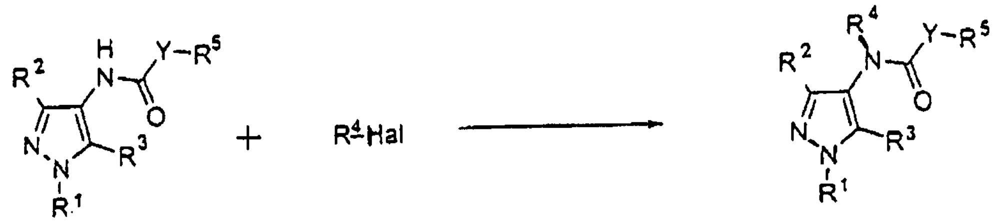

(VI)

(I') (I)

wherein $R^1$, $R^2$, $R^3$, $R^4$ (with a proviso of excluding a hydrogen atom), $R^5$, Hal and Y are the same as described above.

An N-(4-pyrazolyl) amide derivative represented by the general formula (I) can be produced by reacting a compound represented by the general formula (I') with a compound represented by the general formula (VI) in the presences of an inert solvent and a base.

The inert solvent which can be used in the present reaction, may be any solvent so long as it is not markedly detrimental to proceeding of the present reaction, and is exemplified by inert solvents including aromatic hydrocarbons such as benzene, toluene and xylene; halogenated hydrocarbons such as methylene chloride, chloroform and carbon tetrachloride; linear or cyclic ethers such as diethyl ether, dioxane and tetrahydrofuran; esters such as ethyl acetate; linear or cyclic amides such as dimethylformamide, dimethylacetamide and N-methylpyrrolidone. These inert solvents can be used singly or in combination of two or more kinds thereof. As the base, an inorganic base and an organic base can be used. An inorganic base includes a hydroxide of an alkali metal atom such as potassium hydroxide and sodium hydroxide; a carbonate such as potassium carbonate and sodium carbonate; and a hydrogenated product such as sodium hydride; and an organic base includes, for instantce, an organic base such as triethylamine and pyridine. An amount to be used thereof can be appropriately selected in the range of 0.5 to 3 equivalent based on that of the compound represented by the general formula (I').

As the present reaction is an equimolar reaction, each of reactants may be used in an equimolar amount, and an amount of the compound represented by the general formula (VI) can be used by appropriately selecting in the range of 0.5 to 2 equivalents based on that of the compound represented by the general formula (I').

With regard to a reaction temperature, the reaction can be conducted at a temperature from 0°C to the reflux temperature of an inert solvent used. A reaction time can be appropriately selected in the range from several minutes to 48 hours, though it is varied depending on a reaction scale, a reaction temperature, and the like.

After finishing the reaction, the desired compound may be isolated from the reaction system containing it by a conventional method, and if necessary, purified by recrystallization, column chromatography, or the like to obtain the desired compound.

In this connection, in the above-described production processes according to the present invention, a compound represented by the general formula (IV) as a starting material can be produced by the methods as described in Journal of Medicinal Chemistry (J.M.C.1979, Vol.22, No.9, p.1068), Bulletin of Chemical Society of Japan [Bull. Chem. Soc. Japan, 52(7), p.2013-2022(1979)], or based thereon, and said carboxylic acid can be employed as it is in a reaction, or employed in a reaction after being derived to a corresponding ester [a compound represented by the general formula (V)] or a corresponding acid halide [a compound represented by the general formula (II)] according to a conventional method.

Further, an aminopyrazole compound represented by the general formula (III) can be produced by preparing a nitropyrazole by a method as described in Chemical Abstract Vol. 60 (1964) 12020d and the like, and then by reducing based on the method as described in U.S.Patent No.4,171,367 and the like.

Representative examples of a compound represented by the general formula (I) are illustrated in Table 1-1 to Table 6-203, and Tables A to D. However, the present invention is not limited thereto.

In the following Tables, "Me" represents a methyl group, "Et" does an ethyl group, "Pr" does a propyl group, "Bu" does a butyl group, "Pen" does a pentyl group, "Hex" does a hexyl group, "Hep" does a heptyl group, "Oct" does an octyl group, "Dec" does a decyl group, "Ac" does an acetyl group, "Ph" does a phenyl group, "Bz" does a benzyl group, and "c-" does an alicyclic hydrocarbon group.

The general formula (I-1)

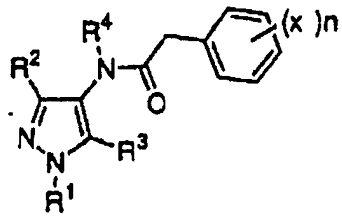

(I-1)

Table 1-1

| $R^1$ | $R^2$ | $R^3$ | $R^4$ | (X)n |
|---|---|---|---|---|
| Me | Me | Cl | H | H |
| Me | Me | Cl | H | 2-F |
| Me | Me | Cl | H | 3-F |
| Me | Me | Cl | H | 4-F |
| Me | Me | Cl | H | 2-Cl |
| Me | Me | Cl | H | 3-Cl |
| Me | Me | Cl | H | 4-Cl |
| Me | Me | Cl | H | 2-Br |
| Me | Me | Cl | H | 3-Br |
| Me | Me | Cl | H | 4-Br |
| Me | Me | Cl | H | 2-Me |
| Me | Me | Cl | H | 3-Me |
| Me | Me | Cl | H | 4-Me |
| Me | Me | Cl | H | 4-n-Bu |
| Me | Me | Cl | H | 4-t-Bu |
| Me | Me | Cl | H | 4-C≡$CCMe_3$ |
| Me | Me | Cl | H | 4-C≡$CC(Me)_2OH$ |
| Me | Me | Cl | H | 4-c-Hex |
| Me | Me | Cl | H | 2-OMe |
| Me | Me | Cl | H | 3-OMe |
| Me | Me | Cl | H | 4-OMe |
| Me | Me | Cl | H | 2-$CF_3$ |
| Me | Me | Cl | H | 3-$CF_3$ |
| Me | Me | Cl | H | 4-$CF_3$ |
| Me | Me | Cl | H | 2-$OCF_3$ |
| Me | Me | Cl | H | 3-$OCF_3$ |
| Me | Me | Cl | H | 4-$OCF_3$ |
| Me | Me | Cl | H | 4-$OCH_2CF_3$ |
| Me | Me | Cl | H | 4-$OCF_2CHF_2$ |
| Me | Me | Cl | H | 4-$OCHF_2$ |
| Me | Me | Cl | H | 4-$OCH_2CF_2CHF_2$ |
| Me | Me | Cl | H | 4-$OCH(Me)CF_3$ |
| Me | Me | Cl | H | 4-OEt |
| Me | Me | Cl | H | 4-0-n-Bu |
| Me | Me | Cl | H | 4-0-t-Bu |

Table 1-1 (continued)

| $R^1$ | $R^2$ | $R^3$ | $R^4$ | (X)n |
|---|---|---|---|---|
| Me | Me | Cl | H | 4-0-n-Pen |
| Me | Me | Cl | H | 2-OAc |
| Me | Me | Cl | H | 3-OAc |
| Me | Me | Cl | H | 4-OAc |
| Me | Me | Cl | H | 2-$OCO_2Me$ |
| Me | Me | Cl | H | 3-$OCO_2Me$ |
| Me | Me | Cl | H | 4-$OCO_2Me$ |
| Me | Me | Cl | H | 2-CN |
| Me | Me | Cl | H | 3-CN |
| Me | Me | Cl | H | 4-CN |
| Me | Me | Cl | H | 2-$CO_2H$ |
| Me | Me | Cl | H | 3-$CO_2H$ |
| Me | Me | Cl | H | 4-$CO_2H$ |
| Me | Me | Cl | H | 2-$CO_2Me$ |
| Me | Me | Cl | H | 3-$CO_2Me$ |
| Me | Me | Cl | H | 4-$CO_2Me$ |
| Me | Me | Cl | H | 2-CONHMe |
| Me | Me | Cl | H | 3-CONHMe |
| Me | Me | Cl | H | 4-CONHMe |
| Me | Me | Cl | H | 2-$CONMe_2$ |
| Me | Me | Cl | H | 3-$CONMe_2$ |
| Me | Me | Cl | H | 4-$CONMe_2$ |
| Me | Me | Cl | H | 2-$NO_2$ |
| Me | Me | Cl | H | 3-$NO_2$ |
| Me | Me | Cl | H | 4-$NO_2$ |
| Me | Me | Cl | H | 2-$NH_2$ |
| Me | Me | Cl | H | 3-$NH_2$ |
| Me | Me | Cl | H | 4-$NH_2$ |
| Me | Me | Cl | H | 2-NHMe |
| Me | Me | Cl | H | 3-NHMe |
| Me | Me | Cl | H | 4-NHMe |
| Me | Me | Cl | H | 2-$NMe_2$ |
| Me | Me | Cl | H | 3-$NMe_2$ |
| Me | Me | Cl | H | 4-$NMe_2$ |
| Me | Me | Cl | H | 2-NHAc |
| Me | Me | Cl | H | 3-NHAc |
| Me | Me | Cl | H | 4-NHAc |
| Me | Me | Cl | H | 2-$NHCO_2Me$ |
| Me | Me | Cl | H | 3-$NHCO_2Me$ |
| Me | Me | Cl | H | 4-$NHCO_2Me$ |
| Me | Me | Cl | H | 2-$NHCO_2Et$ |
| Me | Me | Cl | H | 3-$NHCO_2Et$ |
| Me | Me | Cl | H | 4-$NHCO_2Et$ |
| Me | Me | Cl | H | 2-$NHCO_2$-i-Pr |
| Me | Me | Cl | H | 3-$NHCO_2$-i-Pr |
| Me | Me | Cl | H | 4-$NHCO_2$-i-Pr |
| Me | Me | Cl | H | 2-$NHCO_2CH_2CH{=}CH_2$ |
| Me | Me | Cl | H | 3-$NHCO_2CH_2CH{=}CH_2$ |
| Me | Me | Cl | H | 4-$NHCO_2CH_2CH{=}CH_2$ |
| Me | Me | Cl | H | 2-$NHCO_2Ph$ |

Table 1-1 (continued)

| $R^1$ | $R^2$ | $R^3$ | $R^4$ | (X)n |
|---|---|---|---|---|
| Me | Me | Cl | H | 3-$NHCO_2Ph$ |
| Me | Me | Cl | H | 4-$NHCO_2Ph$ |
| Me | Me | Cl | H | 2-$NHCO_2CH_2Ph$ |
| Me | Me | Cl | H | 3-$NHCO_2CH_2Ph$ |
| Me | Me | Cl | H | 4-$NHCO_2CH_2Ph$ |
| Me | Me | Cl | H | 2-NHCO-t-Bu |
| Me | Me | Cl | H | 3-NHCO-t-Bu |
| Me | Me | Cl | H | 4-NHCO-t-Bu |
| Me | Me | Cl | H | 2-$NHCOCF_3$ |
| Me | Me | Cl | H | 3-$NHCOCF_3$ |
| Me | Me | Cl | H | 4-$NHCOCF_3$ |
| Me | Me | Cl | H | 2-$NHCOCHF_2$ |
| Me | Me | Cl | H | 2-$NHCOCHF_2$ |
| Me | Me | Cl | H | 4-$NHCOCHF_2$ |
| Me | Me | Cl | H | 2-NHCOCH=CHMe |
| Me | Me | Cl | H | 3-NHCOCH=CHMe |
| Me | Me | Cl | H | 4-NHCOCH=CHMe |
| Me | Me | Cl | H | 2-NHCOPh |
| Me | Me | Cl | H | 3-NHCOPh |
| Me | Me | Cl | H | 4-NHCOPh |
| Me | Me | Cl | H | 2-$NHSO_2Me$ |
| Me | Me | Cl | H | 3-$NHSO_2Me$ |
| Me | Me | Cl | H | 4-$NHSO_2Me$ |
| Me | Me | Cl | H | 2-$NHSO_2CF_3$ |
| Me | Me | Cl | H | 3-$NHSO_2CF_3$ |
| Me | Me | Cl | H | 4-$NHSO_2CF_3$ |
| Me | Me | Cl | H | 2-$NHSO_2Ph$ |
| Me | Me | Cl | H | 3-$NHSO_2Ph$ |
| Me | Me | Cl | H | 4-$NHSO_2Ph$ |
| Me | Me | Cl | H | 2-$SCF_3$ |
| Me | Me | Cl | H | 3-$SCF_3$ |
| Me | Me | Cl | H | 4-$SCF_3$ |
| Me | Me | Cl | H | 2-$SOCF_3$ |
| Me | Me | Cl | H | 3-$SOCF_3$ |
| Me | Me | Cl | H | 4-$SOCF_3$ |
| Me | Me | Cl | H | 2-$SO_2CF_3$ |
| Me | Me | Cl | H | 3-$SO_2CF_3$ |
| Me | Me | Cl | H | 4-$SO_2CF_3$ |
| Me | Me | Cl | H | 2-SMe |
| Me | Me | Cl | H | 3-SMe |
| Me | Me | Cl | H | 4-SMe |
| Me | Me | Cl | H | 2-SOMe |
| Me | Me | Cl | H | 3-SOMe |
| Me | Me | Cl | H | 4-SOMe |
| Me | Me | Cl | H | 2-$SO_2Me$ |
| Me | Me | Cl | H | 3-$SO_2Me$ |
| Me | Me | Cl | H | 4-$SO_2Me$ |
| Me | Me | Cl | H | 2-Ac |
| Me | Me | Cl | H | 3-Ac |
| Me | Me | Cl | H | 4-Ac |

Table 1-1 (continued)

| $R^1$ | $R^2$ | $R^3$ | $R^4$ | (X)n |
|---|---|---|---|---|
| Me | Me | Cl | H | 2-C(Me)=NOH |
| Me | Me | Cl | H | 3-C(Me)=NOH |
| Me | Me | Cl | H | 4-C(Me)=NOH |
| Me | Me | Cl | H | 2-C(Me)=NOMe |
| Me | Me | Cl | H | 3-C(Me)=NOMe |
| Me | Me | Cl | H | 4-C(Me)=NOMe |
| Me | Me | Cl | H | 2-C(Me)=$NOCH_2CH=CH_2$ |
| Me | Me | Cl | H | 2-C(Me)=NO-t-Bu |
| Me | Me | Cl | H | 2-C(Me)=$NOCH_2Ph$ |
| Me | Me | Cl | H | 2-C (Me) =$NOCH_2$(4-$CF_3$-Ph) |
| Me | Me | Cl | H | 2-C(Me)=$NOCH_2$(2,4-$Cl_2$-Ph) |
| Me | Me | Cl | H | 2-C(Me)=$NOCH_2$(2,6-$Cl_2$-Ph) |
| Me | Me | Cl | H | 2-C (Me) =$NOCH_2$(2-Cl-pyridine-5-yl) |
| Me | Me | Cl | H | 3-C(Me)=$NOCH_2CH=CH_2$ |
| Me | Me | Cl | H | 3-C(Me)-NO-t-Bu |
| Me | Me | Cl | H | 3-C(Me)=$NOCH_2Ph$ |
| Me | Me | Cl | H | 3-C (Me) =$NOCH_2$(4-$CF_3$-Ph) |
| Me | Me | Cl | H | 3-C (Me)=$NOCH_2$(2,4-$Cl_2$-Ph) |
| Me | Me | Cl | H | 3-C (Me) =$NOCH_2$(2,6-$Cl_2$-Ph) |
| Me | Me | Cl | H | 2-C (Me)=$NOCH_2$(2-Cl-pyridine-5-yl) |
| Me | Me | Cl | H | 4-C(Me)=$NOCH_2CH=CH_2$ |
| Me | Me | Cl | H | 4-C(Me)=NO-t-Bu |
| Me | Me | Cl | H | 4-C(Me)=$NOCH_2Ph$ |
| Me | Me | Cl | H | 4-C (Me) =$NOCH_2$(4-$CF_3$-Ph) |
| Me | Me | Cl | H | 4-C (Me) =$NOCH_3$(2,4-$Cl_2$-Ph) |
| Me | Me | Cl | H | 4-C (Me) =$NOCH_2$(2,6-$Cl_2$-Ph) |
| Me | Me | Cl | H | 2-C (Me)=$NOCH_2$(2-Cl-pyridine-5-yl) |
| Me | Me | Cl | H | 3,5-$F_2$ |
| Me | Me | Cl | H | 2,3-$Cl_2$ |
| Me | Me | Cl | H | 2,4-$Cl_2$ |
| Me | Me | Cl | H | 2,5-$Cl_2$ |
| Me | Me | Cl | H | 2,6-$Cl_2$ |
| Me | Me | Cl | H | 3,4-$Cl_2$ |
| Me | Me | Cl | H | 3,5-$Cl_2$ |
| Me | Me | Cl | H | 2,3-$Me_2$ |
| Me | Me | Cl | H | 2,4-$Me_2$ |
| Me | Me | Cl | H | 2,5-$Me_2$ |
| Me | Me | Cl | H | 2,6-$Me_2$ |
| Me | Me | Cl | H | 3,4-$Me_2$ |
| Me | Me | Cl | H | 3,5-$Me_2$ |
| Me | Me | Cl | H | 3,5-$(CF_3)_2$ |
| Me | Me | Cl | H | 3-F-4-$CH_2CF_3$ |
| Me | Me | Cl | H | 2,4,6-$Cl_3$ |
| Me | Me | Cl | H | 2,4,6-$Me_3$ |
| Me | Me | Cl | H | 2,4,5-$F_3$-6-$CF_3$ |
| Me | Me | Cl | H | 4-morpholino |
| Me | Me | Cl | H | 5-$CF_3$-pyridine-2-yl |
| Me | Me | Cl | H | 3-Cl-5-$CF_3$-pyridine-2-yl |
| Me | Me | Cl | H | 3-F-5-$CF_3$-pyridine-2-yl |
| Me | Me | Cl | H | 3,5-$Cl_2$-pyridine-2-yl |

Table 1-1 (continued)

| $R^1$ | $R^2$ | $R^3$ | $R^4$ | (X)n |
|---|---|---|---|---|
| Me | Me | Cl | H | 3,5-$F_2$-pyridine-2-yl |

Table 1-2

Table 1-2 represents 186 compounds wherein, in the general formula (I-1), $R^1$ represents Me, $R^2$ does Et, $R^3$ does Cl, and $R^4$ does H, and (X)n corresponds to each of all the substituents shown in Table 1-1.

Table 1-3

Table 1-3 represents 186 compounds wherein, in the general formula (I-1), $R^1$ represents Me, $R^2$ does i-Pr, $R^3$ does Cl, and $R^4$ does H, and (X)n corresponds to each of all the substituents shown in Table 1-1.

Table 1-4

Table 1-4 represents 186 compounds wherein, in the general formula (I-1), $R^1$ represents Me, $R^2$ does n-Pr, $R^3$ does Cl, and $R^4$ does H, and (X)n corresponds to each of all the substituents shown in Table 1-1.

Table 1-5

Table 1-5 represents 186 compounds wherein, in the general formula (I-1), $R^1$ represents Me, $R^2$ does t-Bu, $R^3$ does Cl, and $R^4$ does H, and (X)n corresponds to each of all the substituents shown in Table 1-1.

Table 1-6

Table 1-6 represents 186 compounds wherein, in the general formula (I-1), $R^1$ represents Me, $R^2$ does n-Bu, $R^3$ does Cl, and $R^4$ does H, and (X)n corresponds to each of all the substituents shown in Table 1-1.

Table 1-7

Table 1-7 represents 186 compounds wherein, in the general formula (I-1), $R^1$ represents Me, $R^2$ does $CF_3$, $R^3$ does Cl, and $R^4$ does H, and (X)n corresponds to each of all the substituents shown in Table 1-1.

Table 1-8

Table 1-8 represents 186 compounds wherein, in the general formula (I-1), $R^1$ represents Me, $R^2$ does OMe, $R^3$ does Cl, and $R^4$ does H, and (X)n corresponds to each of all the substituents shown in Table 1-1.

Table 1-9

Table 1-9 represents 186 compounds wherein, in the general formula (I-1), $R^1$ represents Me, $R^2$ does $OCHF_2$, $R^3$ does Cl, and $R^4$ does H, and (X)n corresponds to each of all the substituents shown in Table 1-1.

Table 1-10

Table 1-10 represents 186 compounds wherein, in the general formula (I-1), $R^1$ represents Me, $R^2$ does O-i-Pr, $R^3$ does Cl, and $R^4$ does H, and (X)n corresponds to each of all the substituents shown in Table 1-1.

Table 1-11

Table 1-11 represents 186 compounds wherein, in the general formula (I-1), $R^1$ represents Me, $R^2$ does Cl, $R^3$ does Cl, and $R^4$ does H, and (X)n corresponds to each of all the substituents shown in Table 1-1.

Table 1-12

Table 1-12 represents 186 compounds wherein, in the general formula (I-1), $R^1$ represents Me, $R^2$ does Ph, $R^3$ does Cl, and $R^4$ does H, and (X)n corresponds to each of all the substituents shown in Table 1-1.

Table 1-13

Table 1-13 represents 186 compounds wherein, in the general formula (I-1), $R^1$ represents Me, $R^2$ does 2-Cl-Ph, $R^3$ Cl, and $R^4$ does H, and (X)n corresponds to each of all the substituents shown in Table 1-1.

Table 1-14

Table 1-14 represents 186 compounds wherein, in the general formula (I-1), $R^1$ represents Me, $R^2$ does 3-Cl-Ph, $R^3$ does Cl, and $R^4$ does H, and (X)n corresponds to each of all the substituents shown in Table 1-1.

Table 1-15

Table 1-15 represents 186 compounds wherein, in the general formula (I-1), $R^1$ represents Me, $R^2$ does 4-Cl-Ph, $R^3$ does Cl, and $R^4$ does H, and (X)n corresponds to each of all the substituents shown in Table 1-1.

Table 1-16

Table 1-16 represents 186 compounds wherein, in the general formula (I-1), $R^1$ represents Me, $R^2$ does 2,4-$Cl_2$-Ph, $R^3$ does Cl, and $R^4$ does H, and (X)n corresponds to each of all the substituents shown in Table 1-1.

Table 1-17

Table 1-17 represents 186 compounds wherein, in the general formula (I-1), $R^1$ represents Me, $R^2$ does 2-Me-Ph, $R^3$ does Cl, and does $R^4$ H, and (X)n corresponds to each of all the substituents shown in Table 1-1.

Table 1-18

Table 1-18 represents 186 compounds wherein, in the general formula (I-1), $R^1$ represents Me, $R^2$ does 3-Me-Ph, $R^3$ does Cl, and $R^4$ does H, and (X)n corresponds to each of all the substituents shown in Table 1-1.

Table 1-19

Table 1-19 represents 186 compounds wherein, in the general formula (I-1), $R^1$ represents Me, $R^2$ does 4-Me-Ph, $R^3$ does Cl, and $R^4$ does H, and (X)n corresponds to each of all the substituents shown in Table 1-1.

Table 1-20

Table 1-20 represents 186 compounds wherein, in the general formula (I-1), $R^1$ represents Me, $R^2$ does 2-OMe-Ph, $R^3$ does Cl, and $R^4$ does H, and (X)n corresponds to each of all the substituents shown in Table 1-1.

Table 1-21

Table 1-21 represents 186 compounds wherein, in the general formula (I-1), $R^1$ represents Me, $R^2$ does 3-OMe-Ph, $R^3$ does Cl, and $R^4$ does H, and (X)n corresponds to each of all the substituents shown in Table 1-1.

Table 1-22

Table 1-22 represents 186 compounds wherein, in the general formula (I-1), $R^1$ represents Me, $R^2$ does 4-OMe-Ph, $R^3$ does Cl, and $R^4$ does H, and (X)n corresponds to each of all the substituents shown in Table 1-1.

Table 1-23

Table 1-23 represents 186 compounds wherein, in the general formula (I-1), $R^1$ represents Me, $R^2$ does H,

$R^3$ does Cl, and $R^4$ does H, and (X)n corresponds to each of all the substituents shown in Table 1-1.

Table 1-24

Table 1-24 represents 186 compounds wherein, in the general formula (I-1), $R^1$ represents H, $R^2$ does H, $R^3$ does Cl, and $R^4$ does H, and (X)n corresponds to each of all the substituents shown in Table 1-1.

Table 1-25

Table 1-25 represents 186 compounds wherein, in the general formula (I-1), $R^1$ represents Et, $R^2$ does H, $R^3$ does Cl, and $R^4$ does H, and (X)n corresponds to each of all the substituents shown in Table 1-1.

Table 1-26

Table 1-26 represents 186 compounds wherein, in the general formula (I-1), $R^1$ represents i-Pr, $R^2$ does H, $R^3$ does Cl, and $R^4$ does H, and (X)n corresponds to each of all the substituents shown in Table 1-1.

Table 1-27

Table 1-27 represents 186 compounds wherein, in the general formula (I-1), $R^1$ represents n-Pr, $R^2$ does H, $R^3$ does Cl, and $R^4$ does H, and (X)n corresponds to each of all the substituents shown in Table 1-1.

Table 1-28

Table 1-28 represents 186 compounds wherein, in the general formula (I-1), $R^1$ represents t-Bu, $R^2$ does H, $R^3$ does Cl, and $R^4$ does H, and (X)n corresponds to each of all the substituents shown in Table 1-1.

Table 1-29

Table 1-29 represents 186 compounds wherein, in the general formula (I-1), $R^1$ represents n-Bu, $R^2$ does H, $R^3$ does Cl, and $R^4$ does H, and (X)n corresponds to each of all the substituents shown in Table 1-1.

Table 1-30

Table 1-30 represents 186 compounds wherein, in the general formula (I-1), $R^1$ represents $CH{=}CH_2$, $R^2$ does H, $R^3$ does Cl, and $R^4$ does H, and (X)n corresponds to each of all the substituents shown in Table 1-1.

Table 1-31

Table 1-31 represents 186 compounds wherein, in the general formula (I-1), $R^1$ represents $CH_2CH{=}CH_2$, $R^2$ does H, $R^3$ does Cl, and $R^4$ does H, and (X)n corresponds to each of all the substituents shown in Table 1-1.

Table 1-32

Table 1-32 represents 186 compounds wherein, in the general formula (I-1), $R^1$ represents $CH{\equiv}CH_2$, $R^2$ does H, $R^3$ does Cl, and $R^4$ does H, and (X)n corresponds to each of all the substituents shown in Table 1-1.

Table 1-33

Table 1-33 represents 186 compounds wherein, in the general formula (I-1), $R^1$ represents $CH_2CN$, $R^2$ does H, $R^3$ does Cl, and $R^4$ does H, and (X)n corresponds to each of all the substituents shown in Table 1-1.

Table 1-34

Table 1-34 represents 186 compounds wherein, in the general formula (I-1), $R^1$ represents $CH_2CF_3$, $R^2$ does H, $R^3$ does Cl, and $R^4$ does H, and (X)n corresponds to each of all the substituents shown in Table 1-1.

Table 1-35

Table 1-35 represents 186 compounds wherein, in the general formula (I-1), $R^1$ represents $CH_2Ph$, $R^2$ does H, $R^3$ does Cl, and $R^4$ does H, and (X)n corresponds to each of all the substituents shown in Table 1-1.

Table 1-36

Table 1-36 represents 186 compounds wherein, in the general formula (I-1), $R^1$ represents Ph, $R^2$ does H, $R^3$ does Cl, and $R^4$ does H, and (X)n corresponds to each of all the substituents shown in Table 1-1.

Table 1-37

Table 1-37 represents 186 compounds wherein, in the general formula (I-1), $R^1$ represents 2-Cl-Ph, $R^2$ does H, $R^3$ does Cl, and $R^4$ does H, and (X)n corresponds to each of all the substituents shown in Table 1-1.

Table 1-38

Table 1-38 represents 186 compounds wherein, in the general formula (I-1), $R^1$ represents 3-Cl-Ph, $R^2$ does H, $R^3$ does Cl, and $R^4$ does H, and (X)n corresponds to each of all the substituents shown in Table 1-1.

Table 1-39

Table 1-39 represents 186 compounds wherein, in the general formula (I-1), $R^1$ represents 4-Cl-Ph, $R^2$ does H, $R^3$ does Cl, and $R^4$ does H, and (X)n corresponds to each of all the substituents shown in Table 1-1.

Table 1-40

Table 1-40 represents 186 compounds wherein, in the general formula (I-1), $R^1$ represents 2,4-$Cl_2$-Ph, $R^2$ does H, $R^3$ does Cl, and $R^4$ does H, and (X)n corresponds to each of all the substituents shown in Table 1-1.

Table 1-41

Table 1-41 represents 186 compounds wherein, in the general formula (I-1), $R^1$ represents Ac, $R^2$ does H, $R^3$ does Cl, and $R^4$ does H, and (X)n corresponds to each of all the substituents shown in Table 1-1.

Table 1-42

Table 1-42 represents 186 compounds wherein, in the general formula (I-1), $R^1$ represents CO-Ph, $R^2$ does H, $R^3$ does Cl, and $R^4$ does H, and (X)n corresponds to each of all the substituents shown in Table 1-1.

Table 1-43

Table 1-43 represents 186 compounds wherein, in the general formula (I-1), $R^1$ represents $CH_2$ $CH_2F$, $R^2$ does H, $R^3$ does Cl, and $R^4$ does H, and (X)n corresponds to each of all the substituents shown in Table 1-1.

Table 1-44

Table 1-44 represents 186 compounds wherein, in the general formula (I-1), $R^1$ represents $CH_2$ $CH_2Cl$, $R^2$ does H, $R^3$ does Cl, and $R^4$ does H, and (X)n corresponds to each of all the substituents shown in Table 1-1.

Table 1-45

Table 1-45 represents 186 compounds wherein, in the general formula (I-1), $R^1$ represents $CH_2$ $CH_2Br$, $R^2$ does H, $R^3$ does Cl, and $R^4$ does H, and (X)n corresponds to each of all the substituents shown in Table 1-1.

Table 1-46

Table 1-46 represents 186 compounds wherein, in the general formula (I-1), $R^1$ represents $CH_2$ $CH_2OH$, $R^2$

does H, $R^3$ does Cl, and $R^4$ does H, and (X)n corresponds to each of all the substituents shown in Table 1-1.

Table 1-47

Table 1-47 represents 186 compounds wherein, in the general formula (I-1), $R^1$ represents $CH_2$ $CH_2OMe$, $R^2$ does H, $R^3$ does Cl, and $R^4$ does H, and (X)n corresponds to each of all the substituents shown in Table 1-1.

Table 1-48

Table 1-48 represents 186 compounds wherein, in the general formula (I-1), $R^1$ represents $CH_2OMe$, $R^2$ does H, $R^3$ does Cl, and $R^4$ does H, and (X)n corresponds to each of all the substituents shown in Table 1-1.

Table 1-49

Table 1-49 represents 186 compounds wherein, in the general formula (I-1), $R^1$ represents $CH_2OEt$, $R^2$ does H, $R^3$ does Cl, and $R^4$ does H, and (X)n corresponds to each of all the substituents shown in Table 1-1.

Table 1-50 .

Table 1-50 represents 186 compounds wherein, in the general formula (I-1), $R^1$ represents $CH_2COOH$, $R^2$ does H, $R^3$ does Cl, and $R^4$ does H, and (X)n corresponds to each of all the substituents shown in Table 1-1.

Table 1-51

Table 1-51 represents 186 compounds wherein, in the general formula (I-1), $R^1$ represents $CH_2COOEt$, $R^2$ does H, $R^3$ does Cl, and $R^4$ does H, and (X)n corresponds to each of all the substituents shown in Table 1-1.

Table 1-52

Table 1-52 represents 186 compounds wherein, in the general formula (I-1), $R^1$ represents $CH_2CONHMe$, $R^2$ does H, $R^3$ does Cl, and $R^4$ does H, and (X)n corresponds to each of all the substituents shown in Table 1-1.

Table 1-53

Table 1-53 represents 186 compounds wherein, in the general formula (I-1), $R^1$ represents $CH_2CONMe_2$, $R^2$ does H, $R^3$ does Cl, and $R^4$ does H, and (X)n corresponds to each of all the substituents shown in Table 1-1.

Table 1-54

Table 1-54 represents 186 compounds wherein, in the general formula (I-1), $R^1$ represents $CH_2CHO$, $R^2$ does H, $R^3$ does Cl, and $R^4$ does H, and (X)n corresponds to each of all the substituents shown in Table 1-1.

Table 1-55

Table 1-55 represents 186 compounds wherein, in the general formula (I-1), $R^1$ represents $CH_2CH{=}NOMe$, $R^2$ does H, $R^3$ does Cl, and $R^4$ does H, and (X)n corresponds to each of all the substituents shown in Table 1-1.

Table 1-56

Table 1-56 represents 186 compounds wherein, in the general formula (I-1), $R^1$ represents Et, $R^2$ does H, $R^3$ does H, and $R^4$ does H, and (X)n corresponds to each of all the substituents shown in Table 1-1.

Table 1-57

Table 1-57 represents 186 compounds wherein, in the general formula (I-1), $R^1$ represents Et, $R^2$ does H, $R^3$ does F, and $R^4$ does H, and (X)n corresponds to each of all the substituents shown in Table 1-1.

Table 1-58

Table 1-58 represents 186 compounds wherein, in the general formula (I-1), $R^1$ represents Et, $R^2$ does H, $R^3$ does Br, and $R^4$ does H, and (X)n corresponds to each of all the substituents shown in Table 1-1.

Table 1-59

Table 1-59 represents 186 compounds wherein, in the general formula (I-1), $R^1$ represents Et, $R^2$ does H, $R^3$ does I, and $R^4$ does H, and (X)n corresponds to each of all the substituents shown in Table 1-1.

Table 1-60

Table 1-60 represents 186 compounds wherein, in the general formula (I-1), $R^1$ represents Et, $R^2$ does H, $R^3$ does $CF_3$, and $R^4$ does H, and (X)n corresponds to each of all the substituents shown in Table 1-1.

Table 1-61

Table 1-61 represents 186 compounds wherein, in the general formula (I-1), $R^1$ represents Et, $R^2$ does H, $R^3$ does CN, and $R^4$ does H, and (X)n corresponds to each of all the substituents shown in Table 1-1.

Table 1-62

Table 1-62 represents 186 compounds wherein, in the general formula (I-1), $R^1$ represents Et, $R^2$ does H, $R^3$ does COOMe, and $R^4$ does H, and (X)n corresponds to each of all the substituents shown in Table 1-1.

Table 1-63

Table 1-63 represents 186 compounds wherein, in the general formula (1-1), $R^1$ represents Et, $R^2$ does H, $R^3$ does CONHMe, and $R^4$ does H, and (X)n corresponds to each of all the substituents shown in Table 1-1.

Table 1-64

Table 1-64 represents 186 compounds wherein, in the general formula (I-1), $R^1$ represents Et, $R^2$ does H, $R^3$ does $CONMe_2$, and $R^4$ does H, and (X)n corresponds to each of all the substituents shown in Table 1-1.

Table 1-65

Table 1-65 represents 186 compounds wherein, in the general formula (I-1), $R^1$ represents Et, $R^2$ does H, $R^3$ does SMe, and $R^4$ does H, and (X)n corresponds to each of all the substituents shown in Table 1-1.

Table 1-66

Table 1-66 represents 186 compounds wherein, in the general formula (I-1), $R^1$ represents Et, $R^2$ does H, $R^3$ does SPh, and $R^4$ does H, and (X)n corresponds to each of all the substituents shown in Table 1-1.

Table 1-67

Table 1-67 represents 186 compounds wherein, in the general formula (I-1), $R^1$ represents Et, $R^2$ does H, $R^3$ does OH, and $R^4$ does H, and (X)n corresponds to each of all the substituents shown in Table 1-1.

Table 1-68

Table 1-68 represents 186 compounds wherein, in the general formula (I-1), $R^1$ represents Et, $R^2$ does H, $R^3$ does OMe, and $R^4$ does H, and (X)n corresponds to each of all the substituents shown in Table 1-1.

Table 1-69

Table 1-69 represents 186 compounds wherein, in the general formula (I-1), $R^1$ represents Et, $R^2$ does H, $R^3$

does OPh, and $R^4$ does H, and (X)n corresponds to each of all the substituents shown in Table 1-1.

Table 1-70

Table 1-70 represents 186 compounds wherein, in the general formula (I-1), $R^1$ represents Et, $R^2$ does H, $R^3$ does $NMe_2$, and $R^4$ does H, and (X)n corresponds to each of all the substituents shown in Table 1-1.

Table 1-71

Table 1-71 represents 186 compounds wherein, in the general formula (I-1), $R^1$ represents Et, $R^2$ does H, $R^3$ does Cl, and $R^4$ does Me, and (X)n corresponds to each of all the substituents shown in Table 1-1.

Table 1-72

Table 1-72 represents 186 compounds wherein, in the general formula (I-1), $R^1$ represents Et, $R^2$ does H, $R^3$ does Cl, and $R^4$ does Et, and (X)n corresponds to each of all the substituents shown in Table 1-1.

Table 1-73

Table 1-73 represents 186 compounds wherein, in the general formula (I-1), $R^1$ represents Et, $R^2$ does H, $R^3$ does Cl, and $R^4$ does n-Pr, and (X)n corresponds to each of all the substituents shown in Table 1-1.

Table 1-74

Table 1-74 represents 186 compounds wherein, in the general formula (I-1), $R^1$ represents Et, $R^2$ does H, $R^3$ does Cl, and $R^4$ does i-Pr, and (X)n corresponds to each of all the substituents shown in Table 1-1.

Table 1-75

Table 1-75 represents 186 compounds wherein, in the general formula (I-1), $R^1$ represents Et, $R^2$ does H, $R^3$ does Cl, and $R^4$ does $CH_2CH{=}CH_2$, and (X)n corresponds to each of all the substituents shown in Table 1-1.

Table 1-76

Table 1-76 represents 186 compounds wherein, in the general formula (1-1), $R^1$ represents Et, $R^2$ does H, $R^3$ does Cl, and $R^4$ does $CH_2C{\equiv}CH$, and (X)n corresponds to each of all the substituents shown in Table 1-1.

Table 1-77

Table 1-77 represents 186 compounds wherein, in the general formula (I-1), $R^1$ represents Et, $R^2$ does H, $R^3$ does Cl, and $R^4$ does $CH_2CF_3$, and (X)n corresponds to each of all the substituents shown in Table 1-1.

Table 1-78

Table 1-78 represents 186 compounds wherein, in the general formula (I-1), $R^1$ represents Et, $R^2$ does H, $R^3$ does Cl, and $R^4$ does $CH_2CN$, and (X)n corresponds to each of all the substituents shown in Table 1-1.

Table 1-79

Table 1-79 represents 186 compounds wherein, in the general formula (I-1), $R^1$ represents Et, $R^2$ does H, $R^3$ does Cl, and $R^4$ does $CH_2OMe$, and (X)n corresponds to each of all the substituents shown in Table 1-1.

Table 1-80

Table 1-80 represents 186 compounds wherein, in the general formula (I-1), $R^1$ represents Et, $R^2$ does H, $R^3$ does Cl, and $R^4$ does $CH_2OEt$, and (X)n corresponds to each of all the substituents shown in Table 1-1.

Table 1-81

Table 1-81 represents 186 compounds wherein, in the general formula (I-1), $R^1$ represents Et, $R^2$ does H, $R^3$ does Cl, and $R^4$ does $CH_2SEt$, and (X)n corresponds to each of all the substituents shown in Table 1-1.

Table 1-82

Table 1-82 represents 186 compounds wherein, in the general formula (I-1), $R^1$ represents Et, $R^2$ does H, $R^3$ does Cl, and $R^4$ does $CH_2OOEt$, and (X)n corresponds to each of all the substituents shown in Table 1-1.

Table 1-83

Table 1-83 represents 186 compounds wherein, in the general formula (I-1), $R^1$ represents Et, $R^2$ does H, $R^3$ does Cl, and $R^4$ does COOMe, and (X)n corresponds to each of all the substituents shown in Table 1-1.

Table 1-84

Table 1-84 represents 186 compounds wherein, in the general formula (I-1), $R^1$ represents Et, $R^2$ does H, $R^3$ does Cl, and $R^4$ does CONHMe, and (X)n corresponds to each of all the substituents shown in Table 1-1.

Table 1-85

Table 1-85 represents 186 compounds wherein, in the general formula (I-1), $R^1$ represents Et, $R^2$ does H, $R^3$ does Cl, and $R^4$ does $CONMe_2$, and (X)n corresponds to each of all the substituents shown in Table 1-1.

Table 1-86

Table 1-86 represents 186 compounds wherein, in the general formula (I-1), $R^1$ represents Et, $R^2$ does H, $R^3$ does Cl, and $R^4$ does $COCH_3$, and (X)n corresponds to each of all the substituents shown in Table 1-1.

Table 1-87

Table 1-87 represents 186 compounds wherein, in the general formula (I-1), $R^1$ represents Et, $R^2$ does H, $R^3$ does Cl, and $R^4$ does SPh, and (X)n corresponds to each of all the substituents shown in Table 1-1.

Table 1-88

Table 1-88 represents 186 compounds wherein, in the general formula (I-1), $R^1$ represents Et, $R^2$ does H, $R^3$ does Cl, and $R^4$ does $CH_2$-c-Pr, and (X)n corresponds to each of all the substituents shown in Table 1-1.

The general formula (I-1a)

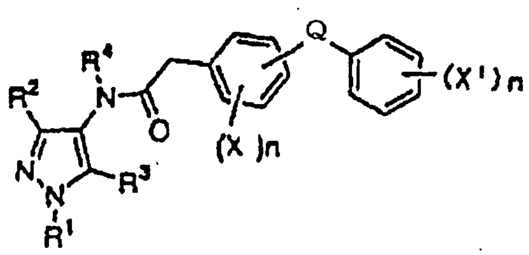

(I-1a)

Table 2-1

| $R^1$ | $R^2$ | $R^3$ | $R^4$ | Q | Bond | (X)n | $(X^1)$n |
|---|---|---|---|---|---|---|---|
| Me | Me | Cl | H | 0 | 4-position | H | H |

Table 2-1 (continued)

| $R^1$ | $R^2$ | $R^3$ | $R^4$ | Q | Bond | (X)n | $(X^1)n$ |
|---|---|---|---|---|---|---|---|
| Me | Me | Cl | H | 0 | 4-position | H | 2-F |
| Me | Me | Cl | H | 0 | 4-position | H | 3-F |
| Me | Me | Cl | H | 0 | 4-position | H | 4-F |
| Me | Me | Cl | H | 0 | 4-position | H | 2-Cl |
| Me | Me | Cl | H | 0 | 4-position | H | 3-Cl |
| Me | Me | Cl | H | 0 | 4-position | H | 4-Cl |
| Me | Me | Cl | H | 0 | 4-position | H | 2-Br |
| Me | Me | Cl | H | 0 | 4-position | H | 3-Br |
| Me | Me | Cl | H | 0 | 4-position | H | 4-Br |
| Me | Me | Cl | H | 0 | 4-position | H | 2-Me |
| Me | Me | Cl | H | 0 | 4-position | H | 3-Me |
| Me | Me | Cl | H | 0 | 4-position | H | 4-Me |
| Me | Me | Cl | H | 0 | 4-position | H | 2-OMe |
| Me | Me | Cl | H | 0 | 4-position | H | 3-OMe |
| Me | Me | Cl | H | 0 | 4-position | H | 4-OMe |
| Me | Me | Cl | H | 0 | 4-position | H | 2-$CF_3$ |
| Me | Me | Cl | H | 0 | 4-position | H | 3-$CF_3$ |
| Me | Me | Cl | H | 0 | 4-position | H | 4-$CF_3$ |
| Me | Me | Cl | H | 0 | 4-position | H | 4-$CF(CF_3)_2$ |
| Me | Me | Cl | H | 0 | 4-position | H | 4-$CH(CF_3)_2$ |
| Me | Me | Cl | H | 0 | 4-position | H | 4-$CF_2CF_3$ |
| Me | Me | Cl | H | 0 | 4-position | H | 2-$OCF_3$ |
| Me | Me | Cl | H | 0 | 4-position | H | 3-$OCF_3$ |
| Me | Me | Cl | H | 0 | 4-position | H | 4-$OCF_3$ |
| Me | Me | Cl | H | 0 | 4-position | H | 4-$OCH_2CF_3$ |
| Me | Me | Cl | H | 0 | 4-position | H | 4-$OCF_2CHF_2$ |
| Me | Me | Cl | H | 0 | 4-position | H | 4-$OCHF_2$ |
| Me | Me | Cl | H | 0 | 4-position | H | 4-$OCH(Me)CF_3$ |
| Me | Me | Cl | H | 0 | 4-position | H | 4-$OCH_2CF_2CHF_2$ |
| Me | Me | Cl | H | 0 | 4-position | H | 4-$OCH(CF_3)_2$ |
| Me | Me | Cl | H | 0 | 4-position | H | 2-OAc |
| Me | Me | Cl | H | 0 | 4-position | H | 3-OAc |
| Me | Me | Cl | H | 0 | 4-position | H | 4-OAc |
| Me | Me | Cl | H | 0 | 4-position | H | 2-$OCO_2Me$ |
| Me | Me | Cl | H | 0 | 4-position | H | 3-$OCO_2Me$ |
| Me | Me | Cl | H | 0 | 4-position | H | 4-$OCO_2Me$ |
| Me | Me | Cl | H | 0 | 4-position | H | 2-CN |
| Me | Me | Cl | H | 0 | 4-position | H | 3-CN |
| Me | Me | Cl | H | 0 | 4-position | H | 4-CN |
| Me | Me | Cl | H | 0 | 4-position | H | 2-$CO_2H$ |
| Me | Me | Cl | H | 0 | 4-position | H | 3-$CO_2H$ |
| Me | Me | Cl | H | 0 | 4-position | H | 4-$CO_2H$ |
| Me | Me | Cl | H | 0 | 4-position | H | 2-$CO_2Me$ |
| Me | Me | Cl | H | 0 | 4-position | H | 3-$CO_2Me$ |
| Me | Me | Cl | H | 0 | 4-position | H | 4-$CO_2Me$ |
| Me | Me | Cl | H | 0 | 4-position | H | 2-CONHMe |
| Me | Me | Cl | H | 0 | 4-position | H | 3-CONHMe |
| Me | Me | Cl | H | 0 | 4-position | H | 4-CONHMe |
| Me | Me | Cl | H | 0 | 4-position | H | 2-$CONMe_2$ |
| Me | Me | Cl | H | 0 | 4-position | H | 3-$CONMe_2$ |

Table 2-1 (continued)

| $R^1$ | $R^2$ | $R^3$ | $R^4$ | Q | Bond | (X)n | $(X^1)n$ |
|---|---|---|---|---|---|---|---|
| Me | Me | Cl | H | 0 | 4-position | H | 4-$CONMe_2$ |
| Me | Me | Cl | H | 0 | 4-position | H | 2-$NO_2$ |
| Me | Me | Cl | H | 0 | 4-position | H | 3-$NO_2$ |
| Me | Me | Cl | H | 0 | 4-position | H | 4-$NO_2$ |
| Me | Me | Cl | H | 0 | 4-position | H | 2-$NH_2$ |
| Me | Me | Cl | H | 0 | 4-position | H | 3-$NH_2$ |
| Me | Me | Cl | H | 0 | 4-position | H | 4-$NH_2$ |
| Me | Me | Cl | H | 0 | 4-position | H | 2-NHMe |
| Me | Me | Cl | H | 0 | 4-position | H | 3-NHMe |
| Me | Me | Cl | H | 0 | 4-position | H | 4-NHMe |
| Me | Me | Cl | H | 0 | 4-position | H | 2-$NMe_2$ |
| Me | Me | Cl | H | 0 | 4-position | H | 3-$NMe_2$ |
| Me | Me | Cl | H | 0 | 4-position | H | 4-$NMe_2$ |
| Me | Me | Cl | H | 0 | 4-position | H | 2-$NHCO_2Me$ |
| Me | Me | Cl | H | 0 | 4-position | H | 3-$NHCO_2Me$ |
| Me | Me | Cl | H | 0 | 4-position | H | 4-$NHCO_2Me$ |
| Me | Me | Cl | H | 0 | 4-position | H | 2-$NHCO_2Et$ |
| Me | Me | Cl | H | 0 | 4-position | H | 3-$NHCO_2Et$ |
| Me | Me | Cl | H | 0 | 4-position | H | 4-$NHCO_2Et$ |
| Me | Me | Cl | H | 0 | 4-position | H | 2-$NHCO_2$-i-Pr |
| Me | Me | Cl | H | 0 | 4-position | H | 3-$NHCO_2$-i-Pr |
| Me | Me | Cl | H | 0 | 4-position | H | 4-$NHCO_2$-i-Pr |
| Me | Me | Cl | H | 0 | 4-position | H | 2-$NHCO_2CH_2CH{=}CH_2$ |
| Me | Me | Cl | H | 0 | 4-position | H | 3-$NHCO_2CH_2CH{=}CH_2$ |
| Me | Me | Cl | H | 0 | 4-position | H | 4-$NHCO_2CH_2CH{=}CH_2$ |
| Me | Me | Cl | H | 0 | 4-position | H | 4-$NHCO_2CH_2C{\equiv}CH$ |
| Me | Me | Cl | H | 0 | 4-position | H | 2-$NHCO_2Ph$ |
| Me | Me | Cl | H | 0 | 4-position | H | 3-$NHCO_2Ph$ |
| Me | Me | Cl | H | 0 | 4-position | H | 4-$NHCO_2Ph$ |
| Me | Me | Cl | H | 0 | 4-position | H | 2-$NHCO_2CH_2Ph$ |
| Me | Me | Cl | H | 0 | 4-position | H | 3-$NHCO_2CH_2Ph$ |
| Me | Me | Cl | H | 0 | 4-position | H | 4-$NHCO_2CH_2Ph$ |
| Me | Me | Cl | H | 0 | 4-position | H | 2-NHCO-t-Bu |
| Me | Me | Cl | H | 0 | 4-position | H | 3-NHCO-t-Bu |
| Me | Me | Cl | H | 0 | 4-position | H | 4-NHCO-t-Bu |
| Me | Me | Cl | H | 0 | 4-position | H | 2-$NHCOCF_3$ |
| Me | Me | Cl | H | 0 | 4-position | H | 3-$NHCOCF_3$ |
| Me | Me | Cl | H | 0 | 4-position | H | 4-$NHCOCF_3$ |
| Me | Me | Cl | H | 0 | 4-position | H | 4-$NHCOCH_2Cl$ |
| Me | Me | Cl | H | 0 | 4-position | H | 2-NHAc |
| Me | Me | Cl | H | 0 | 4-position | H | 3-NHAc |
| Me | Me | Cl | H | 0 | 4-position | H | 4-NHAc |
| Me | Me | Cl | H | 0 | 4-position | H | 2-NHCOCH=CHMe |
| Me | Me | Cl | H | 0 | 4-position | H | 3-NHCOCH=CHMe |
| Me | Me | Cl | H | 0 | 4-position | H | 4-NHCOCH=CHMe |
| Me | Me | Cl | H | 0 | 4-position | H | 4-NHCO-c-Pr |
| Me | Me | Cl | H | 0 | 4-position | H | 2-NHCOPh |
| Me | Me | Cl | H | 0 | 4-position | H | 3-NHCOPh |
| Me | Me | Cl | H | O | 4-position | H | 4-NHCOPh |
| Me | Me | Cl | H | 0 | 4-position | H | 4-NHCO(thiophene-2-yl) |

Table 2-1 (continued)

| $R^1$ | $R^2$ | $R^3$ | $R^4$ | Q | Bond | (X)n | $(X^1)n$ |
|---|---|---|---|---|---|---|---|
| Me | Me | Cl | H | 0 | 4-position | H | 4-NHCO(pyridine-2-yl) |
| Me | Me | Cl | H | 0 | 4-position | H | 4-NHCO(pyridine-3-yl) |
| Me | Me | Cl | H | 0 | 4-position | H | 2-$NHSO_2Me$ |
| Me | Me | Cl | H | 0 | 4-position | H | 3-$NHSO_2Me$ |
| Me | Me | Cl | H | 0 | 4-position | H | 4-$NHSO_2Me$ |
| Me | Me | Cl | H | 0 | 4-position | H | 4-$NHSO_2$-c-Pr |
| Me | Me | Cl | H | 0 | 4-position | H | 2-$NHSO_2CF_3$ |
| Me | Me | Cl | H | 0 | 4-position | H | 3-$NHSO_2CF_3$ |
| Me | Me | Cl | H | 0 | 4-position | H | 4-$NHSO_2CF_3$ |
| Me | Me | Cl | H | O | 4-position | H | 2-$NHSO_2Ph$ |
| Me | Me | Cl | H | 0 | 4-position | H | 3-$NHSO_2Ph$ |
| Me | Me | Cl | H | 0 | 4-position | H | 4-$NHSO_2Ph$ |
| Me | Me | Cl | H | 0 | 4-position | H | 2-$SCF_3$ |
| Me | Me | Cl | H | 0 | 4-position | H | 3-$SCF_3$ |
| Me | Me | Cl | H | 0 | 4-position | H | 4-$SCF_3$ |
| Me | Me | Cl | H | 0 | 4-position | H | 2-$SOCF_3$ |
| Me | Me | Cl | H | 0 | 4-position | H | 3-$SOCF_3$ |
| Me | Me | Cl | H | 0 | 4-position | H | 4-$SOCF_3$ |
| Me | Me | Cl | H | 0 | 4-position | H | 2-$SO_2CF_3$ |
| Me | Me | Cl | H | 0 | 4-position | H | 3-$SO_2CF_3$ |
| Me | Me | Cl | H | 0 | 4-position | H | 4-$SO_2CF_3$ |
| Me | Me | Cl | H | 0 | 4-position | H | 2-SMe |
| Me | Me | Cl | H | 0 | 4-position | H | 3-SMe |
| Me | Me | Cl | H | 0 | 4-position | H | 4-SMe |
| Me | Me | Cl | H | 0 | 4-position | H | 2-SOMe |
| Me | Me | Cl | H | 0 | 4-position | H | 3-SOMe |
| Me | Me | Cl | H | 0 | 4-position | H | 4-SOMe |
| Me | Me | Cl | H | 0 | 4-position | H | 2-$SO_2Me$ |
| Me | Me | Cl | H | 0 | 4-position | H | 3-$SO_2Me$ |
| Me | Me | Cl | H | 0 | 4-position | H | 4-$SO_2Me$ |
| Me | Me | Cl | H | 0 | 4-position | H | 4-t-Bu |
| Me | Me | Cl | H | 0 | 4-position | H | 2-Ac |
| Me | Me | Cl | H | 0 | 4-position | H | 3-Ac |
| Me | Me | Cl | H | 0 | 4-position | H | 4-Ac |
| Me | Me | Cl | H | 0 | 4-position | H | 2-C(Me)=NOH |
| Me | Me | Cl | H | 0 | 4-position | H | 3-C(Me)=NOH |
| Me | Me | Cl | H | 0 | 4-position | H | 4-C(Me)=NOH |
| Me | Me | Cl | H | 0 | 4-position | H | 2-C(Me)=NOMe |
| Me | Me | Cl | H | 0 | 4-position | H | 3-C(Me)=NOMe |
| Me | Me | Cl | H | O | 4-position | H | 4-C(Me)=NOMe |
| Me | Me | Cl | H | 0 | 4-position | H | 2-C(Me)=$NOCH_2CH=CH_2$ |
| Me | Me | Cl | H | 0 | 4-position | H | 2-C(Me)=NO-t-Bu |
| Me | Me | Cl | H | 0 | 4-position | H | 2-C(Me)=$NOCH_2Ph$ |
| Me | Me | Cl | H | 0 | 4-position | H | 2-C(Me)=$NOCH_2$(4-$CF_3$-Ph) |
| Me | He | Cl | H | 0 | 4-position | H | 2-C(Me)=$NOCH_2$(2,4-$Cl_2$-Ph |
| Me | Me | Cl | H | 0 | 4-position | H | 2-C(Me)=$NOCH_2$(2,6-$Cl_2$-Ph |
| Me | Me | Cl | H | 0 | 4-position | H | 2-C(Me)=$NOCH_2$(2-Cl-pyridine-5-yl) |
| Me | Me | Cl | H | 0 | 4-position | H | 3-C(Me)=$NOCH_2CH=CH_2$ |
| Me | Me | Cl | H | 0 | 4-position | H | 3-C(Me)=NO-t-Bu |
| Me | Me | Cl | H | 0 | 4-position | H | 3-C(Me)=$NOCH_2Ph$ |

Table 2-1 (continued)

| $R^1$ | $R^2$ | $R^3$ | $R^4$ | Q | Bond | (X)n | $(X^1)n$ |
|---|---|---|---|---|---|---|---|
| Me | Me | Cl | H | 0 | 4-position | H | 3-C(Me)=$NOCH_2$(4-CF-$_3$Ph) |
| Me | Me | Cl | H | 0 | 4-position | H | 3-C(Me)=$NOCH_2$(2,4-$Cl_2$-Ph) |
| Me | Me | Cl | H | 0 | 4-position | H | 3-C(Me)=$NOCH_2$(2,6-$Cl_2$-Ph) |
| Me | Me | Cl | H | 0 | 4-position | H | 3-C(Me)=$NOCH_2$(2-Cl-pyridine-5-yl) |
| Me | Me | Cl | H | 0 | 4-position | H | 4-C(Me)=$NOCH_2CH=CH_2$ |
| Me | Me | Cl | H | 0 | 4-position | H | 4-C(Me)=NO-t-Bu |
| Me | Me | Cl | H | 0 | 4-position | H | 4-C(Me)=$NOCH_2Ph$ |
| Me | Me | Cl | H | 0 | 4-position | H | 4-C(Me)=$NOCH_2$(4-$CF_3$-Ph) |
| Me | Me | Cl | H | 0 | 4-position | H | 4-C(Me)=$NOCH_2$(2,4-$Cl_2$-Ph) |
| Me | Me | Cl | H | 0 | 4-position | H | 4-C(Me)=$NOCH_2$(2,6-$Cl_2$-Ph) |
| Me | Me | Cl | H | 0 | 4-position | H | 4-C(Me)=$NOCH_2$(2-Cl-pyridine-5-yl) |
| Me | Me | Cl | H | 0 | 4-position | H | 2,3-$Cl_2$ |
| Me | Me | Cl | H | 0 | 4-position | H | 2,4-$Cl_2$ |
| Me | Me | Cl | H | 0 | 4-position | H | 2,5-$Cl_2$ |
| Me | Me | Cl | H | 0 | 4-position | H | 2,6-$Cl_2$ |
| Me | Me | Cl | H | 0 | 4-position | H | 3,4-$Cl_2$ |
| Me | Me | Cl | H | 0 | 4-position | H | 3,5-$Cl_2$ |
| Me | Me | Cl | H | O | 4-position | H | 2,3-$Me_2$ |
| Me | Me | Cl | H | O | 4-position | H | 2,4-$Me_2$ |
| Me | Me | Cl | H | 0 | 4-position | H | 2,5-$Me_2$ |
| Me | Me | Cl | H | 0 | 4-position | H | 2,6-$Me_2$ |
| Me | Me | Cl | H | 0 | 4-position | H | 3,4-$C1_2$ |
| Me | Me | Cl | H | 0 | 4-position | H | 2-F-4-CN |
| Me | Me | Cl | H | 0 | 4-position | H | 2-F-4-$NO_2$ |
| Me | Me | Cl | H | 0 | 4-position | H | 2,5-$F_2$-4-CN |
| Me | Me | Cl | H | 0 | 4-position | H | 2,4,6-$Cl_3$ |
| Me | Me | Cl | H | 0 | 4-position | H | 2,4,6-$Me_3$ |
| Me | Me | Cl | H | 0 | 4-position | H | 4-oxadiazole-3-yl |

Note: "Bond" in the Table means the bonded position of the group Q.

Table 2-2

Table 2-2 represents 169 compounds wherein, in the general formula (I-1a), $R^1$ represents Me, $R^2$ does Et, $R^3$ does Cl, $R^4$ does H, Q does an oxygen atom and the bonded position thereof being a 4-position, (X)n does H, and $(X^1)n$ corresponds to each of all the substituents shown in Table 2-1.

Table 2-3

Table 2-3 represents 169 compounds wherein, in the general formula (I-1a), $R^1$ represents Me, $R^2$ does i-Pr, $R^3$ does Cl, $R^4$ does H, Q does an oxygen atom and the bonded position thereof being a 4-position, (X)n does H, and $(X^1)n$ corresponds to each of all the substituents shown in Table 2-1.

Table 2-4

Table 2-4 represents 169 compounds wherein, in the general formula (I-1a), $R^1$ represents Me, $R^2$ does n-Pr, $R^3$ does Cl, $R^4$ does H, Q does an oxygen atom and the bonded position thereof being a 4-position, (X)n does H, and $(X^1)n$ corresponds to each of all the substituents shown in Table 2-1.

Table 2-5

Table 2-5 represents 169 compounds wherein, in the general formula (I-1a), $R^1$ represents Me, $R^2$ does t-Bu, $R^3$ does Cl, $R^4$ does H, Q does an oxygen atom and the bonded position thereof being a 4-position, (X)n does H, and

$(X^1)$n corresponds to each of all the substituents shown in Table 2-1.

Table 2-6

Table 2-6 represents 169 compounds wherein, in the general formula (I-1a), $R^1$ represents Me, $R^2$ does n-Bu, $R^3$ does Cl, $R^4$ does H, Q does an oxygen atom and the bonded position thereof being a 4-position, (X)n does H, and $(X^1)$n corresponds to each of all the substituents shown in Table 2-1.

Table 2-7

Table 2-7 represents 169 compounds wherein, in the general formula (I-1a), $R^1$ represents Me, $R^2$ does $CF_3$, $R^3$ does Cl, $R^4$ does H, Q does an oxygen atom and the bonded position thereof being a 4-position, (X)n does H, and $(X^1)$n corresponds to each of all the substituents shown in Table 2-1.

Table 2-8

Table 2-8 represents 169 compounds wherein, in the general formula (I-1a), $R^1$ represents Me, $R^2$ does OMe, $R^3$ does Cl, $R^4$ does H, Q does an oxygen atom and the bonded position thereof being a 4-position, (X)n does H, and $(X^1)$n corresponds to each of all the substituents shown in Table 2-1.

Table 2-9

Table 2-9 represents 169 compounds wherein, in the general formula (I-1a), $R^1$ represents Me, $R^2$ does $OCHF_2$, $R^3$ does Cl, $R^4$ does H, Q does an oxygen atom and the bonded position thereof being a 4-position, (X)n does H, and $(X^1)$n corresponds to each of all the substituents shown in Table 2-1.

Table 2-10

Table 2-10 represents 169 compounds wherein, in the general formula (I-1a), $R^1$ represents Me, $R^2$ does O-i-Pr, $R^3$ does Cl, $R^4$ does H, Q does an oxygen atom and the bonded position thereof being a 4-position, (X)n H, and $(X^1)$n corresponds to each of all the substituents shown in Table 2-1.

Table 2-11

Table 2-11 represents 169 compounds wherein, in the general formula (I-1a), $R^1$ represents Me, $R^2$ does Cl, $R^3$ does Cl, $R^4$ does H, Q does an oxygen atom and the bonded position thereof being a 4-position, (X)n H, and $(X^1)$n corresponds to each of all the substituents shown in Table 2-1.

Table 2-12

Table 2-12 represents 169 compounds wherein, in the general formula (I-1a), $R^1$ represents Me, $R^2$ does Ph, $R^3$ does Cl, $R^4$ does H, Q does an oxygen atom and the bonded position thereof being a 4-position, (X)n does H, and $(X^1)$n corresponds to each of all the substituents shown in Table 2-1.

Table 2-13

Table 2-13 represents 169 compounds wherein, in the general formula (I-1a), $R^1$ represents Me, $R^2$ does 2-Cl-Ph, $R^3$ does Cl, $R^4$ does H, Q does an oxygen atom and the bonded position thereof being a 4-position, (X)n does H, and $(X^1)$n corresponds to each of all the substituents shown in Table 2-1.

Table 2-14

Table 2-14 represents 169 compounds wherein, in the general formula (I-1a), $R^1$ represents Me, $R^2$ does 3-Cl-Ph, $R^3$ does Cl, $R^4$ does H, Q does an oxygen atom and the bonded position thereof being a 4-position, (X)n does H, and $(X^1)$n corresponds to each of all the substituents shown in Table 2-1.

Table 2-15

Table 2-15 represents 169 compounds wherein, in the general formula (I-1a), $R^1$ represents Me, $R^2$ does 4-Cl-Ph, $R^3$ does Cl, $R^4$ does H, Q does an oxygen atom and the bonded position thereof being a 4-position, (X)n does H, and $(X^1)$n corresponds to each of all the substituents shown in Table 2-1.

Table 2-16

Table 2-16 represents 169 compounds wherein, in the general formula (I-1a), $R^1$ represents Me, $R^2$ does 2,4-$Cl_2$-Ph, $R^3$ does Cl, $R^4$ does H, Q does an oxygen atom and the bonded position thereof being a 4-position, (X)n does H, and $(X^1)$n corresponds to each of all the substituents shown in Table 2-1.

Table 2-17

Table 2-17 represents 169 compounds wherein, in the general formula (I-1a), $R^1$ represents Me, $R^2$ does 2-Me-Ph, $R^3$ does Cl, $R^4$ does H, Q does an oxygen atom and the bonded position thereof being a 4-position, (X)n does H, and $(X^1)$n corresponds to each of all the substituents shown in Table 2-1.

Table 2-18

Table 2-18 represents 169 compounds wherein, in the general formula (I-la), $R^1$ represents Me, $R^2$ does 3-Me-Ph, $R^3$ does Cl, $R^4$ does H, Q does an oxygen atom and the bonded position thereof being a 4-position, (X)n does H, and $(X^1)$n corresponds to each of all the substituents shown in Table 2-1.

Table 2-19

Table 2-19 represents 169 compounds wherein, in the general formula (I-1a), $R^1$ represents Me, $R^2$ does 4-Me-Ph, $R^3$ does Cl, $R^4$ does H, Q does an oxygen atom and the bonded position thereof being a 4-position, (X)n does H, and $(X^1)$n corresponds to each of all the substituents shown in Table 2-1.

Table 2-20

Table 2-20 represents 169 compounds wherein, in the general formula (I-1a), $R^1$ represents Me, $R^2$ does 2-OMe-Ph, $R^3$ does Cl, $R^4$ does H, Q does an oxygen atom and the bonded position thereof being a 4-position, (X)n does H, and $(X^1)$n corresponds to each of all the substituents shown in Table 2-1.

Table 2-21

Table 2-21 represents 169 compounds wherein, in the general formula (I-1a), $R^1$ represents Me, $R^2$ does 3-OMe-Ph, $R^3$ does Cl, $R^4$ does H, Q does an oxygen atom and the bonded position thereof being a 4-position, (X)n does H, and $(X^1)$n corresponds to each of all the substituents shown in Table 2-1.

Table 2-22

Table 2-22 represents 169 compounds wherein, in the general formula (I-1a), $R^1$ represents Me, $R^2$ does 4-OMe-Ph, $R^3$ does Cl, $R^4$ does H, Q does an oxygen atom and the bonded position thereof being a 4-position, (X)n does H, and $(X^1)$n corresponds to each of all the substituents shown in Table 2-1.

Table 2-23

Table 2-23 represents 169 compounds wherein, in the general formula (I-1a), $R^1$ represents Me, $R^2$ does H, $R^3$ does Cl, $R^4$ does H, Q does an oxygen atom and the bonded position thereof being a 4-position, (X)n does 3-OMe, and $(X^1)$n corresponds to each of all the substituents shown in Table 2-1.

Table 2-24

Table 2-24 represents 169 compounds wherein, in the general formula (I-1a), $R^1$ represents H, $R^2$ does H, $R^3$ does Cl, $R^4$ does H, Q does an oxygen atom and the bonded position thereof being a 4-position, (X)n does 3-OMe,

and $(X^1)$n corresponds to each of all the substituents shown in Table 2-1.

Table 2-25

Table 2-25 represents 169 compounds wherein, in the general formula (I-1a), $R^1$ represents Et, $R^2$ does H, $R^3$ does Cl, $R^4$ does H, Q does an oxygen atom and the bonded position thereof being a 4-position, (X)n does 3-OMe, and $(X^1)$n corresponds to each of all the substituents shown in Table 2-1.

Table 2-26

Table 2-26 represents 169 compounds wherein, in the general formula (I-la), $R^1$ represents i-Pr, $R^2$ does H, $R^3$ does Cl, $R^4$ does H, Q does an oxygen atom and the bonded position thereof being a 4-position, (X)n does 3-OMe, and $(X^1)$n corresponds to each of all the substituents shown in Table 2-1.

Table 2-27

Table 2-27 represents 169 compounds wherein, in the general formula (I-la), $R^1$ represents n-Pr, $R^2$ does H, $R^3$ does Cl, $R^4$ does H, Q an does oxygen atom and the bonded position thereof being a 4-position, (X)n does 3-OMe, and $(X^1)$n corresponds to each of all the substituents shown in Table 2-1.

Table 2-28

Table 2-28 represents 169 compounds wherein, in the general formula (I-1a), $R^1$ represents t-Bu, $R^2$ does H, $R^3$ does Cl, $R^4$ does H, Q does an oxygen atom and the bonded position thereof being a 4-position, (X)n does 3-OMe, and $(X^1)$n corresponds to each of all the substituents shown in Table 2-1.

Table 2-29

Table 2-29 represents 169 compounds wherein, in the general formula (I-1a), $R^1$ represents n-Bu, $R^2$ does H, $R^3$ does Cl, $R^4$ does H, Q does an oxygen atom and the bonded position thereof being a 4-position, (X)n does 3-OMe, and $(X^1)$n corresponds to each of all the substituents shown in Table 2-1.

Table 2-30

Table 2-30 represents 169 compounds wherein, in the general formula (I-1a), $R^1$ represents $CH=CH_2$, $R^2$ does H, $R^3$ does Cl, $R^4$ does H, Q does an oxygen atom and the bonded position thereof being a 4-position, (X)n does 3-OMe, and $(X^1)$n corresponds to each of all the substituents shown in Table 2-1.

Table 2-31

Table 2-31 represents 169 compounds wherein, in the general formula (I-1a), $R^1$ represents $CH_2CH=CH_2$, $R^2$ does H, $R^3$ does Cl, $R^4$ does H, Q does an oxygen atom and the bonded position thereof being a 4-position, (X)n does 3-OMe, and $(X^1)$n corresponds to each of all the substituents shown in Table 2-1.

Table 2-32

Table 2-32 represents 169 compounds wherein, in the general formula (I-1a), $R^1$ represents $CH_2C\equiv CH$, $R^2$ does H, $R^3$ does Cl, $R^4$ does H, Q does an oxygen atom and the bonded position thereof being a 4-position, (X)n does 3-OMe, and $(X^1)$n corresponds to each of all the substituents shown in Table 2-1.

Table 2-33

Table 2-33 represents 169 compounds wherein, in the general formula (I-1a), $R^1$ represents $CH_2CN$, $R^2$ does H, $R^3$ does Cl, $R^4$ does H, Q does an oxygen atom and the bonded position thereof being a 4-position, (X)n does 3-OMe, and $(X^1)$n corresponds to each of all the substituents shown in Table 2-1.

Table 2-34

Table 2-34 represents 169 compounds wherein, in the general formula (I-1a), $R^1$ represents $CH_2CF_3$, $R^2$ does H, $R^3$ does Cl, $R^4$ does H, Q does an oxygen atom and the bonded position thereof being a 4-position, (X)n does 3-OMe, and ($X^1$)n corresponds to each of all the substituents shown in Table 2-1.

Table 2-35

Table 2-35 represents 169 compounds wherein, in the general formula (I-1a), $R^1$ represents $CH_2Ph$, $R^2$ does H, $R^3$ does Cl, $R^4$ does H, Q does an oxygen atom and the bonded position thereof being a 4-position, (X)n does 3-OMe, and ($X^1$)n corresponds to each of all the substituents shown in Table 2-1.

Table 2-36

Table 2-36 represents 169 compounds wherein, in the general formula (I-1a), $R^1$ represents Ph, $R^2$ does H, $R^3$ does Cl, $R^4$ does H, Q does an oxygen atom and the bonded position thereof being a 4-position, (X)n does 3-OMe, and ($X^1$)n corresponds to each of all the substituents shown in Table 2-1.

Table 2-37

Table 2-37 represents 169 compounds wherein, in the general formula (I-1a), $R^1$ represents 2-Cl-Ph, $R^2$ does H, $R^3$ does Cl, $R^4$ does H, Q does an oxygen atom and the bonded position thereof being a 4-position, (X)n 3-OMe, and ($X^1$)n corresponds to each of all the substituents shown in Table 2-1.

Table 2-38

Table 2-38 represents 169 compounds wherein, in the general formula (I-1a), $R^1$ represents 3-Cl-Ph, $R^2$ does H, $R^3$ does Cl, $R^4$ does H, Q does an oxygen atom and the bonded position thereof being a 4-position, (X)n does 3-OMe, and ($X^1$)n corresponds to each of all the substituents shown in Table 2-1.

Table 2-39

Table 2-39 represents 169 compounds wherein, in the general formula (I-1a), $R^1$ represents 4-Cl-Ph, $R^2$ does H, $R^3$ does Cl, $R^4$ does H, Q does an oxygen atom and the bonded position thereof being a 4-position, (X)n does 3-OMe, and ($X^1$)n corresponds to each of all the substituents shown in Table 2-1.

Table 2-40

Table 2-40 represents 169 compounds wherein, in the general formula (I-1a), $R^1$ represents 2,4-$Cl_2$-Ph, $R^2$ does H, $R^3$ does Cl, $R^4$ does H, Q does an oxygen atom and the bonded position thereof being a 4-position, (X)n does 3-OMe, and ($X^1$)n corresponds to each of all the substituents shown in Table 2-1.

Table 2-41

Table 2-41 represents 169 compounds wherein, in the general formula (I-1a), $R^1$ represents Ac, $R^2$ does H, $R^3$ does Cl, $R^4$ does H, Q does an oxygen atom and the bonded position thereof being a 4-position, (X)n does 3-OMe, and ($X^1$)n corresponds to each of all the substituents shown in Table 2-1.

Table 2-42

Table 2-42 represents 169 compounds wherein, in the general formula (I-1a), $R^1$ represents COPh, $R^2$ does H, $R^3$ does Cl, $R^4$ does H, Q an oxygen does atom and the bonded position thereof being a 4-position, (X)n does 3-OMe, and ($X^1$)n corresponds to each of all the substituents shown in Table 2-1.

Table 2-43

Table 2-43 represents 169 compounds wherein, in the general formula (I-1a), $R^1$ represents $CH_2CH_2F$, $R^2$ does H, $R^3$ does Cl, $R^4$ does H, Q does an oxygen atom and the bonded position thereof being a 4-position, (X)n does

3-OMe, and $(X^1)$n corresponds to each of all the substituents shown in Table 2-1.

Table 2-44

Table 2-44 represents 169 compounds wherein, in the general formula (I-1a), $R^1$ represents $CH_2CH_2Cl$, $R^2$ does H, $R^3$ does Cl, $R^4$ does H, Q does an oxygen atom and the bonded position thereof being a 4-position, (X)n does 3-OMe, and $(X^1)$n corresponds to each of all the substituents shown in Table 2-1.

Table 2-45

Table 2-45 represents 169 compounds wherein, in the general formula (I-1a), $R^1$ represents $CH_2CH_2Br$, $R^2$ does H, $R^3$ does Cl, $R^4$ does H, Q does an oxygen atom and the bonded position thereof being a 4-position, (X)n does 3-OMe, and $(X^1)$n corresponds to each of all the substituents shown in Table 2-1.

Table 2-46

Table 2-46 represents 169 compounds wherein, in the general formula (I-1a), $R^1$ represents $CH_2CH_2OH$, $R^2$ does H, $R^3$ does Cl, $R^4$ does H, Q does an oxygen atom and the bonded position thereof being a 4-position, (X)n does 3-OMe, and $(X^1)$n corresponds to each of all the substituents shown in Table 2-1.

Table 2-47

Table 2-47 represents 169 compounds wherein, in the general formula (I-1a), $R^1$ represents $CH_2CH_2OMe$, $R^2$ does H, $R^3$ does Cl, $R^4$ does H, Q does an oxygen atom and the bonded position thereof being a 4-position, (X)n does 3-OMe, and $(X^1)$n corresponds to each of all the substituents shown in Table 2-1.

Table 2-48

Table 2-48 represents 169 compounds wherein, in the general formula (I-1a), $R^1$ represents $CH_2OMe$, $R^2$ does H, $R^3$ does Cl, $R^4$ does H, Q does an oxygen atom and the bonded position thereof being a 4-position, (X)n does 3-OMe, and $(X^1)$n corresponds to each of all the substituents shown in Table 2-1.

Table 2-49

Table 2-49 represents 169 compounds wherein, in the general formula (I-1a), $R^1$ represents $CH_2OEt$, $R^2$ does H, $R^3$ does Cl, $R^4$ does H, Q does an oxygen atom and the bonded position thereof being a 4-position, (X)n does 3-OMe, and $(X^1)$n corresponds to each of all the substituents shown in Table 2-1.

Table 2-50

Table 2-50 represents 169 compounds wherein, in the general formula (I-1a), $R^1$ represents $CH_2COOH$, $R^2$ does H, $R^3$ does Cl, $R^4$ does H, Q does an oxygen atom and the bonded position thereof being a 4-position, (X)n does 3-OMe, and $(X^1)$n corresponds to each of all the substituents shown in Table 2-1.

Table 2-51

Table 2-51 represents 169 compounds wherein, in the general formula (I-1a), $R^1$ represents $CH_2COOEt$, $R^2$ does H, $R^3$ does Cl, $R^4$ does H, Q does an oxygen atom and the bonded position thereof being a 4-position, (X)n does 3-OMe, and $(X^1)$n corresponds to each of all the substituents shown in Table 2-1.

Table 2-52

Table 2-52 represents 169 compounds wherein, in the general formula (I-1a), $R^1$ represents $CH_2CONHMe$, $R^2$ does H, $R^3$ does Cl, $R^4$ does H, Q does an oxygen atom and the bonded position thereof being a 4-position, (X)n does 3-OMe, and $(X^1)$n corresponds to each of all the substituents shown in Table 2-1.

Table 2-53

Table 2-53 represents 169 compounds wherein, in the general formula (I-1a), $R^1$ represents $CH_2CONMe_2$, $R^2$ does H, $R^3$ does Cl, $R^4$ does H, Q does an oxygen atom and the bonded position thereof being a 4-position, (X)n does 3-OMe, and $(X^1)$n corresponds to each of all the substituents shown in Table 2-1.

Table 2-54

Table 2-54 represents 169 compounds wherein, in the general formula (I-1a), $R^1$ represents $CH_2CHO$, $R^2$ does H, $R^3$ does Cl, $R^4$ does H, Q does an oxygen atom and the bonded position thereof being a 4-position, (X)n does 3-OMe, and $(X^1)$n corresponds to each of all the substituents shown in Table 2-1.

Table 2-55

Table 2-55 represents 169 compounds wherein, in the general formula (I-1a), $R^1$ represents $CH_2CH=NOMe$, $R^2$ does H, $R^3$ does Cl, $R^4$ does H, Q does an oxygen atom and the bonded position thereof being a 4-position, (X)n does 3-OMe, and $(X^1)$n corresponds to each of all the substituents shown in Table 2-1.

Table 2-56

Table 2-56 represents 169 compounds wherein, in the general formula (I-1a), $R^1$ represents Et, $R^2$ does H, $R^3$ does H, $R^4$ does H, Q does an oxygen atom and the bonded position thereof being a 4-position, (X)n does 3-OMe, and $(X^1)$n corresponds to each of all the substituents shown in Table 2-1.

Table 2-57

Table 2-57 represents 169 compounds wherein, in the general formula (I-1a), $R^1$ represents Et, $R^2$ does H, $R^3$ does F, $R^4$ does H, Q does an oxygen atom and the bonded position thereof being a 4-position, (X)n does 3-OMe, and $(X^1)$n corresponds to each of all the substituents shown in Table 2-1.

Table 2-58

Table 2-58 represents 169 compounds wherein, in the general formula (I-1a), $R^1$ represents Et, $R^2$ does H, $R^3$ does Br, $R^4$ does H, Q does an oxygen atom and the bonded position thereof being a 4-position, (X)n does 3-OMe, and $(X^1)$n corresponds to each of all the substituents shown in Table 2-1.

Table 2-59

Table 2-59 represents 169 compounds wherein, in the general formula (I-1a), $R^1$ represents Et, $R^2$ does H, $R^3$ does I, $R^4$ does H, Q does an oxygen atom and the bonded position thereof being a 4-position, (X)n does 3-OMe, and $(X^1)$n corresponds to each of all the substituents shown in Table 2-1.

Table 2-60

Table 2-60 represents 169 compounds wherein, in the general formula (I-1a), $R^1$ represents Et, $R^2$ does H, $R^3$ does $CF_3$, $R^4$ does H, Q does an oxygen atom and the bonded position thereof being a 4-position, (X)n does 3-OMe, and $(X^1)$n corresponds to each of all the substituents shown in Table 2-1.

Table 2-61

Table 2-61 represents 169 compounds wherein, in the general formula (I-1a), $R^1$ represents Et, $R^2$ does H, $R^3$ does CN, $R^4$ does H, Q does an oxygen atom and the bonded position thereof being a 4-position, (X)n does 3-OMe, and $(X^1)$n corresponds to each of all the substituents shown in Table 2-1.

Table 2-62

Table 2-62 represents 169 compounds wherein, in the general formula (I-1a), $R^1$ represents Et, $R^2$ does H, $R^3$ does COOMe, $R^4$ does H, Q does an oxygen atom and the bonded position thereof being a 4-position, (X)n does

3-OMe, and $(X^1)n$ corresponds to each of all the substituents shown in Table 2-1.

Table 2-63

Table 2-63 represents 169 compounds wherein, in the general formula (I-1a), $R^1$ represents Et, $R^2$ does H, $R^3$ does CONHMe, $R^4$ does H, Q does an oxygen atom and the bonded position thereof being a 4-position, (X)n does 3-OMe, and $(X^1)n$ corresponds to each of all the substituents shown in Table 2-1.

Table 2-64

Table 2-64 represents 169 compounds wherein, in the general formula (I-1a), $R^1$ represents Et, $R^2$ does H, $R^3$ does $CONMe_2$, $R^4$ does H, Q does an oxygen atom and the bonded position thereof being a 4-position, (X)n does 3-OMe, and $(X^1)n$ corresponds to each of all the substituents shown in Table 2-1.

Table 2-65

Table 2-65 represents 169 compounds wherein, in the general formula (I-1a), $R^1$ represents Et, $R^2$ does H, $R^3$ does SMe, $R^4$ does H, Q does an oxygen atom and the bonded position thereof being a 4-position, (X)n does 3-OMe, and $(X^1)n$ corresponds to each of all the substituents shown in Table 2-1.

Table 2-66

Table 2-66 represents 169 compounds wherein, in the general formula (I-1a), $R^1$ represents Et, $R^2$ does H, $R^3$ does SPh, $R^4$ does H, Q does an oxygen atom and the bonded position thereof being a 4-position, (X)n does 3-OMe, and $(X^1)n$ corresponds to each of all the substituents shown in Table 2-1.

Table 2-67

Table 2-67 represents 169 compounds wherein, in the general formula (I-1a), $R^1$ represents Et, $R^2$ does H, $R^3$ does OH, $R^4$ does H, Q does an oxygen atom and the bonded position thereof being a 4-position, (X)n does 3-OMe, and $(X^1)n$ corresponds to each of all the substituents shown in Table 2-1.

Table 2-68

Table 2-68 represents 169 compounds wherein, in the general formula (I-1a), $R^1$ represents Et, $R^2$ does H, $R^3$ does OMe, $R^4$ does H, Q does an oxygen atom and the bonded position thereof being a 4-position, (X)n does 3-OMe, and $(X^1)n$ corresponds to each of all the substituents shown in Table 2-1.

Table 2-69

Table 2-69 represents 169 compounds wherein, in the general formula (I-1a), $R^1$ represents Et, $R^2$ does H, $R^3$ does OPh, $R^4$ does H, Q does an oxygen atom and the bonded position thereof being a 4-position, (X)n does 3-OMe, and $(X^1)n$ corresponds to each of all the substituents shown in Table 2-1.

Table 2-70

Table 2-70 represents 169 compounds wherein, in the general formula (I-1a), $R^1$ represents Et, $R^2$ does H, $R^3$ does $NMe_2$, $R^4$ does H, Q does an oxygen atom and the bonded position thereof being a 4-position, (X)n does 3-OMe, and $(X^1)n$ corresponds to each of all the substituents shown in Table 2-1.

Table 2-71

Table 2-71 represents 169 compounds wherein, in the general formula (I-1a), $R^1$ represents Et, $R^2$ does H, $R^3$ does Cl, $R^4$ does Me, Q does an oxygen atom and the bonded position thereof being a 4-position, (X)n does 3-OMe, and $(X^1)n$ corresponds to each of all the substituents shown in Table 2-1.

Table 2-72

Table 2-72 represents 169 compounds wherein, in the general formula (I-1a), $R^1$ represents Et, $R^2$ does H, $R^3$ does Cl, $R^4$ does Et, Q does an oxygen atom and the bonded position thereof being a 4-position, (X)n does 3-OMe, and $(X^1)$n corresponds to each of all the substituents shown in Table 2-1.

Table 2-73

Table 2-73 represents 169 compounds wherein, in the general formula (I-1a), $R^1$ represents Et, $R^2$ does H, $R^3$ does Cl, $R^4$ does n-Pr, Q does an oxygen atom and the bonded position thereof being a 4-position, (X)n does 3-OMe, and $(X^1)$n corresponds to each of all the substituents shown in Table 2-1.

Table 2-74

Table 2-74 represents 169 compounds wherein, in the general formula (I-1a), $R^1$ represents Et, $R^2$ does H, $R^3$ does Cl, $R^4$ does i-Pr, Q does an oxygen atom and the bonded position thereof being a 4-position, (X)n does 3-OMe, and $(X^1)$n corresponds to each of all the substituents shown in Table 2-1.

Table 2-75

Table 2-75 represents 169 compounds wherein, in the general formula (I-1a), $R^1$ represents Et, $R^2$ does H, $R^3$ does Cl, $R^4$ does $CH_2CH{=}CH_2$, Q does an oxygen atom and the bonded position thereof being a 4-position, (X)n does 3-OMe, and $(X^1)$n corresponds to each of all the substituents shown in Table 2-1.

Table 2-76

Table 2-76 represents 169 compounds wherein, in the general formula (I-1a), $R^1$ represents Et, $R^2$ does H, $R^3$ does Cl, $R^4$ does $CH_2C{\equiv}CH$, Q does an oxygen atom and the bonded position thereof being a 4-position, (X)n does 3-OMe, and $(X^1)$n corresponds to each of all the substituents shown in Table 2-1.

Table 2-77

Table 2-77 represents 169 compounds wherein, in the general formula (I-1a), $R^1$ represents Et, $R^2$ does H, $R^3$ does Cl, $R^4$ does $CH_2CF_3$, Q does an oxygen atom and the bonded position thereof being a 4-position, (X)n does 3-OMe, and $(X^1)$n corresponds to each of all the substituents shown in Table 2-1.

Table 2-78

Table 2-78 represents 169 compounds wherein, in the general formula (I-1a), $R^1$ represents Et, $R^2$ does H, $R^3$ does Cl, $R^4$ does $CH_2CN$, Q does an oxygen atom and the bonded position thereof being a 4-position, (X)n 3-OMe, and $(X^1)$n corresponds to each of all the substituents shown in Table 2-1.

Table 2-79

Table 2-79 represents 169 compounds wherein, in the general formula (I-1a), $R^1$ represents Et, $R^2$ does H, $R^3$ does Cl, $R^4$ does $CH_2OMe$, Q does an oxygen atom and the bonded position thereof being a 4-position, (X)n does 3-OMe, and $(X^1)$n corresponds to each of all the substituents shown in Table 2-1.

Table 2-80

Table 2-80 represents 169 compounds wherein, in the general formula (I-1a), $R^1$ represents Et, $R^2$ does H, $R^3$ does Cl, $R^4$ does $CH_2OEt$, Q does an oxygen atom and the bonded position thereof being a 4-position, (X)n does 3-OMe, and $(X^1)$n corresponds to each of all the substituents shown in Table 2-1.

Table 2-81

Table 2-81 represents 169 compounds wherein, in the general formula (I-1a), $R^1$ represents Et, $R^2$ does H, $R^3$ does Cl, $R^4$ does $CH_2SMe$, Q does an oxygen atom and the bonded position thereof being a 4-position, (X)n does

3-OMe, and $(X^1)$n corresponds to each of all the substituents shown in Table 2-1.

Table 2-82

Table 2-82 represents 169 compounds wherein, in the general formula (I-1a), $R^1$ represents Et, $R^2$ does H, $R^3$ does Cl, $R^4$ does $CH_2COOEt$, Q does an oxygen atom and the bonded position thereof being a 4-position, (X)n does 3-OMe, and $(X^1)$n corresponds to each of all the substituents shown in Table 2-1.

Table 2-83

Table 2-83 represents 169 compounds wherein, in the general formula (I-1a), $R^1$ represents Et, $R^2$ does H, $R^3$ does Cl, $R^4$ does COOMe, Q does an oxygen atom and the bonded position thereof being a 4-position, (X)n does 3-OMe, and $(X^1)$n corresponds to each of all the substituents shown in Table 2-1.

Table 2-84

Table 2-84 represents 169 compounds wherein, in the general formula (I-1a), $R^1$ represents Et, $R^2$ does H, $R^3$ does Cl, $R^4$ does COOEt, Q does an oxygen atom and the bonded position thereof being a 4-position, (X)n does 3-OMe, and $(X^1)$n corresponds to each of all the substituents shown in Table 2-1.

Table 2-85

Table 2-85 represents 169 compounds wherein, in the general formula (I-1a), $R^1$ represents Et, $R^2$ does H, $R^3$ does Cl, $R^4$ does COO-n-Pr, Q does an oxygen atom and the bonded position thereof being a 4-position, (X)n does 3-OMe, and $(X^1)$n corresponds to each of all the substituents shown in Table 2-1.

Table 2-86

Table 2-86 represents 169 compounds wherein, in the general formula (I-1a), $R^1$ represents Et, $R^2$ does H, $R^3$ does Cl, $R^4$ does COO-i-Pr, Q does an oxygen atom and the bonded position thereof being a 4-position, (X)n does 3-OMe, and $(X^1)$n corresponds to each of all the substituents shown in Table 2-1.

Table 2-87

Table 2-87 represents 169 compounds wherein, in the general formula (I-1a), $R^1$ represents Et, $R^2$ does H, $R^3$ does Cl, $R^4$ does COO-t-Bu, Q does an oxygen atom and the bonded position thereof being a 4-position, (X)n does 3-OMe, and $(X^1)$n corresponds to each of all the substituents shown in Table 2-1.

Table 2-88

Table 2-88 represents 169 compounds wherein, in the general formula (I-1a), $R^1$ represents Et, $R^2$ does H, $R^3$ does Cl, $R^4$ does COOPh, Q does an oxygen atom and the bonded position thereof being a 4-position, (X)n does 3-OMe, and $(X^1)$n corresponds to each of all the substituents shown in Table 2-1.

Table 2-89

Table 2-89 represents 169 compounds wherein, in the general formula (I-1a), $R^1$ represents Et, $R^2$ does H, $R^3$ does Cl, $R^4$ does $COOCH_2Ph$, Q does an oxygen atom and the bonded position thereof being a 4-position, (X)n does 3-OMe, and $(X^1)$n corresponds to each of all the substituents shown in Table 2-1.

Table 2-90

Table 2-90 represents 169 compounds wherein, in the general formula (I-1a), $R^1$ represents Et, $R^2$ does H, $R^3$ does Cl, $R^4$ does CONHMe, Q does an oxygen atom and the bonded position thereof being a 4-position, (X)n does 3-OMe, and $(X^1)$n corresponds to each of all the substituents shown in Table 2-1.

Table 2-91

Table 2-91 represents 169 compounds wherein, in the general formula (I-1a), $R^1$ represents Et, $R^2$ does H, $R^3$ does Cl, $R^4$ does $CONMe_2$, Q does an oxygen atom and the bonded position thereof being a 4-position, (X)n does 3-OMe, and $(X^1)$n corresponds to each of all the substituents shown in Table 2-1.

Table 2-92

Table 2-92 represents 169 compounds wherein, in the general formula (I-1a), $R^1$ represents Et, $R^2$ does H, $R^3$ does Cl, $R^4$ does COMe, Q does an oxygen atom and the bonded position thereof being a 4-position, (X)n does 3-OMe, and $(X^1)$n corresponds to each of all the substituents shown in Table 2-1.

Table 2-93

Table 2-93 represents 169 compounds wherein, in the general formula (I-1a), $R^1$ represents Et, $R^2$ does H, $R^3$ does Cl, $R^4$ does COEt, Q does an oxygen atom and the bonded position thereof being a 4-position, (X)n does 3-OMe, and $(X^1)$n corresponds to each of all the substituents shown in Table 2-1.

Table 2-94

Table 2-94 represents 169 compounds wherein, in the general formula (I-1a), $R^1$ represents Et, $R^2$ does H, $R^3$ does Cl, $R^4$ does CO-n-Pr, Q does an oxygen atom and the bonded position thereof being a 4-position, (X)n does 3-OMe, and $(X^1)$n corresponds to each of all the substituents shown in Table 2-1.

Table 2-95

Table 2-95 represents 169 compounds wherein, in the general formula (I-1a), $R^1$ represents Et, $R^2$ does H, $R^3$ does Cl, $R^4$ does CO-i-Pr, Q does an oxygen atom and the bonded position thereof being a 4-position, (X)n does 3-OMe, and $(X^1)$n corresponds to each of all the substituents shown in Table 2-1.

Table 2-96

Table 2-96 represents 169 compounds wherein, in the general formula (I-1a), $R^1$ represents Et, $R^2$ does H, $R^3$ does Cl, $R^4$ does CO-t-Bu, Q does an oxygen atom and the bonded position thereof being a 4-position, (X)n does 3-OMe, and $(X^1)$n corresponds to each of all the substituents shown in Table 2-1.

Table 2-97

Table 2-97 represents 169 compounds wherein, in the general formula (I-1a), $R^1$ represents Et, $R^2$ does H, $R^3$ does Cl, $R^4$ does $COCF_3$, Q does an oxygen atom and the bonded position thereof being a 4-position, (X)n does 3-OMe, and $(X^1)$n corresponds to each of all the substituents shown in Table 2-1.

Table 2-98

Table 2-98 represents 169 compounds wherein, in the general formula (I-1a), $R^1$ represents Et, $R^2$ does H, $R^3$ does Cl, $R^4$ does COPh, Q does an oxygen atom and the bonded position thereof being a 4-position, (X)n does 3-OMe, and $(X^1)$n corresponds to each of all the substituents shown in Table 2-1.

Table 2-99

Table 2-99 represents 169 compounds wherein, in the general formula (I-1a), $R^1$ represents Et, $R^2$ does H, $R^3$ does Cl, $R^4$ does CO-c-Pr, Q does an oxygen atom and the bonded position thereof being a 4-position, (X)n does 3-OMe, and $(X^1)$n corresponds to each of all the substituents shown in Table 2-1.

Table 2-100

Table 2-100 represents 169 compounds wherein, in the general formula (I-1a), $R^1$ represents Et, $R^2$ does H, $R^3$ does Cl, $R^4$ does COCH=CHMe, Q does an oxygen atom and the bonded position thereof being a 4-position, (X)n does 3-OMe, and $(X^1)n$ corresponds to each of all the substituents shown in Table 2-1.

Table 2-101

Table 2-101 represents 169 compounds wherein, in the general formula (I-1a), $R^1$ represents Et, $R^2$ does H, $R^3$ does Cl, $R^4$ does SPh, Q does an oxygen atom and the bonded position thereof being a 4-position, (X)n does 3-OMe, and $(X^1)n$ corresponds to each of all the substituents shown in Table 2-1.

Table 2-102

Table 2-102 represents 169 compounds wherein, in the general formula (I-1a), $R^1$ represents Et, $R^2$ does H, $R^3$ does Cl, $R^4$ does $CH_2$-c-Pr, Q does an oxygen atom and the bonded position thereof being a 4-position, (X)n does 3-OMe, and $(X^1)n$ corresponds to each of all the substituents shown in Table 2-1.

Table 2-103

Table 2-103 represents 169 compounds wherein, in the general formula (I-1a), $R^1$ represents Et, $R^2$ H, $R^3$ Cl, $R^4$ H, Q a sulfur atom, the bonded position thereof a 4-position, (X)n 3-OMe, and $(X^1)n$ corresponds to each of all the substituents shown in Table 2-1.

Table 2-104

Table 2-104 represents 169 compounds wherein, in the general formula (I-1a), $R^1$ represents Et, $R^2$ does H, $R^3$ does Cl, $R^4$ does H, Q does NH and the bonded position thereof being a 4-position, (X)n does 3-OMe, and $(X^1)n$ corresponds to each of all the substituents shown in Table 2-1.

Table 2-105

Table 2-105 represents 169 compounds wherein, in the general formula (I-1a), $R^1$ represents Et, $R^2$ does H, $R^3$ does Cl, $R^4$ does H, Q does NMe and the bonded position thereof being a 4-position, (X)n does 3-OMe, and $(X^1)$ n corresponds to each of all the substituents shown in Table 2-1.

Table 2-106

Table 2-106 represents 169 compounds wherein, in the general formula (I-1a), $R^1$ represents Et, $R^2$ does H, $R^3$ does Cl, $R^4$ does H, Q does NAc and the bonded position thereof being a 4-position, (X)n does 3-OMe, and $(X^1)n$ corresponds to each of all the substituents shown in Table 2-1.

Table 2-107

Table 2-107 represents 169 compounds wherein, in the general formula (I-1a), $R^1$ represents Et, $R^2$ does H, $R^3$ does Cl, $R^4$ does H, Q does CH=CH and the bonded position thereof being a 4-position, (X)n 3-OMe, and $(X^1)n$ corresponds to each of all the substituents shown in Table 2-1.

Table 2-108

Table 2-108 represents 169 compounds wherein, in the general formula (I-1a), $R^1$ represents Et, $R^2$ does H, $R^3$ does Cl, $R^4$ does H, Q does $COCH_2$ and the bonded position thereof being a 4-position, (X)n does 3-OMe, and $(X^1)$ n corresponds to each of all the substituents shown in Table 2-1.

Table 2-109

Table 2-109 represents 169 compounds wherein, in the general formula (I-1a), $R^1$ represents Et, $R^2$ does H, $R^3$ does Cl, $R^4$ does H, Q does C(OMe)=CH and the bonded position thereof being a 4-position, (X)n does 3-OMe, and $(X^1)n$ corresponds to each of all the substituents shown in Table 2-1.

Table 2-110

Table 2-110 represents 169 compounds wherein, in the general formula (I-1a), $R^1$ represents Et, $R^2$ does H, $R^3$ does Cl, $R^4$ does H, Q does C≡C and the bonded position thereof being a 4-position, (X)n does 3-OMe, and ($X^1$)n corresponds to each of all the substituents shown in Table 2-1.

Table 2-111

Table 2-111 represents 169 compounds wherein, in the general formula (I-1a), $R^1$ represents Et, $R^2$ does H, $R^3$ does Cl, $R^4$ does H, Q does $CH_2CH_2$ and the bonded position thereof being a 4-position, (X)n does 3-OMe, and ($X^1$)n corresponds to each of all the substituents shown in Table 2-1.

Table 2-112

Table 2-112 represents 169 compounds wherein, in the general formula (I-1a), $R^1$ represents Et, $R^2$ does H, $R^3$ does Cl, $R^4$ does H, Q does $CH(OH)CH_2$ and the bonded position thereof being a 4-position, (X)n does 3-OMe, and ($X^1$)n corresponds to each of all the substituents shown in Table 2-1.

Table 2-113

Table 2-113 represents 169 compounds wherein, in the general formula (I-1a), $R^1$ represents Et, $R^2$ does H, $R^3$ does Cl, $R^4$ does H, Q does $CH(OMe)CH_2$ and the bonded position thereof being a 4-position, (X)n does 3-OMe, and ($X^1$)n corresponds to each of all the substituents shown in Table 2-1.

Table 2-114

Table 2-114 represents 169 compounds wherein, in the general formula (I-1a), $R^1$ represents Et, $R^2$ does H, $R^3$ does Cl, $R^4$ does H, Q does $OCH_2$ and the bonded position thereof being a 4-position, (X)n does 3-OMe, and ($X^1$)n corresponds to each of all the substituents shown in Table 2-1.

Table 2-115

Table 2-115 represents 169 compounds wherein, in the general formula (I-1a), $R^1$ represents Et, $R^2$ does H, $R^3$ does Cl, $R^4$ does H, Q does OCH(Me) and the bonded position thereof being a 4-position, (X)n does 3-OMe, and ($X^1$)n corresponds to each of all the substituents shown in Table 2-1.

Table 2-116

Table 2-116 represents 169 compounds wherein, in the general formula (I-1a), $R^1$ represents Et, $R^2$ does H, $R^3$ does Cl, $R^4$ does H, Q does $OCH_2CH_2$ and the bonded position thereof being a 4-position, (X)n does 3-OMe, and ($X^1$)n corresponds to each of all the substituents shown in Table 2-1.

Table 2-117

Table 2-117 represents 169 compounds wherein, in the general formula (I-1a), $R^1$ represents Et, $R^2$ does H, $R^3$ does Cl, $R^4$ does H, Q does $OCH_2CH{=}CH$ and the bonded position thereof being a 4-position, (X)n does 3-OMe, and ($X^1$)n corresponds to each of all the substituents shown in Table 2-1.

Table 2-118

Table 2-118 represents 169 compounds wherein, in the general formula (I-1a), $R^1$ represents Et, $R^2$ does H, $R^3$ does Cl, $R^4$ does H, Q does $SCH_2$ and the bonded position thereof being a 4-position, (X)n does 3-OMe, and ($X^1$)n corresponds to each of all the substituents shown in Table 2-1.

Table 2-119

Table 2-119 represents 169 compounds wherein, in the general formula (I-1a), $R^1$ represents Et, $R^2$ does H, $R^3$ does Cl, $R^4$ does H, Q does $SCH_2CH_2$ and the bonded position thereof being a 4-position, (X)n does 3-OMe, and

$(X^1)$n corresponds to each of all the substituents shown in Table 2-1.

Table 2-120

Table 2-120 represents 169 compounds wherein, in the general formula (I-1a), $R^1$ represents Et, $R^2$ does H, $R^3$ does Cl, $R^4$ does H, Q does $NHCH_2$ and the bonded position thereof being a 4-position, (X)n does 3-OMe, and $(X^1)$n corresponds to each of all the substituents shown in Table 2-1.

Table 2-121

Table 2-121 represents 169 compounds wherein, in the general formula (I-1a), $R^1$ represents Et, $R^2$ does H, $R^3$ does Cl, $R^4$ does H, Q does $NHCH_2CH_2$ and the bonded position thereof being a 4-position, (X)n does 3-OMe, and $(X^1)$n corresponds to each of all the substituents shown in Table 2-1.

Table 2-122

Table 2-122 represents 169 compounds wherein, in the general formula (I-1a), $R^1$ represents Et, $R^2$ does H, $R^3$ does Cl, $R^4$ does H, Q does $N(Me)CH_2$ and the bonded position thereof being a 4-position, (X)n does 3-OMe, and $(X^1)$n corresponds to each of all the substituents shown in Table 2-1.

Table 2-123

Table 2-123 represents 169 compounds wherein, in the general formula (I-1a), $R^1$ represents Et, $R^2$ does H, $R^3$ does Cl, $R^4$ does H, Q does $N(Me)CH_2CH_2$ and the bonded position thereof being a 4-position, (X)n does 3-OMe, and $(X^1)$n corresponds to each of all the substituents shown in Table 2-1.

Table 2-124

Table 2-124 represents 169 compounds wherein, in the general formula (I-1a), $R^1$ represents Et, $R^2$ does H, $R^3$ does Cl, $R^4$ does H, Q does NHCO and the bonded position thereof being a 4-position, (X)n does 3-OMe, and $(X^1)$n corresponds to each of all the substituents shown in Table 2-1.

Table 2-125

Table 2-125 represents 169 compounds wherein, in the general formula (I-1a), $R^1$ represents Et, $R^2$ does H, $R^3$ does Cl, $R^4$ does H, Q does N(Me)CO and the bonded position thereof being a 4-position, (X)n does 3-OMe, and $(X^1)$n corresponds to each of all the substituents shown in Table 2-1.

Table 2-126

Table 2-126 represents 169 compounds wherein, in the general formula (I-1a), $R^1$ represents Et, $R^2$ does H, $R^3$ does Cl, $R^4$ does H, Q does NHCOCH=CH and the bonded position thereof being a 4-position, (X)n does 3-OMe, and $(X^1)$n corresponds to each of all the substituents shown in Table 2-1.

Table 2-127

Table 2-127 represents 169 compounds wherein, in the general formula (I-1a), $R^1$ represents Et, $R^2$ does H, $R^3$ does Cl, $R^4$ does H, Q does CO and the bonded position thereof being a 4-position, (X)n does 3-OMe, and $(X^1)$n corresponds to each of all the substituents shown in Table 2-1.

Table 2-128

Table 2-128 represents 169 compounds wherein, in the general formula (I-1a), $R^1$ represents Et, $R^2$ does H, $R^3$ does Cl, $R^4$ does H, Q does C=NOMe and the bonded position thereof being a 4-position, (X)n does 3-OMe, and $(X^1)$n corresponds to each of all the substituents shown in Table 2-1.

Table 2-129

Table 2-129 represents 169 compounds wherein, in the general formula (I-1a), $R^1$ represents Et, $R^2$ does H, $R^3$ does Cl, $R^4$ does H, Q does an oxygen atom and the bonded position thereof being a 3-position, (X)n does H, and $(X^1)$n corresponds to each of all the substituents shown in Table 2-1.

Table 2-130

Table 2-130 represents 169 compounds wherein, in the general formula (I-1a), $R^1$ represents Et, $R^2$ does H, $R^3$ does Cl, $R^4$ does H, Q does an oxygen atom and the bonded position thereof being a 2-position, (X)n does H, and $(X^1)$n corresponds to each of all the substituents shown in Table 2-1.

Table 2-131

Table 2-131 represents 169 compounds wherein, in the general formula (I-1a), $R^1$ represents Et, $R^2$ does H, $R^3$ does Cl, $R^4$ does H, Q does an oxygen atom and the bonded position thereof being a 4-position, (X)n does 2-Cl, and $(X^1)$n corresponds to each of all the substituents shown in Table 2-1.

Table 2-132

Table 2-132 represents 169 compounds wherein, in the general formula (I-1a), $R^1$ represents Et, $R^2$ does H, $R^3$ does Cl, $R^4$ does H, Q does an oxygen atom and the bonded position thereof being a 4-position, (X)n does 3-Cl, and $(X^1)$n corresponds to each of all the substituents shown in Table 2-1.

Table 2-133

Table 2-133 represents 169 compounds wherein, in the general formula (I-1a), $R^1$ represents Et, $R^2$ does H, $R^3$ does Cl, $R^4$ does H, Q does an oxygen atom and the bonded position thereof being a 4-position, (X)n does 2-Me, and $(X^1)$n corresponds to each of all the substituents shown in Table 2-1.

Table 2-134

Table 2-134 represents 169 compounds wherein, in the general formula (I-1a), $R^1$ represents Et, $R^2$ does H, $R^3$ does Cl, $R^4$ does H, Q does an oxygen atom and the bonded position thereof being a 4-position, (X)n does 3-Me, and $(X^1)$n corresponds to each of all the substituents shown in Table 2-1.

Table 2-135

Table 2-135 represents 169 compounds wherein, in the general formula (I-1a), $R^1$ represents Et, $R^2$ does H, $R^3$ does Cl, $R^4$ does H, Q does an oxygen atom and the bonded position thereof being a 4-position, (X)n does 2-OMe, and $(X^1)$n corresponds to each of all the substituents shown in Table 2-1.

Table 2-136

Table 2-136 represents 169 compounds wherein, in the general formula (I-1a), $R^1$ represents Et, $R^2$ does H, $R^3$ does Cl, $R^4$ does H, Q does an oxygen atom and the bonded position thereof being a 4-position, (X)n does 2-$CF_3$, and $(X^1)$n corresponds to each of all the substituents shown in Table 2-1.

Table 2-137

Table 2-137 represents 169 compounds wherein, in the general formula (I-1a), $R^1$ represents Et, $R^2$ does H, $R^3$ does Cl, $R^4$ does H, Q does an oxygen atom and the bonded position thereof being a 4-position, (X)n does 3-$CF_3$, and $(X^1)$n corresponds to each of all the substituents shown in Table 2-1.

Table 2-138

Table 2-138 represents 169 compounds wherein, in the general formula (I-1a), $R^1$ represents Et, $R^2$ does H, $R^3$ does Cl, $R^4$ does H, Q does an oxygen atom and the bonded position thereof being a 4-position, (X)n does 3-OH,

and $(X^1)n$ corresponds to each of all the substituents shown in Table 2-1.

Table 2-139

Table 2-139 represents 169 compounds wherein, in the general formula (I-1a), $R^1$ represents Et, $R^2$ does H, $R^3$ does Cl, $R^4$ does H, Q does an oxygen atom and the bonded position thereof being a 4-position, (X)n does 3-$OCHF_2$, and $(X^1)n$ corresponds to each of all the substituents shown in Table 2-1.

Table 2-140

Table 2-140 represents 169 compounds wherein, in the general formula (I-1a), $R^1$ represents Et, $R^2$ does H, $R^3$ does Cl, $R^4$ does H, Q does an oxygen atom and the bonded position thereof being a 4-position, (X)n does 3-$OCF_3$, and $(X^1)n$ corresponds to each of all the substituents shown in Table 2-1.

Table 2-141

Table 2-141 represents 169 compounds wherein, in the general formula (I-1a), $R^1$ represents Et, $R^2$ does H, $R^3$ does Cl, $R^4$ does H, Q does an oxygen atom and the bonded position thereof being a 4-position, (X)n does 3-$OCF_2CHF_2$, and $(X^1)n$ corresponds to each of all the substituents shown in Table 2-1.

Table 2-142

Table 2-142 represents 169 compounds wherein, in the general formula (I-1a), $R^1$ represents Et, $R^2$ does H, $R^3$ does Cl, $R^4$ does H, Q does an oxygen atom and the bonded position thereof being a 4-position, (X)n does 3-$OCH_2CF_3$, and $(X^1)n$ corresponds to each of all the substituents shown in Table 2-1.

Table 2-143

Table 2-143 represents 169 compounds wherein, in the general formula (I-1a), $R^1$ represents Et, $R^2$ does H, $R^3$ does Cl, $R^4$ does H, Q does an oxygen atom and the bonded position thereof being a 4-position, (X)n does 3-$NH_2$, and $(X^1)n$ corresponds to each of all the substituents shown in Table 2-1.

Table 2-144

Table 2-144 represents 169 compounds wherein, in the general formula (I-1a), $R^1$ represents Et, $R^2$ does H, $R^3$ does Cl, $R^4$ does H, Q does an oxygen atom and the bonded position thereof being a 4-position, (X)n does 3-NHMe, and $(X^1)n$ corresponds to each of all the substituents shown in Table 2-1.

Table 2-145

Table 2-145 represents 169 compounds wherein, in the general formula (I-1a), $R^1$ represents Et, $R^2$ does H, $R^3$ does Cl, $R^4$ does H, Q does an oxygen atom and the bonded position thereof being a 4-position, (X)n does 3-$NMe_2$, and $(X^1)n$ corresponds to each of all the substituents shown in Table 2-1.

Table 2-146

Table 2-146 represents 169 compounds wherein, in the general formula (I-1a), $R^1$ represents Et, $R^2$ does H, $R^3$ does Cl, $R^4$ does H, Q does an oxygen atom and the bonded position thereof being a 4-position, (X)n does 3-NHAc, and $(X^1)n$ corresponds to each of all the substituents shown in Table 2-1.

Table 2-147

Table 2-147 represents 169 compounds wherein, in the general formula (I-1a), $R^1$ represents Et, $R^2$ does H, $R^3$ does Cl, $R^4$ does H, Q does an oxygen atom and the bonded position thereof being a 4-position, (X)n does 3-$NHCOCF_3$, and $(X^1)n$ corresponds to each of all the substituents shown in Table 2-1.

Table 2-148

Table 2-148 represents 169 compounds wherein, in the general formula (I-1a), $R^1$ represents Et, $R^2$ does H, $R^3$ does Cl, $R^4$ does H, Q does an oxygen atom and the bonded position thereof being a 4-position, (X)n does 3-NH-COOMe, and $(X^1)$n corresponds to each of all the substituents shown in Table 2-1.

Table 2-149

Table 2-149 represents 169 compounds wherein, in the general formula (I-1a), $R^1$ represents Et, $R^2$ does H, $R^3$ does Cl, $R^4$ does H, Q does an oxygen atom and the bonded position thereof being a 4-position, (X)n does 3-NH-COOMe, and $(X^1)$n corresponds to each of all the substituents shown in Table 2-1.

Table 2-150

Table 2-150 represents 169 compounds wherein, in the general formula (I-1a), $R^1$ represents Et, $R^2$ does H, $R^3$ does Cl, $R^4$ does H, Q does an oxygen atom and the bonded position thereof being a 4-position, (X)n does 3-$NHCH_2CN$, and $(X^1)$n corresponds to each of all the substituents shown in Table 2-1.

Table 2-151

Table 2-151 represents 169 compounds wherein, in the general formula (I-1a), $R^1$ represents Et, $R^2$ does H, $R^3$ does Cl, $R^4$ does H, Q does an oxygen atom and the bonded position thereof being a 4-position, (X)n does 3-NH-CONHMe, and $(X^1)$n corresponds to each of all the substituents shown in Table 2-1.

Table 2-152

Table 2-152 represents 169 compounds wherein, in the general formula (I-1a), $R^1$ represents Et, $R^2$ does H, $R^3$ does Cl, $R^4$ does H, Q does an oxygen atom and the bonded position thereof being a 4-position, (X)n does 3-$NHSO_2Me$, and $(X^1)$n corresponds to each of all the substituents shown in Table 2-1.

Table 2-153

Table 2-153 represents 169 compounds wherein, in the general formula (I-1a), $R^1$ represents Et, $R^2$ does H, $R^3$ does Cl, $R^4$ does H, Q does an oxygen atom and the bonded position thereof being a 4-position, (X)n does 3-$NHSO_2CF_3$, and $(X^1)$n corresponds to each of all the substituents shown in Table 2-1.

Table 2-154

Table 2-154 represents 169 compounds wherein, in the general formula (I-1a), $R^1$ represents Et, $R^2$ does H, $R^3$ does Cl, $R^4$ does H, Q does an oxygen atom and the bonded position thereof being a 4-position, (X)n does 3-$NHCHF_2$, and $(X^1)$n corresponds to each of all the substituents shown in Table 2-1.

Table 2-155

Table 2-155 represents 169 compounds wherein, in the general formula (I-1a), $R^1$ represents Et, $R^2$ does H, $R^3$ does Cl, $R^4$ does H, Q does an oxygen atom and the bonded position thereof being a 4-position, (X)n does 3-$N(CHF_2)_2$, and $(X^1)$n corresponds to each of all the substituents shown in Table 2-1.

Table 2-156

Table 2-156 represents 169 compounds wherein, in the general formula (I-1a), $R^1$ represents Et, $R^2$ does H, $R^3$ does Cl, $R^4$ does H, Q does an oxygen atom and the bonded position thereof being a 4-position, (X)n does 3-$NHCF_2CHF_2$, and $(X^1)$n corresponds to each of all the substituents shown in Table 2-1.

Table 2-157

Table 2-157 represents 169 compounds wherein, in the general formula (I-1a), $R^1$ represents Et, $R^2$ does H, $R^3$ does Cl, $R^4$ does H, Q does an oxygen atom and the bonded position thereof being a 4-position, (X)n does

3-$NHCH_2CF_3$, and $(X^1)$n corresponds to each of all the substituents shown in Table 2-1.

Table 2-158

Table 2-158 represents 169 compounds wherein, in the general formula (I-1a), $R^1$ represents Et, $R^2$ does H, $R^3$ does Cl, $R^4$ does H, Q does an oxygen atom and the bonded position thereof being a 4-position, (X)n does 3-SH, and $(X^1)$n corresponds to each of all the substituents shown in Table 2-1.

Table 2-159

Table 2-159 represents 169 compounds wherein, in the general formula (I-1a), $R^1$ represents Et, $R^2$ does H, $R^3$ does Cl, $R^4$ does H, Q does an oxygen atom and the bonded position thereof being a 4-position, (X)n does 3-SMe, and $(X^1)$n corresponds to each of all the substituents shown in Table 2-1.

Table 2-160

Table 2-160 represents 169 compounds wherein, in the general formula (I-1a), $R^1$ represents Et, $R^2$ does H, $R^3$ does Cl, $R^4$ does H, Q does an oxygen atom and the bonded position thereof being a 4-position, (X)n does 3-SOMe, and $(X^1)$n corresponds to each of all the substituents shown in Table 2-1.

Table 2-161

Table 2-161 represents 169 compounds wherein, in the general formula (I-1a), $R^1$ represents Et, $R^2$ does H, $R^3$ does Cl, $R^4$ does H, Q does an oxygen atom and the bonded position thereof being a 4-position, (X)n does 3-$SO_2Me$, and $(X^1)$n corresponds to each of all the substituents shown in Table 2-1.

Table 2-162

Table 2-162 represents 169 compounds wherein, in the general formula (I-1a), $R^1$ represents Et, $R^2$ does H, $R^3$ does Cl, $R^4$ does H, Q does an oxygen atom and the bonded position thereof being a 4-position, (X)n does 3-$SCHF_2$, and $(X^1)$n corresponds to each of all the substituents shown in Table 2-1.

Table 2-163

Table 2-163 represents 169 compounds wherein, in the general formula (I-1a), $R^1$ represents Et, $R^2$ does H, $R^3$ does Cl, $R^4$ does H, Q does an oxygen atom and the bonded position thereof being a 4-position, (X)n does 3-$SOCHF_2$, and $(X^1)$n corresponds to each of all the substituents shown in Table 2-1.

Table 2-164

Table 2-164 represents 169 compounds wherein, in the general formula (I-1a), $R^1$ represents Et, $R^2$ does H, $R^3$ does Cl, $R^4$ does H, Q does an oxygen atom and the bonded position thereof being a 4-position, (X)n does 3-$SO_2CHF_2$, and $(X^1)$n corresponds to each of all the substituents shown in Table 2-1.

Table 2-165

Table 2-162 represents 169 compounds wherein, in the general formula (I-1a), $R^1$ represents Et, $R^2$ does H, $R^3$ does Cl, $R^4$ does H, Q does an oxygen atom and the bonded position thereof being a 4-position, (X)n does 3-$SCF_3$, and $(X^1)$n corresponds to each of all the substituents shown in Table 2-1.

Table 2-166

Table 2-166 represents 169 compounds wherein, in the general formula (I-1a), $R^1$ represents Et, $R^2$ does H, $R^3$ does Cl, $R^4$ does H, Q does an oxygen atom and the bonded position thereof being a 4-position, (X)n does 3-$SCOCF_3$, and $(X^1)$n corresponds to each of all the substituents shown in Table 2-1.

Table 2-167

Table 2-167 represents 169 compounds wherein, in the general formula (I-1a), $R^1$ represents Et, $R^2$ does H, $R^3$ does Cl, $R^4$ does H, Q does an oxygen atom and the bonded position thereof being a 4-position, (X)n does 3-$SO_2CF_3$, and ($X^1$)n corresponds to each of all the substituents shown in Table 2-1.

Table 2-168

Table 2-168 represents 169 compounds wherein, in the general formula (I-1a), $R^1$ represents Et, $R^2$ H, $R^3$ Cl, $R^4$ H, Q an oxygen atom, the bonded position thereof a 4-position, (X)n 3-SC $F_2CHF_2$, and ($X^1$)n corresponds to each of all the substituents shown in Table 2-1.

Table 2-169

Table 2-169 represents 169 compounds wherein, in the general formula (I-1a), $R^1$ represents Et, $R^2$ does H, $R^3$ does Cl, $R^4$ does H, Q does an oxygen atom and the bonded position thereof being a 4-position, (X)n does 3-SOC $F_2CHF_2$, and ($X^1$)n corresponds to each of all the substituents shown in Table 2-1.

Table 2-170

Table 2-170 represents 169 compounds wherein, in the general formula (I-1a), $R^1$ represents Et, $R^2$ does H, $R^3$ does Cl, $R^4$ does H, Q does an oxygen atom and the bonded position thereof being a 4-position, (X)n does S-$SO_2C$ $F_2CHF_2$, and ($X^1$)n corresponds to each of all the substituents shown in Table 2-1.

Table 2-171

Table 2-171 represents 169 compounds wherein, in the general formula (I-1a), $R^1$ represents Et, $R^2$ does H, $R^3$ does Cl, $R^4$ does H, Q does an oxygen atom and the bonded position thereof being a 4-position, (X)n does 3-$SCH_2CF_3$, and ($X^1$)n corresponds to each of all the substituents shown in Table 2-1.

Table 2-172

Table 2-172 represents 169 compounds wherein, in the general formula (I-1a), $R^1$ represents Et, $R^2$ does H, $R^3$ does Cl, $R^4$ does H, Q does an oxygen atom and the bonded position thereof being a 4-position, (X)n does 3-$SOCH_2CF_3$, and ($X^1$)n corresponds to each of all the substituents shown in Table 2-1.

Table 2-173

Table 2-173 represents 169 compounds wherein, in the general formula (I-1a), $R^1$ represents Et, $R^2$ does H, $R^3$ does Cl, $R^4$ does H, Q does an oxygen atom and the bonded position thereof being a 4-position, (X)n does 3-$SO_2CH_2CF_3$, and ($X^1$)n corresponds to each of all the substituents shown in Table 2-1.

Table 2-174

Table 2-174 represents 169 compounds wherein, in the general formula (I-1a), $R^1$ represents Et, $R^2$ does H, $R^3$ does Cl, $R^4$ does H, Q does an oxygen atom and the bonded position thereof being a 4-position, (X)n does 3,5-$Cl_2$, and ($X^1$)n corresponds to each of all the substituents shown in Table 2-1.

Table 2-175

Table 2-175 represents 169 compounds wherein, in the general formula (I-1a), $R^1$ represents Et, $R^2$ does H, $R^3$ does Cl, $R^4$ does H, Q does an oxygen atom and the bonded position thereof being a 4-position, (X)n does 2,3-$Cl_2$, and ($X^1$)n corresponds to each of all the substituents shown in Table 2-1.

Table 2-176

Table 2-176 represents 169 compounds wherein, in the general formula (I-1a), $R^1$ represents Et, $R^2$ does H, $R^3$ does Cl, $R^4$ does H, Q does an oxygen atom and the bonded position thereof being a 4-position, (X)n does 2,5-$Cl_2$,

and $(X^1)$n corresponds to each of all the substituents shown in Table 2-1.

Table 2-177

Table 2-177 represents 169 compounds wherein, in the general formula (I-1a), $R^1$ represents Et, $R^2$ does H, $R^3$ does Cl, $R^4$ does H, Q does an oxygen atom and the bonded position thereof being a 4-position, (X)n does 3,5-$Me_2$, and $(X^1)$n corresponds to each of all the substituents shown in Table 2-1.

Table 2-178

Table 2-178 represents 169 compounds wherein, in the general formula (I-1a), $R^1$ represents Et, $R^2$ does H, $R^3$ does Cl, $R^4$ does H, Q does an oxygen atom and the bonded position thereof being a 4-position, (X)n does 2,3-$Me_2$, and $(X^1)$n corresponds to each of all the substituents shown in Table 2-1.

Table 2-179

Table 2-179 represents 169 compounds wherein, in the general formula (I-1a), $R^1$ represents Et, $R^2$ does H, $R^3$ does Cl, $R^4$ does H, Q does an oxygen atom and the bonded position thereof being a 4-position, (X)n does 2,5-$Me_2$, and $(X^1)$n corresponds to each of all the substituents shown in Table 2-1.

Table 2-180

Table 2-180 represents 169 compounds wherein, in the general formula (I-1a), $R^1$ represents Et, $R^2$ does H, $R^3$ does Cl, $R^4$ does H, Q does an oxygen atom and the bonded position thereof being a 4-position, (X)n does 3,5-$(OMe)_2$, and $(X^1)$n corresponds to each of all the substituents shown in Table 2-1.

Table 2-181

Table 2-181 represents 169 compounds wherein, in the general formula (I-1a), $R^1$ represents Et, $R^2$ does H, $R^3$ does Cl, $R^4$ does H, Q does an oxygen atom and the bonded position thereof being a 4-position, (X)n does 2,3-$(OMe)_2$, and $(X^1)$n corresponds to each of all the substituents shown in Table 2-1.

Table 2-182

Table 2-182 represents 169 compounds wherein, in the general formula (I-1a), $R^1$ represents Et, $R^2$ does H, $R^3$ does Cl, $R^4$ does H, Q does an oxygen atom and the bonded position thereof being a 4-position, (X)n does 2,5-$(OMe)_2$, and $(X^1)$n corresponds to each of all the substituents shown in Table 2-1.

Table 2-183

Table 2-183 represents 169 compounds wherein, in the general formula (I-1a), $R^1$ represents Et, $R^2$ does H, $R^3$ does Cl, $R^4$ does H, Q does an oxygen atom and the bonded position thereof being a 4-position, (X)n does 2,6-$(OMe)_2$, and $(X^1)$n corresponds to each of all the substituents shown in Table 2-1.

Table 2-184

Table 2-184 represents 169 compounds wherein, in the general formula (I-1a), $R^1$ represents Et, $R^2$ does H, $R^3$ does Cl, $R^4$ does H, Q does an oxygen atom and the bonded position thereof being a 3-position, (X)n does 2-Cl, and $(X^1)$n corresponds to each of all the substituents shown in Table 2-1.

Table 2-185

Table 2-185 represents 169 compounds wherein, in the general formula (I-1a), $R^1$ represents Et, $R^2$ does H, $R^3$ does Cl, $R^4$ does H, Q does an oxygen atom and the bonded position thereof being a 3-position, (X)n does 4-Cl, and $(X^1)$n corresponds to each of all the substituents shown in Table 2-1.

Table 2-186

Table 2-186 represents 169 compounds wherein, in the general formula (I-1a), $R^1$ represents Et, $R^2$ does H, $R^3$ does Cl, $R^4$ does H, Q does an oxygen atom and the bonded position thereof being a 3-position, (X)n does 5-Cl, and $(X^1)$n corresponds to each of all the substituents shown in Table 2-1.

Table 2-187

Table 2-187 represents 169 compounds wherein, in the general formula (I-1a), $R^1$ represents Et, $R^2$ does H, $R^3$ does Cl, $R^4$ does H, Q does an oxygen atom and the bonded position thereof being a 3-position, (X)n does 6-Cl, and $(X^1)$n corresponds to each of all the substituents shown in Table 2-1.

Table 2-188

Table 2-188 represents 169 compounds wherein, in the general formula (I-1a), $R^1$ represents Et, $R^2$ does H, $R^3$ does Cl, $R^4$ does H, Q does an oxygen atom and the bonded position thereof being a 3-position, (X)n does 2-Me, and $(X^1)$n corresponds to each of all the substituents shown in Table 2-1.

Table 2-189

Table 2-189 represents 169 compounds wherein, in the general formula (I-1a), $R^1$ represents Et, $R^2$ does H, $R^3$ does Cl, $R^4$ does H, Q does an oxygen atom and the bonded position thereof being a 3-position, (X)n does 4-Me, and $(X^1)$n corresponds to each of all the substituents shown in Table 2-1.

Table 2-190

Table 2-190 represents 169 compounds wherein, in the general formula (I-1a), $R^1$ represents Et, $R^2$ does H, $R^3$ does Cl, $R^4$ does H, Q does an oxygen atom and the bonded position thereof being a 3-position, (X)n does 5-Me, and $(X^1)$n corresponds to each of all the substituents shown in Table 2-1.

Table 2-191

Table 2-191 represents 169 compounds wherein, in the general formula (I-1a), $R^1$ represents Et, $R^2$ does H, $R^3$ does Cl, $R^4$ does H, Q does an oxygen atom and the bonded position thereof being a 3-position, (X)n does 6-Me, and $(X^1)$n corresponds to each of all the substituents shown in Table 2-1.

Table 2-192

Table 2-192 represents 169 compounds wherein, in the general formula (I-1a), $R^1$ represents Et, $R^2$ does H, $R^3$ does Cl, $R^4$ does H, Q does an oxygen atom and the bonded position thereof being a 3-position, (X)n does 2-OMe, and $(X^1)$n corresponds to each of all the substituents shown in Table 2-1.

Table 2-193

Table 2-193 represents 169 compounds wherein, in the general formula (I-1a), $R^1$ represents Et, $R^2$ does H, $R^3$ does Cl, $R^4$ does H, Q does an oxygen atom and the bonded position thereof being a 3-position, (X)n does 4-OMe, and $(X^1)$n corresponds to each of all the substituents shown in Table 2-1.

Table 2-194

Table 2-194 represents 169 compounds wherein, in the general formula (I-1a), $R^1$ represents Et, $R^2$ does H, $R^3$ does Cl, $R^4$ does H, Q does an oxygen atom and the bonded position thereof being a 3-position, (X)n does 5-OMe, and $(X^1)$n corresponds to each of all the substituents shown in Table 2-1.

Table 2-195

Table 2-195 represents 169 compounds wherein, in the general formula (I-1a), $R^1$ represents Et, $R^2$ does H, $R^3$ does Cl, $R^4$ does H, Q does an oxygen atom and the bonded position thereof being a 3-position, (X)n does 6-OMe,

and $(X^1)n$ corresponds to each of all the substituents shown in Table 2-1.

Table 2-196

Table 2-196 represents 169 compounds wherein, in the general formula (I-1a), $R^1$ represents Et, $R^2$ does H, $R^3$ does Cl, $R^4$ does H, Q does an oxygen atom and the bonded position thereof being a 3-position, (X)n does 4-$OCF_3$, and $(X^1)n$ corresponds to each of all the substituents shown in Table 2-1.

Table 2-197

Table 2-197 represents 169 compounds wherein, in the general formula (I-1a), $R^1$ represents Et, $R^2$ does H, $R^3$ does Cl, $R^4$ does H, Q does an oxygen atom and the bonded position thereof being a 3-position, (X)n does 4-$OCHF_2$, and $(X^1)n$ corresponds to each of all the substituents shown in Table 2-1.

Table 2-198

Table 2-198 represents 169 compounds wherein, in the general formula (I-1a), $R^1$ represents Et, $R^2$ does H, $R^3$ does Cl, $R^4$ does H, Q does an oxygen atom and the bonded position thereof being a 3-position, (X)n does 4-$OCF_2CHF_2$, and $(X^1)n$ corresponds to each of all the substituents shown in Table 2-1.

Table 2-199

Table 2-199 represents 169 compounds wherein, in the general formula (I-1a), $R^1$ represents Et, $R^2$ does H, $R^3$ does Cl, $R^4$ does H, Q does an oxygen atom and the bonded position thereof being a 3-position, (X)n does 4-$OCH_2CF_3$, and $(X^1)n$ corresponds to each of all the substituents shown in Table 2-1.

Table 2-200

Table 2-200 represents 169 compounds wherein, in the general formula (I-1a), $R^1$ represents Et, $R^2$ does H, $R^3$ does Cl, $R^4$ does H, Q does an oxygen atom and the bonded position thereof being a 3-position, (X)n does 4-$CF_3$, and $(X^1)n$ corresponds to each of all the substituents shown in Table 2-1.

Table 2-201

Table 2-201 represents 169 compounds wherein, in the general formula (I-1a), $R^1$ represents Et, $R^2$ does H, $R^3$ does Cl, $R^4$ does H, Q does an oxygen atom and the bonded position thereof being a 2-position, (X)n does 3-Cl, and $(X^1)n$ corresponds to each of all the substituents shown in Table 2-1.

Table 2-202

Table 2-202 represents 169 compounds wherein, in the general formula (I-1a), $R^1$ represents Et, $R^2$ does H, $R^3$ does Cl, $R^4$ does H, Q does an oxygen atom and the bonded position thereof being a 2-position, (X)n does 4-Cl, and $(X^1)n$ corresponds to each of all the substituents shown in Table 2-1.

Table 2-203

Table 2-203 represents 169 compounds wherein, in the general formula (I-1a), $R^1$ represents Et, $R^2$ does H, $R^3$ does Cl, $R^4$ does H, Q does an oxygen atom and the bonded position thereof being a 2-position, (X)n does 5-Cl, and $(X^1)n$ corresponds to each of all the substituents shown in Table 2-1.

Table 2-204

Table 2-204 represents 169 compounds wherein, in the general formula (I-1a), $R^1$ represents Et, $R^2$ does H, $R^3$ does Cl, $R^4$ does H, Q does an oxygen atom and the bonded position thereof being a 2-position, (X)n does 6-Cl, and $(X^1)n$ corresponds to each of all the substituents shown in Table 2-1.

Table 2-205

Table 2-205 represents 169 compounds wherein, in the general formula (I-1a), $R^1$ represents Et, $R^2$ does H, $R^3$ does Cl, $R^4$ does H, Q does an oxygen atom and the bonded position thereof being a 2-position, (X)n does 3-Me, and $(X^1)$n corresponds to each of all the substituents shown in Table 2-1.

Table 2-206

Table 2-206 represents 169 compounds wherein, in the general formula (I-1a), $R^1$ represents Et, $R^2$ does H, $R^3$ does Cl, $R^4$ does H, Q does an oxygen atom and the bonded position thereof being a 2-position, (X)n does 4-Me, and $(X^1)$n corresponds to each of all the substituents shown in Table 2-1.

Table 2-207

Table 2-207 represents 169 compounds wherein, in the general formula (I-1a), $R^1$ represents Et, $R^2$ does H, $R^3$ does Cl, $R^4$ does H, Q does an oxygen atom and the bonded position thereof being a 2-position, (X)n does 5-Me, and $(X^1)$n corresponds to each of all the substituents shown in Table 2-1.

Table 2-208

Table 2-208 represents 169 compounds wherein, in the general formula (I-1a), $R^1$ represents Et, $R^2$ does H, $R^3$ does Cl, $R^4$ does H, Q does an oxygen atom and the bonded position thereof being a 2-position, (X)n does 6-Me, and $(X^1)$n corresponds to each of all the substituents shown in Table 2-1.

Table 2-209

Table 2-209 represents 169 compounds wherein, in the general formula (I-1a), $R^1$ represents Et, $R^2$ does H, $R^3$ does Cl, $R^4$ does H, Q does an oxygen atom and the bonded position thereof being a 2-position, (X)n does 3-OMe, and $(X^1)$n corresponds to each of all the substituents shown in Table 2-1.

Table 2-210

Table 2-210 represents 169 compounds wherein, in the general formula (I-1a), $R^1$ represents Et, $R^2$ does H, $R^3$ does Cl, $R^4$ does H, Q does an oxygen atom and the bonded position thereof being a 2-position, (X)n does 4-OMe, and $(X^1)$n corresponds to each of all the substituents shown in Table 2-1.

Table 2-211

Table 2-211 represents 169 compounds wherein, in the general formula (I-1a), $R^1$ represents Et, $R^2$ does H, $R^3$ does Cl, $R^4$ does H, Q does an oxygen atom and the bonded position thereof being a 2-position, (X)n does 5-OMe, and $(X^1)$n corresponds to each of all the substituents shown in Table 2-1.

Table 2-212

Table 2-212 represents 169 compounds wherein, in the general formula (I-1a), $R^1$ represents Et, $R^2$ does H, $R^3$ does Cl, $R^4$ does H, Q does an oxygen atom and the bonded position thereof being a 2-position, (X)n does 6-OMe, and $(X^1)$n corresponds to each of all the substituents shown in Table 2-1.

The general formula (I-2)

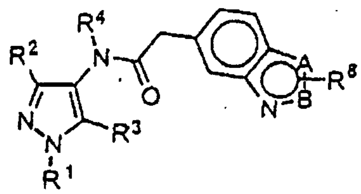

(I-2)

Table 3-1

| $R^1$ | $R^2$ | $R^3$ | $R^4$ | $R^8$ | A | B |
|---|---|---|---|---|---|---|
| Me | Me | Cl | H | H | 0 | $CR^8$ |
| Me | Me | Cl | H | Me | 0 | $CR^8$ |
| Me | Me | Cl | H | Et | 0 | $CR^8$ |
| Me | Me | Cl | H | n-Pr | 0 | $CR^8$ |
| Me | Me | Cl | H | i-Pr | 0 | $CR^8$ |
| Me | Me | Cl | H | t-Bu | 0 | $CR^8$ |
| Me | Me | Cl | H | s-Bu | 0 | $CR^8$ |
| Me | Me | Cl | H | $CH(Me)CH_2CH_3Me$ | 0 | $CR^8$ |
| Me | Me | Cl | H | $CHEt_2$ | 0 | $CR^8$ |
| Me | Me | Cl | H | $CH_2CHMe_2$ | 0 | $CR^8$ |
| Me | Me | Cl | H | $CH_2CMe_3$ | 0 | $CR^8$ |
| Me | Me | Cl | H | $CH_2CH_2CMe_3$ | 0 | $CR^8$ |
| Me | Me | Cl | H | $C(Me_2)CH_2CH_2Me$ | 0 | $CR^8$ |
| Me | Me | Cl | H | c-Pr | 0 | $CR^8$ |
| Me | Me | Cl | H | 1-Me-c-Pr | 0 | $CR^8$ |
| Me | Me | Cl | H | c-Bu | 0 | $CR^8$ |
| Me | Me | Cl | H | c-Pen | 0 | $CR^8$ |
| Me | Me | Cl | H | c-Hex | 0 | $CR^8$ |
| Me | Me | Cl | H | 1-Me-c-Hex | 0 | $CR^8$ |
| Me | Me | Cl | H | $CH_2$-c-Pr | 0 | $CR^8$ |
| Me | Me | Cl | H | $CH_2$-c-Pen | 0 | $CR^8$ |
| Me | Me | Cl | H | $CH=CH_2$ | 0 | $CR^8$ |
| Me | Me | Cl | H | CH=CHMe | 0 | $CR^8$ |
| Me | Me | Cl | H | $CH=CMe_2$ | 0 | $CR^8$ |
| Me | Me | Cl | H | C(Me)=CHMe | 0 | $CR^8$ |
| Me | Me | Cl | H | 4-$NO_2$-styryl | 0 | $CR^8$ |
| Me | Me | Cl | H | $C\equiv CH$ | 0 | $CR^8$ |
| Me | Me | Cl | H | $C\equiv CMe$ | 0 | $CR^8$ |
| Me | Me | Cl | H | $C\equiv CCH_2Me$ | 0 | $CR^8$ |
| Me | Me | Cl | H | $CH_2C\equiv CH$ | 0 | $CR^8$ |
| Me | Me | Cl | H | $CMe_2C\equiv CH$ | 0 | $CR^8$ |
| He | Me | Cl | H | $CH_2F$ | 0 | $CR^8$ |
| Me | Me | Cl | H | $CHF_2$ | 0 | $CR^8$ |
| Me | Me | Cl | H | $CF_3$ | 0 | $CR^8$ |
| Me | Me | Cl | H | $CFMe_2$ | 0 | $CR^8$ |
| Me | Me | Cl | H | $CH_2CF_3$ | 0 | $CR^8$ |
| Me | Me | Cl | H | $CF_2CF_3$ | 0 | $CR^8$ |

Table 3-1 (continued)

| $R^1$ | $R^2$ | $R^3$ | $R^4$ | $R^8$ | A | B |
|---|---|---|---|---|---|---|
| Me | Me | Cl | H | $CF_2CHF_2$ | 0 | $CR^8$ |
| Me | Me | Cl | H | $CF_2CF_2CF_3$ | 0 | $CR^8$ |
| Me | Me | Cl | H | $CF_2CF_2CF_2CF_3$ | 0 | $CR^8$ |
| Me | Me | Cl | H | $CF_2Cl$ | 0 | $CR^8$ |
| Me | Me | Cl | H | $CF_2Br$ | 0 | $CR^8$ |
| Me | Me | Cl | H | $CF_2CF_2Cl$ | 0 | $CR^8$ |
| Me | Me | Cl | H | $CH_2Cl$ | 0 | $CR^8$ |
| Me | Me | Cl | H | $CH_2CH_2Cl$ | 0 | $CR^8$ |
| Me | Me | Cl | H | CH(Cl)Me | 0 | $CR^8$ |
| Me | Me | Cl | H | CH(Cl)Ph | 0 | $CR^8$ |
| Me | Me | Cl | H | $CH(Cl)CH_2Me$ | 0 | $CR^8$ |
| Me | Me | Cl | H | $CMe_2CH_2Cl$ | 0 | $CR^8$ |
| Me | Me | Cl | H | $CCl_3$ | 0 | $CR^8$ |
| Me | Me | Cl | H | $CH_2Br$ | 0 | $CR^8$ |
| Me | Me | Cl | H | $CHBr_2$ | 0 | $CR^8$ |
| Me | Me | Cl | H | $CH_2CN$ | 0 | $CR^8$ |
| Me | Me | Cl | H | CH(Me)CN | 0 | $CR^8$ |
| Me | Me | Cl | H | $C(Me_2)CN$ | 0 | $CR^8$ |
| Me | Me | Cl | H | $CH_2CO_2Me$ | 0 | $CR^8$ |
| Me | Me | Cl | H | $CH_2CO_2Et$ | 0 | $CR^8$ |
| Me | Me | Cl | H | $CH(Me)CO_2Me$ | 0 | $CR^8$ |
| Me | Me | Cl | H | $C(Me_2)CO_2Me$ | 0 | $CR^8$ |
| Me | Me | Cl | H | $CH_2CH_2OMe$ | 0 | $CR^8$ |
| Me | Me | Cl | H | $CH_2CH_2OH$ | 0 | $CR^8$ |
| Me | Me | Cl | H | $CH_2OPh$ | 0 | $CR^8$ |
| Me | Me | Cl | H | CH(Me)OPh | 0 | $CR^8$ |
| Me | Me | Cl | H | $CH_2O(4\text{-}NO_2\text{-}Ph)$ | 0 | $CR^8$ |
| Me | Me | Cl | H | tetrahydrofuran-2-yl | 0 | $CR^8$ |
| Me | Me | Cl | H | tetrahydrofuran-3-yl | 0 | $CR^8$ |
| Me | Me | Cl | H | CHO | 0 | $CR^8$ |
| Me | Me | Cl | H | COMe | 0 | $CR^8$ |
| Me | Me | Cl | H | COEt | 0 | $CR^8$ |
| Me | Me | Cl | H | $CO_2Me$ | 0 | $CR^8$ |
| Me | Me | Cl | H | $CO_2$-i-Pr | 0 | $CR^8$ |
| Me | Me | Cl | H | $CH_2CH_2CO_2Me$ | 0 | $CR^8$ |
| Me | Me | Cl | H | CH=NOH | 0 | $CR^8$ |
| Me | Me | Cl | H | CH=NOMe | 0 | $CR^8$ |
| Me | Me | Cl | H | C(Me)=NOH | 0 | $CR^8$ |
| Me | Me | Cl | H | C(Me)=NOMe | 0 | $CR^8$ |
| Me | Me | Cl | H | $C(Me)=NOCH_2Ph$ | 0 | $CR^8$ |
| Me | Me | Cl | H | $CH_2Me$ | 0 | $CR^8$ |
| Me | Me | Cl | H | $CH_2CH_2SMe$ | 0 | $CR^8$ |
| Me | Me | Cl | H | $CH_2SPh$ | 0 | $CR^8$ |
| Me | Me | Cl | H | $CH_2NH_2$ | 0 | $CR^8$ |
| Me | Me | Cl | H | NHMe | 0 | $CR^8$ |
| Me | Me | Cl | H | NHEt | 0 | $CR^8$ |
| Me | Me | Cl | H | $NMe_2$ | 0 | $CR^8$ |
| Me | Me | Cl | H | morpholino | 0 | $CR^8$ |
| Me | Me | Cl | H | NHCHO | 0 | $CR^8$ |
| Me | Me | Cl | H | NHAc | 0 | $CR^8$ |

Table 3-1 (continued)

| $R^1$ | $R^2$ | $R^3$ | $R^4$ | $R^8$ | A | B |
|---|---|---|---|---|---|---|
| Me | Me | Cl | H | NHCOPh | 0 | $CR^8$ |
| Me | Me | Cl | H | N(Me)CHO | 0 | $CR^8$ |
| Me | Me | Cl | H | N(Me)Ac | 0 | $CR^8$ |
| Me | Me | Cl | H | $NHSO_2Me$ | 0 | $CR^8$ |
| Me | Me | Cl | H | $NHSO_2Ph$ | 0 | $CR^8$ |
| Me | Me | Cl | H | OMe | 0 | $CR^8$ |
| Me | Me | Cl | H | OEt | 0 | $CR^8$ |
| Me | Me | Cl | H | O-n-Pr | 0 | $CR^8$ |
| Me | Me | Cl | H | O-i-Pr | 0 | $CR^8$ |
| Me | Me | Cl | H | O-t-Bu | 0 | $CR^8$ |
| Me | Me | Cl | H | $OCH_2CF_3$ | 0 | $CR^8$ |
| Me | Me | Cl | H | $OCHF_2$ | 0 | $CR^8$ |
| Me | Me | Cl | H | $OCF_2CHF_2$ | 0 | $CR^8$ |
| Me | Me | Cl | H | $OCH(CF_3)_2$ | 0 | $CR^8$ |
| Me | Me | Cl | H | $OCH_2Ph$ | 0 | $CR^8$ |
| Me | Me | Cl | H | $OCH_2$ (4-F-Ph) | 0 | $CR^8$ |
| Me | Me | Cl | H | $OCH_2$ (2,4-$Cl_2$-Ph) | 0 | $CR^8$ |
| Me | Me | Cl | H | $OCH_2CH=CH_2$ | 0 | $CR^8$ |
| Me | Me | Cl | H | OPh | 0 | $CR^8$ |
| Me | Me | Cl | H | O(4-Cl-Ph) | 0 | $CR^8$ |
| Me | Me | Cl | H | $ON=CMe_2$ | 0 | $CR^8$ |
| Me | Me | Cl | H | SMe | 0 | $CR^8$ |
| Me | Me | Cl | H | SEt | 0 | $CR^8$ |
| Me | Me | Cl | H | S-n-Pr | 0 | $CR^8$ |
| Me | Me | Cl | H | S-i-Pr | 0 | $CR^8$ |
| Me | Me | Cl | H | S-t-Bu | 0 | $CR^8$ |
| Me | Me | Cl | H | $SCF_3$ | 0 | $CR^8$ |
| Me | Me | Cl | H | $SCH_2CF_3$ | 0 | $CR^8$ |
| Me | Me | Cl | H | $SCHF_2$ | 0 | $CR^8$ |
| Me | Me | Cl | H | $SCF_2CHF_2$ | 0 | $CR^8$ |
| Me | Me | Cl | H | $SCH(CF_3)_2$ | 0 | $CR^8$ |
| Me | Me | Cl | H | $SCH_2Ph$ | 0 | $CR^8$ |
| Me | Me | Cl | H | SPh | 0 | $CR^8$ |
| Me | Me | Cl | H | $SCH_2CN$ | 0 | $CR^8$ |
| Me | Me | Cl | H | $SCH_2CO_2Me$ | 0 | $CR^8$ |
| Me | Me | Cl | H | $CH_2Ph$ | 0 | $CR^8$ |
| Me | Me | Cl | H | CH(Me)Ph | 0 | $CR^8$ |
| Me | Me | Cl | H | $C(Me)_2Ph$ | 0 | $CR^8$ |
| Me | Me | Cl | H | $CH_2C_6F_5$ | 0 | $CR^8$ |
| Me | Me | Cl | H | $CHPh_2$ | 0 | $CR^8$ |
| Me | Me | Cl | H | $CH_2CH_2Ph$ | 0 | $CR^8$ |
| Me | Me | Cl | H | Ph | 0 | $CR^8$ |
| Me | Me | Cl | H | 2-F-Ph | 0 | $CR^8$ |
| Me | Me | Cl | H | 3-F-Ph | 0 | $CR^8$ |
| Me | Me | Cl | H | 4-F-Ph | 0 | $CR^8$ |
| Me | Me | Cl | H | 2-Cl-Ph | 0 | $CR^8$ |
| Me | Me | Cl | H | 3-Cl-Ph | 0 | $CR^8$ |
| Me | Me | Cl | H | 4-Cl-Ph | 0 | $CR^8$ |
| Me | Me | Cl | H | 2-Br-Ph | 0 | $CR^8$ |
| Me | Me | Cl | H | 3-Br-Ph | 0 | $CR^8$ |

Table 3-1 (continued)

| $R^1$ | $R^2$ | $R^3$ | $R^4$ | $R^8$ | A | B |
|---|---|---|---|---|---|---|
| Me | Me | Cl | H | 4-Br-Ph | 0 | $CR^8$ |
| Me | Me | Cl | H | 2-Me-Ph | 0 | $CR^8$ |
| Me | Me | Cl | H | 3-Me-Ph | 0 | $CR^8$ |
| Me | Me | Cl | H | 4-Me-Ph | 0 | $CR^8$ |
| Me | Me | Cl | H | 2-OMe-Ph | 0 | $CR^8$ |
| Me | Me | Cl | H | 3-OMe-Ph | 0 | $CR^8$ |
| Me | Me | Cl | H | 4-OMe-Ph | 0 | $CR^8$ |
| Me | Me | Cl | H | 2-$NO_2$-Ph | 0 | $CR^8$ |
| Me | Me | Cl | H | 3-$NO_2$-Ph | 0 | $CR^8$ |
| Me | Me | Cl | H | 4-$NO_2$-Ph | 0 | $CR^8$ |
| Me | Me | Cl | H | 2-CN-Ph | 0 | $CR^8$ |
| Me | Me | Cl | H | 3-CN-Ph | 0 | $CR^8$ |
| Me | Me | Cl | H | 4-CN-Ph | 0 | $CR^8$ |
| Me | Me | Cl | H | 2-$CF_3$-Ph | 0 | $CR^8$ |
| Me | Me | Cl | H | 3-$CF_3$-Ph | 0 | $CR^8$ |
| Me | Me | Cl | H | 4-$CF_3$-Ph | 0 | $CR^8$ |
| Me | Me | Cl | H | 2-$OCF_3$-Ph | 0 | $CR^8$ |
| Me | Me | Cl | H | 3-$OCF_3$-Ph | 0 | $CR^8$ |
| Me | Me | Cl | H | 4-$OCF_3$-Ph | 0 | $CR^8$ |
| Me | Me | Cl | H | 4-t-Bu-Ph | 0 | $CR^8$ |
| Me | Me | Cl | H | 4-$CO_2$Me-Ph | 0 | $CR^8$ |
| Me | Me | Cl | H | 2,3-$Cl_2$-Ph | 0 | $CR^8$ |
| Me | Me | Cl | H | 2,4-$Cl_2$-Ph | 0 | $CR^8$ |
| Me | Me | Cl | H | 2,5-$Cl_2$-Ph | 0 | $CR^8$ |
| Me | Me | Cl | H | 2,6-$Cl_2$-Ph | 0 | $CR^8$ |
| Me | Me | Cl | H | 3,4-$Cl_2$-Ph | 0 | $CR^8$ |
| Me | Me | Cl | H | 3,5-$Cl_2$-Ph | 0 | $CR^8$ |
| Me | Me | Cl | H | 2,4,6-$Cl_3$-Ph | 0 | $CR^8$ |
| Me | Me | Cl | H | 2-Cl-4-$NO_2$-Ph | 0 | $CR^8$ |
| Me | Me | Cl | H | 2-Cl-4-$CF_3$-Ph | 0 | $CR^8$ |
| Me | Me | Cl | H | 2-Cl-4-$SO_2$Me-Ph | 0 | $CR^8$ |
| Me | Me | Cl | H | 2-Cl-6-$CF_3$-Ph | 0 | $CR^8$ |
| Me | Me | Cl | H | 2,6-$Cl_2$-4-$CF_3$-Ph | 0 | $CR^8$ |
| Me | Me | Cl | H | 2-$NO_2$-4-$SO_2$Me-Ph | 0 | $CR^8$ |
| Me | Me | Cl | H | thiophene-2-yl | 0 | $CR^8$ |
| Me | Me | Cl | H | 3-Cl-thiophene-2-yl | 0 | $CR^8$ |
| Me | Me | Cl | H | $CH_2$(thiophene-2-yl) | 0 | $CR^8$ |
| Me | Me | Cl | H | 5-$NO_2$-furan-2-yl | 0 | $CR^8$ |
| Me | Me | Cl | H | pyridine-2-yl | 0 | $CR^8$ |
| Me | Me | Cl | H | pyridine-3-yl | 0 | $CR^8$ |
| Me | Me | Cl | H | pyridine-4-yl | 0 | $CR^8$ |
| Me | Me | Cl | H | 3-Cl-pyridine-2-yl | 0 | $CR^8$ |
| Me | Me | Cl | H | 6-Cl-pyridine-2-yl | 0 | $CR^8$ |
| Me | Me | Cl | H | 2-Cl-pyridine-3-yl | 0 | $CR^8$ |
| Me | Me | Cl | H | 2-O-Ph-pyridine-3-yl | 0 | $CR^8$ |
| Me | Me | Cl | H | biphenyl-4-yl | 0 | $CR^8$ |
| Me | Me | Cl | H | 1,3-$Me_2$-pyrazol-5-yl | 0 | $CR^8$ |
| Me | Me | Cl | H | isoxazole-5-yl | 0 | $CR^8$ |
| Me | Me | Cl | H | OH | 0 | $CR^8$ |
| Me | Me | Cl | H | SH | 0 | $CR^8$ |

Table 3-2

Table 3-2 represents 187 compounds wherein, in the general formula (I-2), $R^1$ represents Me, $R^2$ does Et, $R^3$ does Cl, $R^4$ does H, A does an oxygen atom, B does $CR^8$, and $R^8$ corresponds to each of all the substituents shown in Table 3-1.

Table 3-3

Table 3-3 represents 187 compounds wherein, in the general formula (I-2), $R^1$ represents Me, $R^2$ does i-Pr, $R^3$ does Cl, $R^4$ does H, A does an oxygen atom, B does $CR^8$, and $R^8$ corresponds to each of all the substituents shown in Table 3-1.

Table 3-4

Table 3-4 represents 187 compounds wherein, in the general formula (I-2), $R^1$ represents Me, $R^2$ does n-Pr, $R^3$ does Cl, $R^4$ does H, A does an oxygen atom, B does $CR^8$, and $R^8$ corresponds to each of all the substituents shown in Table 3-1.

Table 3-5

Table 3-5 represents 187 compounds wherein, in the general formula (I-2), $R^1$ represents Me, $R^2$ does t-Bu, $R^3$ does Cl, $R^4$ does H, A does an oxygen atom, B does $CR^8$, and $R^8$ corresponds to each of all the substituents shown in Table 3-1.

Table 3-6

Table 3-6 represents 187 compounds wherein, in the general formula (I-2), $R^1$ represents Me, $R^2$ does n-Bu, $R^3$ does Cl, $R^4$ does H, A does an oxygen atom, B does $CR^8$, and $R^8$ corresponds to each of all the substituents shown in Table 3-1.

Table 3-7

Table 3-7 represents 187 compounds wherein, in the general formula (I-2), $R^1$ represents Me, $R^2$ does $CF_3$, $R^3$ does Cl, $R^4$ does H, A does an oxygen atom, B does $CR^8$, and $R^8$ corresponds to each of all the substituents shown in Table 3-1.

Table 3-8

Table 3-8 represents 187 compounds wherein, in the general formula (I-2), $R^1$ represents Me, $R^2$ does OMe, $R^3$ does Cl, $R^4$ does H, A does an oxygen atom, B does $CR^8$, and $R^8$ corresponds to each of all the substituents shown in Table 3-1.

Table 3-9

Table 3-9 represents 187 compounds wherein, in the general formula (I-2), $R^1$ represents Me, $R^2$ does $OCHF_2$, $R^3$ does Cl, $R^4$ does H, A does an oxygen atom, B does $CR^8$, and $R^8$ corresponds to each of all the substituents shown in Table 3-1.

Table 3-10

Table 3-10 represents 187 compounds wherein, in the general formula (I-2), $R^1$ represents Me, $R^2$ does O-i-Pr, $R^3$ does Cl, $R^4$ does H, A does an oxygen atom, B does $CR^8$, and $R^8$ corresponds to each of all the substituents shown in Table 3-1.

Table 3-11

Table 3-11 represents 187 compounds wherein, in the general formula (I-2), $R^1$ represents Me, $R^2$ does Cl, $R^3$ does Cl, $R^4$ does H, A does an oxygen atom, B does $CR^8$, and $R^8$ corresponds to each of all the substituents shown

in Table 3-1.

Table 3-12

Table 3-12 represents 187 compounds wherein, in the general formula (I-2), $R^1$ represents Me, $R^2$ does Ph, $R^3$ does Cl, $R^4$ does H, A does an oxygen atom, B does $CR^8$, and $R^8$ corresponds to each of all the substituents shown in Table 3-1.

Table 3-13

Table 3-13 represents 187 compounds wherein, in the general formula (I-2), $R^1$ represents Me, $R^2$ does 2-Cl-Ph, $R^3$ does Cl, $R^4$ does H, A does an oxygen atom, B does $CR^8$, and $R^8$ corresponds to each of all the substituents shown in Table 3-1.

Table 3-14

Table 3-14 represents 187 compounds wherein, in the general formula (I-2), $R^1$ represents Me, $R^2$ does 3-Cl-Ph, $R^3$ does Cl, $R^4$ does H, A does an oxygen atom, B does $CR^8$, and $R^8$ corresponds to each of all the substituents shown in Table 3-1.

Table 3-15

Table 3-15 represents 187 compounds wherein, in the general formula (I-2), $R^1$ represents Me, $R^2$ does 4-Cl-Ph, $R^3$ does Cl, $R^4$ does H, A does an oxygen atom, B does $CR^8$, and $R^8$ corresponds to each of all the substituents shown in Table 3-1.

Table 3-16

Table 3-16 represents 187 compounds wherein, in the general formula (I-2), $R^1$ represents Me, $R^2$ does 2,4-$Cl_2$-Ph, $R^3$ does Cl, $R^4$ does H, A does an oxygen atom, B does $CR^8$, and $R^8$ corresponds to each of all the substituents shown in Table 3-1.

Table 3-17

Table 3-17 represents 187 compounds wherein, in the general formula (I-2), $R^1$ represents Me, $R^2$ does 2-Me-Ph, $R^3$ does Cl, $R^4$ does H, A does an oxygen atom, B does $CR^8$, and $R^8$ corresponds to each of all the substituents shown in Table 3-1.

Table 3-18

Table 3-18 represents 187 compounds wherein, in the general formula (I-2), $R^1$ represents Me, $R^2$ does 3-Me-Ph, $R^3$ does Cl, $R^4$ does H, A does an oxygen atom, B does $CR^8$, and $R^8$ corresponds to each of all the substituents shown in Table 3-1.

Table 3-19

Table 3-19 represents 187 compounds wherein, in the general formula (I-2), $R^1$ represents Me, $R^2$ does 4-Me-Ph, $R^3$ does Cl, $R^4$ does H, A does an oxygen atom, B does $CR^8$, and $R^8$ corresponds to each of all the substituents shown in Table 3-1.

Table 3-20

Table 3-20 represents 187 compounds wherein, in the general formula (I-2), $R^1$ represents Me, $R^2$ does 2-OMe-Ph, $R^3$ does Cl, $R^4$ does H, A does an oxygen atom, B does $CR^8$, and $R^8$ corresponds to each of all the substituents shown in Table 3-1.

Table 3-21

Table 3-21 represents 187 compounds wherein, in the general formula (I-2), $R^1$ represents Me, $R^2$ does 3-OMe-Ph, $R^3$ does Cl, $R^4$ does H, A does an oxygen atom, B does $CR^8$, and $R^8$ corresponds to each of all the substituents shown in Table 3-1.

Table 3-22

Table 3-22 represents 187 compounds wherein, in the general formula (I-2), $R^1$ represents Me, $R^2$ does 4-OMe-Ph, $R^3$ does Cl, $R^4$ does H, A does an oxygen atom, B does $CR^8$, and $R^8$ corresponds to each of all the substituents shown in Table 3-1.

Table 3-23

Table 3-23 represents 187 compounds wherein, in the general formula (I-2), $R^1$ represents Me, $R^2$ does H, $R^3$ does Cl, $R^4$ does H, A does an oxygen atom, B does $CR^8$, and $R^8$ corresponds to each of all the substituents shown in Table 3-1.

Table 3-24

Table 3-24 represents 187 compounds wherein, in the general formula (I-2), $R^1$ represents H, $R^2$ does H, $R^3$ does Cl, $R^4$ does H, A does an oxygen atom, B does $CR^8$, and $R^8$ corresponds to each of all the substituents shown in Table 3-1.

Table 3-25

Table 3-25 represents 187 compounds wherein, in the general formula (I-2), $R^1$ represents Et, $R^2$ does H, $R^3$ does Cl, $R^4$ does H, A does an oxygen atom, B does $CR^8$, and $R^8$ corresponds to each of all the substituents shown in Table 3-1.

Table 3-26

Table 3-26 represents 187 compounds wherein, in the general formula (I-2), $R^1$ represents i-Pr, $R^2$ does H, $R^3$ does Cl, $R^4$ does H, A does an oxygen atom, B does $CR^8$, and $R^8$ corresponds to each of all the substituents shown in Table 3-1.

Table 3-27

Table 3-27 represents 187 compounds wherein, in the general formula (I-2), $R^1$ represents n-Pr, $R^2$ does H, $R^3$ does Cl, $R^4$ does H, A does an oxygen atom, B does $CR^8$, and $R^8$ corresponds to each of all the substituents shown in Table 3-1.

Table 3-28

Table 3-28 represents 187 compounds wherein, in the general formula (I-2), $R^1$ represents t-Bu, $R^2$ does H, $R^3$ does Cl, $R^4$ does H, A does an oxygen atom, B does $CR^8$, and $R^8$ corresponds to each of all the substituents shown in Table 3-1.

Table 3-29

Table 3-29 represents 187 compounds wherein, in the general formula (I-2), $R^1$ represents n-Bu, $R^2$ does H, $R^3$ does Cl, $R^4$ does H, A does an oxygen atom, B does $CR^8$, and $R^8$ corresponds to each of all the substituents shown in Table 3-1.

Table 3-30

Table 3-30 represents 187 compounds wherein, in the general formula (I-2), $R^1$ represents $CH=CH_2$, $R^2$ does H, $R^3$ does Cl, $R^4$ does H, A does an oxygen atom, B does $CR^8$, and $R^8$ corresponds to each of all the substituents

shown in Table 3-1.

Table 3-31

Table 3-31 represents 187 compounds wherein, in the general formula (I-2), $R^1$ represents $CH_2CH=CH_2$, $R^2$ does H, $R^3$ does Cl, $R^4$ does H, A does an oxygen atom, B does $CR^8$, and $R^8$ corresponds to each of all the substituents shown in Table 3-1.

Table 3-32

Table 3-32 represents 187 compounds wherein, in the general formula (I-2), $R^1$ represents $CH_2C\equiv CH$, $R^2$ does H, $R^3$ does Cl, $R^4$ does H, A does an oxygen atom, B does $CR^8$, and $R^8$ corresponds to each of all the substituents shown in Table 3-1.

Table 3-33

Table 3-33 represents 187 compounds wherein, in the general formula (I-2), $R^1$ represents $CH_2CN$, $R^2$ does H, $R^3$ does Cl, $R^4$ does H, A does an oxygen atom, B does $CR^8$, and $R^8$ corresponds to each of all the substituents shown in Table 3-1.

Table 3-34

Table 3-34 represents 187 compounds wherein, in the general formula (I-2), $R^1$ represents $CH_2CF_3$, $R^2$ does H, $R^3$ does Cl, $R^4$ does H, A does an oxygen atom, B does $CR^8$, and $R^8$ corresponds to each of all the substituents shown in Table 3-1.

Table 3-35

Table 3-35 represents 187 compounds wherein, in the general formula (I-2), $R^1$ represents $CH_2Ph$, $R^2$ does H, $R^3$ does Cl, $R^4$ does H, A does an oxygen atom, B does $CR^8$, and $R^8$ corresponds to each of all the substituents shown in Table 3-1.

Table 3-36

Table 3-36 represents 187 compounds wherein, in the general formula (I-2), $R^1$ represents Ph, $R^2$ does H, $R^3$ does Cl, $R^4$ does H, A does an oxygen atom, B does $CR^8$, and $R^8$ corresponds to each of all the substituents shown in Table 3-1.

Table 3-37

Table 3-37 represents 187 compounds wherein, in the general formula (I-2), $R^1$ represents 2-Cl-Ph, $R^2$ does H, $R^3$ does Cl, $R^4$ does H, A does an oxygen atom, B does $CR^8$, and $R^8$ corresponds to each of all the substituents shown in Table 3-1.

Table 3-38

Table 3-38 represents 187 compounds wherein, in the general formula (I-2), $R^1$ represents 3-Cl-Ph, $R^2$ does H, $R^3$ does Cl, $R^4$ does H, A does an oxygen atom, B does $CR^8$, and $R^8$ corresponds to each of all the substituents shown in Table 3-1.

Table 3-39

Table 3-39 represents 187 compounds wherein, in the general formula (I-2), $R^1$ represents 4-Cl-Ph, $R^2$ does H, $R^3$ does Cl, $R^4$ does H, A does an oxygen atom, B does $CR^8$, and $R^8$ corresponds to each of all the substituents shown in Table 3-1.

Table 3-40

Table 3-40 represents 187 compounds wherein, in the general formula (I-2), $R^1$ represents 2,4-$Cl_2$-Ph, $R^2$ does H, $R^3$ does Cl, $R^4$ does H, A does an oxygen atom, B does $CR^8$, and $R^8$ corresponds to each of all the substituents shown in Table 3-1.

Table 3-41

Table 3-41 represents 187 compounds wherein, in the general formula (I-2), $R^1$ represents Ac, $R^2$ does H, $R^3$ does Cl, $R^4$ does H, A does an oxygen atom, B does $CR^8$, and $R^8$ corresponds to each of all the substituents shown in Table 3-1.

Table 3-42

Table 3-42 represents 187 compounds wherein, in the general formula (I-2), $R^1$ represents COPh, $R^2$ does H, $R^3$ does Cl, $R^4$ does H, A does an oxygen atom, B does $CR^8$, and $R^8$ corresponds to each of all the substituents shown in Table 3-1.

Table 3-43

Table 3-43 represents 187 compounds wherein, in the general formula (I-2), $R^1$ represents $CH_2CH_2F$, $R^2$ does H, $R^3$ does Cl, $R^4$ does H, A does an oxygen atom, B does $CR^8$, and $R^8$ corresponds to each of all the substituents shown in Table 3-1.

Table 3-44

Table 3-44 represents 187 compounds wherein, in the general formula (I-2), $R^1$ represents $CH_2CH_2Cl$, $R^2$ does H, $R^3$ does Cl, $R^4$ does H, A does an oxygen atom, B does $CR^8$, and $R^8$ corresponds to each of all the substituents shown in Table 3-1.

Table 3-45

Table 3-45 represents 187 compounds wherein, in the general formula (I-2), $R^1$ represents $CH_2CH_2Br$, $R^2$ does H, $R^3$ does Cl, $R^4$ does H, A does an oxygen atom, B does $CR^8$, and $R^8$ corresponds to each of all the substituents shown in Table 3-1.

Table 3-46

Table 3-46 represents 187 compounds wherein, in the general formula (I-2), $R^1$ represents $CH_2CH_2OH$, $R^2$ does H, $R^3$ does Cl, $R^4$ does H, A does an oxygen atom, B does $CR^8$, and $R^8$ corresponds to each of all the substituents shown in Table 3-1.

Table 3-47

Table 3-47 represents 187 compounds wherein, in the general formula (I-2), $R^1$ represents $CH_2CH_2OMe$, $R^2$ does H, $R^3$ does Cl, $R^4$ does H, A does an oxygen atom, B does $CR^8$, and $R^8$ corresponds to each of all the substituents shown in Table 3-1.

Table 3-48

Table 3-48 represents 187 compounds wherein, in the general formula (I-2), $R^1$ represents $CH_2OMe$, $R^2$ does H, $R^3$ does Cl, $R^4$ does H, A does an oxygen atom, B does $CR^8$, and $R^8$ corresponds to each of all the substituents shown in Table 3-1.

Table 3-49

Table 3-49 represents 187 compounds wherein, in the general formula (I-2), $R^1$ represents $CH_2OEt$, $R^2$ does H, $R^3$ does Cl, $R^4$ does H, A does an oxygen atom, B does $CR^8$, and $R^8$ corresponds to each of all the substituents

shown in Table 3-1.

Table 3-50

Table 3-50 represents 187 compounds wherein, in the general formula (I-2), $R^1$ represents $CH_2COOH$, $R^2$ does H, $R^3$ does Cl, $R^4$ does H, A does an oxygen atom, B does $CR^8$, and $R^8$ corresponds to each of all the substituents shown in Table 3-1.

Table 3-51

Table 3-51 represents 187 compounds wherein, in the general formula (I-2), $R^1$ represents $CH_2COOEt$, $R^2$ does H, $R^3$ does Cl, $R^4$ does H, A does an oxygen atom, B does $CR^8$, and $R^8$ corresponds to each of all the substituents shown in Table 3-1.

Table 3-52

Table 3-52 represents 187 compounds wherein, in the general formula (I-2), $R^1$ represents $CH_2CONHMe$, $R^2$ does H, $R^3$ does Cl, $R^4$ does H, A does an oxygen atom, B does $CR^8$, and $R^8$ corresponds to each of all the substituents shown in Table 3-1.

Table 3-53

Table 3-53 represents 187 compounds wherein, in the general formula (I-2), $R^1$ represents $CH_2CONMe_2$, $R^2$ does H, $R^3$ does Cl, $R^4$ does H, A does an oxygen atom, B does $CR^8$, and $R^8$ corresponds to each of all the substituents shown in Table 3-1.

Table 3-54

Table 3-54 represents 187 compounds wherein, in the general formula (I-2), $R^1$ represents $CH_2CHO$, $R^2$ does H, $R^3$ does Cl, $R^4$ does H, A does an oxygen atom, B does $CR^8$, and $R^8$ corresponds to each of all the substituents shown in Table 3-1.

Table 3-55

Table 3-55 represents 187 compounds wherein, in the general formula (I-2), $R^1$ represents $CH_2CH=NOMe$, $R^2$ does H, $R^3$ does Cl, $R^4$ does H, A does an oxygen atom, B does $CR^8$, and $R^8$ corresponds to each of all the substituents shown in Table 3-1.

Table 3-56

Table 3-56 represents 187 compounds wherein, in the general formula (I-2), $R^1$ represents Et, $R^2$ does H, $R^3$ does H, $R^4$ does H, A does an oxygen atom, B does $CR^8$, and $R^8$ corresponds to each of all the substituents shown in Table 3-1.

Table 3-57

Table 3-57 represents 187 compounds wherein, in the general formula (I-2), $R^1$ represents Et, $R^2$ does H, $R^3$ does F, $R^4$ does H, A does an oxygen atom, B does $CR^8$, and $R^8$ corresponds to each of all the substituents shown in Table 3-1.

Table 3-58

Table 3-58 represents 187 compounds wherein, in the general formula (I-2), $R^1$ represents Et, $R^2$ does H, $R^3$ does Br, $R^4$ does H, A does an oxygen atom, B does $CR^8$, and $R^8$ corresponds to each of all the substituents shown in Table 3-1.

Table 3-59

Table 3-59 represents 187 compounds wherein, in the general formula (I-2), $R^1$ represents Et, $R^2$ does H, $R^3$ does I, $R^4$ does H, A does an oxygen atom, B does $CR^8$, and $R^8$ corresponds to each of all the substituents shown in Table 3-1.

Table 3-60

Table 3-60 represents 187 compounds wherein, in the general formula (I-2), $R^1$ represents Et, $R^2$ does H, $R^3$ does $CF_3$, $R^4$ does H, A does an oxygen atom, B does $CR^8$, and $R^8$ corresponds to each of all the substituents shown in Table 3-1.

Table 3-61

Table 3-61 represents 187 compounds wherein, in the general formula (I-2), $R^1$ represents Et, $R^2$ does H, $R^3$ does CN, $R^4$ does H, A does an oxygen atom, B does $CR^8$, and $R^8$ corresponds to each of all the substituents shown in Table 3-1.

Table 3-62

Table 3-62 represents 187 compounds wherein, in the general formula (I-2), $R^1$ represents Et, $R^2$ does H, $R^3$ does COOMe, $R^4$ does H, A does an oxygen atom, B does $CR^8$, and $R^8$ corresponds to each of all the substituents shown in Table 3-1.

Table 3-63

Table 3-63 represents 187 compounds wherein, in the general formula (I-2), $R^1$ represents Et, $R^2$ does H, $R^3$ does CONHMe, $R^4$ does H, A does an oxygen atom, B does $CR^8$, and $R^8$ corresponds to each of all the substituents shown in Table 3-1.

Table 3-64

Table 3-64 represents 187 compounds wherein, in the general formula (I-2), $R^1$ represents Et, $R^2$ does H, $R^3$ does $CONMe_2$, $R^4$ does H, A does an oxygen atom, B does $CR^8$, and $R^8$ corresponds to each of all the substituents shown in Table 3-1.

Table 3-65

Table 3-65 represents 187 compounds wherein, in the general formula (I-2), $R^1$ represents Et, $R^2$ does H, $R^3$ does SMe, $R^4$ does H, A does an oxygen atom, B does $CR^8$, and $R^8$ corresponds to each of all the substituents shown in Table 3-1.

Table 3-66

Table 3-66 represents 187 compounds wherein, in the general formula (I-2), $R^1$ represents Et, $R^2$ does H, $R^3$ does SPh, $R^4$ does H, A does an oxygen atom, B does $CR^8$, and $R^8$ corresponds to each of all the substituents shown in Table 3-1.

Table 3-67

Table 3-67 represents 187 compounds wherein, in the general formula (I-2), $R^1$ represents Et, $R^2$ does H, $R^3$ does OH, $R^4$ does H, A does an oxygen atom, B does $CR^8$, and $R^8$ corresponds to each of all the substituents shown in Table 3-1.

Table 3-68

Table 3-68 represents 187 compounds wherein, in the general formula (I-2), $R^1$ represents Et, $R^2$ does H, $R^3$ does OMe, $R^4$ does H, A does an oxygen atom, B does $CR^8$, and $R^8$ corresponds to each of all the substituents shown

in Table 3-1.

Table 3-69

Table 3-69 represents 187 compounds wherein, in the general formula (I-2), $R^1$ represents Et, $R^2$ does H, $R^3$ does OPh, $R^4$ does H, A does an oxygen atom, B does $CR^8$, and $R^8$ corresponds to each of all the substituents shown in Table 3-1.

Table 3-70

Table 3-70 represents 187 compounds wherein, in the general formula (I-2), $R^1$ represents Et, $R^2$ does H, $R^3$ does $NMe_2$, $R^4$ does H, A does an oxygen atom, B does $CR^8$, and $R^8$ corresponds to each of all the substituents shown in Table 3-1.

Table 3-71

Table 3-71 represents 187 compounds wherein, in the general formula (I-2), $R^1$ represents Et, $R^2$ does H, $R^3$ does Cl, $R^4$ does Me, A does an oxygen atom, B does $CR^8$, and $R^8$ corresponds to each of all the substituents shown in Table 3-1.

Table 3-72

Table 3-72 represents 187 compounds wherein, in the general formula (I-2), $R^1$ represents Et, $R^2$ does H, $R^3$ does Cl, $R^4$ does Et, A does an oxygen atom, B does $CR^8$, and $R^8$ corresponds to each of all the substituents shown in Table 3-1.

Table 3-73

Table 3-73 represents 187 compounds wherein, in the general formula (I-2), $R^1$ represents Et, $R^2$ does H, $R^3$ does Cl, $R^4$ does n-Pr, A does an oxygen atom, B does $CR^8$, and $R^8$ corresponds to each of all the substituents shown in Table 3-1.

Table 3-74

Table 3-74 represents 187 compounds wherein, in the general formula (I-2), $R^1$ represents Et, $R^2$ does H, $R^3$ does Cl, $R^4$ does i-Pr, A does an oxygen atom, B does $CR^8$, and $R^8$ corresponds to each of all the substituents shown in Table 3-1.

Table 3-75

Table 3-75 represents 187 compounds wherein, in the general formula (I-2), $R^1$ represents Et, $R^2$ does H, $R^3$ does Cl, $R^4$ does $CH_2CH=CH_2$, A does an oxygen atom, B does $CR^8$, and $R^8$ corresponds to each of all the substituents shown in Table 3-1.

Table 3-76

Table 3-76 represents 187 compounds wherein, in the general formula (I-2), $R^1$ represents Et, $R^2$ does H, $R^3$ does Cl, $R^4$ does $CH_2C{\equiv}CH$, A does an oxygen atom, B does $CR^8$, and $R^8$ corresponds to each of all the substituents shown in Table 3-1.

Table 3-77

Table 3-77 represents 187 compounds wherein, in the general formula (I-2), $R^1$ represents Et, $R^2$ does H, $R^3$ does Cl, $R^4$ does $CH_2CF_3$, A does an oxygen atom, B does $CR^8$, and $R^8$ corresponds to each of all the substituents shown in Table 3-1.

Table 3-78

Table 3-78 represents 187 compounds wherein, in the general formula (I-2), $R^1$ represents Et, $R^2$ does H, $R^3$ Cl, $R^4$ does $CH_2CN$, A does an oxygen atom, B does $CR^8$, and $R^8$ corresponds to each of all the substituents shown in Table 3-1.

Table 3-79

Table 3-79 represents 187 compounds wherein, in the general formula (I-2), $R^1$ represents Et, $R^2$ does H, $R^3$ does Cl, $R^4$ does $CH_2OMe$, A does an oxygen atom, B does $CR^8$, and $R^8$ corresponds to each of all the substituents shown in Table 3-1.

Table 3-80

Table 3-80 represents 187 compounds wherein, in the general formula (I-2), $R^1$ represents Et, $R^2$ does H, $R^3$ does Cl, $R^4$ does $CH_2OEt$, A does an oxygen atom, B does $CR^8$, and $R^8$ corresponds to each of all the substituents shown in Table 3-1.

Table 3-81

Table 3-81 represents 187 compounds wherein, in the general formula (I-2), $R^1$ represents Et, $R^2$ does H, $R^3$ does Cl, $R^4$ does $CH_2SMe$, A does an oxygen atom, B does $CR^8$, and $R^8$ corresponds to each of all the substituents shown in Table 3-1.

Table 3-82

Table 3-82 represents 187 compounds wherein, in the general formula (I-2), $R^1$ represents Et, $R^2$ does H, $R^3$ Cl, $R^4$ does $CH_2COOEt$, A does an oxygen atom, B does $CR^8$, and $R^8$ corresponds to each of all the substituents shown in Table 3-1.

Table 3-83

Table 3-83 represents 187 compounds wherein, in the general formula (I-2), $R^1$ represents Et, $R^2$ does H, $R^3$ Cl, $R^4$ does COOMe, A does an oxygen atom, B does $CR^8$, and $R^8$ corresponds to each of all the substituents shown in Table 3-1.

Table 3-84

Table 3-84 represents 187 compounds wherein, in the general formula (I-2), $R^1$ represents Et, $R^2$ does H, $R^3$ Cl, $R^4$ does CONHMe, A does an oxygen atom, B does $CR^8$, and $R^8$ corresponds to each of all the substituents shown in Table 3-1.

Table 3-85

Table 3-85 represents 187 compounds wherein, in the general formula (I-2), $R^1$ represents Et, $R^2$ does H, $R^3$ does Cl, $R^4$ does $CONMe_2$, A does an oxygen atom, B does $CR^8$, and $R^8$ corresponds to each of all the substituents shown in Table 3-1.

Table 3-86

Table 3-86 represents 187 compounds wherein, in the general formula (I-2), $R^1$ represents Et, $R^2$ does H, $R^3$ Cl, $R^4$ does COMe, A does an oxygen atom, B does $CR^8$, and $R^8$ corresponds to each of all the substituents shown in Table 3-1.

Table 3-87

Table 3-87 represents 187 compounds wherein, in the general formula (I-2), $R^1$ represents Et, $R^2$ does H, $R^3$ does Cl, $R^4$ does SPh, A does an oxygen atom, B does $CR^8$, and $R^8$ corresponds to each of all the substituents shown

in Table 3-1.

Table 3-88

Table 3-88 represents 187 compounds wherein, in the general formula (I-2), $R^1$ represents Et, $R^2$ does H, $R^3$ Cl, $R^4$ does $CH_2$-c-Pr, A does an oxygen atom, B does $CR^8$, and $R^8$ corresponds to each of all the substituents shown in Table 3-1.

Table 3-89

Table 3-89 represents 187 compounds wherein, in the general formula (I-2), $R^1$ represents Et, $R^2$ does H, $R^3$ does Cl, $R^4$ does H, A does a sulfur atom, B does $CR^8$, and $R^8$ corresponds to each of all the substituents shown in Table 3-1.

Table 3-90

Table 3-90 represents 187 compounds wherein, in the general formula (I-2), $R^1$ represents Et, $R^2$ does H, $R^3$ does Cl, $R^4$ does H, A does NH, B does $CR^8$, and $R^8$ corresponds to each of all the substituents shown in Table 3-1.

Table 3-91

Table 3-91 represents 187 compounds wherein, in the general formula (I-2), $R^1$ represents Et, $R^2$ does H, $R^3$ does Cl, $R^4$ does H, A does NMe, B does $CR^8$, and $R^8$ corresponds to each of all the substituents shown in Table 3-1.

Table 3-92

Table 3-92 represents 187 compounds wherein, in the general formula (I-2), $R^1$ represents Et, $R^2$ does H, $R^3$ does Cl, $R^4$ does H, A does CH, B does $NR^8$, and $R^8$ corresponds to each of all the substituents shown in Table 3-1.

Table 3-93

Table 3-93 represents 187 compounds wherein, in the general formula (I-2), $R^1$ represents Et, $R^2$ does H, $R^3$ does Cl, $R^4$ does H, A does CCl, B does $NR^8$, and $R^8$ corresponds to each of all the substituents shown in Table 3-1.

Table 3-94

Table 3-94 represents 187 compounds wherein, in the general formula (I-2), $R^1$ represents Et, $R^2$ does H, $R^3$ does Cl, $R^4$ does H, A does CH, B does N, and $R^8$ corresponds to each of all the substituents shown in Table 3-1.

Table 3-95

Table 3-95 represents 187 compounds wherein, in the general formula (I-2), $R^1$ represents Et, $R^2$ does H, $R^3$ does Cl, $R^4$ does H, A does CCl, B does N, and $R^8$ corresponds to each of all the substituents shown in Table 3-1.

Table 3-96

Table 3-96 represents 187 compounds wherein, in the general formula (I-2), $R^1$ represents Et, $R^2$ does H, $R^3$ does Cl, $R^4$ does H, A does N, B does $NR^8$, and $R^8$ corresponds to each of all the substituents shown in Table 3-1.

The general formula (I)

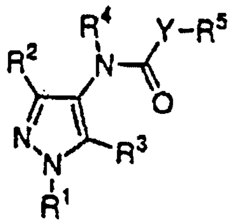

(I)

Table 4-1

| $R^1$ | $R^2$ | $R^3$ | $R^4$ | $R^5$ | Y |
|---|---|---|---|---|---|
| Me | Me | Cl | H | naphthalene-1-yl | $CH_2$ |
| Me | Me | Cl | H | naphthalene-2-yl | $CH_2$ |
| Me | Me | Cl | H | 2-OMe-naphthalene-1-yl | $CH_2$ |
| Me | Me | Cl | H | 4-OMe-naphthalene-1-yl | $CH_2$ |
| Me | Me | Cl | H | 6-OMe-naphthalene-2-yl | $CH_2$ |
| Me | Me | Cl | H | 5-Cl-thiophene-2-yl | $CH_2$ |
| Me | Me | Cl | H | 2-OPh-thiophene-5-yl | $CH_2$ |
| Me | Me | Cl | H | 2-0(4-CN-Ph)-thiophene-5-yl | $CH_2$ |
| Me | Me | Cl | H | 2-$OCH_2$Ph-thiophene-5-yl | $CH_2$ |
| Me | Me | Cl | H | 2-$OCH_2$ (4-CN-Ph)-thiophene-5-yl | $CH_2$ |
| Me | Me | Cl | H | 2-Br-thiazole-5-yl | $CH_2$ |
| Me | Me | Cl | H | indole-3-yl | $CH_2$ |
| Me | Me | Cl | H | 5-Br-indole-3-yl | $CH_2$ |
| Me | Me | Cl | H | 5-OMe-indole-3-yl | $CH_2$ |
| Me | Me | Cl | H | 2-Me-indole-3-yl | $CH_2$ |
| Me | Me | Cl | H | berizofuran-2-yl | $CH_2$ |
| Me | Me | Cl | H | benzimidazole-2-yl | $CH_2$ |
| Me | Me | Cl | H | benzothiazole-2-yl | $CH_2$ |
| Me | Me | Cl | H | 4-Me-benzothiazole-2-yl | $CH_2$ |
| Me | Me | Cl | H | 5-Cl-benzothiazole-2-yl | $CH_2$ |
| Me | Me | Cl | H | 5-OMe-benzothiazole-2-yl | $CH_2$ |
| Me | Me | Cl | H | 6-OMe-benzothiazole-2-yl | $CH_2$ |
| Me | Me | Cl | H | 6-F-benzothiazole-2-yl | $CH_2$ |
| Me | Me | Cl | H | 6-Me-benzothiazole-2-yl | $CH_2$ |
| Me | Me | Cl | H | 5,6-$Me_2$-benzothiazole-2-yl | $CH_2$ |
| Me | Me | Cl | H | pyridine-2-yl | $CH_2$ |
| Me | Me | Cl | H | pyridine-3-yl | $CH_2$ |
| Me | Me | Cl | H | pyridine-4-yl | $CH_2$ |
| Me | Me | Cl | H | 5-Bu-pyridine-2-yl | $CH_2$ |
| Me | Me | Cl | H | 3-Cl-pyridine-2-yl | $CH_2$ |
| Me | Me | Cl | H | 5-Br-pyridine-2-yl | $CH_2$ |
| Me | Me | Cl | H | 5-$CF_3$-pyridine-2-yl | $CH_2$ |
| Me | Me | Cl | H | 6-Br-pyridine-2-yl | $CH_2$ |
| Me | Me | Cl | H | 6-F-pyridine-2-yl | $CH_2$ |
| Me | Me | Cl | H | 6-Me-pyridine-2-yl | $CH_2$ |
| Me | Me | Cl | H | 3-$NO_2$-6-Cl-pyridine-2-yl | $CH_2$ |
| Me | Me | Cl | H | 3,5-$(CF_3)_2$-pyridine-2-yl | $CH_2$ |

Table 4-1 (continued)

| $R^1$ | $R^2$ | $R^3$ | $R^4$ | $R^5$ | Y |
|---|---|---|---|---|---|
| Me | Me | Cl | H | 4,5-$(CF_3)_2$-pyridine-2-yl | $CH_2$ |
| Me | Me | Cl | H | 2-Cl-pyridine-3-yl | $CH_2$ |
| Me | Me | Cl | H | 6-Cl-pyridine-3-yl | $CH_2$ |
| Me | Me | Cl | H | 5-Br-pyridine-3-yl | $CH_2$ |
| Me | Me | Cl | H | 2-Me-pyridine-3-yl | $CH_2$ |
| Me | Me | Cl | H | 6-Me-pyridine-3-yl | $CH_2$ |
| Me | Me | Cl | H | 2-OMe-pyridine-3-yl | $CH_2$ |
| Me | Me | Cl | H | 2-SMe-pyridine-3-yl | $CH_2$ |
| Me | Me | Cl | H | 5,6-$Cl_2$-pyridine-3-yl | $CH_2$ |
| Me | Me | Cl | H | 2,6-$Cl_2$-pyridine-3-yl | $CH_2$ |
| Me | Me | Cl | H | 2, 6- $(OMe)_2$-pyridine-3-yl | $CH_2$ |
| Me | Me | Cl | H | 2-Cl-6-Me-pyridine-3-yl | $CH_2$ |
| Me | Me | Cl | H | pyrazine-2-yl | $CH_2$ |
| Me | Me | Cl | H | pyrimidine-2-yl | $CH_2$ |
| Me | Me | Cl | H | pyrimidine-4-yl | $CH_2$ |
| Me | Me | Cl | H | 2-Cl-pyrimidine-5-yl | $CH_2$ |
| Me | Me | Cl | H | 2-SMe-pyrimidine-5-yl | $CH_2$ |
| Me | Me | Cl | H | 2-OPh-pyrimidine-5-yl | $CH_2$ |
| Me | Me | Cl | H | 2-0(4-CN-Ph)-pyrimidine-5-yl | $CH_2$ |
| Me | Me | Cl | H | 2-$OCH_2$Ph-pyrimidine-5-yl | $CH_2$ |
| Me | Me | Cl | H | 2-$OCH_2$ (4-CN-Ph)-pyrimidine-5-yl | $CH_2$ |
| Me | Me | Cl | H | pyridazine-3-yl | $CH_2$ |
| Me | Me | Cl | H | 6-Ph-pyridazine-3-yl | $CH_2$ |
| Me | Me | Cl | H | 6-Cl-pyridazine-3-yl | $CH_2$ |
| Me | Me | Cl | H | 6-OMe-pyridazine-3-yl | $CH_2$ |
| Me | Me | Cl | H | 6-OPh-pyridazine-3-yl | $CH_2$ |
| Me | Me | Cl | H | 6-0 (4-CN-Ph) -pyridazine-3-yl | $CH_2$ |
| Me | Me | Cl | H | quinoline-2-yl | $CH_2$ |
| Me | Me | Cl | H | quinoline-3-yl | $CH_2$ |
| Me | Me | Cl | H | isoquinoline-2-yl | $CH_2$ |
| Me | Me | Cl | H | isoquinoline-3-yl | $CH_2$ |
| Me | Me | Cl | H | 2-OPh-pyridine-3-yl | $CH_2$ |
| Me | Me | Cl | H | 2-0 (2-F-Ph) -pyridine-5-yl | $CH_2$ |
| Me | Me | Cl | H | 2-0 (3-F-Ph) -pyridine-5-yl | $CH_2$ |
| Me | Me | Cl | H | 2-0 (4-F-Ph) -pyridine-5-yl | $CH_2$ |
| Me | Me | Cl | H | 2-0(2-Cl-Ph)-pyridine-5-yl | $CH_2$ |
| Me | Me | Cl | H | 2-0(3-Cl-Ph)-pyridine-5-yl | $CH_2$ |
| Me | Me | Cl | H | 2-0(4-Cl-Ph)-pyridine-5-yl | $CH_2$ |
| Me | Me | Cl | H | 2-0(2-Br-Ph)-pyridine-5-yl | $CH_2$ |
| Me | Me | Cl | H | 2-0(3-Br-Ph)-pyridine-5-yl | $CH_2$ |
| Me | Me | Cl | H | 2-0 (4-Br-Ph) -pyridine-5-yl | $CH_2$ |
| Me | Me | Cl | H | 2-0 (2-Me-Ph) -pyridine-5-yl | $CH_2$ |
| Me | Me | Cl | H | 2-0(3-Me-Ph)-pyridine-5-yl | $CH_2$ |
| Me | Me | Cl | H | 2-0(4-Me-Ph)-pyridine-5-yl | $CH_2$ |
| Me | Me | Cl | H | 2-0(4-t-Bu-Ph)-pyridine-5-yl | $CH_2$ |
| Me | Me | Cl | H | 2-0 (2-OMe-Ph) -pyridine-5-yl | $CH_2$ |
| Me | Me | Cl | H | 2-0 (3-OMe-Ph) -pyridine-5-yl | $CH_2$ |
| Me | Me | Cl | H | 2-0 (4-OMe-Ph)-pyridine-5-yl | $CH_2$ |
| Me | Me | Cl | H | 2-0(2-$CF_3$-Ph)-pyridine-5-yl | $CH_2$ |
| Me | Me | Cl | H | 2-0(3-$CF_3$-Ph)-pyridine-5-yl | $CH_2$ |

Table 4-1 (continued)

| $R^1$ | $R^2$ | $R^3$ | $R^4$ | $R^5$ | Y |
|---|---|---|---|---|---|
| Me | Me | Cl | H | 2-0(4-$CF_3$-Ph)-pyridine-5-yl | $CH_2$ |
| Me | Me | Cl | H | 2-0(2-$OCF_3$-Ph)-pyridine-5-yl | $CH_2$ |
| Me | Me | Cl | H | 2-0(3-$OCF_3$-Ph)-pyridine-5-yl | $CH_2$ |
| Me | Me | Cl | H | 2-0(4-$OCF_3$-Ph)-pyridine-5-yl | $CH_2$ |
| Me | Me | Cl | H | 2-0(2-$NO_2$-Ph)-pyridine-5-yl | $CH_2$ |
| Me | Me | Cl | H | 2-O(3-$NO_2$-Ph)-pyridine-5-yl | $CH_2$ |
| Me | Me | Cl | H | 2-O(4-$NO_2$-Ph)-pyridine-5-yl | $CH_2$ |
| Me | Me | Cl | H | 2-0 (2-CN-Ph) -pyridine-5-yl | $CH_2$ |
| Me | Me | Cl | H | 2-0 (3-CN-Ph)-pyridine-5-yl | $CH_2$ |
| Me | Me | Cl | H | 2-0 (4-CN-Ph)-pyridine-5-yl | $CH_2$ |
| Me | Me | Cl | H | 2-O(2-$CO_2$Me-Ph)-pyridine-5-yl | $CH_2$ |
| Me | Me | Cl | H | 2-O(3-$CO_2$Me-Ph)-pyridine-5-yl | $CH_2$ |
| Me | Me | Cl | H | 2-O(4-$CO_2$Me-Ph)-pyridine-5-yl | $CH_2$ |
| Me | Me | Cl | H | 2-0 (2-SMe-Ph) -pyridine-5-yl | $CH_2$ |
| Me | Me | Cl | H | 2-0 (3-SMe-Ph) -pyridine-5-yl | $CH_2$ |
| Me | Me | Cl | H | 2-0 (4-SMe-Ph)-pyridine-5-yl | $CH_2$ |
| Me | Me | Cl | H | 2-0 (2-SOMe-Ph) -pyridine-5-yl | $CH_2$ |
| Me | Me | Cl | H | 2-0 (3-SOMe-Ph)-pyridine-5-yl | $CH_2$ |
| Me | Me | Cl | H | 2-0 (4-SOMe-Ph)-pyridine-5-yl | $CH_2$ |
| Me | Me | Cl | H | 2-O(2-$SO_2$Me-Ph)-pyridine-5-yl | $CH_2$ |
| Me | Me | Cl | H | 2-O(3-$SO_2$Me-Ph)-pyridine-5-yl | $CH_2$ |
| Me | Me | Cl | H | 2-O(4-$SO_2$Me-Ph)-pyridine-5-yl | $CH_2$ |
| Me | Me | Cl | H | 2-O(2-$SCF_3$-Ph)-pyridine-5-yl | $CH_2$ |
| Me | Me | Cl | H | 2-O(3-$SCF_3$-Ph)-pyridine-5-yl | $CH_2$ |
| Me | Me | Cl | H | 2-O(4-$SCF_3$-Ph)-pyridine-5-yl | $CH_2$ |
| Me | Me | Cl | H | 2-O(2-$SOCF_3$-Ph)-pyridine-5-yl | $CH_2$ |
| Me | Me | Cl | H | 2-O(3-$SOCF_3$-Ph)-pyridine-5-yl | $CH_2$ |
| Me | Me | Cl | H | 2-O(4-$SOCF_3$-Ph)-pyridine-5-yl | $CH_2$ |
| Me | Me | Cl | H | 2-O(2-$SO_2CF_3$-Ph)-pyridine-5-yl | $CH_2$ |
| Me | Me | Cl | H | 2-O(3-$SO_2CF_3$-Ph)-pyridine-5-yl | $CH_2$ |
| Me | Me | Cl | H | 2-O(4-$SO_2CF_3$-Ph)-pyridine-5-yl | $CH_2$ |
| Me | Me | Cl | H | 2-$OCH_2$Ph-pyridine-5-yl | $CH_2$ |
| Me | Me | Cl | H | 2-$OCH_2$(2-F-Ph)-pyridine-5-yl | $CH_2$ |
| Me | Me | Cl | H | 2-$OCH_2$(3-F-Ph)-pyridine-5-yl | $CH_2$ |
| Me | Me | Cl | H | 2-$OCH_2$(4-F-Ph)-pyridine-5-yl | $CH_2$ |
| Me | Me | Cl | H | 2-$OCH_2$(2-Cl-Ph)-pyridine-5-yl | $CH_2$ |
| Me | Me | Cl | H | 2-$OCH_2$(3-Cl-Ph)-pyridine-5-yl | $CH_2$ |
| Me | Me | Cl | H | 2-$OCH_2$(4-Cl-Ph)-pyridine-5-yl | $CH_2$ |
| Me | Me | Cl | H | 2-$OCH_2$(2-Br-Ph)-pyridine-5-yl | $CH_2$ |
| Me | Me | Cl | H | 2-$OCH_2$(3-Br-Ph)-pyridine-5-yl | $CH_2$ |
| Me | Me | Cl | H | 2-$OCH_2$ (4-Br-Ph)-pyridine-5-yl | $CH_2$ |
| Me | Me | Cl | H | 2-$OCH_2$(2-Me-Ph)-pyridine-5-yl | $CH_2$ |
| Me | Me | Cl | H | 2-$OCH_2$(3-Me-Ph)-pyridine-5-yl | $CH_2$ |
| Me | Me | Cl | H | 2-$OCH_2$(4-Me-Ph)-pyridirie-5-yl | $CH_2$ |
| Me | Me | Cl | H | 2-$OCH_2$(4-t-Bu-Ph)-pyridine-5-yl | $CH_2$ |
| Me | Me | Cl | H | 2-$OCH_2$(2-OMe-Ph)-pyridine-5-yl | $CH_2$ |
| Me | Me | Cl | H | 2-$OCH_3$(3-OMe-Ph)-pyridine-5-yl | $CH_2$ |
| Me | Me | Cl | H | 2-$OCH_2$(4-OMe-Ph)-pyridine-5-yl | $CH_2$ |
| Me | Me | Cl | H | 2-$OCH_2$(2-$CF_3$-Ph)-pyridine-5-yl | $CH_2$ |
| Me | Me | Cl | H | 2-$OCH_3$(3-$CF_3$-Ph)-pyridine-5-yl | $CH_2$ |

Table 4-1 (continued)

| $R^1$ | $R^2$ | $R^3$ | $R^4$ | $R^5$ | Y |
|---|---|---|---|---|---|
| Me | Me | Cl | H | 2-$OCH_2$(4-$CF_3$-Ph)-pyridine-5-yl | $CH_2$ |
| Me | Me | Cl | H | 2-$OCH_2$(2-$OCF_3$-Ph)-pyridine-5-yl | $CH_2$ |
| Me | Me | Cl | H | 2-$OCH_2$(3-$OCF_3$-Ph)-pyridine-5-yl | $CH_2$ |
| Me | Me | Cl | H | 2-$OCH_2$(4-$OCF_3$-Ph)-pyridine-5-yl | $CH_2$ |
| Me | Me | Cl | H | 2-$OCH_2$(2-$NO_2$-Ph)-pyridine-5-yl | $CH_2$ |
| Me | Me | Cl | H | 2-$OCH_2$(3-$NO_2$-Ph) -pyridine-5-yl | $CH_2$ |
| Me | Me | Cl | H | 2-$OCH_2$(4-$NO_2$-Ph)-pyridine-5-yl | $CH_2$ |
| Me | Me | Cl | H | 2-$OCH_2$(2-CN-Ph)-pyridine-5-yl | $CH_2$ |
| Me | Me | Cl | H | 2-$OCH_2$(3-CN-Ph)-pyridine-5-yl | $CH_2$ |
| Me | Me | Cl | H | 2-$OCH_2$(4-CN-Ph)-pyridine-5-yl | $CH_2$ |
| Me | Me | Cl | H | 2-$OCH_2$(2-$CO_2$Me-Ph)-pyridine-5-yl | $CH_2$ |
| Me | Me | Cl | H | 2-$OCH_2$(3-$CO_2$Me-Ph)-pyridine-5-yl | $CH_2$ |
| Me | Me | Cl | H | 2-$OCH_2$(4-$CO_2$Me-Ph)-pyridirie-5-yl | $CH_2$ |
| Me | Me | Cl | H | 2-$OCH_2$(2-SMe-Ph)-pyridine-5-yl | $CH_2$ |
| Me | Me | Cl | H | 2-$OCH_2$(3-SMe-Ph)-pyridine-5-yl | $CH_2$ |
| Me | Me | Cl | H | 2-$OCH_2$(4-SMe-Ph)-pyridine-5-yl | $CH_2$ |
| Me | Me | Cl | H | 2-$OCH_2$ (2-SOMe-Ph) -pyridine-5-yl | $CH_2$ |
| Me | Me | Cl | H | 2-$OCH_2$(3-SOMe-Ph)-pyridine-5-yl | $CH_2$ |
| Me | Me | Cl | H | 2-$OCH_2$(4-SOMe-Ph)-pyridine-5-yl | $CH_2$ |
| Me | Me | Cl | H | 2-$OCH_2$(2-$SO_2$Me-Ph)-pyridine-5-yl | $CH_2$ |
| Me | Me | Cl | H | 2-$OCH_2$(3-$SO_2$Me-Ph)-pyridine-5-yl | $CH_2$ |
| Me | Me | Cl | H | 2-$OCH_2$(4-$SO_2$Me-Ph)-pyridine-5-yl | $CH_2$ |
| Me | Me | Cl | H | 2-$OCH_2$(2-$SCF_3$-Ph)-pyridine-5-yl | $CH_2$ |
| Me | Me | Cl | H | 2-$OCH_2$(3-$SCF_3$-Ph)-pyridine-5-yl | $CH_2$ |
| Me | Me | Cl | H | 2-$OCH_2$(4-$SCF_3$-Ph)-pyridine-5-yl | $CH_2$ |
| Me | Me | Cl | H | 2-$OCH_2$(2-$SOCF_3$-Ph)-pyridine-5-yl | $CH_2$ |
| Me | Me | Cl | H | 2-$OCH_2$(3-$SOCF_3$-Ph)-pyridine-5-yl | $CH_2$ |
| Me | Me | Cl | H | 2-$OCH_2$(4-$SOCF_3$-Ph)-pyridine-5-yl | $CH_2$ |
| Me | Me | Cl | H | 2-$OCH_2$(2-$SO_2CF_3$-Ph)-pyridine-5-yl | $CH_2$ |
| Me | Me | Cl | H | 2-$OCH_2$(3-$SO_2CF_3$-Ph)-pyridine-5-yl | $CH_2$ |
| Me | Me | Cl | H | 2-$OCH_2$ (4-$SO_2CF_3$-Ph)-pyridine-5-yl | $CH_2$ |
| Me | Me | Cl | H | 5-OPh-pyridine-2-yl | $CH_2$ |
| Me | Me | Cl | H | 5-0(2-F-Ph)-pyridine-2-yl | $CH_2$ |
| Me | Me | Cl | H | 5-0(3-F-Ph)-pyridine-2-yl | $CH_2$ |
| Me | Me | Cl | H | 5-0(4-F-Ph)-pyridine-2-yl | $CH_2$ |
| Me | Me | Cl | H | 5-0(2-Cl-Ph)-pyridine-2-yl | $CH_2$ |
| Me | Me | Cl | H | 5-0(3-Cl-Ph)-pyridine-2-yl | $CH_2$ |
| Me | Me | Cl | H | 5-0(4-Cl-Ph)-pyridine-2-yl | $CH_2$ |
| Me | Me | Cl | H | 5-0 (2-Br-Ph) -pyridine-2-yl | $CH_2$ |
| Me | Me | Cl | H | 5-0(3-Br-Ph)-pyridine-2-yl | $CH_2$ |
| Me | Me | Cl | H | 5-0(4-Br-Ph)-pyridine-2-yl | $CH_2$ |
| Me | Me | Cl | H | 5-0(2-Me-Ph)-pyridine-2-yl | $CH_2$ |
| Me | Me | Cl | H | 5-0 (3-Me-Ph) -pyridine-2-yl | $CH_2$ |
| Me | Me | Cl | H | 5-0(4-Me-Ph)-pyridine-2-yl | $CH_2$ |
| Me | Me | Cl | H | 5-0 (4-t-Bu-Ph)-pyridine-2-yl | $CH_2$ |
| Me | Me | Cl | H | 5-0 (2-OMe-Ph)-pyridine-2-yl | $CH_2$ |
| Me | Me | Cl | H | 5-0 (3-OMe-Ph) -pyridine-2-yl | $CH_2$ |
| Me | Me | Cl | H | 5-0 (4-OMe-Ph) -pyridine-2-yl | $CH_2$ |
| Me | Me | Cl | H | 5-0 (2-$CF_3$-Ph)-pyridine-2-yl | $CH_2$ |
| Me | Me | Cl | H | 5-0 (3-$CF_3$-Ph)-pyridine-2-yl | $CH_2$ |

Table 4-1 (continued)

| $R^1$ | $R^2$ | $R^3$ | $R^4$ | $R^5$ | Y |
|---|---|---|---|---|---|
| Me | Me | Cl | H | 5-0 (4-$CF_3$-Ph)-pyridine-2-yl | $CH_2$ |
| Me | Me | Cl | H | 5-0 (2-$OCF_3$-Ph)-pyridine-2-yl | $CH_2$ |
| Me | Me | Cl | H | 5-0 (3-$OCF_3$-Ph)-pyridine-2-yl | $CH_2$ |
| Me | Me | Cl | H | 5-0 (4-$OCF_3$-Ph)-pyridine-2-yl | $CH_2$ |
| Me | Me | Cl | H | 5-0 (2-$NO_2$-Ph)-pyridine-2-yl | $CH_2$ |
| Me | Me | Cl | H | 5-O(3-$NO_2$-Ph)-pyridine-2-yl | $CH_2$ |
| Me | Me | Cl | H | 5-O(4-$NO_2$-Ph)-pyridine-2-yl | $CH_2$ |
| Me | Me | Cl | H | 5-0 (2-CN-Ph)-pyridine-2-yl | $CH_2$ |
| Me | Me | Cl | H | 5-0 (3-CN-Ph)-pyridine-2-yl | $CH_2$ |
| Me | Me | Cl | H | 5-0 (4-CN-Ph)-pyridine-2-yl | $CH_2$ |
| Me | Me | Cl | H | 5-0 (2-$CO_2$Me-Ph)-pyridine-2-yl | $CH_2$ |
| Me | Me | Cl | H | 5-0 (3-$CO_2$Me-Ph)-pyridine-2-yl | $CH_2$ |
| Me | Me | Cl | H | 5-0(4-$CO_2$Me-Ph)-pyridine-2-yl | $CH_2$ |
| Me | Me | Cl | H | 5-0 (2-SMe-Ph)-pyridine-2-yl | $CH_2$ |
| Me | Me | Cl | H | 5-0 (3-SMe-Ph)-pyridine-2-yl | $CH_2$ |
| Me | Me | Cl | H | 5-0 (4-SMe-Ph)-pyridine-2-yl | $CH_2$ |
| Me | Me | Cl | H | 5-0 (2-SOMe-Ph) -pyridine-2-yl | $CH_2$ |
| Me | Me | Cl | H | 5-0 (3-SOMe-Ph)-pyridine-2-yl | $CH_2$ |
| Me | Me | Cl | H | 5-0 (4-SOMe-Ph) -pyridine-2-yl | $CH_2$ |
| Me | Me | Cl | H | 5-0 (2-$SO_2$Me-Ph)-pyridine-2-yl | $CH_2$ |
| Me | Me | Cl | H | 5-0 (3-$SO_2$Me-Ph)-pyridine-2-yl | $CH_2$ |
| Me | Me | Cl | H | 5-0 (4-$SO_2$Me-Ph)-pyridine-2-yl | $CH_2$ |
| Me | Me | Cl | H | 5-0 (2-$SCF_3$-Ph)-pyridine-2-yl | $CH_2$ |
| Me | Me | Cl | H | 5-0 (3-$SCF_3$-Ph)-pyridine-2-yl | $CH_2$ |
| Me | Me | Cl | H | 5-0 (4-$SCF_3$-Ph)-pyridine-2-yl | $CH_2$ |
| Me | Me | Cl | H | 5-0 (2-$SOCF_3$-Ph)-pyridine-2-yl | $CH_2$ |
| Me | Me | Cl | H | 5-0 (3-$SOCF_3$-Ph)-pyridine-2-yl | $CH_2$ |
| Me | Me | Cl | H | 5-0 (4-$SOCF_3$-Ph)-pyridine-2-yl | $CH_2$ |
| Me | Me | Cl | H | 5-0 (2-$SO_2CF_3$-Ph)-pyridine-2-yl | $CH_2$ |
| Me | Me | Cl | H | 5-0(3-$SO_2CF_3$-Ph)-pyridine-2-yl | $CH_2$ |
| Me | Me | Cl | H | 5-0 (4-$SO_2CF_3$-Ph)-pyridine-2-yl | $CH_2$ |
| Me | Me | Cl | H | 5-$OCH_2$Ph-pyridine-2-yl | $CH_2$ |
| Me | Me | Cl | H | 5-$OCH_2$(2-F-Ph)-pyridine-2-yl | $CH_2$ |
| Me | Me | Cl | H | 5-$OCH_2$(3-F-Ph) -pyridine-2-yl | $CH_2$ |
| Me | Me | Cl | H | 5-$OCH_2$(4-F-Ph)-pyridine-2-yl | $CH_2$ |
| Me | Me | Cl | H | 5-$OCH_2$ (2-Cl-Ph)-pyridine-2-yl | $CH_2$ |
| Me | Me | Cl | H | 5-$OCH_2$ (3-Cl-Ph)-pyridine-2-yl | $CH_2$ |
| Me | Me | Cl | H | 5-$OCH_2$ (4-Cl-Ph)-pyridine-2-yl | $CH_2$ |
| Me | Me | Cl | H | 5-$OCH_2$ (2-Br-Ph)-pyridine-2-yl | $CH_2$ |
| Me | Me | Cl | H | 5-$OCH_2$ (3-Br-Ph)-pyridine-2-yl | $CH_2$ |
| Me | Me | Cl | H | 5-$OCH_2$ (4-Br-Ph)-pyridine-2-yl | $CH_2$ |
| Me | Me | Cl | H | 5-$OCH_2$ (2-Me-Ph)-pyridine-2-yl | $CH_2$ |
| Me | Me | Cl | H | 5-$OCH_2$ (3-Me-Ph)-pyridine-2-yl | $CH_2$ |
| Me | Me | Cl | H | 5-$OCH_2$ (4-Me-Ph)-pyridine-2-yl | $CH_2$ |
| Me | Me | Cl | H | 5-$OCH_2$(4-t-Bu-Ph)-pyridine-2-yl | $CH_2$ |
| Me | Me | Cl | H | 5-$OCH_2$(2-OMe-Ph)-pyridine-2-yl | $CH_2$ |
| Me | Me | Cl | H | 5-$OCH_2$(3-OMe-Ph)-pyridine-2-yl | $CH_2$ |
| Me | Me | Cl | H | 5-$OCH_2$(4-OMe-Ph)-pyridine-2-yl | $CH_2$ |
| Me | Me | Cl | H | 5-$OCH_2$(2-$CF_3$-Ph)-pyridine-2-yl | $CH_2$ |
| Me | Me | Cl | H | 5-$OCH_2$(3-$CF_3$-Ph)-pyridine-2-yl | $CH_2$ |

Table 4-1 (continued)

| $R^1$ | $R^2$ | $R^3$ | $R^4$ | $R^5$ | Y |
|---|---|---|---|---|---|
| Me | Me | Cl | H | 5-$OCH_2$(4-$CF_3$-Ph)-pyridine-2-yl | $CH_2$ |
| Me | Me | Cl | H | 5-$OCH_2$(2-$OCF_3$-Ph)-pyridine-2-yl | $CH_2$ |
| Me | Me | Cl | H | 5-$OCH_2$(3-$OCF_3$-Ph)-pyridine-2-yl | $CH_2$ |
| Me | Me | Cl | H | 5-$OCH_2$(4-$OCF_3$-Ph)-pyridine-2-yl | $CH_2$ |
| Me | Me | Cl | H | 5-$OCH_2$(2-$NO_2$-Ph)-pyridine-2-yl | $CH_2$ |
| Me | Me | Cl | H | 5-$OCH_2$(3-$NO_2$-Ph)-pyridine-2-yl | $CH_2$ |
| Me | Me | Cl | H | 5-$OCH_2$(4-$NO_2$-Ph)-pyridine-2-yl | $CH_2$ |
| Me | Me | Cl | H | 5-$OCH_2$(2-CN-Ph)-pyridine-2-yl | $CH_2$ |
| Me | Me | Cl | H | 5-$OCH_2$ (3-CN-Ph) -pyridine-2-yl | $CH_2$ |
| Me | Me | Cl | H | 5-$OCH_2$ (4-CN-Ph)-pyridine-2-yl | $CH_2$ |
| Me | Me | Cl | H | 5-$OCH_2$ (2-$CO_2$Me-Ph)-pyridine-2-yl | $CH_2$ |
| Me | Me | Cl | H | 5-$OCH_2$ (3-$CO_2$Me-Ph)-pyridine-2-yl | $CH_2$ |
| Me | Me | Cl | H | 5-$OCH_2$ (4-$CO_2$Me-Ph) -pyridine-2-yl | $CH_2$ |
| Me | Me | Cl | H | 5-$OCH_2$ (2-SMe-Ph)-pyridine-2-yl | $CH_2$ |
| Me | Me | Cl | H | 5-$OCH_2$ (3-SMe-Ph)-pyridine-2-yl | $CH_2$ |
| Me | Me | Cl | H | 5-$OCH_2$ (4-SMe-Ph)-pyridine-2-yl | $CH_2$ |
| Me | Me | Cl | H | 5-$OCH_2$ (2-SOMe-Ph)-pyridine-2-yl | $CH_2$ |
| Me | Me | Cl | H | 5-$OCH_2$ (3-SOMe-Ph)-pyridine-2-yl | $CH_2$ |
| Me | Me | Cl | H | 5-$OCH_2$ (4-SOMe-Ph)-pyridine-2-yl | $CH_2$ |
| Me | Me | Cl | H | 5-$OCH_2$ (2-$SO_2$Me-Ph)-pyridine-2-yl | $CH_2$ |
| Me | Me | Cl | H | 5-$OCH_2$ (3-$SO_2$Me-Ph)-pyridine-2-yl | $CH_2$ |
| Me | Me | Cl | H | 5-$OCH_2$ (4-$SO_2$Me-Ph)-pyridine-2-yl | $CH_2$ |
| Me | Me | Cl | H | 5-$OCH_2$ (2-$SCF_3$-Ph)-pyridine-2-yl | $CH_2$ |
| Me | Me | Cl | H | 5-$OCH_2$ (3-$SCF_3$-Ph)-pyridine-2-yl | $CH_2$ |
| Me | Me | Cl | H | 5-$OCH_2$ (4-$SCF_3$-Ph)-pyridine-2-yl | $CH_2$ |
| Me | Me | Cl | H | 5-$OCH_2$ (2-$SOCF_3$-Ph)-pyridine-2-yl | $CH_2$ |
| Me | Me | Cl | H | 5-$OCH_2$ (3-$SOCF_3$-Ph)-pyridine-2-yl | $CH_2$ |
| Me | Me | Cl | H | 5-$OCH_2$ (4-$SOCF_3$-Ph)-pyridine-2-yl | $CH_2$ |
| Me | Me | Cl | H | 5-$OCH_2$ (2-$SO_2CF_3$-Ph)-pyridine-2-yl | $CH_2$ |
| Me | Me | Cl | H | 5-$OCH_2$ (3-$SO_2CF_3$-Ph)-pyridine-2-yl | $CH_2$ |
| Me | Me | Cl | H | 5-$OCH_2$(4-$SO_2CF_3$-Ph)-pyridine-2-yl | $CH_2$ |

Table 4-2

Table 4-2 represents 268 compounds wherein, in the general formula (I), $R^1$ represents Me, $R^2$ does Et, $R^3$ does Cl, $R^4$ does H, Y does $CH_2$, and $R^5$ corresponds to each of all the substituents shown in Table 4-1.

Table 4-3

Table 4-3 represents 268 compounds wherein, in the general formula (I), $R^1$ represents Me, $R^2$ does i-Pr, $R^3$ does Cl, $R^4$ does H, Y does $CH_2$, and $R^5$ corresponds to each of all the substituents shown in Table 4-1.

Table 4-4

Table 4-4 represents 268 compounds wherein, in the general formula (I), $R^1$ represents Me, $R^2$ does n-Pr, $R^3$ does Cl, $R^4$ does H, Y does $CH_2$, and $R^5$ corresponds to each of all the substituents shown in Table 4-1.

Table 4-5

Table 4-5 represents 268 compounds wherein, in the general formula (I), $R^1$ represents Me, $R^2$ does t-Bu, $R^3$ does Cl, $R^4$ does H, Y does $CH_2$, and $R^5$ corresponds to each of all the substituents shown in Table 4-1.

Table 4-6

Table 4-6 represents 268 compounds wherein, in the general formula (I), $R^1$ represents Me, $R^2$ does n-Bu, $R^3$ does Cl, $R^4$ does H, Y does $CH_2$, and $R^5$ corresponds to each of all the substituents shown in Table 4-1.

Table 4-7

Table 4-7 represents 268 compounds wherein, in the general formula (I), $R^1$ represents Me, $R^2$ does $CF_3$, $R^3$ does Cl, $R^4$ does H, Y does $CH_2$, and $R^5$ corresponds to each of all the substituents shown in Table 4-1.

Table 4-8

Table 4-8 represents 268 compounds wherein, in the general formula (I), $R^1$ represents Me, $R^2$ does OMe, $R^3$ does Cl, $R^4$ does H, Y does $CH_2$, and $R^5$ corresponds to each of all the substituents shown in Table 4-1.

Table 4-9

Table 4-9 represents 268 compounds wherein, in the general formula (I), $R^1$ represents Me, $R^2$ does $OCHF_2$, $R^3$ does Cl, $R^4$ does H, Y does $CH_2$, and $R^5$ corresponds to each of all the substituents shown in Table 4-1.

Table 4-10

Table 4-10 represents 268 compounds wherein, in the general formula (I), $R^1$ represents Me, $R^2$ does O-i-Pr, $R^3$ does Cl, $R^4$ does H, Y does $CH_2$, and $R^5$ corresponds to each of all the substituents shown in Table 4-1.

Table 4-11

Table 4-11 represents 268 compounds wherein, in the general formula (I), $R^1$ represents Me, $R^2$ does Cl, $R^3$ does Cl, $R^4$ does H, Y does $CH_2$, and $R^5$ corresponds to each of all the substituents shown in Table 4-1.

Table 4-12

Table 4-12 represents 268 compounds wherein, in the general formula (I), $R^1$ represents Me, $R^2$ does Ph, $R^3$ does Cl, $R^4$ does H, Y does $CH_2$, and $R^5$ corresponds to each of all the substituents shown in Table 4-1.

Table 4-13

Table 4-13 represents 268 compounds wherein, in the general formula (I), $R^1$ represents Me, $R^2$ does 2-Cl-Ph, $R^3$ does Cl, $R^4$ does H, Y does $CH_2$, and $R^5$ corresponds to each of all the substituents shown in Table 4-1.

Table 4-14

Table 4-14 represents 268 compounds wherein, in the general formula (I), $R^1$ represents Me, $R^2$ does 3-Cl-Ph, $R^3$ does Cl, $R^4$ does H, Y does $CH_2$, and $R^5$ corresponds to each of all the substituents shown in Table 4-1.

Table 4-15

Table 4-15 represents 268 compounds wherein, in the general formula (I), $R^1$ represents Me, $R^2$ does 4-Cl-Ph, $R^3$ does Cl, $R^4$ does H, Y does $CH_2$, and $R^5$ corresponds to each of all the substituents shown in Table 4-1.

Table 4-16

Table 4-16 represents 268 compounds wherein, in the general formula (I), $R^1$ represents Me, $R^2$ does 2,4-$Cl_2$-Ph, $R^3$ does Cl, $R^4$ does H, Y does $CH_2$, and $R^5$ corresponds to each of all the substituents shown in Table 4-1.

Table 4-17

Table 4-17 represents 268 compounds wherein, in the general formula (I), $R^1$ represents Me, $R^2$ does 2-Me-

Ph, $R^3$ does Cl, $R^4$ does H, Y does $CH_2$, and $R^5$ corresponds to each of all the substituents shown in Table 4-1.

Table 4-18

Table 4-18 represents 268 compounds wherein, in the general formula (I), $R^1$ represents Me, $R^2$ does 3-Me-Ph, $R^3$ does Cl, $R^4$ does H, Y does $CH_2$, and $R^5$ corresponds to each of all the substituents shown in Table 4-1.

Table 4-19

Table 4-19 represents 268 compounds wherein, in the general formula (I), $R^1$ represents Me, $R^2$ does 4-Me-Ph, $R^3$ does Cl, $R^4$ does H, Y does $CH_2$, and $R^5$ corresponds to each of all the substituents shown in Table 4-1.

Table 4-20

Table 4-20 represents 268 compounds wherein, in the general formula (I), $R^1$ represents Me, $R^2$ does 2-OMe-Ph, $R^3$ does Cl, $R^4$ does H, Y does $CH_2$, and $R^5$ corresponds to each of all the substituents shown in Table 4-1.

Table 4-21

Table 4-21 represents 268 compounds wherein, in the general formula (I), $R^1$ represents Me, $R^2$ does 3-OMe-Ph, $R^3$ does Cl, $R^4$ does H, Y does $CH_2$, and $R^5$ corresponds to each of all the substituents shown in Table 4-1.

Table 4-22

Table 4-22 represents 268 compounds wherein, in the general formula (I), $R^1$ represents Me, $R^2$ does 4-OMe-Ph, $R^3$ does Cl, $R^4$ does H, Y does $CH_2$, and $R^5$ corresponds to each of all the substituents shown in Table 4-1.

Table 4-23

Table 4-23 represents 268 compounds wherein, in the general formula (I), $R^1$ represents Me, $R^2$ does H, $R^3$ does Cl, $R^4$ does H, Y does $CH_2$, and $R^5$ corresponds to each of all the substituents shown in Table 4-1.

Table 4-24

Table 4-24 represents 268 compounds wherein, in the general formula (I), $R^1$ represents H, $R^2$ does H, $R^3$ does Cl, $R^4$ does H, Y does $CH_2$, and $R^5$ corresponds to each of all the substituents shown in Table 4-1.

Table 4-25

Table 4-25 represents 268 compounds wherein, in the general formula (I), $R^1$ represents Et, $R^2$ does H, $R^3$ does Cl, $R^4$ does H, Y does $CH_2$, and $R^5$ corresponds to each of all the substituents shown in Table 4-1.

Table 4-26

Table 4-26 represents 268 compounds wherein, in the general formula (I), $R^1$ represents i-Pr, $R^2$ does H, $R^3$ does Cl, $R^4$ does H, Y does $CH_2$, and $R^5$ corresponds to each of all the substituents shown in Table 4-1.

Table 4-27

Table 4-27 represents 268 compounds wherein, in the general formula (I), $R^1$ represents n-Pr, $R^2$ does H, $R^3$ does Cl, $R^4$ does H, Y does $CH_2$, and $R^5$ corresponds to each of all the substituents shown in Table 4-1.

Table 4-28

Table 4-28 represents 268 compounds wherein, in the general formula (I), $R^1$ represents t-Bu, $R^2$ does H, $R^3$ does Cl, $R^4$ does H, Y does $CH_2$, and $R^5$ corresponds to each of all the substituents shown in Table 4-1.

Table 4-29

Table 4-29 represents 268 compounds wherein, in the general formula (I), $R^1$ represents n-Bu, $R^2$ does H, $R^3$ does Cl, $R^4$ does H, Y does $CH_2$, and $R^5$ corresponds to each of all the substituents shown in Table 4-1.

Table 4-30

Table 4-30 represents 268 compounds wherein, in the general formula (I), $R^1$ represents $CH=CH_2$, $R^2$ does H, $R^3$ does Cl, $R^4$ does H, Y does $CH_2$, and $R^5$ corresponds to each of all the substituents shown in Table 4-1.

Table 4-31

Table 4-31 represents 268 compounds wherein, in the general formula (I), $R^1$ represents $CH_2CH=CH_2$, $R^2$ does H, $R^3$ does Cl, $R^4$ does H, Y does $CH_2$, and $R^5$ corresponds to each of all the substituents shown in Table 4-1.

Table 4-32

Table 4-32 represents 268 compounds wherein, in the general formula (I), $R^1$ represents $CH_2C{\equiv}CH$, $R^2$ does H, $R^3$ does Cl, $R^4$ does H, Y does $CH_2$, and $R^5$ corresponds to each of all the substituents shown in Table 4-1.

Table 4-33

Table 4-33 represents 268 compounds wherein, in the general formula (I), $R^1$ represents $CH_2CN$, $R^2$ does H, $R^3$ does Cl, $R^4$ does H, Y does $CH_2$, and $R^5$ corresponds to each of all the substituents shown in Table 4-1.

Table 4-34

Table 4-34 represents 268 compounds wherein, in the general formula (I), $R^1$ represents $CH_2CF_3$, $R^2$ does H, $R^3$ does Cl, $R^4$ does H, Y does $CH_2$, and $R^5$ corresponds to each of all the substituents shown in Table 4-1.

Table 4-35

Table 4-35 represents 268 compounds wherein, in the general formula (I), $R^1$ represents $CH_2Ph$, $R^2$ does H, $R^3$ does Cl, $R^4$ does H, Y does $CH_2$, and $R^5$ corresponds to each of all the substituents shown in Table 4-1.

Table 4-36

Table 4-36 represents 268 compounds wherein, in the general formula (I), $R^1$ represents Ph, $R^2$ does H, $R^3$ does Cl, $R^4$ does H, Y does $CH_2$, and $R^5$ corresponds to each of all the substituents shown in Table 4-1.

Table 4-37

Table 4-37 represents 268 compounds wherein, in the general formula (I), $R^1$ represents 2-Cl-Ph, $R^2$ does H, $R^3$ does Cl, $R^4$ does H, Y does $CH_2$, and $R^5$ corresponds to each of all the substituents shown in Table 4-1.

Table 4-38

Table 4-38 represents 268 compounds wherein, in the general formula (I), $R^1$ represents 3-Cl-Ph, $R^2$ does H, $R^3$ does Cl, $R^4$ does H, Y does $CH_2$, and $R^5$ corresponds to each of all the substituents shown in Table 4-1.

Table 4-39

Table 4-39 represents 268 compounds wherein, in the general formula (I), $R^1$ represents 4-Cl-Ph, $R^2$ does H, $R^3$ does Cl, $R^4$ does H, Y does $CH_2$, and $R^5$ corresponds to each of all the substituents shown in Table 4-1.

Table 4-40

Table 4-40 represents 268 compounds wherein, in the general formula (I), $R^1$ represents 2,4-$Cl_2$-Ph, $R^2$ does

H, $R^3$ does Cl, $R^4$ does H, Y does $CH_2$, and $R^5$ corresponds to each of all the substituents shown in Table 4-1.

Table 4-41

Table 4-41 represents 268 compounds wherein, in the general formula (I), $R^1$ represents Ac, $R^2$ does H, $R^3$ does Cl, $R^4$ does H, Y does $CH_2$, and $R^5$ corresponds to each of all the substituents shown in Table 4-1.

Table 4-42

Table 4-42 represents 268 compounds wherein, in the general formula (I), $R^1$ represents COPh, $R^2$ does H, $R^3$ does Cl, $R^4$ does H, Y does $CH_2$, and $R^5$ corresponds to each of all the substituents shown in Table 4-1.

Table 4-43

Table 4-43 represents 268 compounds wherein, in the general formula (I), $R^1$ represents $CH_2CH_2F$, $R^2$ does H, $R^3$ does Cl, $R^4$ does H, Y does $CH_2$, and $R^5$ corresponds to each of all the substituents shown in Table 4-1.

Table 4-44

Table 4-44 represents 268 compounds wherein, in the general formula (I), $R^1$ represents $CH_2CH_2Cl$, $R^2$ does H, $R^3$ does Cl, $R^4$ does H, Y does $CH_2$, and $R^5$ corresponds to each of all the substituents shown in Table 4-1.

Table 4-45

Table 4-45 represents 268 compounds wherein, in the general formula (I), $R^1$ represents $CH_2CH_2Br$, $R^2$ does H, $R^3$ does Cl, $R^4$ does H, Y does $CH_2$, and $R^5$ corresponds to each of all the substituents shown in Table 4-1.

Table 4-46

Table 4-46 represents 268 compounds wherein, in the general formula (I), $R^1$ represents $CH_2CH_2OH$, $R^2$ does H, $R^3$ does Cl, $R^4$ does H, Y does $CH_2$, and $R^5$ corresponds to each of all the substituents shown in Table 4-1.

Table 4-47

Table 4-47 represents 268 compounds wherein, in the general formula (I), $R^1$ represents $CH_2CH_2OMe$, $R^2$ does H, $R^3$ does Cl, $R^4$ does H, Y does $CH_2$, and $R^5$ corresponds to each of all the substituents shown in Table 4-1.

Table 4-48

Table 4-48 represents 268 compounds wherein, in the general formula (I) , $R^1$ represents $CH_2OMe$, $R^2$ does H, $R^3$ does Cl, $R^4$ does H, Y does $CH_2$, and $R^5$ corresponds to each of all the substituents shown in Table 4-1. Table 4-49

Table 4-49 represents 268 compounds wherein, in the general formula (I), $R^1$ represents $CH_2OEt$, $R^2$ does H, $R^3$ does Cl, $R^4$ does H, Y does $CH_2$, and $R^5$ corresponds to each of all the substituents shown in Table 4-1.

Table 4-50

Table 4-50 represents 268 compounds wherein, in the general formula (I), $R^1$ represents $CH_2COOH$, $R^2$ does H, $R^3$ does Cl, $R^4$ does H, Y does $CH_2$, and $R^5$ corresponds to each of all the substituents shown in Table 4-1.

Table 4-51

Table 4-51 represents 268 compounds wherein, in the general formula (I), $R^1$ represents $CH_2COOEt$, $R^2$ does H, $R^3$ does Cl, $R^4$ does H, Y does $CH_2$, and $R^5$ corresponds to each of all the substituents shown in Table 4-1.

Table 4-52

Table 4-52 represents 268 compounds wherein, in the general formula (I), $R^1$ represents $CH_2CONHMe$, $R^2$ does H, $R^3$ does Cl, $R^4$ does H, Y does $CH_2$, and $R^5$ corresponds to each of all the substituents shown in Table 4-1.

Table 4-53

Table 4-53 represents 268 compounds wherein, in the general formula (I), $R^1$ represents $CH_2CONMe_2$, $R^2$ does H, $R^3$ does Cl, $R^4$ does H, Y does $CH_2$, and $R^5$ corresponds to each of all the substituents shown in Table 4-1.

Table 4-54

Table 4-54 represents 268 compounds wherein, in the general formula (I), $R^1$ represents $CH_2CHO$, $R^2$ does H, $R^3$ does Cl, $R^4$ does H, Y does $CH_2$, and $R^5$ corresponds to each of all the substituents shown in Table 4-1.

Table 4-55

Table 4-55 represents 268 compounds wherein, in the general formula (I), $R^1$ represents $CH_2CH=NOMe$, $R^2$ does H, $R^3$ does Cl, $R^4$ does H, Y does $CH_2$, and $R^5$ corresponds to each of all the substituents shown in Table 4-1.

Table 4-56

Table 4-56 represents 268 compounds wherein, in the general formula (I), $R^1$ represents Et, $R^2$ does H, $R^3$ does H, $R^4$ does H, Y does $CH_2$, and $R^5$ corresponds to each of all the substituents shown in Table 4-1.

Table 4-57

Table 4-57 represents 268 compounds wherein, in the general formula (I), $R^1$ represents Et, $R^2$ does H, $R^3$ does F, $R^4$ does H, Y does $CH_2$, and $R^5$ corresponds to each of all the substituents shown in Table 4-1.

Table 4-58

Table 4-58 represents 268 compounds wherein, in the general formula (I), $R^1$ represents Et, $R^2$ does H, $R^3$ does Br, $R^4$ does H, Y does $CH_2$, and $R^5$ corresponds to each of all the substituents shown in Table 4-1.

Table 4-59

Table 4-59 represents 268 compounds wherein, in the general formula (I), $R^1$ represents Et, $R^2$ does H, $R^3$ does I, $R^4$ does H, Y does $CH_2$, and $R^5$ corresponds to each of all the substituents shown in Table 4-1.

Table 4-60

Table 4-60 represents 268 compounds wherein, in the general formula (I), $R^1$ represents Et, $R^2$ does H, $R^3$ does $CF_3$, $R^4$ does H, Y does $CH_2$, and $R^5$ corresponds to each of all the substituents shown in Table 4-1.

Table 4-61

Table 4-61 represents 268 compounds wherein, in the general formula (I), $R^1$ represents Et, $R^2$ does H, $R^3$ does CN, $R^4$ does H, Y does $CH_2$, and $R^5$ corresponds to each of all the substituents shown in Table 4-1.

Table 4-62

Table 4-62 represents 268 compounds wherein, in the general formula (I), $R^1$ represents Et, $R^2$ does H, $R^3$ does COOMe, $R^4$ does H, Y does $CH_2$, and $R^5$ corresponds to each of all the substituents shown in Table 4-1.

Table 4-63

Table 4-63 represents 268 compounds wherein, in the general formula (I), $R^1$ represents Et, $R^2$ does H, $R^3$ does CONHMe, $R^4$ does H, Y does $CH_2$, and $R^5$ corresponds to each of all the substituents shown in Table 4-1.

Table 4-64

Table 4-64 represents 268 compounds wherein, in the general formula (I), $R^1$ represents Et, $R^2$ does H, $R^3$

does $CONMe_2$, $R^4$ does H, Y does $CH_2$, and $R^5$ corresponds to each of all the substituents shown in Table 4-1.

Table 4-65

Table 4-65 represents 268 compounds wherein, in the general formula (I), $R^1$ represents Et, $R^2$ does H, $R^3$ does SMe, $R^4$ does H, Y does $CH_2$, and $R^5$ corresponds to each of all the substituents shown in Table 4-1.

Table 4-66

Table 4-66 represents 268 compounds wherein, in the general formula (I), $R^1$ represents Et, $R^2$ does H, $R^3$ does SPh, $R^4$ does H, Y does $CH_2$, and $R^5$ corresponds to each of all the substituents shown in Table 4-1.

Table 4-67

Table 4-67 represents 268 compounds wherein, in the general formula (I), $R^1$ represents Et, $R^2$ does H, $R^3$ does OH, $R^4$ does H, Y does $CH_2$, and $R^5$ corresponds to each of all the substituents shown in Table 4-1.

Table 4-68

Table 4-68 represents 268 compounds wherein, in the general formula (I), $R^1$ represents Et, $R^2$ does H, $R^3$ does OMe, $R^4$ does H, Y does $CH_2$, and $R^5$ corresponds to each of all the substituents shown in Table 4-1.

Table 4-69

Table 4-69 represents 268 compounds wherein, in the general formula (I), $R^1$ represents Et, $R^2$ does H, $R^3$ does OPh, $R^4$ does H, Y does $CH_2$, and $R^5$ corresponds to each of all the substituents shown in Table 4-1.

Table 4-70

Table 4-70 represents 268 compounds wherein, in the general formula (I), $R^1$ represents Et, $R^2$ does H, $R^3$ does $NMe_2$, $R^4$ does H, Y does $CH_2$, and $R^5$ corresponds to each of all the substituents shown in Table 4-1.

Table 4-71

Table 4-71 represents 268 compounds wherein, in the general formula (I), $R^1$ represents Et, $R^2$ does H, $R^3$ does Cl, $R^4$ does Me, Y does $CH_2$, and $R^5$ corresponds to each of all the substituents shown in Table 4-1.

Table 4-72

Table 4-72 represents 268 compounds wherein, in the general formula (I), $R^1$ represents Et, $R^2$ does H, $R^3$ does Cl, $R^4$ does Et, Y does $CH_2$, and $R^5$ corresponds to each of all the substituents shown in Table 4-1.

Table 4-73

Table 4-73 represents 268 compounds wherein, in the general formula (I), $R^1$ represents Et, $R^2$ does H, $R^3$ does Cl, $R^4$ does n-Pr, Y does $CH_2$, and $R^5$ corresponds to each of all the substituents shown in Table 4-1.

Table 4-74

Table 4-74 represents 268 compounds wherein, in the general formula (I), $R^1$ represents Et, $R^2$ does H, $R^3$ does Cl, $R^4$ does i-Pr, Y does $CH_2$, and $R^5$ corresponds to each of all the substituents shown in Table 4-1.

Table 4-75

Table 4-75 represents 268 compounds wherein, in the general formula (I), $R^1$ represents Et, $R^2$ does H, $R^3$ does Cl, $R^4$ does $CH_2CH=CH_2$, Y does $CH_2$, and $R^5$ corresponds to each of all the substituents shown in Table 4-1.

Table 4-76

Table 4-76 represents 268 compounds wherein, in the general formula (I), $R^1$ represents Et, $R^2$ does H, $R^3$ does Cl, $R^4$ does $CH_2C{\equiv}CH$, Y does $CH_2$, and $R^5$ corresponds to each of all the substituents shown in Table 4-1.

Table 4-77

Table 4-77 represents 268 compounds wherein, in the general formula (I), $R^1$ represents Et, $R^2$ does H, $R^3$ does Cl, $R^4$ does $CH_2CF_3$, Y does $CH_2$, and $R^5$ corresponds to each of all the substituents shown in Table 4-1.

Table 4-78

Table 4-78 represents 268 compounds wherein, in the general formula (I), $R^1$ represents Et, $R^2$ does H, $R^3$ does Cl, $R^4$ does $CH_2CN$, Y does $CH_2$, and $R^5$ corresponds to each of all the substituents shown in Table 4-1.

Table 4-79

Table 4-79 represents 268 compounds wherein, in the general formula (I), $R^1$ represents Et, $R^2$ does H, $R^3$ does Cl, $R^4$ does $CH_2OMe$, Y does $CH_2$, and $R^5$ corresponds to each of all the substituents shown in Table 4-1.

Table 4-80

Table 4-80 represents 268 compounds wherein, in the general formula (I), $R^1$ represents Et, $R^2$ does H, $R^3$ does Cl, $R^4$ does $CH_2OEt$, Y does $CH_2$, and $R^5$ corresponds to each of all the substituents shown in Table 4-1.

Table 4-81

Table 4-81 represents 268 compounds wherein, in the general formula (I), $R^1$ represents Et, $R^2$ does H, $R^3$ does Cl, $R^4$ does $CH_2SMe$, Y does $CH_2$, and $R^5$ corresponds to each of all the substituents shown in Table 4-1.

Table 4-82

Table 4-82 represents 268 compounds wherein, in the general formula (I), $R^1$ represents Et, $R^2$ does H, $R^3$ does Cl, $R^4$ does $CH_2COOEt$, Y does $CH_2$, and $R^5$ corresponds to each of all the substituents shown in Table 4-1.

Table 4-83

Table 4-83 represents 268 compounds wherein, in the general formula (I), $R^1$ represents Et, $R^2$ does H, $R^3$ does Cl, $R^4$ does COOMe, Y does $CH_2$, and $R^5$ corresponds to each of all the substituents shown in Table 4-1.

Table 4-84

Table 4-84 represents 268 compounds wherein, in the general formula (I), $R^1$ represents Et, $R^2$ does H, $R^3$ does Cl, $R^4$ does CONHMe, Y does $CH_2$, and $R^5$ corresponds to each of all the substituents shown in Table 4-1.

Table 4-85

Table 4-85 represents 268 compounds wherein, in the general formula (I), $R^1$ represents Et, $R^2$ does H, $R^3$ does Cl, $R^4$ does $CONMe_2$, Y does $CH_2$, and $R^5$ corresponds to each of all the substituents shown in Table 4-1.

Table 4-86

Table 4-86 represents 268 compounds wherein, in the general formula (I), $R^1$ represents Et, $R^2$ does H, $R^3$ does Cl, $R^4$ does Ac, Y does $CH_2$, and $R^5$ corresponds to each of all the substituents shown in Table 4-1.

Table 4-87

Table 4-87 represents 268 compounds wherein, in the general formula (I), $R^1$ represents Et, $R^2$ does H, $R^3$

does Cl, $R^4$ does SPh, Y does $CH_2$, and $R^5$ corresponds to each of all the substituents shown in Table 4-1.

Table 4-88

Table 4-88 represents 268 compounds wherein, in the general formula (I), $R^1$ represents Et, $R^2$ does H, $R^3$ does Cl, $R^4$ does $CH_2$-c-Pr, Y does $CH_2$, and $R^5$ corresponds to each of all the substituents shown in Table 4-1.

Table 4-89

Table 4-89 represents 268 compounds wherein, in the general formula (I), $R^1$ represents Et, $R^2$ does H, $R^3$ does Cl, $R^4$ does H, Y does CHMe, and $R^5$ corresponds to each of all the substituents shown in Table 4-1.

Table 4-90

Table 4-90 represents 268 compounds wherein, in the general formula (I), $R^1$ represents Et, $R^2$ does H, $R^3$ does Cl, $R^4$ does H, Y does $CMe_2$, and $R^5$ corresponds to each of all the substituents shown in Table 4-1.

Table 4-91

Table 4-91 represents 268 compounds wherein, in the general formula (I), $R^1$ represents Et, $R^2$ does H, $R^3$ does Cl, $R^4$ does H, Y does CHOMe, and $R^5$ corresponds to each of all the substituents shown in Table 4-1.

Table 4-92

Table 4-92 represents 268 compounds wherein, in the general formula (I), $R^1$ represents Et, $R^2$ does H, $R^3$ does Cl, $R^4$ does H, Y does CHMe, and $R^5$ corresponds to each of all the substituents shown in Table 4-1.

Table 4-93

Table 4-93 represents 268 compounds wherein, in the general formula (I), $R^1$ represents Et, $R^2$ does H, $R^3$ does Cl, $R^4$ does H, Y does $C(CH_2)_2$, and $R^5$ corresponds to each of all the substituents shown in Table 4-1.

Table 4-94

Table 4-94 represents 268 compounds wherein, in the general formula (I), $R^1$ represents Et, $R^2$ does H, $R^3$ does Cl, $R^4$ does H, Y does $CH_2CH_2$, and $R^5$ corresponds to each of all the substituents shown in Table 4-1.

Table 4-95

Table 4-95 represents 268 compounds wherein, in the general formula (I), $R^1$ represents Et, $R^2$ does H, $R^3$ does Cl, $R^4$ does H, Y does CH=CH, and $R^5$ corresponds to each of all the substituents shown in Table 4-1.

Table 4-96

Table 4-96 represents 268 compounds wherein, in the general formula (I), $R^1$ represents Et, $R^2$ does H, $R^3$ does Cl, $R^4$ does H, Y does $COCH_2$, and $R^5$ corresponds to each of all the substituents shown in Table 4-1.

Table 4-97

Table 4-97 represents 268 compounds wherein, in the general formula (I), $R^1$ represents Et, $R^2$ does H, $R^3$ does Cl, $R^4$ does H, Y does C(OMe)=CH, and $R^5$ corresponds to each of all the substituents shown in Table 4-1.

Table 4-98

Table 4-98 represents 268 compounds wherein, in the general formula (I), $R^1$ represents Et, $R^2$ does H, $R^3$ does Cl, $R^4$ does H, Y does C≡C, and $R^5$ corresponds to each of all the substituents shown in Table 4-1.

Table 4-99

Table 4-99 represents 268 compounds wherein, in the general formula (I), $R^1$ represents Et, $R^2$ does H, $R^3$ does Cl, $R^4$ does H, Y does $CH(OH)CH_2$, and $R^5$ corresponds to each of all the substituents shown in Table 4-1.

Table 4-100

Table 4-100 represents 268 compounds wherein, in the general formula (I), $R^1$ represents Et, $R^2$ does H, $R^3$ does Cl, $R^4$ does H, Y does $CH(OMe)CH_2$, and $R^5$ corresponds to each of all the substituents shown in Table 4-1.

Table 4-101

Table 4-101 represents 268 compounds wherein, in the general formula (I), $R^1$ represents Et, $R^2$ does H, $R^3$ does Cl, $R^4$ does H, Y does $OCH_2$, and $R^5$ corresponds to each of all the substituents shown in Table 4-1.

Table 4-102

Table 4-102 represents 268 compounds wherein, in the general formula (I), $R^1$ represents Et, $R^2$ does H, $R^3$ does Cl, $R^4$ does H, Y does OCH(Me), and $R^5$ corresponds to each of all the substituents shown in Table 4-1.

Table 4-103

Table 4-103 represents 268 compounds wherein, in the general formula (I), $R^1$ represents Et, $R^2$ does H, $R^3$ does Cl, $R^4$ does H, Y does $OCH_2CH_2$, and $R^5$ corresponds to each of all the substituents shown in Table 4-1.

Table 4-104

Table 4-104 represents 268 compounds wherein, in the general formula (I), $R^1$ represents Et, $R^2$ does H, $R^3$ does Cl, $R^4$ does H, Y does $OCH_2CH=CH$, and $R^5$ corresponds to each of all the substituents shown in Table 4-1.

Table 4-105

Table 4-105 represents 268 compounds wherein, in the general formula (I), $R^1$ represents Et, $R^2$ does H, $R^3$ does Cl, $R^4$ does H, Y does $SCH_2$, and $R^5$ corresponds to each of all the substituents shown in Table 4-1.

Table 4-106

Table 4-106 represents 268 compounds wherein, in the general formula (I), $R^1$ represents Et, $R^2$ does H, $R^3$ does Cl, $R^4$ does H, Y does $SCH_2CH_2$, and $R^5$ corresponds to each of all the substituents shown in Table 4-1.

Table 4-107

Table 4-107 represents 268 compounds wherein, in the general formula (I), $R^1$ represents Et, $R^2$ does H, $R^3$ does Cl, $R^4$ does H, Y does $NHCH_2$, and $R^5$ corresponds to each of all the substituents shown in Table 4-1.

Table 4-108

Table 4-108 represents 268 compounds wherein, in the general formula (I), $R^1$ represents Et, $R^2$ does H, $R^3$ does Cl, R4 does H, Y does $NHCH_2CH_2$, and $R^5$ corresponds to each of all the substituents shown in Table 4-1.

Table 4-109

Table 4-109 represents 268 compounds wherein, in the general formula (I), $R^1$ represents Et, $R^2$ does H, $R^3$ does Cl, $R^4$ does H, Y does $N(Me)CH_2$, and $R^5$ corresponds to each of all the substituents shown in Table 4-1.

Table 4-110

Table 4-110 represents 268 compounds wherein, in the general formula (I), $R^1$ represents Et, $R^2$ does H, $R^3$

does Cl, $R^4$ does H, Y does $N(Me)CH_2CH_2$, and $R^5$ corresponds to each of all the substituents shown in Table 4-1.

Table 4-111

Table 4-111 represents 268 compounds wherein, in the general formula (I), $R^1$ represents Et, $R^2$ does H, $R^3$ does Cl, $R^4$ does H, Y does C=O, and $R^5$ corresponds to each of all the substituents shown in Table 4-1.

Table 4-112

Table 4-112 represents 268 compounds wherein, in the general formula (I), $R^1$ represents Et, $R^2$ does H, $R^3$ does Cl, $R^4$ does H, Y does C=NOMe, and $R^5$ corresponds to each of all the substituents shown in Table 4-1.

Table 4-113

Table 4-113 represents 268 compounds wherein, in the general formula (I), $R^1$ represents Et, $R^2$ does H, $R^3$ does Cl, $R^4$ does H, Y does $C((CH_2)_2)CH_2$, and $R^5$ corresponds to each of all the substituents shown in Table 4-1.

Table 4-114

Table 4-114 represents 268 compounds wherein, in the general formula (I), $R^1$ represents Et, $R^2$ does H, $R^3$ does Cl, $R^4$ does H, Y does CH(OH), and $R^5$ corresponds to each of all the substituents shown in Table 4-1.

Table 4-115

Table 4-115 represents 268 compounds wherein, in the general formula (I), $R^1$ represents Et, $R^2$ does H, $R^3$ does Cl, $R^4$ does H, Y does CH(OMe), and $R^5$ corresponds to each of all the substituents shown in Table 4-1.

Table 4-116

Table 4-116 represents 268 compounds wherein, in the general formula (I), $R^1$ represents Et, $R^2$ does H, $R^3$ does Cl, $R^4$ does H, Y does $CH(OCH_2OEt)$, and $R^5$ corresponds to each of all the substituents shown in Table 4-1.

Table 4-117

Table 4-117 represents 268 compounds wherein, in the general formula (I), $R^1$ represents Et, $R^2$ does H, $R^3$ does Cl, $R^4$ does H, Y does $CH(OCH_2C=CH_2)$, and $R^5$ corresponds to each of all the substituents shown in Table 4-1.

Table 4-118

Table 4-118 represents 268 compounds wherein, in the general formula (I), $R^1$ represents Et, $R^2$ does H, $R^3$ does Cl, $R^4$ does H, Y does $CH(OCH_2C{\equiv}CH)$, and $R^5$ corresponds to each of all the substituents shown in Table 4-1.

The general formula (I-4):

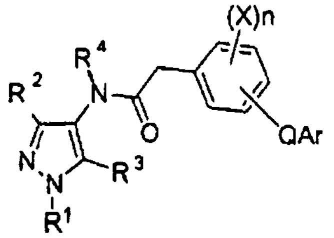

(I-4)

Table 5-1

| $R^1$ | $R^2$ | $R^3$ | $R^4$ | Bond | (X)n | Q | Ar |
|---|---|---|---|---|---|---|---|
| Me | Me | Cl | H | 4-position | H | 0 | pyridine-2-yl |
| Me | Me | Cl | H | 4-position | H | 0 | 2-F-pyridine-2-yl |
| Me | Me | Cl | H | 4-position | H | 0 | 5-F-pyridine-2-yl |
| Me | Me | Cl | H | 4-position | H | 0 | 2-Cl-pyridine-5-yl |
| Me | Me | Cl | H | 4-position | H | 0 | 5-Cl-pyridine-2-yl |
| Me | Me | Cl | H | 4-position | H | 0 | 2-Br-pyridine-5-yl |
| Me | Me | Cl | H | 4-position | H | 0 | 5-Br-pyridine-2-yl |
| Me | Me | Cl | H | 4-position | H | 0 | 2-I-pyridine-5-yl |
| Me | Me | Cl | H | 4-position | H | 0 | 5-I-pyridirie-2-yl |
| Me | Me | Cl | H | 4-position | H | 0 | 5-$CF_3$-pyridine-2-yl |
| Me | Me | Cl | H | 4-position | H | 0 | 5-$NO_2$-pyridine-2-yl |
| Me | Me | Cl | H | 4-position | H | 0 | 5-CN-pyridirie-2-yl |
| Me | Me | Cl | H | 4-position | H | 0 | 5-$CF(CF_3)_2$-pyridine-2-yl |
| Me | Me | Cl | H | 4-position | H | 0 | 5-$CH(CF_3)_2$-pyridine-2-yl |
| Me | Me | Cl | H | 4-position | H | 0 | 5-$CF_2CF_3$-pyridine-2-yl |
| Me | Me | Cl | H | 4-position | H | 0 | 5-NHMe-pyridine-2-yl |
| Me | Me | Cl | H | 4-position | H | 0 | 5-$NMe_2$-pyridine-2-yl |
| Me | Me | Cl | H | 4-position | H | 0 | 5-NHAc-pyridine-2-yl |
| Me | Me | Cl | H | 4-position | H | 0 | 5-NHCOOMe-pyridine-2-yl |
| Me | Me | Cl | H | 4-position | H | 0 | 5-$NHCH_2COOMe$-pyridine-2-yl |
| Me | Me | Cl | H | 4-position | H | 0 | 5-$NHCOCF_3$-pyridine-2-yl |
| Me | Me | Cl | H | 4-position | H | 0 | 5-$NHCOCH_2Cl$-pyridine-2-yl |
| Me | Me | Cl | H | 4-position | H | 0 | 5-$NRSO_2Me$-pyridine-2-yl |
| Me | Me | Cl | H | 4-position | H | 0 | 5-$NHSO_2CF_3$-pyridine-2-yl |
| Me | Me | Cl | H | 4-position | H | 0 | 5-SH-pyridine-2-yl |
| Me | Me | Cl | H | 4-position | H | 0 | 5-SMe-pyridine-2-yl |
| Me | Me | Cl | H | 4-position | H | 0 | 5-SOMe-pyridine-2-yl |
| Me | Me | Cl | H | 4-position | H | 0 | 5-$SO_2Me$-pyridine-2-yl |
| Me | Me | Cl | H | 4-position | H | 0 | 5-$SCF_3$-pyridine-2-yl |
| Me | Me | Cl | H | 4-position | H | 0 | 5-$SOCF_3$-pyridine-2-yl |
| Me | Me | Cl | H | 4-position | H | 0 | 5-$SO_2CF_3$-pyridine-2-yl |
| Me | Me | Cl | H | 4-position | H | 0 | 5-OH-pyridine-2-yl |
| Me | Me | Cl | H | 4-position | H | 0 | 5-OMe-pyridine-2-yl |
| Me | Me | Cl | H | 4-position | H | 0 | 5-$OCF_3$-pyridine-2-yl |
| Me | Me | Cl | H | 4-position | H | 0 | 5-$OCHF_2$-pyridine-2-yl |
| Me | Me | Cl | H | 4-position | H | 0 | 5-$OCF_2CHF_2$-pyridine-2-yl |
| Me | Me | Cl | H | 4-position | H | 0 | 5-$OCH(CF_3)_2$-pyridine-2-yl |
| Me | Me | Cl | H | 4-position | H | 0 | 5-$OCH_2CF_3$-pyridine-2-yl |
| Me | Me | Cl | H | 4-position | H | 0 | 5-OAc-pyridine-2-yl |
| Me | Me | Cl | H | 4-position | H | 0 | 5-OCOOMe-pyridine-2-yl |
| Me | Me | Cl | H | 4-position | H | 0 | 5-COOH-pyridine-2-yl |
| Me | Me | Cl | H | 4-position | H | 0 | 5-COOMe-pyridine-2-yl |
| Me | Me | Cl | H | 4-position | H | 0 | 5-$CONH_2$-pyridine-2-yl |
| Me | Me | Cl | H | 4-position | H | 0 | 5-CONHMe-pyridine-2-yl |
| Me | Me | Cl | H | 4-position | H | 0 | 5-$CON(Me)_2$-pyridine-2-yl |
| Me | Me | Cl | H | 4-position | H | 0 | 5-Ac-pyridine-2-yl |
| Me | Me | Cl | H | 4-position | H | 0 | 5-C(Me)=NOH-pyridirie-2-yl |
| Me | Me | Cl | H | 4-position | H | 0 | 5-C(Me)=NOMe-pyridine-2-yl |
| Me | Me | Cl | H | 4-position | H | 0 | 5-C(Me)=$NOCH_2PhMe$-pyridine-2-yl |

Table 5-1 (continued)

| $R^1$ | $R^2$ | $R^3$ | $R^4$ | Bond | (X)n | Q | Ar |
|---|---|---|---|---|---|---|---|
| Me | Me | Cl | H | 4-position | H | 0 | 3,5-$F_2$-pyridine-2-yl |
| Me | Me | Cl | H | 4-position | H | 0 | 3,5-$Cl_2$-pyridine-2-yl |
| Me | Me | Cl | H | 4-position | H | 0 | 3,5-$Br_2$-pyridine-2-yl |
| Me | Me | Cl | H | 4-position | H | 0 | 3,5-$I_2$-pyridine-2-yl |
| Me | Me | Cl | H | 4-position | H | 0 | 3-F-5-Cl-pyridine-2-yl |
| Me | Me | Cl | H | 4-position | H | 0 | 3-Br-5-Cl-pyridine-2-yl |
| Me | Me | Cl | H | 4-position | H | 0 | 3-F-5-$CF_3$-pyridine-2-yl |
| Me | Me | Cl | H | 4-position | H | 0 | 3-Cl-5-$CF_3$-pyridine-2-yl |
| Me | Me | Cl | H | 4-position | H | 0 | 3-Br-5-$CF_3$-pyridine-2-yl |
| Me | Me | Cl | H | 4-position | H | 0 | 3,5-$(CF_3)_2$-pyridine-2-yl |
| Me | Me | Cl | H | 4-position | H | 0 | 4,6-$(CF_3)_2$-pyridine-2-yl |
| Me | Me | Cl | H | 4-position | H | 0 | 3-Cl-5-CN-pyridine-2-yl |
| Me | Me | Cl | H | 4-position | H | 0 | 2-$CF(CF_3)_2$-pyridine-2-yl |
| Me | Me | Cl | H | 4-position | H | 0 | 2-$CH(CF_3)_2$-pyridine-2-yl |
| Me | Me | Cl | H | 4-position | H | 0 | 2-$CF_2CF_3$-pyridine-2-yl |
| Me | Me | Cl | H | 4-position | H | 0 | 2-$CF_3$-pyridine-5-yl |
| Me | Me | Cl | H | 4-position | H | 0 | 2-$CF_3$-pyridine-6-yl |
| Me | Me | Cl | H | 4-position | H | 0 | 6-F-pyridazine-3-yl |
| Me | Me | Cl | H | 4-position | H | 0 | 6-Cl-pyridazine-3-yl |
| Me | Me | Cl | H | 4-position | H | 0 | 6-Br-pyridazine-3-yl |
| Me | Me | Cl | H | 4-position | H | 0 | 6-I-pyridazine-3-yl |
| Me | Me | Cl | H | 4-position | H | 0 | 6-$CF_3$-pyridazine-3-yl |
| Me | Me | Cl | H | 4-position | H | 0 | 6-CN-pyridazine-3-yl |
| Me | Me | Cl | H | 4-position | H | 0 | 6-COOH-pyridazine-3-yl |
| Me | Me | Cl | H | 4-position | H | 0 | 6-COOMe-pyridazine-3-yl |
| Me | Me | Cl | H | 4-position | H | 0 | 6-CONHMe-pyridazine-3-yl |
| Me | Me | Cl | H | 4-position | H | 0 | 6-$CON(Me)_2$-pyridazine-3-yl |
| Me | Me | Cl | H | 4-position | H | 0 | 6-OH-pyridazine-3-yl |
| Me | Me | Cl | H | 4-position | H | 0 | 6-OMe-pyridazine-3-yl |
| Me | Me | Cl | H | 4-position | H | 0 | 6-$OCF_3$-pyridazine-3-yl |
| Me | Me | Cl | H | 4-position | H | 0 | 6-$OCHF_2$-pyridazine-3-yl |
| Me | Me | Cl | H | 4-position | H | 0 | 6-$OCF_2CHF_2$-pyridazine-3-yl |
| Me | Me | Cl | H | 4-position | H | 0 | 6-$CF(CF_3)_2$-pyridazine-3-yl |
| Me | Me | Cl | H | 4-position | H | 0 | 6-$CH(CF_3)_2$-pyridazine-3-yl |
| Me | Me | Cl | H | 4-position | H | 0 | 6-$CF_2CF_3$-pyridazine-3-yl |
| Me | Me | Cl | H | 4-position | H | 0 | pyrimidine-2-yl |
| Me | Me | Cl | H | 4-position | H | 0 | 5-F-pyrimidine-2-yl |
| Me | Me | Cl | H | 4-position | H | 0 | 5-Cl-pyrimidine-2-yl |
| Me | Me | Cl | H | 4-position | H | 0 | 5-Br-pyrimidine-2-yl |
| Me | Me | Cl | H | 4-position | H | 0 | 5-$CF_3$-pyrimidine-2-yl |
| Me | Me | Cl | H | 4-position | H | 0 | 5-$CF(CF_3)_2$-pyrimidine-2-yl |
| Me | Me | Cl | H | 4-position | H | 0 | 5-$CH(CF_3)_2$-pyrimidirie-2-yl |
| Me | Me | Cl | H | 4-position | H | 0 | 5-$CF_2CF_3$-pyrimidine-2-yl |
| Me | Me | Cl | H | 4-position | H | 0 | 5-$NO_2$-pyrimidine-2-yl |
| Me | Me | Cl | H | 4-position | H | 0 | 5-CN-pyrimidine-2-yl |
| Me | Me | Cl | H | 4-position | H | 0 | pyrazine-2-yl |
| Me | Me | Cl | H | 4-position | H | 0 | 5-$CF_3$-pyrazine-2-yl |
| Me | Me | Cl | H | 4-position | H | 0 | 4,6-$Cl_2$-triazine-2-yl |
| Me | Me | Cl | H | 4-position | H | 0 | thiophene-2-yl |
| Me | Me | Cl | H | 4-position | H | 0 | 5-Cl-thiophene-2-yl |

Table 5-1 (continued)

| $R^1$ | $R^2$ | $R^3$ | $R^4$ | Bond | (X)n | Q | Ar |
|---|---|---|---|---|---|---|---|
| Me | Me | Cl | H | 4-position | H | 0 | 5-$CF_3$-thiophene-2-yl |
| Me | Me | Cl | H | 4-position | H | 0 | 1-Me-pyrazole-5-yl |
| Me | Me | Cl | H | 4-position | H | 0 | 1-Me-3-Cl-pyrazole-5-yl |
| Me | Me | Cl | H | 4-position | H | 0 | 1-Me-3-$CF_3$-pyrazole-5-yl |
| Me | Me | Cl | H | 4-position | H | 0 | 3-$CF_3$-isoxazole-5-yl |
| Me | Me | Cl | H | 4-position | H | 0 | 5-$CF_3$-isoxazole-3-yl |
| Me | Me | Cl | H | 4-position | H | 0 | 3-$CF_3$-isothiazole-5-yl |
| Me | Me | Cl | H | 4-position | H | 0 | 5-Cl-thiazole-2-yl |
| Me | Me | Cl | H | 4-position | H | 0 | 5-Br-thiazole-2-yl |
| Me | Me | Cl | H | 4-position | H | 0 | 5-$NO_2$-thiazole-2-yl |
| Me | Me | Cl | H | 4-position | H | 0 | 5-CN-thiazole-2-yl |
| Me | Me | Cl | H | 4-position | H | 0 | 4-Cl-thiazole-2-yl |
| Me | He | Cl | H | 4-position | H | 0 | 4-Br-thiazole-2-yl |
| Me | Me | Cl | H | 4-position | H | 0 | 4-$CF_3$-thiazole-2-yl |
| Me | He | Cl | H | 4-position | H | 0 | 1-Me-4-Cl-imidazole-2-yl |
| Me | Me | Cl | H | 4-position | H | 0 | 1-Me-4-Br-imidazole-2-yl |
| Me | Me | Cl | H | 4-position | H | 0 | 1-Me-3-Cl-triazole-5-yl |
| Me | Me | Cl | H | 4-position | H | 0 | quinoline-2-yl |
| Me | Me | Cl | H | 4-position | H | 0 | benzothiazole-2-yl |
| Me | Me | Cl | H | 4-position | H | 0 | 1-Me-benzimidazole-2-yl |
| Me | Me | Cl | H | 4-position | H | 0 | benzoxazole-2-yl |
| Me | Me | Cl | H | 4-position | H | 0 | 1-Me-indole-2-yl |
| Me | Me | Cl | H | 4-position | H | 0 | benzofuran-2-yl |
| Me | Me | Cl | H | 4-position | H | 0 | benzothiophene-2-yl |
| Note: "Bond" in the Table means the bonded position of the group -Q-Ar. | | | | | | | |

Table 5-2

Table 5-2 represents 132 compounds wherein, in the general formula (I-4), $R^1$ represents Me, $R^2$ does Et, $R^3$ does Cl, $R^4$ does H, Q does an oxygen atom and the bonded position thereof being a 4-position, (X)n does H, and Ar corresponds to each of all the substituents shown in Table 5-1.

Table 5-3

Table 5-3 represents 132 compounds wherein, in the general formula (I-4), $R^1$ represents Me, $R^2$ does i-Pr, $R^3$ does Cl, $R^4$ does H, Q does an oxygen atom and the bonded position thereof being a 4-position, (X)n does H, and Ar corresponds to each of all the substituents shown in Table 5-1.

Table 5-4

Table 5-4 represents 132 compounds wherein, in the general formula (I-4), $R^1$ represents Me, $R^2$ does n-Pr, $R^3$ does Cl, $R^4$ does H, Q does an oxygen atom and the bonded position thereof being a 4-position, (X)n does H, and Ar corresponds to each of all the substituents shown in Table 5-1.

Table 5-5

Table 5-5 represents 132 compounds wherein, in the general formula (I-4), $R^1$ represents Me, $R^2$ does t-Bu, $R^3$ does Cl, $R^4$ does H, Q does an oxygen atom and the bonded position thereof being a 4-position, (X)n does H, and Ar corresponds to each of all the substituents shown in Table 5-1.

Table 5-6

Table 5-6 represents 132 compounds wherein, in the general formula (I-4), $R^1$ represents Me, $R^2$ does n-Bu, $R^3$ does Cl, $R^4$ does H, Q does an oxygen atom and the bonded position thereof being a 4-position, (X)n does H, and Ar corresponds to each of all the substituents shown in Table 5-1.

Table 5-7

Table 5-7 represents 132 compounds wherein, in the general formula (1-4), $R^1$ represents Me, $R^2$ does $CF_3$, $R^3$ does Cl, $R^4$ does H, Q does an oxygen atom and the bonded position thereof being a 4-position, (X)n does H, and Ar corresponds to each of all the substituents shown in Table 5-1.

Table 5-8

Table 5-8 represents 132 compounds wherein, in the general formula (I-4), $R^1$ represents Me, $R^2$ does OMe, $R^3$ does Cl, $R^4$ does H, Q does an oxygen atom and the bonded position thereof being a 4-position, (X)n does H, and Ar corresponds to each of all the substituents shown in Table 5-1.

Table 5-9

Table 5-9 represents 132 compounds wherein, in the general formula (I-4), $R^1$ represents Me, $R^2$ does $OCHF_2$, $R^3$ does Cl, $R^4$ does H, Q does an oxygen atom and the bonded position thereof being a 4-position, (X)n does H, and Ar corresponds to each of all the substituents shown in Table 5-1.

Table 5-10

Table 5-10 represents 132 compounds wherein, in the general formula (I-4), $R^1$ represents Me, $R^2$ does O-i-P $R^3$ does Cl, $R^4$ does H, Q does an oxygen atom and the bonded position thereof being a 4-position, (X)n does H, and Ar corresponds to each of all the substituents shown in Table 5-1.

Table 5-11

Table 5-11 represents 132 compounds wherein, in the general formula (I-4), $R^1$ represents Me, $R^2$ does Cl, $R^3$ does Cl, $R^4$ does H, Q does an oxygen atom and the bonded position thereof being a 4-position, (X)n does H, and Ar corresponds to each of all the substituents shown in Table 5-1.

Table 5-12

Table 5-12 represents 132 compounds wherein, in the general formula (I-4), $R^1$ represents Me, $R^2$ does Ph, $R^3$ does Cl, $R^4$ does H, Q does an oxygen atom and the bonded position thereof being a 4-position, (X)n does H, and Ar corresponds to each of all the substituents shown in Table 5-1.

Table 5-13

Table 5-13 represents 132 compounds wherein, in the general formula (I-4), $R^1$ represents Me, $R^2$ does 2-Cl-Ph, $R^3$ does Cl, $R^4$ does H, Q does an oxygen atom and the bonded position thereof being a 4-position, (X)n does H, and Ar corresponds to each of all the substituents shown in Table 5-1.

Table 5-14

Table 5-14 represents 132 compounds wherein, in the general formula (I-4), $R^1$ represents Me, $R^2$ does 3-Cl-Ph, $R^3$ does Cl, $R^4$ does H, Q does an oxygen atom and the bonded position thereof being a 4-position, (X)n does H, and Ar corresponds to each of all the substituents shown in Table 5-1.

Table 5-15

Table 5-15 represents 132 compounds wherein, in the general formula (I-4), $R^1$ represents Me, $R^2$ does 4-Cl-Ph, $R^3$ does Cl, $R^4$ does H, Q does an oxygen atom and the bonded position thereof being a 4-position, (X)n does H,

and Ar corresponds to each of all the substituents shown in Table 5-1.

Table 5-16

Table 5-16 represents 132 compounds wherein, in the general formula (I-4), $R^1$ represents Me, $R^2$ does 2,4-$Cl_2$-Ph, $R^3$ does Cl, $R^4$ does H, Q does an oxygen atom and the bonded position thereof being a 4-position, (X)n does H, and Ar corresponds to each of all the substituents shown in Table 5-1.

Table 5-17

Table 5-17 represents 132 compounds wherein, in the general formula (I-4), $R^1$ represents Me, $R^2$ does 2-Me-Ph, $R^3$ does Cl, $R^4$ does H, Q does an oxygen atom and the bonded position thereof being a 4-position, (X)n does H, and Ar corresponds to each of all the substituents shown in Table 5-1.

Table 5-18

Table 5-18 represents 132 compounds wherein, in the general formula (I-4), $R^1$ represents Me, $R^2$ does 3-Me-Ph, $R^3$ does Cl, $R^4$ does H, Q does an oxygen atom and the bonded position thereof being a 4-position, (X)n does H, and Ar corresponds to each of all the substituents shown in Table 5-1.

Table 5-19

Table 5-19 represents 132 compounds wherein, in the general formula (I-4), $R^1$ represents Me, $R^2$ does 4-Me-Ph, $R^3$ does Cl, $R^4$ does H, Q does an oxygen atom and the bonded position thereof being a 4-position, (X)n does H, and Ar corresponds to each of all the substituents shown in Table 5-1.

Table 5-20

Table 5-20 represents 132 compounds wherein, in the general formula (I-4), $R^1$ represents Me, $R^2$ does 2-OMe-Ph, $R^3$ does Cl, $R^4$ does H, Q does an oxygen atom and the bonded position thereof being a 4-position, (X)n does H, and Ar corresponds to each of all the substituents shown in Table 5-1.

Table 5-21

Table 5-21 represents 132 compounds wherein, in the general formula (I-4), $R^1$ represents Me, $R^2$ does 3-OMe-Ph, $R^3$ does Cl, $R^4$ does H, Q does an oxygen atom and the bonded position thereof being a 4-position, (X)n does H, and Ar corresponds to each of all the substituents shown in Table 5-1.

Table 5-22

Table 5-22 represents 132 compounds wherein, in the general formula (I-4), $R^1$ represents Me, $R^2$ does 4-OMe-Ph, $R^3$ does Cl, $R^4$ does H, Q does an oxygen atom and the bonded position thereof being a 4-position, (X)n does H, and Ar corresponds to each of all the substituents shown in Table 5-1.

Table 5-23

Table 5-23 represents 132 compounds wherein, in the general formula (I-4), $R^1$ represents Me, $R^2$ does H, $R^3$ does Cl, $R^4$ does H, Q does an oxygen atom and the bonded position thereof being a 4-position, (X)n does 3-OMe, and Ar corresponds to each of all the substituents shown in Table 5-1.

Table 5-24

Table 5-24 represents 132 compounds wherein, in the general formula (I-4), $R^1$ represents H, $R^2$ does H, $R^3$ does Cl, $R^4$ does H, Q does an oxygen atom and the bonded position thereof being a 4-position, (X)n does 3-OMe, and Ar corresponds to each of all the substituents shown in Table 5-1.

Table 5-25

Table 5-25 represents 132 compounds wherein, in the general formula (I-4), $R^1$ represents Et, $R^2$ does H, $R^3$ does Cl, $R^4$ does H, Q does an oxygen atom and the bonded position thereof being a 4-position, (X)n does 3-OMe, and Ar corresponds to each of all the substituents shown in Table 5-1.

Table 5-26

Table 5-26 represents 132 compounds wherein, in the general formula (I-4), $R^1$ represents i-Pr, $R^2$ does H, $R^3$ does Cl, $R^4$ does H, Q does an oxygen atom and the bonded position thereof being a 4-position, (X)n does 3-OMe, and Ar corresponds to each of all the substituents shown in Table 5-1.

Table 5-27

Table 5-27 represents 132 compounds wherein, in the general formula (I-4), $R^1$ represents n-Pr, $R^2$ does H, $R^3$ does Cl, $R^4$ does H, Q does an oxygen atom and the bonded position thereof being a 4-position, (X)n does 3-OMe, and Ar corresponds to each of all the substituents shown in Table 5-1.

Table 5-28

Table 5-28 represents 132 compounds wherein, in the general formula (I-4), $R^1$ represents t-Bu, $R^2$ does H, $R^3$ does Cl, $R^4$ does H, Q does an oxygen atom and the bonded position thereof being a 4-position, (X)n does 3-OMe, and Ar corresponds to each of all the substituents shown in Table 5-1.

Table 5-29

Table 5-29 represents 132 compounds wherein, in the general formula (I-4), $R^1$ represents n-Bu, $R^2$ does H, $R^3$ does Cl, $R^4$ does H, Q does an oxygen atom and the bonded position thereof being a 4-position, (X)n does 3-OMe, and Ar corresponds to each of all the substituents shown in Table 5-1.

Table 5-30

Table 5-30 represents 132 compounds wherein, in the general formula (I-4), $R^1$ represents $CH=CH_2$, $R^2$ does H, $R^3$ does Cl, $R^4$ does H, Q does an oxygen atom and the bonded position thereof being a 4-position, (X)n does 3-OMe, and Ar corresponds to each of all the substituents shown in Table 5-1.

Table 5-31

Table 5-31 represents 132 compounds wherein, in the general formula (I-4), $R^1$ represents $CH_2CH=CH_2$, $R^2$ does H, $R^3$ does Cl, $R^4$ does H, Q does an oxygen atom and the bonded position thereof being a 4-position, (X)n does 3-OMe, and Ar corresponds to each of all the substituents shown in Table 5-1.

Table 5-32

Table 5-32 represents 132 compounds wherein, in the general formula (I-4), $R^1$ represents $CH_2C\equiv CH$, $R^2$ does H, $R^3$ does Cl, $R^4$ does H, Q does an oxygen atom and the bonded position thereof being a 4-position, (X)n does 3-OMe, and Ar corresponds to each of all the substituents shown in Table 5-1.

Table 5-33

Table 5-33 represents 132 compounds wherein, in the general formula (I-4), $R^1$ represents $CH_2CN$, $R^2$ does H, $R^3$ does Cl, $R^4$ does H, Q does an oxygen atom and the bonded position thereof being a 4-position, (X)n does 3-OMe, and Ar corresponds to each of all the substituents shown in Table 5-1.

Table 5-34

Table 5-34 represents 132 compounds wherein, in the general formula (I-4), $R^1$ represents $CH_2CF_3$, $R^2$ does H, $R^3$ does Cl, $R^4$ does H, Q does an oxygen atom and the bonded position thereof being a 4-position, (X)n does

3-OMe, and Ar corresponds to each of all the substituents shown in Table 5-1.

Table 5-35

Table 5-35 represents 132 compounds wherein, in the general formula (I-4), $R^1$ represents $CH_2Ph$, $R^2$ does H, $R^3$ does Cl, $R^4$ does H, Q does an oxygen atom and the bonded position thereof being a 4-position, (X)n does 3-OMe, and Ar corresponds to each of all the substituents shown in Table 5-1.

Table 5-36

Table 5-36 represents 132 compounds wherein, in the general formula (I-4), $R^1$ represents Ph, $R^2$ does H, $R^3$ does Cl, $R^4$ does H, Q does an oxygen atom and the bonded position thereof being a 4-position, (X)n does 3-OMe, and Ar corresponds to each of all the substituents shown in Table 5-1.

Table 5-37

Table 5-37 represents 132 compounds wherein, in the general formula (I-4), $R^1$ represents 2-Cl-Ph, $R^2$ does H, $R^3$ does Cl, $R^4$ does H, Q does an oxygen atom and the bonded position thereof being a 4-position, (X)n does 3-OMe, and Ar corresponds to each of all the substituents shown in Table 5-1.

Table 5-38

Table 5-38 represents 132 compounds wherein, in the general formula (I-4), $R^1$ represents 3-Cl-Ph, $R^2$ does H, $R^3$ does Cl, $R^4$ does H, Q does an oxygen atom and the bonded position thereof being a 4-position, (X)n does 3-OMe, and Ar corresponds to each of all the substituents shown in Table 5-1.

Table 5-39

Table 5-39 represents 132 compounds wherein, in the general formula (I-4), $R^1$ represents 4-Cl-Ph, $R^2$ does H, $R^3$ does Cl, $R^4$ does H, Q does an oxygen atom and the bonded position thereof being a 4-position, (X)n does 3-OMe, and Ar corresponds to each of all the substituents shown in Table 5-1.

Table 5-40

Table 5-40 represents 132 compounds wherein, in the general formula (1-4), $R^1$ represents 2,4-$Cl_2$-Ph, $R^2$ does H, $R^3$ does Cl, $R^4$ does H, Q does an oxygen atom and the bonded position thereof being a 4-position, (X)n does 3-OMe, and Ar corresponds to each of all the substituents shown in Table 5-1.

Table 5-41

Table 5-41 represents 132 compounds wherein, in the general formula (I-4), $R^1$ represents Ac, $R^2$ does H, $R^3$ does Cl, $R^4$ does H, Q does an oxygen atom and the bonded position thereof being a 4-position, (X)n does 3-OMe, and Ar corresponds to each of all the substituents shown in Table 5-1.

Table 5-42

Table 5-42 represents 132 compounds wherein, in the general formula (I-4), $R^1$ represents COPh, $R^2$ does H, $R^3$ does Cl, $R^4$ does H, Q does an oxygen atom and the bonded position thereof being a 4-position, (X)n does 3-OMe, and Ar corresponds to each of all the substituents shown in Table 5-1.

Table 5-43

Table 5-43 represents 132 compounds wherein, in the general formula (I-4), $R^1$ represents $CH_2CH_2F$, $R^2$ does H, $R^3$ does Cl, $R^4$ does H, Q does an oxygen atom and the bonded position thereof being a 4-position, (X)n does 3-OMe, and Ar corresponds to each of all the substituents shown in Table 5-1.

Table 5-44

Table 5-44 represents 132 compounds wherein, in the general formula (I-4), $R^1$ represents $CH_2CH_2Cl$, $R^2$ does H, $R^3$ does Cl, $R^4$ does H, Q does an oxygen atom and the bonded position thereof being a 4-position, (X)n does 3-OMe, and Ar corresponds to each of all the substituents shown in Table 5-1.

Table 5-45

Table 5-45 represents 132 compounds wherein, in the general formula (I-4), $R^1$ represents $CH_2CH_2Br$, $R^2$ does H, $R^3$ does Cl, $R^4$ does H, Q does an oxygen atom and the bonded position thereof being a 4-position, (X)n does 3-OMe, and Ar corresponds to each of all the substituents shown in Table 5-1.

Table 5-46

Table 5-46 represents 132 compounds wherein, in the general formula (I-4), $R^1$ represents $CH_2CH_2OH$, $R^2$ does H, $R^3$ does Cl, $R^4$ does H, Q does an oxygen atom and the bonded position thereof being a 4-position, (X)n does 3-OMe, and Ar corresponds to each of all the substituents shown in Table 5-1.

Table 5-47

Table 5-47 represents 132 compounds wherein, in the general formula (I-4), $R^1$ represents $CH_2CH_2OMe$, $R^2$ does H, $R^3$ does Cl, $R^4$ does H, Q does an oxygen atom and the bonded position thereof being a 4-position, (X)n does 3-OMe, and Ar corresponds to each of all the substituents shown in Table 5-1.

Table 5-48

Table 5-48 represents 132 compounds wherein, in the general formula (I-4), $R^1$ represents $CH_2OMe$, $R^2$ does H, $R^3$ does Cl, $R^4$ does H, Q does an oxygen atom and the bonded position thereof being a 4-position, (X)n does 3-OMe, and Ar corresponds to each of all the substituents shown in Table 5-1.

Table 5-49

Table 5-49 represents 132 compounds wherein, in the general formula (I-4), $R^1$ represents $CH_2OEt$, $R^2$ does H, $R^3$ does Cl, $R^4$ does H, Q does an oxygen atom and the bonded position thereof being a 4-position, (X)n does 3-OMe, and Ar corresponds to each of all the substituents shown in Table 5-1.

Table 5-50

Table 5-50 represents 132 compounds wherein, in the general formula (I-4), $R^1$ represents $CH_2COOH$, $R^2$ does H, $R^3$ does Cl, $R^4$ does H, Q does an oxygen atom and the bonded position thereof being a 4-position, (X)n does 3-OMe, and Ar corresponds to each of all the substituents shown in Table 5-1.

Table 5-51

Table 5-51 represents 132 compounds wherein, in the general formula (I-4), $R^1$ represents $CH_2COOEt$, $R^2$ does H, $R^3$ does Cl, $R^4$ does H, Q does an oxygen atom and the bonded position thereof being a 4-position, (X)n does 3-OMe, and Ar corresponds to each of all the substituents shown in Table 5-1.

Table 5-52

Table 5-52 represents 132 compounds wherein, in the general formula (I-4), $R^1$ represents $CH_2CONHMe$, $R^2$ does H, $R^3$ does Cl, $R^4$ does H, Q does an oxygen atom and the bonded position thereof being a 4-position, (X)n does 3-OMe, and Ar corresponds to each of all the substituents shown in Table 5-1.

Table 5-53

Table 5-53 represents 132 compounds wherein, in the general formula (I-4), $R^1$ represents $CH_2CONMe_2$, $R^2$ does H, $R^3$ does Cl, $R^4$ does H, Q does an oxygen atom and the bonded position thereof being a 4-position, (X)n does

3-OMe, and Ar corresponds to each of all the substituents shown in Table 5-1.

Table 5-54

Table 5-54 represents 132 compounds wherein, in the general formula (I-4), $R^1$ represents $CH_2CHO$, $R^2$ does H, $R^3$ does Cl, $R^4$ does H, Q does an oxygen atom and the bonded position thereof being a 4-position, (X)n does 3-OMe, and Ar corresponds to each of all the substituents shown in Table 5-1.

Table 5-55

Table 5-55 represents 132 compounds wherein, in the general formula (I-4), $R^1$ represents $CH_2CH=NOMe$, $R^2$ does H, $R^3$ does Cl, $R^4$ does H, Q does an oxygen atom and the bonded position thereof being a 4-position, (X)n does 3-OMe, and Ar corresponds to each of all the substituents shown in Table 5-1.

Table 5-56

Table 5-56 represents 132 compounds wherein, in the general formula (I-4), $R^1$ represents Et, $R^2$ does H, $R^3$ does H, $R^4$ does H, Q does an oxygen atom and the bonded position thereof being a 4-position, (X)n does 3-OMe, and Ar corresponds to each of all the substituents shown in Table 5-1.

Table 5-57

Table 5-57 represents 132 compounds wherein, in the general formula (I-4), $R^1$ represents Et, $R^2$ does H, $R^3$ does F, $R^4$ does H, Q does an oxygen atom and the bonded position thereof being a 4-position, (X)n does 3-OMe, and Ar corresponds to each of all the substituents shown in Table 5-1.

Table 5-58

Table 5-58 represents 132 compounds wherein, in the general formula (I-4), $R^1$ represents Et, $R^2$ does H, $R^3$ does Br, $R^4$ does H, Q does an oxygen atom and the bonded position thereof being a 4-position, (X)n does 3-OMe, and Ar corresponds to each of all the substituents shown in Table 5-1.

Table 5-59

Table 5-59 represents 132 compounds wherein, in the general formula (I-4), $R^1$ represents Et, $R^2$ does H, $R^3$ does I, $R^4$ does H, Q does an oxygen atom and the bonded position thereof being a 4-position, (X)n does 3-OMe, and Ar corresponds to each of all the substituents shown in Table 5-1.

Table 5-60

Table 5-60 represents 132 compounds wherein, in the general formula (I-4), $R^1$ represents Et, $R^2$ does H, $R^3$ does $CF_3$, $R^4$ does H, Q does an oxygen atom and the bonded position thereof being a 4-position, (X)n does 3-OMe, and Ar corresponds to each of all the substituents shown in Table 5-1.

Table 5-61

Table 5-61 represents 132 compounds wherein, in the general formula (I-4), $R^1$ represents Et, $R^2$ does H, $R^3$ does CN, $R^4$ does H, Q does an oxygen atom and the bonded position thereof being a 4-position, (X)n does 3-OMe, and Ar corresponds to each of all the substituents shown in Table 5-1.

Table 5-62

Table 5-62 represents 132 compounds wherein, in the general formula (I-4), $R^1$ represents Et, $R^2$ does H, $R^3$ does COOMe, $R^4$ does H, Q does an oxygen atom and the bonded position thereof being a 4-position, (X)n does 3-OMe, and Ar corresponds to each of all the substituents shown in Table 5-1.

Table 5-63

Table 5-63 represents 132 compounds wherein, in the general formula (I-4), $R^1$ represents Et, $R^2$ does H, $R^3$ does COONHMe, $R^4$ does H, Q does an oxygen atom and the bonded position thereof being a 4-position, (X)n does 3-OMe, and Ar corresponds to each of all the substituents shown in Table 5-1.

Table 5-64

Table 5-64 represents 132 compounds wherein, in the general formula (I-4), $R^1$ represents Et, $R^2$ does H, $R^3$ does $COONMe_2$, $R^4$ does H, Q does an oxygen atom and the bonded position thereof being a 4-position, (X)n does 3-OMe, and Ar corresponds to each of all the substituents shown in Table 5-1.

Table 5-65

Table 5-65 represents 132 compounds wherein, in the general formula (I-4), $R^1$ represents Et, $R^2$ does H, $R^3$ does SMe, $R^4$ does H, Q does an oxygen atom and the bonded position thereof being a 4-position, (X)n does 3-OMe, and Ar corresponds to each of all the substituents shown in Table 5-1.

Table 5-66

Table 5-66 represents 132 compounds wherein, in the general formula (I-4), $R^1$ represents Et, $R^2$ does H, $R^3$ does SPh, $R^4$ does H, Q does an oxygen atom and the bonded position thereof being a 4-position, (X)n does 3-OMe, and Ar corresponds to each of all the substituents shown in Table 5-1.

Table 5-67

Table 5-67 represents 132 compounds wherein, in the general formula (I-4), $R^1$ represents Et, $R^2$ does H, $R^3$ does OH, $R^4$ does H, Q does an oxygen atom and the bonded position thereof being a 4-position, (X)n does 3-OMe, and Ar corresponds to each of all the substituents shown in Table 5-1.

Table 5-68

Table 5-68 represents 132 compounds wherein, in the general formula (I-4), $R^1$ represents Et, $R^2$ does H, $R^3$ does OMe, $R^4$ does H, Q does an oxygen atom and the bonded position thereof being a 4-position, (X)n does 3-OMe, and Ar corresponds to each of all the substituents shown in Table 5-1.

Table 5-69

Table 5-69 represents 132 compounds wherein, in the general formula (I-4), $R^1$ represents Et, $R^2$ does H, $R^3$ does OPh, $R^4$ does H, Q does an oxygen atom and the bonded position thereof being a 4-position, (X)n does 3-OMe, and Ar corresponds to each of all the substituents shown in Table 5-1.

Table 5-70

Table 5-70 represents 132 compounds wherein, in the general formula (I-4), $R^1$ represents Et, $R^2$ does H, $R^3$ does $NMe_2$, $R^4$ does H, Q does an oxygen atom and the bonded position thereof being a 4-position, (X)n does 3-OMe, and Ar corresponds to each of all the substituents shown in Table 5-1.

Table 5-71

Table 5-71 represents 132 compounds wherein, in the general formula (I-4), $R^1$ represents Et, $R^2$ does H, $R^3$ does Cl, $R^4$ does Me, Q does an oxygen atom and the bonded position thereof being a 4-position, (X)n does 3-OMe, and Ar corresponds to each of all the substituents shown in Table 5-1.

Table 5-72

Table 5-72 represents 132 compounds wherein, in the general formula (I-4), $R^1$ represents Et, $R^2$ does H, $R^3$ does Cl, $R^4$ does Et, Q does an oxygen atom and the bonded position thereof being a 4-position, (X)n does 3-OMe,

and Ar corresponds to each of all the substituents shown in Table 5-1.

Table 5-73

Table 5-73 represents 132 compounds wherein, in the general formula (I-4), $R^1$ represents Et, $R^2$ does H, $R^3$ does Cl, $R^4$ does n-Pr, Q does an oxygen atom and the bonded position thereof being a 4-position, (X)n does 3-OMe, and Ar corresponds to each of all the substituents shown in Table 5-1.

Table 5-74

Table 5-74 represents 132 compounds wherein, in the general formula (I-4), $R^1$ represents Et, $R^2$ does H, $R^3$ does Cl, $R^4$ does i-Pr, Q does an oxygen atom and the bonded position thereof being a 4-position, (X)n does 3-OMe, and Ar corresponds to each of all the substituents shown in Table 5-1.

Table 5-75

Table 5-75 represents 132 compounds wherein, in the general formula (I-4), $R^1$ represents Et, $R^2$ does H, $R^3$ does Cl, $R^4$ does $CH_2CH=CH_2$, Q does an oxygen atom and the bonded position thereof being a 4-position, (X)n does 3-OMe, and Ar corresponds to each of all the substituents shown in Table 5-1.

Table 5-76

Table 5-76 represents 132 compounds wherein, in the general formula (I-4), $R^1$ represents Et, $R^2$ does H, $R^3$ does Cl, $R^4$ does $CH_2C\equiv CH$, Q does an oxygen atom and the bonded position thereof being a 4-position, (X)n does 3-OMe, and Ar corresponds to each of all the substituents shown in Table 5-1.

Table 5-77

Table 5-77 represents 132 compounds wherein, in the general formula (I-4), $R^1$ represents Et, $R^2$ does H, $R^3$ does Cl, $R^4$ does $CH_2CF_3$, Q does an oxygen atom and the bonded position thereof being a 4-position, (X)n does 3-OMe, and Ar corresponds to each of all the substituents shown in Table 5-1.

Table 5-78

Table 5-78 represents 132 compounds wherein, in the general formula (I-4), $R^1$ represents Et, $R^2$ does H, $R^3$ does Cl, $R^4$ does $CH_2CN$, Q does an oxygen atom and the bonded position thereof being a 4-position, (X)n does 3-OMe, and Ar corresponds to each of all the substituents shown in Table 5-1.

Table 5-79

Table 5-79 represents 132 compounds wherein, in the general formula (I-4), $R^1$ represents Et, $R^2$ does H, $R^3$ does Cl, $R^4$ does $CH_2OMe$, Q does an oxygen atom and the bonded position thereof being a 4-position, (X)n does 3-OMe, and Ar corresponds to each of all the substituents shown in Table 5-1.

Table 5-80

Table 5-80 represents 132 compounds wherein, in the general formula (I-4), $R^1$ represents Et, $R^2$ does H, $R^3$ does Cl, $R^4$ does $CH_2OEt$, Q does an oxygen atom and the bonded position thereof being a 4-position, (X)n does 3-OMe, and Ar corresponds to each of all the substituents shown in Table 5-1.

Table 5-81

Table 5-81 represents 132 compounds wherein, in the general formula (I-4), $R^1$ represents Et, $R^2$ does H, $R^3$ does Cl, $R^4$ does $CH_2SMe$, Q does an oxygen atom and the bonded position thereof being a 4-position, (X)n does 3-OMe, and Ar corresponds to each of all the substituents shown in Table 5-1.

Table 5-82

Table 5-82 represents 132 compounds wherein, in the general formula (I-4), $R^1$ represents Et, $R^2$ does H, $R^3$ does Cl, $R^4$ does $CH_2COOEt$, Q does an oxygen atom and the bonded position thereof being a 4-position, (X)n does 3-OMe, and Ar corresponds to each of all the substituents shown in Table 5-1.

Table 5-83

Table 5-83 represents 132 compounds wherein, in the general formula (I-4), $R^1$ represents Et, $R^2$ does H, $R^3$ does Cl, $R^4$ does COOMe, Q does an oxygen atom and the bonded position thereof being a 4-position, (X)n does 3-OMe, and Ar corresponds to each of all the substituents shown in Table 5-1.

Table 5-84

Table 5-84 represents 132 compounds wherein, in the general formula (I-4), $R^1$ represents Et, $R^2$ does H, $R^3$ does Cl, $R^4$ does COOEt, Q does an oxygen atom and the bonded position thereof being a 4-position, (X)n does 3-OMe, and Ar corresponds to each of all the substituents shown in Table 5-1.

Table 5-85

Table 5-85 represents 132 compounds wherein, in the general formula (I-4), $R^1$ represents Et, $R^2$ does H, $R^3$ does Cl, $R^4$ does COO-n-Pr, Q does an oxygen atom and the bonded position thereof being a 4-position, (X)n does 3-OMe, and Ar corresponds to each of all the substituents shown in Table 5-1.

Table 5-86

Table 5-86 represents 132 compounds wherein, in the general formula (I-4), $R^1$ represents Et, $R^2$ does H, $R^3$ does Cl, $R^4$ does COO-i-Pr, Q does an oxygen atom and the bonded position thereof being a 4-position, (X)n does 3-OMe, and Ar corresponds to each of all the substituents shown in Table 5-1.

Table 5-87

Table 5-87 represents 132 compounds wherein, in the general formula (I-4), $R^1$ represents Et, $R^2$ does H, $R^3$ does Cl, $R^4$ does COO-t-Bu, Q does an oxygen atom and the bonded position thereof being a 4-position, (X)n does 3-OMe, and Ar corresponds to each of all the substituents shown in Table 5-1.

Table 5-88

Table 5-88 represents 132 compounds wherein, in the general formula (I-4), $R^1$ represents Et, $R^2$ does H, $R^3$ Cl, $R^4$ does COOPh, Q does an oxygen atom and the bonded position thereof being a 4-position, (X)n does 3-OMe, and Ar corresponds to each of all the substituents shown in Table 5-1.

Table 5-89

Table 5-89 represents 132 compounds wherein, in the general formula (I-4), $R^1$ represents Et, $R^2$ does H, $R^3$ does Cl, $R^4$ does $COOCH_2Ph$, Q does an oxygen atom and the bonded position thereof being a 4-position, (X)n does 3-OMe, and Ar corresponds to each of all the substituents shown in Table 5-1.

Table 5-90

Table 5-90 represents 132 compounds wherein, in the general formula (I-4), $R^1$ represents Et, $R^2$ does H, $R^3$ does Cl, $R^4$ does CONHMe, Q does an oxygen atom and the bonded position thereof being a 4-position, (X)n does 3-OMe, and Ar corresponds to each of all the substituents shown in Table 5-1.

Table 5-91

Table 5-91 represents 132 compounds wherein, in the general formula (I-4), $R^1$ represents Et, $R^2$ does H, $R^3$ does Cl, $R^4$ does $CONMe_2$, Q does an oxygen atom and the bonded position thereof being a 4-position, (X)n does

3-OMe, and Ar corresponds to each of all the substituents shown in Table 5-1.

Table 5-92

Table 5-92 represents 132 compounds wherein, in the general formula (I-4), $R^1$ represents Et, $R^2$ does H, $R^3$ does Cl, $R^4$ does Ac, Q does an oxygen atom and the bonded position thereof being a 4-position, (X)n does 3-OMe, and Ar corresponds to each of all the substituents shown in Table 5-1.

Table 5-93

Table 5-93 represents 132 compounds wherein, in the general formula (I-4), $R^1$ represents Et, $R^2$ does H, $R^3$ does Cl, $R^4$ does COEt, Q does an oxygen atom and the bonded position thereof being a 4-position, (X)n does 3-OMe, and Ar corresponds to each of all the substituents shown in Table 5-1.

Table 5-94

Table 5-94 represents 132 compounds wherein, in the general formula (I-4), $R^1$ represents Et, $R^2$ does H, $R^3$ does Cl, $R^4$ does CO-n-Pr, Q does an oxygen atom and the bonded position thereof being a 4-position, (X)n does 3-OMe, and Ar corresponds to each of all the substituents shown in Table 5-1.

Table 5-95

Table 5-95 represents 132 compounds wherein, in the general formula (I-4), $R^1$ represents Et, $R^2$ does H, $R^3$ does Cl, $R^4$ does CO-i-Pr, Q does an oxygen atom and the bonded position thereof being a 4-position, (X)n does 3-OMe, and Ar corresponds to each of all the substituents shown in Table 5-1.

Table 5-96

Table 5-96 represents 132 compounds wherein, in the general formula (I-4), $R^1$ represents Et, $R^2$ does H, $R^3$ does Cl, $R^4$ does CO-t-Bu, Q does an oxygen atom and the bonded position thereof being a 4-position, (X)n does 3-OMe, and Ar corresponds to each of all the substituents shown in Table 5-1.

Table 5-97

Table 5-97 represents 132 compounds wherein, in the general formula (I-4), $R^1$ represents Et, $R^2$ does H, $R^3$ does Cl, $R^4$ does $COCF_3$, Q does an oxygen atom and the bonded position thereof being a 4-position, (X)n does 3-OMe, and Ar corresponds to each of all the substituents shown in Table 5-1.

Table 5-98

Table 5-98 represents 132 compounds wherein, in the general formula (I-4), $R^1$ represents Et, $R^2$ does H, $R^3$ does Cl, $R^4$ does COPh, Q does an oxygen atom and the bonded position thereof being a 4-position, (X)n does 3-OMe, and Ar corresponds to each of all the substituents shown in Table 5-1.

Table 5-99

Table 5-99 represents 132 compounds wherein, in the general formula (I-4), $R^1$ represents Et, $R^2$ does H, $R^3$ does Cl, $R^4$ does $COCH_2Ph$, Q does an oxygen atom and the bonded position thereof being a 4-position, (X)n does 3-OMe, and Ar corresponds to each of all the substituents shown in Table 5-1.

Table 5-100

Table 5-100 represents 132 compounds wherein, in the general formula (I-4), $R^1$ represents Et, $R^2$ does H, $R^3$ does Cl, $R^4$ does CO-c-Pr, Q does an oxygen atom and the bonded position thereof being a 4-position, (X)n does 3-OMe, and Ar corresponds to each of all the substituents shown in Table 5-1.

Table 5-101

Table 5-101 represents 132 compounds wherein, in the general formula (I-4), $R^1$ represents Et, $R^2$ does H, $R^3$ does Cl, $R^4$ does COCH=CHMe, Q does an oxygen atom and the bonded position thereof being a 4-position, (X) n does 3-OMe, and Ar corresponds to each of all the substituents shown in Table 5-1.

Table 5-102

Table 5-102 represents 132 compounds wherein, in the general formula (I-4), $R^1$ represents Et, $R^2$ does H, $R^3$ does Cl, $R^4$ does SPh, Q does an oxygen atom and the bonded position thereof being a 4-position, (X)n does 3-OMe, and Ar corresponds to each of all the substituents shown in Table 5-1.

Table 5-103

Table 5-103 represents 132 compounds wherein, in the general formula (I-4), $R^1$ represents Et, $R^2$ does H, $R^3$ does Cl, $R^4$ does $CH_2$-c-Pr, Q does an oxygen atom and the bonded position thereof being a 4-position, (X)n does 3-OMe, and Ar corresponds to each of all the substituents shown in Table 5-1.

Table 5-104

Table 5-104 represents 132 compounds wherein, in the general formula (I-4), $R^1$ represents Et, $R^2$ does H, $R^3$ does Cl, $R^4$ does H, Q does a sulfur atom and the bonded position thereof being a 4-position, (X)n does 3-OMe, and Ar corresponds to each of all the substituents shown in Table 5-1.

Table 5-105

Table 5-105 represents 132 compounds wherein, in the general formula (I-4), $R^1$ represents Et, $R^2$ does H, $R^3$ does Cl, $R^4$ does H, Q does NH and the bonded position thereof being a 4-position, (X)n does 3-OMe, and Ar corresponds to each of all the substituents shown in Table 5-1.

Table 5-106

Table 5-106 represents 132 compounds wherein, in the general formula (I-4), $R^1$ represents Et, $R^2$ does H, $R^3$ does Cl, $R^4$ does H, Q does NMe and the bonded position thereof being a 4-position, (X)n does 3-OMe, and Ar corresponds to each of all the substituents shown in Table 5-1.

Table 5-107

Table 5-107 represents 132 compounds wherein, in the general formula (I-4), $R^1$ represents Et, $R^2$ does H, $R^3$ does Cl, $R^4$ does H, Q does NAc and the bonded position thereof being a 4-position, (X)n does 3-OMe, and Ar corresponds to each of all the substituents shown in Table 5-1.

Table 5-108

Table 5-108 represents 132 compounds wherein, in the general formula (I-4), $R^1$ represents Et, $R^2$ does H, $R^3$ does Cl, $R^4$ does H, Q does CH=CH and the bonded position thereof being a 4-position, (X)n does 3-OMe, and Ar corresponds to each of all the substituents shown in Table 5-1.

Table 5-109

Table 5-109 represents 132 compounds wherein, in the general formula (I-4), $R^1$ represents Et, $R^2$ H, $R^3$ Cl, $R^4$ H, Q $COCH_2$, the bonding position thereof a 4-position, (X)n 3-OMe, and Ar corresponds to each of all the substituents shown in Table 5-1.

Table 5-110

Table 5-110 represents 132 compounds wherein, in the general formula (I-4), $R^1$ represents Et, $R^2$ does H, $R^3$ does Cl, $R^4$ does H, Q does C(OMe)=CH and the bonded position thereof being a 4-position, (X)n does 3-OMe,

and Ar corresponds to each of all the substituents shown in Table 5-1.

Table 5-111

Table 5-111 represents 132 compounds wherein, in the general formula (I-4), $R^1$ represents Et, $R^2$ does H, $R^3$ does Cl, $R^4$ does H, Q does C≡C and the bonded position thereof being a 4-position, (X)n does 3-OMe, and Ar corresponds to each of all the substituents shown in Table 5-1.

Table 5-112

Table 5-112 represents 132 compounds wherein, in the general formula (I-4), $R^1$ represents Et, $R^2$ does H, $R^3$ does Cl, $R^4$ does H, Q does $CH_2=CH_2$ and the bonded position thereof being a 4-position, (X)n does 3-OMe, and Ar corresponds to each of all the substituents shown in Table 5-1.

Table 5-113

Table 5-113 represents 132 compounds wherein, in the general formula (I-4), $R^1$ represents Et, $R^2$ does H, $R^3$ does Cl, $R^4$ does H, Q does $CH(OH)CH_2$ and the bonded position thereof being a 4-position, (X)n does 3-OMe, and Ar corresponds to each of all the substituents shown in Table 5-1.

Table 5-114

Table 5-114 represents 132 compounds wherein, in the general formula (I-4), $R^1$ represents Et, $R^2$ does H, $R^3$ does Cl, $R^4$ does H, Q does $CH(OMe)CH_2$ and the bonded position thereof being a 4-position, (X)n does 3-OMe, and Ar corresponds to each of all the substituents shown in Table 5-1.

Table 5-115

Table 5-115 represents 132 compounds wherein, in the general formula (I-4), $R^1$ represents Et, $R^2$ does H, $R^3$ does Cl, $R^4$ does H, Q does $OCH_2$ and the bonded position thereof being a 4-position, (X)n does 3-OMe, and Ar corresponds to each of all the substituents shown in Table 5-1.

Table 5-116

Table 5-116 represents 132 compounds wherein, in the general formula (I-4), $R^1$ represents Et, $R^2$ does H, $R^3$ does Cl, $R^4$ does H, Q does OCH(Me) and the bonded position thereof being a 4-position, (X)n does 3-OMe, and Ar corresponds to each of all the substituents shown in Table 5-1.

Table 5-117

Table 5-117 represents 132 compounds wherein, in the general formula (I-4), $R^1$ represents Et, $R^2$ does H, $R^3$ does Cl, $R^4$ does H, Q does $OCH_2CH_2$ and the bonded position thereof being a 4-position, (X)n does 3-OMe, and Ar corresponds to each of all the substituents shown in Table 5-1.

Table 5-118

Table 5-118 represents 132 compounds wherein, in the general formula (I-4), $R^1$ represents Et, $R^2$ does H, $R^3$ does Cl, $R^4$ does H, Q does $OCH_2CH=CH$ and the bonded position thereof being a 4-position, (X)n does 3-OMe, and Ar corresponds to each of all the substituents shown in Table 5-1.

Table 5-119

Table 5-119 represents 132 compounds wherein, in the general formula (I-4), $R^1$ represents Et, $R^2$ does H, $R^3$ does Cl, $R^4$ does H, Q does $SCH_2$ and the bonded position thereof being a 4-position, (X)n 3-OMe, and Ar corresponds to each of all the substituents shown in Table 5-1.

Table 5-120

Table 5-120 represents 132 compounds wherein, in the general formula (I-4), $R^1$ represents Et, $R^2$ does H, $R^3$ does Cl, $R^4$ does H, Q does $SCH_2CH_2$ and the bonded position thereof being a 4-position, (X)n does 3-OMe, and Ar corresponds to each of all the substituents shown in Table 5-1.

Table 5-121

Table 5-121 represents 132 compounds wherein, in the general formula (I-4), $R^1$ represents Et, $R^2$ does H, $R^3$ does Cl, $R^4$ does H, Q does $NHCH_2$ and the bonded position thereof being a 4-position, (X)n does 3-OMe, and Ar corresponds to each of all the substituents shown in Table 5-1.

Table 5-122

Table 5-122 represents 132 compounds wherein, in the general formula (I-4), $R^1$ represents Et, $R^2$ does H, $R^3$ does Cl, $R^4$ does H, Q does $NHCH_2CH_2$ and the bonded position thereof being a 4-position, (X)n does 3-OMe, and Ar corresponds to each of all the substituents shown in Table 5-1.

Table 5-123

Table 5-123 represents 132 compounds wherein, in the general formula (I-4), $R^1$ represents Et, $R^2$ does H, $R^3$ does Cl, $R^4$ does H, Q does $N(Me)CH_2$ and the bonded position thereof being a 4-position, (X)n does 3-OMe, and Ar corresponds to each of all the substituents shown in Table 5-1.

Table 5-124

Table 5-124 represents 132 compounds wherein, in the general formula (I-4), $R^1$ represents Et, $R^2$ does H, $R^3$ does Cl, $R^4$ does H, Q does $N(Me)CH_2CH_2$ and the bonded position thereof being a 4-position, (X)n does 3-OMe, and Ar corresponds to each of all the substituents shown in Table 5-1.

Table 5-125

Table 5-125 represents 132 compounds wherein, in the general formula (I-4), $R^1$ represents Et, $R^2$ does H, $R^3$ does Cl, $R^4$ does H, Q does NHCO and the bonded position thereof being a 4-position, (X)n does 3-OMe, and Ar corresponds to each of all the substituents shown in Table 5-1.

Table 5-126

Table 5-126 represents 132 compounds wherein, in the general formula (I-4), $R^1$ represents Et, $R^2$ does H, $R^3$ does Cl, $R^4$ does H, Q does N(Me)CO and the bonded position thereof being a 4-position, (X)n does 3-OMe, and Ar corresponds to each of all the substituents shown in Table 5-1.

Table 5-127

Table 5-127 represents 132 compounds wherein, in the general formula (I-4), $R^1$ represents Et, $R^2$ does H, $R^3$ does Cl, $R^4$ does H, Q does NHCOCH=CH and the bonded position thereof being a 4-position, (X)n does 3-OMe, and Ar corresponds to each of all the substituents shown in Table 5-1.

Table 5-128

Table 5-128 represents 132 compounds wherein, in the general formula (I-4), $R^1$ represents Et, $R^2$ does H, $R^3$ does Cl, $R^4$ does H, Q does C=O and the bonded position thereof being a 4-position, (X)n does 3-OMe, and Ar corresponds to each of all the substituents shown in Table 5-1.

Table 5-129

Table 5-129 represents 132 compounds wherein, in the general formula (I-4), $R^1$ represents Et, $R^2$ does H, $R^3$ does Cl, $R^4$ does H, Q does C=NOMe and the bonded position thereof being a 4-position, (X)n does 3-OMe, and

Ar corresponds to each of all the substituents shown in Table 5-1.

Table 5-130

Table 5-130 represents 132 compounds wherein, in the general formula (I-4), $R^1$ represents Et, $R^2$ does H, $R^3$ does Cl, $R^4$ does H, Q does an oxygen atom and the bonded position thereof being a 3-position, (X)n does H, and Ar corresponds to each of all the substituents shown in Table 5-1.

Table 5-131

Table 5-131 represents 132 compounds wherein, in the general formula (I-4), $R^1$ represents Et, $R^2$ does H, $R^3$ does Cl, $R^4$ does H, Q does an oxygen atom and the bonded position thereof being a 2-position, (X)n does H, and Ar corresponds to each of all the substituents shown in Table 5-1.

Table 5-132

Table 5-132 represents 132 compounds wherein, in the general formula (I-4), $R^1$ represents Et, $R^2$ does H, $R^3$ does Cl, $R^4$ does H, Q does an oxygen atom and the bonded position thereof being a 4-position, (X)n does 3-Cl, and Ar corresponds to each of all the substituents shown in Table 5-1.

Table 5-133

Table 5-133 represents 132 compounds wherein, in the general formula (I-4), $R^1$ represents Et, $R^2$ does H, $R^3$ does Cl, $R^4$ does H, Q does an oxygen atom and the bonded position thereof being a 4-position, (X)n does 2-Cl, and Ar corresponds to each of all the substituents shown in Table 5-1.

Table 5-134

Table 5-134 represents 132 compounds wherein, in the general formula (I-4), $R^1$ represents Et, $R^2$ does H, $R^3$ does Cl, $R^4$ does H, Q does an oxygen atom and the bonded position thereof being a 4-position, (X)n does 3-Me, and Ar corresponds to each of all the substituents shown in Table 5-1.

Table 5-135

Table 5-135 represents 132 compounds wherein, in the general formula (I-4), $R^1$ represents Et, $R^2$ does H, $R^3$ does Cl, $R^4$ does H, Q does an oxygen atom and the bonded position thereof being a 4-position, (X)n does 2-Me, and Ar corresponds to each of all the substituents shown in Table 5-1.

Table 5-136

Table 5-136 represents 132 compounds wherein, in the general formula (I-4), $R^1$ represents Et, $R^2$ does H, $R^3$ does Cl, $R^4$ does H, Q does an oxygen atom and the bonded position thereof being a 4-position, (X)n does 2-OMe, and Ar corresponds to each of all the substituents shown in Table 5-1.

Table 5-137

Table 5-137 represents 132 compounds wherein, in the general formula (I-4), $R^1$ represents Et, $R^2$ does H, $R^3$ does Cl, $R^4$ does H, Q does an oxygen atom and the bonded position thereof being a 4-position, (X)n does 3-$CF_3$, and Ar corresponds to each of all the substituents shown in Table 5-1.

Table 5-138

Table 5-138 represents 132 compounds wherein, in the general formula (I-4), $R^1$ represents Et, $R^2$ does H, $R^3$ does Cl, $R^4$ does H, Q does an oxygen atom and the bonded position thereof being a 4-position, (X)n does 2-$CF_3$, and Ar corresponds to each of all the substituents shown in Table 5-1.

Table 5-139

Table 5-139 represents 132 compounds wherein, in the general formula (I-4), $R^1$ represents Et, $R^2$ does H, $R^3$ does Cl, $R^4$ does H, Q does an oxygen atom and the bonded position thereof being a 4-position, (X)n does 3-OH, and Ar corresponds to each of all the substituents shown in Table 5-1.

Table 5-140

Table 5-140 represents 132 compounds wherein, in the general formula (I-4), $R^1$ represents Et, $R^2$ does H, $R^3$ does Cl, $R^4$ does H, Q does an oxygen atom and the bonded position thereof being a 4-position, (X)n does 3-$OCHF_2$, and Ar corresponds to each of all the substituents shown in Table 5-1.

Table 5-141

Table 5-141 represents 132 compounds wherein, in the general formula (I-4), $R^1$ represents Et, $R^2$ does H, $R^3$ does Cl, $R^4$ does H, Q does an oxygen atom and the bonded position thereof being a 4-position, (X)n does 3-$OCF_3$, and Ar corresponds to each of all the substituents shown in Table 5-1.

Table 5-142

Table 5-142 represents 132 compounds wherein, in the general formula (I-4), $R^1$ represents Et, $R^2$ does H, $R^3$ does Cl, $R^4$ does H, Q does an oxygen atom and the bonded position thereof being a 4-position, (X)n does 3-$OCF_2CHF_2$, and Ar corresponds to each of all the substituents shown in Table 5-1.

Table 5-143

Table 5-143 represents 132 compounds wherein, in the general formula (I-4), $R^1$ represents Et, $R^2$ does H, $R^3$ does Cl, $R^4$ does H, Q does an oxygen atom and the bonded position thereof being a 4-position, (X)n does 3-$OCH_2CF_3$, and Ar corresponds to each of all the substituents shown in Table 5-1.

Table 5-144

Table 5-144 represents 132 compounds wherein, in the general formula (I-4), $R^1$ represents Et, $R^2$ does H, $R^3$ does Cl, $R^4$ does H, Q does an oxygen atom and the bonded position thereof being a 4-position, (X)n does 3-$NH_2$, and Ar corresponds to each of all the substituents shown in Table 5-1.

Table 5-145

Table 5-145 represents 132 compounds wherein, in the general formula (I-4), $R^1$ represents Et, $R^2$ does H, $R^3$ does Cl, $R^4$ does H, Q does an oxygen atom and the bonded position thereof being a 4-position, (X)n does 3-NHMe, and Ar corresponds to each of all the substituents shown in Table 5-1.

Table 5-146

Table 5-146 represents 132 compounds wherein, in the general formula (I-4), $R^1$ represents Et, $R^2$ does H, $R^3$ does Cl, $R^4$ does H, Q does an oxygen atom and the bonded position thereof being a 4-position, (X)n does 3-$NMe_2$, and Ar corresponds to each of all the substituents shown in Table 5-1.

Table 5-147

Table 5-147 represents 132 compounds wherein, in the general formula (I-4), $R^1$ represents Et, $R^2$ does H, $R^3$ does Cl, $R^4$ does H, Q does an oxygen atom and the bonded position thereof being a 4-position, (X)n does 3-NHAc, and Ar corresponds to each of all the substituents shown in Table 5-1.

Table 5-148

Table 5-148 represents 132 compounds wherein, in the general formula (I-4), $R^1$ represents Et, $R^2$ does H, $R^3$ does Cl, $R^4$ does H, Q does an oxygen atom and the bonded position thereof being a 4-position, (X)n does

3-$NHCOCF_3$, and Ar corresponds to each of all the substituents shown in Table 5-1.

Table 5-149

Table 5-149 represents 132 compounds wherein, in the general formula (I-4), $R^1$ represents Et, $R^2$ does H, $R^3$ does Cl, $R^4$ does H, Q does an oxygen atom and the bonded position thereof being a 4-position, (X)n does 3-NHCOOMe, and Ar corresponds to each of all the substituents shown in Table 5-1.

Table 5-150

Table 5-150 represents 132 compounds wherein, in the general formula (I-4), $R^1$ represents Et, $R^2$ does H, $R^3$ does Cl, $R^4$ does H, Q does an oxygen atom and the bonded position thereof being a 4-position, (X)n does 3-$NHCH_2COOMe$, and Ar corresponds to each of all the substituents shown in Table 5-1.

Table 5-151

Table 5-151 represents 132 compounds wherein, in the general formula (I-4), $R^1$ represents Et, $R^2$ does H, $R^3$ does Cl, $R^4$ does H, Q does an oxygen atom and the bonded position thereof being a 4-position, (X)n does 3-$NHCH_2CN$, and Ar corresponds to each of all the substituents shown in Table 5-1.

Table 5-152

Table 5-152 represents 132 compounds wherein, in the general formula (I-4), $R^1$ represents Et, $R^2$ does H, $R^3$ does Cl, $R^4$ does H, Q does an oxygen atom and the bonded position thereof being a 4-position, (X)n does 3-NHCONHMe, and Ar corresponds to each of all the substituents shown in Table 5-1.

Table 5-153

Table 5-153 represents 132 compounds wherein, in the general formula (I-4), $R^1$ represents Et, $R^2$ does H, $R^3$ does Cl, $R^4$ does H, Q does an oxygen atom and the bonded position thereof being a 4-position, (X)n does 3-$NHSO_2Me$, and Ar corresponds to each of all the substituents shown in Table 5-1.

Table 5-154

Table 5-154 represents 132 compounds wherein, in the general formula (I-4), $R^1$ represents Et, $R^2$ does H, $R^3$ does Cl, $R^4$ does H, Q does an oxygen atom and the bonded position thereof being a 4-position, (X)n does 3-$NHSO_2CF_3$, and Ar corresponds to each of all the substituents shown in Table 5-1.

Table 5-155

Table 5-155 represents 132 compounds wherein, in the general formula (I-4), $R^1$ represents Et, $R^2$ does H, $R^3$ does Cl, $R^4$ does H, Q does an oxygen atom and the bonded position thereof being a 4-position, (X)n does 3-$NHCHF_2$, and Ar corresponds to each of all the substituents shown in Table 5-1.

Table 5-156

Table 5-156 represents 132 compounds wherein, in the general formula (I-4), $R^1$ represents Et, $R^2$ does H, $R^3$ does Cl, $R^4$ does H, Q does an oxygen atom and the bonded position thereof being a 4-position, (X)n does 3-$N(CHF_2)_2$, and Ar corresponds to each of all the substituents shown in Table 5-1.

Table 5-157

Table 5-157 represents 132 compounds wherein, in the general formula (I-4), $R^1$ represents Et, $R^2$ does H, $R^3$ does Cl, $R^4$ does H, Q does an oxygen atom and the bonded position thereof being a 4-position, (X)n does 3-$NHCF_2CHF_2$, and Ar corresponds to each of all the substituents shown in Table 5-1.

Table 5-158

Table 5-158 represents 132 compounds wherein, in the general formula (I-4), $R^1$ represents Et, $R^2$ does H, $R^3$ does Cl, $R^4$ does H, Q does an oxygen atom and the bonded position thereof being a 4-position, (X)n does 3-$NHCH_2CF_3$, and Ar corresponds to each of all the substituents shown in Table 5-1.

Table 5-159

Table 5-159 represents 132 compounds wherein, in the general formula (I-4), $R^1$ represents Et, $R^2$ does H, $R^3$ does Cl, $R^4$ does H, Q does an oxygen atom and the bonded position thereof being a 4-position, (X)n does 3-SH, and Ar corresponds to each of all the substituents shown in Table 5-1.

Table 5-160

Table 5-160 represents 132 compounds wherein, in the general formula (I-4), $R^1$ represents Et, $R^2$ does H, $R^3$ does Cl, $R^4$ does H, Q does an oxygen atom and the bonded position thereof being a 4-position, (X)n does 3-SMe, and Ar corresponds to each of all the substituents shown in Table 5-1.

Table 5-161

Table 5-161 represents 132 compounds wherein, in the general formula (I-4), $R^1$ represents Et, $R^2$ does H, $R^3$ does Cl, $R^4$ does H, Q does an oxygen atom and the bonded position thereof being a 4-position, (X)n does 3-SOMe, and Ar corresponds to each of all the substituents shown in Table 5-1.

Table 5-162

Table 5-162 represents 132 compounds wherein, in the general formula (I-4), $R^1$ represents Et, $R^2$ does H, $R^3$ does Cl, $R^4$ does H, Q does an oxygen atom and the bonded position thereof being a 4-position, (X)n does 3-$SO_2Me$, and Ar corresponds to each of all the substituents shown in Table 5-1.

Table 5-163

Table 5-163 represents 132 compounds wherein, in the general formula (I-4), $R^1$ represents Et, $R^2$ does H, $R^3$ does Cl, $R^4$ does H, Q does an oxygen atom and the bonded position thereof being a 4-position, (X)n does 3-$SCHF_2$, and Ar corresponds to each of all the substituents shown in Table 5-1.

Table 5-164

Table 5-164 represents 132 compounds wherein, in the general formula (I-4), $R^1$ represents Et, $R^2$ does H, $R^3$ does Cl, $R^4$ does H, Q does an oxygen atom and the bonded position thereof being a 4-position, (X)n does 3-$SOCHF_2$, and Ar corresponds to each of all the substituents shown in Table 5-1.

Table 5-165

Table 5-165 represents 132 compounds wherein, in the general formula (I-4), $R^1$ represents Et, $R^2$ does H, $R^3$ does Cl, $R^4$ does H, Q does an oxygen atom and the bonded position thereof being a 4-position, (X)n does 3-$SO_2CHF_2$, and Ar corresponds to each of all the substituents shown in Table 5-1.

Table 5-166

Table 5-166 represents 132 compounds wherein, in the general formula (I-4), $R^1$ represents Et, $R^2$ does H, $R^3$ does Cl, $R^4$ does H, Q does an oxygen atom and the bonded position thereof being a 4-position, (X)n does 3-$SCF_3$, and Ar corresponds to each of all the substituents shown in Table 5-1.

Table 5-167

Table 5-167 represents 132 compounds wherein, in the general formula (I-4), $R^1$ represents Et, $R^2$ does H, $R^3$ does Cl, $R^4$ does H, Q does an oxygen atom and the bonded position thereof being a 4-position, (X)n does 3-$SOCF_3$,

and Ar corresponds to each of all the substituents shown in Table 5-1.

Table 5-168

Table 5-168 represents 132 compounds wherein, in the general formula (I-4), $R^1$ represents Et, $R^2$ does H, $R^3$ does Cl, $R^4$ does H, Q does an oxygen atom and the bonded position thereof being a 4-position, (X)n does 3-$SO_2CF_3$, and Ar corresponds to each of all the substituents shown in Table 5-1.

Table 5-169

Table 5-169 represents 132 compounds wherein, in the general formula (I-4), $R^1$ represents Et, $R^2$ does H, $R^3$ does Cl, $R^4$ does H, Q does an oxygen atom and the bonded position thereof being a 4-position, (X)n does 3-$SCF_2CHF_2$, and Ar corresponds to each of all the substituents shown in Table 5-1.

Table 5-170

Table 5-170 represents 132 compounds wherein, in the general formula (I-4), $R^1$ represents Et, $R^2$ does H, $R^3$ does Cl, $R^4$ does H, Q does an oxygen atom and the bonded position thereof being a 4-position, (X)n does 3-$SOCF_2CHF_2$, and Ar corresponds to each of all the substituents shown in Table 5-1.

Table 5-171

Table 5-171 represents 132 compounds wherein, in the general formula (I-4), $R^1$ represents Et, $R^2$ does H, $R^3$ does Cl, $R^4$ does H, Q does an oxygen atom and the bonded position thereof being a 4-position, (X)n does 3-$SO_2CF_2CHF_2$, and Ar corresponds to each of all the substituents shown in Table 5-1.

Table 5-172

Table 5-172 represents 132 compounds wherein, in the general formula (I-4), $R^1$ represents Et, $R^2$ does H, $R^3$ does Cl, $R^4$ does H, Q does an oxygen atom and the bonded position thereof being a 4-position, (X)n does 3-$SCH_2CF_3$, and Ar corresponds to each of all the substituents shown in Table 5-1.

Table 5-173

Table 5-173 represents 132 compounds wherein, in the general formula (I-4), $R^1$ represents Et, $R^2$ does H, $R^3$ does Cl, $R^4$ does H, Q does an oxygen atom and the bonded position thereof being a 4-position, (X)n does 3-$SOCH_2CF_3$, and Ar corresponds to each of all the substituents shown in Table 5-1.

Table 5-174

Table 5-174 represents 132 compounds wherein, in the general formula (I-4), $R^1$ represents Et, $R^2$ does H, $R^3$ does Cl, $R^4$ does H, Q does an oxygen atom and the bonded position thereof being a 4-position, (X)n does 3-$SO_2CH_2CF_3$, and Ar corresponds to each of all the substituents shown in Table 5-1.

Table 5-175

Table 5-175 represents 132 compounds wherein, in the general formula (I-4), $R^1$ represents Et, $R^2$ does H, $R^3$ does Cl, $R^4$ does H, Q does an oxygen atom and the bonded position thereof being a 4-position, (X)n does 3,5-$Cl_2$, and Ar corresponds to each of all the substituents shown in Table 5-1.

Table 5-176

Table 5-176 represents 132 compounds wherein, in the general formula (I-4), $R^1$ represents Et, $R^2$ does H, $R^3$ does Cl, $R^4$ does H, Q does an oxygen atom and the bonded position thereof being a 4-position, (X)n does 2,3-$Cl_2$, and Ar corresponds to each of all the substituents shown in Table 5-1.

Table 5-177

Table 5-177 represents 132 compounds wherein, in the general formula (I-4), $R^1$ represents Et, $R^2$ does H, $R^3$ does Cl, $R^4$ does H, Q does an oxygen atom and the bonded position thereof being a 4-position, (X)n does 3,5-$(OMe)_2$, and Ar corresponds to each of all the substituents shown in Table 5-1.

Table 5-178

Table 5-178 represents 132 compounds wherein, in the general formula (I-4), $R^1$ represents Et, $R^2$ does H, $R^3$ does Cl, $R^4$ does H, Q does an oxygen atom and the bonded position thereof being a 4-position, (X)n does 2,3-$(OMe)_2$, and Ar corresponds to each of all the substituents shown in Table 5-1.

Table 5-179

Table 5-179 represents 132 compounds wherein, in the general formula (I-4), $R^1$ represents Et, $R^2$ does H, $R^3$ does Cl, $R^4$ does H, Q does an oxygen atom and the bonded position thereof being a 4-position, (X)n does 2,5-$(OMe)_2$, and Ar corresponds to each of all the substituents shown in Table 5-1.

The general formula (I-5):

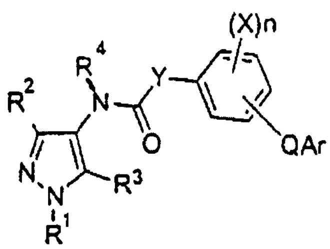

(I-5)

Table 6-1

| $R^1$ | $R^2$ | $R^3$ | $R^4$ | Y | Bond | (X)n | Q | Ar |
|---|---|---|---|---|---|---|---|---|
| Me | Me | Cl | H | CH(Me) | 4-position | H | 0 | pyridine-2-yl |
| Me | Me | Cl | H | CH(Me) | 4-position | H | 0 | 5-F-pyridine-2-yl |
| Me | Me | Cl | H | CH(Me) | 4-position | H | 0 | 5-Cl-pyridine-2-yl |
| Me | Me | Cl | H | CH(Me) | 4-position | H | 0 | 5-Br-pyridine-2-yl |
| Me | Me | Cl | H | CH(Me) | 4-position | H | 0 | 5-I-pyridine-2-yl |
| Me | Me | Cl | H | CH(Me) | 4-position | H | 0 | 5-CF3-pyridine-2-yl |
| Me | Me | Cl | H | CH(Me) | 4-position | H | 0 | 5-$NO_2$-pyridine-2-yl |
| Me | Me | Cl | H | CH(Me) | 4-position | H | 0 | 5-CN-pyridine-2-yl |
| Me | Me | Cl | H | CH(Me) | 4-position | H | 0 | 5-$CF(CF_3)_2$-pyridine-2-yl |
| Me | Me | Cl | H | CH(Me) | 4-position | H | 0 | 5-$CH(CF_3)_2$-pyridine-2-yl |
| Me | Me | Cl | H | CH(Me) | 4-position | H | 0 | 5-$CF_2CF_3$-pyridine-2-yl |
| Me | Me | Cl | H | CH(Me) | 4-position | H | 0 | 5-NHMe-pyridine-2-yl |
| Me | Me | Cl | H | CH(Me) | 4-position | H | 0 | 5-$NMe_2$-pyridine-2-yl |
| Me | Me | Cl | H | CH(Me) | 4-position | H | 0 | 5-NHAc-pyridine-2-yl |
| Me | Me | Cl | H | CH(Me) | 4-position | H | 0 | 5-NHCOOMe-pyridine-2-yl |
| Me | Me | Cl | H | CH(Me) | 4-position | H | 0 | 5-$NHCH_2COOMe$-pyridine-2-yl |
| Me | Me | Cl | H | CH(Me) | 4-position | H | 0 | 5-$NHCOCF_3$-pyridine-2-yl |

Table 6-1 (continued)

| $R^1$ | $R^2$ | $R^3$ | $R^4$ | Y | Bond | (X)n | Q | Ar |
|---|---|---|---|---|---|---|---|---|
| Me | Me | Cl | H | CH(Me) | 4-position | H | 0 | 5-NHCOCH$_2$Cl-pyridine-2-yl |
| Me | Me | Cl | H | CH(Me) | 4-position | H | 0 | 5-NHCOCH$_2$Cl-pyridine-2-yl |
| Me | Me | Cl | H | CH(Me) | 4-position | H | 0 | 5-NRSO$_2$Me-pyridine-2-yl |
| Me | Me | Cl | H | CH(Me) | 4-position | H | 0 | 5-NHSO$_2$CF$_3$-pyridine-2-yl |
| Me | Me | Cl | H | CH(Me) | 4-position | H | 0 | 5-SH-pyridine-2-yl |
| Me | Me | Cl | H | CH(Me) | 4-position | H | 0 | 5-SMe-pyridine-2-yl |
| Me | Me | Cl | H | CH(Me) | 4-position | H | 0 | 5-SOMe-pyridine-2-yl |
| Me | Me | Cl | H | CH(Me) | 4-position | H | 0 | 5-SO$_2$Me-pyridine-2-yl |
| Me | Me | Cl | H | CH(Me) | 4-position | H | 0 | 5-SCF$_3$-pyridine-2-yl |
| Me | He | Cl | H | CH(Me) | 4-position | H | 0 | 5-SOCF$_3$-pyridine-2-yl |
| Me | He | Cl | H | CH(Me) | 4-position | H | 0 | 5-SO$_2$CF$_3$-pyridine-2-yl |
| Me | Me | Cl | H | CH(Me) | 4-position | H | 0 | 5-OH-pyridine-2-yl |
| Me | Me | Cl | H | CH(Me) | 4-position | H | 0 | 5-OMe-pyridine-2-yl |
| Me | Me | Cl | H | CH(Me) | 4-position | H | 0 | 5-OCF$_3$-pyridine-2-yl |
| Me | Me | Cl | H | CH(Me) | 4-position | H | 0 | 5-OCHF$_2$-pyridine-2-yl |
| Me | Me | Cl | H | CH(Me) | 4-position | H | 0 | 5-OCF$_2$CHF$_2$-pyridine-2-yl |
| Me | Me | Cl | H | CH(Me) | 4-position | H | 0 | 5-OCH(CF$_3$)$_2$-pyridine-2-yl |
| Me | Me | Cl | H | CH(Me) | 4-position | H | 0 | 5-OCH$_2$CF$_3$-pyridine-2-yl |
| Me | Me | Cl | H | CH(Me) | 4-position | H | 0 | 5-OAc-pyridine-2-yl |
| Me | Me | Cl | H | CH(Me) | 4-position | H | 0 | 5-OCOOMe-pyridine-2-yl |
| Me | Me | Cl | H | CH(Me) | 4-position | H | 0 | 5-COOH-pyridine-2-yl |
| Me | Me | Cl | H | CH(Me) | 4-position | H | 0 | 5-COOMe-pyridine-2-yl |
| Me | Me | Cl | H | CH(Me) | 4-position | H | 0 | 5-CONH$_2$-pyridine-2-yl |
| Me | Me | Cl | H | CH(Me) | 4-position | H | 0 | 5-CONHMe-pyridirie-2-yl |
| Me | Me | Cl | H | CH(Me) | 4-position | H | 0 | 5-CON(Me)$_2$-pyridine-2-yl |
| Me | Me | Cl | H | CH(Me) | 4-position | H | 0 | 5-Ac-pyridine-2-yl |
| Me | Me | Cl | H | CH(Me) | 4-position | H | 0 | 5-C(Me)=NOH-pyridine-2-yl |
| Me | Me | Cl | H | CH(Me) | 4-position | H | 0 | 5-C(Me)=NOMe-pyridine-2-yl |
| Me | Me | Cl | H | CH(Me) | 4-position | H | 0 | 5-C(Me)=NOCH$_2$Ph-pyridine-2-yl |
| Me | Me | Cl | H | CH(Me) | 4-position | H | 0 | 3,5-F$_2$-pyridine-2-yl |
| Me | Me | Cl | H | CH(Me) | 4-position | H | 0 | 3,5-Cl$_2$-pyridine-2-yl |
| Me | Me | Cl | H | CH(Me) | 4-position | H | 0 | 3,5-Br$_2$-pyridine-2-yl |
| Me | Me | Cl | H | CH(Me) | 4-position | H | 0 | 3,5-I$_2$-pyridine-2-yl |
| Me | Me | Cl | H | CH(Me) | 4-position | H | 0 | 3-F-5-Cl-pyridine-2-yl |
| Me | Me | Cl | H | CH(Me) | 4-position | H | 0 | 3-Br-5-Cl-pyridine-2-yl |
| Me | Me | Cl | H | CH(Me) | 4-position | H | 0 | 3-F-5-CF$_3$-pyridine-2-yl |
| Me | Me | Cl | H | CH(Me) | 4-position | H | 0 | 3-Cl-5-CF$_3$-pyridine-2-yl |
| Me | Me | Cl | H | CH(Me) | 4-position | H | 0 | 2-F-pyridine-5-yl |
| Me | Me | Cl | H | CH(Me) | 4-position | H | 0 | 2-Cl-pyridine-5-yl |
| Me | Me | Cl | H | CH(Me) | 4-position | H | 0 | 2-Br-pyridine-5-yl |
| Me | Me | Cl | H | CH(Me) | 4-position | H | 0 | 2-I-pyridine-5-yl |
| Me | Me | Cl | H | CH(Me) | 4-position | H | 0 | 2-CF(CF$_3$)$_2$-pyridine-5-yl |
| Me | Me | Cl | H | CH(Me) | 4-position | H | 0 | 2-CH(CF$_3$)$_2$ pyridine-5-yl |
| Me | Me | Cl | H | CH(Me) | 4-position | H | 0 | 2-CF$_2$CF$_3$-pyridine-5-yl |
| Me | Me | Cl | H | CH(Me) | 4-position | H | 0 | 2-CF$_3$-pyridine-5-yl |
| Me | Me | Cl | H | CH(Me) | 4-position | H | 0 | 2-CF$_3$-pyridine-6-yl |
| Me | Me | Cl | H | CH(Me) | 4-position | H | 0 | 6-F-pyridazine-3-yl |
| Me | Me | Cl | H | CH(Me) | 4-position | H | 0 | 6-Cl-pyridazine-3-yl |
| Me | Me | Cl | H | CH(Me) | 4-position | H | 0 | 6-Br-pyridazine-3-yl |
| Me | Me | Cl | H | CH(Me) | 4-position | H | 0 | 6-I-pyridazine-3-yl |

Table 6-1 (continued)

| $R^1$ | $R^2$ | $R^3$ | $R^4$ | Y | Bond | (X)n | Q | Ar |
|---|---|---|---|---|---|---|---|---|
| Me | Me | Cl | H | CH(Me) | 4-position | H | 0 | 6-$CF_3$-pyridazine-3-yl |
| Me | Me | Cl | H | CH(Me) | 4-position | H | 0 | 6-CN-pyridazine-3-yl |
| Me | Me | Cl | H | CH(Me) | 4-position | H | 0 | 6-COOH-pyridazine-3-yl |
| Me | Me | Cl | H | CH(Me) | 4-position | H | 0 | 6-COOMe-pyridazine-3-yl |
| Me | Me | Cl | H | CH(Me) | 4-position | H | 0 | 6-CONHMe-pyridazine-3-yl |
| Me | Me | Cl | H | CH(Me) | 4-position | H | 0 | 6-$CONMe_2$-pyridazine-3-yl |
| Me | Me | Cl | H | CH(Me) | 4-position | H | 0 | 6-OH-pyridazine-3-yl |
| Me | Me | Cl | H | CH(Me) | 4-position | H | 0 | 6-OMe-pyridazine-3-yl |
| Me | Me | Cl | H | CH(Me) | 4-position | H | 0 | 6-$OCF_3$-pyridazine-3-yl |
| Me | Me | Cl | H | CH(Me) | 4-position | H | 0 | 6-$OCHF_2$-pyridazine-3-yl |
| Me | Me | Cl | H | CH(Me) | 4-position | H | 0 | 6-$OCF_2CHF_2$-pyridazine-3-yl |
| Me | Me | Cl | H | CH(Me) | 4-position | H | 0 | 6-$CF(CF_3)_2$-pyridazine-3-yl |
| Me | Me | Cl | H | CH(Me) | 4-position | H | 0 | 6-$CH(CF_3)_2$-pyridazine-3-yl |
| Me | Me | Cl | H | CH(Me) | 4-position | H | 0 | 6-$CF_2CF_3$-pyridazine-3-yl |
| Me | Me | Cl | H | CH(Me) | 4-position | H | 0 | pyrimidine-2-yl |
| Me | Me | Cl | H | CH(Me) | 4-position | H | 0 | 5-F-pyrimidine-2-yl |
| Me | Me | Cl | H | CH(Me) | 4-position | H | 0 | 5-Cl-pyrimidine-2-yl |
| Me | Me | Cl | H | CH(Me) | 4-position | H | 0 | 5-Br-pyrimidine-2-yl |
| Me | Me | Cl | H | CH(Me) | 4-position | H | 0 | 5-$CF_3$-pyrimidine-2-yl |
| Me | Me | Cl | H | CH(Me) | 4-position | H | 0 | 5-$CF(CF_3)_2$-pyrimidine-2-yl |
| Me | Me | Cl | H | CH(Me) | 4-position | H | 0 | 5-$CH(CF_3)_2$-pyrimidine-2-yl |
| Me | Me | Cl | H | CH(Me) | 4-position | H | 0 | 5-$CF_2CF_3$-pyrimidine-2-yl |
| Me | Me | Cl | H | CH(Me) | 4-position | H | 0 | 5-$NO_2$-pyrimidine-2-yl |
| Me | Me | Cl | H | CH(Me) | 4-position | H | 0 | 5-CN-pyrimidine-2-yl |
| Me | Me | Cl | H | CH(Me) | 4-position | H | 0 | pyrazine-2-yl |
| Me | Me | Cl | H | CH(Me) | 4-position | H | 0 | 5-$CF_3$-pyrazine-2-yl |
| Me | Me | Cl | H | CH(Me) | 4-position | H | 0 | 4,6-$Cl_2$-triazine-2-yl |
| Me | Me | Cl | H | CH(Me) | 4-position | H | 0 | thiophene-2-yl |
| Me | Me | Cl | H | CH(Me) | 4-position | H | 0 | 5-Cl-thiophene-2-yl |
| Me | Me | Cl | H | CH(Me) | 4-position | H | 0 | 5-$CF_3$-thiophene-2-yl |
| Me | Me | Cl | H | CH(Me) | 4-position | H | 0 | 1-Me-pyrazole-5-yl |
| Me | Me | Cl | H | CH(Me) | 4-position | H | 0 | 1-Me-3-Cl-pyrazole-5-yl |
| Me | Me | Cl | H | CH(Me) | 4-position | H | 0 | 1-Me-3-$CF_3$-pyrazole-5-yl |
| Me | Me | Cl | H | CH(Me) | 4-position | H | 0 | 3-$CF_3$-isoxazole-5-yl |
| Me | Me | Cl | H | CH(Me) | 4-position | H | 0 | 5-$CF_3$-isoxazole-3-yl |
| Me | Me | Cl | H | CH(Me) | 4-position | H | 0 | 3-$CF_3$-isoxazole-5-yl |
| Me | Me | Cl | H | CH(Me) | 4-position | H | 0 | 5-Cl-thiazole-2-yl |
| Me | Me | Cl | H | CH(Me) | 4-position | H | 0 | 5-Br-thiazole-2-yl |
| Me | Me | Cl | H | CH(Me) | 4-position | H | 0 | 5-$NO_2$-thiazole-2-yl |
| Me | Me | Cl | H | CH(Me) | 4-position | H | 0 | 5-CN-thiazole-2-yl |
| Me | Me | Cl | H | CH(Me) | 4-position | H | 0 | 4-Cl-thiazole-2-yl |
| Me | Me | Cl | H | CH(Me) | 4-position | H | 0 | 4-Br-thiazole-2-yl |
| Me | Me | Cl | H | CH(Me) | 4-position | H | 0 | 4-$CF_3$-thiazole-2-yl |
| Me | Me | Cl | H | CH(Me) | 4-position | H | 0 | 1-Me-4-Cl-imidazole-2-yl |

Table 6-1 (continued)

| $R^1$ | $R^2$ | $R^3$ | $R^4$ | Y | Bond | (X)n | Q | Ar |
|---|---|---|---|---|---|---|---|---|
| Me | Me | Cl | H | CH(Me) | 4-position | H | 0 | 1-Me-4-Br-imidazole-2-yl |
| Me | Me | Cl | H | CH(Me) | 4-position | H | 0 | 1-Me-3-Cl-triazole-5-yl |
| Me | Me | Cl | H | CH(Me) | 4-position | H | 0 | quinoline-2-yl |
| Me | Me | Cl | H | CH(Me) | 4-position | H | 0 | benzothiazole-2-yl |
| Me | Me | Cl | H | CH(Me) | 4-position | H | 0 | 1-Me-benzothiazole-2-yl |
| Me | Me | Cl | H | CH(Me) | 4-position | H | 0 | benzoxazole-2-yl |
| Me | Me | Cl | H | CH(Me) | 4-position | H | 0 | 1-Me-indole-2-yl |
| Me | Me | Cl | H | CH(Me) | 4-position | H | 0 | benzofuran-2-yl |
| Me | Me | Cl | H | CH(Me) | 4-position | H | 0 | benzothiophene-2-yl |
| Me | Me | Cl | H | CH(Me) | 4-position | H | 0 | 4-F-Ph |
| Me | Me | Cl | H | CH(Me) | 4-position | H | 0 | 4-Cl-Ph |
| Me | Me | Cl | H | CH(Me) | 4-position | H | 0 | 4-Br-Ph |
| Me | Me | Cl | H | CH(Me) | 4-position | H | 0 | 4-Me-Ph |
| Me | Me | Cl | H | CH(Me) | 4-position | H | 0 | 4-OMe-Ph |
| Me | Me | Cl | H | CH(Me) | 4-position | H | 0 | 4-$CF_3$-Ph |
| Me | Me | Cl | H | CH(Me) | 4-position | H | 0 | 4-$CF_2CF_3$-Ph |
| Me | Me | Cl | H | CH(Me) | 4-position | H | 0 | 4-$CF(CF_3)_2$-Ph |
| Me | Me | Cl | H | CH(Me) | 4-position | H | 0 | 4-$CH(CF_3)_2$-Ph |
| Me | Me | Cl | H | CH(Me) | 4-position | H | 0 | 4-$OCF_3$-Ph |
| Me | Me | Cl | H | CH(Me) | 4-position | H | 0 | 4-$OCH_2CF_3$-Ph |
| Me | Me | Cl | H | CH(Me) | 4-position | H | 0 | 4-$OCF_2CHF_2$-Ph |
| Me | Me | Cl | H | CH(Me) | 4-position | H | 0 | 4-$OCHF_2$-Ph |
| Me | Me | Cl | H | CH(Me) | 4-position | H | 0 | 4-OCH(Me)$CF_3$-Ph |
| Me | Me | Cl | H | CH(Me) | 4-position | H | 0 | 4-$OCH_2CF_2CHF_2$-Ph |
| Me | Me | Cl | H | CH(Me) | 4-position | H | 0 | 4-OAc-Ph |
| Me | Me | Cl | H | CH(Me) | 4-position | H | 0 | 4-OCOOMe-Ph |
| Me | Me | Cl | H | CH(Me) | 4-position | H | 0 | 4-CN-Ph |
| Me | Me | Cl | H | CH(Me) | 4-position | H | 0 | 4-COOH-Ph |
| Me | Me | Cl | H | CH(Me) | 4-position | H | 0 | 4-COOMe-P |
| Me | Me | Cl | H | CH(Me) | 4-position | H | 0 | 4-CONHMe-Ph |
| Me | Me | Cl | H | CH(Me) | 4-position | H | 0 | 4-$CONMe_2$-Ph |
| Me | Me | Cl | H | CH(Me) | 4-position | H | 0 | 4-$NO_2$-Ph |
| Me | Me | Cl | H | CH(Me) | 4-position | H | 0 | 4-$NH_2$-Ph |
| Me | Me | Cl | H | CH(Me) | 4-position | H | 0 | 4-NHMe-Ph |
| Me | Me | Cl | H | CH(Me) | 4-position | H | 0 | 4-$NMe_2$-Ph |
| Me | Me | Cl | H | CH(Me) | 4-position | H | 0 | 4-NHCOOMe-Ph |
| Me | Me | Cl | H | CH(Me) | 4-position | H | 0 | 4-NHCOOEt-Ph |
| Me | Me | Cl | H | CH(Me) | 4-position | H | 0 | 4-NHCOO-i-Pr-Ph |
| Me | Me | Cl | H | CH(Me) | 4-position | H | 0 | 4-$NHCOOCH_2CH=CH_2$-Ph |
| Me | Me | Cl | H | CH(Me) | 4-position | H | 0 | 4-NHCOOCRC≡CH-Ph |
| Me | Me | Cl | H | CH(Me) | 4-position | H | 0 | 4-NHCOOPh-Ph |
| Me | Me | Cl | H | CH(Me) | 4-position | H | 0 | 4-$NHCOOCH_2Ph$-Ph |
| Me | Me | Cl | H | CH(Me) | 4-position | H | 0 | 4-NHCO-t-Bu-Ph |
| Me | Me | Cl | H | CH(Me) | 4-position | H | 0 | 4-$NHCOCF_3$-Ph |
| Me | Me | Cl | H | CH(Me) | 4-position | H | 0 | 4-$NHCOCH_2Cl$-Ph |
| Me | Me | Cl | H | CH(Me) | 4-position | H | 0 | 4-NHAc-Ph |
| Me | Me | Cl | H | CH(Me) | 4-position | H | 0 | 4-NHCOCH=CHMe-Ph |

Table 6-1 (continued)

| $R^1$ | $R^2$ | $R^3$ | $R^4$ | Y | Bond | (X)n | Q | Ar |
|---|---|---|---|---|---|---|---|---|
| Me | Me | Cl | H | CH(Me) | 4-position | H | 0 | 4-NHCO-c-Pr-Ph |
| Me | Me | Cl | H | CH(Me) | 4-position | H | 0 | 4-NHCOPh-Ph |
| Me | Me | Cl | H | CH(Me) | 4-position | H | 0 | 4-NHCO(thiophene-2-yl)-Ph |
| Me | Me | Cl | H | CH(Me) | 4-position | H | 0 | 4-NHCO(pyridine-2-yl)-Ph |
| Me | Me | Cl | H | CH(Me) | 4-position | H | 0 | 4-$NHSO_2$Me-Ph |
| Me | Me | Cl | H | CH(Me) | 4-position | H | 0 | 4-$NHSO_2$-c-Pr-Ph |
| Me | Me | Cl | H | CH(Me) | 4-position | H | 0 | 4-$NHSO_2CF_3$-Ph |
| Me | Me | Cl | H | CH(Me) | 4-position | H | 0 | 4-$NHSO_2$Ph-Ph |
| Me | Me | Cl | H | CH(Me) | 4-position | H | 0 | 4-$SCF_3$-Ph |
| Me | Me | Cl | H | CH(Me) | 4-position | H | 0 | 4-$SOCF_3$-Ph |
| Me | Me | Cl | H | CH(Me) | 4-position | H | 0 | 4-$SO_2CF_3$-Ph |
| Me | Me | Cl | H | CH(Me) | 4-position | H | 0 | 4-SMe-Ph |
| Me | Me | Cl | H | CH(Me) | 4-position | H | 0 | 4-SOMe-P |
| Me | Me | Cl | H | CH(Me) | 4-position | H | 0 | 4-$SO_2$Me-Ph |
| Me | Me | Cl | H | CH(Me) | 4-position | H | 0 | 4-t-Bu-Ph |
| Me | Me | Cl | H | CH(Me) | 4-position | H | 0 | 4-Ac-Ph |
| Me | Me | Cl | H | CH(Me) | 4-position | H | 0 | 4-(C(Me)=NOH)-Ph |
| Me | Me | Cl | H | CH(Me) | 4-position | H | 0 | 4-(C(Me)=NOMe)-Ph |
| Me | Me | Cl | H | CH(Me) | 4-position | H | 0 | 4-(C(Me)=$NOCH_2$ CR=$CH_2$)-Ph |
| Me | Me | Cl | H | CH(Me) | 4-position | H | 0 | 4-(C(Me)=NO-t-Bu)-Ph |
| Me | Me | Cl | H | CH(Me) | 4-position | H | 0 | 4-(C(Me)=$NOCH_2$ (4-F-Ph) ) -Ph |
| Me | Me | Cl | H | CH(Me) | 4-position | H | 0 | 4-(C(Me)=$NOCH_2$(2,4-$Cl_2$-Ph))-Ph |
| Me | Me | Cl | H | CH(Me) | 4-position | H | 0 | 4-(C(Me)=$NOCH_2$(2,6-$Cl_2$-Ph))-Ph |
| Me | Me | Cl | H | CH(Me) | 4-position | H | 0 | 4-(C(Me)=$NOCH_2C\equiv$ CH)-Ph |
| Me | Me | Cl | H | CH(Me) | 4-position | H | 0 | 4-(C(Me)=NO-t-$C_5$ $H_{11}$)-Ph |
| Me | Me | Cl | H | CH(Me) | 4-position | H | 0 | 4-(C(Me)=$NOCH_2$(4-Cl-Ph))-Ph |
| Me | Me | Cl | H | CH(Me) | 4-position | H | 0 | 4-(C(Me)=$NOCH_2$(4-CF 3-Ph))-Ph |
| Me | Me | Cl | H | CH(Me) | 4-position | H | 0 | 4-(C(Me)=$NOCH_2$(2,3-$Cl_2$-Ph))-Ph |
| Me | Me | Cl | H | CH(Me) | 4-position | H | 0 | 4-(C(Me)=$NOCR_2$(2,5-$Cl_2$-Ph))-Ph |
| Me | Me | Cl | H | CH(Me) | 4-position | H | 0 | 4-(C(Me)=$NOCH_2$(2-Cl-pyridine-5-yl))-Ph |

Note: "Bond" in the Tables means the bonded position of the group -Q-Ar.

Table 6-2

Table 6-2 represents 198 compounds wherein, in the general formula (I-5), $R^1$ represents Me, $R^2$ does Et, $R^3$ does Cl, $R^4$ does H, Q does an oxygen atom ad the bonded position thereof being a 4-position, (X)n does H, Y does CH(Me), and Ar corresponds to each of all the substituents shown in Table 6-1.

Table 6-3

Table 6-3 represents 198 compounds wherein, in the general formula (I-5), $R^1$ represents Me, $R^2$ does i-Pr, $R^3$ does Cl, $R^4$ does H, Q does an oxygen atom and the bonded position thereof being a 4-position, (X)n does H, Y does CH(Me), and Ar corresponds to each of all the substituents shown in Table 6-1.

Table 6-4

Table 6-4 represents 198 compounds wherein, in the general formula (I-5), $R^1$ represents Me, $R^2$ does n-Pr, $R^3$ does Cl, $R^4$ does H, Q does an oxygen atom and the bonded position thereof being a 4-position, (X)n does H, Y does CH(Me), and Ar corresponds to each of all the substituents shown in Table 6-1.

Table 6-5

Table 6-5 represents 198 compounds wherein, in the general formula (I-5), $R^1$ represents Me, $R^2$ does t-Bu, $R^3$ does Cl, $R^4$ does H, Q does an oxygen atom and the bonded position thereof being a 4-position, (X)n does H, Y does CH(Me), and Ar corresponds to each of all the substituents shown in Table 6-1.

Table 6-6

Table 6-6 represents 198 compounds wherein, in the general formula (I-5), $R^1$ represents Me, $R^2$ does n-Bu, $R^3$ does Cl, $R^4$ does H, Q does an oxygen atom and the bonded position thereof being a 4-position, (X)n does H, Y does CH(Me), and Ar corresponds to each of all the substituents shown in Table 6-1.

Table 6-7

Table 6-7 represents 198 compounds wherein, in the general formula (I-5), $R^1$ represents Me, $R^2$ does $CF_3$, $R^3$ does Cl, $R^4$ does H, Q does an oxygen atom and the bonded position thereof being a 4-position, (X)n does H, Y does CH(Me), and Ar corresponds to each of all the substituents shown in Table 6-1.

Table 6-8

Table 6-8 represents 198 compounds wherein, in the general formula (I-5), $R^1$ represents Me, $R^2$ does OMe, $R^3$ does Cl, $R^4$ does H, Q does an oxygen atom and the bonded position thereof being a 4-position, (X)n does H, Y does CH(Me), and Ar corresponds to each of all the substituents shown in Table 6-1.

Table 6-9

Table 6-9 represents 198 compounds wherein, in the general formula (I-5), $R^1$ represents Me, $R^2$ does $OCHF_2$, $R^3$ does Cl, $R^4$ does H, Q does an oxygen atom and the bonded position thereof being a 4-position, (X)n does H, Y does CH(Me), and Ar corresponds to each of all the substituents shown in Table 6-1.

Table 6-10

Table 6-10 represents 198 compounds wherein, in the general formula (I-5), $R^1$ represents Me, $R^2$ does O-i-Pr, $R^3$ does Cl, $R^4$ does H, Q does an oxygen atom and the bonded position thereof being a 4-position, (X)n does H, Y does CH(Me), and Ar corresponds to each of all the substituents shown in Table 6-1.

Table 6-11

Table 6-11 represents 198 compounds wherein, in the general formula (I-5), $R^1$ represents Me, $R^2$ does Cl, $R^3$ does Cl, $R^4$ does H, Q does an oxygen atom and the bonded position thereof being a 4-position, (X)n does H, Y does CH(Me), and Ar corresponds to each of all the substituents shown in Table 6-1.

Table 6-12

Table 6-12 represents 198 compounds wherein, in the general formula (I-5), $R^1$ represents Me, $R^2$ does Ph, $R^3$ does Cl, $R^4$ does H, Q does an oxygen atom and the bonded position thereof being a 4-position, (X)n does H, Y does CH(Me), and Ar corresponds to each of all the substituents shown in Table 6-1.

Table 6-13

Table 6-13 represents 198 compounds wherein, in the general formula (I-5), $R^1$ represents Me, $R^2$ does 2-Cl-Ph, $R^3$ does Cl, $R^4$ does H, Q does an oxygen atom and the bonded position thereof being a 4-position, (X)n does H, Y does CH(Me), and Ar corresponds to each of all the substituents shown in Table 6-1.

Table 6-14

Table 6-14 represents 198 compounds wherein, in the general formula (I-5), $R^1$ represents Me, $R^2$ does 3-Cl-Ph, $R^3$ does Cl, $R^4$ does H, Q does an oxygen atom and the bonded position thereof being a 4-position, (X)n does H,

Y does CH(Me), and Ar corresponds to each of all the substituents shown in Table 6-1.

Table 6-15

Table 6-15 represents 198 compounds wherein, in the general formula (I-5), $R^1$ represents Me, $R^2$ does 4-Cl-Ph, $R^3$ does Cl, $R^4$ does H, Q does an oxygen atom and the bonded position thereof being a 4-position, (X)n does H, Y does CH(Me), and Ar corresponds to each of all the substituents shown in Table 6-1.

Table 6-16

Table 6-16 represents 198 compounds wherein, in the general formula (I-5), $R^1$ represents Me, $R^2$ does 2,4-$Cl_2$-Ph, $R^3$ does Cl, $R^4$ does H, Q does an oxygen atom and the bonded position thereof being a 4-position, (X)n does H, Y does CH(Me), and Ar corresponds to each of all the substituents shown in Table 6-1.

Table 6-17

Table 6-17 represents 198 compounds wherein, in the general formula (I-5), $R^1$ represents Me, $R^2$ does 2-Me-Ph, $R^3$ does Cl, $R^4$ does H, Q does an oxygen atom and the bonded position thereof being a 4-position, (X)n does H, Y does CH(Me), and Ar corresponds to each of all the substituents shown in Table 6-1.

Table 6-18

Table 6-18 represents 198 compounds wherein, in the general formula (I-5), $R^1$ represents Me, $R^2$ does 3-Me-Ph, $R^3$ does Cl, $R^4$ does H, Q does an oxygen atom and the bonded position thereof being a 4-position, (X)n does H, Y does CH(Me), and Ar corresponds to each of all the substituents shown in Table 6-1.

Table 6-19

Table 6-19 represents 198 compounds wherein, in the general formula (I-5), $R^1$ represents Me, $R^2$ does 4-Me-Ph, $R^3$ does Cl, $R^4$ does H, Q does an oxygen atom and the bonded position thereof being a 4-position, (X)n does H, Y does CH(Me), and Ar corresponds to each of all the substituents shown in Table 6-1.

Table 6-20

Table 6-20 represents 198 compounds wherein, in the general formula (I-5), $R^1$ represents Me, $R^2$ does 2-OMe-Ph, $R^3$ does Cl, $R^4$ does H, Q does an oxygen atom and the bonded position thereof being a 4-position, (X)n does H, Y does CH(Me), and Ar corresponds to each of all the substituents shown in Table 6-1.

Table 6-21

Table 6-21 represents 198 compounds wherein, in the general formula (I-5), $R^1$ represents Me, $R^2$ does 3-OMe-Ph, $R^3$ does Cl, $R^4$ does H, Q does an oxygen atom and the bonded position thereof being a 4-position, (X)n does H, Y does CH(Me), and Ar corresponds to each of all the substituents shown in Table 6-1.

Table 6-22

Table 6-22 represents 198 compounds wherein, in the general formula (I-5), $R^1$ represents Me, $R^2$ does 4-OMe-Ph, $R^3$ does Cl, $R^4$ does H, Q does an oxygen atom and the bonded position thereof being a 4-position, (X)n does H, Y does CH(Me), and Ar corresponds to each of all the substituents shown in Table 6-1.

Table 6-23

Table 6-23 represents 198 compounds wherein, in the general formula (I-5), $R^1$ represents Me, $R^2$ does H, $R^3$ does Cl, $R^4$ does H, Q does an oxygen atom and the bonded position thereof being a 4-position, (X)n does 3-OMe, Y does CH(Me), and Ar corresponds to each of all the substituents shown in Table 6-1.

Table 6-24

Table 6-24 represents 198 compounds wherein, in the general formula (I-5), $R^1$ represents H, $R^2$ does H, $R^3$ does Cl, $R^4$ does H, Q does an oxygen atom and the bonded position thereof being a 4-position, (X)n does 3-OMe, Y does CH(Me), and Ar corresponds to each of all the substituents shown in Table 6-1.

Table 6-25

Table 6-25 represents 198 compounds wherein, in the general formula (I-5), $R^1$ represents Et, $R^2$ does H, $R^3$ does Cl, $R^4$ does H, Q does an oxygen atom and the bonded position thereof being a 4-position, (X)n does 3-OMe, Y does CH(Me), and Ar corresponds to each of all the substituents shown in Table 6-1.

Table 6-26

Table 6-26 represents 198 compounds wherein, in the general formula (I-5), $R^1$ represents i-Pr, $R^2$ does H, $R^3$ does Cl, $R^4$ does H, Q does an oxygen atom and the bonded position thereof being a 4-position, (X)n does 3-OMe, Y does CH(Me), and Ar corresponds to each of all the substituents shown in Table 6-1.

Table 6-27

Table 6-27 represents 198 compounds wherein, in the general formula (I-5), $R^1$ represents n-Pr, $R^2$ does H, $R^3$ does Cl, $R^4$ does H, Q does an oxygen atom and the bonded position thereof being a 4-position, (X)n does 3-OMe, Y does CH(Me), and Ar corresponds to each of all the substituents shown in Table 6-1.

Table 6-28

Table 6-28 represents 198 compounds wherein, in the general formula (I-5), $R^1$ represents t-Bu, $R^2$ does H, $R^3$ does Cl, $R^4$ does H, Q does an oxygen atom and the bonded position thereof being a 4-position, (X)n does 3-OMe, Y does CH(Me), and Ar corresponds to each of all the substituents shown in Table 6-1.

Table 6-29

Table 6-29 represents 198 compounds wherein, in the general formula (I-5), $R^1$ represents n-Bu, $R^2$ does H, $R^3$ does Cl, $R^4$ does H, Q does an oxygen atom and the bonded position thereof being a 4-position, (X)n does 3-OMe, Y does CH(Me), and Ar corresponds to each of all the substituents shown in Table 6-1.

Table 6-30

Table 6-30 represents 198 compounds wherein, in the general formula (I-5), $R^1$ represents $CH=CH_2$, $R^2$ does H, $R^3$ does Cl, $R^4$ does H, Q does an oxygen atom and the bonded position thereof being a 4-position, (X)n does 3-OMe, Y does CH(Me), and Ar corresponds to each of all the substituents shown in Table 6-1.

Table 6-31

Table 6-31 represents 198 compounds wherein, in the general formula (I-5), $R^1$ represents $CH_2CH=CH_2$, $R^2$ does H, $R^3$ does Cl, $R^4$ does H, Q does an oxygen atom and the bonded position thereof being a 4-position, (X)n does 3-OMe, Y does CH(Me), and Ar corresponds to each of all the substituents shown in Table 6-1.

Table 6-32

Table 6-32 represents 198 compounds wherein, in the general formula (I-5), $R^1$ represents $CH_2C{\equiv}CH$, $R^2$ does H, $R^3$ does Cl, $R^4$ does H, Q does an oxygen atom and the bonded position thereof being a 4-position, (X)n does 3-OMe, Y does CH(Me), and Ar corresponds to each of all the substituents shown in Table 6-1.

Table 6-33

Table 6-33 represents 198 compounds wherein, in the general formula (I-5), $R^1$ represents $CH_2CN$, $R^2$ does H, $R^3$ does Cl, $R^4$ does H, Q does an oxygen atom and the bonded position thereof being a 4-position, (X)n does

3-OMe, Y does CH(Me), and Ar corresponds to each of all the substituents shown in Table 6-1.

Table 6-34

Table 6-34 represents 198 compounds wherein, in the general formula (I-S), $R^1$ represents $CH_2CF_3$, $R^2$ does H, $R^3$ does Cl, $R^4$ does H, Q does an oxygen atom an the bonded position thereof being a 4-position, (X)n does 3-OMe, Y does CH(Me), and Ar corresponds to each of all the substituents shown in Table 6-1.

Table 6-35

Table 6-35 represents 198 compounds wherein, in the general formula (I-5), $R^1$ represents $CH_2Ph$, $R^2$ does H, $R^3$ does Cl, $R^4$ does H, Q does an oxygen atom and the bonded position thereof being a 4-position, (X)n does 3-OMe, Y does CH(Me), and Ar corresponds to each of all the substituents shown in Table 6-1.

Table 6-36

Table 6-36 represents 198 compounds wherein, in the general formula (I-5), $R^1$ represents Ph, $R^2$ does H, $R^3$ does Cl, $R^4$ does H, Q does an oxygen atom and the bonded position thereof being a 4-position, (X)n does 3-OMe, Y does CH(Me), and Ar corresponds to each of all the substituents shown in Table 6-1.

Table 6-37

Table 6-37 represents 198 compounds wherein, in the general formula (I-5), $R^1$ represents 2-Cl-Ph, $R^2$ does H, $R^3$ does Cl, $R^4$ does H, Q does an oxygen atom and the bonded position thereof being a 4-position, (X)n does 3-OMe, Y does CH(Me), and Ar corresponds to each of all the substituents shown in Table 6-1.

Table 6-38

Table 6-38 represents 198 compounds wherein, in the general formula (I-5), $R^1$ represents 3-Cl-Ph, $R^2$ does H, $R^3$ does Cl, $R^4$ does H, Q does an oxygen atom and the bonded position thereof being a 4-position, (X)n does 3-OMe, Y does CH(Me), and Ar corresponds to each of all the substituents shown in Table 6-1.

Table 6-39

Table 6-39 represents 198 compounds wherein, in the general formula (I-5), $R^1$ represents 4-Cl-Ph, $R^2$ does H, $R^3$ does Cl, $R^4$ does H, Q does an oxygen atom and the bonded position thereof being a 4-position, (X)n does 3-OMe, Y does CH(Me), and Ar corresponds to each of all the substituents shown in Table 6-1.

Table 6-40

Table 6-40 represents 198 compounds wherein, in the general formula (I-5), $R^1$ represents 2,4-$Cl_2$-Ph, $R^2$ does H, $R^3$ does Cl, $R^4$ does H, Q does an oxygen atom and the bonded position thereof being a 4-position, (X)n does 3-OMe, Y does CH(Me), and Ar corresponds to each of all the substituents shown in Table 6-1.

Table 6-41

Table 6-41 represents 198 compounds wherein, in the general formula (I-5), $R^1$ represents Ac, $R^2$ does H, $R^3$ does Cl, $R^4$ does H, Q does an oxygen atom and the bonded position thereof being a 4-position, (X)n does 3-OMe, Y does CH(Me), and Ar corresponds to each of all the substituents shown in Table 6-1.

Table 6-42

Table 6-42 represents 198 compounds wherein, in the general formula (I-5), $R^1$ represents COPh, $R^2$ does H, $R^3$ does Cl, $R^4$ does H, Q does an oxygen atom and the bonded position thereof being a 4-position, (X)n does 3-OMe, Y does CH(Me), and Ar corresponds to each of all the substituents shown in Table 6-1.

Table 6-43

Table 6-43 represents 198 compounds wherein, in the general formula (I-5), $R^1$ represents $CH_2CH_2F$, $R^2$ does H, $R^3$ does Cl, $R^4$ does H, Q does an oxygen atom and the bonded position thereof being a 4-position, (X)n does 3-OMe, Y does CH(Me), and Ar corresponds to each of all the substituents shown in Table 6-1.

Table 6-44

Table 6-44 represents 198 compounds wherein, in the general formula (I-5), $R^1$ represents $CH_2CH_2Cl$, $R^2$ does H, $R^3$ does Cl, $R^4$ does H, Q does an oxygen atom and the bonded position thereof being a 4-position, (X)n does 3-OMe, Y does CH(Me), and Ar corresponds to each of all the substituents shown in Table 6-1.

Table 6-45

Table 6-45 represents 198 compounds wherein, in the general formula (I-5), $R^1$ represents $CH_2CH_2Br$, $R^2$ does H, $R^3$ does Cl, $R^4$ does H, Q does an oxygen atom and the bonded position thereof being a 4-position, (X)n does 3-OMe, Y does CH(Me), and Ar corresponds to each of all the substituents shown in Table 6-1.

Table 6-46

Table 6-46 represents 198 compounds wherein, in the general formula (1-5), $R^1$ represents $CH_2CH_2OH$, $R^2$ does H, $R^3$ does Cl, $R^4$ does H, Q does an oxygen atom and the bonded position thereof being a 4-position, (X)n does 3-OMe, Y does CH(Me), and Ar corresponds to each of all the substituents shown in Table 6-1.

Table 6-47

Table 6-47 represents 198 compounds wherein, in the general formula (I-5), $R^1$ represents $CH_2CH_2OMe$, $R^2$ does H, $R^3$ does Cl, $R^4$ does H, Q does an oxygen atom and the bonded position thereof being a 4-position, (X)n does 3-OMe, Y does CH(Me), and Ar corresponds to each of all the substituents shown in Table 6-1.

Table 6-48

Table 6-48 represents 198 compounds wherein, in the general formula (I-5), $R^1$ represents $CH_2OMe$, $R^2$ does H, $R^3$ does Cl, $R^4$ does H, Q does an oxygen atom and the bonded position thereof being a 4-position, (X)n does 3-OMe, Y does CH(Me), and Ar corresponds to each of all the substituents shown in Table 6-1.

Table 6-49

Table 6-49 represents 198 compounds wherein, in the general formula (I-5), $R^1$ represents $CH_2OEt$, $R^2$ does H, $R^3$ does Cl, $R^4$ does H, Q does an oxygen atom and the bonded position thereof being a 4-position, (X)n does 3-OMe, Y does CH(Me), and Ar corresponds to each of all the substituents shown in Table 6-1.

Table 6-50

Table 6-50 represents 198 compounds wherein, in the general formula (I-5), $R^1$ represents $CH_2COOH$, $R^2$ does H, $R^3$ does Cl, $R^4$ does H, Q does an oxygen atom and the bonded position thereof being a 4-position, (X)n does 3-OMe, Y does CH(Me), and Ar corresponds to each of all the substituents shown in Table 6-1.

Table 6-51

Table 6-51 represents 198 compounds wherein, in the general formula (I-5), $R^1$ represents $CH_2COOEt$, $R^2$ does H, $R^3$ does Cl, $R^4$ does H, Q does an oxygen atom and the bonded position thereof being a 4-position, (X)n does 3-OMe, Y does CH(Me), and Ar corresponds to each of all the substituents shown in Table 6-1.

Table 6-52

Table 6-52 represents 198 compounds wherein, in the general formula (I-5), $R^1$ represents $CH_2CONHMe$, $R^2$ does H, $R^3$ does Cl, $R^4$ does H, Q does an oxygen atom and the bonded position thereof being a 4-position, (X)n does

3-OMe, Y does CH(Me), and Ar corresponds to each of all the substituents shown in Table 6-1.

Table 6-53

Table 6-53 represents 198 compounds wherein, in the general formula (I-5), $R^1$ represents $CH_2CONMe_2$, $R^2$ does H, $R^3$ does Cl, $R^4$ does H, Q does an oxygen atom and the bonded position thereof being a 4-position, (X)n does 3-OMe, Y does CH(Me), and Ar corresponds to each of all the substituents shown in Table 6-1.

Table 6-54

Table 6-54 represents 198 compounds wherein, in the general formula (I-5), $R^1$ represents $CH_2CHO$, $R^2$ does H, $R^3$ does Cl, $R^4$ does H, Q does an oxygen atom and the bonded position thereof being a 4-position, (X)n does 3-OMe, Y does CH(Me), and Ar corresponds to each of all the substituents shown in Table 6-1.

Table 6-55

Table 6-55 represents 198 compounds wherein, in the general formula (I-5), $R^1$ represents $CH_2CH=NOMe$, $R^2$ does H, $R^3$ does Cl, $R^4$ does H, Q does an oxygen atom and the bonded position thereof being a 4-position, (X)n does 3-OMe, Y does CH(Me), and Ar corresponds to each of all the substituents shown in Table 6-1.

Table 6-56

Table 6-56 represents 198 compounds wherein, in the general formula (I-5), $R^1$ represents Et, $R^2$ does H, $R^3$ does H, $R^4$ does H, Q does an oxygen atom and the bonded position thereof being a 4-position, (X)n does 3-OMe, Y does CH(Me), and Ar corresponds to each of all the substituents shown in Table 6-1.

Table 6-57

Table 6-57 represents 198 compounds wherein, in the general formula (I-5), $R^1$ represents Et, $R^2$ does H, $R^3$ does F, $R^4$ does H, Q does an oxygen atom and the bonded position thereof being a 4-position, (X)n does 3-OMe, Y does CH(Me), and Ar corresponds to each of all the substituents shown in Table 6-1.

Table 6-58

Table 6-58 represents 198 compounds wherein, in the general formula (I-5), $R^1$ represents Et, $R^2$ H, $R^3$ does Br, $R^4$ does H, Q does an oxygen atom and the bonded position thereof being a 4-position, (X)n does 3-OMe, Y does CH(Me), and Ar corresponds to each of all the substituents shown in Table 6-1.

Table 6-59

Table 6-59 represents 198 compounds wherein, in the general formula (I-5), $R^1$ represents Et, $R^2$ does H, $R^3$ does I, $R^4$ does H, Q does an oxygen atom and the bonded position thereof being a 4-position, (X)n does 3-OMe, Y does CH(Me), and Ar corresponds to each of all the substituents shown in Table 6-1.

Table 6-60

Table 6-60 represents 198 compounds wherein, in the general formula (I-5), $R^1$ represents Et, $R^2$ does H, $R^3$ does $CF_3$, $R^4$ does H, Q does an oxygen atom and the bonded position thereof being a 4-position, (X)n does 3-OMe, Y does CH(Me), and Ar corresponds to each of all the substituents shown in Table 6-1.

Table 6-61

Table 6-61 represents 198 compounds wherein, in the general formula (I-5), $R^1$ represents Et, $R^2$ does H, $R^3$ does CN, $R^4$ does H, Q does an oxygen atom and the bonded position thereof being a 4-position, (X)n does 3-OMe, Y does CH(Me), and Ar corresponds to each of all the substituents shown in Table 6-1.

Table 6-62

Table 6-62 represents 198 compounds wherein, in the general formula (I-5), $R^1$ represents Et, $R^2$ does H, $R^3$ does COOMe, $R^4$ does H, Q does an oxygen atom and the bonded position thereof being a 4-position, (X)n does 3-OMe, Y does CH(Me), and Ar corresponds to each of all the substituents shown in Table 6-1.

Table 6-63

Table 6-63 represents 198 compounds wherein, in the general formula (I-5), $R^1$ represents Et, $R^2$ does H, $R^3$ does CONHMe, $R^4$ does H, Q does an oxygen atom and the bonded position thereof being a 4-position, (X)n does 3-OMe, Y does CH(Me), and Ar corresponds to each of all the substituents shown in Table 6-1.

Table 6-64

Table 6-64 represents 198 compounds wherein, in the general formula (I-5), $R^1$ represents Et, $R^2$ does H, $R^3$ does $CONMe_2$, $R^4$ does H, Q does an oxygen atom and the bonded position thereof being a 4-position, (X)n does 3-OMe, Y does CH(Me), and Ar corresponds to each of all the substituents shown in Table 6-1.

Table 6-65

Table 6-65 represents 198 compounds wherein, in the general formula (I-5), $R^1$ represents Et, $R^2$ does H, $R^3$ does SMe, $R^4$ does H, Q does an oxygen atom and the bonded position thereof being a 4-position, (X)n does 3-OMe, Y does CH(Me), and Ar corresponds to each of all the substituents shown in Table 6-1.

Table 6-66

Table 6-66 represents 198 compounds wherein, in the general formula (I-5), $R^1$ represents Et, $R^2$ does H, $R^3$ does SPh, $R^4$ does H, Q does an oxygen atom and the bonded position thereof being a 4-position, (X)n does 3-OMe, Y does CH(Me), and Ar corresponds to each of all the substituents shown in Table 6-1.

Table 6-67

Table 6-67 represents 198 compounds wherein, in the general formula (I-5), $R^1$ represents Et, $R^2$ does H, $R^3$ does OH, $R^4$ does H, Q does an oxygen atom and the bonded position thereof being a 4-position, (X)n does 3-OMe, Y does CH(Me), and Ar corresponds to each of all the substituents shown in Table 6-1.

Table 6-68

Table 6-68 represents 198 compounds wherein, in the general formula (I-5), $R^1$ represents Et, $R^2$ does H, $R^3$ does OMe, $R^4$ does H, Q does an oxygen atom and the bonded position thereof being a 4-position, (X)n does 3-OMe, Y does CH(Me), and Ar corresponds to each of all the substituents shown in Table 6-1.

Table 6-69

Table 6-69 represents 198 compounds wherein, in the general formula (I-5), $R^1$ represents Et, $R^2$ does H, $R^3$ does OPh, $R^4$ does H, Q does an oxygen atom and the bonded position thereof being a 4-position, (X)n does 3-OMe, Y does CH(Me), and Ar corresponds to each of all the substituents shown in Table 6-1.

Table 6-70

Table 6-70 represents 198 compounds wherein, in the general formula (I-5), $R^1$ represents Et, $R^2$ does H, $R^3$ does $NMe_2$, $R^4$ does H, Q does an oxygen atom and the bonded position thereof being a 4-position, (X)n does 3-OMe, Y does CH(Me), and Ar corresponds to each of all the substituents shown in Table 6-1.

Table 6-71

Table 6-71 represents 198 compounds wherein, in the general formula (I-5), $R^1$ represents Et, $R^2$ does H, $R^3$ does Cl, $R^4$ does Me, Q does an oxygen atom and the bonded position thereof being a 4-position, (X)n does 3-OMe,

Y does CH(Me), and Ar corresponds to each of all the substituents shown in Table 6-1.

Table 6-72

Table 6-72 represents 198 compounds wherein, in the general formula (I-5), $R^1$ represents Et, $R^2$ does H, $R^3$ does Cl, $R^4$ does Et, Q does an oxygen atom and the bonded position thereof being a 4-position, (X)n does 3-OMe, Y does CH(Me), and Ar corresponds to each of all the substituents shown in Table 6-1.

Table 6-73

Table 6-73 represents 198 compounds wherein, in the general formula (I-5), $R^1$ represents Et, $R^2$ does H, $R^3$ does Cl, $R^4$ does n-Pr, Q does an oxygen atom and the bonded position thereof being a 4-position, (X)n does 3-OMe, Y does CH(Me), and Ar corresponds to each of all the substituents shown in Table 6-1.

Table 6-74

Table 6-74 represents 198 compounds wherein, in the general formula (I-5), $R^1$ represents Et, $R^2$ does H, $R^3$ does Cl, $R^4$ does i-Pr, Q does an oxygen atom and the bonded position thereof being a 4-position, (X)n does 3-OMe, Y does CH(Me), and Ar corresponds to each of all the substituents shown in Table 6-1.

Table 6-75

Table 6-75 represents 198 compounds wherein, in the general formula (I-5), $R^1$ represents Et, $R^2$ does H, $R^3$ does Cl, $R^4$ does $CH_2CH=CH_2$, Q does an oxygen atom and the bonded position thereof being a 4-position, (X)n does 3-OMe, Y does CH(Me), and Ar corresponds to each of all the substituents shown in Table 6-1.

Table 6-76

Table 6-76 represents 198 compounds wherein, in the general formula (I-5), $R^1$ represents Et, $R^2$ does H, $R^3$ does Cl, $R^4$ does $CH_2C{\equiv}CH$, Q does an oxygen atom and the bonded position thereof being a 4-position, (X)n does 3-OMe, Y does CH(Me), and Ar corresponds to each of all the substituents shown in Table 6-1.

Table 6-77

Table 6-77 represents 198 compounds wherein, in the general formula (I-5), $R^1$ represents Et, $R^2$ does H, $R^3$ does Cl, $R^4$ does $CH_2CF_3$, Q does an oxygen atom and the bonded position thereof being a 4-position, (X)n does 3-OMe, Y does CH(Me), and Ar corresponds to each of all the substituents shown in Table 6-1.

Table 6-78

Table 6-78 represents 198 compounds wherein, in the general formula (I-5), $R^1$ represents Et, $R^2$ does H, $R^3$ does Cl, $R^4$ does $CH_2CN$, Q does an oxygen atom and the bonded position thereof being a 4-position, (X)n does 3-OMe, Y does CH(Me), and Ar corresponds to each of all the substituents shown in Table 6-1.

Table 6-79

Table 6-79 represents 198 compounds wherein, in the general formula (I-5), $R^1$ represents Et, $R^2$ does H, $R^3$ does Cl, $R^4$ does $CH_2OMe$, Q does an oxygen atom and the bonded position thereof being a 4-position, (X)n does 3-OMe, Y does CH(Me), and Ar corresponds to each of all the substituents shown in Table 6-1.

Table 6-80

Table 6-80 represents 198 compounds wherein, in the general formula (I-5), $R^1$ represents Et, $R^2$ does H, $R^3$ does Cl, $R^4$ does $CH_2OEt$, Q does an oxygen atom and the bonded position thereof being a 4-position, (X)n does 3-OMe, Y does CH(Me), and Ar corresponds to each of all the substituents shown in Table 6-1.

Table 6-81

Table 6-81 represents 198 compounds wherein, in the general formula (I-5), $R^1$ represents Et, $R^2$ does H, $R^3$ does Cl, $R^4$ does $CH_2SMe$, Q does an oxygen atom and the bonded position thereof being a 4-position, (X)n does 3-OMe, Y does CH(Me), and Ar corresponds to each of all the substituents shown in Table 6-1.

Table 6-82

Table 6-82 represents 198 compounds wherein, in the general formula (I-5), $R^1$ represents Et, $R^2$ does H, $R^3$ Cl, $R^4$ does $CH_2COOEt$, Q does an oxygen atom and the bonded position thereof being a 4-position, (X)n does 3-OMe, Y does CH(Me), and Ar corresponds to each of all the substituents shown in Table 6-1.

Table 6-83

Table 6-83 represents 198 compounds wherein, in the general formula (I-5), $R^1$ represents Et, $R^2$ does H, $R^3$ Cl, $R^4$ does COOMe, Q does an oxygen atom and the bonded position thereof being a 4-position, (X)n does 3-OMe, Y does CH(Me), and Ar corresponds to each of all the substituents shown in Table 6-1.

Table 6-84

Table 6-84 represents 198 compounds wherein, in the general formula (I-5), $R^1$ represents Et, $R^2$ does H, $R^3$ does Cl, $R^4$ does COOEt, Q does an oxygen atom and the bonded position thereof being a 4-position, (X)n does 3-OMe, Y does CH(Me), and Ar corresponds to each of all the substituents shown in Table 6-1.

Table 6-85

Table 6-85 represents 198 compounds wherein, in the general formula (I-5), $R^1$ represents Et, $R^2$ does H, $R^3$ does Cl, $R^4$ does COO-n-Pr, Q does an oxygen atom and the bonded position thereof being a 4-position, (X)n does 3-OMe, Y does CH(Me), and Ar corresponds to each of all the substituents shown in Table 6-1.

Table 6-86

Table 6-86 represents 198 compounds wherein, in the general formula (I-5), $R^1$ represents Et, $R^2$ does H, $R^3$ does Cl, $R^4$ does COO-i-Pr, Q does an oxygen atom and the bonded position thereof being a 4-position, (X)n does 3-OMe, Y does CH(Me), and Ar corresponds to each of all the substituents shown in Table 6-1.

Table 6-87

Table 6-87 represents 198 compounds wherein, in the general formula (I-5), $R^1$ represents Et, $R^2$ does H, $R^3$ does Cl, $R^4$ does COO-t-Bu, Q does an oxygen atom and the bonded position thereof being a 4-position, (X)n does 3-OMe, Y does CH(Me), and Ar corresponds to each of all the substituents shown in Table 6-1.

Table 6-88

Table 6-88 represents 198 compounds wherein, in the general formula (I-5), $R^1$ represents Et, $R^2$ does H, $R^3$ does Cl, $R^4$ does COOPh, Q does an oxygen atom and the bonded position thereof being a 4-position, (X)n does 3-OMe, Y does CH(Me), and Ar corresponds to each of all the substituents shown in Table 6-1.

Table 6-89

Table 6-89 represents 198 compounds wherein, in the general formula (I-5), $R^1$ represents Et, $R^2$ does H, $R^3$ does Cl, $R^4$ does $COOCH_2Ph$, Q does an oxygen atom and the bonded position thereof being a 4-position, (X)n does 3-OMe, Y does CH(Me), and Ar corresponds to each of all the substituents shown in Table 6-1.

Table 6-90

Table 6-90 represents 198 compounds wherein, in the general formula (I-5), $R^1$ represents Et, $R^2$ does H, $R^3$ Cl, $R^4$ does CONHMe, Q does an oxygen atom and the bonded position thereof being a 4-position, (X)n does 3-OMe,

Y does CH(Me), and Ar corresponds to each of all the substituents shown in Table 6-1.

Table 6-91

Table 6-91 represents 198 compounds wherein, in the general formula (I-5), $R^1$ represents Et, $R^2$ does H, $R^3$ does Cl, $R^4$ does $CONMe_2$, Q does an oxygen atom and the bonded position thereof being a 4-position, (X)n does 3-OMe, Y does CH(Me), and Ar corresponds to each of all the substituents shown in Table 6-1.

Table 6-92

Table 6-92 represents 198 compounds wherein, in the general formula (I-5), $R^1$ represents Et, $R^2$ does H, $R^3$ does Cl, $R^4$ does Ac, Q does an oxygen atom and the bonded position thereof being a 4-position, (X)n does 3-OMe, Y does CH(Me), and Ar corresponds to each of all the substituents shown in Table 6-1.

Table 6-93

Table 6-93 represents 198 compounds wherein, in the general formula (I-5), $R^1$ represents Et, $R^2$ does H, $R^3$ does Cl, $R^4$ does COEt, Q does an oxygen atom and the bonded position thereof being a 4-position, (X)n does 3-OMe, Y does CH(Me), and Ar corresponds to each of all the substituents shown in Table 6-1.

Table 6-94

Table 6-94 represents 198 compounds wherein, in the general formula (I-5), $R^1$ represents Et, $R^2$ does H, $R^3$ does Cl, $R^4$ does CO-n-Pr, Q does an oxygen atom and the bonded position thereof being a 4-position, (X)n does 3-OMe, Y does CH(Me), and Ar corresponds to each of all the substituents shown in Table 6-1.

Table 6-95

Table 6-95 represents 198 compounds wherein, in the general formula (I-5), $R^1$ represents Et, $R^2$ does H, $R^3$ does Cl, $R^4$ does CO-i-Pr, Q does an oxygen atom and the bonded position thereof being a 4-position, (X)n does 3-OMe, Y does CH(Me), and Ar corresponds to each of all the substituents shown in Table 6-1.

Table 6-96

Table 6-96 represents 198 compounds wherein, in the general formula (I-5), $R^1$ represents Et, $R^2$ does H, $R^3$ does Cl, $R^4$ does CO-t-Bu, Q does an oxygen atom and the bonded position thereof being a 4-position, (X)n does 3-OMe, Y does CH(Me), and Ar corresponds to each of all the substituents shown in Table 6-1.

Table 6-97

Table 6-97 represents 198 compounds wherein, in the general formula (I-5), $R^1$ represents Et, $R^2$ does H, $R^3$ does Cl, $R^4$ does $COCF_3$, Q does an oxygen atom and the bonded position thereof being a 4-position, (X)n does 3-OMe, Y does CH(Me), and Ar corresponds to each of all the substituents shown in Table 6-1.

Table 6-98

Table 6-98 represents 198 compounds wherein, in the general formula (I-5), $R^1$ represents Et, $R^2$ does H, $R^3$ does Cl, $R^4$ does COPh, Q does an oxygen atom and the bonded position thereof being a 4-position, (X)n does 3-OMe, Y does CH(Me), and Ar corresponds to each of all the substituents shown in Table 6-1.

Table 6-99

Table 6-99 represents 198 compounds wherein, in the general formula (I-5), $R^1$ represents Et, $R^2$ does H, $R^3$ Cl, $R^4$ does $COCH_2Ph$, Q does an oxygen atom and the bonded position thereof being a 4-position, (X)n does 3-OMe, Y does CH(Me), and Ar corresponds to each of all the substituents shown in Table 6-1.

Table 6-100

Table 6-100 represents 198 compounds wherein, in the general formula (I-5), $R^1$ represents Et, $R^2$ does H, $R^3$ does Cl, $R^4$ does CO-c-Pr, Q does an oxygen atom and the bonded position thereof being a 4-position, (X)n does 3-OMe, Y does CH(Me), and Ar corresponds to each of all the substituents shown in Table 6-1.

Table 6-101

Table 6-101 represents 198 compounds wherein, in the general formula (I-5), $R^1$ represents Et, $R^2$ does H, $R^3$ does Cl, $R^4$ does COCH=CHMe, Q does an oxygen atom and the bonded position thereof being a 4-position, (X) n does 3-OMe, Y does CH(Me), and Ar corresponds to each of all the substituents shown in Table 6-1.

Table 6-102

Table 6-102 represents 198 compounds wherein, in the general formula (I-5), $R^1$ represents Et, $R^2$ does H, $R^3$ Cl, $R^4$ does SPh, Q does an oxygen atom and the bonded position thereof being a 4-position, (X)n does 3-OMe, Y does CH(Me), and Ar corresponds to each of all the substituents shown in Table 6-1.

Table 6-103

Table 6-103 represents 198 compounds wherein, in the general formula (I-5), $R^1$ represents Et, $R^2$ does H, $R^3$ does Cl, $R^4$ does $CH_2$-c-Pr, Q does an oxygen atom and the bonded position thereof being a 4-position, (X)n does 3-OMe, Y does CH(Me), and Ar corresponds to each of all the substituents shown in Table 6-1.

Table 6-104

Table 6-104 represents 198 compounds wherein, in the general formula (I-5), $R^1$ represents Et, $R^2$ does H, $R^3$ does Cl, $R^4$ does H, Q does a sulfur atom and the bonded position thereof being a 4-position, (X)n does 3-OMe, Y does CH(Me), and Ar corresponds to each of all the substituents shown in Table 6-1.

Table 6-105

Table 6-105 represents 198 compounds wherein, in the general formula (I-5), $R^1$ represents Et, $R^2$ does H, $R^3$ does Cl, $R^4$ does H, Q does NH and the bonded position thereof being a 4-position, (X)n does 3-OMe, Y does CH (Me), and Ar corresponds to each of all the substituents shown in Table 6-1.

Table 6-106

Table 6-106 represents 198 compounds wherein, in the general formula (I-5), $R^1$ represents Et, $R^2$ does H, $R^3$ does Cl, $R^4$ does H, Q does NMe and the bonded position thereof being a 4-position, (X)n does 3-OMe, Y does CH(Me), and Ar corresponds to each of all the substituents shown in Table 6-1.

Table 6-107

Table 6-107 represents 198 compounds wherein, in the general formula (I-5), $R^1$ represents Et, $R^2$ does H, $R^3$ does Cl, $R^4$ does H, Q does NAc and the bonded position thereof being a 4-position, (X)n does 3-OMe, Y does CH (Me), and Ar corresponds to each of all the substituents shown in Table 6-1.

Table 6-108

Table 6-108 represents 198 compounds wherein, in the general formula (I-5), $R^1$ represents Et, $R^2$ does H, $R^3$ does Cl, $R^4$ does H, Q does CH=CH and the bonded position thereof being a 4-position, (X)n does 3-OMe, Y does CH(Me), and Ar corresponds to each of all the substituents shown in Table 6-1.

Table 6-109

Table 6-109 represents 198 compounds wherein, in the general formula (I-5), $R^1$ represents Et, $R^2$ does H, $R^3$ does Cl, $R^4$ does H, Q does $COCH_2$ and the bonded position thereof being a 4-position, (X)n does 3-OMe, Y does

CH(Me), and Ar corresponds to each of all the substituents shown in Table 6-1.

Table 6-110

Table 6-110 represents 198 compounds wherein, in the general formula (I-5), $R^1$ represents Et, $R^2$ does H, $R^3$ does Cl, $R^4$ does H, Q does CH(OMe)=CH and the bonded position thereof being a 4-position, (X)n does 3-OMe, Y does CH(Me), and Ar corresponds to each of all the substituents shown in Table 6-1.

Table 6-111

Table 6-111 represents 198 compounds wherein, in the general formula (I-5), $R^1$ represents Et, $R^2$ does H, $R^3$ does Cl, $R^4$ does H, Q does C≡C and the bonded position thereof being a 4-position, (X)n does 3-OMe, Y does CH(Me), and Ar corresponds to each of all the substituents shown in Table 6-1.

Table 6-112

Table 6-112 represents 198 compounds wherein, in the general formula (I-5), $R^1$ represents Et, $R^2$ does H, $R^3$ does Cl, $R^4$ does H, Q does $CH_2CH_2$ and the bonded position thereof being a 4-position, (X)n does 3-OMe, Y does CH(Me), and Ar corresponds to each of all the substituents shown in Table 6-1.

Table 6-113

Table 6-113 represents 198 compounds wherein, in the general formula (I-5), $R^1$ represents Et, $R^2$ does H, $R^3$ does Cl, $R^4$ does H, Q does $CH(OH)CH_2$ and the bonded position thereof being a 4-position, (X)n does 3-OMe, Y does CH(Me), and Ar corresponds to each of all the substituents shown in Table 6-1.

Table 6-114

Table 6-114 represents 198 compounds wherein, in the general formula (I-5), $R^1$ represents Et, $R^2$ does H, $R^3$ does Cl, $R^4$ does H, Q does $CH(OMe)CH_2$ and the bonded position thereof being a 4-position, (X)n does 3-OMe, Y does CH(Me), and Ar corresponds to each of all the substituents shown in Table 6-1.

Table 6-115

Table 6-115 represents 198 compounds wherein, in the general formula (I-5), $R^1$ represents Et, $R^2$ does H, $R^3$ does Cl, $R^4$ does H, Q does $OCH_2$ and the bonded position thereof being a 4-position, (X)n does 3-OMe, Y does CH(Me), and Ar corresponds to each of all the substituents shown in Table 6-1.

Table 6-116

Table 6-116 represents 198 compounds wherein, in the general formula (I-5), $R^1$ represents Et, $R^2$ does H, $R^3$ does Cl, $R^4$ does H, Q does OCHMe and the bonded position thereof being a 4-position, (X)n does 3-OMe, Y does CH(Me), and Ar corresponds to each of all the substituents shown in Table 6-1.

Table 6-117

Table 6-117 represents 198 compounds wherein, in the general formula (I-5), $R^1$ represents Et, $R^2$ does H, $R^3$ does Cl, $R^4$ does H, Q does $OCH_2CH_2$ and the bonded position thereof being a 4-position, (X)n does 3-OMe, Y does CH(Me), and Ar corresponds to each of all the substituents shown in Table 6-1.

Table 6-118

Table 6-118 represents 198 compounds wherein, in the general formula (I-5), $R^1$ represents Et, $R^2$ does H, $R^3$ does Cl, $R^4$ does H, Q does $OCH_2CH{=}CH$ and the bonded position thereof being a 4-position, (X)n does 3-OMe, Y does CH(Me), and Ar corresponds to each of all the substituents shown in Table 6-1.

Table 6-119

Table 6-119 represents 198 compounds wherein, in the general formula (I-5), $R^1$ represents Et, $R^2$ does H, $R^3$ does Cl, $R^4$ does H, Q does $SCH_2$ and the bonded position thereof being a 4-position, (X)n does 3-OMe, Y does CH(Me), and Ar corresponds to each of all the substituents shown in Table 6-1.

Table 6-120

Table 6-120 represents 198 compounds wherein, in the general formula (I-5), $R^1$ represents Et, $R^2$ does H, $R^3$ does Cl, $R^4$ does H, Q does $SCH_2CH_2$ and the bonded position thereof being a 4-position, (X)n does 3-OMe, Y does CH(Me), and Ar corresponds to each of all the substituents shown in Table 6-1.

Table 6-121

Table 6-121 represents 198 compounds wherein, in the general formula (I-5), $R^1$ represents Et, $R^2$ does H, $R^3$ does Cl, $R^4$ does H, Q does $NHCH_2$ and the bonded position thereof being a 4-position, (X)n does 3-OMe, Y does CH(Me), and Ar corresponds to each of all the substituents shown in Table 6-1.

Table 6-122

Table 6-122 represents 198 compounds wherein, in the general formula (I-5), $R^1$ represents Et, $R^2$ does H, $R^3$ does Cl, $R^4$ does H, Q does $NHCH_2CH_2$ and the bonded position thereof being a 4-position, (X)n does 3-OMe, Y does CH(Me), and Ar corresponds to each of all the substituents shown in Table 6-1.

Table 6-123

Table 6-123 represents 198 compounds wherein, in the general formula (I-5), $R^1$ represents Et, $R^2$ does H, $R^3$ does Cl, $R^4$ does H, Q does $N(Me)CH_2$ and the bonded position thereof being a 4-position, (X)n does 3-OMe, Y does CH(Me), and Ar corresponds to each of all the substituents shown in Table 6-1.

Table 6-124

Table 6-124 represents 198 compounds wherein, in the general formula (I-5), $R^1$ represents Et, $R^2$ does H, $R^3$ does Cl, $R^4$ does H, Q does $N(Me)CH_2CH_2$ and the bonded position thereof being a 4-position, (X)n does 3-OMe, Y does CH(Me), and Ar corresponds to each of all the substituents shown in Table 6-1.

Table 6-125

Table 6-125 represents 198 compounds wherein, in the general formula (I-5), $R^1$ represents Et, $R^2$ does H, $R^3$ does Cl, $R^4$ does H, Q does NHCO and the bonded position thereof being a 4-position, (X)n does 3-OMe, Y does CH(Me), and Ar corresponds to each of all the substituents shown in Table 6-1.

Table 6-126

Table 6-126 represents 198 compounds wherein, in the general formula (I-5), $R^1$ represents Et, $R^2$ does H, $R^3$ does Cl, $R^4$ does H, Q does N(Me)CO and the bonded position thereof being a 4-position, (X)n does 3-OMe, Y does CH(Me), and Ar corresponds to each of all the substituents shown in Table 6-1.

Table 6-127

Table 6-127 represents 198 compounds wherein, in the general formula (I-5), $R^1$ represents Et, $R^2$ does H, $R^3$ does Cl, $R^4$ does H, Q does NHCOCH=CH and the bonded position thereof being a 4-position, (X)n does 3-OMe, Y does CH(Me), and Ar corresponds to each of all the substituents shown in Table 6-1.

Table 6-128

Table 6-128 represents 198 compounds wherein, in the general formula (I-5), $R^1$ represents Et, $R^2$ does H, $R^3$ does Cl, $R^4$ does H, Q does C=O and the bonded position thereof being a 4-position, (X)n does 3-OMe, Y does CH(Me), and Ar corresponds to each of all the substituents shown in Table 6-1.

Table 6-129

Table 6-129 represents 198 compounds wherein, in the general formula (I-5), $R^1$ represents Et, $R^2$ does H, $R^3$ does Cl, $R^4$ does H, Q does C=NOMe and the bonded position thereof being a 4-position, (X)n does 3-OMe, Y does CH(Me), and Ar corresponds to each of all the substituents shown in Table 6-1.

Table 6-130

Table 6-130 represents 198 compounds wherein, in the general formula (I-5), $R^1$ represents Et, $R^2$ does H, $R^3$ does Cl, $R^4$ does H, Q does an oxygen atom and the bonded position thereof being a 3-position, (X)n does H, Y does CH(Me), and Ar corresponds to each of all the substituents shown in Table 6-1.

Table 6-131

Table 6-131 represents 198 compounds wherein, in the general formula (I-5), $R^1$ represents Et, $R^2$ does H, $R^3$ does Cl, $R^4$ does H, Q does an oxygen atom and the bonded position thereof being a 2-position, (X)n does H, Y does CH(Me), and Ar corresponds to each of all the substituents shown in Table 6-1.

Table 6-132

Table 6-132 represents 198 compounds wherein, in the general formula (I-5), $R^1$ represents Et, $R^2$ does H, $R^3$ does Cl, $R^4$ does H, Q does an oxygen atom and the bonded position thereof being a 4-position, (X)n does 3-Cl, Y does CH(Me), and Ar corresponds to each of all the substituents shown in Table 6-1.

Table 6-133

Table 6-133 represents 198 compounds wherein, in the general formula (I-5), $R^1$ represents Et, $R^2$ does H, $R^3$ does Cl, $R^4$ does H, Q does an oxygen atom and the bonded position thereof being a 4-position, (X)n does 2-Cl, Y does CH(Me), and Ar corresponds to each of all the substituents shown in Table 6-1.

Table 6-134

Table 6-134 represents 198 compounds wherein, in the general formula (I-5), $R^1$ represents Et, $R^2$ does H, $R^3$ does Cl, $R^4$ does H, Q does an oxygen atom and the bonded position thereof being a 4-position, (X)n does 3-Me, Y does CH(Me), and Ar corresponds to each of all the substituents shown in Table 6-1.

Table 6-135

Table 6-135 represents 198 compounds wherein, in the general formula (I-5), $R^1$ represents Et, $R^2$ does H, $R^3$ does Cl, $R^4$ does H, Q does an oxygen atom and the bonded position thereof being a 4-position, (X)n does 2-Me, Y does CH(Me), and Ar corresponds to each of all the substituents shown in Table 6-1.

Table 6-136

Table 6-136 represents 198 compounds wherein, in the general formula (I-5), $R^1$ represents Et, $R^2$ does H, $R^3$ does Cl, $R^4$ does H, Q does an oxygen atom and the bonded position thereof being a 4-position, (X)n does 2-OMe, Y does CH(Me), and Ar corresponds to each of all the substituents shown in Table 6-1.

Table 6-137

Table 6-137 represents 198 compounds wherein, in the general formula (I-5), $R^1$ represents Et, $R^2$ does H, $R^3$ does Cl, $R^4$ does H, Q does an oxygen atom and the bonded position thereof being a 4-position, (X)n does 3-$CF_3$, Y does CH(Me), and Ar corresponds to each of all the substituents shown in Table 6-1.

Table 6-138

Table 6-138 represents 198 compounds wherein, in the general formula (I-5), $R^1$ represents Et, $R^2$ does H, $R^3$ does Cl, $R^4$ does H, Q does an oxygen atom and the bonded position thereof being a 4-position, (X)n does 2-$CF_3$, Y does CH(Me), and Ar corresponds to each of all the substituents shown in Table 6-1.

Table 6-139

Table 6-139 represents 198 compounds wherein, in the general formula (I-5), $R^1$ represents Et, $R^2$ does H, $R^3$ does Cl, $R^4$ does H, Q does an oxygen atom and the bonded position thereof being a 4-position, (X)n does 3-OH, Y does CH(Me), and Ar corresponds to each of all the substituents shown in Table 6-1.

Table 6-140

Table 6-140 represents 198 compounds wherein, in the general formula (I-5), $R^1$ represents Et, $R^2$ does H, $R^3$ does Cl, $R^4$ does H, Q does an oxygen atom and the bonded position thereof being a 4-position, (X)n does 3-$OCHF_2$, Y does CH(Me), and Ar corresponds to each of all the substituents shown in Table 6-1.

Table 6-141

Table 6-141 represents 198 compounds wherein, in the general formula (I-5), $R^1$ represents Et, $R^2$ does H, $R^3$ does Cl, $R^4$ does H, Q does an oxygen atom and the bonded position thereof being a 4-position, (X)n does 3-$OCF_3$, Y does CH(Me), and Ar corresponds to each of all the substituents shown in Table 6-1.

Table 6-142

Table 6-142 represents 198 compounds wherein, in the general formula (I-5), $R^1$ represents Et, $R^2$ does H, $R^3$ does Cl, $R^4$ does H, Q does an oxygen atom and the bonded position thereof being a 4-position, (X)n does 3-$OCF_2CHF_2$, Y does CH(Me), and Ar corresponds to each of all the substituents shown in Table 6-1.

Table 6-143

Table 6-143 represents 198 compounds wherein, in the general formula (I-5), $R^1$ represents Et, $R^2$ does H, $R^3$ does Cl, $R^4$ does H, Q does an oxygen atom and the bonded position thereof being a 4-position, (X)n does 3-$OCH_2CF_3$, Y does CH(Me), and Ar corresponds to each of all the substituents shown in Table 6-1.

Table 6-144

Table 6-144 represents 198 compounds wherein, in the general formula (I-5), $R^1$ represents Et, $R^2$ does H, $R^3$ does Cl, $R^4$ does H, Q does an oxygen atom and the bonded position thereof being a 4-position, (X)n does 3-$OCH_2CF_3$, Y does CH(Me), and Ar corresponds to each of all the substituents shown in Table 6-1.

Table 6-145

Table 6-145 represents 198 compounds wherein, in the general formula (I-5), $R^1$ represents Et, $R^2$ does H, $R^3$ does Cl, $R^4$ does H, Q does an oxygen atom and the bonded position thereof being a 4-position, (X)n does 3-NHMe, Y does CH(Me), and Ar corresponds to each of all the substituents shown in Table 6-1.

Table 6-146

Table 6-146 represents 198 compounds wherein, in the general formula (I-5), $R^1$ represents Et, $R^2$ does H, $R^3$ does Cl, $R^4$ does H, Q does an oxygen atom and the bonded position thereof being a 4-position, (X)n does 3-$NMe_2$, Y does CH(Me), and Ar corresponds to each of all the substituents shown in Table 6-1.

Table 6-147

Table 6-147 represents 198 compounds wherein, in the general formula (I-5), $R^1$ represents Et, $R^2$ does H, $R^3$ does Cl, $R^4$ does H, Q does an oxygen atom and the bonded position thereof being a 4-position, (X)n does 3-NHAc,

Y does CH(Me), and Ar corresponds to each of all the substituents shown in Table 6-1.

Table 6-148

Table 6-148 represents 198 compounds wherein, in the general formula (I-5), $R^1$ represents Et, $R^2$ does H, $R^3$ does Cl, $R^4$ does H, Q does an oxygen atom and the bonded position thereof being a 4-position, (X)n does 3-NHCOC$F_3$, Y does CH(Me), and Ar corresponds to each of all the substituents shown in Table 6-1.

Table 6-149

Table 6-149 represents 198 compounds wherein, in the general formula (I-S), $R^1$ represents Et, $R^2$ does H, $R^3$ does Cl, $R^4$ does H, Q does an oxygen atom and the bonded position thereof being a 4-position, (X)n does 3-NHCOOMe, Y does CH(Me), and Ar corresponds to each of all the substituents shown in Table 6-1.

Table 6-150

Table 6-150 represents 198 compounds wherein, in the general formula (I-5), $R^1$ represents Et, $R^2$ does H, $R^3$ does Cl, $R^4$ does H, Q does an oxygen atom and the bonded position thereof being a 4-position, (X)n does 3-NHC$H_2$COOMe, Y does CH(Me), and Ar corresponds to each of all the substituents shown in Table 6-1.

Table 6-151

Table 6-151 represents 198 compounds wherein, in the general formula (I-5), $R^1$ represents Et, $R^2$ does H, $R^3$ does Cl, $R^4$ does H, Q does an oxygen atom and the bonded position thereof being a 4-position, (X)n does 3-NHC$H_2$CN, Y does CH(Me), and Ar corresponds to each of all the substituents shown in Table 6-1.

Table 6-152

Table 6-152 represents 198 compounds wherein, in the general formula (I-5), $R^1$ represents Et, $R^2$ does H, $R^3$ does Cl, $R^4$ does H, Q does an oxygen atom and the bonded position thereof being a 4-position, (X)n does 3-NHCONHMe, Y does CH(Me), and Ar corresponds to each of all the substituents shown in Table 6-1.

Table 6-153

Table 6-153 represents 198 compounds wherein, in the general formula (I-5), $R^1$ represents Et, $R^2$ H, $R^3$ Cl, $R^4$ H, Q an oxygen atom, the bonding position thereof a 4-position, (X)n 3-NHS$O_2$Me, Y CH(Me), and Ar corresponds to each of all the substituents shown in Table 6-1.

Table 6-154

Table 6-154 represents 198 compounds wherein, in the general formula (1-5), $R^1$ represents Et, $R^2$ does H, $R^3$ does Cl, $R^4$ does H, Q does an oxygen atom and the bonded position thereof being a 4-position, (X)n does 3-NHS$O_2$C$F_3$, Y does CH(Me), and Ar corresponds to each of all the substituents shown in Table 6-1.

Table 6-155

Table 6-155 represents 198 compounds wherein, in the general formula (I-5), $R^1$ represents Et, $R^2$ does H, $R^3$ does Cl, $R^4$ does H, Q does an oxygen atom and the bonded position thereof being a 4-position, (X)n does 3-NHCH$F_2$, Y does CH(Me), and Ar corresponds to each of all the substituents shown in Table 6-1.

Table 6-156

Table 6-156 represents 198 compounds wherein, in the general formula (I-5), $R^1$ represents Et, $R^2$ does H, $R^3$ does Cl, $R^4$ does H, Q does an oxygen atom and the bonded position thereof being a 4-position, (X)n does 3-N$(CHF_2)_2$, Y does CH(Me), and Ar corresponds to each of all the substituents shown in Table 6-1.

Table 6-157

Table 6-157 represents 198 compounds wherein, in the general formula (I-5), $R^1$ represents Et, $R^2$ does H, $R^3$ does Cl, $R^4$ does H, Q does an oxygen atom and the bonded position thereof being a 4-position, (X)n does 3-$NHCF_2CHF_2$, Y does CH(Me), and Ar corresponds to each of all the substituents shown in Table 6-1.

Table 6-158

Table 6-158 represents 198 compounds wherein, in the general formula (I-5), $R^1$ represents Et, $R^2$ does H, $R^3$ does Cl, $R^4$ does H, Q does an oxygen atom and the bonded position thereof being a 4-position, (X)n does 3-$NHCH_2CF_3$, Y does CH(Me), and Ar corresponds to each of all the substituents shown in Table 6-1.

Table 6-159

Table 6-159 represents 198 compounds wherein, in the general formula (I-S), $R^1$ represents Et, $R^2$ does H, $R^3$ does Cl, $R^4$ does H, Q does an oxygen atom and the bonded position thereof being a 4-position, (X)n does 3-SH, Y does CH(Me), and Ar corresponds to each of all the substituents shown in Table 6-1.

Table 6-160

Table 6-160 represents 198 compounds wherein, in the general formula (I-5), $R^1$ represents Et, $R^2$ does H, $R^3$ does Cl, $R^4$ does H, Q does an oxygen atom and the bonded position thereof being a 4-position, (X)n does 3-SMe, Y does CH(Me), and Ar corresponds to each of all the substituents shown in Table 6-1.

Table 6-161

Table 6-161 represents 198 compounds wherein, in the general formula (I-5), $R^1$ represents Et, $R^2$ does H, $R^3$ does Cl, $R^4$ does H, Q does an oxygen atom and the bonded position thereof being a 4-position, (X)n does 3-SOMe, Y does CH(Me), and Ar corresponds to each of all the substituents shown in Table 6-1.

Table 6-162

Table 6-162 represents 198 compounds wherein, in the general formula (I-5), $R^1$ represents Et, $R^2$ does H, $R^3$ does Cl, $R^4$ does H, Q does an oxygen atom and the bonded position thereof being a 4-position, (X)n does 3-$SO_2Me$, Y does CH(Me), and Ar corresponds to each of all the substituents shown in Table 6-1.

Table 6-163

Table 6-163 represents 198 compounds wherein, in the general formula (I-5), $R^1$ represents Et, $R^2$ does H, $R^3$ does Cl, $R^4$ does H, Q does an oxygen atom and the bonded position thereof being a 4-position, (X)n does 3-$SCHF_2$, Y does CH(Me), and Ar corresponds to each of all the substituents shown in Table 6-1.

Table 6-164

Table 6-164 represents 198 compounds wherein, in the general formula (I-5), $R^1$ represents Et, $R^2$ does H, $R^3$ does Cl, $R^4$ does H, Q does an oxygen atom and the bonded position thereof being a 4-position, (X)n does 3-$SOCHF_2$, Y does CH(Me), and Ar corresponds to each of all the substituents shown in Table 6-1.

Table 6-165

Table 6-165 represents 198 compounds wherein, in the general formula (I-5), $R^1$ represents Et, $R^2$ does H, $R^3$ does Cl, $R^4$ does H, Q does an oxygen atom and the bonded position thereof being a 4-position, (X)n does 3-$SO_2CHF_2$, Y does CH(Me), and Ar corresponds to each of all the substituents shown in Table 6-1.

Table 6-166

Table 6-166 represents 198 compounds wherein, in the general formula (I-5), $R^1$ represents Et, $R^2$ does H, $R^3$ does Cl, $R^4$ does H, Q does an oxygen atom and the bonded position thereof being a 4-position, (X)n does 3-$SCF_3$,

Y does CH(Me), and Ar corresponds to each of all the substituents shown in Table 6-1.

Table 6-167

Table 6-167 represents 198 compounds wherein, in the general formula (I-5), $R^1$ represents Et, $R^2$ does H, $R^3$ does Cl, $R^4$ does H, Q does an oxygen atom and the bonded position thereof being a 4-position, (X)n does 3-$SOCF_3$, Y does CH(Me), and Ar corresponds to each of all the substituents shown in Table 6-1.

Table 6-168

Table 6-168 represents 198 compounds wherein, in the general formula (I-5), $R^1$ represents Et, $R^2$ does H, $R^3$ does Cl, $R^4$ does H, Q does an oxygen atom and the bonded position thereof being a 4-position, (X)n does 3-$SO_2CF_3$, Y does CH(Me), and Ar corresponds to each of all the substituents shown in Table 6-1.

Table 6-169

Table 6-169 represents 198 compounds wherein, in the general formula (I-5), $R^1$ represents Et, $R^2$ does H, $R^3$ does Cl, $R^4$ does H, Q does an oxygen atom and the bonded position thereof being a 4-position, (X)n does 3-$SCF_2CHF_2$, Y does CH(Me), and Ar corresponds to each of all the substituents shown in Table 6-1.

Table 6-170

Table 6-170 represents 198 compounds wherein, in the general formula (I-5), $R^1$ represents Et, $R^2$ does H, $R^3$ does Cl, $R^4$ does H, Q does an oxygen atom and the bonded position thereof being a 4-position, (X)n does 3-$SOCF_2CHF_2$, Y does CH(Me), and Ar corresponds to each of all the substituents shown in Table 6-1.

Table 6-171

Table 6-171 represents 198 compounds wherein, in the general formula (I-5), $R^1$ represents Et, $R^2$ does H, $R^3$ does Cl, $R^4$ does H, Q does an oxygen atom and the bonded position thereof being a 4-position, (X)n does 3-$SO_2CF_2CHF_2$, Y does CH(Me), and Ar corresponds to each of all the substituents shown in Table 6-1.

Table 6-172

Table 6-172 represents 198 compounds wherein, in the general formula (I-5), $R^1$ represents Et, $R^2$ does H, $R^3$ does Cl, $R^4$ does H, Q does an oxygen atom and the bonded position thereof being a 4-position, (X)n does 3-$SCH_2CF_3$, Y does CH(Me), and Ar corresponds to each of all the substituents shown in Table 6-1.

Table 6-173

Table 6-173 represents 198 compounds wherein, in the general formula (I-5), $R^1$ represents Et, $R^2$ does H, $R^3$ does Cl, $R^4$ does H, Q does an oxygen atom and the bonded position thereof being a 4-position, (X)n does 3-$SOCH_2CF_3$, Y does CH(Me), and Ar corresponds to each of all the substituents shown in Table 6-1.

Table 6-174

Table 6-174 represents 198 compounds wherein, in the general formula (I-5), $R^1$ represents Et, $R^2$ does H, $R^3$ does Cl, $R^4$ does H, Q does an oxygen atom and the bonded position thereof being a 4-position, (X)n does 3-$SO_2CH_2CF_3$, Y does CH(Me), and Ar corresponds to each of all the substituents shown in Table 6-1.

Table 6-175

Table 6-175 represents 198 compounds wherein, in the general formula (I-5), $R^1$ represents Et, $R^2$ does H, $R^3$ does Cl, $R^4$ does H, Q does an oxygen atom and the bonded position thereof being a 4-position, (X)n does 3,5-$Cl_2$, Y does CH(Me), and Ar corresponds to each of all the substituents shown in Table 6-1.

Table 6-176

Table 6-176 represents 198 compounds wherein, in the general formula (I-5), $R^1$ represents Et, $R^2$ does H, $R^3$ does Cl, $R^4$ does H, Q does an oxygen atom and the bonded position thereof being a 4-position, (X)n does 2,3-$Cl_2$, Y does CH(Me), and Ar corresponds to each of all the substituents shown in Table 6-1.

Table 6-177

Table 6-177 represents 198 compounds wherein, in the general formula (I-5), $R^1$ represents Et, $R^2$ does H, $R^3$ does Cl, $R^4$ does H, Q does an oxygen atom and the bonded position thereof being a 4-position, (X)n does 3,5-$(OMe)_2$, Y does CH(Me), and Ar corresponds to each of all the substituents shown in Table 6-1.

Table 6-178

Table 6-178 represents 198 compounds wherein, in the general formula (I-5), $R^1$ represents Et, $R^2$ does H, $R^3$ does Cl, $R^4$ does H, Q does an oxygen atom and the bonded position thereof being a 4-position, (X)n does 2,3-$(OMe)_2$, Y does CH(Me), and Ar corresponds to each of all the substituents shown in Table 6-1.

Table 6-179

Table 6-179 represents 198 compounds wherein, in the general formula (I-5), $R^1$ represents Et, $R^2$ does H, $R^3$ does Cl, $R^4$ does H, Q does an oxygen atom and the bonded position thereof being a 4-position, (X)n does 2,5-$(OMe)_2$, Y does CH(Me), and Ar corresponds to each of all the substituents shown in Table 6-1.

Table 6-180

Table 6-180 represents 198 compounds wherein, in the general formula (I-5), $R^1$ represents Et, $R^2$ does H, $R^3$ does Cl, $R^4$ does H, Q does an oxygen atom and the bonded position thereof being a 4-position, (X)n does 3-OMe, Y does CH(Me), and Ar corresponds to each of all the substituents shown in Table 6-1.

Table 6-181

Table 6-181 represents 198 compounds wherein, in the general formula (I-5), $R^1$ represents Et, $R^2$ does H, $R^3$ does Cl, $R^4$ does H, Q does an oxygen atom and the bonded position thereof being a 4-position, (X)n does 3-OMe, Y does CH(OMe), and Ar corresponds to each of all the substituents shown in Table 6-1.

Table 6-182

Table 6-182 represents 198 compounds wherein, in the general formula (I-5), $R^1$ represents Et, $R^2$ does H, $R^3$ does Cl, $R^4$ does H, Q does an oxygen atom and the bonded position thereof being a 4-position, (X)n does 3-OMe, Y does CH(Et), and Ar corresponds to each of all the substituents shown in Table 6-1.

Table 6-183

Table 6-183 represents 198 compounds wherein, in the general formula (I-5), $R^1$ represents Et, $R^2$ does H, $R^3$ does Cl, $R^4$ does H, Q does an oxygen atom and the bonded position thereof being a 4-position, (X)n does 3-OMe, Y does $C(CH_2)_2$, and Ar corresponds to each of all the substituents shown in Table 6-1.

Table 6-184

Table 6-184 represents 198 compounds wherein, in the general formula (I-S), $R^1$ represents Et, $R^2$ does H, $R^3$ does Cl, $R^4$ does H, Q does an oxygen atom and the bonded position thereof being a 4-position, (X)n does 3-OMe, Y does $CH_2CH_2$, and Ar corresponds to each of all the substituents shown in Table 6-1.

Table 6-185

Table 6-185 represents 198 compounds wherein, in the general formula (I-5), $R^1$ represents Et, $R^2$ does H, $R^3$ does Cl, $R^4$ does H, Q does an oxygen atom and the bonded position thereof being a 4-position, (X)n does 3-OMe,

Y does CH=CH, and Ar corresponds to each of all the substituents shown in Table 6-1.

Table 6-186

Table 6-186 represents 198 compounds wherein, in the general formula (I-5), $R^1$ represents Et, $R^2$ does H, $R^3$ does Cl, $R^4$ does H, Q does an oxygen atom and the bonded position thereof being a 4-position, (X)n does 3-OMe, Y does $COCH_2$, and Ar corresponds to each of all the substituents shown in Table 6-1.

Table 6-187

Table 6-187 represents 198 compounds wherein, in the general formula (I-5), $R^1$ represents Et, $R^2$ does H, $R^3$ does Cl, $R^4$ does H, Q does an oxygen atom and the bonded position thereof being a 4-position, (X)n does 3-OMe, Y does C(OMe)=CH, and Ar corresponds to each of all the substituents shown in Table 6-1.

Table 6-188

Table 6-188 represents 198 compounds wherein, in the general formula (I-5), $R^1$ represents Et, $R^2$ does H, $R^3$ does Cl, $R^4$ does H, Q does an oxygen atom and the bonded position thereof being a 4-position, (X)n does 3-OMe, Y does C≡C, and Ar corresponds to each of all the substituents shown in Table 6-1.

Table 6-189

Table 6-189 represents 198 compounds wherein, in the general formula (I-5), $R^1$ represents Et, $R^2$ does H, $R^3$ does Cl, $R^4$ does H, Q does an oxygen atom and the bonded position thereof being a 4-position, (X)n does 3-OMe, Y does $CH(OH)CH_2$, and Ar corresponds to each of all the substituents shown in Table 6-1.

Table 6-190

Table 6-190 represents 198 compounds wherein, in the general formula (I-5), $R^1$ represents Et, $R^2$ does H, $R^3$ does Cl, $R^4$ does H, Q does an oxygen atom and the bonded position thereof being a 4-position, (X)n does 3-OMe, Y does $CH(OMe)CH_2$, and Ar corresponds to each of all the substituents shown in Table 6-1.

Table 6-191

Table 6-191 represents 198 compounds wherein, in the general formula (I-5), $R^1$ represents Et, $R^2$ does H, $R^3$ does Cl, $R^4$ does H, Q does an oxygen atom and the bonded position thereof being a 4-position, (X)n does 3-OMe, Y does $OCH_2$, and Ar corresponds to each of all the substituents shown in Table 6-1.

Table 6-192

Table 6-192 represents 198 compounds wherein, in the general formula (I-5), $R^1$ represents Et, $R^2$ does H, $R^3$ does Cl, $R^4$ does H, Q does an oxygen atom and the bonded position thereof being a 4-position, (X)n does 3-OMe, Y does OCH(Me), and Ar corresponds to each of all the substituents shown in Table 6-1.

Table 6-193

Table 6-193 represents 198 compounds wherein, in the general formula (I-5), $R^1$ represents Et, $R^2$ does H, $R^3$ does Cl, $R^4$ does H, Q does an oxygen atom and the bonded position thereof being a 4-position, (X)n does 3-OMe, Y does $OCH_2CH_2$, and Ar corresponds to each of all the substituents shown in Table 6-1.

Table 6-194

Table 6-194 represents 198 compounds wherein, in the general formula (I-5), $R^1$ represents Et, $R^2$ does H, $R^3$ does Cl, $R^4$ does H, Q does an oxygen atom and the bonded position thereof being a 4-position, (X)n does 3-OMe, Y does $OCH_2CH=CH$, and Ar corresponds to each of all the substituents shown in Table 6-1.

Table 6-195

Table 6-195 represents 198 compounds wherein, in the general formula (I-5), $R^1$ represents Et, $R^2$ does H, $R^3$ does Cl, $R^4$ does H, Q does an oxygen atom and the bonded position thereof being a 4-position, (X)n does 3-OMe, Y does $SCH_2$, and Ar corresponds to each of all the substituents shown in Table 6-1.

Table 6-196

Table 6-196 represents 198 compounds wherein, in the general formula (I-5), $R^1$ represents Et, $R^2$ does H, $R^3$ does Cl, $R^4$ does H, Q does an oxygen atom and the bonded position thereof being a 4-position, (X)n does 3-OMe, Y does $SCH_2CH_2$, and Ar corresponds to each of all the substituents shown in Table 6-1.

Table 6-197

Table 6-197 represents 198 compounds wherein, in the general formula (I-5), $R^1$ represents Et, $R^2$ does H, $R^3$ does Cl, $R^4$ does H, Q does an oxygen atom and the bonded position thereof being a 4-position, (X)n does 3-OMe, Y does $NHCH_2$, and Ar corresponds to each of all the substituents shown in Table 6-1.

Table 6-198

Table 6-198 represents 198 compounds wherein, in the general formula (I-5), $R^1$ represents Et, $R^2$ does H, $R^3$ does Cl, $R^4$ does H, Q does an oxygen atom and the bonded position thereof being a 4-position, (X)n does 3-OMe, Y does $NHCH_2CH_2$, and Ar corresponds to each of all the substituents shown in Table 6-1.

Table 6-199

Table 6-199 represents 198 compounds wherein, in the general formula (I-5), $R^1$ represents Et, $R^2$ does H, $R^3$ does Cl, $R^4$ does H, Q does an oxygen atom and the bonded position thereof being a 4-position, (X)n does 3-OMe, Y does $N(Me)CH_2$, and Ar corresponds to each of all the substituents shown in Table 6-1.

Table 6-200

Table 6-200 represents 198 compounds wherein, in the general formula (I-5), $R^1$ represents Et, $R^2$ does H, $R^3$ does Cl, $R^4$ does H, Q does an oxygen atom and the bonded position thereof being a 4-position; (X)n does 3-OMe, Y does $N(Me)CH_2CH_2$, and Ar corresponds to each of all the substituents shown in Table 6-1.

Table 6-201

Table 6-201 represents 198 compounds wherein, in the general formula (I-5), $R^1$ represents Et, $R^2$ does H, $R^3$ does Cl, $R^4$ does H, Q does an oxygen atom and the bonded position thereof being a 4-position, (X)n does 3-OMe, Y does C=O, and Ar corresponds to each of all the substituents shown in Table 6-1.

Table 6-202

Table 6-202 represents 198 compounds wherein, in the general formula (I-5), $R^1$ represents Et, $R^2$ does H, $R^3$ does Cl, $R^4$ does H, Q does an oxygen atom and the bonded position thereof being a 4-position, (X)n does 3-OMe, Y does C=NOMe, and Ar corresponds to each of all the substituents shown in Table 6-1.

Table 6-203

Table 6-203 represents 198 compounds wherein, in the general formula (I-5), $R^1$ represents Et, $R^2$ does H, $R^3$ does Cl, $R^4$ does H, Q does an oxygen atom and the bonded position thereof being a 4-position, (X)n does 3-OMe, Y does $C((CH_2)_2)CH_2$, and Ar corresponds to each of all the substituents shown in Table 6-1.

Table 6-204

Table 6-204 represents 198 compounds wherein, in the general formula (I-5), $R^1$ represents Et, $R^2$ does H, $R^3$ does Cl, $R^4$ does H, Q does an oxygen atom and the bonded position thereof being a 4-position, (X)n does 3-OMe,

Y does $CH(OCH_2OEt)$, and Ar corresponds to each of all the substituents shown in Table 6-1.

Table 6-205

Table 6-205 represents 198 compounds wherein, in the general formula (I-5), $R^1$ represents Et, $R^2$ does H, $R^3$ does Cl, $R^4$ does H, Q does an oxygen atom and the bonded position thereof being a 4-position, (X)n does 3-OMe, Y does $CH(OCH_2CH=CH_2)$, and Ar corresponds to each of all the substituents shown in Table 6-1.

Table 6-206

Table 6-206 represents 198 compounds wherein, in the general formula (I-5), $R^1$ represents Et, $R^2$ does H, $R^3$ does Cl, $R^4$ does H, Q does an oxygen atom and the bonded position thereof being a 4-position, (X)n does 3-OMe, Y does $CH(OCH_2C{\equiv}CH)$, and Ar corresponds to each of all the substituents shown in Table 6-1.

The N-(4-pyrazolyl)amide derivative of the present invention represented by the general formula (I) or a salt thereof is useful for agrohorticultural agent, especially for agrohorticultural fungicides or fungicides. Roughly saying, the composition of the present invention is effective against the diseases due to mold fungi diseases, for example, the diseases due to Deuteromycetes such as Genus Botrytis, Genus Helminthosporium, Genus Fusarium, Genus Septoria, Genus Cercospora, Genus Pyricularia and Genus Alternaria; the diseases due to Basidiomycetes such as Genus Hemileia, Genus Rhizoctonia and Genus Puccinia; the diseases due to Ascomycetes such as Genus Venturia, Genus Podosphaera, Genus Erysiphe, Genus Monilinia and Genus Unsinula; and the diseases due to the other fungi such as Genus Ascochyta, Genus Phoma, Genus Pythium, Genus Corticium and Genus Pyrenophora.

Specific examples of the diseases against which the composition of the present invention exhibit a marked effect include rice blast (Pyricularia oryzae), rice sheath blight (Rhizoctonia solani), rice Helminthosporium leaf spot (Cochliobolus miyabeanus), rice seedling blight (Rhizopus chinensis, Pythium graminicola, Fusarium graminicola, Fusarium roseum, Mucor sp., Phoma sp., Tricoderma sp.), rice bakanae disease (Gibberella fujikuroi), powdery mildew of barley and wheat (Erysiphe graminis), powdery mildew of cucumber (Sphaerotheca fuliginea), powdery mildew of other host plants, eye spot of barley and wheat (Pseudocercosporella herpotrichoides), flag smut of wheat, etc. (Urocystis tritici), snow mold of barley and wheat (Fusarium nivale, Pythium iwayamai, Typhla ishikariensis, Sclerotinia boreasis), oats crown rust (Puccinia coronata), stem rust of other plants, gray mold of cucumber and strawberry (Botrytis cinerea), sclerotinia rot of tomato and cabbage (Sclerotinia sclerotiorum), late blight of potato and tomato (Phytophthora infestans), Phytophthora rot of other plants, downy mildew of various plants such as cucumber downy mildew (Pseudoperonospora cubensis), grape downy mildew (Plasmopara viticola), etc., apple scab (Venturia inaequalis), apple Alternaria leaf spot (Alternaria mali), pear black spot (Alternaria kikuchiana), citrus melanose (Diaporthe citri), citrus scab (Elsinoe fawcetti), sugar beet Cercospora leaf spot (Cercospora beticola), peanut brown leaf spot (Cercospora arachidicola), peanut leaf spot (Cercospora personata), septoria leaf spot of wheat (Septoria tritici), glume blotch of wheat (Septoria nodorum), scald of barley (Rhynchosporium secalis), bunt of wheat (Tilletia caries), lawn grass brown patch (Rhizoctonia solani), lawn grass dollar spot of lawn grass (Sclerotinia homoeocarpe) etc.

The agrohorticultural fungicide, containing the N-(4-pyrazolyl)amide derivative represented by formula (I) or salt thereof of the present invention, has a marked controlling effect on the above-exemplified diseases, which are injurious to paddy field crops, upland crops, fruit trees, vegetables and other crops, flowers and ornament plants, and the like. Therefore, the desired effect of the agrohorticultural fungicide of the present invention can be exhibited by applying the fungicide to the paddy field water, stalks and leaves or soil of paddy field, upland field, fruit trees, vegetables, other crops or flowers and ornament plants at a season at which the diseases are expected to appear, before their appearance or at the time when their appearance is confirmed.

The agrohorticultural agent, especially agrohorticultural insecticides or nematocides, containing the N-(4-pyrazolyl)amide derivative represented by the formula (I) or salt thereof of the present invention as an active ingredient, are suitable for controlling various insect pests such as agrohorticultural insect pests, stored grain insect pests, sanitary insect pests, nematodes, etc., which are injurious to paddy rice, fruit trees, vegetables, other crops, flowers, ornamental plants, etc. They have a marked insecticidal effect, for example, on LEPIDOPTERA including summer fruit tortrix (Adoxophes orana fasciata), smaller tea tortrix (Adoxophyes sp.), Manchurian fruit moth (Grapholita inopinata), oriental fruit moth (Grapholita molesta), soybean pod border (Leguminovora glycinivorella), mulberry leafroller (Olethreutes mori), tea leafroller (Caloptilia thevivora), Caloptilia sp. (Caloptilia zachrysa), apple leafminer (Phyllonorycter ringoniella), pear barkminer (Spulerrina astaurota), common white (Piers rapae crucivora), tobacco budworm (Heliothis sp.), codling moth (Laspey resia pomonella), diamondback moth (Plutella xylostella), apple fruit moth (Argyresthia conjugella), peach fruit moth (Carposina niponensis), rice stem borer (Chilo suppressalis), rice leafroller (Cnaphalocrocis medinalis), tobacco moth (Ephestia elutella), mulberry pyralid (Glyphodes pyloalis), yellow rice borer (Scirpophaga incertulas), rice skipper (Parnara guttata), rice armyworm (Pseudaletia separata), pink borer (Sesamia inferens), common cutworm (Spodoptera litura), beet armyworm (Spodoptera exigua), etc.; HEMIPTERA including aster leafhopper (Macrosteles

fascifrons), green rice leafhopper (Nephotettix cincticepts), brown rice planthopper (Nilaparvata lugens), whitebacked rice planthopper (Sogatella furcifera), citrus psylla (Diaphorina citri), grape whitefly (Aleurolibus taonabae), sweetpotato whitefly (Bemisia tabaci), greenhouse whitefly (Trialeurodes vaporariorum), turnup aphid (Lipaphis erysimi), green peach aphid (Myzus persicae), Indian wax scale (Ceroplastes ceriferus), cottony citrus scale (Pulvinaria aurantii), camphor scale (Pseudaonidia duplex), san Jose scale (Comstockaspis perniciosa), arrowhead scale (Unapsis yanonensis), etc.; TYLENCHIDA including soybean beetle (Anomala rufocuprea), Japanese beetle (Popillia japonica), tobacco beetle (Lasioderma serricorne), powderpost beetle (Lyctus brunneus), twenty-eight-spotted ladybird (Epilachna vigintiotopunctata), azuki bean weevil (Callosobruchus chinensis), vegetable weevil (Listroderes costirostris), maize weevil (Sitophilus zeamais), boll weevil (Anthonomus gradis gradis), rice water weevil (Lissorhoptrus oryzophilus), cucurbit leaf beetle (Aulacophora femoralis), rice leaf beetle (Oulema oryzae), striped flea beetle (Phyllotreta striolata), pine shoot beetle (Tomicus piniperda), Colorado potato beetle (Leptinotarsa decemlineata), Mexican bean beetle (Epilachna varivestis), corn rootworm (Diabrotica sp.), etc.; DIPTERA including (Dacus(Zeugodacus) cucurbitae), oriental fruit fly (Dacus(Bactrocera) dorsalis), rice leafminer (Agnomyza oryzae), onion maggot (Delia antiqua), seedcorn maggot (Delia platura), soybean pod gall midge (Asphondylia sp.), muscid fly (Musca domestica), house mosquito (Culex pipiens pipiens), etc.; and TYLENCHIDA including root-lesion nematode (Pratylenchus sp.), coffee root-lesion nematode (Pratylenchus coffeae), potato cyst nematode (Globodera rostochiensis), root-knot nematode (Meloidogyne sp.), citrus riematode (Tylenchulus semipenetrans), Aphelenchus sp. (Aphelenchus avenae), chrysanthemum foliar (Aphelenchoides ritzemabosi), etc.

The agrohorticultural insecticide, containing the N-(4-pyrazolyl)amide derivative represented by formula (I) or salt thereof of the present invention, has a marked controlling effect on the above-exemplified insect pests, sanitary pests and/or nematodes, which are injurious to paddy field crops, upland crops, fruit trees, vegetables and other crops, flowers and ornament plants, and the like. Therefore, the desired effect of the agrohorticultural insecticide of the present invention can be exhibited by applying the insecticide to the paddy field water, stalks and leaves or soil of paddy field, upland field, fruit trees, vegetables, other crops or flowers and ornament plants at a season at which the insect pests, sanitary pests or nematodes are expected to appear, before their appearance or at the time when their appearance is confirmed.

The useful plants to which the composition for agrohorticultural agent of the present invention can be applied are not particularly limited, and the following plants can be referred to as examples thereof:

cereals such as rice, barley, wheat, rye, oat, corn, kaoliang, etc.; beans and peas such as soybean, red bean, broad bean, pea, peanut, etc.; fruit trees such as apple, citrus trees and fruits, pear, grape, peach, plum, cherry, walnut, almond, banana, strawberry, etc.; vegetables such as cabbage, tomato, spinach, broccoli, lettuce, onion, stone-leek, Spanish paprika, etc.; root crops such as carrot, potato, sweet potato, radish, lotus rhizome, turnip, etc.; processing crops such as cotton, flax, paper mulberry, mitsumata (Edgeworthia papyrifera), coleseed, beet, hop, sugar can, sugar beet, olive, gum, coffee, tobacco, tea, etc.; cucurbitaceous plants such as pumpkin, cucumber, water melon, melon, etc.; pasture plants such as orchard grass, sorghum, timothy, clover, alfalfa, etc.; lawn grasses such as mascarenegrass, bent grass, etc.; and perfumery crops such as lavender, rosemary, thyme, parsley, pepper, ginger, etc.; flowers and ornamental plants such as chrysanthemum, rose, orchid, etc.

In general, the agrohorticultural composition of the present invention, containing the N-(4-pyrazolyl)amide derivative of the formula (I) or salt thereof, is used after being prepared into conveniently usable forms according to ordinary manner for preparation of agrochemicals.

That is, the 4-(pyrazolyl)amide derivative of formula (I) or salt thereof and an appropriate carrier are blended optionally together with an adjuvant in a proper proportion and prepared into a suitable preparation form such as suspension, emulsifiable concentrate, soluble concentrate, wettable powder, granules, dust or tablets through dissolution, separation, suspension, mixing, impregnation, adsorption or sticking.

The inert carrier used in the present invention may be either solid or liquid. As the solid carrier, soybean flour, cereal flour, wood flour, bark flour, saw dust, powdered tobacco stalks, powdered walnut shells, bran, powdered cellulose, extraction residues of vegetables, powdered synthetic polymers or resins, clay (e.g. kaolin, bentonite and acid clay), talc (e.g. talc and pyrophyllite), silica materials (e.g. diatomaceous earth, siliceous sand, mica, white carbon, i. e. synthetic high-dispersion silicic acid, also called finely divided hydrated silica or hydrated silicic acid, some of the commercially available products contain calcium silicate as the major component), activated carbon, powdered sulfur, pumice, calcined diatomaceous earth, ground brick, fly ash, sand, calcium carbonate, calcium phosphate and other inorganic or mineral powders, chemical fertilizers such as ammonium sulfate, ammonium phosphate, ammonium nitrate, urea, ammonium chloride and the like, and compost. These carriers may be used either alone or as a mixture of two or more carriers.

The liquid carrier is that which itself has a solubility or which is without such solubility but is capable of dispersing an active ingredient with the aid of an adjuvant. The following are typical examples of the liquid carrier and can be used alone or as a mixture thereof. Water; alcohols such as methanol, ethanol, isopropanol, butanol and ethylene glycol; ketones such as acetone, methyl ethyl ketone, methyl isobutyl ketone, diisobutyl ketone and cyclohexanone;

ethers such as ethyl ether, dioxane, cellosolve, dipropyl ether and tetrahydrofuran; aliphatic hydrocarbons such as kerosene and mineral oil; aromatic hydrocarbons such as benzene, toluene, xylene, solvent naphtha and alkylnaphthalene; halogenated hydrocarbons such as dichlorethane, chloroform, carbon tetrachloride and chlorobenzene; esters such as ethyl acetate, diisopropyl phthalate, dibutyl phthalate and dioctyl phthalate; amides such as dimethylformamide, diethylformamide and dimethylacetamide; nitriles such as acetonitrile; and dimethyl sulfoxide.

The following are typical examples of the adjuvant, which are used depending upon purposes and used alone or in combination of two or more adjuvants in some cases, or need not to be used at all.

To emulsify, disperse, dissolve and/or wet an active ingredient, a surfactant is used. As the surfactant, there can be exemplified polyoxyethylene alkyl ethers, polyoxyethylene alkylaryl ethers, polyoxyethylene higher fatty acid esters, polyoxyethylene resinates, polyoxyethylene sorbitan monolaurate, polyoxyethylene sorbitan monooleate, alkylarylsulfonates, naphthalene-sulfonic acid condensation products, ligninsulfonates and higher alcohol sulfate esters.

Further, to stabilize the dispersion of an active ingredient, tackify it and/or bind it, there may be used adjuvants such as casein, gelatin, starch, methyl cellulose, carboxymethyl cellulose, gum arabic, polyvinyl alcohols, turpentine, bran oil, bentonite and ligninsulfonates.

To improve the flowability of a solid product, there may be used adjuvants such as waxes, stearates and alkyl phosphates.

Adjuvants such as naphthalenesulfonic acid condensation products and polycondensates of phosphates may be used as a peptizer for dispersible products.

Adjuvants such as silicone oil may also be used as a defoaming agent.

The content of the active ingredient may be varied according to the need, thus, it can be properly selected from the range between 0.01 and 90% by weight in terms of 100% by weight of the agrohorticultural insecticide of the present invention. For example, in dusts or granules, the suitable content thereof is from 0.01 to 50% by weight. In emulsifiable concentrate and flowable wettable powder, too, the suitable content is from 0.01 to 50% by weight.

The agrohorticultural composition of the present invention is used to control a variety of insect pests in the following manner. That is, it is applied to a crop on which the diseases and insect pests are expected to appear or a site where appearance of the diseases and insect pests is undesirable, as it is or after being properly diluted with or suspended in water or the like, in an amount effective for control of the diseases and insect pests.

The applying dosage of the agrohorticultural composition of the present invention, containing N-(4-pyrazolyl) amide derivative of the formula (I) or salt thereof, is varied depending upon various factors such as a purpose, diseases and insect pests to be controlled, a growth state of a plant, tendency of diseases and insect pests appearance, weather, environmental conditions, a preparation form, an application method, an application site and an application time. It may be properly chosen in a range of 0.1 g to 1 kg (in terms of active ingredient compound) per 10 ares depending upon purposes.

The agrohorticultural composition of the present invention, containing N-(4-pyrazolyl)amide derivative of the formula (I) or salt thereof, may be used in admixture with other agrohorticultural agents, in order to expand both spectrum of controllable diseases and insect pest species and the period of time when effective applications are possible or to reduce the dosage.

Next, typical examples of the containing N-(4-pyrazolyl)amide derivative of the present invention are presented in Tables A to D, and typical examples of the intermediate thereof are presented in Table E. However, the present invention is by no means limited by these examples.

In the Tables, the column of the "Property" indicates the melting point (°C) or refractive index of each compound.

The general formula (I-1)

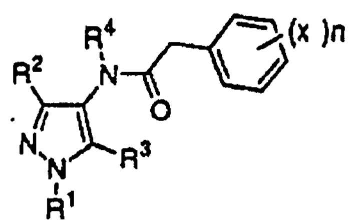

(I-1)

Table A

| No. | $R^1$ | $R^2$ | $R^3$ | $R^4$ | (X)n | Property (°C) |
|---|---|---|---|---|---|---|
| A- 1 | Me | Me | Cl | H | H | 145-147 |
| A- 2 | Me | Me | Cl | H | 2-F | 180-182 |
| A- 3 | Me | Me | Cl | H | 3-F | 166-167 |
| A- 4 | Me | Me | Cl | H | 4-F | 161-163 |
| A- 5 | Me | Me | Cl | H | 2-Cl | 199-200 |
| A- 6 | Me | Me | Cl | H | 3-Cl | 176-178 |
| A- 7 | Me | Me | Cl | H | 4-Cl | 193-196 |
| A- 8 | Me | Me | Cl | H | 4-n-Bu | 143-149 |
| A- 9 | Me | Me | Cl | H | 4-t-Bu | 86-87 |
| A-10 | Me | Me | Cl | H | $4\text{-}C\equiv CCMe_3$ | 139-141 |
| A-11 | Me | Me | Cl | H | $4\text{-}C{=}CC(Me)_2OH$ | amorphous solid |
| A-12 | Me | Me | Cl | H | 4-c-Hex | 143-145 |
| A-13 | Me | Me | Cl | H | 2-OMe | 162-164 |
| A-14 | Me | Me | Cl | H | 3-OMe | 131-133 |
| A-15 | Me | Me | Cl | H | 4-OMe | 156-159 |
| A-16 | Me | Me | Cl | H | $4\text{-}OCR_2C\equiv CH$ | 131-133 |
| A-17 | Me | Me | Cl | H | $3,4\text{-}(OMe)_2$ | 132-135 |
| A-18 | Me | Me | Cl | H | $3\text{-}CF_3$ | 140-141 |
| A-19 | Me | Me | Cl | H | $4\text{-}CF_3$ | 174-175 |
| A-20 | Me | Me | Cl | H | 4-O-n-Bu | 136-138 |
| A-21 | Me | Me | Cl | H | $4\text{-}NO_2$ | 204-207 |
| A-22 | Me | Me | Cl | H | $3,5\text{-}F_2$ | 166-167 |
| A-23 | Me | Me | Cl | H | $2,4\text{-}Cl_2$ | amorphous solid |
| A-24 | Me | Me | Cl | H | $2,6\text{-}Cl_2$ | amorphous solid |
| A-25 | Me | Me | Cl | H | $3,4\text{-}Cl_2$ | 179-181 |
| A-26 | Me | Me | Cl | H | $3,5\text{-}(CF_3)_2$ | 189-191 |
| A-27 | Me | Me | Cl | H | morpholine-4-yl | 206-218 |
| A-28 | Me | Me | Cl | H | $OCH_2$(pyridine-4-yl) | 148-152 |
| A-29 | Me | Me | Cl | H | $OCH_2CO$-t-Bu | 181-185 |
| A-30 | Me | Me | Cl | H | $4\text{-}OCH_2$ (pyridine-2-yl) | 136-142 |
| A-31 | Me | Me | Cl | H | 4-0-i-Bu | 144-145 |
| A-32 | Me | Me | Cl | H | $4\text{-}OCH_2CH_2OCH_3$ | 128-129 |
| A-33 | Me | Me | Cl | H | $4\text{-}OCH_2$-c-Rex | 124-128 |
| A-34 | Me | Me | Cl | H | $4\text{-}OCH_2CH_2CH_2CN$ | 132-135 |
| A-35 | Me | Me | Cl | H | $3\text{-}OCH_3$-4-O-n-Rex | 128-129 |
| A-36 | Me | Me | Cl | H 3-Cl-4-O-n-Hex | 109-111 | |
| A-37 | Me | Me | Cl | H 4-O-n-Hep | 127-134 | |
| A-38 | Me | Me | Cl | H 4-O-n-Oct | 135-137 | |
| A-39 | Me | Me | Cl | H 4-O-n-Dec | 122-128 | |
| A-40 | Me | Me | Cl | H 3-O-n-Hex | 107-109 | |
| A-41 | Me | Me | Cl | H $4\text{-}OCH_2CO_2$-t-Bu | 99-104 | |
| A-42 | Me | Me | Cl | H 4-O-n-Pen | 137-139 | |
| A-43 | Me | Me | Cl | H 4-O-n-Hex | 136-137 | |
| A-44 | Me | H | Cl | H 4-n-Bu | 131-132 | |
| A-45 | Me | H | Cl | H 4-O-n-Bu | 130-132 | |
| A-46 | Me | H | Cl | H 4-O-n-Pen | 127-129 | |
| A-47 | Me | H | Cl | H 4-O-n-Hex | 129-131 | |
| A-48 | Me | H | Cl | H 4-t-Bu | nD1.5524 (26.8°C) | |

Table A (continued)

| No. | $R^1$ | $R^2$ | $R^3$ | $R^4$ | (X)n | Property (°C) |
|---|---|---|---|---|---|---|
| A-49 | Me | H | Cl | H 4-O-n-Hex | 84-85 | |
| A-50 | Me | Me | Cl | H 3,5-$(OMe)_2$-4-n-Hex | 75-78 | |
| A-51 | Me | Me | Cl | H 3,4-(dimethylmethylene)-dioxy | 104-105 | |
| A-52 | Me | Me | Cl | H 3,4-(diphenylmethylene)-dioxy | amorphous | |
| A-53 | Me | H | Cl | H 3, 4-(dimethylmethylene)-dioxy | 105-109 | |
| A-54 | Et | H | Cl | H 4-OCOOMe | 96-98 | |
| A-55 | Et | H | Cl | H 4-$OCH_2COOMe$ | 88-92 | |
| A-56 | Et | H | Cl | H 3,4-(α-phenethylidene)-dioxy | nD 1.5945 (25.5°C) | |
| A-57 | Et | H | Cl | H $OSiMe_3$ | 120-125 | |
| A-58 | Et | H | Cl | H 3,4-(α-4-Br-phenethylidene)dioxy | nD 1.5995 (24. 6°C) | |

The general formula (I-1a)

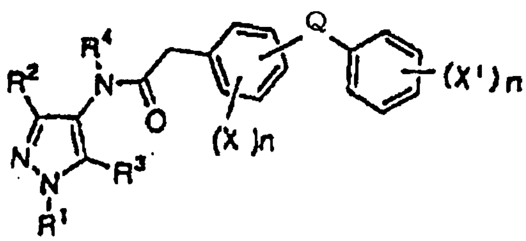

(I-1a)

Table B

| Table B ($R^4$=H except the $R^4$ specifically defined) | | | | | | | | |
|---|---|---|---|---|---|---|---|---|
| No. | $R^1$ | $R^2$ | $R^3$ | Q | Bond | $(X^1)n$ | (X)n | Property (°C) |
| B- 1 | Me | Me | Cl | 0 | 4-position | H | H | 152-154 |
| B- 2 | Me | Me | Cl | 0 | 4-position | H | 4-Cl | 140-142 |
| B- 3 | Me | Me | Cl | 0 | 4-position | H | 4-CN | 145-147 |
| B- 4 | Me | Me | Cl | 0 | 4-position | H | 4-$NO_2$ | 150-151 |
| B- 5 | Me | Me | Cl | 0 | 4-position | H | 4-$SO_2$Me | 171-176 |
| B- 6 | Me | Me | Cl | 0 | 4-position | H | 4-t-Bu | 131-134 |
| B- 7 | Me | Me | Cl | 0 | 4-position | H | 3-$CF_3$ | 109-112 |
| B- 8 | Me | Me | Cl | 0 | 4-position | 3-Cl | 4-CN | 177-179 |
| B- 9 | Me | Me | Cl | 0 | 2-position | H | 4-CN | 184-185 |
| B-10 | Me | Me | Cl | 0 | 3-position | H | 4-CN | 167-169 |
| B-11 | Me | Me | CN | 0 | 4-position | H | 4-CN | 138-141 |
| B-12 | Me | $CF_3$ | Cl | 0 | 4-position | H | 4-CN | 164-166 |
| B-13 | Me | Et | Cl | 0 | 4-position | H | 4-CN | 146-148 |
| B-14 | Me | n-Pr | Cl | 0 | 4-position | H | 4-CN | 150-152 |
| B-15 | Me | i-Pr | Cl | 0 | 4-position | H | 4-CN | 153-156 |
| B-16 | Me | Ph | Cl | 0 | 4-position | H | 4-CN | 177-183 |
| B-17 | Ph | Me | Cl | 0 | 4-position | H | 4-CN | 141-143 |
| B-18 | Me | Me | SMe | 0 | 4-position | H | 4-CN | 141-143 |
| B-19 | Me | OR | SMe | 0 | 4-position | H | 4-CN | nD 1.5975 (23.3°C) |
| B-20 | Et | Me | Cl | 0 | 4-position | H | 4-CN | 134-139 |
| B-21 | Me | $OCHF_2OPh$ | | 0 | 4-position | H | 4-CN | 107-110 |
| B-22 | Me | Me | Cl | bond | 4-position | H | H | 171-174 |
| B-23 | Me | Me | Cl | bond | 4-position | H | $OCF_3$ | 123 |
| B-24 | Me | Me | Cl | C=C | 4-position | H | H | 118-122 |
| B-25 | Me | Me | Cl | C≡C | 4-position | H | H | 190-192 |
| B-26 | Me | Me | Cl | C≡C | 4-position | H | 4-CN | 198-205 |
| B-27 | Me | Me | Cl | $OCH_2$ | 4-position | H | H | 152-154 |
| B-28 | Me | Me | Cl | $OCH_2$ | 4-position | 3-Cl | H | 145-147 |
| B-29 | Me | Me | Cl | $OCH_2$ | 4-position | H | 4-CN | 190-195 |
| B-30 | Me | Me | Cl | OCH(Me) | 4-position | H | H | 111-112 |
| B-31 | Me | Me | Cl | $NHCH_2$ | 4-position | H | H | 141-143 |

Table B (continued)

| Table B ($R^4$=H except the $R^4$ specifically defined) | | | | | | | | |
|---|---|---|---|---|---|---|---|---|
| No. | $R^1$ | $R^2$ | $R^3$ | Q | Bond | $(X^1)n$ | (X)n | Property (°C) |
| B-32 | Me | Me | Cl | $N(Ac)CH_2$ | 4-position | H | H | 128-130 |
| B-33 | Me | Me | Cl | NHCO | 4-position | H | H | 187-189 |
| B-34 | Me | Me | Cl | NHCO | 4-position | H | 4-CN | 255-259 |
| B-35 | Me | Me | Cl | NHCO | 4-position | H | 4-t-Bu | 275-278 |
| B-36 | Me | Me | Cl | $OCH_2$ | 4-position | H | 2-Cl | 172-174 |
| B-37 | Me | Me | Cl | $OCH_2$ | 4-position | H | 3-Cl | 169-171 |
| B-38 | Me | Me | Cl | $OCH_2$ | 4-position | H | 4-Cl | 149-153 |
| B-39 | Me | Me | Cl | $OCH_2$ | 4-position | 3-F | H | 129-132 |
| B-40 | Me | Me | Cl | $OCH_2$ | 4-position | 3-OMe | H | 154-158 |
| B-41 | Me | Me | Cl | $OCH_2$ | 4-position | 3-O-Bz | H | 139-142 |
| B-42 | Me | Me | Cl | $OCH_2$ | 4-position | H | 4-Me | 160-162 |
| B-43 | Me | Me | Cl | $OCH_2$ | 4-position | H | 4-F | 154-155 |
| B-44 | Me | Me | Cl | $OCH_2$ | 4-position | H | 2,6-$F_2$ | 145-146 |
| B-45 | Me | Me | Cl | $OCH_2$ | 4-position | H | 2,4-$F_2$ | 146-149 |
| B-46 | Me | Me | Cl | $OCH_2$ | 4-position | H | 4-i-Pr | 157-162 |
| B-47 | Me | Me | Cl | bond | 4-position | H | 4-Cl | 184-185 |
| B-48 | Me | Me | Cl | bond | 4-position | H | 3-Cl | 142-145 |
| B-49 | Me | Me | Cl | $OCH_2CH_2$ | 4-position | H | H | 123-125 |
| B-50 | Me | Me | Cl | $OCH_2CH_2CH_2$ | 4-position | H | H | 130-133 |
| B-51 | Me | Me | Cl | $OCH_2CH_2CH_2O$ | 4-position | H | H | 153-156 |
| B-52 | Me | Me | Cl | $OCH_2OCH_2$ | 4-position | H | H | 124-127 |
| B-53 | Me | Me | Cl | 0 | 4-position | 3-OMe | 4-CN | 133-139 |
| B-54 | $ClCH_2CH_2$ | Me | Cl | 0 | 4-position | H | 4-CN | 147-153 |
| B-55 | H | Me | Cl | 0 | 4-position | H | 4-CN | 192-201 |
| B-56 | Me | H | Cl | 0 | 4-position | H | 4-CN | 127-128 |
| B-57 | $OCHF_2$ | Me | SMe | 0 | 4-position | H | 4-CN | 125-127 |
| B-58 | Cl | Me | Cl | 0 | 4-position | H | 4-CN | 139-141 |
| B-59 | Me | Me | Cl | $OCH_2CH$=CH | 4-position | H | H | 181-185 |
| B-60 | Me | H | Cl | $OCH_2$ | 4-position | H | H | 148-149 |
| B-61 | Me | H | Cl | $OCH_2$ | 4-position | H | 4-CN | 162-164 |
| B-62 | Me | H | Cl | 0 | 4-position | 3-OMe | 4-CN | 144-146 |
| B-63 | Me | H | Cl | 0 | 4-position | H | 5-$CF_3$-pyridine-2-yl | 79-80 |

Table B (continued)

| Table B ($R^4$=H except the $R^4$ specifically defined) | | | | | | | | |
|---|---|---|---|---|---|---|---|---|
| No. | $R^1$ | $R^2$ | $R^3$ | Q | Bond | $(X^1)n$ | (X)n | Property (°C) |
| B-64 | Me | H | Cl | bond | 4-position | H | $3-CF_3$ | 146-147 |
| B-65 | Me | H | Cl | bond | 4-position | H | $4-CF_3$ | 193-194 |
| B-66 | Me | H | Cl | bond | 4-position | H | 4-Me | 209-210 |
| B-67 | Me | H | Cl | bond | 4-position | H | 2-Me | nD 1.5985 (26.1°C) |
| B-68 | Me | H | Cl | bond | 4-position | H | 3-Me | 166-168 |
| B-69 | Me | H | Cl | bond | 4-position | H | $2-CF_3$ | 128-129 |
| B-70 | Me | H | Cl | bond | 4-position | H | H | 177-178 |
| B-71 | Me | H | Cl | bond | 4-position | H | $4-OCF_3$ | 188-190 |
| B-72 | Me | Me | Cl | $OCH_2$ | 4-position | H | 4-Me | 154-158 |
| B-73 | Me | Me | Cl | $OCH_2$ | 4-position | H | $4-NO_2$ | 148-153 |
| B-74 | Me | Me | Cl | $OCH_2$ | 3-position | H | H | 123-129 |
| B-75 | Me | Me | Cl | $OCH_2$ | 2-position | H | H | 166-169 |
| B-76 | Me | Me | Cl | C=0 | 4-position | H | H | 147 |
| B-77 | Me | Me | Cl | C=NOMe | 4-position | H | H | 139-142 |
| B-78 | Me | Me | Cl | 0 | 4-position | H | $4-C(NH_2)$ =NOH | 192-193 |
| B-79 | Me | Me | Cl | $OCH_2$ | 4-position | $3-NO_2$ | H | 169-173 |
| B-80 | Me | H | Cl | bond | 3-position | H | H | 124-126 |
| B-81 | Me | H | Cl | bond | 4-position | H | 2-Cl | nD 1.6145 (26.8°C) |
| B-82 | Me | Me | Cl | dioxolane | 4-position | H | H | nD 1.5879 (25.7°C) |
| B-83 | Me | H | Cl | $OCH_2$ | 3-position | H | H | 111-112 |
| B-84 | Me | H | Cl | 0 | 4-position | H | $4-CF_3$ | 145-146 |
| B-85 | Me | Me | Cl | $OCH_2$ | 3-position | 4-OMe | H | 167-171 |
| B-86 | Me | Me | Cl | $OCH_2$ | 4-position | 3,5-(OMe) | $_2$H | 165-167 |
| B-87 | Me | Me | Cl | $OCH_2$ | 4-position | 3-OMe | 3-OMe | 180-183 |
| B-88 | Me | Me | Cl | 0 | 4-position | H | 4-CN | amorphous ($R^4$=Me) |
| B-89 | Me | Cl | H | 0 | 4-position | H | 4-CN | amorphous |
| B-90 | Me | Me | Cl | 0 | 4-position | $3-NO_2$ | 4-CN | 207 |
| B-91 | Me | Cl | Cl | 0 | 4-position | 3-OMe | 4-CN | amorphous |
| B-92 | Me | Me | Cl | 0 | 4-position | H | 4-F | 128-131 |
| B-93 | Me | H | Cl | 0 | 4-position | H | 4-F | 122-124 |
| B-94 | Me | H | Cl | 0 | 4-position | 3-OMe | 4-Cl | 86-91 |
| B-95 | Me | Me | Cl | 0 | 4-position | 3-OMe | 4-OMe | 116-119 |

Table B (continued)

| Table B ($R^4$=H except the $R^4$ specifically defined) | | | | | | | | |
|---|---|---|---|---|---|---|---|---|
| No. | $R^1$ | $R^2$ | $R^3$ | Q | Bond | $(X^1)n$ | (X)n | Property (°C) |
| B-96 | Me | Me | Cl | 0 | 4-position | 3-OMe | 4-Ac | 120-123 |
| B-97 | Me | Me | Cl | 0 | 4-position | 3-OMe | 4-I | 148-151 |
| B-98 | Me | Me | Cl | $CH_2$ | 4-position | H | H | 166 |
| B-99 | Me | H | Cl | 0 | 4-position | H | 4-Br | 99-102 |
| B-100 | Me | H | Cl | 0 | 4-position | H | 4-I | 101-106 |
| B-101 | Me | Me | Cl | NH | 4-position | H | 4-Cl | 145-154 |
| B-102 | Et | H | Cl | 0 | 4-position | H | 4-CN | 111-123 |
| B-103 | Me | H | Cl | 0 | 4-position | 3-OMe | 4-F | amorphous |
| B-104 | Me | H | Cl | $CH_2Ph$ | 4-position | H | H | 152-155 |
| B-105 | Me | Me | Cl | 0 | 4-position | $3-NO_2$ | 4-Cl | 182 |
| B-106 | Me | H | Cl | NH | 4-position | H | 4-F | 124-128 |
| B-107 | Me | H | Cl | 0 | 4-position | H | $2,4-Cl_2$ | 120-126 |
| B-108 | Me | H | Cl | 0 | 4-position | H | $3,4-Cl_2$ | 81-89 |
| B-109 | Me | Me | H | 0 | 4-position | H | 4-CN | amorphous |
| B-110 | Et | H | Cl | 0 | 4-position | 3-OMe | 4-CN | 119-124 |
| B-111 | Et | H | Cl | 0 | 4-position | 3-OMe | 4-Cl | amorphous |
| B-112 | Me | Me | Cl | 0 | 4-position | 3-NHMe | 4-Cl | amorphous |
| B-113 | Et | Me | Cl | 0 | 4-position | H | 4-Cl | 127-128 |
| B-114 | Me | H | Cl | 0 | 4-position | H | 4-Cl | 115-117 |
| B-115 | Me | H | Cl | 0 | 4-position | 3-SMe | 4-Cl | nD 1.6050 (19. 7°C) |
| B-116 | Et | H | Cl | 0 | 4-position | H | 4-F | 72-74 |
| B-117 | Et | H | Cl | 0 | 4-position | 3-OMe | 4-F | 82-87 |
| B-118 | Me | H | Cl | 0 | 4-position | 3-F | 4-F | 110-114 |
| B-119 | Me | H | Cl | 0 | 4-position | 3-0-i-Pr | 4-F | 89-91 |
| B-120 | Me | H | Cl | 0 | 4-position | 3-0-i-Pr | 4-Cl | amorphous |
| B-121 | Me | Me | Cl | 0 | 4-position | H | 4-Br | 102-105 |
| B-122 | Me | H | Cl | 0 | 4-position | H | 4-CN | 139-141 |
| B-123 | Me | H | Cl | 0 | 4-position | 3-OMe $R^4$= $EtOCH_2$ | 4-F | nD 1.4765 (27.7°C) |
| B-124 | Me | H | Cl | 0 | 4-position | 3-OMe $R^4$= Et | 4-CN | nD 1.4461 (26.3°C) |
| B-125 | Me | H | Cl | 0 | 4-position | 3-OMe | 4-CN | nD 1.5605 |

Table B (continued)

| Table B ($R^4$=H except the $R^4$ specifically defined) | | | | | | | | |
|---|---|---|---|---|---|---|---|---|
| No. | $R^1$ | $R^2$ | $R^3$ | Q | Bond | $(X^1)n$ | (X)n | Property (°C) |
| | | | | | | $R^4$= $EtOCH_2$ | | (25.8°C) |
| B-126 | Et | H | OH | 0 | 4-position | 3-OMe | 4-F | |
| B-127 | Et | H | OMe | 0 | 4-position | 3-OMe | 4-F | |
| B-128 | Et | H | Cl | 0 | 4-position | 3-OH | 4-$CF_3$ | |
| B-129 | Et | H | Cl | 0 | 4-position | 3-OMe | 4-$CF(CF_3)_2$ | |
| B-130 | Et | H | Cl | 0 | 4-position | 3-OMe | 4-$CH(CF_3)_2$ | |
| B-131 | Et | H | Cl | 0 | 4-position | 3-OMe | 4-$CF_2CF_3$ | |
| B-132 | Et | H | Cl | 0 | 4-position | 3-OH | 4-F | |
| B-133 | Et | H | Cl | 0 | 4-position | 3-$CHF_2$ | 4-F | |
| B-134 | Et | H | Cl | 0 | 4-position | 3-$NH_2$ | 4-F | |
| B-135 | Et | H | Cl | 0 | 4-position | 3-NHMe | 4-F | |
| B-136 | Et | H | Cl | 0 | 4-position | 3-$NM_2$ | 4-F | |
| B-137 | Et | H | Cl | 0 | 4-position | 3-NHAc | 4-F | |
| B-138 | Et | H | Cl | 0 | 4-position | 3-$NHCOCF_3$ | 4-F | |
| B-139 | Et | H | Cl | 0 | 4-position | 3-$NHSO_2Me$ | 4-F | |
| B-140 | Et | H | Cl | 0 | 4-position | 3-$NHSO_2CF_3$ | 4-F | |
| B-141 | Et | H | Cl | 0 | 4-position | 3-SH | 4-F | |
| B-142 | Et | H | Cl | 0 | 4-position | 3-SMe | 4-F | |
| B-143 | Et | H | Cl | 0 | 4-position | 3-SOMe | 4-F | |
| B-144 | Et | H | Cl | 0 | 4-position | 3-$SO_2Me$ | 4-F | |
| B-145 | Et | H | Cl | 0 | 4-position | 3-$SCHF_2$ | 4-F | |
| B-146 | Et | H | Cl | 0 | 4-position | 3-$SOCRF_2$ | 4-F | |
| B-147 | Et | H | Cl | 0 | 4-position | 3-$SO_2CHF_2$ | 4-F | |
| B-148 | $CH_2$=CH | H | Cl | 0 | 4-position | 3-OMe | 4-F | |
| B-149 | $NCCH_2$ | H | Cl | 0 | 4-position | 3-OMe | 4-F | |
| B-150 | $OHCCH_2$ | H | Cl | 0 | 4-position | 3-OMe | 4-F | |
| B-151 | MeON=$CHCH_2$ | H | Cl | 0 | 4-position | 3-OMe | 4-F | |
| B-152 | $EtOOCCH_2$ | H | Cl | 0 | 4-position | 3-OMe | 4-F | |
| B-153 | $Me_2NOCCH_2$ | H | Cl | 0 | 4-position | 3-OMe | 4-F | |
| B-154 | $CH_2CF_3$ | H | Cl | 0 | 4-position | 3-OMe | 4-F | |
| B-155 | $CH_2CH_2F$ | H | Cl | 0 | 4-position | 3-OMe | 4-F | |

Table B (continued)

| Table B ($R^4$=H except the $R^4$ specifically defined) | | | | | | | | |
|---|---|---|---|---|---|---|---|---|
| No. | $R^1$ | $R^2$ | $R^3$ | Q | Bond | $(X^1)n$ | (X)n | Property (°C) |
| B-156 | $CH_2CH_2Cl$ | H | Cl | 0 | 4-position | 3-OMe | 4-F | |
| B-157 | n-Pr | H | Cl | 0 | 4-position | 3-OMe | 4-F | |
| B-158 | Et | H | Cl | 0 | 4-position | 3-OMe<br>$R^4$=$MeOCH_2$ | 4-F | |
| B-159 | Et | H | Cl | 0 | 4-position | 3-OMe<br>$R^4$=$CH_2CH$=$CH_2$ | 4-F | |
| B-160 | Et | H | Cl | 0 | 4-position | 3-OMe<br>$R^4$=$CH_2C{\equiv}CH$ | 4-F | |
| B-161 | Et | H | Cl | 0 | 4-position | 3-OMe<br>$R^4$=$PhCH_2$ | 4-F | |
| B-162 | Et | H | Cl | 0 | 4-position | 3-OMe<br>$R^4$=i-Pr | 4-F | |
| B-163 | Et | H | Cl | 0 | 4-position | 3-OMe<br>$R^4$=n-Pr | 4-F | |
| B-164 | Et | H | Cl | 0 | 4-position | 3-OMe<br>$R^4$=$MeOCH_2CH_2OCH_2$ | 4-F | |
| B-165 | Et | H | Cl | 0 | 4-position | 3-OMe<br>$R^4$=$NCCH_2$ | 4-F | |
| B-166 | Et | H | Cl | 0 | 4-position | 3-OMe<br>$R^4$= $EtOOCCH_2$ | 4-F | |
| B-167 | Et | H | Cl | 0 | 4-position | 3-OMe<br>$R^4$= Ac | 4-F | |
| B-168 | Et | H | Cl | 0 | 4-position | 3-OMe<br>$R^4$= EtCO | 4-F | |
| B-169 | Et | H | Cl | 0 | 4-position | 3-OMe<br>$R^4$= n-PrCO | 4-F | |
| B-170 | Et | H | Cl | 0 | 4-position | 3-OMe<br>$R^4$ = i-PrCO | 4-F | |
| B-171 | Et | H | Cl | 0 | 4-position | 3-OMe<br>$R^4$ = t-BuCO | 4-F | |
| B-172 | Et | H | Cl | 0 | 4-position | 3-OMe<br>$R^4$= PhCO | 4-F | |

Table B (continued)

| Table B ($R^4$=H except the $R^4$ specifically defined) | | | | | | | | |
|---|---|---|---|---|---|---|---|---|
| No. | $R^1$ | $R^2$ | $R^3$ | Q | Bond | $(X^1)n$ | (X)n | Property (°C) |
| B-173 | Et | H | Cl | 0 | 4-position | 3-OMe<br>$R^4$= $PhCH_2CO$ | 4-F | |
| B-174 | Et | H | Cl | 0 | 4-position | 3-OMe<br>$R^4$= c-PrCO | 4-F | |
| B-175 | Et | H | Cl | 0 | 4-position | 3-OMe<br>$R^4$= MeCH=CHCO | 4-F | |
| B-176 | Et | H | Cl | 0 | 4-position | 3-OMe<br>$R^4$= MeOOC | 4-F | |
| B-177 | Et | H | Cl | 0 | 4-position | 3-OMe<br>$R^4$= EtOOC | 4-F | |
| B-178 | Et | H | Cl | 0 | 4-position | 3-OMe<br>$R^4$= n-PrOOC | 4-F | |
| B-179 | Et | H | Cl | 0 | 4-position | 3-OMe<br>$R^4$= i-PrOOC | 4-F | |
| B-180 | Et | H | Cl | 0 | 4-position | 3-OMe<br>$R^4$ = t-BuOOC | 4-F | |
| B-181 | Et | H | Cl | 0 | 4-position | 3-OMe<br>$R^4$ = PhOOC | 4-F | |
| B-182 | Et | H | Cl | 0 | 4-position | 3-OMe<br>$R^4$ = $PhCH_2OOC$ | 4-F | |
| B-183 | Et | H | Cl | 0 | 4-position | 3-OMe<br>$R^4$ = $MeSCH_2$ | 4-F | |
| B-184 | Et | H | Cl | 0 | 4-position | 3-OMe<br>$R^4$=$CF_3CH_2$ | 4-F | |
| B-185 | Et | H | Cl | 0 | 4-position | 3-OMe | 4-$CF_3$-2-$OCH_2Ph$ | 84-89 |
| B-186 | Et | H | Cl | bond | 4-position | H | 4-Cl | 134-140 |

In the Table B, dioxolane as Q of the compound No. B-82 is represented by the following formula, and "Bond" in the Table B means the bonded position of the group Q.

dioxolane:

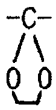

The general formula (I-2)

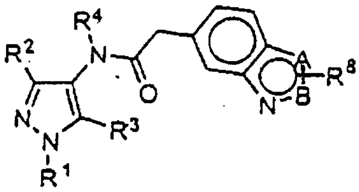

(I-2)

Table C

| No. | $R^1$ | $R^2$ | $R^3$ | $R^4$ | A | B | $R^8$ | Property (°C) |
|---|---|---|---|---|---|---|---|---|
| C-1 | Me | Me | Cl | H | 0 | $CR^8$ | $CH_2$-t-Bu | 130 |
| C-2 | Et | H | Cl | H | N | $NR^8$ | n-Pr | 114 |
| C-3 | Et | H | Cl | H | $NR^8$ | N | n-Pr | nD 1.5820 (22. 0°C) |
| C-4 | Et | H | Cl | H | 0 | $CR^8$ | $CH_2$-t-Bu | |
| C-5 | $CH_2$=CH | H | Cl | H | 0 | $CR^8$ | $CH_2$-t-Bu | |
| C-6 | $NCCH_2$ | H | Cl | H | 0 | $CR^8$ | $CH_2$-t-Bu | |
| C-7 | $OHCCH_2$ | H | Cl | H | 0 | CR | $CH_2$-t-Bu | |
| C-8 | MeON=$CHCH_2$ | H | Cl | H | 0 | $CR^8$ | $CH_2$-t-Bu | |
| C-9 | $EtOOCCH_2$ | H | Cl | H | 0 | $CR^8$ | $CH_2$-t-Bu | |
| C-10 | $Me_2NOCCH_2$ | H | Cl | H | 0 | $CR^8$ | $CH_2$-t-Bu | |
| C-11 | $CH_2CF_3$ | H | Cl | H | 0 | $CR^8$ | $CH_2$-t-Bu | |
| C-12 | $CH_2CH_2F$ | H | Cl | H | 0 | $CR^8$ | $CH_2$-t-Bu | |
| C-13 | $CH_2CH_2Cl$ | H | Cl | H | 0 | $CR^8$ | $CH_2$-t-Bu | |
| C-14 | n-Pr | H | Cl | H | 0 | $CR^8$ | $CH_2$-t-Bu | |
| C-15 | Et | H | OH | H | 0 | $CR^8$ | $CH_2$-t-Bu | |
| C-16 | Et | H | OMe | H | 0 | $CR^8$ | $CH_2$-t-Bu | |
| C-17 | Et | H | Cl | $MeOCH_2$ | 0 | $CR^8$ | $CH_2$-t-Bu | |
| C-18 | Et | H | Cl | $CH_2$=$CHCH_2$ | 0 | $CR^8$ | $CH_2$-t-Bu | |
| C-19 | Et | H | Cl | $CH\equiv CCH_2$ | 0 | $CR^8$ | $CH_2$-t-Bu | |
| C-20 | Et | H | Cl | $PhCH_2$ | | $CR^8$ | $CH_2$-t-Bu | |
| C-21 | Et | H | Cl | i-Pr | 0 | $CR^8$ | $CH_2$-t-Bu | |
| C-22 | Et | H | Cl | n-Pr | 0 | $CR^8$ | $CH_2$-t-Bu | |
| C-23 | Et | H | Cl | $MeOCH_2CH_2OCH_2$ | 0 | $CR^8$ | $CH_2$-t-Bu | |
| C-24 | Et | H | Cl | $NCCH_2$ | 0 | $CR^8$ | $CH_2$-t-Bu | |
| C-25 | Et | H | Cl | $EtOOCCH_2$ | 0 | $CR^8$ | $CH_2$-t-Bu | |
| C-26 | Et | H | Cl | Ac | 0 | $CR^8$ | $CH_2$-t-Bu | |
| C-27 | Et | H | Cl | EtCO | 0 | $CR^8$ | $CH_2$-t-Bu | |
| C-28 | Et | H | Cl | n-PrCO | 0 | $CR^8$ | $CH_2$-t-Bu | |
| C-29 | Et | H | Cl | i-PrCO | 0 | $CR^8$ | $CH_2$-t-Bu | |

Table C (continued)

| No. | $R^1$ | $R^2$ | $R^3$ | $R^4$ | A | B | $R^8$ | Property (°C) |
|---|---|---|---|---|---|---|---|---|
| C-30 | Et | H | Cl | t-BuCO | 0 | $CR^8$ | $CH_2$-t-Bu | |
| C-31 | Et | H | Cl | PhC | 0 | $CR^8$ | $CH_2$-t-Bu | |
| C-32 | Et | H | Cl | $PhCH_2CO$ | 0 | $CR^8$ | $CH_2$-t-Bu | |
| C-33 | Et | H | Cl | c-PrCO | 0 | $CR^8$ | $CH_2$-t-Bu | |
| C-34 | Et | H | Cl | MeCH=CHCO | 0 | $CR^8$ | $CH_2$-t-Bu | |
| C-35 | Et | H | Cl | MeOOC | 0 | $CR^8$ | $CH_2$-t-Bu | |
| C-36 | Et | H | Cl | EtOOC | 0 | $CR^8$ | $CH_2$-t-Bu | |
| C-37 | Et | H | Cl | n-PrOOC | 0 | $CR^8$ | $CH_2$-t-Bu | |
| C-38 | Et | H | Cl | i-PrOOC | 0 | $CR^8$ | $CH_2$-t-Bu | |
| C-39 | Et | H | Cl | t-BuOOC | 0 | $CR^8$ | $CH_2$-t-Bu | |
| C-40 | Et | H | Cl | PhOOC | | $CR^8$ | $CH_2$-t-Bu | |
| C-41 | Et | H | Cl | $PhCH_2OOC$ | 0 | $CR^8$ | $CH_2$-t-Bu | |
| C-42 | Et | H | Cl | $eSCH_2$ | 0 | $CR^8$ | $CH_2$-t-Bu | |
| C-43 | Et | H | Cl | $CF_3CH_2$ | 0 | $CR^8$ | $CH_2$-t-Bu | |

The general formula (I)

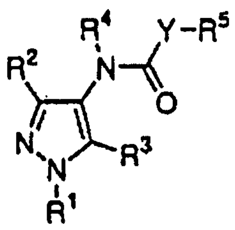

(I)

Table D

Table D ($R^4$=H except the $R^4$ specifically defined)

| No. | $R^1$ | $R^2$ | $R^3$ | $R^5$ | Y | Property (℃) |
|---|---|---|---|---|---|---|
| D-1 | Me | Me | Cl | naphthalene-1-yl | $CH_2$ | 222-223 |
| D-2 | Me | Me | Cl | naphthalene-2-yl | $CH_2$ | 206-207 |
| D-3 | Me | Me | Cl | 5-Cl-thiophene-2-yl | $CH_2$ | 118-121 |
| D-4 | Me | Me | Cl | indole-3-yl | $CH_2$ | 131-135 |
| D-5 | Me | Me | Cl | 4-O-(5-$CF_3$-pyridine-2-yl)-Ph | $CH_2$ | 139-140 |
| D-6 | Me | Me | Cl | 4-O-(3-Cl-5-$CF_3$-pyridine-2-yl)-Ph | $CH_2$ | 200-204 |
| D-7 | Me | Me | Cl | 4-Cl-Ph | $CMe_2$ | 129-130 |
| D-8 | Me | Me | Cl | 4-O-(4-CN-Ph)-Ph | $CH_2CH_2$ | 138-141 |
| D-9 | Me | Me | Cl | 4-OPh-Ph | CH=CH | 173-176 |
| D-10 | Me | Me | Cl | 4-O-(4-CN-Ph)-Ph | CH=CH | 156-160 |
| D-11 | Me | Me | Cl | 4-OPh-Ph | $CH_2O$ | 109-110 |
| D-12 | Me | Me | Cl | 5-Br-thiophene-2-yl | $CH_2$ | 136-138 |
| D-13 | Me | Me | Cl | 4-O-(5-$NO_2$-pyridine-2-yl)-Ph | $CH_2$ | 156-162 |

Table D (cont'd)

| No. | $R^1$ | $R^2$ | $R^3$ | $R^5$ | Y | Property (℃) |
|---|---|---|---|---|---|---|
| D-14 | Me | Me | Cl | 3-OMe-4-O-(5-$CF_3$-pyridine-2-yl)-Ph | $CH_2$ | 160-162 |
| D-15 | Me | Me | Cl | 3-OMe-4-O-(5-$NO_2$-pyridine- 2-yl)-Ph | $CH_2$ | 163-165 |
| D-16 | Me | Me | Cl | 5-(4-Cl-Ph)-thiophene -2-yl | $CH_2$ | 159-161 |
| D-17 | Me | Me | Cl | 4-OMe-3-O-(5-$CF_3$-pyridine-2-yl)-Ph | $CH_2$ | 114-117 |
| D-18 | Me | H | Cl | 3-OMe-4-O-(5-$CF_3$-pyridine-2-yl)-Ph | $CH_2$ | 132-135 |
| D-19 | Me | Me | Cl | 3-(thiophene-2-yl)Ph | $CH_2$ | 152-155 |
| D-20 | Me | H | Cl | 4-OMe-3-O-(5-$CF_3$-pyridine-2-yl)-Ph | $CH_2$ | 117-121 |
| D-21 | Me | Me | Cl | 4-(thiophene-2-yl)-Ph | $CH_2$ | 187-192 |
| D-22 | Me | Me | Cl | 4-(thiophene-3-yl)-Ph | $CH_2$ | 200-204 |
| D-23 | Me | Cl | H | 4-O(5-$CF_3$-pyridine-2-yl)-Ph | $CH_2$ | amorphous |
| D-24 | Me | Me | Cl | 3-OEt-4-O-(5-$CF_3$-pyridine-2-yl)-Ph | $CH_2$ | 123-126 |
| D-25 | Me | H | Cl | 3-OEt-4-O-(5-$CF_3$-pyridine-2-yl)-Ph | $CH_2$ | 104-110 |
| D-26 | Me | Me | Cl | 3-Me-4-O-(5-$CF_3$-pyridine-2-yl)-Ph | $CH_2$ | 144-148 |
| D-27 | Me | Me | Cl | benzothiophene-3-yl | $CH_2$ | 185-190 |

Table D (cont'd)

| No. | $R^1$ | $R^2$ | $R^3$ | $R^5$ | Y | Property (℃) |
|---|---|---|---|---|---|---|
| D-28 | Me | H | Cl | 3-Me-4-O-(5-$CF_3$-pyridine-2-yl)-Ph | $CH_2$ | 115-117 |
| D-29 | Me | Me | Cl | 3-$NO_2$-4-O-(5-$CF_3$-pyridine-2-yl)-Ph | $CH_2$ | amorphous |
| D-30 | Me | Me | Cl | 2-O(4-Cl-Ph)-pyridine-5-yl | $CH_2$ | 135-140 |
| D-31 | Me | Me | Cl | 2-Me-4-O-(5-$CF_3$-pyridine-2-yl)-Ph | $CH_2$ | 126-131 |
| D-32 | Me | H | Cl | 2-Me-4-O-(5-$CF_3$--pyridine-2-yl)-Ph | $CH_2$ | 86-89 |
| D-33 | Et | H | Cl | 3-OMe-4-O-(5-$CF_3$-pyridine-2-yl)-Ph | $CH_2$ | 95-99 |
| D-34 | Me | H | Cl | 2-OMe-4-O-(5-$CF_3$-pyridine-2-yl)-Ph | $CH_2$ | amorphous |
| D-35 | Me | H | Cl | 4-O(4,6-$Me_2$-pyrimidine-2-yl)Ph | $CH_2$ | 129-133 |
| D-36 | Me | H | Cl | 4-O-(pyrazine-2-yl)-Ph | $CH_2$ | 91-96 |
| D-37 | Me | Me | Cl | 2-t-Bu-thiazole-4-yl | $CH_2$ | 113-115 |
| D-38 | Et | Me | Cl | 3-OMe-4-O-(5-$CF_3$-pyridine-2-yl)-Ph | $CH_2$ | 124-127 |
| D-39 | Me | Me | Cl | 5-O-(5-Cl-pyridine-2-yl)-Ph | $CH_2$ | 130-133 |
| D-40 | Et | H | Cl | 5-O-(5-Cl-pyridine-2-yl)-Ph | $CH_2$ | nD 1.5908 (22.1℃) |
| D-41 | Et | H | Cl | 4-O-(5-$CF_3$-pyridine-2-yl)-Ph | $CH_2$ | 138-139 |

Table D (cont'd)

| No. | $R^1$ | $R^2$ | $R^3$ | $R^5$ | Y | Property (℃) |
|---|---|---|---|---|---|---|
| D-42 | Me | H | Cl | 2,3-$(OMe)_2$-4-O-(5-$CF_3$-pyridine-2-yl)-Ph | $CH_2$ | amorphous |
| D-43 | Me | H | Cl | 3,5-$(OMe)_2$-4-O-(5-$CF_3$-pyridine-2-yl)-Ph | $CH_2$ | 81-87 |
| D-44 | Me | H | Cl | 2,3-$Me_2$-4-O-(5-$CF_3$-pyridine-2-yl)-Ph | $CH_2$ | 156-158 |
| D-45 | Me | H | Cl | 3-Cl-4-O-(5-$CF_3$-pyridine-2-yl)-Ph | $CH_2$ | 119-123 |
| D-46 | Me | H | Cl | 3-F-4-O-(5-$CF_3$-pyridine-2-yl)-Ph | $CH_2$ | 136-138 |
| D-47 | Me | H | Cl | 3-OMe-4-O-(5-Cl-pyridine-2-yl)-Ph | $CH_2$ | 94-97 |
| D-48 | Et | H | Cl | 3-OMe-4-O-(5-Cl-pyridine-2-yl)-Ph | $CH_2$ | 80-81 |
| D-49 | Me | H | Cl | 2-O-(4-F-Ph)-pyridine-5-yl | $CH_2$ | 142-145 |
| D-50 | Me | H | Cl | 2-O-(4-F-Ph)-3-Cl-pyridine-5-yl | $CH_2$ | 145-152 |
| D-51 | n-Pr | H | Cl | 3-OMe-4-O-(5-$CF_3$-pyridine-2-yl)-Ph | $CH_2$ | amorphous |
| D-52 | Me | H | Cl | 3-N(Me)OH-4-O-(5-$CF_3$-pyridine-2-yl)-Ph | $CH_2$ | 175-176 |
| D-53 | Et | H | Cl | 3-OMe-4-O-(5-$NO_2$-pyridine-2-yl)-Ph | $CH_2$ | 135-140 |

Table D (cont'd)

| No. | $R^1$ | $R^2$ | $R^3$ | $R^5$ | Y | Property (℃) |
|---|---|---|---|---|---|---|
| D-54 | Et | H | Cl | 3-OMe-4-O-(5-$NH_2$-pyridine-2-yl)-Ph | $CH_2$ | 59-61 |
| D-55 | Et | H | Cl | 4-O-(6-Cl-pyridazine-3-yl)-Ph | $CH_2$ | 146-149 |
| D-56 | Me | H | Cl | 4-O-(6-Cl-pyridazine-3-yl)-Ph | $CH_2$ | 122-127 |
| D-57 | Me | H | Cl | 3-OMe-4-O-(6-Cl-pyridazine-3-yl)-Ph | $CH_2$ | 128-131 |
| D-58 | Et | H | Cl | 3-OMe-4-O-(6-Cl-pyridazine-3-yl)-Ph | $CH_2$ | 158-162 |
| D-59 | Et | H | Cl | 3-OMe-4-O-(5-F-pyridine-2-yl)-Ph | $CH_2$ | 104-108 |
| D-60 | Et | H $R^4$=EtO$CH_2$ | Cl | 3-OMe-4-O-(5-$CF_3$-pyridine-2-yl)-Ph | $CH_2$ | 81-82 |
| D-61 | Ph | H | Cl | 3-OMe-4-O-(5-$CF_3$-pyridine-2-yl)-Ph | $CH_2$ | 132-145 |
| D-62 | i-Pr | H | Cl | 3-OMe-4-O-(5-$CF_3$-pyridine-2-yl)-Ph | $CH_2$ | 51-60 |
| D-63 | Et | H $R^4$=MeO$CH_2$ | Cl | 3-OMe-4-O-(5-$CF_3$-pyridine-2-yl)-Ph | $CH_2$ | nD 1.5359 (20.0℃) |
| D-64 | Et | H | Cl | 3-OMe-4-O-(5-Br-pyridine-2-yl)-Ph | $CH_2$ | 90-92 |
| D-65 | Et | H | Cl | 3-OMe-4-O-(6-$CF_3$-pyridine-2-yl)-Ph | $CH_2$ | amorphous |

Table D (cont'd)

| No. | $R^1$ | $R^2$ | $R^3$ | $R^5$ | Y | Property (℃) |
|---|---|---|---|---|---|---|
| D-66 | t-Bu | H | Cl | 3-OMe-4-O-(5-$CF_3$-pyridine-2-yl)-Ph | $CH_2$ | 45-52 |
| D-67 | Et | H $R^4$= Et | Cl | 3-OMe-4-O-(5-$CF_3$-pyridine-2-yl)-Ph | $CH_2$ | nD 1.5465 (21.1℃) |
| D-68 | Et | H $R^4$= Me | Cl | 3-OMe-4-O-(5-$CF_3$-pyridine-2-yl)-Ph | $CH_2$ | nD 1.5322 (28.6℃) |
| D-69 | Et | H $R^4$= $CH_2CH=CH_2$ | Cl | 3-OMe-4-O-(5-$CF_3$-pyridine-2-yl)-Ph | $CH_2$ | nD 1.5366 (28.6℃) |
| D-70 | $CH_2CF_3$ | H | Cl | 3-OMe-4-O-(5-$CF_3$-pyridine-2-yl)-Ph | $CH_2$ | 123-128 |
| D-71 | $CH_2CH_2Cl$ | H | Cl | 3-OMe-4-O-(5-$CF_3$-pyridine-2-yl)-Ph | $CH_2$ | amorphous |
| D-72 | Et | H | Cl | 3-OMe-4-O-(5-CN-pyridine-6-yl)-Ph | $CH_2$ | 142-145 |
| D-73 | Et | H | Cl | 3-OMe-4-O-(2-Me-3-$NO_2$-pyridine-6-yl)-Ph | $CH_2$ | 132-135 |
| D-74 | Et | H | Cl | 3-OMe-4-O-(benzothiazole-2-yl)-Ph | $CH_2$ | nD 1.5895 (25.6℃) |
| D-75 | Me | H | Cl | 3-OMe-4-O-(5-$CF_3$-pyridine-2-yl)-Ph | NH | 185-192 |
| D-76 | Et | H | Cl | 3-OMe-4-O-(5-$CF_3$-pyridine-2-yl)-Ph | NH | 190-195 |

Table D (cont'd)

| No. | $R^1$ | $R^2$ | $R^3$ | $R^5$ | Y | Property (℃) |
|---|---|---|---|---|---|---|
| D-77 | Et | H | Cl | 3-OMe-4-O-(5-I-pyridine-2-yl)-Ph | $CH_2$ | 87-89 |
| D-78 | Et | H | Cl | 3-OMe-4-O-(6-$CF_3$-pyridazine-3-yl)-Ph | $CH_2$ | 122-127 |
| D-79 | Et | H | Br | 3-OMe-4-O-(5-$CF_3$-pyridine-2-yl)-Ph | $CH_2$ | 105-107 |
| D-80 | Et | H | Cl | 3,4-(O-(5-$CF_3$-pyridine-2-yl))$_2$-Ph | $CH_2$ | nD 1.5408 (24.2℃) |
| D-81 | Et | H | Cl | 3-OMe-4-O-(5-Br-pyrimidine-2-yl)-Ph | $CH_2$ | 93-98 |
| D-82 | Et | H $R^4$= n-Pr | Cl | 3-OMe-4-O-(5-$CF_3$-pyridine-2-yl)-Ph | $CH_2$ | nD 1.5281 (24.0℃) |
| D-83 | Et | H $R^4$= i-Pr | Cl | 3-OMe-4-O-(5-$CF_3$-pyridine-2-yl)-Ph | $CH_2$ | 136-144 |
| D-84 | Et | H | Cl | 3-OMe-4-O-(quinoline-2-yl)-Ph | $CH_2$ | 100-105 |
| D-85 | Et | H | Cl | 3-OMe-4-O-(5-Br-thiazole-2-yl)-Ph | $CH_2$ | 78-82 |
| D-86 | Et | H | Cl | 3-$OCHF_2$-4-O-(5-$CF_3$-pyridine-2-yl)-Ph | $CH_2$ | nD 1.5265 (24.3℃) |
| D-87 | Et | H | I | 3-OMe-4-O-(5-$CF_3$-pyridine-2-yl)-Ph | $CH_2$ | 160-161 |
| D-88 | Et | H $R^4$= $MeOCH_2CH_2OCH_2$ | Cl | 3-OMe-4-O-(5-$CF_3$-pyridine-2-yl)-Ph | $CH_2$ | nD 1.5235 (25.4℃) |

Table D (cont'd)

| No. | $R^1$ | $R^2$ | $R^3$ | $R^5$ | Y | Property (℃) |
|---|---|---|---|---|---|---|
| D-89 | Et | H<br>$R^4 = CH_2C{\equiv}CH_2$ | Cl | 3-OMe-4-O-(5-$CF_3$-pyridine-2-yl)-Ph | $CH_2$ | nD 1.5422 (25.1℃) |
| D-90 | Et | H<br>$R^4 = PhCH_2$ | Cl | 3-OMe-4-O-(5-$CF_3$-pyridine-2-yl)-Ph | $CH_2$ | nD 1.5508 (25.5℃) |
| D-91 | Et | H<br>$R^4 = EtOCH_2$ | Cl | 3-OMe-4-O-(5-Cl-pyridine-2-yl)-Ph | $CH_2$ | nD 1.5449 (27.4℃) |
| D-92 | Et | H<br>$R^4 = NCCH_2$ | Cl | 3-OMe-4-O-(5-$CF_3$-pyridine-2-yl)-Ph | $CH_2$ | 102-108 |
| D-93 | Et | H | Cl | 3-OMe-4-O-(3,5-$Cl_2$-pyridine-2-yl)-Ph | $CH_2$ | 78-83 |
| D-94 | Et | H | Cl | 3-OMe-4-O-(4-$CF_3$-thiazole-2-yl)-Ph | $CH_2$ | 107-109 |
| D-95 | $CH_2CH_2F$ | H | Cl | 3-OMe-4-O-(5-$CF_3$-pyridine-2-yl)-Ph | $CH_2$ | 102-105 |
| D-96 | Et | H | Cl | 3-OMe-4-O-(3-Cl-5-CN-pyridine-2-yl)-Ph | $CH_2$ | 155-159 |
| D-97 | Et | H | Cl | 3-OMe-4-O-(quinoxaline-2-yl)-Ph | $CH_2$ | 89-94 |
| D-98 | Et | H<br>$R^4 = EtOOCCH_2$ | Cl | 3-OMe-4-O-(5-$CF_3$-pyridine-2-yl)-Ph | $CH_2$ | nD 1.5263 (26.6℃) |
| D-99 | Et | H<br>$R^4$ = Ac | Cl | 3-OMe-4-O-(5-$CF_3$-pyridine-2-yl)-Ph | $CH_2$ | nD 1.5290 (26.6℃) |
| D-100 | Et | H<br>$R^4$ = EtCO | Cl | 3-OMe-4-O-(5-$CF_3$-pyridine-2-yl)-Ph | $CH_2$ | nD 1.5220 (27.1℃) |

Table D (cont'd)

| No. | $R^1$ | $R^2$ | $R^3$ | $R^5$ | Y | Property (℃) |
|---|---|---|---|---|---|---|
| D-101 | Et | H<br>$R^4$= n-PrCO | Cl | 3-OMe-4-O-(5-$CF_3$-pyridine-2-yl)-Ph | $CH_2$ | nD 1.5161 (27.2℃) |
| D-102 | Et | H<br>$R^4$= i-PrCO | Cl | 3-OMe-4-O-(5-$CF_3$-pyridine-2-yl)-Ph | $CH_2$ | nD 1.5361 (28.0℃) |
| D-103 | Et | H<br>$R^4$= t-BuCO | Cl | 3-OMe-4-O-(5-$CF_3$-pyridine-2-yl)-Ph | $CH_2$ | |
| D-104 | Et | H<br>$R^4$= PhCO | Cl | 3-OMe-4-O-(5-$CF_3$-pyridine-2-yl)-Ph | $CH_2$ | |
| D-105 | Et | H<br>$R^4$= $PhCH_2CO$ | Cl | 3-OMe-4-O-(5-$CF_3$-pyridine-2-yl)-Ph | $CH_2$ | |
| D-106 | Et | H<br>$R^4$= c-PrCO | Cl | 3-OMe-4-O-(5-$CF_3$-pyridine-2-yl)-Ph | $CH_2$ | nD 1.5379 (25.8℃) |
| D-107 | Et | H<br>$R^4$= MeCH=CHCO | Cl | 3-OMe-4-O-(5-$CF_3$-pyridine-2-yl)-Ph | $CH_2$ | amorphous |
| D-108 | Et | H<br>$R^4$= MeOOC | Cl | 3-OMe-4-O-(5-$CF_3$-pyridine-2-yl)-Ph | $CH_2$ | nD 1.5268 (26.6℃) |
| D-109 | Et | H<br>$R^4$= EtOOC | Cl | 3-OMe-4-O-(5-$CF_3$-pyridine-2-yl)-Ph | $CH_2$ | |
| D-110 | Et | H<br>$R^4$= n-PrOOC | Cl | 3-OMe-4-O-(5-$CF_3$-pyridine-2-yl)-Ph | $CH_2$ | |
| D-111 | Et | H<br>$R^4$= i-PrOOC | Cl | 3-OMe-4-O-(5-$CF_3$-pyridine-2-yl)-Ph | $CH_2$ | |

Table D (cont'd)

| No. | $R^1$ | $R^2$ | $R^3$ | $R^5$ | Y | Property (℃) |
|---|---|---|---|---|---|---|
| D-112 | Et | H<br>$R^4$= t-BuOOC | Cl | 3-OMe-4-O-(5-$CF_3$-pyridine-2-yl)-Ph | $CH_2$ | |
| D-113 | Et | H<br>$R^4$= PhOOC | Cl | 3-OMe-4-O-(5-$CF_3$-pyridine-2-yl)-Ph | $CH_2$ | |
| D-114 | Et | H<br>$R^4$= $PhCH_2OOC$ | Cl | 3-OMe-4-O-(5-$CF_3$-pyridine-2-yl)-Ph | $CH_2$ | |
| D-115 | Et | H<br>$R^4$= $MeSCH_2$ | Cl | 3-OMe-4-O-(5-$CF_3$-pyridine-2-yl)-Ph | $CH_2$ | |
| D-116 | Et | H<br>$R^4$= $CF_3CH_2$ | Cl | 3-OMe-4-O-(5-$CF_3$-pyridine-2-yl)-Ph | $CH_2$ | |
| D-117 | Et | H | F | 3-OMe-4-O-(5-$CF_3$-pyridine-2-yl)-Ph | $CH_2$ | |
| D-118 | Et | H | OH | 3-OMe-4-O-(5-$CF_3$-pyridine-2-yl)-Ph | $CH_2$ | |
| D-119 | Et | H | OMe | 3-OMe-4-O-(5-$CF_3$-pyridine-2-yl)-Ph | $CH_2$ | |
| D-120 | $CH_2$=$CH_2$ | H | Cl | 3-OMe-4-O-(5-$CF_3$-pyridine-2-yl)-Ph | $CH_2$ | 122-128 |
| D-121 | $NCCH_2$ | H | Cl | 3-OMe-4-O-(5-$CF_3$-pyridine-2-yl)-Ph | $CH_2$ | |
| D-122 | $OHCCH_2$ | H | Cl | 3-OMe-4-O-(5-$CF_3$-pyridine-2-yl)-Ph | $CH_2$ | |

Table D (cont'd)

| No. | $R^1$ | $R^2$ | $R^3$ | $R^5$ | Y | Property (℃) |
|---|---|---|---|---|---|---|
| D-123 | $MeON=CHCH_2$ | H | Cl | 3-OMe-4-O-(5-$CF_3$-pyridine-2-yl)-Ph | $CH_2$ | |
| D-124 | $EtOOCCH_2$ | H | Cl | 3-OMe-4-O-(5-$CF_3$-pyridine-2-yl)-Ph | $CH_2$ | |
| D-125 | $Me_2NOCCH_2$ | H | Cl | 3-OMe-4-O-(5-$CF_3$-pyridine-2-yl)-Ph | $CH_2$ | |
| D-126 | Et | H | Cl | 3-OMe-4-O-(5-$CF_3$-pyridine-2-yl)-Ph | C=O | nD 1.5735 (25.7℃) |
| D-127 | Et | H | Cl | 3-OMe-4-O-(5-$CF_3$-pyridine-2-yl)-Ph | $COCH_2$ | |
| D-128 | Et | H | Cl | 3-OMe-4-O-(5-$CF_3$-pyridine-2-yl)-Ph | C=NOMe | amorphous |
| D-129 | Et | H | Cl | 3-OMe-4-O-(5-$CF_3$-pyridine-2-yl)-Ph | $C(=NOMe)CH_2$ | |
| D-130 | Et | H | Cl | 3-OMe-4-O-(5-$CF_3$-pyridine-2-yl)-Ph | 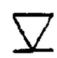 | |
| D-131 | Et | H | Cl | 3-OMe-4-O-(5-$CF_3$-pyridine-2-yl)-Ph | 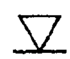 $CH_2$ | |
| D-132 | Et | H | Cl | 3-OMe-4-O-(5-$CF_3$-pyridine-2-yl)-Ph | CHOH | 49 |
| D-133 | Et | H | Cl | 3-OMe-4-O-(5-$CF_3$-pyridine-2-yl)-Ph | CHOMe | |

Table D (cont'd)

| No. | $R^1$ | $R^2$ | $R^3$ | $R^5$ | Y | Property (℃) |
|---|---|---|---|---|---|---|
| D-134 | Et | H | Cl | 3-OMe-4-O-(5-$CF_3$-pyridine-2-yl)-Ph | CHMe | |
| D-135 | Et | H | Cl | 3-OMe-4-O-(5-$CF_3$-pyridine-2-yl)-Ph | $CMe_2$ | |
| D-136 | Et | H | Cl | 3-OMe-4-O-(5-$CF(CF_3)_2$-pyridine-2-yl)-Ph | $CH_2$ | |
| D-137 | Et | H | Cl | 3-OMe-4-O-(5-$CH(CF_3)_2$-pyridine-2-yl)-Ph | $CH_2$ | |
| D-138 | Et | H | Cl | 3-OMe-4-O-(5-$CF_2CF_3$-pyridine-2-yl)-Ph | $CH_2$ | 56-60 |
| D-139 | Et | H | Cl | 3-OMe-4-O-(2-$CF_3$-pyridine-5-yl)-Ph | $CH_2$ | 133-136 |
| D-140 | Et | H | Cl | 3-OMe-4-O-(6-$CF(CF_3)_2$-pyridazine-3-yl)-Ph | $CH_2$ | |
| D-141 | Et | H | Cl | 3-OMe-4-O-(6-$CH(CF_3)_2$-pyridazine-3-yl)-Ph | $CH_2$ | |
| D-142 | Et | H | Cl | 3-OMe-4-O-(6-$CF_2CF_3$-pyridazine-3-yl)-Ph | $CH_2$ | |
| D-143 | Et | H | Cl | 3-OMe-4-O-(5-$CF_3$-pyrimidine-2-yl)-Ph | $CH_2$ | |
| D-144 | Et | H | Cl | 3-OMe-4-O-(5-$CF(CF_3)_2$-pyrimidine-2-yl)-Ph | $CH_2$ | |

Table D (cont'd)

| No. | $R^1$ | $R^2$ | $R^3$ | $R^5$ | Y | Property (℃) |
|---|---|---|---|---|---|---|
| D-145 | Et | H | Cl | 3-OMe-4-O-(5-$CH(CF_3)_2$-pyrimidine-2-yl)-Ph | $CH_2$ | |
| D-146 | Et | H | Cl | 3-OMe-4-O-(5-$CF_2CF_3$-pyrimidine-2-yl)-Ph | $CH_2$ | |
| D-147 | Et | H | Cl | 3-OMe-4-O-(5-$CF_3$-pyrazine-2-yl)-Ph | $CH_2$ | |
| D-148 | Et | H | Cl | 3-OMe-4-O-(4-Br-thiazole-2-yl)-Ph | $CH_2$ | |
| D-149 | Et | H | Cl | 3-OMe-4-O-(4-Cl-thiazole-2-yl)-Ph | $CH_2$ | |
| D-150 | Et | H | Cl | 3-OMe-4-O-(5-Cl-thiazole-2-yl)-Ph | $CH_2$ | |
| D-151 | Et | H | Cl | 3-OMe-4-O-(5-$NO_2$-thiazole-2-yl)-Ph | $CH_2$ | |
| D-152 | Et | H | Cl | 3-OMe-4-O-(5-Cl-thiophene-2-yl)-Ph | $CH_2$ | |
| D-153 | Et | H | Cl | 3-OMe-4-O-(5-Br-thiophene-2-yl)-Ph | $CH_2$ | |
| D-154 | Et | H | Cl | 3-OMe-4-O-(5-$CF_3$-thiophene-2-yl)-Ph | $CH_2$ | |
| D-155 | Et | H | Cl | 3-OMe-4-O-(5-$CF_3$-thiadiazole-2-yl)-Ph | $CH_2$ | |

Table D (cont'd)

| No. | $R^1$ | $R^2$ | $R^3$ | $R^5$ | Y | Property (℃) |
|---|---|---|---|---|---|---|
| D-156 | Et | H | Cl | 3-$NH_2$-4-O-(5-$CF_3$-pyridine-2-yl)-Ph | $CH_2$ | nD 1.5305 (25.6℃) |
| D-157 | Et | H | Cl | 3-NHMe-4-O-(5-$CF_3$-pyridine-2-yl)-Ph | $CH_2$ | nD 1.5201 (25.4℃) |
| D-158 | Et | H | Cl | 3-$NMe_2$-4-O-(5-$CF_3$-pyridine-2-yl)-Ph | $CH_2$ | |
| D-159 | Et | H | Cl | 3-NHAc-4-O-(5-$CF_3$-pyridine-2-yl)-Ph | $CH_2$ | |
| D-160 | Et | H | Cl | 3-$NHCOCF_3$-4-O-(5-$CF_3$-pyridine-2-yl)-Ph | $CH_2$ | |
| D-161 | Et | H | Cl | 3-$NHSO_2Me$-4-O-(5-$CF_3$-pyridine-2-yl)-Ph | $CH_2$ | |
| D-162 | Et | H | Cl | 3-$NHSO_2CF_3$-4-O-(5-$CF_3$-pyridine-2-yl)-Ph | $CH_2$ | |
| D-163 | Et | H | Cl | 3-SH-4-O-(5-$CF_3$-pyridine-2-yl)-Ph | $CH_2$ | |
| D-164 | Et | H | Cl | 3-SMe-4-O-(5-$CF_3$-pyridine-2-yl)-Ph | $CH_2$ | |
| D-165 | Et | H | Cl | 3-SOMe-4-O-(5-$CF_3$-pyridine-2-yl)-Ph | $CH_2$ | |
| D-166 | Et | H | Cl | 3-$SO_2Me$-4-O-(5-$CF_3$-pyridine-2-yl)-Ph | $CH_2$ | |

Table D (cont'd)

| No. | $R^1$ | $R^2$ | $R^3$ | $R^5$ | Y | Property (℃) |
|---|---|---|---|---|---|---|
| D-167 | Et | H | Cl | 3-$SCHF_2$-4-O-(5-$CF_3$-pyridine-2-yl)-Ph | $CH_2$ | |
| D-168 | Et | H | Cl | 3-$SOCHF_2$-4-O-(5-$CF_3$-pyridine-2-yl)-Ph | $CH_2$ | |
| D-169 | Et | H | Cl | 3-$SO_2CHF_2$-4-O-(5-$CF_3$-pyridine-2-yl)-Ph | $CH_2$ | |
| D-170 | Et | H | Cl | 3-OMe-4-O-(4-F-Ph)-Ph | C=O | |
| D-171 | Et | H | Cl | 3-OMe-4-O-(4-F-Ph)-Ph | $COCH_2$ | |
| D-172 | Et | H | Cl | 3-OMe-4-O-(4-F-Ph)-Ph | C=NOMe | |
| D-173 | Et | H | Cl | 3-OMe-4-O-(4-F-Ph)-Ph | C(=NOMe)$CH_2$ | |
| D-174 | Et | H | Cl | 3-OMe-4-O-(4-F-Ph)-Ph | 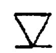 | |
| D-175 | Et | H | Cl | 3-OMe-4-O-(4-F-Ph)-Ph | 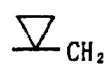 | |
| D-176 | Et | H | Cl | 3-OMe-4-O-(4-F-Ph)-Ph | CHOH | |
| D-177 | Et | H | Cl | 3-OMe-4-O-(4-F-Ph)-Ph | CHOMe | |
| D-178 | Et | H | Cl | 3-OMe-4-O-(4-F-Ph)-Ph | CHMe | |
| D-179 | Et | H | Cl | 3-OMe-4-O-(4-F-Ph)-Ph | $C(Me)_2$ | |
| D-180 | Et | H | Cl | 3-OMe-4-(6-Cl-benzothiazole-2-yl)-Ph | $CH_2$ | 142-145 |
| D-181 | Et | H | Cl | 3-OMe-4-(6-OMe-benzothiazole-2-yl)-Ph | $CH_2$ | 96-99 |
| D-182 | Et | H | Cl | 3-OMe-4-$OCH_2$(pyridine-2-yl)-Ph | $CH_2$ | 106-108 |

Table D (cont'd)

| No. | $R^1$ | $R^2$ | $R^3$ | $R^5$ | Y | Property (℃) |
|---|---|---|---|---|---|---|
| D-183 | Et | H | Cl | 3-OMe-4-$OCH_2CH_2$(pyridine-2-yl)-Ph | $CH_2$ | 90-96 |
| D-184 | Et | H | Cl | 3-OMe-4-O-(5-$CF_3$-pyridine-2-yl)-Ph | CH(OCH(Me)OEt) | nD 1.5389 (25.6℃) |
| D-185 | Me | H | Br | 3-OMe-4-O-(5-$CF_3$-pyridine-2-yl)-Ph | $CH_2$ | 129-134 |
| D-186 | Et | H | Cl | -4-O-(pyridine-2-yl)-Ph | $CH_2$ | 106-109 |
| D-187 | Et | H | Cl | -4-O-(pyridine-3-yl)-Ph | $CH_2$ | 97-99 |
| D-188 | Et | H $R^4$= CSNHMe | Cl | 3-OMe-4-O-(5-$CF_3$-pyridine-2-yl)-Ph | $CH_2$ | 145-147 |
| D-189 | Et | H $R^4$= CONHMe | Cl | 3-OMe-4-O-(5-$CF_3$-pyridine-2-yl)-Ph | $CH_2$ | nD 1.5369 (26.2℃) |
| D-190 | Et | H $R^4$= CHO | Cl | 3-OMe-4-O-(5-$CF_3$-pyridine-2-yl)-Ph | $CH_2$ | nD 1.5125 (25.5℃) |
| D-191 | Et | H $R^4$= Ac | Br | 3-OMe-4-O-(5-$CF_3$-pyridine-2-yl)-Ph | $CH_2$ | nD 1.5345 (26.4℃) |
| D-192 | Et | H | Br | 3-$NO_2$-4-O-(5-$CF_3$-pyridine-2-yl)-Ph | $CH_2$ | nD 1.5258 (25.6℃) |
| D-193 | Et | Cl | Cl | 3-OMe-4-O-(5-$CF_3$-pyridine-2-yl)-Ph | $CH_2$ | nD 1.5182 (25.0℃) |
| D-194 | Me | $CF_3$ | I | 3-OMe-4-O-(5-$CF_3$-pyridine-2-yl)-Ph | $CH_2$ | 178-180 |
| D-195 | Me | $CF_3$ | Br | 3-OMe-4-O-(5-$CF_3$-pyridine-2-yl)-Ph | $CH_2$ | 173 |
| D-196 | Et | H | Cl | 3-NHOH-4-O-(5-$CF_3$-pyridine-2-yl)-Ph | $CH_2$ | 218 |

The general formula (III)

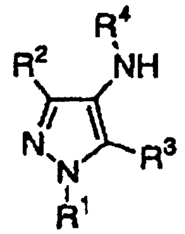

(III)

Table E

| No. | $R^1$ | $R^2$ | $R^3$ | $R^4$ | $^1$H-NMR($CDCl_3$/TMS,ppm) |
|---|---|---|---|---|---|
| E-1 | Me | Me | Cl | H | 2.16(3H. s), 2.60(2H. brs), 3.64(3H. s). |
| E-2 | Et | Me | Cl | H | 1.33(3H. t), 2.71(2H. brs). 3.64(3H, s), 4.02(2H. q). |
| E-3 | Me | H | Cl | H-HCl | 2.41(2H. brs), 3.80(3H. s), 7.68(1H. s), |
| E-4 | Et | H | Cl | H | 1.38(3H. t), 3.00(2H. brs), 4.10(2H. q), 7.20(1H. s). |
| E-5 | Et | H | Br | H | 1.38(3H. t), 2.94(2H. brs), 4.12(2H. q), 7.23(1H. s). |

Hereinbelow, representative examples for producing an N-(4-pyrazolyl)amide derivative according to the present invention will be illustrated. However, the present invention is not construed to be limitative thereto.

EXAMPLES

Example 1: Production of 5-chloro-4-(4-(4-cyanophenoxy) phenylacetoamino)-1,3-dimethylpyrazole (B-3)

4-Amino-5-chloro-1,3-dimethylpyrazole (0.20 g, 1.37 mmol), 4-(4-cyanophenoxy) phenylacetic acid (0.35 g, 1.37 mmol), 2-chloro-1-methylpyridinium iodide (0.38 g, 1.5 mmol), and triethylamine (0.15 g, 1.51 mmol) were dissolved in tetrahydrofuran (THF, 10 ml), and stirred at a room temperature for 2 hours. After the reaction was finished, the desired compound was extracted with ethyl acetate, and the organic layer was washed with 1N-hydrochloric acid aqueous solution, saturated sodium bicarbonate aqueous solution, and saturated saline in this order, followed by drying with anhydrous magnesium sulfate. The solvent was distilled off under a reduced pressure. Crystallization from a mixture of n-hexane and ether gave 0.27 g of the desired compound. The yield was 52%.

Example 2: Production of 5-chloro-4-(4-(4-trifluoromethylphenyl)phenylacetoamino)-1-methylpyrazole (B-65)

A hydrochloric acid salt of 4-amino-5-chloro-1- methylpyrazole (0.24 g, 1.4 mmol), 4-(4-trifluoromethylphenyl) phenylacetic acid (0.39 g, 1.4 mmol), 2-chloro-1-methylpyridinium iodide (0.39 g, 1.54 mmol), and triethylamine (0.3 g, 3.0 mmol) were dissolved in THF (20 ml), and reacted under reflux for 4 hours. After being left to stand, the reaction solution was poured into water. The desired compound was extracted with ethyl acetate, and the organic layer was washed with saturated sodium bicarbonate aqueous solution, saturated saline, 1N-hydrochloric acid aqueous solution, and saturated saline in this order, followed by drying with anhydrous sodium sulfate. The solvent was distilled off under a reduced pressure. The crystal obtained was suspended in a mixture of n-hexane and methyl butyl ether, then filtered to obtain 0.23 g of the desired compound. The yield was 42%.

Example 3: Production of 5-chloro-1,3-dimethyl-4-(2-chlorophenylacetoamino)pyrazole (A-5)

4-Amino-5-chloro-1,3-dimethylpyrazole (0.14 g, 0.96 mmol) and triethylamine (0.10 g, 0.96 mmol) were dissolved in THF (25 ml), and thereto 2-chloro-phenylacetyl chloride (10.18 g, 0.96 mmol) was added. The solution was

reacted at room temperature for 24 hours, then the reaction solution was poured into water. The desired compound was extracted with ethyl acetate, and the organic layer was washed with 1N-hydrochloric acid aqueous solution, saturated sodium bicarbonate aqueous solution, and saturated saline in this order, followed by drying with anhydrous sodium sulfate. The solvent was distilled off under a reduced pressure. A crystal obtained was suspended in a mixture of acetone and ether, then filtered to obtain 0.11 g of the desired compound. The yield was 37%.

Example 4: Production of 5-chloro-4-(3-methoxy-4-(5-trifluoromethylpyridine-2-yloxy)phenylacetylamino)-1-ethylpyrazole (D-33)

A hydrochloric acid salt of 4-amino-5-chloro-1- ethylpyrazole (0.20 g, 1.10 mmol), 3-methoxy-4-(5-trifluoromethylpyridine-2-yloxy) phenylacetic acid (0.36 g, 1.10 mmol), 2-chloro-1-methylpyridinium iodide (0.62 g, 2.43 mmol), and triethylamine (0.34 g, 3.3 mmol) were dissolved in THF (30 ml), and stirred while heating for 2 hours. After the reaction was finished, the desired compound was extracted with ethyl acetate, and the organic layer was washed with 1N-hydrochloric acid aqueous solution, saturated sodium bicarbonate aqueous solution, and saturated saline in this order, followed by drying with anhydrous magnesium sulfate. The solvent was distilled off under a reduced pressure. The residue was purified by a column chromatography (n-hexane / ethyl acetate = 1/3) to obtain 0.28 g of the desired compound. The yield was 56%.

Example 5: Production of 5-chloro-4-(3-difluoromethoxy-4-(5-trifluoromethylpyridine-2-yloxy)phenylacetylamino)-1-ethylpyrazole (D-86)

4-Amino-5-chloro-1-ethylpyrazole (0.30 g, 2.08 mmol), 3-difluoromethoxy-4-(5-trifluoromethylpyridine-2-yloxy)phenylacetic acid (0.75 g, 2.08 mmol), 2-chloro-1-methyl- pyridinium iodide (1.17 g, 4.58 mmol), and triethylamine (0.63 g, 6.24 mmol) were dissolved in THF (30 ml), and stirred while heating for 2 hours. After the reaction was finished, the desired compound was extracted with ethyl acetate, and the organic layer was washed with 1N-hydrochloric acid aqueous solution, saturated sodium bicarbonate aqueous solution, and saturated saline in this order, followed by drying with anhydrous magnesium sulfate. The solvent was distilled off under a reduced pressure. The residue was purified by a column chromatography (n-hexane / ethyl acetate = 1/1) to obtain 0.57 g of the desired compound. The yield was 56%.

Example 6: Production of 5-bromo-4-(3-methoxy-4-(5-trifluoromethylpyridine-2-yloxy)phenylacetylamino)-1-ethylpyrazole (D-79)

4-Amino-5-bromo-1-ethylpyrazole (0.36 g, 1.90 mmol), 3-methoxy-4-(5-trifluoromethylpyridine-2-yloxy)phenylacetic acid (0.62 g, 1.90 mmol), 2-chloro-1-methylpyridinium iodide (1.07 g, 4.18 mmol), and triethylamine (0.58 g, 5.7 mmol) were dissolved in THF (30 ml), and stirred while heating for 2 hours. After the reaction was finished, the desired compound was extracted with ethyl acetate, and the organic layer was washed with 1N-hydrochloric acid aqueous solution, saturated sodium bicarbonate aqueous solution, and saturated saline in this order, followed by drying with anhydrous magnesium sulfate. The solvent was distilled off under a reduced pressure. The residue was purified by a column chromatography (n-hexane / ethyl acetate = 1/3) to obtain 0.86 g of the desired compound. The yield was 91%.

Example 7: Production of 5-chloro-4-(3-methoxy-4-(5-trifluoromethylpyridine-2-yloxy)phenylacetylamino)-1-(2-chloroethyl) pyrazole (D-71)

4-Amino-5-chloro-1-(2-chloroethyl)pyrazole (0.56 g, 3.13 mmol), 3-methoxy-4-(5-trifluoromethylpyridine-2-yloxy) phenylacetic acid (1.02 g, 3.13 mmol), 2-chloro-1-methylpyridinium iodide (1.76 g, 6.89 mmol), and triethylamine (0.95 g, 9.39 mmol) were dissolved in THF (30 ml), and stirred while heating for 2 hours. After the reaction was finished, the desired compound was extracted with ethyl acetate, and the organic layer was washed with 1N-hydrochloric acid aqueous solution, saturated sodium bicarbonate aqueous solution, and saturated saline in this order, followed by drying with anhydrous magnesium sulfate. The solvent was distilled off under a reduced pressure. The residue was purified by a column chromatography (n-hexane / ethyl acetate = 1/1) to obtain 0.57 g of the desired compound. The yield was 37%.

Example 8: Production of 5-chloro-4-(3-methoxy-4-(6-trifluoromethylpyridazine-3-yloxy)phenylacetylamino)-1-ethylpyrazole (D-78)

4-Amino-5-chloro-1-ethylpyrazole (0.13 g, 0.91 mmol), 3-methoxy-4-(6-trifluoromethylpyridazine-3-yloxy)phenylacetic acid (0.30 g, 0.91 mmol), 2-chloro-1-methyl- pyridinium iodide (0.28 g, 1.09 mmol), and triethylamine (0.11 g, 1.09 mmol) were dissolved in THF (20 ml), and stirred while heating for 1 hour. After the reaction was finished, the desired compound was extracted with ethyl acetate, and the organic layer was washed with 1N-hydrochloric acid aqueous solution, saturated sodium bicarbonate aqueous solution, and saturated saline in this order, followed by drying with anhydrous magnesium sulfate. The solvent was distilled off under a reduced pressure. The residue was dissolved in ethyl acetate, and thereto an activated carbon was added. The solution was stirred while heating for 1 hour. After filtration, the solvent was distilled off under a reduced pressure.
Crystallization from a mixture of n-hexane and ether gave 0.23 g of the desired compound. The yield was 57%.

Example 9: Production of 5-chloro-4-(3-methoxy-4-(4-trifluoromethylthiazole-2-yloxy)phenylacetylamino)-1-ethylpyrazole (D-94)

A hydrochloric acid salt of 4-amino-5-chloro-1-ethyl- pyrazole (0.27 g, 1.50 mmol), 3-methoxy-4-(4-trifluoromethylthiazole-2-yloxy)phenylacetic acid (0.50 g, 1.50 mmol), 2-chloro-1-methylpyridinium iodide (0.46 g, 1.80 mmol), and triethylamine (0.38 g, 3.75 mmol) were dissolved in THF (30 ml), and stirred while heating for 2 hours. After the reaction was finished, the desired compound was extracted with ethyl acetate, and the organic layer was washed with 1N-hydrochloric acid aqueous solution, saturated sodium bicarbonate aqueous solution, and saturated saline in this order, followed by drying with anhydrous magnesium sulfate. The solvent was distilled off under a reduced pressure. The residue was purified by a column chromatography (n-hexane / ethyl acetate = 1/1) to obtain 0.12 g of the desired compound. The yield was 18%.

Example 10: Production of 5-chloro-4-(3-methoxy-4-(4-fluorophenyloxy)phenylacetylamino)-1-methylpyrazole (B-103)

4-Amino-5-chloro-1-methylpyrazole (0.10 g, 0.76 mmol), 3-methoxy-4-(4-fluorophenyloxy) phenylacetic acid (0.21 g, 0.76 mmol), 2-chloro-1-methylpyridinium iodide (0.23 g, 0.91 mmol), and triethylamine (0.1 g, 0.91 mmol) were dissolved in THF (20 ml), and stirred while heating for 2 hours. After the reaction was finished, the desired compound was extracted with ethyl acetate, and the organic layer was washed with 1N-hydrochloric acid aqueous solution, saturated sodium bicarbonate aqueous solution, and saturated saline in this order, followed by drying with anhydrous magnesium sulfate. The solvent was distilled off under a reduced pressure. The residue was dissolved in ethyl acetate, and thereto an activated carbon was added. The solution was stirred while heating for 1 hour. After filtration, the solvent was distilled off under a reduced pressure.
Crystallization from a mixture of n-hexane and ether gave 0.20 g of the desired compound. The yield was 65%.

Example 11: Production of 5-chloro-4-(3-methoxy-4-(4-fluorophenyloxy)phenylacetylamino)-1-ethylpyrazole (B-117)

4-Amino-5-chloro-1-ethylpyrazole (0.20 g, 1.09 mmol), 3-methoxy-4-(4-fluorophenyloxy) phenylacetic acid (0.30 g, 1.09 mmol), 2-chloro-1-methylpyridinium iodide (0.33 g, 1.31 mmol), and triethylamine (0.28 g, 2.73 mmol) were dissolved in THF (20 ml), and stirred while heating for 2 hours. After the reaction was finished, the desired compound was extracted with ethyl acetate, and the organic layer was washed with IN-hydrochloric acid aqueous solution, saturated sodium bicarbonate aqueous solution, and saturated saline in this order, followed by drying with anhydrous magnesium sulfate. The solvent was distilled off under a reduced pressure. The residue was dissolved in ethyl acetate, and thereto an activated carbon was added. The solution was stirred while heating for 1 hour. After filtration, the solvent was distilled off under a reduced pressure.
Crystallization from a mixture of n-hexane and ether gave 0.30 g of the desired compound. The yield was 68%.

Example 12: Production of 5-chloro-4-(4-(4-trifluoromethoxyphenyl)phenylacetylamino)-1,3-dimethylpyrazole (B-23)

4-Amino-5-chloro-1, 3-dimethylpyrazole (0.14 g, 0.75 mmol), 3-methoxy-4-(4-trifluoromethoxyphenyloxy) phenylacetic acid (0.27 g, 0.75 mmol), diethyl cyanophosphate (0.16 g, 1.00 mmol), and triethylamine (0.15 g, 1.50 mmol) were dissolved in THF (20 ml), and were stirred at room temperature for 10 hours. After the reaction was finished, the desired compound was extracted with ethyl acetate, and the organic layer was washed with 1N-hydrochloric acid aqueous solution, saturated sodium bicarbonate aqueous solution, and saturated saline in this order, followed by drying with anhydrous magnesium sulfate. The solvent was distilled off under a reduced pressure. The residue was dissolved in ethyl acetate, and thereto an activated carbon was added. The solution was stirred while heating for 1 hour. After filtration, the solvent was distilled off under a reduced pressure. Crystallization from a mixture of n-hexane and ether gave 0.14 g of the desired compound. The yield was 37%.

Example 13: Production of 5-chloro-4-(2-(2,2-dimethylpropyl) benzoxazole-5-ylacetylamino)-1,3-dimethylpyrazole (C-1)

A hydrochloric acid salt of 4-amino-5-chloro-1,3-dimethylpyrazole (0.22 g, 1.21 mmol), 2-(2,2-dimethylpropyl) benzoxazole-5-ylphenylacetic acid chloride (0.32 g, 1.21 mmol), and triethylamine (0.37 g, 3.63 mmol) were dissolved in THF (30 ml), and stirred at a room temperature for 10 hours. After the reaction was finished, the desired compound was extracted with ethyl acetate, and the organic layer was washed with 1N-hydrochloric acid aqueous solution, saturated sodium bicarbonate aqueous solution, and saturated saline in this order, followed by drying with anhydrous magnesium sulfate. The solvent was distilled off under a reduced pressure. The residue was purified by a column chromatography (n-hexane / ethyl acetate = 1/5) to obtain 0.05 g of the desired compound. The yield was 10%.

Example 14: Production of 5-chloro-4-(N-ethyl-N-(3-methoxy-4-(5-trifluoromethylpyridine-2-yloxy)phenylacetyl) amino)-1-ethylpyrazole (D-67)

5-Chloro-4-(3-methoxy-4-(5-trifluoromethylpyridine-2-yloxy) phenylacetylamino)-1-ethylpyrazole (D-33) (0.50 g, 1.10 mmol) was dissolved in methylene chloride (15 ml), and ethyl iodide (0.43 g, 2.76 mmol), benzyltriethylammonium chloride (0.015 g, 0.066 mmol), and a 50 % aqueous solution of sodium hydroxide (0.44 ml) were added thereto. The solution was stirred while heating for 10 hours. After the reaction was finished, the desired compound was extracted with ethyl acetate, and the organic layer was washed with 1N-hydrochloric acid aqueous solution, saturated sodium bicarbonate aqueous solution, and saturated saline in this order, followed by drying with anhydrous magnesium sulfate. The solvent was distilled off under a reduced pressure. The residue was purified by a column chromatography (n-hexane / ethyl acetate = 1/1) to obtain 0.35 g of the desired compound. The yield was 66%.

Example 15: Production of 5-chloro-4-(N-ethoxycarbonylmethyl-N-(3-methoxy-4-(5-trifluoromethylpyridine-2-yloxy) phenylacetyl)amino)-1-ethylpyrazole (D-98)

5-Chloro-4-(3-methoxy-4-(5-trifluoromethylpyridine-2-yloxy) phenylacetylamino)-1-ethylpyrazole (D-33) (0.80 g, 1.76 mmol) was dissolved in DMF (40 ml), and ethyl chloroacetate (0.45 g, 3.67 mmol) and potassium carbonate (0.80 g, 5.79 mmol) were added thereto. The solution was stirred while heating for 5 hours. After the reaction was finished, the reaction solution was poured into water, and then the desired compound was extracted with ethyl acetate. The organic layer was washed with 1N-hydrochloric acid aqueous solution, saturated sodium bicarbonate aqueous solution, and saturated saline in this order, followed by drying with anhydrous magnesium sulfate. The solvent was distilled off under a reduced pressure. The residue was purified by a column chromatography (n-hexane / ethyl acetate = 2/1) to obtain 0.41 g of the desired compound. The yield was 43%.

Example 16: Production of 5-chloro-4-(N-ethoxymethyl-N-(3-methoxy-4-(4-fluorophenyloxy)phenylacetyl)amino)-1-methyl- pyrazole (B-123)

5-Chloro-4-(3-methoxy-4-(4-fluorophenyloxy)-phenylacetylamino)-1-methylpyrazole (B-103, 0.15 g, 0.38 mmol) was dissolved in methylene chloride (5 ml), and ethoxymethyl chloride (0.09 g, 0.96 mmol), benzyltriethylammonium chloride (0.08 g, 0.03 mmol), and a 50 % aqueous solution of sodium hydroxide (0.15 ml) were added thereto. The solution was stirred at room temperature for one hour. After the reaction was finished, the desired compound was extracted with ethyl acetate, and the organic layer was washed with 1N-hydrochloric acid aqueous solution, saturated sodium bicarbonate aqueous solution, and saturated saline in this order, followed by drying with anhydrous magnesium sulfate. The solvent was distilled off under a reduced pressure. The residue was purified by a column chromatography (n-hexane / ethyl acetate = 1/1) to obtain 0.09 g of the desired compound. The yield was 52%.

Typical formulations and test examples of the present invention are described below but they should not be construed as limiting the scope of the invention.

As used in the examples, the terms "part" and "parts" are by weight.

| Formulation Example 1 | |
|---|---|
| Compound of the present invention | 50 parts |
| Xylene | 40 parts |
| Mixture of polyoxyethylene nonylphenyl | 10 parts |

ether and calcium alkylbenzenesulfonate

An emulsifiable concentrate was prepared by mixing uniformly the above ingredients to effect dissolution.

| Formulation Example 2 | |
|---|---|
| Compound of the present invention | 3 parts |
| Clay powder | 82 parts |
| Diatomaceous earth powder | 15 parts |

A dust was prepared by mixing uniformly and grinding the above ingredients.

| Formulation Example 3 | |
|---|---|
| Compound of the present invention | 5 parts |
| Mixed powder of bentonite and clay | 90 parts |
| Calcium ligninsulfonate | 5 parts |

Granules were prepared by mixing the above ingredients uniformly, and kneading the resulting mixture together with a suitable amount of water, followed by granulation and drying.

| Formulation Example 4 | |
|---|---|
| Compound of the present invention | 20 parts |
| Mixture of kaolin and synthetic kaoline and high-dispersion silicic acid | 75 parts |
| Mixture of polyoxyethylene nonylphenyl ether and calcium alkylbenzenesulfonate | 5 parts |

A wettable powder was prepared by mixing uniformly and grinding the above ingredients.

Test Example 1: Control effect on apple scab (Venturia inaequalis)

The agent in accordance with the formulations mentioned above was prepared and diluted with water in the prescribed concentration to obtain a test solution. The stalks and leaves of the potted apple seedling (cultivar: Ohrin) were sprayed with the test solution. After one day, they were inoculated with the suspension of conidia spores of apple scab (Venturia inaequalis) by spraying. Two weeks after the inoculation, the lesion area of each leaf was measured and then compared with that on the untreated plot, whereby the controlling effect was judged according to the following criterion.
Criterion for judgment:

A: control efficacy 100 to 90%
B: control efficacy 89 to 80%
C: control efficacy 79 to 50%
D: control efficacy 49 to 0%

The results of the test shown below are indicated as Table-compound No. As a result of the Test Example 1, the following compounds had an activity rated A: A-1 to A-5, A-7 to A-10, A-13, A-14, A-20, A-22 to A-25, A-27, A-30, A-31, A-33 to A-40, A-43 to A-49, A-52, A-53, A-54, A-55, A-56, A-57, B-1, B-3, B-5, B-7 to B-12, B-16 to B-22, B-24, B-26, B-27, B-29, B-30, B-38, B-40, B-47, B-49, B-53, B-56, B-64 to B-69, B-74, B-76, B-77, B-80 to B-87, B-89, B-91, B-93, B-94, B-97 to B-100, B-102 to B-104, B-106, B-107, B-110, B-113, B-114, B-117 to B-120, B-123 to B-125, B-186, D-3 to D-6, D-10, D-13, D-14, D-16, D-18, D-21 to D-23, D-27 to D-31, D-33, D-34, D-38, D-41, D-42, D-45 to D-47, D-53, D-54, D-57, D-58, D-60, D-61, D-68, D-71, D-73 to D-76, D-82 to D-88, D-92, D-94, D-98, D-99, D-101, D-102, D-106, D-108, D-126, D-128, D-132, D-139, D-181, D-184, D-185, D-186, D-187, D-189 and D-190.

Test Example 2: Control effect on cucumber downy mildew (Pseudoperonospora cubensis)

The agent in accordance with the formulations mentioned above was prepared and diluted with water in the prescribed concentration to obtain a test solution. The stalks and leaves of the potted cucumber at the 1.5 leaf stage (cultivar: Suyo) were sprayed with the test solution. After one day, they were inoculated with zoospore of cucumber

downy mildew (Pseudoperonospora cubensis) by spraying. Seven days after the inoculation, the lesion area of each leaf was measured and then compared with that on the untreated plot, whereby the controlling effect was judged according to the criterion shown in Test Example 1.

As a result of the following compounds had an activity rated A: the compounds Nos. A-3 to A-10, A-13, A-14, A-16, A-19 to A-25, A-27, A-28, A-30, A-31, A-33, A-35, A-36, A-40 to A-49, A-51, A-53, B-1 to B-11, B-13, B-18 to B-20, B-22, B-24, B-26 to B-31, B-38 to B-40, B-42 to B-45, B-47 to B-51, B-53, B-56, B-58, B-60 to B-69, B-71, B-72, B-74, B-76 to B-119, B-123 to B-125, B-183, B-184, B-186, C-1, C-2, D-1 to D-6, D-12 to D-16, D-18, D-19, D-22 to D-36, D-38 to D-42, D-44 to D-53, D-55 to D-60, D-62 to D-74, D-77 to D-79, D-82, D-84 to D-92, D-94, D-96 to D-98, D-100, D-101, D-102, D-106, D-107, D-108, D-120, D-126, D-128, D-132, D-138, D-180, D-181, D-185, D-186, D-190, D-191 and D-192.

Test Example 3: Controlling effect on Chinese cabbage black spot (Alternaria brassicae)

The agent in accordance with the formulations mentioned above was prepared and diluted with water in the prescribed concentration to obtain a test solution. The stalks and leaves of the potted Chinese cabbage at the 1.5 leaf stage (cultivar: Muso) were sprayed with the test solution. After one day, they were inoculated with the suspension of conidia spores of Chinese cabbage black spot (Alternaria brassicae) by spraying. Seven days after the inoculation, the lesion area of each leaf was measured and then compared with that on the untreated plot, whereby the controlling effect was judged according to the criterion shown in Test Example 1.

As a result of the following compounds had an activity rated A: the compounds Nos. A-5, A-7 to A-9, A-20, A-33, A-35 to A-40, A-43 to A-47, A-49, A-53, B-3, B-6 to B-8, B-19, B-22, B-25, to B-27, B-39, B-40, B-42, B-44, B-47, B-49, B-50, B-53, B-54, B-56, B-61, B-63, B-70, B-71, B-79 to B-84, B-92, B-93, B-102, B-103, B-106, B-108, B-111, B-114, B-117, B-118, B-121, C-2, D-1 to D-3, D-9, D-14 to D-16, D-18, D-21, D-22, D-24, D-25, D-33 to D-37, D-39, D-41, D-44 to D-53, D-48, D-49, D-51, D-53, D-57, D-58, D-84 to D-87, D-94, D-100, D-102, D-106, D-108, D-139, D-186 and D-192.

Test Example 4: Controlling effect on tomato late blight (Phytophthora infestans)

The agent in accordance with the formulations mentioned above was prepared and diluted with water in the prescribed concentration to obtain a test solution. The stalks and leaves of the potted tomato at the 3 leaf stage (cultivar: Ponterosa) were sprayed with the test solution. After one day, they were inoculated with the suspension of zoospores of tomato late blight (Phytophthora infestans) by spraying. Five days after the inoculation, the lesion area of each leaf was measured and then compared with that on the untreated plot, whereby the controlling effect was judged according to the criterion shown in Test Example 1.

As a result of the following compounds had an activity rated A: the compounds Nos. A-3, A-8, A-16, A-24, A-25, A-41 to A-43, A-55, A-56, B-3, B-10, B-36, B-38, B-56 to B-59, B-62, B-70, B-72, B-78, B-99, B-100, B-111 to B-114, B-119, C-1, C-2, D-4, D-14, D-15, D-21, D-30 to D-32, D-38, D-39, D-56, D-58, D-59, D-132, D-184, D-185 and D-186.

Test Example 5: Controlling effect on barley powdery mildew

The agent in accordance with the formulations mentioned above was prepared and diluted with water in the prescribed concentration to obtain a test solution. The stalks and leaves of the potted barley plants at the 1.5 leaf stage (cultivar: Kanto No. 6) were sprayed with the test solution. After one day, they were inoculated with conidia spores of powdery mildew fungus (Erysiphe graminis hordei) by sprinkling. Seven days after the inoculation, the lesion area of each leaf was measured and then compared with that on the untreated plot, whereby the controlling effect was judged according to the criterion shown in Test Example 1.

As a result of the following compounds had an activity rated A: the compounds Nos. A-2, A-7, A-9, A-22, A-48, A-55, B-1, B-3, B-20, B-41, B-62, B-63, B-67, B-72, B-73, B-89, B-91, B-94, B-99, B-102, B-103, B-105, B-108, B-110, B-111, B-116, B-117, B-123 to B-125, B-186, D-1, D-18, D-28, D-32, D-33, D-38, D-40, D-47, D-48, D-51, D-56 to D-60, D-62 to D-65, D-67 to D-69, D-71 to D-74, D-77 to D-79, D-81, D-82, D-85 to D-95, D-99, D-100, D-101, D-102, D-106, D-107, D-108, D-126, D-128, D-132, D-138, D-139, D-180, D-181, D-183, D-184, D-185, D-186, D-190 and D-191.

Test Example 6: Insecticidal effect on diamond back moth (Plutella xylostella)

Adult diamond back moths were released and allowed to oviposit on a Chinese cabbage seedling. Two days after the release, the seedling having the eggs deposited thereon was immersed for about 30 seconds in a test solution

prepared by diluting an agent according to the formulations mentioned above into the concentration of 500 ppm. After air-dryness, it was allowed to stand in a room thermostatted at 25°C. Six days after the immersion, the hatched insects were counted. The mortality was calculated according to the following equation and the insecticidal effect was judged according to the criterion shown below. The test was carried out with triplicate groups of 10 insects.

$$\text{Corrected mortality(\%)} = \frac{\text{Number of hatched insects in untreated group - Number of hatched insects in treated group}}{\text{Number of hatched insects in untreated group}} \times 100$$

Criterion:

A --- Mortality 100%
B --- Mortality 99-90%
C --- Mortality 89-80%
D --- Mortality 79-50%

As a result, the following compounds had an activity rated A: the compound Nos. A-9, A-19, A-21, A-23, A-25, A-32, A-35, B-3 to B-5, B-11, B-18 to B-21, B-30, B-53, B-54, B-58, B-61 to B-63, B-67, B-75, B-76, B-78, B-84, B-90, B-93, B-96 to B-99, B-102, B-105, B-110, B-113, B-117 to B-119, B-121, B-124, C-2, D-6, D-8, D-12, D-14, D-15, D-26, D-29, D-31, D-33, D-38 to D-46, D-48, D-52, D-53, D-55, D-57, D-61, D-68, D-69, D-72, D-78 to D-81, D-86, D-89, D-91, D-93, D-95, D-99, D-100, D-132, D-139, D-185 and D-187.

Test Example 7: Insecticidal effect on green peach aphid (Myzus persicae)

A Chinese cabbage plant was planted in each of plastic pots with a diameter of 8 cm and a height of 8 cm, and green peach aphids were propagated on the plant. The number of parasites of each pot was counted.
The agent in accordance with the formulations mentioned above was dispersed in water and diluted into a test solution having a concentration of 500 ppm. Then, the stems and leaves were sufficiently sprayed with the test solution. After air-drying, the pots were allowed to stand in a greenhouse. Six days after the spraying, green peach aphids parasitic on each Chinese cabbage plant were counted and the control efficacy was calculated, whereby the insecticidal effect was judged according to the criterion shown in Test Example 6.

$$\text{Control efficacy (\%)} = 100 - [(T \times Ca)/(Ta \times C)] \times 100$$

Ta: number of parasites before spraying in treated group,
T : number of parasites after spraying in treated group,
Ca: number of parasites before spraying in untreated group,
T : number of parasites after spraying in untreated group.

As a result, the following compounds were rated A: the compound Nos. A-5, A-8, A-9, A-35, A-36, A-56, B-1, B-4, B-9, B-10, B-20, B-23 to B-25, B-58, B-62, B-64, B-69, B-80, B-84, B-87, B-99, B-100, B-102, B-110 to B-115, B-118, B-125, C-1, D-8, D-33, D-40, D-41, D-45 to D-48, D-53, D-54, D-56, D-57, D-60, D-62, D-71 to D-73, D-77 to D-80, D-82, D-91 to D-94, D-97 to D-99, D-100, D-101, D-108, D-126, D-132, D-139, D-184, D-185, D-186 and D-187.

Test Example 8: Insecticidal effect on brown rice planthopper (Nilaparvata lugens)

The agent in accordance with the formulations mentioned above was dispersed in water and diluted into a test solution having a concentration of 500 ppm. Rice seedlings (variety: Nihombare) were dipped in the test solution for 30 seconds. After air-dryness, each seedling was introduced into a glass-made test tube, and inoculated with ten 3rd inster nimphs of brown rice planthopper. Then, the test tube was stoppered with cotton. Eight days after the treatment, the numbers of dead or alive insects were counted, and the insect death rate was calculated by the following equation. The insecticidal effect was judged according to the criterion shown in Test example 6.

The insect death rate (%)=(The number of dead insects/

The number of inoculated insects) × 100

As a result, the following compounds had an activity rated A: the compound Nos. B-23, B-62, B-63, B-67, B-78, B-84, B-88, B-90, B-93, B-98 to B-102, B-108, B-111 to B-121, B-124, B-125, D-22, D-33, D-40, D-45 to D-48, D-51, D-55 to D-57, D-60, D-63, D-65, D-67 to D-69, D-71, D-77, D-79, D-82, D-83, D-86, D-89, D-91, D-99, D-100, D-101, D-108, D-126 and D-184.

## Claims

**1.** An N-(4-pyrazolyl)amide derivative represented by the general formula (I):

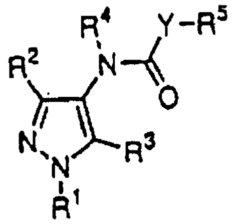

(I)

wherein $R^1$ represents a hydrogen atom; a $(C_1-C_6)$alkyl group; a halo$(C_1-C_6)$alkyl group; a hydroxy $(C_1-C_6)$alkyl group; a cyano$(C_1-C_6)$alkyl group; a formyl$(C_1-C_6)$alkyl group; a $(C_2-C_6)$alkenyl group; a halo$(C_2-C_6)$alkenyl group; a $(C_2-C_6)$alkynyl group; a halo$(C_2-C_6)$alkynyl group; a $(C_1-C_6)$alkoxy$(C_1-C_6)$alkyl group; a halo$(C_1-C_6)$alkoxy $(C_1-C_6)$alkyl group; a $(C_1-C_6)$alkylthio$(C_1-C_6)$-alkyl group; a halo$(C_1-C_6)$alkylthio$(C_1-C_6)$alkyl group; a $(C_1-C_6)$alkylsulfinyl$(C_1-C_6)$alkyl group; a halo$(C_1-C_6)$-alkylsulfinyl$(C_1-C_6)$alkyl group; a $(C_1-C_6)$alkylsulfonyl-$(C_1-C_6)$alkyl group; a halo$(C_1-C_6)$alkylsulfonyl$(C_1-C_6)$-alkyl group; a mono$(C_1-C_6)$alkylamino$(C_1-C_6)$alkyl group; a di$(C_1-C_6)$alkylamino $(C_1-C_6)$alkyl group whose $(C_1-C_6)$alkyl groups may be the same or different; a $(C_1-C_6)$alkylsulfonyl group; a halo $(C_1-C_6)$alkylsulfonyl group; a mono$(C_1-C_6)$alkylaminosulfonyl group; a di$(C_1-C_6)$alkylaminosulfonyl group whose $(C_1-C_6)$alkyl groups may be the same or different; a $(C_1-C_6)$alkyloxycarbonyl$(C_1-C_6)$alkyl group; a mono$(C_1-C_6)$ alkylaminocarbonyl$(C_1-C_6)$alkyl group; a di $(C_1-C_6)$alkylaminocarbonyl$(C_1-C_6)$alkyl group whose $(C_1-C_6)$alkyl groups may be the same or different; a $(C_1-C_6)$alkyloxyimino$(C_1-C_6)$alkyl group; a $(C_1-C_6)$alkylsulfonyloxy$(C_1-C_6)$ alkyl group; a halo$(C_1-C_6)$alkylsulfonyloxy$(C_1-C_6)$alkyl group; a $(C_1-C_6)$alkylcarbonyloxy $(C_1-C_6)$alkyl group; a phenylsulfonyl group; a substituted phenylsulfonyl group having at least one substituent which may be the same or different and is selected from the group consisting of a halogen atom, a cyano group, a nitro group, a hydroxyl group, an amino group, a mercapto group, a $(C_1-C_6)$alkyl group, a halo$(C_1-C_6)$alkyl group, a $(C_2-C_6)$alkenyl group, a halo$(C_2-C_6)$alkenyl group, a $(C_2-C_6)$alkynyl group, a halo$(C_2-C_6)$alkynyl group, a $(C_1-C_6)$alkoxyl group, a halo $(C_1-C_6)$alkoxyl group, a $(C_1-C_6)$alkylthio group, a halo$(C_1-C_6)$alkylthio group, a $(C_1-C_6)$alkylsulfinyl group, a halo $(C_1-C_6)$alkylsulfinyl group, a $(C_1-C_6)$alkylsulfonyl group, a halo$(C_1-C_6)$alkylsulfonyl group, a mono-$(C_1-C_6)$alkylamino group, a di$(C_1-C_6)$alkylamino group whose $(C_1-C_6)$alkyl groups may be the same or different, a $(C_1-C_6)$alkylsulfonylamino group, and a halo$(C_1-C_6)$-alkylsulfonylamino group; a $(C_3-C_6)$cycloalkyl group; a halo$(C_3-C_6)$cycloalkyl group; a $(C_3-C_6)$cycloalkenyl group; a halo$(C_3-C_6)$cycloalkenyl group; a phenyl group; a substituted phenyl group having at least one substituent which may be the same or different and is selected from the group consisting of a halogen atom, a cyano group, a nitro group, a hydroxyl group, an amino group, a mercapto group, a $(C_1-C_6)$ alkyl group, a halo$(C_1-C_6)$alkyl group, a $(C_2-C_6)$alkenyl group, a halo$(C_2-C_6)$alkenyl group, a $(C_2-C_6)$alkynyl group, a halo$(C_2-C_6)$alkynyl group, a $(C_1-C_6)$alkoxyl group, a halo$(C_1-C_6)$alkoxyl group, a $(C_1-C_6)$alkylthio group, a halo $(C_1-C_6)$alkylthio group, a $(C_1-C_6)$alkylsulfinyl group, a halo$(C_1-C_6)$alkylsulfinyl group, a $(C_1-C_6)$alkylsulfonyl group, a halo$(C_1-C_6)$alkylsulfonyl group, a mono-$(C_1-C_6)$alkylamino group, a di$(C_1-C_6)$alkylamino group whose $(C_1-C_6)$ alkyl groups may be the same or different, a $(C_1-C_6)$alkylsulfonylamino group, and a halo$(C_1-C_6)$-alkylsulfonylamino group; or a $-COZ_m(R^6)$ group {wherein, $R^6$ represents a hydrogen atom; a $(C_1-C_6)$alkyl group; a halo$(C_1-C_6)$alkyl group; a $(C_2-C_6)$alkenyl group; a halo$(C_2-C_6)$alkenyl group; a $(C_2-C_6)$alkynyl group; a halo$(C_2-C_6)$alkynyl group; a $(C_1-C_6)$alkoxy$(C_1-C_6)$alkyl group; a halo$(C_1-C_6)$alkoxy$(C_1-C_6)$alkyl group; a $(C_1-C_6)$alkylthio$(C_1-C_6)$alkyl group; a halo$(C_1-C_6)$alkylthio$(C_1-C_6)$alkyl group; a $(C_1-C_6)$alkylsulfinyl$(C_1-C_6)$-alkyl group; a halo$(C_1-C_6)$alkylsulfinyl$(C_1-C_6)$ alkyl group; a $(C_1-C_6)$alkylsulfonyl$(C_1-C_6)$alkyl group; a halo$(C_1-C_6)$alkylsulfonyl $(C_1-C_6)$alkyl group; a mono $(C_1-C_6)$alkylamino$(C_1-C_6)$alkyl group; a di $(C_1-C_6)$alkylamino$(C_1-C_6)$alkyl group whose $(C_1-C_6)$alkyl groups may

be the same or different; a $(C_3-C_6)$cycloalkyl group; a halo$(C_3-C_6)$cycloalkyl group; a $(C_3-C_6)$cycloalkenyl group; a halo$(C_3-C_6)$cycloalkenyl group; a phenyl group; or a substituted phenyl group having at least one substituent which may be the same or different and is selected from the group consisting of a halogen atom, a cyano group, a nitro group, a hydroxyl group, an amino group, a mercapto group, a $(C_1-C_6)$alkyl group, a halo$(C_1-C_6)$alkyl group, a $(C_2-C_6)$alkenyl group, a halo$(C_2-C_6)$alkenyl group, a $(C_2-C_6)$alkynyl group, a halo$(C_2-C_6)$alkynyl group, a $(C_1-C_6)$ alkoxyl group, a halo$(C_1-C_6)$alkoxyl group, a $(C_1-C_6)$alkylthio group, a halo$(C_1-C_6)$alkylthio group, a $(C_1-C_6)$alkylsulfinyl group, a halo$(C_1-C_6)$alkylsulfinyl group, a $(C_1-C_6)$alkylsulfonyl group, a halo$(C_1-C_6)$alkylsulfonyl group, a mono$(C_1-C_6)$-alkylamino group, a di$(C_1-C_6)$alkylamino group whose $(C_1-C_6)$alkyl groups may be the same or different, a $(C_1-C_6)$alkylsulfonylamino group, and a halo$(C_1-C_6)$-alkylsulfonylamino group; Z represents an oxygen atom; a sulfur atom; or $N(R^7)$ [wherein, $R^7$ represents a hydrogen atom; a $(C_1-C_6)$alkyl group; a halo$(C_1-C_6)$alkyl group; a $(C_2-C_6)$alkenyl group; a halo$(C_2-C_6)$alkenyl group; a $(C_2-C_6)$alkynyl group; a halo$(C_2-C_6)$alkynyl group; a $(C_1-C_6)$alkoxy $(C_1-C_6)$alkyl group; a halo-$(C_1-C_6)$alkoxy$(C_1-C_6)$alkyl group; a $(C_1-C_6)$alkylthio-$(C_1-C_6)$alkyl group; a halo$(C_1-C_6)$alkylthio$(C_1-C_6)$alkyl group; a $(C_1-C_6)$alkylsulfinyl$(C_1-C_6)$alkyl group; a halo$(C_1-C_6)$alkylsulfinyl $(C_1-C_6)$alkyl group; a $(C_1-C_6)$-alkylsulfonyl$(C_1-C_6)$alkyl group; a halo$(C_1-C_6)$alkylsulfonyl$(C_1-C_6)$alkyl group; a mono$(C_1-C_6)$alkylamino-$(C_1-C_6)$alkyl group; a di$(C_1-C_6)$alkylamino$(C_1-C_6)$alkyl group whose $(C_1-C_6)$alkyl groups may be the same or different; a $(C_3-C_6)$cycloalkyl group; a halo$(C_3-C_6)$cycloalkyl group; a $(C_3-C_6)$cycloalkenyl group; or a halo-$(C_3-C_6)$cycloalkenyl group]; and m represents an integer of 0 to 1};

$R^2$ and $R^3$ may be same or different, and represent a hydrogen atom, a halogen atom; a cyano group; a nitro group; a hydroxyl group; a mercapto group; an amino group; a $(C_1-C_6)$alkyl group; a halo$(C_1-C_6)$alkyl group; a $(C_2-C_6)$alkenyl group; a halo $(C_2-C_6)$alkenyl group; a $(C_2-C_6)$alkynyl group; a halo$(C_2-C_6)$alkynyl group; a $(C_1-C_6)$ alkoxyl group; a halo$(C_1-C_6)$alkoxyl group; a $(C_1-C_6)$alkylthio group; a halo$(C_1-C_6)$alkylthio group; a $(C_1-C_6)$alkylsulfinyl group; a halo$(C_1-C_6)$alkylsulfinyl group; a $(C_1-C_6)$alkylsulfonyl group; a halo$(C_1-C_6)$alkylsulfonyl group; a mono$(C_1-C_6)$alkylamino group; a di$(C_1-C_6)$alkylamino group whose $(C_1-C_6)$alkyl groups may be the same or different; a $(C_1-C_6)$alkoxy$(C_1-C_6)$alkyl group; a halo$(C_1-C_6)$alkoxy-$(C_1-C_6)$alkyl group; a $(C_1-C_6)$alkylthio$(C_1-C_6)$alkyl group; a halo $(C_1-C_6)$alkylthio $(C_1-C_6)$alkyl group; a $(C_1-C_6)$alkylsulfinyl $(C_1-C_6)$alkyl group; a halo$(C_1-C_6)$alkylsulfinyl-$(C_1-C_6)$alkyl group; a $(C_1-C_6)$alkylsulfonyl$(C_1-C_6)$alkyl group; a halo$(C_1-C_6)$alkylsulfonyl$(C_1-C_6)$alkyl group; a mono $(C_1-C_6)$alkylamino$(C_1-C_6)$alkyl group; a di$(C_1-C_6)$alkylamino$(C_1-C_6)$alkyl group whose $(C_1-C_6)$alkyl groups may be the same or different; a $(C_3-C_6)$cycloalkyl group; a halo$(C_3-C_6)$cycloalkyl group; a $(C_3-C_6)$cycloalkenyl group; a halo$(C_3-C_6)$cycloalkenyl group; a phenyl group; a substituted phenyl group having at least one substituent which may be the same or different and is selected from the group consisting of a halogen atom, a cyano group, a nitro group, a hydroxyl group, an amino group, a mercapto group, a $(C_1-C_6)$alkyl group, a halo$(C_1-C_6)$alkyl group, a $(C_2-C_6)$alkenyl group, a halo$(C_2-C_6)$alkenyl group, a $(C_2-C_6)$alkynyl group, a halo$(C_2-C_6)$alkynyl group, a $(C_1-C_6)$ alkoxyl group, a halo$(C_1-C_6)$alkoxyl group, a $(C_1-C_6)$alkylthio group, a halo$(C_1-C_6)$alkylthio group, a $(C_1-C_6)$alkylsulfinyl group, a halo$(C_1-C_6)$alkylsulfinyl group, a $(C_1-C_6)$alkylsulfonyl group, a halo $(C_1-C_6)$alkylsulfonyl group, a mono$(C_1-C_6)$-alkylamino group, a di$(C_1-C_6)$alkylamino group whose $(C_1-C_6)$alkyl groups may be the same or different, a $(C_1-C_6)$alkylsulfonylamino group, and a halo$(C_1-C_6)$alkylsulfonylamino group; a phenoxy group; a substituted phenoxy group having at least one substituent which may be the same or different and is selected from the group consisting of a halogen atom, a cyano group, a nitro group, a hydroxyl group, an amino group, a mercapto group, a $(C_1-C_6)$alkyl group, a halo$(C_1-C_6)$alkyl group, a $(C_2-C_6)$alkenyl group, a halo$(C_2-C_6)$alkenyl group, a $(C_2-C_6)$alkynyl group, a halo$(C_2-C_6)$alkynyl group, a $(C_1-C_6)$alkoxyl group, a halo$(C_1-C_6)$alkoxyl group, a $(C_1-C_6)$ alkylthio group, a halo$(C_1-C_6)$alkylthio group, a $(C_1-C_6)$alkylsulfinyl group, a halo$(C_1-C_6)$alkylsulfinyl group, a $(C_1-C_6)$alkylsulfonyl group, a halo$(C_1-C_6)$alkylsulfonyl group, a mono$(C_1-C_6)$alkylamino group, a di$(C_1-C_6)$ alkylamino group whose $(C_1-C_6)$alkyl groups may be the same or different, a $(C_1-C_6)$alkylsulfonylamino group, and a halo$(C_1-C_6)$alkylsulfonylamino group; a phenylthio group; a substituted phenylthio group having at least one substituent which may be the same or different and is selected from the group consisting of a halogen atom, a cyano group, a nitro group, a hydroxyl group, an amino group, a mercapto group, a $(C_1-C_6)$alkyl group, a halo $(C_1-C_6)$alkyl group, a $(C_2-C_6)$alkenyl group, a halo$(C_2-C_6)$alkenyl group, a $(C_2-C_6)$alkynyl group, a halo$(C_2-C_6)$ alkynyl group, a $(C_1-C_6)$alkoxyl group, a halo$(C_1-C_6)$alkoxyl group, a $(C_1-C_6)$alkylthio group, a halo$(C_1-C_6)$alkylthio group, a $(C_1-C_6)$alkylsulfinyl group, a halo$(C_1-C_6)$alkylsulfinyl group, a $(C_1-C_6)$alkylsulfonyl group, a halo$(C_1-C_6)$ alkylsulfonyl group, a mono$(C_1-C_6)$alkylamino group, a di $(C_1-C_6)$alkylamino group whose $(C_1-C_6)$alkyl groups may be the same or different, a $(C_1-C_6)$alkylsulfonylamino group, and a halo$(C_1-C_6)$alkylsulfonyl amino group; a $-ZCOZ_m$ $(R^6)$ group (wherein, $R^6$, Z, and m are the same as described above) ; or a $-COZ_m(R^6)$ group (wherein, $R^6$, Z, and m are the same as described above) ;

$R^4$ represents a hydrogen atom; a $(C_1-C_6)$alkyl group; a halo$(C_1-C_6)$alkyl group; a cyano$(C_1-C_6)$alkyl group; a $(C_2-C_6)$alkenyl group; a halo$(C_2-C_6)$alkenyl group; a $(C_2-C_6)$alkynyl group; a halo$(C_2-C_6)$alkynyl group; a $(C_1-C_6)$ alkoxy$(C_1-C_6)$alkyl group; a halo$(C_1-C_6)$-alkoxy$(C_1-C_6)$alkyl group; a $(C_1-C_6)$alkoxy$(C_1-C_6)$-alkoxy$(C_1-C_6)$alkyl group; a $(C_1-C_6)$alkylthio$(C_1-C_6)$alkyl group; a halo$(C_1-C_6)$alkylthio$(C_1-C_6)$alkyl group; a $(C_1-C_6)$alkylsulfinyl $(C_1-C_6)$alkyl group; a halo$(C_1-C_6)$alkylsulfinyl$(C_1-C_6)$alkyl group; a $(C_1-C_6)$-alkylsulfonyl$(C_1-C_6)$alkyl group; a halo

$(C_1-C_6)$-alkylsulfonyl$(C_1-C_6)$alkyl group; a mono $(C_1-C_6)$alkylamino $(C_1-C_6)$alkyl group; a di $(C_1-C_6)$ alkylamino $(C_1-C_6)$alkyl group whose $(C_1-C_6)$alkyl groups may be the same or different; a $(C_3-C_6)$cycloalkyl group; a halo $(C_3-C_6)$-cycloalkyl group; a $(C_3-C_6)$cycloalkenyl group; a halo$(C_3-C_6)$cycloalkenyl group; a $-COZ_m(R^6)$ group (wherein, $R^6$, Z, and m are the same as described above), a $-CSNH(R^6)$ group (wherein, $R^6$, is the same as described above), a $(C_1-C_6)$alkylcarbonyl$(C_1-C_6)$alkyl group; a $(C_1-C_6)$alkoxycarbonyl$(C_1-C_6)$alkyl group; a phenyl $(C_1-C_6)$-alkyl group; a substituted phenyl$(C_1-C_6)$alkyl group having on the ring at least one substituent which may be the same or different and is selected from the group consisting of a halogen atom, a cyano group, a nitro group, a hydroxyl group, an amino group, a mercapto group, a $(C_1-C_6)$alkyl group, a halo$(C_1-C_6)$alkyl group, a $(C_2-C_6)$ alkenyl group, a halo$(C_2-C_6)$alkenyl group, a $(C_2-C_6)$alkynyl group, a halo$(C_2-C_6)$alkynyl group, a $(C_1-C_6)$alkoxyl group, a halo$(C_1-C_6)$alkoxyl group, a $(C_1-C_6)$alkylthio group, a halo$(C_1-C_6)$alkylthio group, a $(C_1-C_6)$alkylsulfinyl group, a halo$(C_1-C_6)$alkylsulfinyl group, a $(C_1-C_6)$alkylsulfonyl group, a halo$(C_1-C_6)$alkylsulfonyl group, a mono $(C_1-C_6)$alkylamino group, a di$(C_1-C_6)$alkylamino group whose $(C_1-C_6)$alkyl groups may be the same or different, a $(C_1-C_6)$alkylsulfonylamino group, and a halo$(C_1-C_6)$alkylsulfonylamino group; a heterocyclic$(C_1-C_6)$alkyl group (a heterocyclic ring represents a pyridyl group; a pyridine-N-oxide group; a pyridazinyl group; a pyrimidinyl group; a pyrazyl group; a piperidyl group; a piperazino group; a morpholinyl group; a morpholino group; a furyl group; a tetrahydrofuryl group; a thienyl group; a tetrahydrothienyl group; a pyrrolyl group; a pyrrolidyl group; an imidazolidinyl group; an oxazolyl group; an isoxazolyl group; an oxadiazolyl group; a thiazolyl group; an isothiazolyl group; a thiadiazolyl group; an imidazolyl group; a triazolyl group; a pyrazolyl group; an indolyl group; a benzofuryl group; a benzothienyl group; an indazolyl group; a quinolyl group; an isoquinolyl group; or a quinazolyl group); or a substituted heterocyclic$(C_1-C_6)$alkyl group (the heterocyclic group is the same as the above-described) having on the ring at least one substituent which may be the same or different and is selected from the group consisting of a halogen atom, a cyano group, a nitro group, a hydroxyl group, an amino group, a mercapto group, a $(C_1-C_6)$alkyl group, a halo$(C_1-C_6)$alkyl group, a $(C_2-C_6)$alkenyl group, a halo$(C_2-C_6)$alkenyl group, a $(C_2-C_6)$alkynyl group, a halo$(C_2-C_6)$alkynyl group, a $(C_1-C_6)$alkoxyl group, a halo$(C_1-C_6)$alkoxyl group, a $(C_1-C_6)$alkylthio group, a halo $(C_1-C_6)$alkylthio group, a $(C_1-C_6)$alkylsulfinyl group, a halo$(C_1-C_6)$alkylsulfinyl group, a $(C_1-C_6)$alkylsulfonyl group, a halo$(C_1-C_6)$alkylsulfonyl group, a mono$(C_1-C_6)$alkylamino group, a di$(C_1-C_6)$alkylamino group whose $(C_1-C_6)$ alkyl groups may be the same or different, a $(C_1-C_6)$alkylsulfonylamino group, and a halo$(C_1-C_6)$alkylsulfonylamino group;

$R^5$ represents at least one group selected from the group consisting of (i), (ii), (iii), (iv), (v), and (vi) as described below:

(i) a group represented by the formula (a):

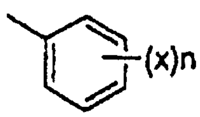

(a)

wherein X may be the same or different, and represents a halogen atom; a cyano group; a nitro group; a hydroxyl group; a mercapto group; an amino group; a $(C_1-C_6)$alkyl group; a halo$(C_1-C_6)$alkyl group; a cyano$(C_1-C_6)$alkyl group; a $(C_2-C_6)$alkenyl group; a halo $(C_2-C_6)$alkenyl group; a $(C_2-C_6)$alkynyl group; a halo$(C_2-C_6)$alkynyl group; a $(C_1-C_6)$alkoxyl group; a halo$(C_1-C_6)$alkoxyl group; a $(C_1-C_6)$alkylthio group; a halo$(C_1-C_6)$alkylthio group; a $(C_1-C_6)$alkylsulfinyl group; a halo$(C_1-C_6)$alkylsulfinyl group; a $(C_1-C_6)$alkylsulfonyl group; a halo$(C_1-C_6)$alkylsulfonyl group; a mono$(C_1-C_6)$alkylamino group; a di$(C_1-C_6)$alkylamino group whose $(C_1-C_6)$alkyl groups may be the same or different; a $(C_1-C_6)$alkoxy$(C_1-C_6)$alkyl group; a halo$(C_1-C_6)$alkoxy-$(C_1-C_6)$alkyl group; a $(C_1-C_6)$ alkylthio $(C_1-C_6)$ alkyl group; a halo$(C_1-C_6)$alkylthio$(C_1-C_6)$alkyl group; a $(C_1-C_6)$alkylsulfinyl $(C_1-C_6)$alkyl group; a halo $(C_1-C_6)$alkylsulfinyl-$(C_1-C_6)$alkyl group; a $(C_1-C_6)$alkylsulfonyl $(C_1-C_6)$alkyl group; a halo$(C_1-C_6)$alkylsulfonyl $(C_1-C_6)$alkyl group; a mono $(C_1-C_6)$alkylamino$(C_1-C_6)$alkyl group; a di$(C_1-C_6)$-alkylamino$(C_1-C_6)$alkyl group whose $(C_1-C_6)$alkyl groups may be the same or different; a $(C_3-C_6)$cycloalkyl group; a halo$(C_3-C_6)$cycloalkyl group; a $(C_1-C_6)$alkylenedioxy group; a $(C_1-C_6)$alkyl$(C_1-C_6)$alkylenedioxy group; a phenyl$(C_1-C_6)$alkylenedioxy group; a substituted phenyl$(C_1-C_6)$alkylenedioxy group having on the ring at least one substituent which may be the same or different and is selected from the group consisting of a halogen atom, a cyano group, a nitro group, a hydroxyl group, an amino group, a mercapto group, a $(C_1-C_6)$alkyl group, a halo$(C_1-C_6)$alkyl group, a $(C_2-C_6)$alkenyl group, a halo$(C_2-C_6)$alkenyl group, a $(C_2-C_6)$alkynyl group, a halo$(C_2-C_6)$alkynyl group, a $(C_1-C_6)$alkoxyl group, a halo $(C_1-C_6)$alkoxyl group, a $(C_1-C_6)$alkylthio group, a halo$(C_1-C_6)$alkylthio group, a $(C_1-C_6)$alkylsulfinyl group, a halo $(C_1-C_6)$alkylsulfinyl group, a $(C_1-C_6)$alkylsulfonyl group, a halo$(C_1-C_6)$alkylsulfonyl group, a mono$(C_1-C_6)$alkylamino group, a di$(C_1-C_6)$alkylamino group whose $(C_1-C_6)$alkyl groups may be the same or different, a $(C_1-C_6)$alkyl-

sulfonyl amino group, and a halo($C_1$-$C_6$)alkylsulfonyl amino group; a phenyl ($C_1$-$C_6$)alkyl($C_1$-$C_6$)alkylenedioxy group; a substituted phenyl ($C_1$-$C_6$) alkyl ($C_1$-$C_6$) alkylenedioxy group having on the ring at least one substituent which may be the same or different and is selected from the group consisting of a halogen atom, a cyano group, a nitro group, a hydroxyl group, an amino group, a mercapto group, a ($C_1$-$C_6$)alkyl group, a halo($C_1$-$C_6$)alkyl group, a ($C_2$-$C_6$)alkenyl group, a halo($C_2$-$C_6$)alkenyl group, a ($C_2$-$C_6$)alkynyl group, a halo($C_2$-$C_6$)alkynyl group, a ($C_1$-$C_6$) alkoxyl group, a halo($C_1$-$C_6$)alkoxyl group, a ($C_1$-$C_6$)alkylthio group, a halo($C_1$-$C_6$)alkylthio group, a ($C_1$-$C_6$)alkylsulfinyl group, a halo($C_1$-$C_6$)alkylsulfinyl group, a ($C_1$-$C_6$)alkylsulfonyl group, a halo($C_1$-$C_6$)alkylsulfonyl group, a mono($C_1$-$C_6$)alkylamino group, a di($C_1$-$C_6$)alkylamino group whose ($C_1$-$C_6$)alkyl groups may be the same or different, a ($C_1$-$C_6$)alkylsulfonylamino group, and a halo($C_1$-$C_6$)alkylsulfonylamino group; a hydroxyimino ($C_1$-$C_6$) alkyl group; a ($C_1$-$C_6$) alkoxyimino ($C_1$-$C_6$)alkyl group; a ($C_2$-$C_6$)alkenyloxyimino($C_1$-$C_6$)alkyl group; a phenyl ($C_1$-$C_6$)alkoxyimino($C_1$-$C_6$)alkyl group; a substituted phenyl($C_1$-$C_6$)alkoxyimino($C_1$-$C_6$)alkyl group having on the ring at least one substituent which may be the same or different and is selected from the group consisting of a halogen atom, a cyano group, a nitro group, a hydroxyl group, an amino group, a mercapto group, a ($C_1$-$C_6$)alkyl group, a halo($C_1$-$C_6$)alkyl group, a ($C_2$-$C_6$)alkenyl group, a halo($C_2$-$C_6$)alkenyl group, a ($C_2$-$C_6$)alkynyl group, a halo($C_2$-$C_6$)alkynyl group, a ($C_1$-$C_6$)alkoxyl group, a halo($C_1$-$C_6$)alkoxyl group, a ($C_1$-$C_6$)alkylthio group, a halo ($C_1$-$C_6$)alkylthio group, a ($C_1$-$C_6$)alkylsulfinyl group, a halo($C_1$-$C_6$)alkylsulfinyl group, a ($C_1$-$C_6$)alkylsulfonyl group, a halo($C_1$-$C_6$)alkylsulfonyl group, a mono($C_1$-$C_6$)alkylamino group, a di($C_1$-$C_6$)alkylamino group whose ($C_1$-$C_6$) alkyl groups may be the same or different, a ($C_1$-$C_6$)alkylsulfonylamino group, and a halo($C_1$-$C_6$)alkylsulfonylamino group; a heterocyclic($C_1$-$C_6$)alkoxyimino($C_1$-$C_6$)alkyl group (the heterocyclic ring is the same as described above); a substituted heterocyclic($C_1$-$C_6$)alkoxyimino($C_1$-$C_6$)alkyl group (the heterocyclic group is the same as described above) having on the ring at least one substituent which may be the same or different and is selected from the group consisting of a halogen atom, a cyano group, a nitro group, a hydroxyl group, an amino group, a mercapto group, a ($C_1$-$C_6$)alkyl group, a halo($C_1$-$C_6$)alkyl group, a ($C_2$-$C_6$)alkenyl group, a halo($C_2$-$C_6$)alkenyl group, a ($C_2$-$C_6$)alkynyl group, a halo($C_2$-$C_6$)alkynyl group, a ($C_1$-$C_6$)alkoxyl group, a halo($C_1$-$C_6$)alkoxyl group, a ($C_1$-$C_6$) alkylthio group, a halo($C_1$-$C_6$)alkylthio group, a ($C_1$-$C_6$)alkylsulfinyl group, a halo($C_1$-$C_6$)-alkylsulfinyl group, a ($C_1$-$C_6$)alkylsulfonyl group, a halo($C_1$-$C_6$)alkylsulfonyl group, a mono($C_1$-$C_6$)alkylamino group, a di($C_1$-$C_6$) alkylamino group whose ($C_1$-$C_6$)alkyl groups may be the same or different, a ($C_1$-$C_6$)alkylsulfonylamino group, and a halo($C_1$-$C_6$)alkylsulfonylamino group; a ($C_1$-$C_6$)alkylcarbonyl($C_1$-$C_6$)alkyl group; a ($C_1$-$C_6$)alkoxycarbonyl ($C_1$-$C_6$)alkyl group; the following group:

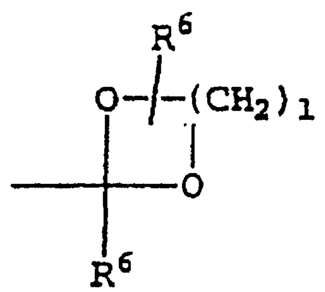

(wherein, $R^6$ may be the same or different, and the same as described above, and 1 is an integer of 2 to 4); a -$COZ_m(R^6)$ group (wherein, $R^6$, Z, and m are the same as described above); a -$ZCOZ_m(R^6)$ group (wherein, $R^6$, Z, and m are the same as described above); a ($C_1$-$C_6$)-alkylsulfonylamino group; a halo($C_1$-$C_6$)alkylsulfonylamino group; a phenyl group; a substituted phenyl group having at least one substituent which may be the same or different and is selected from the group consisting of a halogen atom, a cyano group, a nitro group, a hydroxyl group, an amino group, a mercapto group, a ($C_1$-$C_6$)alkyl group, a halo($C_1$-$C_6$)alkyl group, a ($C_2$-$C_6$)alkenyl group, a halo($C_2$-$C_6$)alkenyl group, a ($C_2$-$C_6$)alkynyl group, a halo($C_2$-$C_6$)alkynyl group, a ($C_1$-$C_6$)alkoxyl group, a halo ($C_1$-$C_6$)alkoxyl group, a ($C_1$-$C_6$)alkylthio group, a halo($C_1$-$C_6$)alkylthio group, a ($C_1$-$C_6$)alkylsulfinyl group, a halo ($C_1$-$C_6$)alkylsulfinyl group, a ($C_1$-$C_6$)alkylsulfonyl group, a halo($C_1$-$C_6$)alkylsulfonyl group, a mono($C_1$-$C_6$)alkylamino group, a di($C_1$-$C_6$)alkylamino group whose ($C_1$-$C_6$)alkyl groups may be the same or different, a -$COZ_m(R^6)$ group (wherein, $R^6$, Z, and m are the same as described above), a -$ZCOZ_m(R^6)$ group (wherein, $R^6$, Z, and m are the same as described above), a ($C_1$-$C_6$)alkylsulfonylamino group, a halo($C_1$-$C_6$)-alkylsulfonylamino group, a hydroxyimino($C_1$-$C_6$)alkyl group, a ($C_1$-$C_6$)alkoxyimino($C_1$-$C_6$)alkyl group, a heterocyclic group (a heterocyclic ring is the same as described above), and a substituted heterocyclic group (a heterocyclic ring is the same as described above) having at least one substituent which may be the same or different and is selected from the group consisting of a halogen atom, a cyano group, a nitro group, a hydroxyl group, an amino group, a mercapto group, a ($C_1$-$C_6$) alkyl group, a halo($C_1$-$C_6$)alkyl group, a ($C_2$-$C_6$)alkenyl group, a halo($C_2$-$C_6$)alkenyl group, a ($C_2$-$C_6$)alkynyl group,

a halo($C_2$-$C_6$)alkynyl group, a ($C_1$-$C_6$)alkoxyl group, a halo($C_1$-$C_6$)alkoxyl group, a ($C_1$-$C_6$)alkylthio group, a halo ($C_1$-$C_6$)alkylthio group, a ($C_1$-$C_6$)alkylsulfinyl group, a halo($C_1$-$C_6$)alkylsulfinyl group, a ($C_1$-$C_6$)alkylsulfonyl group, a halo($C_1$-$C_6$)alkylsulfonyl group, a mono($C_1$-$C_6$)alkylamino group, a di($C_1$-$C_6$)alkylamino group whose ($C_1$-$C_6$) alkyl groups may be the same or different, a ($C_1$-$C_6$)alkylsulfonylamino group, and a halo($C_1$-$C_6$)alkylsulfonylamino group; a naphthyl group; a substituted naphthyl group having at least one substituent which may be the same or different and is selected from the group consisting of a halogen atom, a cyano group, a nitro group, a hydroxyl group, an amino group, a mercapto group, a ($C_1$-$C_6$)alkyl group, a halo($C_1$-$C_6$)alkyl group, a ($C_2$-$C_6$)alkenyl group, a halo($C_2$-$C_6$)alkenyl group, a ($C_2$-$C_6$)alkynyl group, a halo($C_2$-$C_6$)alkynyl group, a ($C_1$-$C_6$)alkoxyl group, a halo ($C_1$-$C_6$)alkoxyl group, a ($C_1$-$C_6$)alkylthio group, a halo($C_1$-$C_6$)alkylthio group, a ($C_1$-$C_6$)alkylsulfinyl group, a halo ($C_1$-$C_6$)alkylsulfinyl group, a ($C_1$-$C_6$)alkylsulfonyl group, a halo($C_1$-$C_6$)alkylsulfonyl group, a mono($C_1$-$C_6$)alkylamino group, a di($C_1$-$C_6$)alkylamino group whose ($C_1$-$C_6$)alkyl groups may be the same or different, a -$COZ_m(R^6)$ group (wherein, $R^6$, Z, and m are the same as described above), a -$ZCOZ_m(R^6)$ group (wherein, $R^6$, Z, and m are the same as described above), a ($C_1$-$C_6$)alkylsulfonylamino group, and a halo($C_1$-$C_6$)alkylsulfonylamino group; a heterocyclic group (a heterocyclic ring is the same as described above); a substituted heterocyclic group(a heterocyclic ring is the same as described above) having at least one substituent which may be the same or different and is selected from the group consisting of a halogen atom, a cyano group, a nitro group, a hydroxyl group, an amino group, a mercapto group, a ($C_1$-$C_6$)alkyl group, a halo($C_1$-$C_6$)alkyl group, a ($C_2$-$C_6$)alkenyl group, a halo ($C_2$-$C_6$)alkenyl group, a ($C_2$-$C_6$)alkynyl group, a halo($C_2$-$C_6$)alkynyl group, a ($C_1$-$C_6$)alkoxyl group, a halo($C_1$-$C_6$) alkoxyl group, a ($C_1$-$C_6$)alkylthio group, a halo($C_1$-$C_6$)alkylthio group, a ($C_1$-$C_6$)alkylsulfinyl group, a halo($C_1$-$C_6$) alkylsulfinyl group, a ($C_1$-$C_6$)alkylsulfonyl group, a halo($C_1$-$C_6$)alkylsulfonyl group, a mono($C_1$-$C_6$)alkylamino group, a di($C_1$-$C_6$)alkylamino group whose ($C_1$-$C_6$)alkyl groups may be the same or different, a -$COZ_m(R^6)$ group (wherein, $R^6$, Z, and m are the same as described above), a -$ZCOZ_m(R^6)$ group (wherein, $R^6$, Z, and m are the same as described above), a ($C_1$-$C_6$)alkylsulfonylamino group, a halo($C_1$-$C_6$)alkylsulfonylamino group, a hydroxyimino($C_1$-$C_6$)alkyl group, and a ($C_1$-$C_6$)-alkoxyimino($C_1$-$C_6$)alkyl group; or -Q-Ar [wherein, Q represents an oxygen atom; a sulfur atom; $N(R^7)$ (wherein, $R^7$ is same as described above); a ($C_1$-$C_6$)-alkylene group; a ($C_1$-$C_6$)alkyl ($C_1$-$C_6$)alkylene group; a halo($C_1$-$C_6$)alkyl($C_1$-$C_6$)alkylene group; a hydroxy ($C_1$-$C_6$)-alkyl($C_1$-$C_6$)alkylene group; a ($C_1$-$C_6$)alkoxy($C_1$-$C_6$)alkyl-($C_1$-$C_6$)alkylene group; a ($C_2$-$C_6$)alkenyl($C_1$-$C_6$)alkylene group; a halo($C_2$-$C_6$)alkenyl ($C_1$-$C_6$)alkylene group; a ($C_2$-$C_6$)alkynyl($C_1$-$C_6$)alkylene group; a halo($C_2$-$C_6$)alkynyl-($C_1$-$C_6$)alkylene group; a hydroxy($C_1$-$C_6$)alkylene group; a ($C_1$-$C_6$)alkoxy($C_1$-$C_6$)alkylene group; a halo($C_1$-$C_6$)alkoxy-($C_1$-$C_6$)alkylene group; a ($C_1$-$C_6$)alkylcarbonyloxy ($C_1$-$C_6$)alkylene group; a cyano($C_1$-$C_6$)alkylene group; a halo($C_1$-$C_6$)alkylene group; a ($C_3$-$C_6$)cycloalkyl($C_1$-$C_6$)-alkylene group; a ($C_3$-$C_6$)cycloalkenyl($C_1$-$C_6$)alkylene group; a ($C_1$-$C_6$)alkylcarbonyl ($C_1$-$C_6$)alkylene group; a ($C_1$-$C_6$)alkoxycarbonyl($C_1$-$C_6$)alkylene group; an amino-($C_1$-$C_6$)alkylene group; a mono ($C_1$-$C_6$)alkylamino($C_1$-$C_6$)-alkylene group; a di($C_1$-$C_6$)alkylamino($C_1$-$C_6$)alkylene group whose ($C_1$-$C_6$)alkyl groups may be the same or different; a ($C_1$-$C_6$)alkylcarbonylamino($C_1$-$C_6$)alkylene group; a ($C_1$-$C_6$)alkoxycarbonylamino ($C_1$-$C_6$)alkylene group; a mercapto ($C_1$-$C_6$)alkylene group; a ($C_1$-$C_6$)alkylthio ($C_1$-$C_6$)alkylene group; an alkylsulfinyl($C_1$-$C_6$)alkylene group; a ($C_1$-$C_6$)alkylsulfonyl($C_1$-$C_6$)alkylene group; a ($C_3$-$C_6$)cycloalkylene group; a ($C_2$-$C_6$) alkenylene group; a ($C_1$-$C_6$)alkyl($C_2$-$C_6$)alkenylene group; a halo($C_1$-$C_6$)alkyl-($C_2$-$C_6$)alkenylene group; a hydroxy ($C_1$-$C_6$)alkyl($C_2$-$C_6$)-alkenylene group; a ($C_1$-$C_6$)alkoxy($C_1$-$C_6$)alkyl($C_2$-$C_6$)-alkenylene group; a ($C_2$-$C_6$)alkenyl ($C_2$-$C_6$)alkenylene group; a halo ($C_2$-$C_6$)alkenyl($C_2$-$C_6$)alkenylene group; a ($C_2$-$C_6$)alkynyl($C_2$-$C_6$)alkenylene group; a halo ($C_2$-$C_6$)-alkynyl($C_2$-$C_6$)alkenylene group; a hydroxy ($C_2$-$C_6$)-alkenylene group; a ($C_1$-$C_6$)alkoxy ($C_2$-$C_6$)alkenylene group; a halo ($C_1$-$C_6$)alkoxy($C_2$-$C_6$)alkenylene group; a ($C_1$-$C_6$)-alkylcarbonyloxy($C_2$-$C_6$)alkenylene group; a cyano ($C_2$-$C_6$)-alkenylene group; a halo($C_2$-$C_6$)alkenylene group; a ($C_3$-$C_6$)cycloalkyl($C_2$-$C_6$)alkenylene group; a ($C_3$-$C_6$)cycloalkenyl($C_2$-$C_6$)alkenylene group; a ($C_1$-$C_6$)alkylcarbonyl-($C_2$-$C_6$)alkenylene group; a ($C_1$-$C_6$)alkoxylcarbonyl($C_2$-$C_6$)-alkenylene group; an amino($C_2$-$C_6$)alkenylene group; a mono ($C_1$-$C_6$)alkylamino ($C_2$-$C_6$)alkenylene group; a di ($C_1$-$C_6$)-alkylamino($C_2$-$C_6$)alkenylene group whose ($C_1$-$C_6$)alkyl groups may be the same or different; a ($C_1$-$C_6$)alkylcarbonylamino($C_2$-$C_6$)alkenylene group; a ($C_1$-$C_6$)alkoxycarbonylamino($C_2$-$C_6$) alkenylene group; a mercapto($C_2$-$C_6$)-alkenylene group; a ($C_1$-$C_6$)alkylthio($C_2$-$C_6$)alkenylene group; a ($C_1$-$C_6$) alkylsulfinyl($C_2$-$C_6$)alkenylene group; a ($C_1$-$C_6$)alkylsulfonyl($C_2$-$C_6$)alkenylene group; a ($C_2$-$C_6$)alkynylene group; a ($C_1$-$C_6$)alkylenoxy group; a ($C_1$-$C_6$)alkyl($C_1$-$C_6$)alkylenoxy group; a ($C_1$-$C_6$)alkylenethio group; a ($C_1$-$C_6$)alkyl ($C_1$-$C_6$)alkylenethio group; a ($C_1$-$C_6$)alkyleneamino group; a ($C_1$-$C_6$)alkyl ($C_1$-$C_6$)-alkyleneamino group; a carbonyl group; a hydroxyimino group; a ($C_1$-$C_6$)alkoxyimino group; a ($C_1$-$C_6$)alkylenedioxy($C_1$-$C_6$)alkylene group; or a Z $(R^6)_m$CO group (wherein, $R^6$, Z, and m are the same as described above);

Ar represents a phenyl group; a substituted phenyl group having at least one substituent which may be the same or different and is selected from the group consisting of a halogen atom, a cyano group, a nitro group, a hydroxyl group, an amino group, a mercapto group, a ($C_1$-$C_6$)alkyl group, a halo($C_1$-$C_6$)alkyl group, a ($C_2$-$C_6$) alkenyl group, a halo($C_2$-$C_6$)alkenyl group, a ($C_2$-$C_6$)alkynyl group, a halo($C_2$-$C_6$)alkynyl group, a ($C_1$-$C_6$)alkoxyl group, a halo($C_1$-$C_6$)alkoxyl group, a ($C_1$-$C_6$)alkylthio group, a halo($C_1$-$C_6$)alkylthio group, a ($C_1$-$C_6$)alkylsulfinyl group, a halo($C_1$-$C_6$)alkylsulfinyl group, a ($C_1$-$C_6$)alkylsulfonyl group, a halo($C_1$-$C_6$)alkylsulfonyl group, a mono

$(C_1-C_6)$alkylamino group, a di$(C_1-C_6)$alkylamino group whose $(C_1-C_6)$alkyl groups may be the same or different, a hydroxyimino$(C_1-C_6)$alkyl group, a $(C_1-C_6)$alkoxyimino $(C_1-C_6)$alkyl group, a $(C_2-C_6)$alkenyloxyimino $(C_1-C_6)$ alkyl group, a $-COZ_m(R^6)$ group (wherein, $R^6$, Z, and m are the same as described above), a $-ZCOZ_m(R^6)$ group (wherein, $R^6$, Z, and m are the same as described above), a $(C_1-C_6)$alkylsulfonylamino group, and a halo$(C_1-C_6)$ alkylsulfonylamino group; a naphthyl group; a substituted naphthyl group having at least one substituent which may be the same or different and is selected from the group consisting of a halogen atom, a cyano group, a nitro group, a hydroxyl group, an amino group, a mercapto group, a $(C_1-C_6)$alkyl group, a halo$(C_1-C_6)$alkyl group, a $(C_2-C_6)$alkenyl group, a halo$(C_2-C_6)$alkenyl group, a $(C_2-C_6)$alkynyl group, a halo$(C_2-C_6)$alkynyl group, a $(C_1-C_6)$ alkoxyl group, a halo$(C_1-C_6)$alkoxyl group, a $(C_1-C_6)$alkylthio group, a halo$(C_1-C_6)$alkylthio group, a $(C_1-C_6)$alkylsulfinyl group, a halo$(C_1-C_6)$alkylsulfinyl group, a $(C_1-C_6)$alkylsulfonyl group, a halo$(C_1-C_6)$alkylsulfonyl group, a mono$(C_1-C_6)$alkylamino group, a di$(C_1-C_6)$alkylamino group whose $(C_1-C_6)$alkyl groups may be the same or different, a $-COZ_m(R^6)$ group (wherein, $R^6$, Z, and m are the same as described above), a $-ZCOZ_m(R^6)$ group (wherein, $R^6$, Z, and m are the same as described above), a $(C_1-C_6)$alkylsulfonylamino group, and a halo$(C_1-C_6)$alkylsulfonylamino group; a heterocyclic group (a heterocyclic ring is the same as described above); or a substituted heterocyclic group (a heterocyclic ring is the same as described above) having at least one substituent which may be the same or different and is selected from the group consisting of a halogen atom, a cyano group, a nitro group, a hydroxyl group, an amino group, a mercapto group, a $(C_1-C_6)$alkyl group, a halo$(C_1-C_6)$alkyl group, a $(C_2-C_6)$ alkenyl group, a halo $(C_2-C_6)$alkenyl group, a $(C_2-C_6)$alkynyl group, a halo$(C_2-C_6)$alkynyl group, a $(C_1-C_6)$alkoxyl group, a halo$(C_1-C_6)$alkoxyl group, a $(C_1-C_6)$alkylthio group, a halo$(C_1-C_6)$alkylthio group, a $(C_1-C_6)$alkylsulfinyl group, a halo$(C_1-C_6)$alkylsulfinyl group, a $(C_1-C_6)$alkylsulfonyl group, a halo$(C_1-C_6)$alkylsulfonyl group, a mono $(C_1-C_6)$-alkylamino group, a di$(C_1-C_6)$alkylamino group whose $(C_1-C_6)$alkyl groups may be the same or different, a $-COZ_m(R^6)$ group (wherein, $R^6$, Z, and m are the same as described above), a $-ZCOZ_m(R^6)$ group (wherein, $R^6$, Z, and m are the same as described above), a $(C_1-C_6)$alkylsulfonylamino group, a halo$(C_1-C_6)$alkylsulfonylamino group, a hydroxyimino$(C_1-C_6)$alkyl group, a $(C_1-C_6)$alkoxyimino$(C_1-C_6)$alkyl group, and a $(C_2-C_6)$alkenyloxyimino-$(C_1-C_6)$alkyl group]; and

n represents an integer of 1 to 5;

(ii) a group represented by the formula (b) :

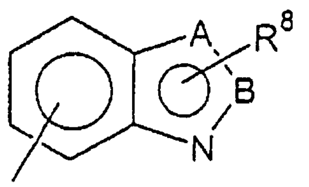

(b)

wherein $R^8$ represents a hydrogen atom; a halogen atom; a cyano group; a nitro group; a hydroxyl group; an amino group; a mercapto group; a $(C_1-C_6)$alkyl group; a halo$(C_1-C_6)$alkyl group; a cyano$(C_1-C_6)$alkyl group; a $(C_3-C_6)$ cycloalkyl group; a halo $(C_3-C_6)$cycloalkyl group; a cyano$(C_3-C_6)$cycloalkyl group; a $(C_1-C_6)$alkyl$(C_3-C_6)$cycloalkyl group; a $(C_1-C_6)$alkyl-halo$(C_3-C_6)$cycloalkyl group; a $(C_3-C_6)$cycloalkyl$(C_1-C_6)$alkyl group; a $(C_2-C_6)$alkenyl group; a $(C_3-C_6)$cycloalkenyl group; a halo$(C_2-C_6)$alkenyl group; a $(C_3-C_6)$cycloalkenyl$(C_1-C_6)$alkyl group; a $(C_2-C_6)$alkynyl group; a halo$(C_2-C_6)$alkynyl group; a $(C_1-C_6)$alkoxyl group; a halo$(C_1-C_6)$alkoxyl group; a $(C_1-C_6)$alkylthio group; a halo$(C_1-C_6)$alkylthio group; a $(C_1-C_6)$alkylsulfinyl group; a halo$(C_1-C_6)$alkylsulfinyl group; a $(C_1-C_6)$alkylsulfonyl group; a halo$(C_1-C_6)$alkylsulfonyl group; a mono$(C_1-C_6)$alkylamino group; a di$(C_1-C_6)$alkylamino group whose $(C_1-C_6)$alkyl groups may be the same or different; a $(C_1-C_6)$alkoxy$(C_1-C_6)$alkyl group; a halo$(C_1-C_6)$alkoxy $(C_1-C_6)$alkyl group; a $(C_1-C_6)$alkylthio$(C_1-C_6)$alkyl group; a halo$(C_1-C_6)$-alkylthio $(C_1-C_6)$alkyl group; a $(C_1-C_6)$alkylsulfinyl$(C_1-C_6)$alkyl group; a halo$(C_1-C_6)$alkylsulfinyl$(C_1-C_6)$alkyl group; a $(C_1-C_6)$alkylsulfonyl$(C_1-C_6)$alkyl group; a halo$(C_1-C_6)$alkylsulfonyl$(C_1-C_6)$alkyl group; a mono $(C_1-C_6)$alkylamino$(C_1-C_6)$alkyl group; a di$(C_1-C_6)$alkylamino $(C_1-C6)$alkyl group whose $(C_1-C_6)$alkyl groups may be the same or different; a carboxy$(C_1-C_6)$alkyl group; a $(C_1-C_6)$-alkylcarbonyl$(C_1-C_6)$alkyl group; a $(C_1-C_6)$alkoxycarbonyl-$(C_1-C_6)$alkyl group; an aminocarbonyl$(C_1-C_6)$ alkyl group; a mono$(C_1-C_6)$alkylaminocarbonyl$(C_1-C_6)$alkyl group; a di$(C_1-C_6)$alkylaminocarbonyl$(C_1-C_6)$alkyl group whose $(C_1-C_6)$alkyl groups may be the same or different; a $-COZ_m(R^6)$ group (in the formula, $R^6$, Z, and m are the same as described above); a $-ZCOZ_m(R^6)$ group (wherein, $R^6$, Z, and m are the same as described above); a $(C_1-C_6)$alkylsulfonylamino group; a halo$(C_1-C_6)$alkylsulfonylamino group; a hydroxyimino$(C_1-C_6)$alkyl group; a $(C_1-C_6)$alkoxyimino$(C_1-C_6)$alkyl group; a $(C_2-C_6)$alkenyloxyimino$(C_1-C_6)$alkyl group; a phenyl group; a substituted phenyl group having at least one substituent which may be the same or different and is selected from the group consisting of a halogen atom, a cyano group, a nitro group, a hydroxyl group, an amino group, a mercapto group,

a $(C_1-C_6)$alkyl group, a halo$(C_1-C_6)$alkyl group, a $(C_2-C_6)$alkenyl group, a halo$(C_2-C_6)$-alkenyl group, a $(C_2-C_6)$alkynyl group, a halo$(C_2-C_6)$-alkynyl group, a $(C_1-C_6)$alkoxyl group, a halo$(C_1-C_6)$-alkoxyl group, a $(C_1-C_6)$alkylthio group, a halo$(C_1-C_6)$-alkylthio group, a $(C_1-C_6)$alkylsulfinyl group, a halo$(C_1-C_6)$alkylsulfinyl group, a $(C_1-C_6)$alkylsulfonyl group, a halo$(C_1-C_6)$alkylsulfonyl group, a mono$(C_1$-C6)alkylamino group, a di$(C_1-C_6)$alkylamino group whose $(C_1-C_6)$alkyl groups may be the same or different, a methylenedioxy group, a -$COZ_m(R^6)$ group (wherein, $R^6$, Z, and m are the same as described above), a -$ZCOZ_m(R^6)$ group (wherein, $R^6$, Z, and m are the same as described above), a $(C_1-C_6)$alkylsulfonylamino group, and a halo$(C_1-C_6)$alkylsulfonylamino group; a phenyl$(C_1-C_6)$alkyl group; a substituted phenyl$(C_1-C_6)$alkyl group having on the ring at least one substituent which may be the same or different and is selected from the group consisting of a halogen atom, a cyano group, a nitro group, a hydroxyl group, an amino group, a mercapto group, a $(C_1-C_6)$alkyl group, a halo$(C_1-C_6)$alkyl group, a $(C_2-C_6)$alkenyl group, a halo$(C_2-C_6)$alkenyl group, a $(C_2-C_6)$alkynyl group, a halo$(C_2-C_6)$alkynyl group, a $(C_1-C_6)$alkoxyl group, a halo$(C_1-C_6)$alkoxyl group, a $(C_1-C_6)$alkylthio group, a halo$(C_1-C_6)$alkylthio group, a $(C_1-C_6)$alkylsulfinyl group, a halo$(C_1-C_6)$alkylsulfinyl group, a $(C_1-C_6)$alkylsulfonyl group, a halo$(C_1-C_6)$alkylsulfonyl group, a mono $(C_1-C_6)$alkylamino group, a di$(C_1-C_6)$alkylamino group whose $(C_1-C_6)$alkyl groups may be the same or different, a -$COZ_m(R^6)$ group (wherein, $R^6$, Z, and m are the same as described above), a -$ZCOZ_m(R^6)$ group (wherein, $R^6$, Z, and m are the same as described above), a $(C_1-C_6)$alkylsulfonylamino group, and a halo$(C_1-C_6)$alkylsulfonylamino group; a naphthyl group; a substituted naphthyl group having at least one substituent which may be the same or different and is selected from the group consisting of a halogen atom, a cyano group, a nitro group, a hydroxyl group, an amino group, a mercapto group, a $(C_1-C_6)$alkyl group, a halo$(C_1-C_6)$alkyl group, a $(C_2-C_6)$alkenyl group, a halo$(C_2-C_6)$alkenyl group, a $(C_2-C_6)$alkynyl group, a halo$(C_2-C_6)$alkynyl group, a $(C_1-C_6)$alkoxyl group, a halo $(C_1-C_6)$alkoxyl group, a $(C_1-C_6)$alkylthio group, a halo$(C_1-C_6)$alkylthio group, a $(C_1-C_6)$alkylsulfinyl group, a halo $(C_1-C_6)$alkylsulfinyl group, a $(C_1-C_6)$alkylsulfonyl group, a halo$(C_1-C_6)$alkylsulfonyl group, a mono$(C_1$-C6)alkylamino group, a di$(C_1-C_6)$alkylamino group whose $(C_1-C_6)$alkyl groups may be the same or different, a -$COZ_m(R^6)$ group (wherein, $R^6$, Z, and m are the same as described above), a -$ZCOZ_m(R^6)$ group (wherein, $R^6$, Z, and m are the same as described above), a $(C_1-C_6)$alkylsulfonylamino group, and a halo$(C_1-C_6)$alkylsulfonylamino group; a heterocyclic group (a heterocyclic ring is the same as described above); a substituted heterocyclic group (a heterocyclic ring is the same as described above) having at least one substituent which may be the same or different and is selected from the group consisting of a halogen atom, a cyano group, a nitro group, a hydroxyl group, an amino group, a mercapto group, a $(C_1-C_6)$alkyl group, a halo$(C_1-C_6)$alkyl group, a $(C_2-C_6)$alkenyl group, a halo $(C_2-C_6)$alkenyl group, a $(C_2-C_6)$alkynyl group, a halo$(C_2-C_6)$alkynyl group, a $(C_1-C_6)$alkoxyl group, a halo$(C_1-C_6)$alkoxyl group, a $(C_1-C_6)$alkylthio group, a halo$(C_1-C_6)$alkylthio group, a $(C_1-C_6)$alkylsulfinyl group, a halo$(C_1-C_6)$alkylsulfinyl group, a $(C_1-C_6)$alkylsulfonyl group, a halo$(C_1-C_6)$alkylsulfonyl group, a mono$(C_1-C_6)$alkylamino group, a di$(C_1-C_6)$alkylamino group whose $(C_1-C_6)$alkyl groups may be the same or different, a -$COZ_m(R^6)$ group (wherein, $R^6$, Z, and m are the same as described above), a -$ZCOZ_m(R^6)$ group (wherein, $R^6$, Z, and m are the same as described above), a $(C_1-C_6)$alkylsulfonylamino group, and a halo$(C_1-C_6)$alkylsulfonylamino group; a heterocyclic-$(C_1-C_6)$alkyl group; a substituted heterocyclic-$(C_1-C_6)$alkyl group (a heterocyclic ring is the same as described above) having at least one substituent which may be the same or different and is selected from the group consisting of same or different a halogen atom, a cyano group, a nitro group, a hydroxyl group, an amino group, a mercapto group, a $(C_1-C_6)$alkyl group, a halo-$(C_1-C_6)$alkyl group, a $(C_2-C_6)$alkenyl group, a halo-$(C_2-C_6)$alkenyl group, a $(C_2-C_6)$alkynyl group, a halo-$(C_2-C_6)$alkynyl group, a $(C_1-C_6)$alkoxyl group, a halo$(C_1-C_6)$alkoxyl group, a $(C_1-C_6)$alkylthio group, a halo $(C_1-C_6)$alkylthio group, a $(C_1-C_6)$alkylsulfinyl group, a halo$(C_1-C_6)$alkylsulfinyl group, a $(C_1-C_6)$alkylsulfonyl group, a halo$(C_1-C_6)$alkylsulfonyl group, a mono$(C_1-C_6)$-alkylamino group, a di $(C_1-C_6)$alkylamino group whose $(C_1-C_6)$alkyl groups may be the same or different, a $(C_1-C_6)$alkylsulfonylamino group, and a halo$(C_1-C_6)$-alkylsulfonylamino group; or -Q-Ar (wherein, Q and Ar are the same as described above);

A represents an oxygen atom; a sulfur atom; N=; $N(R^7)$ (wherein, $R^7$ is the same as described above); or C $(R^9)$ [wherein, $R^9$ represents a hydrogen atom; a halogen atom; a $(C_1-C_6)$alkyl group; a halo$(C_1-C_6)$alkyl group; a $(C_2-C_6)$alkenyl group; a halo$(C_2-C_6)$alkenyl group; a $(C_2-C_6)$alkynyl group; a halo$(C_2-C_6)$alkynyl group; a $(C_1-C_6)$alkoxy$(C_1-C_6)$alkyl group; a halo$(C_1-C_6)$alkoxy$(C_1-C_6)$alkyl group; a $(C_1-C_6)$alkylthio$(C_1-C_6)$-alkyl group; a halo $(C_1-C_6)$alkylthio$(C_1-C_6)$alkyl group; a $(C_1-C_6)$alkylsulfinyl$(C_1-C_6)$alkyl group; a halo$(C_1-C_6)$alkylsulfinyl$(C_1-C_6)$alkyl group; a $(C_1-C_6)$alkylsulfonyl$(C_1-C_6)$alkyl group; a halo$(C_1-C_6)$alkylsulfonyl$(C_1-C_6)$alkyl group; a mono$(C_1-C_6)$alkylamino$(C_1-C_6)$alkyl group; a di $(C_1-C_6)$alkylamino$(C_1-C_6)$alkyl group whose $(C_1-C_6)$alkyl groups may be the same or different; a $(C_3-C_6)$cycloalkyl group; a halo$(C_3-C_6)$cycloalkyl group; a $(C_3-C_6)$cycloalkenyl group; or a halo $(C_3-C_6)$cycloalkenyl group]; and

B represents N=; $N(R^8)$ (wherein, $R^8$ is the same as described above); or $C(R^8)$ (wherein, $R^8$ is the same as described above);

(iii) a naphthyl group;

(iv) a substituted naphthyl group having at least one substituent which may be the same or different and is selected from the group consisting of a halogen atom, a cyano group, a nitro group, a hydroxyl group, an amino group, a

mercapto group, a $(C_1-C_6)$alkyl group, a halo$(C_1-C_6)$alkyl group, a $(C_2-C_6)$alkenyl group, a halo(C2-$C_6$)alkenyl group, a $(C_2-C_6)$alkynyl group, a halo($C_2$-C6)alkynyl group, a $(C_1-C_6)$alkoxyl group, a halo$(C_1-C_6)$alkoxyl group, a $(C_1-C_6)$alkylthio group, a halo$(C_1-C_6)$alkylthio group, a $(C_1-C_6)$alkylsulfinyl group, a halo $(C_1-C_6)$alkylsulfinyl group, a $(C_1-C_6)$alkylsulfonyl group, a halo$(C_1-C_6)$alkylsulfonyl group, a mono$(C_1-C_6)$alkylamino group, a di$(C_1-C_6)$ alkylamino group whose $(C_1-C_6)$alkyl groups may be the same or different, a -$COZ_m(R^6)$ group (wherein, $R^6$, Z, and m are the same as described above), a -$ZCOZ_m(R^6)$ group (wherein, $R^6$, Z, and m are the same as described above), a $(C_1-C_6)$alkylsulfonylamino group, and a halo$(C_1-C_6)$alkylsulfonylamino group;

(v) a heterocyclic group (a heterocyclic ring is the same as described above); or

(vi) a substituted heterocyclic group (a heterocyclic ring is the same as described above) having at least one substituent which may be the same or different and is selected from the group consisting of a halogen atom; a cyano group; a nitro group; a hydroxyl group; a mercapto group; an amino group; a halo$(C_1-C_6)$alkyl group (with the proviso of excluding a trifluoromethyl group); a $(C_2-C_6)$alkenyl group; a halo$(C_2-C_6)$alkenyl group; a $(C_2-C_6)$ alkynyl group; a halo$(C_2-C_6)$alkynyl group; a halo$(C_1-C_6)$alkoxyl group; a halo$(C_1-C_6)$alkylthio group; a $(C_1-C_6)$ alkylsulfinyl group; a halo$(C_1-C_6)$alkylsulfinyl group; a $(C_1-C_6)$alkylsulfonyl group; a halo$(C_1-C_6)$alkylsulfonyl group; a mono$(C_1-C_6)$alkylamino group; a di$(C_1-C_6)$alkylamino group whose $(C_1-C_6)$alkyl groups may be the same or different; a $(C_1-C_6)$alkoxy$(C_1-C_6)$alkyl group; a halo$(C_1-C_6)$alkoxy$(C_1-C_6)$alkyl group; a $(C_1-C_6)$alkylthio$(C_1-C_6)$ alkyl group; a halo$(C_1-C_6)$-alkylthio $(C_1-C_6)$alkyl group; a $(C_1-C_6)$alkylsulfinyl$(C_1-C_6)$alkyl group; a halo$(C_1-C_6)$ alkylsulfinyl$(C_1-C_6)$alkyl group; a $(C_1-C_6)$alkylsulfonyl $(C_1-C_6)$alkyl group; a halo$(C_1-C_6)$alkylsulfonyl$(C_1-C_6)$alkyl group; a mono$(C_1-C_6)$alkylamino$(C_1-C_6)$alkyl group; a di$(C_1-C_6)$alkylamino-$(C_1-C_6)$alkyl group whose $(C_1-C_6)$alkyl groups may be the same or different; a $(C_3-C_6)$cycloalkyl group; a halo$(C_3-C_6)$cycloalkyl group; a hydroxyimino $(C_1-C_6)$alkyl group; a $(C_1-C_6)$alkoxyimino$(C_1-C_6)$alkyl group; a $(C_2-C_6)$alkenyloxyimino$(C_1-C_6)$alkyl group; a -$COZ_m(R^6)$ group (wherein, $R^6$, Z, and m are the same as described above); a -$ZCOZ_m(R^6)$ group (wherein, $R^6$, Z, and m are the same as described above); a $(C_1-C_6)$alkylsulfonylamino group; a halo$(C_1-C_6)$alkylsulfonylamino group; a phenyl group; a substituted phenyl group having at least one substituent which may be the same or different and is selected from the group consisting of an amino group, a mercapto group, a $(C_1-C_6)$alkyl group, a halo$(C_1-C_6)$alkyl group (with the proviso of excluding a trifluoromethyl group), a $(C_2-C_6)$alkenyl group, a halo$(C_2-C_6)$ alkenyl group, a $(C_2-C_6)$alkynyl group, a halo$(C_2-C_6)$alkynyl group, a $(C_1-C_6)$alkoxyl group, a halo$(C_1-C_6)$alkoxyl group, a $(C_1-C_6)$alkylthio group, a halo$(C_1-C_6)$alkylthio group, a $(C_1-C_6)$alkylsulfinyl group, a halo$(C_1-C_6)$alkylsulfinyl group, a $(C_1-C_6)$alkylsulfonyl group, a halo$(C_1-C_6)$alkylsulfonyl group, a mono$(C_1-C_6)$alkylamino group, a di $(C_1-C_6)$alkylamino group whose $(C_1-C_6)$alkyl groups may be the same or different, a -$COZ_m(R^6)$ group (wherein, $R^6$, Z, and m are the same as described above), a -$ZCOZ_m(R^6)$ group (wherein, $R^6$, Z, and m are the same as described above), a $(C_1-C_6)$alkylsulfonylamino group, and a halo$(C_1-C_6)$alkylsulfonylamino group; a naphthyl group; a substituted naphthyl group having at least one substituent which may be the same or different and is selected from the group consisting of a halogen atom, a cyano group, a nitro group, a hydroxyl group, an amino group, a mercapto group, a $(C_1-C_6)$alkyl group, a halo$(C_1-C_6)$alkyl group, a $(C_2-C_6)$alkenyl group, a halo$(C_2-C_6)$ alkenyl group, a $(C_2-C_6)$alkynyl group, a halo$(C_2-C_6)$-alkynyl group, a $(C_1-C_6)$alkoxyl group, a halo($C_1$-C6)alkoxyl group, a $(C_1-C_6)$alkylthio group, a halo$(C_1-C_6)$alkylthio group, a $(C_1-C_6)$alkylsulfinyl group, a halo$(C_1-C_6)$alkylsulfinyl group, a $(C_1-C_6)$alkylsulfonyl group, a halo$(C_1-C_6)$alkylsulfonyl group, a mono$(C_1-C_6)$alkylamino group, a di $(C_1-C_6)$alkylamino group whose $(C_1-C_6)$alkyl groups may be the same or different, a -$COZ_m(R^6)$ group (wherein, $R^6$, Z, and m are the same as described above), a -$ZCOZ_m(R^6)$ group (wherein, $R^6$, Z, and m are the same as described above), a $(C_1-C_6)$alkylsulfonylamino group, and a halo$(C_1-C_6)$alkylsulfonylamino group; a heterocyclic group (a heterocyclic ring is the same as described above); a substituted heterocyclic group (a heterocyclic ring is the same as described above) having at least one substituent which may be the same or different and is selected from the group consisting of a halogen atom, a cyano group, a nitro group, a hydroxyl group, an amino group, a mercapto group, a $(C_1-C_6)$alkyl group, a halo$(C_1-C_6)$alkyl group, a $(C_2-C_6)$alkenyl group, a halo$(C_2-C_6)$alkenyl group, a $(C_2-C_6)$alkynyl group, a halo$(C_2-C_6)$alkynyl group, a $(C_1-C_6)$alkoxyl group, a halo$(C_1-C_6)$alkoxyl group, a $(C_1-C_6)$alkylthio group, a halo$(C_1-C_6)$alkylthio group, a $(C_1-C_6)$alkylsulfinyl group, a halo$(C_1-C_6)$alkylsulfinyl group, a $(C_1-C_6)$alkylsulfonyl group, a halo$(C_1-C_6)$alkylsulfonyl group, a mono $(C_1-C_6)$alkylamino group, a di$(C_1-C_6)$ alkylamino group whose $(C_1-C_6)$alkyl groups may be the same or different, a -$COZ_m(R^6)$ group (wherein, $R^6$, Z, and m are the same as described above), a -$ZCOZ_m(R^6)$ group (wherein, $R^6$, Z, and m are the same as described above), a $(C_1-C_6)$alkylsulfonylamino group, and a halo$(C_1-C_6)$alkylsulfonylamino group; and -Q-Ar (wherein, Q and Ar are the same as described above); Y represents a $(C_1-C_6)$alkylene group; a $(C_1-C_6)$alkyl$(C_1-C_6)$alkylene group; a halo$(C_1-C_6)$alkyl$(C_1-C_6)$alkylene group; a hydroxy$(C_1-C_6)$alkyl$(C_1-C_6)$alkylene group; a $(C_1-C_6)$alkoxy $(C_1-C_6)$alkyl$(C_1-C_6)$alkylene group; a $(C_2-C_6)$alkenyl$(C_1-C_6)$alkylene group; a halo$(C_2-C_6)$alkenyl$(C_1-C_6)$alkylene group; a $(C_2-C_6)$alkynyl$(C_1-C_6)$alkylene group; a halo$(C_2-C_6)$alkynyl$(C_1-C_6)$alkylene group; a hydroxy$(C_1-C_6)$ alkylene group; a $(C_1-C_6)$alkoxy $(C_1-C_6)$alkylene group; a halo$(C_1-C_6)$alkoxy$(C_1-C_6)$alkylene group; a $(C_1-C_6)$alkylcarbonyloxy$(C_1-C_6)$alkylene group; a cyano$(C_1-C_6)$alkylene group; a halo$(C_1-C_6)$alkylene group; a $(C_3-C_6)$cycloalkyl $(C_1-C_6)$alkylene group; a $(C_3-C_6)$cycloalkenyl $(C_1-C_6)$alkylene group; a $(C_1-C_6)$alkylcarbonyl$(C_1-C_6)$

alkylene group; a ($C_1$-$C_6$)alkoxycarbonyl($C_1$-$C_6$)-alkylene group; an amino($C_1$-$C_6$)alkylene group; a mono ($C_1$-$C_6$) alkylamino($C_1$-$C_6$)alkylene group; a di($C_1$-$C_6$)alkylamino($C_1$-$C_6$)alkylene group whose ($C_1$-$C_6$)alkyl groups may be the same or different; a ($C_1$-$C_6$)alkylcarbonylamino($C_1$-$C_6$)alkylene group; a ($C_1$-$C_6$)alkoxycarbonylamino($C_1$-$C_6$) alkylene group; a mercapto($C_1$-$C_6$)alkylene group; a ($C_1$-$C_6$)alkylthio($C_1$-$C_6$)alkylene group; an ($C_1$-$C_6$)alkylsulfinyl ($C_1$-$C_6$)alkylene group; a ($C_1$-$C_6$)alkylsulfonyl($C_1$-$C_6$)alkylene group; a ($C_3$-$C_6$)-cycloalkylene group; a ($C_2$-$C_6$)alkenylene group; a ($C_1$-$C_6$)alkyl($C_2$-$C_6$)alkenylene group; a halo($C_1$-$C_6$)alkyl($C_2$-$C_6$)alkenylene group; a hydroxy ($C_1$-$C_6$)alkyl($C_2$-$C_6$)alkenylene group; a ($C_1$-$C_6$)alkoxy($C_1$-$C_6$)alkyl($C_2$-$C_6$)alkenylene group; a ($C_2$-$C_6$)alkenyl ($C_2$-$C_6$)alkenylene group; a halo($C_2$-$C_6$)alkenyl($C_2$-$C_6$)alkenylene group; a ($C_2$-$C_6$)alkynyl($C_2$-$C_6$)alkenylene group; a halo($C_2$-$C_6$)-alkynyl($C_2$-$C_6$)alkenylene group; a hydroxy($C_2$-$C_6$)alkenylene group; a ($C_1$-$C_6$)alkoxy($C_2$-$C_6$) alkenylene group; a halo($C_1$-$C_6$)alkoxy($C_2$-$C_6$)alkenylene group; a ($C_1$-$C_6$)alkylcarbonyloxy($C_2$-$C_6$)alkenylene group; a cyano($C_2$-$C_6$)alkenylene group; a halo($C_2$-$C_6$)alkenylene group; a ($C_3$-$C_6$)cycloalkyl($C_2$-$C_6$)alkenylene group; a ($C_3$-$C_6$)cycloalkenyl($C_2$-$C_6$)alkenylene group; a ($C_1$-$C_6$)alkylcarbonyl($C_2$-$C_6$)alkenylene group; a ($C_1$-$C_6$) alkoxylcarbonyl ($C_2$-$C_6$)alkenylene group; an amino($C_2$-$C_6$)alkenylene group; a mono($C_1$-$C_6$)alkylamino($C_2$-$C_6$) alkenylene group; a di ($C_1$-$C_6$)alkylamino ($C_2$-$C_6$)-alkenylene group whose ($C_1$-$C_6$)alkyl groups may be the same or different; a ($C_1$-$C_6$)alkylcarbonylamino($C_2$-C6)alkenylene group; a ($C_1$-$C_6$)alkoxycarbonylamino($C_2$-$C_6$)-alkenylene group; a mercapto($C_2$-$C_6$)alkenylene group; a ($C_1$-$C_6$)alkylthio ($C_2$-$C_6$)alkenylene group; a ($C_1$-$C_6$)alkylsulfinyl($C_2$-$C_6$)alkenylene group; a ($C_1$-$C_6$)-alkylsulfonyl($C_2$-$C_6$)alkenylene group; a ($C_2$-$C_6$)alkynylene group; a ($C_1$-$C_6$)alkyleneoxy group; a ($C_1$-$C_6$)alkyl($C_1$-C6)alkyleneoxy group; a ($C_1$-$C_6$)alkylenethio group; a ($C_1$-$C_6$)alkyl ($C_1$-$C_6$)alkylenethio group; a ($C_1$-$C_6$)alkyleneamino group; a ($C_1$-$C_6$)alkyl($C_1$-$C_6$)alkyleneamino group; a carbonyl group; a carbonyl($C_1$-$C_6$)alkylene group; a hydroxyimino group; a hydroxyimino($C_1$-$C_6$)alkylene group; a ($C_1$-$C_6$) alkoxyimino group; a ($C_1$-$C_6$)alkoxyimino($C_1$-$C_6$)alkylene group; or a ($C_1$-$C_6$)alkylenedioxy($C_1$-$C_6$)alkylene group;

with the proviso that when $R^5$ represents the formula (b), then Y excludes a ($C_1$-$C_6$)alkyleneoxy group; and when $R^5$ represents the formula (a), $R^1$ represents a hydrogen atom or a ($C_1$-$C_6$)alkyl group, and $R^3$ represents a carbamoyl group and a nitrile group, then X represents -Q-Ar [wherein, Q and Ar are the same as described above, but Ar excludes a heterocyclic group (a heterocyclic group is the same as described above), or a substituted heterocyclic group (a heterocyclic group is the same as described above).

**2.** The N-(4-pyrazolyl)amide derivative according to Claim 1 represented by the general formula (I-1):

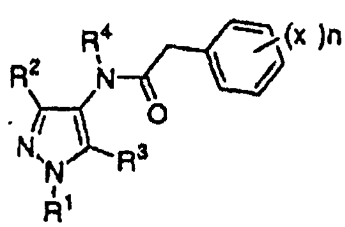

(I-1)

wherein $R^1$ represents a hydrogen atom; a ($C_1$-$C_6$)alkyl group; a halo($C_1$-$C_6$)alkyl group; a hydroxy($C_1$-$C_6$)alkyl group; a cyano($C_1$-$C_6$)alkyl group; a formyl($C_1$-$C_6$)alkyl group; a ($C_2$-$C_6$)alkenyl group; a halo($C_2$-$C_6$)alkenyl group; a ($C_2$-$C_6$)alkynyl group; a halo($C_2$-$C_6$)alkynyl group; a ($C_1$-$C_6$)alkoxy($C_1$-$C_6$)alkyl group; a halo($C_1$-$C_6$)alkoxy ($C_1$-$C_6$)alkyl group; a ($C_1$-$C_6$)alkylthio($C_1$-$C_6$)alkyl group; a halo($C_1$-$C_6$)alkylthio($C_1$-$C_6$)alkyl group; a ($C_1$-$C_6$)alkylsulfinyl($C_1$-$C_6$)alkyl group; a halo-($C_1$-$C_6$)alkylsulfinyl ($C_1$-$C_6$)alkyl group; a ($C_1$-$C_6$)alkylsulfonyl($C_1$-$C_6$)alkyl group; a halo($C_1$-$C_6$)alkylsulfonyl-($C_1$-$C_6$)alkyl group; a mono($C_1$-$C_6$)alkylamino($C_1$-$C_6$)alkyl group; a di($C_1$-$C_6$)alkylamino ($C_1$-$C_6$)alkyl group whose ($C_1$-$C_6$)alkyl groups may be the same or different; a ($C_1$-$C_6$)alkylsulfonyl group; a halo ($C_1$-$C_6$)alkylsulfonyl group; a mono($C_1$-$C_6$)alkylaminosulfonyl group; a di($C_1$-$C_6$)alkylaminosulfonyl group whose ($C_1$-$C_6$)alkyl groups may be the same or different; a ($C_1$-$C_6$)alkyloxycarbonyl-($C_1$-$C_6$)alkyl group; a mono ($C_1$-$C_6$) alkylaminocarbonyl-($C_1$-$C_6$)alkyl group; a di($C_1$-$C_6$)alkylaminocarbonyl-($C_1$-$C_6$)alkyl group whose ($C_1$-$C_6$)alkyl groups may be the same or different; a ($C_1$-$C_6$)alkyloxyimino($C_1$-$C_6$)alkyl group; a ($C_1$-$C_6$)alkylsulfonyloxy($C_1$-$C_6$) alkyl group; a halo ($C_1$-$C_6$)alkylsulfonyloxy($C_1$-$C_6$)alkyl group; a ($C_1$-$C_6$)alkylcarbonyloxy($C_1$-$C_6$)alkyl group; a phenylsulfonyl group; a substituted phenylsulfonyl group having at least one substituent which may be the same or different and is selected from the group consisting of a halogen atom, a cyano group, a nitro group, a hydroxyl group, an amino group, a mercapto group, a ($C_1$-$C_6$)alkyl group, a halo($C_1$-$C_6$)alkyl group, a ($C_2$-$C_6$)alkenyl group, a halo($C_2$-$C_6$)alkenyl group, a ($C_2$-$C_6$)alkynyl group, a halo($C_2$-$C_6$)alkynyl group, a ($C_1$-$C_6$)alkoxyl group, a halo ($C_1$-$C_6$)alkoxyl group, a ($C_1$-$C_6$)alkylthio group, a halo($C_1$-$C_6$)alkylthio group, a ($C_1$-$C_6$)alkylsulfinyl group, a halo ($C_1$-$C_6$)alkylsulfinyl group, a ($C_1$-$C_6$)alkylsulfonyl group, a halo($C_1$-$C_6$)-alkylsulfonyl group, a mono($C_1$-$C_6$)alkylamino group, a di($C_1$-$C_6$)alkylamino group whose ($C_1$-$C_6$)alkyl groups may be the same or different, a ($C_1$-$C_6$)alkyl-

sulfonylamino group, and a halo($C_1$-$C_6$)alkylsulfonylamino group; a ($C_3$-$C_6$)cycloalkyl group; a halo($C_3$-$C_6$)cycloalkyl group; a ($C_3$-$C_6$)cycloalkenyl group; a halo($C_3$-$C_6$)cycloalkenyl group; a phenyl group; a substituted phenyl group having at least one substituent which may be the same or different and is selected from the group consisting of a halogen atom, a cyano group, a nitro group, a hydroxyl group, an amino group, a mercapto group, a ($C_1$-$C_6$) alkyl group, a halo($C_1$-$C_6$)alkyl group, a ($C_2$-$C_6$)alkenyl group, a halo($C_2$-$C_6$)alkenyl group, a ($C_2$-$C_6$)alkynyl group, a halo($C_2$-$C_6$)alkynyl group, a ($C_1$-$C_6$)alkoxyl group, a halo($C_1$-$C_6$)alkoxyl group, a ($C_1$-$C_6$)alkylthio group, a halo ($C_1$-$C_6$)alkylthio group, a ($C_1$-$C_6$)alkylsulfinyl group, a halo($C_1$-$C_6$)alkylsulfinyl group, a ($C_1$-$C_6$)alkylsulfonyl group, a halo($C_1$-$C_6$)alkylsulfonyl group, a mono($C_1$-$C_6$)alkylamino group, a di($C_1$-$C_6$)alkylamino group whose ($C_1$-$C_6$) alkyl groups may be the same or different, a ($C_1$-$C_6$)alkylsulfonylamino group, and a halo($C_1$-$C_6$)alkylsulfonylamino group; or a -$COZ_m(R^6)$ group {wherein, $R^6$ represents a hydrogen atom; a ($C_1$-$C_6$)alkyl group; a halo($C_1$-$C_6$)alkyl group; a ($C_2$-C6)alkenyl group; a halo($C_2$-$C_6$)alkenyl group; a ($C_2$-$C_6$)alkynyl group; a halo($C_2$-$C_6$)alkynyl group; a ($C_1$-$C_6$)alkoxy ($C_1$-$C_6$)alkyl group; a halo ($C_1$-$C_6$)alkoxy ($C_1$-$C_6$)alkyl group; a ($C_1$-$C_6$)alkylthio($C_1$-$C_6$)alkyl group; a halo ($C_1$-$C_6$) alkylthio ($C_1$-$C_6$) alkyl group; a ($C_1$-$C_6$)-alkylsulfinyl($C_1$-$C_6$)alkyl group; a halo($C_1$-$C_6$)alkylsulfinyl ($C_1$-$C_6$)alkyl group; a ($C_1$-$C_6$)alkylsulfonyl($C_1$-$C_6$)alkyl group; a halo($C_1$-$C_6$)alkylsulfonyl($C_1$-$C_6$)alkyl group; a mono ($C_1$-$C_6$)-alkylamino($C_1$-$C_6$)alkyl group; a di ($C_1$-$C_6$)alkylamino($C_1$-$C_6$)alkyl group whose ($C_1$-$C_6$)alkyl groups may be the same or different; a ($C_3$-$C_6$)cycloalkyl group; a halo($C_3$-C6)cycloalkyl group; a ($C_3$-$C_6$)cycloalkenyl group; a halo($C_3$-$C_6$)cycloalkenyl group; a phenyl group; or a substituted phenyl group having at least one substituent which may be the same or different and is selected from the group consisting of a halogen atom, a cyano group, a nitro group, a hydroxyl group, an amino group, a mercapto group, a ($C_1$-$C_6$)alkyl group, a halo($C_1$-$C_6$)alkyl group, a ($C_2$-$C_6$)alkenyl group, a halo($C_2$-$C_6$)alkenyl group, a ($C_2$-$C_6$)alkynyl group, a halo($C_2$-$C_6$)alkynyl group, a ($C_1$-$C_6$) alkoxyl group, a halo($C_1$-$C_6$)alkoxyl group, a ($C_1$-$C_6$)alkylthio group, a halo($C_1$-$C_6$)alkylthio group, a ($C_1$-$C_6$)alkylsulfinyl group, a halo($C_1$-$C_6$)alkylsulfinyl group, a ($C_1$-$C_6$)alkylsulfonyl group, a halo($C_1$-$C_6$)alkylsulfonyl group, a mono($C_1$-C6)alkylamino group, a di($C_1$-$C_6$)alkylamino group whose ($C_1$-$C_6$)alkyl groups may be the same or different, a ($C_1$-C6)alkylsulfonylamino group, and a halo($C_1$-$C_6$)-alkylsulfonylamino group; Z represents an oxygen atom; a sulfur atom; or $N(R^7)$ [wherein, $R^7$ represents a hydrogen atom; a ($C_1$-$C_6$)alkyl group; a halo($C_1$-$C_6$)alkyl group; a ($C_2$-$C_6$)alkenyl group; a halo($C_2$-$C_6$)alkenyl group; a ($C_2$-$C_6$)alkynyl group; a halo($C_2$-$C_6$)alkynyl group; a ($C_1$-$C_6$)alkoxy ($C_1$-$C_6$)alkyl group; a halo($C_1$-$C_6$)alkoxy($C_1$-$C_6$)alkyl group; a ($C_1$-$C_6$)alkylthio ($C_1$-$C_6$)alkyl group; a halo ($C_1$-$C_6$)alkylthio($C_1$-$C_6$)alkyl group; a ($C_1$-$C_6$)alkylsulfinyl($C_1$-$C_6$)alkyl group; a halo($C_1$-$C_6$)alkylsulfinyl ($C_1$-$C_6$)alkyl group; a ($C_1$-$C_6$)alkylsulfonyl($C_1$-$C_6$)alkyl group; a halo($C_1$-$C_6$)alkylsulfonyl ($C_1$-$C_6$)alkyl group; a mono ($C_1$-$C_6$)alkylamino($C_1$-$C_6$)alkyl group; a di ($C_1$-$C_6$)alkylamino($C_1$-$C_6$)alkyl group whose ($C_1$-$C_6$)alkyl groups may be the same or different; a ($C_3$-$C_6$)cycloalkyl group; a halo($C_3$-$C_6$)cycloalkyl group; a ($C_3$-$C_6$)cycloalkenyl group; or a halo($C_3$-$C_6$)cycloalkenyl group]; and m represents an integer of 0 to 1};

$R^2$ and $R^3$ may be the same or different, and represent a hydrogen atom, a halogen atom; a cyano group; a nitro group; a hydroxyl group; a mercapto group; an amino group; a ($C_1$-$C_6$)alkyl group; a halo($C_1$-$C_6$)alkyl group; a ($C_2$-$C_6$)alkenyl group; a halo($C_2$-$C_6$)alkenyl group; a ($C_2$-$C_6$)alkynyl group; a halo($C_2$-$C_6$)alkynyl group; a ($C_1$-$C_6$) alkoxyl group; a halo($C_1$-C6)alkoxyl group; a ($C_1$-$C_6$)alkylthio group; a halo($C_1$-$C_6$)alkylthio group; a ($C_1$-$C_6$)alkylsulfinyl group; a halo($C_1$-$C_6$)alkylsulfinyl group; a ($C_1$-$C_6$)alkylsulfonyl group; a halo($C_1$-$C_6$)alkylsulfonyl group; a mono($C_1$-$C_6$)alkylamino group; a di($C_1$-$C_6$)alkylamino group whose ($C_1$-$C_6$)alkyl groups may be the same or different; a ($C_1$-$C_6$)alkoxy ($C_1$-$C_6$)alkyl group; a halo($C_1$-$C_6$)alkoxy($C_1$-$C_6$)alkyl group; a ($C_1$-$C_6$)alkylthio ($C_1$-$C_6$)alkyl group; a halo($C_1$-$C_6$)alkylthio ($C_1$-$C_6$)alkyl group; a ($C_1$-$C_6$)alkylsulfinyl ($C_1$-$C_6$)alkyl group; a halo($C_1$-$C_6$)alkylsulfinyl-($C_1$-$C_6$)alkyl group; a ($C_1$-$C_6$) alkylsulfonyl ($C_1$-$C_6$)alkyl group; a halo($C_1$-$C_6$)alkylsulfonyl($C_1$-$C_6$)alkyl group; a mono ($C_1$-$C_6$)alkylamino($C_1$-$C_6$)alkyl group; a di ($C_1$-$C_6$)alkylamino($C_1$-$C_6$)alkyl group whose ($C_1$-$C_6$)alkyl groups may be the same or different; a ($C_3$-$C_6$)cycloalkyl group; a halo($C_3$-$C_6$)cycloalkyl group; a ($C_3$-$C_6$)cycloalkenyl group; a halo($C_3$-$C_6$)cycloalkenyl group; a phenyl group; a substituted phenyl group having at least one substituent which may be the same or different and is selected from the group consisting of a halogen atom, a cyano group, a nitro group, a hydroxyl group, an amino group, a mercapto group, a ($C_1$-$C_6$)alkyl group, a halo($C_1$-$C_6$)alkyl group, a ($C_2$-$C_6$)alkenyl group, a halo($C_2$-$C_6$)alkenyl group, a ($C_2$-$C_6$)alkynyl group, a halo($C_2$-$C_6$)alkynyl group, a ($C_1$-$C_6$) alkoxyl group, a halo($C_1$-C6)alkoxyl group, a ($C_1$-$C_6$)alkylthio group, a halo($C_1$-$C_6$)alkylthio group, a ($C_1$-$C_6$)alkylsulfinyl group, a halo($C_1$-$C_6$)alkylsulfinyl group, a ($C_1$-$C_6$)alkylsulfonyl group, a halo($C_1$-$C_6$)alkylsulfonyl group, a mono($C_1$-$C_6$)alkylamino group, a di($C_1$-$C_6$)alkylamino group whose ($C_1$-$C_6$)alkyl groups may be the same or different, a ($C_1$-$C_6$)alkylsulfonylamino group, and a halo($C_1$-$C_6$)alkylsulfonylamino group; a phenoxy group; a substituted phenoxy group having at least one substituent which may be the same or different and is selected from the group consisting of a halogen atom, a cyano group, a nitro group, a hydroxyl group, an amino group, a mercapto group, a ($C_1$-$C_6$)alkyl group, a halo($C_1$-$C_6$)alkyl group, a ($C_2$-$C_6$)alkenyl group, a halo($C_2$-$C_6$)alkenyl group, a ($C_2$-$C_6$)alkynyl group, a halo($C_2$-$C_6$)alkynyl group, a ($C_1$-$C_6$)alkoxyl group, a halo($C_1$-$C_6$)alkoxyl group, a ($C_1$-$C_6$) alkylthio group, a halo($C_1$-$C_6$)alkylthio group, a ($C_1$-$C_6$)alkylsulfinyl group, a halo($C_1$-$C_6$)alkylsulfinyl group, a ($C_1$-$C_6$)alkylsulfonyl group, a halo($C_1$-$C_6$)alkylsulfonyl group, a mono($C_1$-$C_6$)alkylamino group, a di($C_1$-$C_6$) alkylamino group whose ($C_1$-$C_6$)alkyl groups may be the same or different, a ($C_1$-$C_6$)alkylsulfonylamino group,

and a halo($C_1$-$C_6$)-alkylsulfonylamino group; a phenylthio group; a substituted phenylthio group having at least one substituent which may be the same or different and is selected from the group consisting of a halogen atom, a cyano group, a nitro group, a hydroxyl group, an amino group, a mercapto group, a ($C_1$-$C_6$)alkyl group, a halo ($C_1$-$C_6$)alkyl group, a ($C_2$-$C_6$)alkenyl group, a halo($C_2$-$C_6$)alkenyl group, a ($C_2$-$C_6$)alkynyl group, a halo($C_2$-$C_6$) alkynyl group, a ($C_1$-$C_6$)alkoxyl group, a halo($C_1$-$C_6$)alkoxyl group, a ($C_1$-$C_6$)alkylthio group, a halo($C_1$-$C_6$)alkylthio group, a ($C_1$-$C_6$)alkylsulfinyl group, a halo($C_1$-$C_6$)alkylsulfinyl group, a ($C_1$-$C_6$)alkylsulfonyl group, a halo($C_1$-$C_6$) alkylsulfonyl group, a mono($C_1$-$C_6$)alkylamino group, a di($C_1$-$C_6$)alkylamino group whose ($C_1$-$C_6$)alkyl groups may be the same or different, a ($C_1$-$C_6$)alkylsulfonyl amino group, and a halo($C_1$-$C_6$)alkylsulfonyl amino group; a -$ZCOZ_m(R^6)$ group (wherein, $R^6$, Z, and m are the same as described above); or a -$COZ_m(R^6)$ group (wherein, $R^6$, Z, and m are the same as described above);

$R^4$ represents a hydrogen atom; a ($C_1$-$C_6$)alkyl group; a halo($C_1$-$C_6$)alkyl group; a cyano($C_1$-$C_6$)alkyl group; a ($C_2$-$C_6$)alkenyl group; a halo($C_2$-$C_6$)alkenyl group; a ($C_2$-$C_6$)alkynyl group; a halo($C_2$-$C_6$)alkynyl group; a ($C_1$-$C_6$) alkoxy($C_1$-$C_6$)alkyl group; a halo($C_1$-$C_6$)alkoxy($C_1$-$C_6$)alkyl group; a ($C_1$-$C_6$)alkoxy($C_1$-$C_6$)-alkoxy ($C_1$-$C_6$)alkyl group; a ($C_1$-$C_6$)alkylthio ($C_1$-$C_6$)alkyl group; a halo($C_1$-$C_6$)alkylthio($C_1$-$C_6$)alkyl group; a ($C_1$-$C_6$)alkylsulfinyl ($C_1$-$C_6$)alkyl group; a halo($C_1$-$C_6$)alkylsulfinyl($C_1$-$C_6$)alkyl group; a ($C_1$-$C_6$)alkylsulfonyl($C_1$-$C_6$)alkyl group; a halo ($C_1$-$C_6$)-alkylsulfonyl($C_1$-$C_6$)alkyl group; a mono($C_1$-$C_6$)alkylamino ($C_1$-$C_6$)alkyl group; a di ($C_1$-$C_6$)alkylamino ($C_1$-$C_6$)alkyl group whose ($C_1$-$C_6$)alkyl groups may be the same or different; a ($C_3$-$C_6$)cycloalkyl group; a halo ($C_3$-$C_6$)cycloalkyl group; a ($C_3$-$C_6$)cycloalkenyl group; a halo($C_3$-$C_6$)cycloalkenyl group; a -$COZ_m(R^6)$ group (wherein, $R^6$, Z, and m are the same as described above), a -$CSNH(R^6)$ group (wherein, $R^6$, is the same as described above), a ($C_1$-$C_6$)alkylcarbonyl($C_1$-$C_6$)alkyl group; a ($C_1$-$C_6$)alkoxycarbonyl($C_1$-$C_6$)alkyl group; a phenyl ($C_1$-$C_6$)alkyl group; a substituted phenyl($C_1$-$C_6$)alkyl group having on the ring at least one substituent which may be the same or different and is selected from the group consisting of a halogen atom, a cyano group, a nitro group, a hydroxyl group, an amino group, a mercapto group, a ($C_1$-$C_6$)alkyl group, a halo($C_1$-$C_6$)alkyl group, a ($C_2$-$C_6$) alkenyl group, a halo($C_2$-$C_6$)alkenyl group, a ($C_2$-$C_6$)alkynyl group, a halo($C_2$-$C_6$)alkynyl group, a ($C_1$-$C_6$)alkoxyl group, a halo($C_1$-$C_6$)alkoxyl group, a ($C_1$-$C_6$)alkylthio group, a halo($C_1$-$C_6$)alkylthio group, a ($C_1$-$C_6$)alkylsulfinyl group, a halo($C_1$-$C_6$)alkylsulfinyl group, a ($C_1$-$C_6$)alkylsulfonyl group, a halo($C_1$-$C_6$)alkylsulfonyl group, a mono ($C_1$-$C_6$)alkylamino group, a di($C_1$-$C_6$)alkylamino group whose ($C_1$-$C_6$)alkyl groups may be the same or different, a ($C_1$-$C_6$)alkylsulfonylamino group, and a halo($C_1$-$C_6$)alkylsulfonylamino group; a heterocyclic($C_1$-$C_6$)alkyl group (a heterocyclic ring represents a pyridyl group; a pyridine-N-oxide group; a pyridazinyl group; a pyrimidinyl group; a pyrazyl group; a piperidyl group; a piperazino group; a morpholinyl group; a morpholino group; a furyl group; a tetrahydrofuryl group; a thienyl group; a tetrahydrothienyl group; a pyrrolyl group; a pyrrolidyl group; an imidazolidinyl group; an oxazolyl group; an isoxazolyl group; an oxadiazolyl group; a thiazolyl group; an isothiazolyl group; a thiadiazolyl group; an imidazolyl group; a triazolyl group; a pyrazolyl group; an indolyl group; a benzofuryl group; a benzothienyl group; an iridazolyl group; a quinolyl group; an isoquinolyl group; or a quinazolyl group); or a substituted heterocyclic($C_1$-$C_6$)alkyl group (the heterocyclic group is the same as described above) having on the ring at least one substituent which may be the same or different and is selected from the group consisting of a halogen atom, a cyano group, a nitro group, a hydroxyl group, an amino group, a mercapto group, a ($C_1$-$C_6$)alkyl group, a halo($C_1$-$C_6$)alkyl group, a ($C_2$-$C_6$)alkenyl group, a halo($C_2$-$C_6$)alkenyl group, a ($C_2$-$C_6$)alkynyl group, a halo($C_2$-$C_6$) alkynyl group, a ($C_1$-$C_6$)alkoxyl group, a halo($C_1$-$C_6$)alkoxyl group, a ($C_1$-$C_6$)alkylthio group, a halo($C_1$-$C_6$)alkylthio group, a ($C_1$-$C_6$)alkylsulfinyl group, a halo($C_1$-$C_6$)alkylsulfinyl group, a ($C_1$-$C_6$)alkylsulfonyl group, a halo($C_1$-$C_6$) alkylsulfonyl group, a mono($C_1$-$C_6$)alkylamino group, a di($C_1$-$C_6$)alkylamino group whose ($C_1$-$C_6$)alkyl groups may be the same or different, a ($C_1$-$C_6$)alkylsulfonylamino group, and a halo($C_1$-$C_6$)alkylsulfonylamino group;

X may be the same or different, and represents a halogen atom; a cyano group; a nitro group; a hydroxyl group; a mercapto group; an amino group; a ($C_1$-$C_6$)alkyl group; a halo($C_1$-$C_6$)alkyl group; a cyano($C_1$-$C_6$)alkyl group; a ($C_2$-$C_6$)alkenyl group; a halo ($C_2$-$C_6$)alkenyl group; a ($C_2$-$C_6$)alkynyl group; a halo($C_2$-$C_6$)alkynyl group; a ($C_1$-$C_6$)alkoxyl group; a halo($C_1$-$C_6$)alkoxyl group; a ($C_1$-$C_6$)alkylthio group; a halo($C_1$-$C_6$)alkylthio group; a ($C_1$-$C_6$)alkylsulfinyl group; a halo($C_1$-$C_6$)alkylsulfinyl group; a ($C_1$-$C_6$)alkylsulfonyl group; a halo($C_1$-$C_6$)alkylsulfonyl group; a mono($C_1$-$C_6$)alkylamino group; a di($C_1$-$C_6$)alkylamino group whose ($C_1$-$C_6$)alkyl groups may be the same or different; a ($C_1$-$C_6$)alkoxy($C_1$-$C_6$)alkyl group; a halo($C_1$-$C_6$)alkoxy($C_1$-$C_6$)alkyl group; a ($C_1$-$C_6$)alkylthio ($C_1$-$C_6$)alkyl group; a halo($C_1$-$C_6$)alkylthio($C_1$-$C_6$)alkyl group; a ($C_1$-$C_6$)alkylsulfinyl($C_1$-$C_6$)alkyl group; a halo ($C_1$-$C_6$)alkylsulfinyl-($C_1$-$C_6$)alkyl group; a ($C_1$-$C_6$)alkylsulfonyl ($C_1$-$C_6$)alkyl group; a halo($C_1$-$C_6$)alkylsulfonyl ($C_1$-$C_6$)alkyl group; a mono($C_1$-$C_6$)alkylamino($C_1$-$C_6$)alkyl group; a di($C_1$-$C_6$)alkylamino($C_1$-$C_6$)alkyl group whose ($C_1$-$C_6$)alkyl groups may be the same or different; a ($C_3$-$C_6$)cycloalkyl group; a halo($C_3$-$C_6$)cycloalkyl group; a ($C_1$-$C_6$)alkylenedioxy group; a ($C_1$-$C_6$)alkyl($C_1$-$C_6$)alkylenedioxy group; a phenyl($C_1$-$C_6$)alkylenedioxy group; a substituted phenyl($C_1$-$C_6$)alkylenedioxy group having on the ring at least one substituent which may be the same or different and is selected from the group consisting of a halogen atom, a cyano group, a nitro group, a hydroxyl group, an amino group, a mercapto group, a ($C_1$-$C_6$)alkyl group, a halo($C_1$-$C_6$)alkyl group, a ($C_2$-$C_6$)alkenyl group, a halo($C_2$-$C_6$)alkenyl group, a ($C_2$-$C_6$)alkynyl group, a halo($C_2$-$C_6$)alkynyl group, a ($C_1$-$C_6$)alkoxyl group, a halo

$(C_1-C_6)$alkoxyl group, a $(C_1-C_6)$alkylthio group, a halo$(C_1-C_6)$alkylthio group, a $(C_1-C_6)$alkylsulfinyl group, a halo $(C_1-C_6)$alkylsulfinyl group, a $(C_1-C_6)$alkylsulfonyl group, a halo$(C_1-C_6)$alkylsulfonyl group, a mono$(C_1-C_6)$alkylamino group, a di$(C_1-C_6)$alkylamino group whose $(C_1-C_6)$alkyl groups may be the same or different, a $(C_1-C_6)$alkylsulfonylamino group, and a halo$(C_1-C_6)$alkylsulfonylamino group; a phenyl$(C_1-C_6)$alkyl$(C_1-C_6)$alkylenedioxy group; a substituted phenyl $(C_1-C_6)$alkyl$(C_1-C_6)$alkylenedioxy group having on the ring at least one substituent which may be the same or different and is selected from the group consisting of a halogen atom, a cyano group, a nitro group, a hydroxyl group, an amino group, a mercapto group, a $(C_1-C_6)$alkyl group, a halo$(C_1-C_6)$alkyl group, a $(C_2-C_6)$alkenyl group, a halo$(C_2-C_6)$alkenyl group, a $(C_2-C_6)$alkynyl group, a halo$(C_2-C_6)$alkynyl group, a $(C_1-C_6)$ alkoxyl group, a halo$(C_1-C_6)$alkoxyl group, a $(C_1-C_6)$alkylthio group, a halo$(C_1-C_6)$alkylthio group, a $(C_1-C6)$alkylsulfinyl group, a halo$(C_1-C_6)$alkylsulfinyl group, a $(C_1-C_6)$alkylsulfonyl group, a halo$(C_1-C_6)$alkylsulfonyl group, a mono$(C_1-C_6)$alkylamino group, a di$(C_1-C6)$alkylamino group whose $(C_1-C_6)$alkyl groups may be the same or different, a $(C_1-C_6)$alkylsulfonylamino group, and a halo$(C_1-C_6)$alkylsulfonylamino group; a hydroxyimino$(C_1-C_6)$alkyl group; a $(C_1-C_6)$alkoxyimino$(C_1-C_6)$alkyl group; a $(C_2-C_6)$alkenyloxyimino$(C_1-C_6)$alkyl group; a phenyl$(C_1-C_6)$ alkoxyimino$(C_1-C_6)$alkyl group; a substituted phenyl $(C_1-C_6)$alkoxyimino$(C_1-C_6)$alkyl group having on the ring at least one substituent which may be the same or different and is selected from the group consisting of a halogen atom, a cyano group, a nitro group, a hydroxyl group, an amino group, a mercapto group, a $(C_1-C_6)$alkyl group, a halo$(C_1-C_6)$alkyl group, a $(C_2-C_6)$alkenyl group, a halo$(C_2-C_6)$alkenyl group, a $(C_2-C_6)$alkynyl group, a halo$(C_2-C_6)$ alkynyl group, a $(C_1-C_6)$alkoxyl group, a halo$(C_1-C_6)$alkoxyl group, a $(C_1-C_6)$alkylthio group, a halo$(C_1-C_6)$alkylthio group, a $(C_1-C_6)$alkylsulfinyl group, a halo$(C_1-C_6)$alkylsulfinyl group, a $(C_1-C_6)$alkylsulfonyl group, a halo$(C_1-C_6)$ alkylsulfonyl group, a mono$(C_1-C_6)$alkylamino group, a di$(C_1-C6)$alkylamino group whose $(C_1-C_6)$alkyl groups may be the same or different, a $(C_1-C_6)$alkylsulfonylamino group, and a halo$(C_1-C_6)$alkylsulfonylamino group; a heterocyclic $(C_1-C_6)$alkoxyimino$(C_1-C_6)$alkyl group (the heterocyclic ring is the same as described above); a substituted heterocyclic $(C_1-C_6)$alkoxyimino$(C_1-C_6)$alkyl group (the heterocyclic group is the same as described above) having on the ring at least one substituent which may be the same or different and is selected from the group consisting of a halogen atom, a cyano group, a nitro group, a hydroxyl group, an amino group, a mercapto group, a $(C_1-C_6)$ alkyl group, a halo$(C_1-C_6)$alkyl group, a $(C_2-C_6)$alkenyl group, a halo$(C_2-C_6)$alkenyl group, a $(C_2-C_6)$alkynyl group, a halo$(C_2-C_6)$alkynyl group, a $(C_1-C_6)$alkoxyl group, a halo$(C_1-C_6)$alkoxyl group, a $(C_1-C_6)$alkylthio group, a halo $(C_1-C_6)$alkylthio group, a $(C_1-C_6)$alkylsulfinyl group, a halo$(C_1-C_6)$alkylsulfinyl group, a $(C_1-C_6)$alkylsulfonyl group, a halo$(C_1-C_6)$alkylsulfonyl group, a mono$(C_1-C_6)$alkylamino group, a same or different di$(C_1-C_6)$alkylamino group, a $(C_1-C_6)$alkylsulfonylamino group, and a halo$(C_1-C_6)$alkylsulfonylamino group; a $(C_1-C_6)$alkylcarbonyl$(C_1-C_6)$alkyl group; a $(C_1-C_6)$alkoxycarbonyl $(C_1-C_6)$alkyl group; the following group:

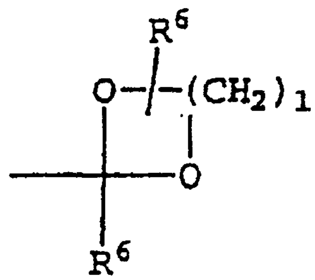

(wherein, $R^6$ is same or different, and the same as described above, and 1 is an integer of 2 to 4); a $-COZ_m(R^6)$ group (wherein, $R^6$, Z, and m are the same as described above); a $-ZCOZ_m(R^6)$ group (wherein, $R^6$, Z, and m are the same as described above); a $(C_1-C_6)$alkylsulfonylamino group; a halo$(C_1-C_6)$alkylsulfonylamino group; a phenyl group; a substituted phenyl group having at least one substituent which may be the same or different and is selected from the group consisting of a halogen atom, a cyano group, a nitro group, a hydroxyl group, an amino group, a mercapto group, a $(C_1-C_6)$alkyl group, a halo$(C_1-C_6)$alkyl group, a $(C_2-C_6)$alkenyl group, a halo$(C_2-C_6)$ alkenyl group, a $(C_2-C_6)$alkynyl group, a halo$(C_2-C_6)$alkynyl group, a $(C_1-C_6)$alkoxyl group, a halo$(C_1-C_6)$alkoxyl group, a $(C_1-C_6)$alkylthio group, a halo$(C_1-C_6)$alkylthio group, a $(C_1-C_6)$alkylsulfinyl group, a halo$(C_1-C_6)$alkylsulfinyl group, a $(C_1-C_6)$alkylsulfonyl group, a halo$(C_1-C_6)$alkylsulfonyl group, a mono$(C_1-C_6)$alkylamino group, a di $(C_1-C_6)$alkylamino group whose $(C_1-C_6)$alkyl groups may be the same or different, a $-COZ_m(R^6)$ group (wherein, $R^6$, Z, and m are the same as described above), a $-ZCOZ_m(R^6)$ group (wherein, $R^6$, Z, and m are the same as described above), a $(C_1-C_6)$alkylsulfonylamino group, a halo$(C_1-C_6)$-alkylsulfonylamino group, a hydroxyimino $(C_1-C_6)$alkyl group, a $(C_1-C_6)$alkoxyimino$(C_1-C_6)$alkyl group, a heterocyclic group (a heterocyclic ring is the same as described above), and a substituted heterocyclic group (a heterocyclic ring is the same as described above) having on the ring at least one substituent which may be the same or different and is selected from the group

consisting of a halogen atom, a cyano group, a nitro group, a hydroxyl group, an amino group, a mercapto group, a $(C_1\text{-}C_6)$alkyl group, a halo$(C_1\text{-}C_6)$alkyl group, a $(C_2\text{-}C_6)$alkenyl group, a halo$(C_2\text{-}C_6)$alkenyl group, a $(C_2\text{-}C_6)$alkynyl group, a halo$(C_2\text{-}C_6)$alkynyl group, a $(C_1\text{-}C_6)$alkoxyl group, a halo$(C_1\text{-}C_6)$alkoxyl group, a $(C_1\text{-}C_6)$alkylthio group, a halo$(C_1\text{-}C_6)$alkylthio group, a $(C_1\text{-}C_6)$alkylsulfinyl group, a halo$(C_1\text{-}C_6)$alkylsulfinyl group, a $(C_1\text{-}C_6)$alkylsulfonyl group, a halo$(C_1\text{-}C_6)$alkylsulfonyl group, a mono$(C_1\text{-}C_6)$alkylamino group, a di$(C_1\text{-}C_6)$alkylamino group whose $(C_1\text{-}C_6)$alkyl groups may be the same or different, a $(C_1\text{-}C_6)$alkylsulfonylamino group, and a halo$(C_1\text{-}C_6)$alkylsulfonylamino group; a heterocyclic group (a heterocyclic ring is the same as described above); a substituted heterocyclic group (a heterocyclic ring is the same as described above) having on the ring at least one substituent which may be the same or different and is selected from the group consisting of a halogen atom, a cyano group, a nitro group, a hydroxyl group, an amino group, a mercapto group, a $(C_1\text{-}C_6)$alkyl group, a halo$(C_1\text{-}C_6)$alkyl group, a $(C_2\text{-}C_6)$alkenyl group, a halo$(C_2\text{-}C_6)$alkenyl group, a $(C_2\text{-}C_6)$alkynyl group, a halo$(C_2\text{-}C_6)$alkynyl group, a $(C_1\text{-}C_6)$alkoxyl group, a halo$(C_1\text{-}C_6)$alkoxyl group, a $(C_1\text{-}C_6)$alkylthio group, a halo$(C_1\text{-}C_6)$alkylthio group, a $(C_1\text{-}C_6)$alkylsulfinyl group, a halo$(C_1\text{-}C_6)$alkylsulfinyl group, a $(C_1\text{-}C_6)$alkylsulfonyl group, a halo$(C_1\text{-}C_6)$alkylsulfonyl group, a mono$(C_1\text{-}C_6)$alkylamino group, a di$(C_1\text{-}C_6)$alkylamino group whose $(C_1\text{-}C_6)$alkyl groups may be the same or different, a $(C_1\text{-}C_6)$alkylsulfonylamino group, and a halo$(C_1\text{-}C_6)$alkylsulfonylamino group; or -Q-Ar [wherein, Q represents an oxygen atom; a sulfur atom; $N(R^7)$ (wherein, $R^7$ is the same as described above); a $(C_1\text{-}C_6)$alkylene group; a $(C_1\text{-}C_6)$alkyl$(C_1\text{-}C_6)$alkylene group; a halo$(C_1\text{-}C_6)$alkyl$(C_1\text{-}C_6)$alkylene group; a hydroxy $(C_1\text{-}C_6)$alkyl $(C_1\text{-}C_6)$alkylene group; a $(C_1\text{-}C_6)$alkoxy$(C_1\text{-}C_6)$alkyl$(C_1\text{-}C_6)$alkylene group; a $(C_2\text{-}C_6)$alkenyl$(C_1\text{-}C_6)$alkylene group; a halo$(C_2\text{-}C_6)$alkenyl$(C_1\text{-}C_6)$alkylene group; a hydroxy$(C_1\text{-}C_6)$alkylene group; a $(C_1\text{-}C_6)$alkoxy$(C_1\text{-}C_6)$alkylene group; a halo$(C_1\text{-}C_6)$alkoxy$(C_1\text{-}C_6)$alkylene group; a $(C_1\text{-}C_6)$alkylcarbonyloxy $(C_1\text{-}C_6)$alkylene group; a cyano$(C_1\text{-}C_6)$alkylene group; a halo$(C_1\text{-}C_6)$alkylene group; a $(C_3\text{-}C_6)$cycloalkyl$(C_1\text{-}C_6)$alkylene group; a $(C_3\text{-}C_6)$cycloalkenyl$(C_1\text{-}C_6)$alkylene group; a $(C_1\text{-}C_6)$alkylcarbonyl$(C_1\text{-}C_6)$alkylene group; a $(C_1\text{-}C_6)$alkoxycarbonyl$(C_1\text{-}C_6)$alkylene group; an amino$(C_1\text{-}C_6)$alkylene group; a mono$(C_1\text{-}C_6)$alkylamino $(C_1\text{-}C_6)$alkylene group; a di$(C_1\text{-}C_6)$alkylamino$(C_1\text{-}C_6)$alkylene group whose $(C_1\text{-}C_6)$alkyl groups may be the same or different; a $(C_1\text{-}C_6)$alkylcarbonylamino$(C_1\text{-}C_6)$alkylene group; a $(C_1\text{-}C_6)$alkoxycarbonylamino$(C_1\text{-}C_6)$alkylene group; a mercapto$(C_1\text{-}C_6)$alkylene group; a $(C_1\text{-}C_6)$alkylthio $(C_1\text{-}C_6)$alkylene group; a $(C_1\text{-}C_6)$alkylsulfinyl$(C_1\text{-}C_6)$alkylene group; a $(C_1\text{-}C_6)$alkylsulfonyl$(C_1\text{-}C_6)$alkylene group; a $(C_3\text{-}C_6)$cycloalkylene group; a $(C_2\text{-}C_6)$alkenylene group; a $(C_1\text{-}C_6)$alkyl $(C_2\text{-}C_6)$alkenylene group; a halo$(C_1\text{-}C_6)$alkyl$(C_2\text{-}C_6)$alkenylene group; a hydroxyl$(C_1\text{-}C_6)$alkyl-$(C_2\text{-}C_6)$alkenylene group; a $(C_1\text{-}C_6)$alkoxy$(C_1\text{-}C_6)$alkyl$(C_2\text{-}C_6)$alkenylene group; a $(C_2\text{-}C_6)$alkenyl$(C_2\text{-}C_6)$alkenylene group; a halo$(C_2\text{-}C_6)$alkenyl$(C_2\text{-}C_6)$alkenylene group; a $(C_2\text{-}C_6)$alkynyl$(C_2\text{-}C_6)$alkenylene group; a halo$(C_2\text{-}C_6)$alkynyl$(C_2\text{-}C_6)$alkenylene group; a hydroxy$(C_2\text{-}C_6)$alkenylene group; a $(C_1\text{-}C_6)$alkoxy$(C_2\text{-}C_6)$alkenylene group; a halo $(C_1\text{-}C_6)$alkoxy$(C_2\text{-}C_6)$alkenylene group; a $(C_1\text{-}C_6)$alkylcarbonyloxy$(C_2\text{-}C_6)$alkenylene group; a cyano$(C_2\text{-}C_6)$alkenylene group; a halo $(C_2\text{-}C_6)$alkenylene group; a $(C_3\text{-}C_6)$cycloalkyl$(C_2\text{-}C_6)$alkenylene group; a $(C_3\text{-}C_6)$cycloalkenyl$(C_2\text{-}C_6)$alkenylene group; a $(C_1\text{-}C_6)$alkylcarbonyl$(C_2\text{-}C_6)$alkenylene group; a $(C_1\text{-}C_6)$alkoxylcarbonyl$(C_2\text{-}C_6)$alkenylene group; an amino$(C_2\text{-}C_6)$alkenylene group; a mono$(C_1\text{-}C_6)$alkylamino$(C_2\text{-}C_6)$-alkenylene group; a di$(C_1\text{-}C_6)$alkylamino$(C_2\text{-}C_6)$alkenylene group whose $(C_1\text{-}C_6)$alkyl groups may be the same or different; a $(C_1\text{-}C_6)$alkylcarbonylamino$(C_2\text{-}C_6)$alkenylene group; a $(C_1\text{-}C_6)$alkoxycarbonylamino$(C_2\text{-}C_6)$alkenylene group; a mercapto$(C_2\text{-}C_6)$alkenylene group; a $(C_1\text{-}C_6)$alkylthio $(C_2\text{-}C_6)$alkenylene group; a $(C_1\text{-}C_6)$alkylsulfinyl$(C_2\text{-}C_6)$alkenylene group; a $(C_1\text{-}C_6)$alkylsulfonyl $(C_2\text{-}C_6)$alkenylene group; a $(C_2\text{-}C_6)$alkynylene group; a $(C_1\text{-}C_6)$alkylenoxy group; a $(C_1\text{-}C_6)$alkyl$(C_1\text{-}C_6)$alkylenoxy group; a $(C_1\text{-}C_6)$alkylenethio group; a $(C_1\text{-}C_6)$alkyl$(C_1\text{-}C_6)$alkylenethio group; a $(C_1\text{-}C_6)$alkyleneamino group; a $(C_1\text{-}C_6)$alkyl$(C_1\text{-}C_6)$alkyleneamino group; a carbonyl group; a hydroxyimino group; a $(C_1\text{-}C_6)$alkoxyimino group; a $(C_1\text{-}C_6)$alkylenedioxy$(C_1\text{-}C_6)$alkylene group; or a $Z(R^6)_mCO$ group (wherein, $R^6$, Z, and m are the same as described above);

Ar represents a phenyl group; a substituted phenyl group having at least one substituent which may be the same or different and is selected from the group consisting of a halogen atom, a cyano group, a nitro group, a hydroxyl group, an amino group, a mercapto group, a $(C_1\text{-}C_6)$alkyl group, a halo$(C_1\text{-}C_6)$alkyl group, a $(C_2\text{-}C_6)$alkenyl group, a halo$(C_2\text{-}C_6)$alkenyl group, a $(C_2\text{-}C_6)$alkynyl group, a halo$(C_2\text{-}C_6)$alkynyl group, a $(C_1\text{-}C_6)$alkoxyl group, a halo$(C_1\text{-}C_6)$alkoxyl group, a $(C_1\text{-}C_6)$alkylthio group, a halo$(C_1\text{-}C_6)$alkylthio group, a $(C_1\text{-}C_6)$alkylsulfinyl group, a halo$(C_1\text{-}C_6)$alkylsulfinyl group, a $(C_1\text{-}C_6)$alkylsulfonyl group, a halo$(C_1\text{-}C_6)$alkylsulfonyl group, a mono $(C_1\text{-}C_6)$alkylamino group, a di$(C_1\text{-}C_6)$alkylamino group whose $(C_1\text{-}C_6)$alkyl groups may be the same or different, a hydroxyimino$(C_1\text{-}C_6)$alkyl group, a $(C_1\text{-}C_6)$alkoxyimino$(C_1\text{-}C_6)$alkyl group, a $(C_2\text{-}C_6)$alkenyloxyimino$(C_1\text{-}C_6)$alkyl group, a $-COZ_m(R^6)$ group (wherein, $R^6$, Z, and m are the same as described above), a $-ZCOZ_m(R^6)$ group (wherein, $R^6$, Z, and m are the same as described above), a $(C_1\text{-}C_6)$alkylsulfonylamino group, and a halo$(C_1\text{-}C_6)$alkylsulfonylamino group; a heterocyclic group (a heterocyclic ring is the same as described above); a substituted heterocyclic group (a heterocyclic ring is the same as described above) having at least one substituent which may be the same or different and is selected from the group consisting of a halogen atom, a cyano group, a nitro group, a hydroxyl group, an amino group, a mercapto group, a $(C_1\text{-}C_6)$alkyl group, a halo$(C_1\text{-}C_6)$alkyl group, a $(C_2\text{-}C_6)$alkenyl group, a halo$(C_2\text{-}C_6)$alkenyl group, a $(C_2\text{-}C_6)$alkynyl group, a halo$(C_2\text{-}C_6)$alkynyl group, a $(C_1\text{-}C_6)$alkoxyl

group, a halo($C_1$-$C_6$)alkoxyl group, a ($C_1$-$C_6$)alkylthio group, a halo($C_1$-$C_6$)alkylthio group, a ($C_1$-$C_6$)alkylsulfinyl group, a halo ($C_1$-$C_6$)alkylsulfinyl group, a ($C_1$-$C_6$)alkylsulfonyl group, a halo($C_1$-$C_6$)alkylsulfonyl group, a mono ($C_1$-$C_6$)alkylamino group, a di($C_1$-$C_6$)alkylamino group whose ($C_1$-$C_6$)alkyl groups may be the same or different, a -$COZ_m(R^6)$ group (wherein, $R^6$, Z, and m are the same as described above), a -$ZCOZ_m(R^6)$ group (wherein, $R^6$, Z, and m are the same as described above), a ($C_1$-$C_6$)alkylsulfonylamino group, a halo($C_1$-$C_6$)alkylsulfonylamino group, a hydroxyimino($C_1$-$C_6$)alkyl group, a ($C_1$-$C_6$)alkoxyimino($C_1$-$C_6$)alkyl group, and a ($C_2$-$C_6$)alkenyloxyimino ($C_1$-$C_6$)alkyl group];

and n represents an integer of 1 to 5.

**3.** The N-(4-pyrazolyl)amide derivative according to Claim 1 represented by the general formula (I-2):

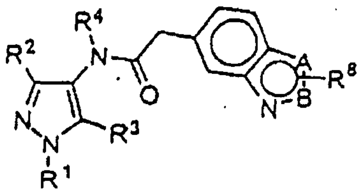

(I-2)

wherein $R^1$ represents a hydrogen atom; a ($C_1$-$C_6$)alkyl group; a halo($C_1$-$C_6$)alkyl group; a hydroxy($C_1$-$C_6$)alkyl group; a cyano($C_1$-$C_6$)alkyl group; a formyl($C_1$-$C_6$)alkyl group; a ($C_2$-$C_6$)alkenyl group; a halo($C_2$-$C_6$)alkenyl group; a ($C_2$-$C_6$)alkynyl group; a halo($C_2$-$C_6$)alkynyl group; a ($C_1$-$C_6$)alkoxy($C_1$-$C_6$)alkyl group; a halo($C_1$-$C_6$)alkoxy ($C_1$-$C_6$)alkyl group; a ($C_1$-$C_6$)alkylthio($C_1$-$C_6$)-alkyl group; a halo($C_1$-$C_6$)alkylthio($C_1$-$C_6$)alkyl group; a ($C_1$-$C_6$)alkylsulfinyl($C_1$-$C_6$)alkyl group; a halo($C_1$-$C_6$)-alkylsulfinyl($C_1$-$C_6$)alkyl group; a ($C_1$-$C_6$)alkylsulfonyl-($C_1$-$C_6$)alkyl group; a halo($C_1$-$C_6$)alkylsulfonyl($C_1$-$C_6$)alkyl group; a mono($C_1$-$C_6$)alkylamino($C_1$-$C_6$)alkyl group; a di ($C_1$-$C_6$)alkylamino ($C_1$-$C_6$)alkyl group whose ($C_1$-$C_6$)alkyl groups may be the same or different; a ($C_1$-$C_6$)alkylsulfonyl group; a halo ($C_1$-$C_6$)alkylsulfonyl group; a mono($C_1$-$C_6$)alkylaminosulfonyl group; a di($C_1$-$C_6$)alkylaminosulfonyl group whose ($C_1$-$C_6$)alkyl groups may be the same or different; a ($C_1$-$C_6$)alkyloxycarbonyl($C_1$-$C_6$)alkyl group; a mono($C_1$-$C_6$) alkylaminocarbonyl($C_1$-$C_6$)alkyl group; a di($C_1$-$C_6$)alkylaminocarbonyl($C_1$-$C_6$)alkyl group whose ($C_1$-$C_6$)alkyl groups may be the same or different; a ($C_1$-$C_6$)alkyloxyimino($C_1$-$C_6$)alkyl group; a ($C_1$-$C_6$)alkylsulfonyloxy($C_1$-$C_6$) alkyl group; a halo($C_1$-$C_6$)alkylsulfonyloxy($C_1$-$C_6$)alkyl group; a ($C_1$-$C_6$)alkylcarbonyloxy ($C_1$-$C_6$)alkyl group; a phenylsulfonyl group; a substituted phenylsulfonyl group having at least one substituent which may be the same or different and is selected from the group consisting of a halogen atom, a cyano group, a nitro group, a hydroxyl group, an amino group, a mercapto group, a ($C_1$-$C_6$)alkyl group, a halo($C_1$-$C_6$)alkyl group, a ($C_2$-$C_6$)alkenyl group, a halo($C_2$-$C_6$)-alkenyl group, a ($C_2$-$C_6$)alkynyl group, a halo($C_2$-$C_6$)-alkynyl group, a ($C_1$-$C_6$)alkoxyl group, a halo ($C_1$-$C_6$)-alkoxyl group, a ($C_1$-$C_6$)alkylthio group, a halo($C_1$-$C_6$)-alkylthio group, a ($C_1$-$C_6$)alkylsulfinyl group, a halo ($C_1$-$C_6$)alkylsulfinyl group, a ($C_1$-$C_6$)alkylsulfonyl group, a halo($C_1$-$C_6$)alkylsulfonyl group, a mono($C_1$-$C_6$)alkylamino group, a di($C_1$-$C_6$)alkylamino group whose ($C_1$-$C_6$)alkyl groups may be the same or different, a ($C_1$-$C_6$)alkylsulfonylamino group, and a halo($C_1$-$C_6$)alkylsulfonylamino group; a ($C_3$-$C_6$)cycloalkyl group; a halo($C_3$-$C_6$)cycloalkyl group; a ($C_3$-$C_6$)cycloalkenyl group; a halo($C_3$-$C_6$)cycloalkenyl group; a phenyl group; a substituted phenyl group having at least one substituent which may be the same or different and is selected from the group consisting of a halogen atom, a cyano group, a nitro group, a hydroxyl group, an amino group, a mercapto group, a ($C_1$-$C_6$) alkyl group, a halo($C_1$-$C_6$)alkyl group, a ($C_2$-$C_6$)alkenyl group, a halo($C_2$-$C_6$)alkenyl group, a ($C_2$-$C_6$)alkynyl group, a halo($C_2$-$C_6$)alkynyl group, a ($C_1$-$C_6$)alkoxyl group, a halo($C_1$-$C_6$)alkoxyl group, a ($C_1$-$C_6$)alkylthio group, a halo ($C_1$-$C_6$)alkylthio group, a ($C_1$-$C_6$)alkylsulfinyl group, a halo($C_1$-$C_6$)alkylsulfinyl group, a ($C_1$-$C_6$)alkylsulfonyl group, a halo($C_1$-$C_6$)alkylsulfonyl group, a mono($C_1$-$C_6$)alkylamino group, a di($C_1$-$C_6$)alkylamino group whose ($C_1$-$C_6$) alkyl groups may be the same or different, a ($C_1$-$C_6$)alkylsulfonyl amino group, and a halo($C_1$-$C_6$)alkylsulfonylamino group; or a -$COZ_m(R^6)$ group {wherein, $R^6$ represents a hydrogen atom; a ($C_1$-$C_6$)alkyl group; a halo ($C_1$-$C_6$)alkyl group; a ($C_2$-$C_6$)alkenyl group; a halo($C_2$-$C_6$)alkenyl group; a ($C_2$-$C_6$)alkynyl group; a halo($C_2$-$C_6$)alkynyl group; a ($C_1$-$C_6$)alkoxy($C_1$-$C_6$)alkyl group; a halo($C_1$-$C_6$)alkoxy($C_1$-$C_6$)alkyl group; a ($C_1$-$C_6$)alkylthio($C_1$-$C_6$)alkyl group; a halo($C_1$-$C_6$)alkylthio ($C_1$-$C_6$)alkyl group; a ($C_1$-$C_6$)alkylsulfinyl ($C_1$-$C_6$)alkyl group; a halo($C_1$-$C_6$)alkylsulfinyl-($C_1$-$C_6$)alkyl group; a ($C_1$-$C_6$)alkylsulfonyl ($C_1$-$C_6$)alkyl group; a halo($C_1$-$C_6$)alkylsulfonyl ($C_1$-$C_6$)alkyl group; a mono($C_1$-$C_6$)alkylamino($C_1$-$C_6$)alkyl group; a di($C_1$-$C_6$)alkylamino($C_1$-$C_6$)alkyl group whose ($C_1$-$C_6$)alkyl groups may be the same or different; a ($C_3$-$C_6$)cycloalkyl group; a halo($C_3$-$C_6$)cycloalkyl group; a ($C_3$-$C_6$)cycloalkenyl group; a halo($C_3$-$C_6$)cycloalkenyl group; a phenyl group; or a substituted phenyl group having at least one substituent which may be the same or different and is selected from the group consisting of a halogen atom, a cyano

group, a nitro group, a hydroxyl group, an amino group, a mercapto group, a $(C_1-C_6)$alkyl group, a halo$(C_1-C_6)$alkyl group, a $(C_2-C_6)$alkenyl group, a halo$(C_2-C_6)$alkenyl group, a $(C_2-C_6)$alkynyl group, a halo$(C_2-C_6)$alkynyl group, a $(C_1-C_6)$alkoxyl group, a halo$(C_1-C_6)$alkoxyl group, a $(C_1-C_6)$alkylthio group, a halo$(C_1-C_6)$alkylthio group, a $(C_1-C_6)$alkylsulfinyl group, a halo$(C_1-C_6)$alkylsulfinyl group, a $(C_1-C_6)$alkylsulfonyl group, a halo$(C_1-C_6)$alkylsulfonyl group, a mono$(C_1-C_6)$alkylamino group, a di$(C_1-C_6)$alkylamino group whose $(C_1-C_6)$alkyl groups may be the same or different, a $(C_1-C_6)$alkylsulfonylamino group, and a halo$(C_1-C_6)$alkylsulfonylamino group; Z represents an oxygen atom; a sulfur atom; or $N(R^7)$ [wherein, $R^7$ represents a hydrogen atom; a $(C_1-C_6)$alkyl group; a halo$(C_1-C_6)$alkyl group; a $(C_2-C_6)$alkenyl group; a halo$(C_2-C_6)$alkenyl group; a $(C_2-C_6)$alkynyl group; a halo$(C_2-C_6)$alkynyl group; a $(C_1-C_6)$alkoxy$(C_1-C_6)$alkyl group; a halo$(C_1-C_6)$alkoxy$(C_1-C_6)$alkyl group; a $(C_1-C_6)$alkylthio$(C_1-C_6)$alkyl group; a halo$(C_1-C_6)$alkylthio$(C_1-C_6)$alkyl group; a $(C_1-C_6)$alkylsulfinyl $(C_1-C_6)$alkyl group; a halo$(C_1-C_6)$alkylsulfinyl $(C_1-C_6)$alkyl group; a $(C_1-C_6)$alkylsulfonyl$(C_1-C_6)$alkyl group; a halo$(C_1-C_6)$alkylsulfonyl$(C_1-C_6)$alkyl group; a mono$(C_1-C_6)$alkylamino$(C_1-C_6)$alkyl group; a di $(C_1-C_6)$alkylamino$(C_1-C_6)$alkyl group whose $(C_1-C_6)$alkyl groups may be the same or different; a $(C_3-C_6)$cycloalkyl group; a halo $(C_3-C_6)$cycloalkyl group; a $(C_3-C_6)$cycloalkenyl group; or a halo$(C_3-C_6)$cycloalkenyl group]; and m represents an integer of 0 to 1};

$R^2$ and $R^3$ may be same or different, and represent a hydrogen atom, a halogen atom; a cyano group; a nitro group; a hydroxyl group; a mercapto group; an amino group; a $(C_1-C_6)$alkyl group; a halo$(C_1-C_6)$alkyl group; a $(C_2-C_6)$alkenyl group; a halo$(C_2-C_6)$alkenyl group; a $(C_2-C_6)$alkynyl group; a halo$(C_2-C_6)$alkynyl group; a $(C_1-C_6)$alkoxyl group; a halo$(C_1-C_6)$alkoxyl group; a $(C_1-C_6)$alkylthio group; a halo$(C_1-C_6)$alkylthio group; a $(C_1-C_6)$alkylsulfinyl group; a halo $(C_1-C_6)$alkylsulfinyl group; a $(C_1-C_6)$alkylsulfonyl group; a halo$(C_1-C_6)$alkylsulfonyl group; a mono$(C_1-C_6)$alkylamino group; a di$(C_1-C_6)$alkylamino group whose $(C_1-C_6)$alkyl groups may be the same or different; a $(C_1-C_6)$alkoxy$(C_1-C_6)$alkyl group; a halo$(C_1-C_6)$alkoxy $(C_1-C_6)$alkyl group; a $(C_1-C_6)$alkylthio$(C_1-C_6)$alkyl group; a halo$(C_1-C_6)$alkylthio $(C_1-C_6)$alkyl group; a $(C_1-C_6)$alkylsulfinyl$(C_1-C_6)$alkyl group; a halo$(C_1-C_6)$alkylsulfinyl $(C_1-C_6)$alkyl group; a $(C_1-C_6)$alkylsulfonyl$(C_1-C_6)$alkyl group; a halo $(C_1-C_6)$alkylsulfonyl$(C_1-C_6)$alkyl group; a mono$(C_1-C_6)$alkylamino$(C_1-C_6)$alkyl group; a di $(C_1-C_6)$alkylamino $(C_1-C_6)$alkyl group whose $(C_1-C_6)$alkyl groups may be the same or different; a $(C_3-C_6)$cycloalkyl group; a halo $(C_3-C_6)$cycloalkyl group; a $(C_3-C_6)$cycloalkenyl group; a halo$(C_3-C_6)$cycloalkenyl group; a phenyl group; a substituted phenyl group having at least one substituent which may be the same or different and is selected from the group consisting of a halogen atom, a cyano group, a nitro group, a hydroxyl group, an amino group, a mercapto group, a $(C_1-C_6)$alkyl group, a halo$(C_1-C_6)$alkyl group, a $(C_2-C_6)$alkenyl group, a halo$(C_2-C_6)$-alkenyl group, a $(C_2-C_6)$alkynyl group, a halo$(C_2-C_6)$alkynyl group, a $(C_1-C_6)$alkoxyl group, a halo$(C_1-C_6)$alkoxyl group, a $(C_1-C_6)$alkylthio group, a halo$(C_1-C_6)$alkylthio group, a $(C_1-C_6)$alkylsulfinyl group, a halo$(C_1-C_6)$alkylsulfinyl group, a $(C_1-C_6)$alkylsulfonyl group, a halo$(C_1-C_6)$alkylsulfonyl group, a mono$(C_1-C_6)$alkylamino group, a di$(C_1-C_6)$alkylamino group whose $(C_1-C_6)$alkyl groups may be the same or different, a $(C_1-C_6)$alkylsulfonylamino group, and a halo$(C_1-C_6)$alkylsulfonylamino group; a phenoxy group; a substituted phenoxy group having at least one substituent which may be the same or different and is selected from the group consisting of a halogen atom, a cyano group, a nitro group, a hydroxyl group, an amino group, a mercapto group, a $(C_1-C_6)$alkyl group, a halo$(C_1-C_6)$alkyl group, a $(C_2-C_6)$alkenyl group, a halo$(C_2-C_6)$alkenyl group, a $(C_2-C_6)$alkynyl group, a halo$(C_2-C_6)$alkynyl group, a $(C_1-C_6)$alkoxyl group, a halo$(C_1-C_6)$alkoxyl group, a $(C_1-C_6)$alkylthio group, a halo$(C_1-C_6)$alkylthio group, a $(C_1-C_6)$alkylsulfinyl group, a halo$(C_1-C_6)$alkylsulfinyl group, a $(C_1-C_6)$alkylsulfonyl group, a halo$(C_1-C_6)$alkylsulfonyl group, a mono$(C_1-C_6)$alkylamino group, a di$(C_1-C6)$alkylamino group whose $(C_1-C_6)$alkyl groups may be the same or different, a $(C_1-C_6)$alkylsulfonylamino group, and a halo$(C_1-C_6)$alkylsulfonylamino group; a phenylthio group; a substituted phenylthio group having at least one substituent which may be the same or different and is selected from the group consisting of a halogen atom, a cyano group, a nitro group, a hydroxyl group, an amino group, a mercapto group, a $(C_1-C_6)$alkyl group, a halo $(C_1-C_6)$alkyl group, a $(C_2-C_6)$alkenyl group, a halo$(C_2-C_6)$alkenyl group, a $(C_2-C_6)$alkynyl group, a halo $(C_2-C_6)$alkynyl group, a $(C_1-C_6)$alkoxyl group, a halo$(C_1-C_6)$alkoxyl group, a $(C_1-C_6)$alkylthio group, a halo$(C_1-C_6)$alkylthio group, a $(C_1-C_6)$alkylsulfinyl group, a halo$(C_1-C_6)$alkylsulfinyl group, a $(C_1-C_6)$alkylsulfonyl group, a halo$(C_1-C_6)$alkylsulfonyl group, a mono$(C_1-C_6)$alkylamino group, a di$(C_1-C_6)$alkylamino group whose $(C_1-C_6)$alkyl groups may be the same or different, a $(C_1-C_6)$alkylsulfonyl amino group, and a halo$(C_1-C_6)$alkylsulfonylamino group; a $-ZCOZ_m(R^6)$ group (wherein, $R^6$, Z, and m are the same as described above); or a $-COZ_m(R^6)$ group (wherein, $R^6$, Z, and m are the same as described above) ;

$R^4$ represents a hydrogen atom; a $(C_1-C_6)$alkyl group; a halo$(C_1-C_6)$alkyl group; a cyano$(C_1-C_6)$alkyl group; a $(C_2-C_6)$alkenyl group; a halo$(C_2-C_6)$alkenyl group; a $(C_2-C_6)$alkynyl group; a halo$(C_2-C_6)$alkynyl group; a $(C_1-C_6)$alkoxy$(C_1-C_6)$alkyl group; a halo$(C_1-C_6)$alkoxy $(C_1-C_6)$alkyl group; a $(C_1-C_6)$alkoxy$(C_1-C_6)$-alkoxy$(C_1-C_6)$alkyl group; a $(C_1-C_6)$alkylthio $(C_1-C_6)$alkyl group; a halo$(C_1-C_6)$alkylthio$(C_1-C_6)$alkyl group; a $(C_1-C_6)$alkylsulfinyl $(C_1-C_6)$alkyl group; a halo$(C_1-C_6)$-alkylsulfinyl$(C_1-C_6)$alkyl group; a $(C_1-C_6)$alkylsulfonyl-$(C_1-C_6)$alkyl group; a halo $(C_1-C_6)$alkylsulfonyl$(C_1-C_6)$alkyl group; a mono$(C_1-C_6)$alkylamino$(C_1-C_6)$alkyl group; a di $(C_1-C_6)$alkylamino $(C_1-C_6)$alkyl group whose $(C_1-C_6)$alkyl groups may be the same or different; a $(C_3-C_6)$cycloalkyl group; a halo $(C_3-C_6)$cycloalkyl group; a $(C_3-C_6)$cycloalkenyl group; a halo$(C_3-C_6)$cycloalkenyl group; a $-COZ_m(R^6)$ group

(wherein, $R^6$, Z, and m are the same as described above), a -CSNH($R^6$) group (wherein, $R^6$, is the same as described above), a ($C_1$-$C_6$)alkylcarbonyl($C_1$-$C_6$)alkyl group; a ($C_1$-$C_6$)alkoxycarbonyl($C_1$-$C_6$)alkyl group; a phenyl ($C_1$-$C_6$)alkyl group; a substituted phenyl($C_1$-$C_6$)alkyl group having on the ring at least one substituent which may be the same or different and is selected from the group consisting of a halogen atom, a cyano group, a nitro group, a hydroxyl group, an amino group, a mercapto group, a ($C_1$-$C_6$)alkyl group, a halo($C_1$-$C_6$)alkyl group, a ($C_2$-$C_6$) alkenyl group, a halo($C_2$-$C_6$)alkenyl group, a ($C_2$-$C_6$)alkynyl group, a halo($C_2$-$C_6$)alkynyl group, a ($C_1$-$C_6$)alkoxyl group, a halo($C_1$-$C_6$)alkoxyl group, a ($C_1$-$C_6$)alkylthio group, a halo($C_1$-$C_6$)alkylthio group, a ($C_1$-$C_6$)alkylsulfinyl group, a halo($C_1$-$C_6$)alkylsulfinyl group, a ($C_1$-$C_6$)alkylsulfonyl group, a halo($C_1$-$C_6$)alkylsulfonyl group, a mono ($C_1$-$C_6$)alkylamino group, a di($C_1$-$C_6$)alkylamino group whose ($C_1$-$C_6$)alkyl groups may be the same or different, a ($C_1$-$C_6$)alkylsulfonylamino group, and a halo($C_1$-$C_6$)alkylsulfonylamino group; a heterocyclic($C_1$-$C_6$)alkyl group (a heterocyclic ring represents a pyridyl group; a pyridine-N-oxide group; a pyridazinyl group; a pyrimidinyl group; a pyrazyl group; a piperidyl group; a piperazino group; a morpholinyl group; a morpholino group; a furyl group; a tetrahydrofuryl group; a thienyl group; a tetrahydrothienyl group; a pyrrolyl group; a pyrrolidyl group; an imidazolidinyl group; an oxazolyl group; an isoxazolyl group; an oxadiazolyl group; a thiazolyl group; an isothiazolyl group; a thiadiazolyl group; an imidazolyl group; a triazolyl group; a pyrazolyl group; an indolyl group; a benzofuryl group; a benzothienyl group; an indazolyl group; a quinolyl group; an isoquinolyl group; or a quinazolyl group); or a substituted heterocyclic($C_1$-$C_6$)alkyl group (the heterocyclic group is the same as the above-described) having on the ring at least one substituent which may be the same or different and is selected from the group consisting of a halogen atom, a cyano group, a nitro group, a hydroxyl group, an amino group, a mercapto group, a ($C_1$-$C_6$)alkyl group, a halo($C_1$-$C_6$)alkyl group, a ($C_2$-$C_6$)alkenyl group, a halo($C_2$-$C_6$)alkenyl group, a ($C_2$-$C_6$)alkynyl group, a halo($C_2$-$C_6$)alkynyl group, a ($C_1$-$C_6$)alkoxyl group, a halo($C_1$-C6)alkoxyl group, a ($C_1$-$C_6$)alkylthio group, a halo ($C_1$-$C_6$)alkylthio group, a ($C_1$-$C_6$)alkylsulfinyl group, a halo($C_1$-$C_6$)alkylsulfinyl group, a ($C_1$-$C_6$)alkylsulfonyl group, a halo($C_1$-$C_6$)alkylsulfonyl group, a mono($C_1$-$C_6$)alkylamino group, a di($C_1$-$C_6$)alkylamino group whose ($C_1$-$C_6$) alkyl groups may be the same or different, a ($C_1$-$C_6$)alkylsulfonylamino group, and a halo($C_1$-$C_6$)alkylsulfonylamino group;

$R^8$ represents a halogen atom; a cyano group; a nitro group; a hydroxyl group; an amino group; a mercapto group; a ($C_1$-$C_6$)alkyl group; a halo($C_1$-$C_6$)alkyl group; a cyano($C_1$-$C_6$)alkyl group; a ($C_3$-$C_6$)cycloalkyl group; a halo($C_3$-$C_6$)cycloalkyl group; a cyano($C_3$-$C_6$)cycloalkyl group; a ($C_1$-$C_6$)alkyl($C_3$-$C_6$)cycloalkyl group; a ($C_1$-$C_6$) alkylhalo($C_3$-$C_6$)cycloalkyl group; a ($C_3$-$C_6$)cycloalkyl($C_1$-$C_6$)alkyl group; a ($C_2$-$C_6$)alkenyl group; a ($C_3$-$C_6$)cycloalkenyl group; a halo($C_2$-$C_6$)alkenyl group; a ($C_3$-$C_6$)cycloalkenyl($C_1$-$C_6$)alkyl group; a ($C_2$-$C_6$)alkynyl group; a halo($C_2$-$C_6$)alkynyl group; a ($C_1$-$C_6$)alkoxyl group; a halo($C_1$-$C_6$)alkoxyl group; a ($C_1$-$C_6$)alkylthio group; a halo ($C_1$-$C_6$)alkylthio group; a ($C_1$-$C_6$)alkylsulfinyl group; a halo($C_1$-$C_6$)alkylsulfinyl group; a ($C_1$-$C_6$)alkylsulfonyl group; a halo($C_1$-$C_6$)alkylsulfonyl group; a mono($C_1$-$C_6$)alkylamino group; a di($C_1$-C6)alkylamino group whose ($C_1$-$C_6$) alkyl groups may be the same or different; a ($C_1$-$C_6$)alkoxy($C_1$-$C_6$)alkyl group; a halo($C_1$-$C_6$)alkoxy($C_1$-$C_6$)alkyl group; a ($C_1$-$C_6$)alkylthio-($C_1$-$C_6$)alkyl group; a halo($C_1$-$C_6$)alkylthio ($C_1$-$C_6$)alkyl group; a ($C_1$-$C_6$)alkylsulfinyl ($C_1$-$C_6$)alkyl group; a halo($C_1$-$C_6$)alkylsulfinyl($C_1$-$C_6$)alkyl group; a ($C_1$-$C_6$)alkylsulfonyl($C_1$-$C_6$)alkyl group; a halo ($C_1$-$C_6$)-alkylsulfonyl($C_1$-$C_6$)alkyl group; a mono($C_1$-$C_6$)alkylamino ($C_1$-$C_6$)alkyl group; a di ($C_1$-$C_6$)alkylamino ($C_1$-$C_6$)alkyl group whose ($C_1$-$C_6$)alkyl groups may be the same or different; a carboxy($C_1$-$C_6$)alkyl group; a ($C_1$-$C_6$) alkylcarbonyl($C_1$-$C_6$)alkyl group; a ($C_1$-$C_6$)alkoxycarbonyl($C_1$-$C_6$)alkyl group; an aminocarbonyl($C_1$-$C_6$)alkyl group; a mono($C_1$-$C_6$)alkylaminocarbonyl($C_1$-$C_6$)alkyl group; a di($C_1$-$C_6$)alkylaminocarbonyl($C_1$-$C_6$)alkyl group whose ($C_1$-$C_6$)alkyl groups may be the same or different; a -$COZ_m$($R^6$) group (in the formula, $R^6$, Z, and m are the same as described above); a -$ZCOZ_m$($R^6$) group (in the formula, $R^6$, Z, and m are the same as described above); a ($C_1$-$C_6$)alkylsulfonylamino group; a halo($C_1$-$C_6$)alkylsulfonylamino group; a hydroxyimino($C_1$-$C_6$)alkyl group; a ($C_1$-$C_6$)alkoxyimino($C_1$-$C_6$)alkyl group; a ($C_2$-$C_6$)alkenyloxyimino($C_1$-$C_6$)alkyl group; a phenyl group; a substituted phenyl group having at least one substituent which may be the same or different and is selected from the group consisting of a halogen atom, a cyano group, a nitro group, a hydroxyl group, an amino group, a mercapto group, a ($C_1$-$C_6$)alkyl group, a halo($C_1$-$C_6$)alkyl group, a ($C_2$-$C_6$)alkenyl group, a halo($C_2$-$C_6$)alkenyl group, a ($C_2$-$C_6$) alkynyl group, a halo($C_2$-$C_6$)alkynyl group, a ($C_1$-$C_6$)alkoxyl group, a halo($C_1$-$C_6$)alkoxyl group, a ($C_1$-$C_6$)alkylthio group, a halo($C_1$-$C_6$)alkylthio group, a ($C_1$-$C_6$)alkylsulfinyl group, a halo($C_1$-$C_6$)alkylsulfinyl group, a ($C_1$-$C_6$)alkylsulfonyl group, a halo($C_1$-$C_6$)alkylsulfonyl group, a mono($C_1$-$C_6$)alkylamino group, a di($C_1$-$C_6$)alkylamino group whose ($C_1$-$C_6$)alkyl groups may be the same or different, a methylenedioxy group, a -$COZ_m$($R^6$) group (in the formula, $R^6$, Z, and m are the same as described above), a -$ZCOZ_m$($R^6$) group (in the formula, $R^6$, Z, and m are the same as described above), a ($C_1$-$C_6$)alkylsulfonylamino group, and a halo($C_1$-$C_6$)-alkylsulfonylamino group; a phenyl($C_1$-$C_6$)alkyl group; a substituted phenyl($C_1$-$C_6$)alkyl group having on the ring at least one substituent which may be the same or different and is selected from the group consisting of a halogen atom, a cyano group, a nitro group, a hydroxyl group, an amino group, a mercapto group, a ($C_1$-$C_6$)alkyl group, a halo($C_1$-$C_6$)alkyl group, a ($C_2$-$C_6$)alkenyl group, a halo($C_2$-$C_6$)alkenyl group, a ($C_2$-$C_6$)alkynyl group, a halo($C_2$-$C_6$)alkynyl group, a ($C_1$-$C_6$) alkoxyl group, a halo($C_1$-$C_6$)alkoxyl group, a ($C_1$-$C_6$)alkylthio group, a halo($C_1$-$C_6$)alkylthio group, a ($C_1$-$C_6$)alkyl-

sulfinyl group, a halo($C_1$-$C_6$)alkylsulfinyl group, a ($C_1$-$C_6$)alkylsulfonyl group, a halo($C_1$-$C_6$)alkylsulfonyl group, a mono($C_1$-$C_6$)alkylamino group, a di($C_1$-$C_6$)alkylamino group whose ($C_1$-$C_6$)alkyl groups may be the same or different, a -$COZ_m(R^6)$ group (in the formula, $R^6$, Z, and m are the same as described above), a -$ZCOZ_m(R^6)$ group (in the formula, $R^6$, Z, and m are the same as described above), a ($C_1$-$C_6$)alkylsulfonylamino group, and a halo ($C_1$-$C_6$)alkylsulfonylamino group; a heterocyclic group (a heterocyclic.ring is the same as described above); a substituted heterocyclic group (a heterocyclic ring is the same as described above) having at least one substituent which may be the same or different and is selected from the group consisting of a halogen atom, a cyano group, a nitro group, a hydroxyl group, an amino group, a mercapto group, a ($C_1$-$C_6$)alkyl group, a halo($C_1$-$C_6$)alkyl group, a ($C_2$-$C_6$)alkenyl group, a halo($C_2$-$C_6$)alkenyl group, a ($C_2$-$C_6$)alkynyl group, a halo($C_2$-$C_6$)alkynyl group, a ($C_1$-$C_6$) alkoxyl group, a halo($C_1$-$C_6$)alkoxyl group, a ($C_1$-$C_6$)alkylthio group, a halo($C_1$-$C_6$)alkylthio group, a ($C_1$-$C_6$)alkylsulfinyl group, a halo($C_1$-$C_6$)alkylsulfinyl group, a ($C_1$-$C_6$)alkylsulfonyl group, a halo($C_1$-$C_6$)alkylsulfonyl group, a mono($C_1$-$C_6$)alkylamino group, a di($C_1$-$C_6$)alkylamino group whose ($C_1$-$C_6$)alkyl groups may be the same or different, a -$COZ_m(R^6)$ group (in the formula, $R^6$, Z, and m are the same as described above), a -$ZCOZ_m(R^6)$ group (in the formula, $R^6$, Z, and m are the same as described above), a ($C_1$-$C_6$)alkylsulfonylamino group, and a halo ($C_1$-$C_6$)alkylsulfonylamino group; a heterocyclic($C_1$-$C_6$)alkyl group; a substituted heterocyclic($C_1$-$C_6$)alkyl group (a heterocyclic ring is the same as described above) having at least one substituent which may be the same or different and is selected from the group consisting of a halogen atom, a cyano group, a nitro group, a hydroxyl group, an amino group, a mercapto group, a ($C_1$-$C_6$)alkyl group, a halo ($C_1$-$C_6$)alkyl group, a ($C_2$-$C_6$)alkenyl group, a halo($C_2$-$C_6$)alkenyl group, a ($C_2$-$C_6$)alkynyl group, a halo($C_2$-$C_6$)alkynyl group, a ($C_1$-$C_6$)alkoxyl group, a halo ($C_1$-$C_6$)alkoxyl group, a ($C_1$-$C_6$)-alkylthio group, a halo($C_1$-$C_6$)alkylthio group, a ($C_1$-$C_6$)-alkylsulfinyl group, a halo ($C_1$-$C_6$)alkylsulfinyl group, a ($C_1$-$C_6$)alkylsulfonyl group, a halo($C_1$-$C_6$)alkylsulfonyl group, a mono($C_1$-$C_6$)alkylamino group, a di($C_1$-$C_6$)alkylamino group whose ($C_1$-$C_6$)alkyl groups may be the same or different, a ($C_1$-$C_6$)alkylsulfonylamino group, and a halo($C_1$-$C_6$)alkylsulfonylamino group; or -Q-Ar, wherein Q represents an oxygen atom; a sulfur atom; $N(R^7)$ (in the formula, $R^7$ is the same as described above); a ($C_1$-$C_6$)alkylene group; a ($C_1$-$C_6$)alkyl ($C_1$-$C_6$)alkylene group; a halo($C_1$-$C_6$)alkyl($C_1$-$C_6$)alkylene group; a hydroxy($C_1$-$C_6$)alkyl($C_1$-$C_6$)alkylene group; a ($C_1$-$C_6$)alkoxy($C_1$-$C_6$)alkyl($C_1$-$C_6$)alkylene group; a ($C_2$-$C_6$)alkenyl($C_1$-$C_6$)alkylene group; a halo($C_2$-$C_6$)alkenyl ($C_1$-$C_6$)alkylene group; a hydroxy($C_1$-$C_6$)alkylene group; a ($C_1$-$C_6$)alkoxy($C_1$-$C_6$)alkylene group; a halo($C_1$-$C_6$) alkoxy($C_1$-$C_6$)alkylene group; a ($C_1$-$C_6$)alkylcarbonyloxy($C_1$-$C_6$)alkylene group; a cyano($C_1$-$C_6$)alkylene group; a halo ($C_1$-$C_6$)alkylene group; a ($C_3$-$C_6$)cycloalkyl($C_1$-$C_6$)alkylene group; a ($C_3$-$C_6$)cycloalkenyl($C_1$-$C_6$)alkylene group; a ($C_1$-$C_6$)alkylcarbonyl($C_1$-$C_6$)alkylene group; a ($C_1$-$C_6$)alkoxycarbonyl($C_1$-$C_6$)alkylene group; an amino ($C_1$-$C_6$)alkylene group; a mono($C_1$-$C_6$)alkylamino($C_1$-$C_6$)alkylene group; a di($C_1$-$C_6$)alkylamino($C_1$-$C_6$)alkylene group whose ($C_1$-$C_6$)alkyl groups may be the same or different; a ($C_1$-$C_6$)alkylcarbonylamino($C_1$-$C_6$)alkylene group; a ($C_1$-$C_6$)alkoxycarbonylamino($C_1$-$C_6$)alkylene group; a mercapto($C_1$-$C_6$)alkylene group; a ($C_1$-$C_6$)alkylthio ($C_1$-$C_6$)alkylene group; an alkylsulfinyl($C_1$-$C_6$)alkylene group; a ($C_1$-$C_6$)alkylsulfonyl($C_1$-$C_6$)alkylene group; a ($C_3$-$C_6$)cycloalkylene group; a ($C_2$-$C_6$)alkenylene group; a ($C_1$-$C_6$)alkyl($C_2$-$C_6$)alkenylene group; a halo($C_1$-$C_6$) alkyl($C_2$-$C_6$)alkenylene group; a hydroxy($C_1$-$C_6$)alkyl($C_2$-$C_6$)alkenylene group; a ($C_1$-$C_6$)alkoxy($C_1$-$C_6$)alkyl ($C_2$-$C_6$)alkenylene group; a ($C_2$-$C_6$)alkenyl($C_2$-$C_6$)alkenylene group; a halo($C_2$-$C_6$)alkenyl($C_2$-$C_6$)alkenylene group; a ($C_2$-$C_6$)alkynyl($C_2$-$C_6$)alkenylene group; a halo($C_2$-$C_6$)alkynyl($C_2$-$C_6$)alkenylene group; a hydroxy($C_2$-$C_6$) alkenylene group; a ($C_1$-$C_6$)alkoxy ($C_2$-$C_6$)alkenylene group; a halo($C_1$-$C_6$)alkoxy($C_2$-$C_6$)alkenylene group; a ($C_1$-$C_6$)alkylcarbonyloxy($C_2$-$C_6$)alkenylene group; a cyano($C_2$-$C_6$)alkenylene group; a halo($C_2$-$C_6$)alkenylene group; a ($C_3$-$C_6$)cycloalkyl($C_2$-$C_6$)alkenylene group; a ($C_3$-$C_6$)cycloalkenyl($C_2$-$C_6$)alkenylene group; a ($C_1$-$C_6$) alkylcarbonyl($C_2$-$C_6$)alkenylene group; a ($C_1$-$C_6$)alkoxylcarbonyl($C_2$-$C_6$)alkenylene group, an amino($C_2$-$C_6$)alkenylene group; a mono($C_1$-$C_6$)alkylamino($C_2$-$C_6$)alkenylene group; a di($C_1$-$C_6$)alkylamino($C_2$-$C_6$)alkenylene group whose ($C_1$-$C_6$)alkyl groups may be the same or different; a ($C_1$-$C_6$)alkylcarbonylamino ($C_2$-$C_6$)alkenylene group; a ($C_1$-$C_6$)alkoxycarbonylamino ($C_2$-$C_6$)alkenylene group; a mercapto($C_2$-$C_6$)alkenylene group; a ($C_1$-$C_6$)alkylthio ($C_2$-$C_6$)alkenylene group; a ($C_1$-$C_6$)alkylsulfinyl($C_2$-$C_6$)alkenylene group; a ($C_1$-$C_6$)alkylsulfonyl($C_2$-$C_6$)alkenylene group; a ($C_2$-$C_6$)alkynylene group; a ($C_1$-$C_6$)alkylenoxy group; a ($C_1$-$C_6$)alkyl($C_1$-$C_6$)alkylenoxy group; a ($C_1$-$C_6$) alkylenethio group; a ($C_1$-$C_6$)alkyl($C_1$-$C_6$)alkylenethio group; a ($C_1$-$C_6$)alkyleneamino group; a ($C_1$-$C_6$)alkyl($C_1$-$C_6$) alkyleneamino group; a carbonyl group; a hydroxyimino group; a ($C_1$-$C_6$)alkoxyimino group; a ($C_1$-$C_6$)alkylenedioxy ($C_1$-$C_6$)alkylene group; or a $Z(R^6)_mCO$ group (in the formula, $R^6$, Z, and m are the same as described above);

Ar represents a phenyl group; a substituted phenyl group having at least one substituent which may be the same or different and is selected from the group consisting of a halogen atom, a cyano group, a nitro group, a hydroxyl group, an amino group, a mercapto group, a ($C_1$-$C_6$)alkyl group, a halo($C_1$-$C_6$)alkyl group, a ($C_2$-$C_6$) alkenyl group, a halo($C_2$-$C_6$)alkenyl group, a ($C_2$-$C_6$)alkynyl group, a halo($C_2$-$C_6$)alkynyl group, a ($C_1$-$C_6$)alkoxyl group, a halo($C_1$-$C_6$)alkoxyl group, a ($C_1$-$C_6$)alkylthio group, a halo($C_1$-$C_6$)alkylthio group, a ($C_1$-$C_6$)alkylsulfinyl group, a halo($C_1$-$C_6$)alkylsulfinyl group, a ($C_1$-$C_6$)alkylsulfonyl group, a halo($C_1$-$C_6$)alkylsulfonyl group, a mono ($C_1$-$C_6$)alkylamino group, a di($C_1$-$C_6$)alkylamino group whose ($C_1$-$C_6$)alkyl groups may be the same or difficult, a hydroxyimino($C_1$-$C_6$)alkyl group, a ($C_1$-$C_6$)alkoxyimino ($C_1$-$C_6$)alkyl group, a ($C_2$-$C_6$)alkenyloxyimino($C_1$-$C_6$)alkyl

group, a -$COZ_m(R^6)$ group (in the formula, $R^6$, Z, and m are the same as described above), a -$ZCOZ_m(R^6)$ group (in the formula, $R^6$, Z, and m are the same as described above), a ($C_1$-$C_6$)alkylsulfonylamino group, and a halo ($C_1$-$C_6$)alkylsulfonylamino group; a heterocyclic group (a heterocyclic ring is the same as described above); or a substituted heterocyclic group (a heterocyclic ring is the same as described above) having at least one substituent which may be the same or different and is selected from the group consisting of a halogen atom, a cyano group, a nitro group, a hydroxyl group, an amino group, a mercapto group, a ($C_1$-$C_6$)alkyl group, a halo($C_1$-$C_6$)alkyl group, a ($C_2$-$C_6$)alkenyl group, a halo($C_2$-$C_6$)alkenyl group, a ($C_2$-$C_6$)alkynyl group, a halo($C_2$-$C_6$)alkynyl group, a ($C_1$-$C_6$) alkoxyl group, a halo($C_1$-$C_6$)alkoxyl group, a ($C_1$-$C_6$)alkylthio group, a halo($C_1$-$C_6$)alkylthio group, a ($C_1$-$C_6$)alkylsulfinyl group, a halo($C_1$-$C_6$)alkylsulfinyl group, a ($C_1$-$C_6$)alkylsulfonyl group, a halo($C_1$-$C_6$)alkylsulfonyl group, a mono($C_1$-$C_6$)alkylamino group, a di($C_1$-$C_6$)alkylamino group whose ($C_1$-$C_6$)alkyl groups may be the same or different, a -$COZ_m(R^6)$ group (in the formula, $R^6$, Z, and m are the same as described above), a -$ZCOZ_m(R^6)$ group (in the formula, $R^6$, Z, and m are the same as described above), a ($C_1$-$C_6$)alkylsulfonylamino group, a halo($C_1$-$C_6$) alkylsulfonylamino group, a hydroxyimino($C_1$-$C_6$)alkyl group, a ($C_1$-$C_6$)alkoxyimino($C_1$-$C_6$)alkyl group, and a ($C_2$-$C_6$)alkenyloxyimino($C_1$-$C_6$)alkyl group;

A represents an oxygen atom; a sulfur atom; N=; N($R^7$) (in the formula, $R^7$ is the same as described above); or C($R^9$) (in the formula, $R^9$ represents a hydrogen atom; a halogen atom; a ($C_1$-$C_6$)alkyl group; a halo($C_1$-$C_6$)alkyl group; a ($C_2$-$C_6$)alkenyl group; a halo($C_2$-$C_6$)alkenyl group; a ($C_2$-$C_6$)alkynyl group; a halo ($C_2$-$C_6$)alkynyl group; a ($C_1$-$C_6$)alkoxy($C_1$-$C_6$)alkyl group; a halo($C_1$-$C_6$)alkoxy($C_1$-$C_6$)alkyl group; a ($C_1$-$C_6$)alkylthio($C_1$-$C_6$)alkyl group; a halo($C_1$-$C_6$)alkylthio($C_1$-$C_6$)alkyl group; a ($C_1$-$C_6$)alkylsulfinyl($C_1$-$C_6$)alkyl group; a halo($C_1$-$C_6$)alkylsulfinyl ($C_1$-$C_6$)alkyl group; a ($C_1$-$C_6$)alkylsulfonyl($C_1$-$C_6$)alkyl group; a halo($C_1$-$C_6$)alkylsulfonyl($C_1$-$C_6$)alkyl group; a mono ($C_1$-$C_6$)alkylamino($C_1$-$C_6$)alkyl group; a di($C_1$-$C_6$)alkylamino ($C_1$-$C_6$)alkyl group whose ($C_1$-$C_6$)alkyl groups may be the same or different; a ($C_3$-$C_6$)cycloalkyl group; a halo($C_3$-$C_6$)cycloalkyl group; a ($C_3$-$C_6$)cycloalkenyl group; or a halo($C_3$-$C_6$)cycloalkenyl group), and

B represents N= or N($R^8$) (wherein, $R^8$ is the same as described above).

**4.** The N-(4-pyrazolyl)amide derivative according to Claim 1 represented by the general formula (I-3):

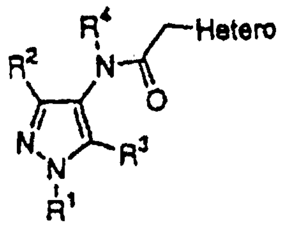

(I-3)

wherein $R^1$ represents a hydrogen atom; a ($C_1$-$C_6$)alkyl group; a halo($C_1$-$C_6$)alkyl group; a hydroxy($C_1$-$C_6$)alkyl group; a cyano($C_1$-$C_6$)alkyl group; a formyl($C_1$-$C_6$)alkyl group; a ($C_2$-$C_6$)alkenyl group; a halo($C_2$-$C_6$)alkenyl group; a ($C_2$-$C_6$)alkynyl group; a halo($C_2$-$C_6$)alkynyl group; a ($C_1$-$C_6$)alkoxy($C_1$-$C_6$)alkyl group; a halo($C_1$-$C_6$)alkoxy ($C_1$-$C_6$)alkyl group; a ($C_1$-$C_6$)alkylthio($C_1$-$C_6$)-alkyl group; a halo($C_1$-$C_6$)alkylthio($C_1$-$C_6$)alkyl group; a ($C_1$-$C_6$)alkylsulfinyl($C_1$-$C_6$)alkyl group; a halo($C_1$-$C_6$)alkylsulfinyl($C_1$-$C_6$)alkyl group; a ($C_1$-$C_6$)alkylsulfonyl($C_1$-$C_6$)alkyl group; a halo($C_1$-$C_6$)alkylsulfonyl($C_1$-$C_6$)alkyl group; a mono($C_1$-$C_6$)alkylamino-($C_1$-$C_6$)alkyl group; a di($C_1$-$C_6$)alkylamino ($C_1$-$C_6$)alkyl group whose ($C_1$-$C_6$)alkyl groups may be the same or different; a ($C_1$-$C_6$)alkylsulfonyl group; a halo ($C_1$-$C_6$)alkylsulfonyl group; a mono($C_1$-$C_6$)alkylaminosulfonyl group; a di($C_1$-$C_6$)alkylaminosulfonyl group whose ($C_1$-$C_6$)alkyl groups may be the same or different; a ($C_1$-$C_6$)alkyloxycarbonyl($C_1$-$C_6$)alkyl group; a mono($C_1$-$C_6$) alkylaminocarbonyl($C_1$-$C_6$)alkyl group; a di($C_1$-$C_6$)-alkylaminocarbonyl($C_1$-$C_6$)alkyl group whose ($C_1$-$C_6$)alkyl groups may be the same or different; a ($C_1$-$C_6$)alkyloxyimino($C_1$-$C_6$)alkyl group; a ($C_1$-$C_6$)alkylsulfonyloxy($C_1$-$C_6$) alkyl group; a halo($C_1$-$C_6$)alkylsulfonyloxy($C_1$-$C_6$)alkyl group; a ($C_1$-$C_6$)alkylcarbonyloxy($C_1$-$C_6$)alkyl group; a phenylsulfonyl group; a substituted phenylsulfonyl group having at least one substituent which may be the same or different and is selected from the group consisting of a halogen atom, a cyano group, a nitro group, a hydroxyl group, an amino group, a mercapto group, a ($C_1$-$C_6$)alkyl group, a halo($C_1$-$C_6$)alkyl group, a ($C_2$-$C_6$)alkenyl group, a halo($C_2$-$C_6$)alkenyl group, a ($C_2$-$C_6$)alkynyl group, a halo($C_2$-$C_6$)alkynyl group, a ($C_1$-$C_6$)alkoxyl group, a halo ($C_1$-$C_6$)alkoxyl group, a ($C_1$-$C_6$)alkylthio group, a halo($C_1$-$C_6$)alkylthio group, a ($C_1$-$C_6$)alkylsulfinyl group, a halo ($C_1$-$C_6$)alkylsulfinyl group, a ($C_1$-$C_6$)alkylsulfonyl group, a halo($C_1$-$C_6$)alkylsulfonyl group, a mono($C_1$-$C_6$)alkylamino group, a di($C_1$-$C_6$)alkylamino group whose ($C_1$-$C_6$)alkyl groups may be the same or different, a ($C_1$-$C_6$)alkylsulfonylamino group, and a halo($C_1$-$C_6$)alkylsulfonylamino group; a ($C_3$-$C_6$)cycloalkyl group; a halo($C_3$-$C_6$)cy-

cloalkyl group; a ($C_3$-$C_6$)cycloalkenyl group; a halo($C_3$-$C_6$)cycloalkenyl group; a phenyl group; a substituted phenyl group having at least one substituent which may be the same or different and is selected from the group consisting of a halogen atom, a cyano group, a nitro group, a hydroxyl group, an amino group, a mercapto group, a ($C_1$-$C_6$) alkyl group, a halo($C_1$-$C_6$)alkyl group, a ($C_2$-$C_6$)alkenyl group, a halo($C_2$-$C_6$)alkenyl group, a ($C_2$-$C_6$)alkynyl group, a halo($C_2$-$C_6$)alkynyl group, a ($C_1$-$C_6$)alkoxyl group, a halo($C_1$-$C_6$)alkoxyl group, a ($C_1$-$C_6$)alkylthio group, a halo ($C_1$-$C_6$)alkylthio group, a ($C_1$-$C_6$)alkylsulfinyl group, a halo($C_1$-$C_6$)alkylsulfinyl group, a ($C_1$-$C_6$)alkylsulfonyl group, a halo($C_1$-$C_6$)alkylsulfonyl group, a mono($C_1$-$C_6$)alkylamino group, a di($C_1$-$C_6$)alkylamino group whose ($C_1$-$C_6$) alkyl groups may be the same or different, a ($C_1$-$C_6$)alkylsulfonylamino group, and a halo($C_1$-$C_6$)alkylsulfonylamino group; or a -$COZ_m(R^6)$ group (wherein, $R^6$ represents a hydrogen atom; a ($C_1$-$C_6$)alkyl group; a halo($C_1$-$C_6$)alkyl group; a ($C_2$-$C_6$)alkenyl group; a halo($C_2$-$C_6$)alkenyl group; a ($C_2$-$C_6$)alkynyl group; a halo($C_2$-$C_6$)alkynyl group; a ($C_1$-$C_6$)alkoxy($C_1$-$C_6$)alkyl group; a halo($C_1$-$C_6$)alkoxy($C_1$-$C_6$)alkyl group; a ($C_1$-$C_6$)alkylthio($C_1$-$C_6$)alkyl group; a halo($C_1$-$C_6$)alkylthio($C_1$-$C_6$)alkyl group; a ($C_1$-$C_6$)alkylsulfinyl($C_1$-$C_6$)alkyl group; a halo($C_1$-$C_6$)alkylsulfinyl($C_1$-$C_6$) alkyl group; a ($C_1$-$C_6$)alkylsulfonyl($C_1$-$C_6$)alkyl group; a halo($C_1$-$C_6$)alkylsulfonyl($C_1$-$C_6$)alkyl group; a mono($C_1$-$C_6$) alkylamino($C_1$-$C_6$)alkyl group; a di ($C_1$-$C_6$)alkylamino($C_1$-$C_6$)alkyl group whose ($C_1$-$C_6$)alkyl groups may be the same or different; a ($C_3$-$C_6$)cycloalkyl group; a halo($C_3$-$C_6$)cycloalkyl group; a ($C_3$-$C_6$)cycloalkenyl group; a halo ($C_3$-$C_6$)cycloalkenyl group; a phenyl group; or a substituted phenyl group having at least one substituent which may be the same or different and is selected from the group consisting of a halogen atom, a cyano group, a nitro group, a hydroxyl group, an amino group, a mercapto group, a ($C_1$-$C_6$)alkyl group, a halo($C_1$-$C_6$)alkyl group, a ($C_2$-$C_6$)alkenyl group, a halo($C_2$-$C_6$)alkenyl group, a ($C_2$-$C_6$)alkynyl group, a halo($C_2$-$C_6$)alkynyl group, a ($C_1$-$C_6$) alkoxyl group, a halo($C_1$-$C_6$)alkoxyl group, a ($C_1$-$C_6$)alkylthio group, a halo($C_1$-$C_6$)alkylthio group, a ($C_1$-$C_6$)alkylsulfinyl group, a halo($C_1$-$C_6$)alkylsulfinyl group, a ($C_1$-$C_6$)alkylsulfonyl group, a halo($C_1$-$C_6$)alkylsulfonyl group, a mono($C_1$-$C_6$)alkylamino group, a di($C_1$-$C_6$)alkylamino group whose ($C_1$-$C_6$)alkyl groups may be the same or different, a ($C_1$-$C_6$)alkylsulfonylamino group, and a halo($C_1$-$C_6$)alkylsulfonylamino group; Z represents an oxygen atom; a sulfur atom; or $N(R^7)$ [wherein, $R^7$ represents a hydrogen atom; a ($C_1$-$C_6$)alkyl group; a halo($C_1$-$C_6$)alkyl group; a ($C_2$-$C_6$)alkenyl group; a halo($C_2$-$C_6$)alkenyl group; a ($C_2$-$C_6$)alkynyl group; a halo($C_2$-$C_6$)alkynyl group; a ($C_1$-$C_6$)alkoxy($C_1$-$C_6$)alkyl group; a halo($C_1$-$C_6$)alkoxy($C_1$-$C_6$)alkyl group; a ($C_1$-$C_6$)alkylthio($C_1$-$C_6$)alkyl group; a halo($C_1$-$C_6$)alkylthio($C_1$-$C_6$)alkyl group; a ($C_1$-$C_6$)alkylsulfinyl($C_1$-$C_6$)alkyl group; a halo($C_1$-$C_6$)alkylsulfinyl-($C_1$-$C_6$) alkyl group; a ($C_1$-$C_6$)alkylsulfonyl($C_1$-$C_6$)alkyl group; a halo($C_1$-$C_6$)alkylsulfonyl($C_1$-$C_6$)alkyl group; a mono($C_1$-$C_6$) alkylamino($C_1$-$C_6$)alkyl group; a di($C_1$-$C_6$)alkylamino($C_1$-$C_6$)alkyl group whose ($C_1$-$C_6$)alkyl groups may be the same or different; a ($C_3$-$C_6$)cycloalkyl group; a halo($C_3$-$C_6$)cycloalkyl group; a ($C_3$-$C_6$)cycloalkenyl group; or a halo ($C_3$-$C_6$)cycloalkenyl group]; and m represents an integer of 0 to 1};

$R^2$ and $R^3$ may be the same or different, and represent a hydrogen atom; a halogen atom; a cyano group; a nitro group; a hydroxyl group; a mercapto group; an amino group; a ($C_1$-$C_6$)alkyl group; a halo($C_1$-$C_6$)alkyl group; a ($C_2$-$C_6$)alkenyl group; a halo($C_2$-$C_6$)alkenyl group; a ($C_2$-$C_6$)alkynyl group; a halo($C_2$-$C_6$)alkynyl group; a ($C_1$-$C_6$) alkoxyl group; a halo($C_1$-$C_6$)alkoxyl group; a ($C_1$-$C_6$)alkylthio group; a halo($C_1$-$C_6$)alkylthio group; a ($C_1$-$C_6$)alkylsulfinyl group; a halo($C_1$-$C_6$)alkylsulfinyl group; a ($C_1$-$C_6$)alkylsulfonyl group; a halo($C_1$-$C_6$)alkylsulfonyl group; a mono($C_1$-$C_6$)alkylamino group; a di($C_1$-$C_6$)alkylamino group whose ($C_1$-$C_6$)alkyl groups may be the same or different; a ($C_1$-$C_6$)alkoxy($C_1$-$C_6$)alkyl group; a halo($C_1$-$C_6$)alkoxy($C_1$-$C_6$)alkyl group; a ($C_1$-$C_6$)alkylthio($C_1$-$C_6$)alkyl group; a halo($C_1$-$C_6$)alkylthio($C_1$-$C_6$)alkyl group; a ($C_1$-$C_6$)alkylsulfinyl($C_1$-$C_6$)alkyl group; a halo($C_1$-$C_6$)alkylsulfinyl($C_1$-$C_6$)alkyl group; a ($C_1$-$C_6$) alkylsulfonyl($C_1$-$C_6$)alkyl group; a halo($C_1$-$C_6$)alkylsulfonyl($C_1$-$C_6$)alkyl group; a mono($C_1$-$C_6$)alkylamino($C_1$-$C_6$)alkyl group; a di($C_1$-$C_6$)alkylamino($C_1$-$C_6$)alkyl group whose ($C_1$-$C_6$)alkyl groups may be the same or different; a ($C_3$-$C_6$)cycloalkyl group; a halo($C_3$-$C_6$)cycloalkyl group; a ($C_3$-$C_6$)cycloalkenyl group; a halo($C_3$-$C_6$)cycloalkenyl group; a phenyl group; a substituted phenyl group having at least one substituent which may be the same or different and is selected from the group consisting of a halogen atom, a cyano group, a nitro group, a hydroxyl group, an amino group, a mercapto group, a ($C_1$-$C_6$)alkyl group, a halo($C_1$-$C_6$)alkyl group, a ($C_2$-$C_6$)alkenyl group, a halo($C_2$-$C_6$)-alkenyl group, a ($C_2$-$C_6$)alkynyl group, a halo($C_2$-$C_6$)alkynyl group, a ($C_1$-$C_6$) alkoxyl group, a halo($C_1$-$C_6$)alkoxyl group, a ($C_1$-$C_6$)alkylthio group, a halo($C_1$-$C_6$)alkylthio group, a ($C_1$-$C_6$)alkylsulfinyl group, a halo($C_1$-$C_6$)alkylsulfinyl group, a ($C_1$-$C_6$)alkylsulfonyl group, a halo($C_1$-$C_6$)alkylsulfonyl group, a mono($C_1$-$C_6$)alkylamino group, a di($C_1$-$C_6$)alkylamino group whose ($C_1$-$C_6$)alkyl groups may be the same or different, a ($C_1$-$C_6$)alkylsulfonylamino group, and a halo($C_1$-$C_6$)alkylsulfonylamino group; a phenoxy group; a substituted phenoxy group having at least one substituent which may be the same or different and is selected from the group consisting of a halogen atom, a cyano group, a nitro group, a hydroxyl group, an amino group, a mercapto group, a ($C_1$-$C_6$)alkyl group, a halo($C_1$-$C_6$)alkyl group, a ($C_2$-$C_6$)alkenyl group, a halo($C_2$-$C_6$)alkenyl group, a ($C_2$-$C_6$)alkynyl group, a halo($C_2$-$C_6$)alkynyl group, a ($C_1$-$C_6$)alkoxyl group, a halo($C_1$-$C_6$)alkoxyl group, a ($C_1$-$C_6$) alkylthio group, a halo($C_1$-$C_6$)alkylthio group, a ($C_1$-$C_6$)alkylsulfinyl group, a halo($C_1$-$C_6$)alkylsulfinyl group, a ($C_1$-$C_6$)alkylsulfonyl group, a halo($C_1$-$C_6$)alkylsulfonyl group, a mono ($C_1$-$C_6$)alkylamino group, a di($C_1$-$C_6$) alkylamino group whose ($C_1$-$C_6$)alkyl groups may be the same or different, a ($C_1$-$C_6$)alkylsulfonylamino group, and a halo($C_1$-$C_6$)alkylsulfonylamino group; a phenylthio group; a substituted phenylthio group having at least one

substituent which may be the same or different and is selected from the group consisting of a halogen atom, a cyano group, a nitro group, a hydroxyl group, an amino group, a mercapto group, a $(C_1-C_6)$alkyl group, a halo $(C_1-C_6)$alkyl group, a $(C_2-C_6)$alkenyl group, a halo$(C_2-C_6)$alkenyl group, a $(C_2-C_6)$alkynyl group, a halo$(C_2-C_6)$ alkynyl group, a $(C_1-C_6)$alkoxyl group, a halo$(C_1-C_6)$alkoxyl group, a $(C_1-C_6)$alkylthio group, a halo$(C_1-C_6)$alkylthio group, a $(C_1-C_6)$alkylsulfinyl group, a halo$(C_1-C_6)$alkylsulfinyl group, a $(C_1-C_6)$alkylsulfonyl group, a halo$(C_1-C_6)$ alkylsulfonyl group, a mono$(C_1-C_6)$alkylamino group, a di$(C_1-C_6)$alkylamino group whose $(C_1-C_6)$alkyl groups may be the same or different, a $(C_1-C_6)$alkylsulfonylamino group, and a halo$(C_1-C_6)$alkylsulfonylamino group; a -$ZCOZ_m$ $(R^6)$ group (wherein, $R^6$, Z, and m are the same as described above); or a -$COZ_m(R^6)$ group (wherein, $R^6$, Z, and m are the same as described above); $R^4$ represents a hydrogen atom; a $(C_1-C_6)$alkyl group; a halo$(C_1-C_6)$alkyl group; a cyano$(C_1-C_6)$alkyl group; a $(C_2-C_6)$alkenyl group; a halo$(C_2-C_6)$alkenyl group; a $(C_2-C_6)$alkynyl group; a halo$(C_2-C_6)$alkynyl group; a $(C_1-C_6)$alkoxy$(C_1-C_6)$alkyl group; a halo$(C_1-C_6)$alkoxy$(C_1-C_6)$alkyl group; a $(C_1-C_6)$ alkoxy$(C_1-C_6)$-alkoxy$(C_1-C_6)$alkyl group; a $(C_1-C_6)$alkylthio$(C_1-C_6)$alkyl group; a halo$(C_1-C_6)$alkylthio$(C_1-C_6)$alkyl group; a $(C_1-C_6)$alkylsulfinyl$(C_1-C_6)$alkyl group; a halo$(C_1-C_6)$alkylsulfinyl$(C_1-C_6)$alkyl group; a $(C_1-C_6)$alkylsulfonyl $(C_1-C_6)$alkyl group; a halo$(C_1-C_6)$alkylsulfonyl-$(C_1-C_6)$alkyl group; a mono $(C_1-C_6)$alkylamino $(C_1-C_6)$alkyl group; a di$(C_1-C_6)$alkylamino $(C_1-C_6)$alkyl group whose $(C_1-C_6)$alkyl groups may be the same or different; a $(C_3-C_6)$cycloalkyl group; a halo$(C_3-C_6)$cycloalkyl group; a $(C_3-C_6)$cycloalkenyl group; a halo$(C_3-C_6)$cycloalkenyl group; a -$COZ_m(R^6)$ group (wherein, $R^6$, Z, and m are the same as described above), a -CSNH$(R^6)$ group (wherein, $R^6$, is the same as described above), a $(C_1-C_6)$alkylcarbonyl $(C_1-C_6)$alkyl group; a $(C_1-C_6)$alkoxycarbonyl$(C_1-C_6)$alkyl group; a phenyl$(C_1-C_6)$alkyl group; a substituted phenyl$(C_1-C_6)$alkyl group having on the ring at least one substituent which may be the same or different and is selected from the group consisting of a halogen atom, a cyano group, a nitro group, a hydroxyl group, an amino group, a mercapto group, a $(C_1-C_6)$alkyl group, a halo$(C_1-C_6)$ alkyl group, a $(C_2-C_6)$alkenyl group, a halo$(C_2-C_6)$alkenyl group, a $(C_2-C_6)$alkynyl group, a halo$(C_2-C_6)$alkynyl group, a $(C_1-C_6)$alkoxyl group, a halo$(C_1-C_6)$alkoxyl group, a $(C_1-C_6)$alkylthio group, a halo$(C_1-C_6)$alkylthio group, a $(C_1-C_6)$alkylsulfinyl group, a halo $(C_1-C_6)$alkylsulfinyl group, a $(C_1-C_6)$alkylsulfonyl group, a halo$(C_1-C_6)$alkylsulfonyl group, a mono$(C_1-C_6)$alkylamino group, a di$(C_1-C_6)$alkylamino group whose $(C_1-C_6)$alkyl groups may be the same or different, a $(C_1-C_6)$alkylsulfonylamino group, and a halo$(C_1-C_6)$alkylsulfonylamino group; a heterocyclic $(C_1-C_6)$alkyl group (a heterocyclic ring represents a pyridyl group; a pyridine-N-oxide group; a pyridazinyl group; a pyrimidinyl group; a pyrazyl group; a piperidyl group; a piperazino group; a morpholinyl group; a morpholino group; a furyl group; a tetrahydrofuryl group; a thienyl group; a tetrahydrothienyl group; a pyrrolyl group; a pyrrolidyl group; an imidazolidinyl group; an oxazolyl group; an isoxazolyl group; an oxadiazolyl group; a thiazolyl group; an isothiazolyl group; a thiadiazolyl group; an imidazolyl group; a triazolyl group; a pyrazolyl group; an indolyl group; a benzofuryl group; a benzothienyl group; an indazolyl group; a quinolyl group; an isoquinolyl group; or a quinazolyl group); or a substituted heterocyclic$(C_1-C_6)$alkyl group (the heterocyclic group is the same as the above-described) having on the ring at least one substituent which may be the same or different and is selected from the group consisting of a halogen atom, a cyano group, a nitro group, a hydroxyl group, an amino group, a mercapto group, a $(C_1-C_6)$alkyl group, a halo$(C_1-C_6)$alkyl group, a $(C_2-C_6)$alkenyl group, a halo$(C_2-C_6)$alkenyl group, a $(C_2-C_6)$ alkynyl group, a halo $(C_2-C_6)$alkynyl group, a $(C_1-C_6)$alkoxyl group, a halo$(C_1-C_6)$alkoxyl group, a $(C_1-C_6)$alkylthio group, a halo$(C_1-C_6)$alkylthio group, a $(C_1-C_6)$alkylsulfinyl group, a halo$(C_1-C_6)$alkylsulfinyl group, a $(C_1-C_6)$alkylsulfonyl group, a halo$(C_1-C_6)$alkylsulfonyl group, a mono$(C_1-C_6)$alkylamino group, a di$(C_1-C_6)$alkylamino group whose $(C_1-C_6)$alkyl groups may be the same or different, a $(C_1-C_6)$alkylsulfonylamino group, and a halo$(C_1-C_6)$ alkylsulfonylamino group;

"Hetero" represents a heterocyclic group (a heterocyclic ring is the same as described above); or a substituted heterocyclic group having at least one substituent which may be the same or different and is selected from the group consisting of a halogen atom, a cyano group, a nitro group, a hydroxyl group, a mercapto group, an amino group, a halo$(C_1-C_6)$alkyl group (with the proviso of excluding a trifluoromethyl group), a $(C_2-C_6)$alkenyl group, a halo$(C_2-C_6)$alkenyl group, a $(C_2-C_6)$alkynyl group, a halo$(C_2-C_6)$alkynyl group, a halo$(C_1-C_6)$alkoxyl group, a halo $(C_1-C_6)$alkylthio group, a $(C_1-C_6)$alkylsulfinyl group, a halo$(C_1-C_6)$alkylsulfinyl group, a $(C_1-C_6)$alkylsulfonyl group, a halo$(C_1-C_6)$alkylsulfonyl group, a mono$(C_1-C_6)$alkylamino group, a di$(C_1-C_6)$alkylamino group whose $(C_1-C_6)$ alkyl groups may be the same or different, a $(C_1-C_6)$alkoxy$(C_1-C_6)$alkyl group, a halo$(C_1-C_6)$alkoxy$(C_1-C_6)$alkyl group, a $(C_1-C_6)$alkylthio$(C_1-C_6)$alkyl group, a halo$(C_1-C_6)$alkylthio$(C_1-C_6)$alkyl group, a $(C_1-C_6)$alkylsulfinyl $(C_1-C_6)$alkyl group, a halo$(C_1-C_6)$alkylsulfinyl$(C_1-C_6)$alkyl group, a $(C_1-C_6)$alkylsulfonyl$(C_1-C_6)$alkyl group, a halo $(C_1-C_6)$alkylsulfonyl$(C_1-C_6)$alkyl group, a mono$(C_1-C_6)$alkylamino$(C_1-C_6)$alkyl group, a di$(C_1-C_6)$alkylamino-$(C_1-C_6)$alkyl group whose $(C_1-C_6)$alkyl groups may be the same or different, a $(C_3-C_6)$cycloalkyl group, a halo $(C_3-C_6)$cycloalkyl group, a hydroxyimino$(C_1-C_6)$alkyl group, a $(C_1-C_6)$alkoxyimino$(C_1-C_6)$alkyl group, a $(C_2-C_6)$ alkenyloxyimino$(C_1-C_6)$alkyl group, a -$COZ_m(R^6)$ group (in the formula, $R^6$, Z, and m are the same as described above), a -$ZCOZ_m(R^6)$ group (in the formula, $R^6$, Z, and m are the same as described above), a $(C_1-C_6)$alkylsulfonylamino group, a halo$(C_1-C_6)$alkylsulfonylamino group, a phenyl group, a substituted phenyl group having on the ring at least one substituent selected from the group consisting of same or different an amino group, a mercapto

group, a $(C_1-C_6)$alkyl group, a halo$(C_1-C_6)$alkyl group (with the proviso of excluding a trifluoromethyl group), a $(C_2-C_6)$alkenyl group, a halo$(C_2-C_6)$alkenyl group, a $(C_2-C_6)$alkynyl group, a halo$(C_2-C_6)$alkynyl group, a $(C_1-C_6)$ alkoxyl group, a halo$(C_1-C_6)$alkoxyl group, a $(C_1-C_6)$alkylthio group, a halo$(C_1-C_6)$alkylthio group, a $(C_1-C_6)$alkylsulfinyl group, a halo$(C_1-C_6)$alkylsulfinyl group, a $(C_1-C_6)$alkylsulfonyl group, a halo$(C_1-C_6)$alkylsulfonyl group, a mono$(C_1-C_6)$alkylamino group, a di$(C_1-C_6)$alkylamino group whose $(C_1-C_6)$alkyl groups may be the same or different, a -$COZ_m(R^6)$ group (in the formula, $R^6$, Z, and m are the same as described above), a -$ZCOZ_m(R^6)$ group (in the formula, $R^6$, Z, and m are the same as described above), a $(C_1-C_6)$alkylsulfonylamino group, and a halo $(C_1-C_6)$alkylsulfonylamino group, a heterocyclic group (a heterocyclic ring is the same as described above), a substituted heterocyclic group (a heterocyclic ring is the same as described above) having on the ring at least one substituent which may be the same or different and is selected from the group consisting of same or different a halogen atom, a cyano group, a nitro group, a hydroxyl group, an amino group, a mercapto group, a $(C_1-C_6)$alkyl group, a halo$(C_1-C_6)$alkyl group, a $(C_2-C_6)$alkenyl group, a halo$(C_2-C_6)$alkenyl group, a $(C_2-C_6)$alkynyl group, a halo$(C_2-C_6)$alkynyl group, a $(C_1-C_6)$alkoxyl group, a halo$(C_1-C_6)$alkoxyl group, a $(C_1-C_6)$alkylthio group, a halo $(C_1-C_6)$alkylthio group, a $(C_1-C_6)$alkylsulfinyl group, a halo$(C_1-C_6)$alkylsulfinyl group, a $(C_1-C_6)$alkylsulfonyl group, a halo$(C_1-C_6)$alkylsulfonyl group, a mono$(C_1-C_6)$alkylamino group, a di$(C_1-C_6)$alkylamino group whose $(C_1-C_6)$ alkyl groups may be the same or different, a -$COZ_m(R^6)$ group (in the formula, $R^6$, Z, and m are the same as described above), a -$ZCOZ_m(R^6)$ group (in the formula, $R^6$, Z, and m are the same as described above), a $(C_1-C_6)$ alkylsulfonylamino group, and a halo$(C_1-C_6)$-alkylsulfonylamino group, and -Q-Ar [wherein, Q represents an oxygen atom; a sulfur atom; $N(R^7)$ (wherein, $R^7$ is the same as described above); a $(C_1-C_6)$alkylene group; a $(C_1-C_6)$ alkyl$(C_1-C_6)$alkylene group; a halo$(C_1-C_6)$alkyl$(C_1-C_6)$alkylene group; a hydroxy$(C_1-C_6)$alkyl$(C_1-C_6)$alkylene group; a $(C_1-C_6)$alkoxy$(C_1-C_6)$alkyl$(C_1-C_6)$alkylene group; a $(C_2-C_6)$alkenyl$(C_1-C_6)$alkylene group; a halo$(C_2-C_6)$ alkenyl$(C_1-C_6)$alkylene group; a hydroxy$(C_1-C_6)$alkylene group; a $(C_1-C_6)$alkoxy$(C_1-C_6)$alkylene group; a halo $(C_1-C_6)$alkoxy $(C_1-C_6)$alkylene group; a $(C_1-C_6)$alkylcarbonyloxy$(C_1-C_6)$alkylene group; a cyano$(C_1-C_6)$alkylene group; a halo$(C_1-C_6)$alkylene group; a $(C_3-C_6)$cycloalkyl$(C_1-C_6)$alkylene group; a $(C_3-C_6)$cycloalkenyl$(C_1-C_6)$ alkylene group; a $(C_1-C_6)$alkylcarbonyl$(C_1-C_6)$alkylene group; a $(C_1-C_6)$alkoxycarbonyl $(C_1-C_6)$alkylene group; an amino$(C_1-C_6)$alkylene group; a mono$(C_1-C_6)$alkylamino$(C_1-C_6)$alkylene group; a di$(C_1-C_6)$alkylamino$(C_1-C_6)$ alkylene group whose $(C_1-C_6)$alkyl groups may be the same or different; a $(C_1-C_6)$alkylcarbonylamino$(C_1-C_6)$ alkylene group; a $(C_1-C_6)$alkoxy carbonylamino$(C_1-C_6)$alkylene group; a mercapto $(C_1-C_6)$alkylene group; a $(C_1-C_6)$alkylthio$(C_1-C_6)$alkylene group; an alkylsulfinyl$(C_1-C_6)$alkylene group; a $(C_1-C_6)$alkylsulfonyl$(C_1-C_6)$ alkylene group; a $(C_3-C_6)$-cycloalkylene group; a $(C_2-C_6)$alkenylene group; a $(C_1-C_6)$alkyl$(C_2-C_6)$alkenylene group; a halo$(C_1-C_6)$alkyl$(C_2-C_6)$alkenylene group; a hydroxy$(C_1-C_6)$alkyl$(C_2-C_6)$alkenylene group; a $(C_1-C_6)$alkoxy $(C_1-C_6)$alkyl$(C_2-C_6)$alkenylene group; a $(C_2-C_6)$alkenyl$(C_2-C_6)$alkenylene group; a halo-$(C_2-C_6)$alkenyl$(C_2-C_6)$ alkenylene group; a $(C_2-C_6)$alkynyl-$(C_2-C_6)$alkenylene group; a halo$(C_2-C_6)$alkynyl$(C_2-C_6)$alkenylene group; a hydroxy$(C_2-C_6)$alkenylene group; a $(C_1-C_6)$alkoxy$(C_2-C_6)$alkenylene group; a halo $(C_1-C_6)$alkoxy-$(C_2-C_6)$alkenylene group; a $(C_1-C_6)$alkylcarbonyloxy$(C_2-C_6)$alkenylene group; a cyano$(C_2-C_6)$alkenylene group; a halo $(C_2-C_6)$ alkenylene group; a $(C_3-C_6)$ cycloalkyl $(C_2-C_6)$alkenylene group; a $(C_3-C_6)$cycloalkenyl $(C_2-C_6)$alkenylene group; a $(C_1-C_6)$alkylcarbonyl$(C_2-C_6)$alkenylene group; a $(C_1-C_6)$alkoxycarbonyl$(C_2-C_6)$alkenylene group; an amino $(C_2-C_6)$alkenylene group; a mono $(C_1-C_6)$alkylamino$(C_2-C_6)$alkenylene group; a di$(C_1-C_6)$alkylamino$(C_2-C_6)$alkenylene group whose $(C_1-C_6)$alkyl groups may be the same or different; a $(C_1-C_6)$alkylcarbonylamino$(C_2-C_6)$alkenylene group; a $(C_1-C_6)$-alkoxycarbonylamino$(C_2-C_6)$alkenylene group; a mercapto$(C_2-C_6)$alkenylene group; a $(C_1-C_6)$alkylthio $(C_2-C_6)$alkenylene group; a $(C_1-C_6)$alkylsulfinyl$(C_2-C_6)$alkenylene group; a $(C_1-C_6)$alkylsulfonyl $(C_2-C_6)$alkenylene group; a $(C_2-C_6)$alkynylene group; a $(C_1-C_6)$alkylenoxy group; a $(C_1-C_6)$alkyl$(C_1-C_6)$alkylenoxy group; a $(C_1-C_6)$alkylenethio group; a $(C_1-C_6)$alkyl$(C_1-C_6)$alkylenethio group; a $(C_1-C_6)$alkyleneamino group; a $(C_1-C_6)$alkyl$(C_1-C_6)$alkylene amino group; a carbonyl group; a hydroxyimino group; a $(C_1-C_6)$alkoxyimino group; an alkylenedioxyalkylene group; or a $Z(R^6)_mCO$ group (in the formula, $R^6$, Z, and m are the same as described above);

Ar represents a phenyl group; a substituted phenyl group having at least one substituent which may be the same or different and is selected from the group consisting of a halogen atom, a cyano group, a nitro group, a hydroxyl group, an amino group, a mercapto group, a $(C_1-C_6)$alkyl group, a halo$(C_1-C_6)$alkyl group, a $(C_2-C_6)$ alkenyl group, a halo$(C_2-C_6)$alkenyl group, a $(C_2-C_6)$alkynyl group, a halo$(C_2-C_6)$alkynyl group, a $(C_1-C_6)$alkoxyl group, a halo$(C_1-C_6)$alkoxyl group, a $(C_1-C_6)$alkylthio group, a halo$(C_1-C_6)$alkylthio group, a $(C_1-C_6)$alkylsulfinyl group, a halo$(C_1-C_6)$alkylsulfinyl group, a $(C_1-C_6)$alkylsulfonyl group, a halo$(C_1-C_6)$alkylsulfonyl group, a mono $(C_1-C_6)$alkylamino group, a di$(C_1-C_6)$alkylamino group whose $(C_1-C_6)$alkyl groups may be the same or different, a hydroxyimino$(C_1-C_6)$alkyl group, a $(C_1-C_6)$alkoxyimino$(C_1-C_6)$alkyl group, a $(C_2-C_6)$alkenyloxyimino$(C_1-C_6)$alkyl group, a -$COZ_m(R^6)$ group (in the formula, $R^6$, Z, and m are the same as described above), a -$ZCOZ_m(R^6)$ group (in the formula, $R^6$, Z, and m are the same as described above), a $(C_1-C_6)$alkylsulfonylamino group, and a halo $(C_1-C_6)$alkylsulfonylamino group; a naphthyl group; a substituted naphthyl group having at least one substituent which may be the same or different and is selected from the group consisting of a halogen atom, a cyano group,

a nitro group, a hydroxyl group, an amino group, a mercapto group, a ($C_1$-$C_6$)alkyl group, a halo($C_1$-$C_6$)alkyl group, a ($C_2$-$C_6$)alkenyl group, a halo($C_2$-$C_6$)alkenyl group, a ($C_2$-$C_6$)alkynyl group, a halo($C_2$-$C_6$)alkynyl group, a ($C_1$-$C_6$) alkoxyl group, a halo($C_1$-$C_6$)alkoxyl group, a ($C_1$-$C_6$)alkylthio group, a halo($C_1$-$C_6$)alkylthio group, a ($C_1$-$C_6$)alkylsulfinyl group, a halo($C_1$-$C_6$)alkylsulfinyl group, a ($C_1$-$C_6$)alkylsulfonyl group, a halo($C_1$-$C_6$)alkylsulfonyl group, a mono($C_1$-$C_6$)alkylamino group, a di($C_1$-$C_6$)alkylamino group whose ($C_1$-$C_6$)alkyl groups may be the same or different, a hydroxyimino($C_1$-$C_6$)alkyl group, a ($C_1$-$C_6$)-alkoxyimino($C_1$-$C_6$)alkyl group, and a ($C_2$-$C_6$)alkenyloxyimino ($C_1$-$C_6$)alkyl group, a -$COZ_m(R^6)$ group (in the formula, $R^6$, Z, and m are the same as described above), a -$ZCOZ_m$ ($R^6$) group (in the formula, $R^6$, Z, and m are the same as described above), a ($C_1$-$C_6$)alkylsulfonylamino group, and a halo($C_1$-$C_6$)alkylsulfonylamino group; a heterocyclic group (a heterocyclic ring is the same as described above); or a substituted heterocyclic group (a heterocyclic ring is the same as described above) having at least one substituent which may be the same or different and is selected from the group consisting of a halogen atom, a cyano group, a nitro group, a hydroxyl group, an amino group, a mercapto group, a ($C_1$-$C_6$)alkyl group, a halo ($C_1$-$C_6$)alkyl group, a ($C_2$-$C_6$)alkenyl group, a halo($C_2$-$C_6$)alkenyl group, a ($C_2$-$C_6$)alkynyl group, a halo($C_2$-$C_6$) alkynyl group, a ($C_1$-$C_6$)alkoxyl group, a halo($C_1$-$C_6$)alkoxyl group, a ($C_1$-$C_6$)alkylthio group, a halo($C_1$-$C_6$)alkylthio group, a ($C_1$-$C_6$)alkylsulfinyl group, a halo($C_1$-$C_6$)alkylsulfinyl group, a ($C_1$-$C_6$)alkylsulfonyl group, a halo($C_1$-$C_6$) alkylsulfonyl group, a mono($C_1$-$C_6$)alkylamino group, a di($C_1$-$C_6$)alkylamino group whose ($C_1$-$C_6$)alkyl groups may be the same or different , a -$COZ_m(R^6)$ group (in the formula, $R^6$, Z, and m are the same as described above), a -$ZCOZ_m(R^6)$ group (in the formula, $R^6$, Z, and m are the same as described above), a ($C_1$-$C_6$)alkylsulfonylamino group, a halo($C_1$-$C_6$)alkylsulfonylamino group, a hydroxy($C_1$-$C_6$)alkyl group, a ($C_1$-$C_6$)alkoxyimino($C_1$-$C_6$)alkyl group, and a ($C_2$-$C_6$)-alkenyloxyimino($C_1$-$C_6$)alkyl group].

**5.** An agricultural and horticultural chemical according to any one of Claims 1 to 4, wherein an N-(4-pyrazolyl)amide derivative is contained as an active ingredient.

**6.** The agricultural and horticultural chemical according to Claim 5, wherein the agricultural or horticultural chemical is a germicide or a fungicide.

**7.** The agricultural and horticultural chemical according to Claim 5, wherein the agricultural or horticultural chemical is an insecticide or a nematocide.

**8.** A method for using an agricultural and horticultural chemical in order to control a botanical germ, a fungus, a noxious insect, a nematode or the like, which is harmful, from a useful crop, wherein an objective crop or soil is treated with an effective amount of the agricultural and horticultural chemical according to any one of Claims 5 to 7.

**9.** The method for using an agricultural and horticultural chemical according to Claim 8, wherein the agricultural or horticultural chemical is a germicide or a fungicide.

**10.** The method for using an agricultural and horticultural chemical according to Claim 8, wherein the agricultural or horticultural chemical is an insecticide or a nematocide.

**INTERNATIONAL SEARCH REPORT**

International application No.
PCT/JP01/08242

A. CLASSIFICATION OF SUBJECT MATTER
Int.Cl[7] C07D231/40, 401/12, 403/12, 405/12, 409/12, 413/12, 417/12, A01N43/50

According to International Patent Classification (IPC) or to both national classification and IPC

B. FIELDS SEARCHED

Minimum documentation searched (classification system followed by classification symbols)
Int.Cl[7] C07D231/40, 401/12, 403/12, 405/12, 409/12, 413/12, 417/12, A01N43/50

Documentation searched other than minimum documentation to the extent that such documents are included in the fields searched

Electronic data base consulted during the international search (name of data base and, where practicable, search terms used)
CAPLUS (STN), REGISTRY (STN)

C. DOCUMENTS CONSIDERED TO BE RELEVANT

| Category* | Citation of document, with indication, where appropriate, of the relevant passages | Relevant to claim No. |
|---|---|---|
| X<br>Y | JP 62-153283 A (Tokuyama Soda Co., Ltd.), 08 July, 1987 (08.07.87), Claims; chemical compound Nos. 12, 16, 17, 21 (Family: none) | 1,5,6,8,9<br>1-9 |
| X<br>Y | JP 62-153273 A (Tokuyama Soda Co., Ltd.), 08 July, 1987 (08.07.87), Claims; chemical compound Nos. 26 to 30, 34, 35, 55 (Family: none) | 1,5,6,8,9<br>1-9 |
| X<br>Y | JP 62-138475 A (Tokuyama Soda Co., Ltd.), 22 June, 1987 (22.06.87), Claims; chemical compound Nos. 37 to 39 (Family: none) | 1,5,6,8,9<br>1-9 |

☒ Further documents are listed in the continuation of Box C. ☐ See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 12 December, 2001 (12.12.01) | 25 December, 2001 (25.12.01) |
| Name and mailing address of the ISA/<br>Japanese Patent Office | Authorized officer |
| Facsimile No. | Telephone No. |

Form PCT/ISA/210 (second sheet) (July 1992)

**INTERNATIONAL SEARCH REPORT**

International application No.
PCT/JP01/08242

C (Continuation). DOCUMENTS CONSIDERED TO BE RELEVANT

| Category* | Citation of document, with indication, where appropriate, of the relevant passages | Relevant to claim No. |
|---|---|---|
| Y | WO 97/26251 A1 (Bayer Aktiengsellschaft), 23 July, 1997 (23.07.97), Full text; especially, Claims & DE 19601139 A & JP 2000-503307 A & US 5942528 A & CN 1208408 A & KR 99077064 A & BR 9706986 A & EP 877741 A1 & NZ 326316 A & HU 9900971 A & AU 9714387 A | 1-9 |
| Y | WO 93/04580 A1 (Dowrlanco), 18 March, 1993 (18.03.93), Full text & FI 9301971 A & AU 9226418 A & EP 555469 A1 & EP 556381 A1 & JP 5-221990 A & BR 9205385 A & BR 9205386 A | 1-9 |
| Y | JP 63-313773 A (Tokuyama Soda Co., Ltd.), 21 December, 1988 (21.12.88), Claims (Family: none) | 1,5,6,8,9 |
| X | JP 63-174905 A (Tokuyama Soda Co., Ltd.), 19 July, 1988 (19.07.88), Claims; working example (Family: none) | 1,5 |
| X | EP 911333 A1 (Pfizer Limited), 28 April, 1999 (28.04.99), working example of each raw material chemical compound & US 6235742 A & CA 2251453 A & BR 9804214 A & JP 11-217383 A | 1,2 |
| PX | WO 01/07020 A2 (Boehringer Ingelheim Pharma. KG), 01 February, 2001 (01.02.01), working example 125 & DE 19935219 A | 1 |

Form PCT/ISA/210 (continuation of second sheet) (July 1992)